# EUROPEAN PATENT APPLICATION

(11) **EP 3 719 118 A1**
(43) Date of publication of application: **07.10.2020**
(21) Application number: 20160436.0
(22) Date of filing: 02.01.2014
(51) Int. Cl.: C12N 5/0784, A61K 35/15

(54) **METHOD FOR OBTAINING IMMUNO-SUPPRESSIVE DENDRITIC CELLS**

(30) Priority: 03.01.2013 GB 201300052; 03.01.2013 US 201361748550 P
(62) Divisional of application: 14700050.9
(71) Applicant: Transimmune AG, 40212 Düsseldorf (DE); Yale University, New Haven, CT 06511 (US)
(72) Inventor: Henco, Karsten, 40629 Düsseldorf (DE); Bauer, Günter, 24640 Schmalfeld (DE); Duckworth, Justin, Weybridge, Surrey KT13 8XB (GB); Hayday, Adrian, Orpington, Kent BR6 9DR (GB); Edelson, Richard, Westport, CT 06880 (US); Tigelaar, Robert, New Haven, CT 06512 (US); Girardi, Michael, Madison, CT 06443 (US)
(74) Representative: Bühler, Dirk

(57) **Abstract**

The present invention relates to methods for producing immuno-suppressive dendritic cells. The present invention further relates to the use of such cells for treating patients suffering from autoimmune diseases, hypersensitivity diseases, rejection on solid-organ transplantation and/or Graft-versus-Host disease.

## Description

### FIELD OF THE INVENTION

The present invention relates to methods for producing immuno-suppressive dendritic cells. The present invention further relates to the use of such cells for treating patients undergoing solid-organ transplantation and/or suffering from Graft-versus-Host-disease, autoimmune diseases, and hypersensitivity diseases. The present invention in particular relates to a method of preferentially producing immuno-suppressive dendritic cells relative to immuno-stimulatory dendritic cells

### BACKGROUND OF THE INVENTION

Dendritic cells (DC) are recognized to be potent antigen presenting cells for the initiation and control of cellular immunologic responses in humans. Since DC can either be immuno-stimulatory or immuno-suppressive, depending on which set of their potential properties they express at the moment of interaction with responsive specific clones of T cells, they are considered profoundly important pivotal players in T cell-mediated immune reactions. As a broad, but widely held generalization, immature DC are more "tolerogenic" than their more mature counterparts, while mature DC are thought to be more "immunogenic" than their immature precursors. The capacity of DC, generated ex vivo from monocytes and armed with specific antigen, to function effectively in either immunologic direction, is dependent on their viability and vigor after being returned to the patient. It is logically concluded that the balance between counteractive immuno-stimulatory and immune-suppressive DC will be a major determinant of both the direction and potency of DC-dependent therapeutic immune responses.

The purpose of this invention is to facilitate production of DC populations particularly conducive to the generation of powerful and clinically relevant immune responses. Despite the tremendous promise of DC-based therapy, such as efforts to enhance anti-cancer immunity, clinical results have generally been disappointing. For example, Provenge, the recently first FDA-approved immunotherapy for a solid tumor, adapting the conventional method of ex vivo production of DC from blood monocytes, has yielded merely a four-month improvement in survival of patients with advanced prostate cancer. Further, Provenge has so far not been shown to diminish the size of the treated prostate cancer. That conventional method of inducing DC, as well as Extracorporeal Photopheresis (ECP), an FDA-approved therapy for the "liquid" malignancy Cutaneous T Cell Lymphoma (CTCL), is encumbered by production of relatively heterogeneous DC populations under conditions which handicap the in vivo vigor, and viability, of the resulting DC. By employing more physiologic conditions to the production of therapeutic DC, the present invention enables production of more maturationally synchronized DC, whose survival and vigor are not inhibited by factors inherent to the method by which they are produced. Moreover, this method is applicable to both human and animal leukocytes.

DC prime both CD8⁺ cytotoxic T-cell (CTL) and CD4⁺ T helper (Th1) responses. DC are capable of capturing and processing antigens and migrating to the regional lymph nodes to present the captured antigens and induce T-cell responses. In humans, DC are a relatively rare component of peripheral blood (<1 % of leukocytes). However, large quantities of DC can be differentiated by laboratory procedures from CD34⁺ precursors or blood monocytes.

For the afore-described properties, DC have been identified as an important cellular agent for eliciting effective anti-tumor immune responses. The idea is to generate dendritic cells, which present tumor-specific antigens on their MHC Class I and MHC Class II complex and can be (re)introduced into a patient to thereby launch an immune-attack against the tumor. However, generation of such immuno-stimulatory dendritic cells usually requires differentiation of CD34⁺ precursors or blood monocytes using complex and rather expensive cytokine cocktails. In those standard methods, the cytokines are employed at concentrations very much higher (often by orders of magnitude) than those encountered in vivo under physiological conditions. Therefore, one proffered reason for the overall disappointing clinical results from DC-based immuno-modulation is that DC produced by the common cytokine method may not function effectively at the far lower cytokine concentrations actually in patients. DC produced ex vivo at markedly supra-physiologic concentrations of growth factors, such as cytokines, are selected to be dependent on conditions which are reproduced in the in vivo environment in patients.

In addition to the afore-mentioned immuno-stimulatory dendritic cells, other types of dendritic cells, namely tolerogenic dendritic cells, which may also be designated as immuno-suppressive or immuno-inhibiting dendritic cells have been described. These types of dendritic cells play an important role in maintaining immune tolerance and they have been discussed for therapeutic settings such as solid-organ transplantation, Graft-versus-Host-disease, autoimmune diseases, and hypersensitivity diseases (see e.g. Hu et al. Immunology, (2010), 132, 307-314).

Classical Extracorporeal Photopheresis (ECP) has been used successfully to treat cutaneous T-cell lymphoma (CTCL) in subsets of patients. In ECP, patients suffering from CTCL receive the photoactivatable compound 8-methoxypsoralen (8-MOP). Patients are then leukapheresed to obtain buffy coats and these buffy coats are passed through a continuous closed circuit ultraviolet exposure device to irradiate the leukapheresed buffy coats and thereby lethally damage exposed lymphocytes. In this manner, 8-MOP is induced to covalently crosslink base pairs of DNA. The concept of ECP is to destroy proliferating metastatic T-cells of CTCL and to then to intravenously re-introduce the dying cells to the patient. It has been learned that this process additionally leads to conversion of passaged blood monocytes to DC without the need for stimulation by addition of exogenous cytokines. These ECP-induced DC are furthermore assumed to internalize, process and display antigens from the tumor cells, which were destroyed by the combination of 8-MOP and UV irradiation. It has been hypothesized that reintroduction of these loaded dendritic cells to the patient account for at least part of the success of ECP when treating CTLC. In fact ECP-like processes, in which neither 8-MOP nor UV light irradiation are used, but in which extracorporeal blood sample comprising monocytes are passed under shear stress through an ECP device have also been assumed to initiate monocyte differentiation into DC.

However, it has also been found that the ECP or ECP-like process leads to truncated, i.e. immuno-suppressive or tolerogenic DC, likely contributing heavily to ECP's clinical efficacy in the treatment of Graft versus Host Disease which commonly follows post-bone marrow stem allotransplants. The precise mechanistic aspects of ECP on differentiation of monocytes into either immuno-stimulatory or immuno-suppressive DC have remained elusive (for review of the ECP process see Girardi et al. (2002), Transfusion and Apheresis Science, 26, 181-190).

The present ECP and ECP-like processes are thus conceived to lead to complex mixtures of immuno-stimulatory and immuno-suppressive DC. Of course, from *inter alia* a clinical perspective, it would be important to understand how the ECP and ECP-like processes can be modified to to overcome these limitations and how one can obtain purposively and selectively preferentially immuno-suppressive relative to immuno-stimulatory DC and vice versa. Further, the classical ECP process is, in principle an in vivo method as the obtained dendritic cell mixtures are reinfused into the patient. It would, however, be desirable to have methods available that allow preferential production of immuno-suppressive over immuno- stimulatory DC and vice versa outside the human or animal body.

Thus, there is a continuing need for methods that allow predictable and reproducible production of individual-specific, i.e. autologous immuno-suppressive dendritic cells, which upon re-introduction into a patient allow for treatment of e.g. autoimmune disease such as multiple sclerosis or Graft-versus-Host disease.

### OBJECTIVES AND SUMMARY OF THE INVENTION

One objective of the present invention is to provide methods for producing immuno-suppressive dendritic cells.

Another objective of the present invention is to provide methods for producing immuno-suppressive antigen-presenting cells.

Another objective of the present invention is to provide methods for producing immuno-suppressive dendritic cells or immuno-suppressive antigen-presenting cells in an extracorporeal amount of blood obtained from a patient.

A further objective of the present invention is to provide methods for producing immuno-suppressive dendritic cells or immuno-suppressive antigen-presenting cells in an extracorporeal amount of blood obtained from a patient without the need for cytokine cocktails.

Yet another objective of the present invention relates to the use of such immuno-suppressive dendritic cells or immuno-suppressive antigen-presenting cells for treating patients undergoing solid-organ transplantation and/or suffering from Graft-versus-Host-disease, autoimmune diseases, and hypersensitivity diseases.

These and other objectives as they will become apparent from the ensuing description hereinafter are solved by the subject matter of the independent claims. Some of the preferred embodiments of the present invention form the subject matter of the dependent claims. Yet other embodiments of the present invention may be taken from the ensuing description.

The present invention is based to some extent on data presented hereinafter, which for a miniaturized and scalable device allowed (i) to mimic some aspects of the classical ECP procedure and (ii) to elucidate the cellular and molecular mechanism and biophysical conditions of induction of differentiation of monocytes into immuno-stimulatory autologous dendritic cells in an extracorporeal amount of blood. This data shows that the activation of platelets and binding of monocytes to such activated platelets under conditions of shear force is essential for obtaining immuno-stimulatory autologous dendritic cells. As is shown by the experiments described hereinafter, these immuno-stimulatory autologous dendritic cells can be characterized by expression of molecular markers indicative of immuno-stimulatory autologous dendritic cells. The data also shows that conditions that lead to an increased expression of Glucocorticoid-induced Leucine Zipper (GILZ) will favorably allow monocytes to differentiate into immuno-suppressive dendritic cells. These immuno-suppressive dendritic cells, which show an increased expression of GILZ as consequence of treating monocyte-derived immature dendritic cells with 8-MOP and exposing to UVA, will show an even stronger expression of GILZ upon contact with apoptotic leukocytes. Further, such dendritic cells show increased expression of IL-10 and decrease production of various pro-inflammatory cytokines and chemokines as can be taken form a reduced IL-10 to IL-12p70 ratio. These findings thus allow for a rationalized approach to obtain immuno-suppressive dendritic cells by thus carefully selecting the properties of the devices to be used and the parameters of the process. The findings of the present invention allows preferential production of immuno-suppressive dendritic cells relative to immuno-stimulatory dendritic cells and thus overcome the limitations of the classical ECP procedure because, in the classical ECP, procedure the lack of understanding what type of dendritic cells are produced and how their production can be manipulated to some extent prevents the extension of using this method for other than the authorized applications (see Girardi et al. (2002), Transfusion and Apheresis Science, 26, 181-190). Moreover, other than for the classical ECP procedure as used in the device obtainable from Therakos, the present invention allows to obtain such immuno-stimulatory dendritic cells in an experimental setting, where the extracorporeal amount of blood is not in a continous connection with the body. The data *inter alia* suggests that the process of obtaining immuno-stimulatory dendritic cells includes a global monocyte activation step and a subsequent monocyte to immuno-stimulatory antigen-presenting cell (e.g. dendritic cell) differentiation step. These steps seem to be initially dependent on physical activation of monocytes with the physical forces occurring during e.g. initial purification or enrichment of monocytes being sufficient for activation even though passage of e.g. initially activated monocytes through devices as described herein allow improvement of activation and differentiation. Further, the data suggest that once monocyte activation has occurred, differentiation is channeled into the direction of immuno-suppressive antigen-presenting dendritic cells by applying photoactivatable agents such as 8-MOP and UV-A thereby increasing expression of GILZ. The present data suggest that these processes take place in the absence of e.g. apoptosis of cytotoxic T-cells or other foreign cells such that the immuno-suppressive antigen-presenting dendritic cells can be obtained before loading them with e.g. a desired antigen.

Some of the embodiments, which are based on this data, are described in more detail hereinafter.

In a first aspect, the invention relates to a method for differentiation of monocytes contained in an extracorporeal quantity of a mammalian subject's blood sample into immuno-suppressive dendritic cells, said method comprising at least the steps of:
a) subjecting said extracorporeal quantity of said mammalian subject's blood sample to a physical force such that said monocytes are activated and induced to differentiate into immuno-suppressive dendritic cells, which are identifiable by at least one molecular marker, wherein said at least one molecular marker is indicative of immuno-suppressive dendritic cells.

Suitable molecular markers indicative of immuno-suppressive dendritic cells include GILZ and/or IL-10. Thus, dendritic cells as they are obtainable by the methods described herein, which show an increased expression of GILZ and/or IL-10 are considered to be immuno-suppressive dendritic cells. GILZ is a preferred molecular marker being indicative for immuno-suppressive dendritic cells. It is to be understood that an increased expression is to be determined in comparison to the monocytes before they are subjected to the methods described herein. Such immuno-suppressive dendritic cells may also show an GILZ-induced reduced IL-10 to IL-12p70 ratio.

In addition, immuno-suppressive dendritic cells can be distinguished from immuno-stimulatory autologous dendritic cells in that they do not show an increased expression of molecular markers, which are indicative of immuno-stimulatory autologous dendritic cells. It is to be understood that an expression is to be determined in comparison to the monocytes before they are subjected to the methods described herein

Molecular markers indicative of immuno-stimulatory autologous dendritic cells are described hereinafter and may be taken from e.g. Table 1. These molecular markers may be grouped according to their know function as e.g. molecular markers of antigen-presenting cells, molecular markers of cellulara adhesion etc. Preferred molecular markers the expression of which is considered indicative of immuno-stimulatory dendritic cells include PLAUR, NEU1, CD80, CCR7, LOX1, CD83, ADAM Decysin, FPRL2, GPNMB, ICAM-1, HLA-DR, and/or CD86. Markers like HLA-DR, PLAUR and ICAM-1 may be considered to be indicative of global monocyte activation while increased expression of e.g. CD83 and ADAM-Decysin seems indicative of monocyte to dendritic cell differentiation. Thus, if these molecular markers show an increased expression and particularly if GILZ expression is not increased, dendritic cells as they are obtainable by the methods described herein will be considered to be immuno-stimulatory autologous dendritic cells. In turn, dendritic cells as they are obtainable by the methods described herein will be considered immuno-suppressive dendritic cells if they show an increased expression of GILZ and/or IL-10 and particularly if molecular markers taken from Table 1 do not show an increased expression.

In one embodiment of this first aspect, activation of monocytes is *inter alia* achieved in that said extracorporeal quantity of said mammalian subject's blood sample is subjected to a physical force by passing or cycling said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample.

Thus, activation of monocytes and induction of differentiation into immuno-suppressive dendritic cells can be achieved and influenced by varying the flow forces of the extracorporeal quantity of the mammalian subject's blood sample through the flow chamber of such a device, by varying the geometry of the flow path of the flow chamber, by varying the dimensions of the flow chamber, by varying the temperature of the flow chamber and thus of the extracorporeal quantity of the mammalian subject's blood sample, by changing the biophysical and geometric surface properties of the flow path, by allowing the exposure of the extracorporeal quantity of the mammalian subject's blood sample in the flow chamber to visible or UV light, by adding DNA-cross linking agents such as 8-MOP, etc..

As is shown hereinafter, activation of monocytes and induction of differentiation into immuno-suppressive dendritic cells is mediated e.g. by interaction of monocytes with activated platelets and/or specific plasma components in a situation where the monocytes experience physical force which may be provided by a device as described hereinafter.

In another embodiment of this first aspect, the present invention thus relates to activation of monocytes, which experience a physical force and which interact with activated platelets and/or plasma components such as fibrinogen or fibronectin. Activation may be a process of subsequent steps including the steps of (i) immobilizing plasma components such as fibrinogen or fibronectin either as isolated components or as part of the extracorporeal quantity of the mammalian subject's blood sample in the flow chamber of said device (ii) passing platelets, which may be obtained as a purified fraction from the extracorporeal quantity of the mammalian subject's blood sample or as part of the extracorporeal quantity of the mammalian subject's blood sample, through the flow chamber such that the platelets can interact with and become activated by the plasma components and (iii) passing monocytes, which may be obtained as a purified fraction from the extracorporeal quantity of the mammalian subject's blood sample or as part of the extracorporeal quantity of the mammalian subject's blood sample, through the flow chamber such that the monocytes can interact with and become activated by the activated platelets and/or the plasma components.

Thus, in addition and/or alternatively to the above described parameters and variable touching on the architecture of and the conditions under which the device is operated, activation of monocytes and induction of differentiation into immuno-suppressive dendritic cells can be achieved and influenced by varying the nature, purity and concentrations of the plasma components, the nature, purity and concentration of the platelets, the order of steps by which the plasma components and/or the platelets are passed through and/or disposed on the flow chamber, the density by which the flow chamber is coated with the plasma components and/or the platelets, the flow forces of the extracorporeal quantity of the mammalian subject's blood sample and in particular the platelets and/or the monocytes are passed through the flow chamber of such a device, the temperature and/or time at which the extracorporeal quantity of the mammalian subject's blood sample and in particular the platelets and/or the monocytes are passed through the flow chamber of such a device, etc., the nature, purity and concentrations of additional factors such as 8-MOP and/or cytokines are added to the extracorporeal quantity of the mammalian subject's blood sample and in particular to the monocytes, etc.

It needs, however, to be understood that while such devices may be particularly effective in inducing monocyte activation and differentiation into immuno-suppressive dendritic cells in the presence of a photo-activatable agent such as 8-MOP and UVA, physical forces which monocytes experience during initial purification or enrichment such as during Ficoll-Hypaque enrichment as described hereinafter may already be sufficient to activate monocytes and to induce their differentiation. Similarly even though activated platelets and/or specific plasma components may be helpful in increasing monocyte activation and differentiation into antigen-presenting cells such as dendritic cells they may not be absolutely necessary. In order to effect monocyte activation and differentiation into antigen-presenting cells such as dendritic cells the invention thus contemplates as a mimimal requirement the application of physical forces. Once the activation and differentiation process has started application of photoactivatable agents such as 8-MOP and UVA or other means that increase GILZ expression (such as dexamethasone) can be used to selectively channel the differentiation process towards immuno-suppressive antigen-presenting cells such as dendritic cells.

In the above and ensuing aspects and embdodiment, the extracorporeal quantity of the mammalian subject's blood sample and in particular the monocytes thus may or may not be obtained by leukapheresis.

Additionally or alternatively to these embodiments, the invention in particular also relates to such methods which are conducted under conditions which favor an increased expression of GILZ and/or an increased number of CD4⁺CD25⁺Foxp3⁺ cells and/or a down-regulations of CD80, CD86 and CD83. The invention thus relates to e.g. methods, which are conducted in the presence of a photoactivatable agent such as 8-MOP and with exposure to light such as UV-A. In one embodiment, the invention correspondingly relates to methods which are conducted under conditions which avoid activation of monocytes and induction of immuno-stimulatory autologous dendritic cells as can be identified by determining expression of GILZ and/or IL-10 and/or of the molecular markers of Table 1. In one embodiment, the invention thus does not relate to e.g. methods, which are conducted in the absence of a photoactivatable agent such as 8-MOP and without exposure to light such as UV-A

Another embodiment relates to methods as described hereinafter for obtaining , autologous and/or allogenic immuno-suppressive antigen-presenting cells, which can be used e.g. in treating autoimmune diseases, hypersensitivity diseases, rejection of solid organ transplant, and Graft-versus-Host Disease.

Another embodiment relates to methods as described hereinafter for producing immune-suppressive antigen presenting cells and preferably immuno-suppressive dendritic cells which may be mammalian cells. Even though some of the preferred embodiments of the invention relate to producing human immuno-suppressive antigen presenting cells and preferably human immuno-suppressive allogeneic dendritic cells, the present invention considers also to use the methods for producing animal immuno-suppressive antigen presenting cells and preferably animal immuno-suppressive dendritic cells such as for mice, rats, etc. These embodiments of the invention provide useful animal models and thus scalability of the methods and results described herein from e.g. mice to man. Moreover, as there are genetically identical lines of animals such as mice available, animal immuno-suppressive antigen presenting cells and preferably animal immuno-suppressive dendritic cells such as mice immuno-suppressive antigen presenting cells and preferably mice immuno-suppressive dendritic cells may be introduced either in the individual from which the extracorporeal amount of blood sample was taken and thus be autologous in the strict sense or be introduced in a genetically identical individual. This will allow e.g. testing for any unexpected effects of these cells.

It is to be understood that the methods described hereinafter have been shown to produce immune-suppressive cells, which due to their molecular markers seem to be related to if not correspond to cells that are commonly named dendritic cells. Thus the immune-suppressive cells according to the invention have been named immune-suppressive dendritic cells. However, dendritic cells are representatives of a broader class of cells, which may be designated as antigen-presenting cells. Thus, the methods as described hereinafter generally refer to the production of immune-suppressive antigen-presenting cells with immune-suppressive dendritic cells being preferred.

A second aspect of the invention relates to autologous and/or allogenic immuno-suppressive dendritic cells or autologous and/or allogenic immuno-suppressive antigen-presenting cells obtainable by a method as described hereinafter for use in treating autoimmune diseases, hypersensitivity diseases, rejection of solid organ transplant, and Graft-versus-Host Disease.

Further embodiments will be described hereinafter.

### FIGURE LEGENDS

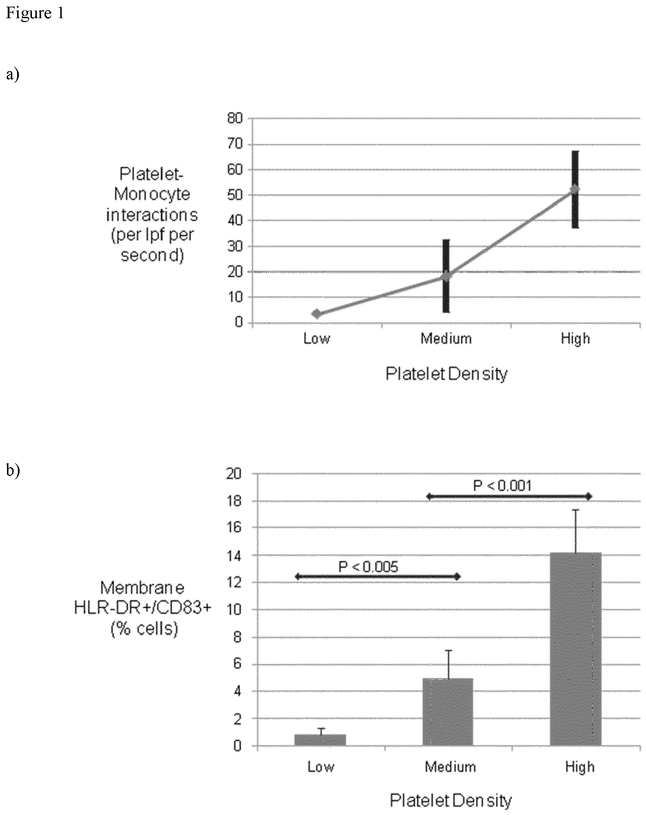

- Figure 1: Effect of platelet density on number of monocyte-platelet interactions and subsequent monocyte phenotype. Monocytes were passed through parallel plates coated with platelets at low, medium, or high density. (A) The number of monocyte-platelet interactions increased substantially for plates coated with higher densities of platelets. (B) After overnight incubation, monocytes which were exposed to high levels of platelets were significantly more likely to develop a phenotype consistent with DC differentiation, as assessed by expression of membrane CD83 and HLA-DR (high versus medium or low density: p <0.0001; medium versus low density: p < 0.005). Data shown are the means (+/- SD) of at least 6 independent experiments. lpf, low power field.

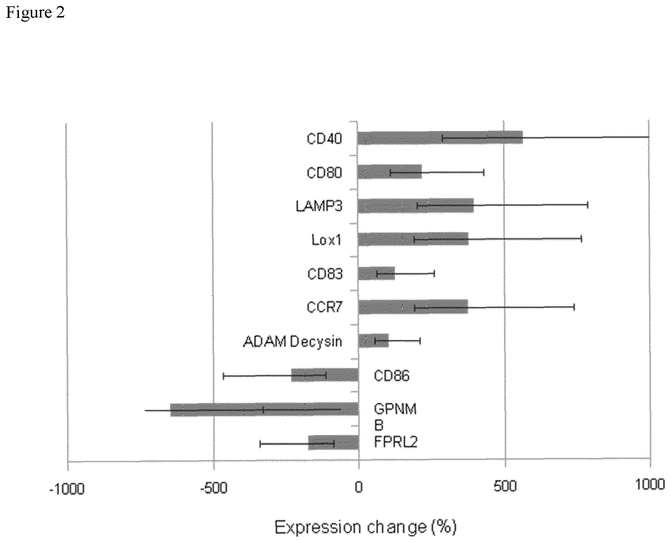

- Figure 2: Gene expression following exposure to platelets. Monocytes were exposed to high or low levels of platelets in flow. Following overnight incubation, cells were assessed for differences in gene expression using RT-PCR. Figure 2 shows gene expression changes in monocytes exposed to high levels of platelets relative to those exposed to low levels. Seven genes associated with DC-differentiation and/or function were found to be upregulated, while three were downregulated. Of the genes downregulated, GPNMB and FPRL2 have known functions in decreasing cytokine production and inhibiting DC maturation, respectively. Of the genes upregulated, all have either pro-immune functions or miscellaneous roles in DC biology. See text for specific description of genes. Data shown are the means (+/- SD) of 2 independent experiments.

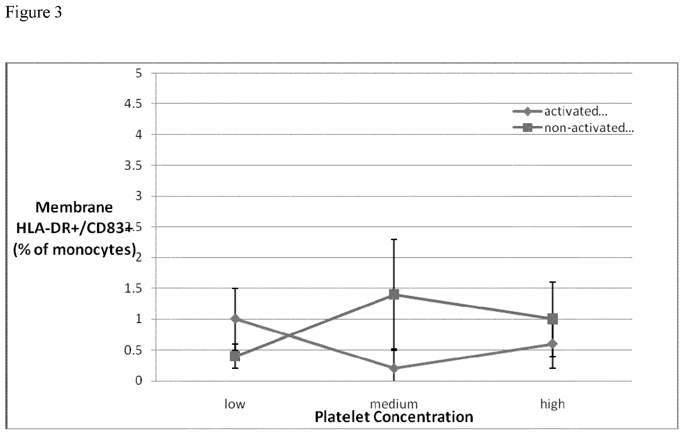

- Figure 3: Platelet influence on monocyte differentiation in static conditions. Monocytes were co-cultured for 18 hours with low, medium, or high concentrations of platelets in static conditions lacking flow. Under these conditions, there was no observable platelet influence on DC differentiation; all conditions resulted in low, baseline levels of cells expressing DC markers. Furthermore, activating platelets with thrombin in culture (blue line) did not cause a discernible difference in monocyte differentiation relative to those cultures containing platelets not activated by thrombin (red line).

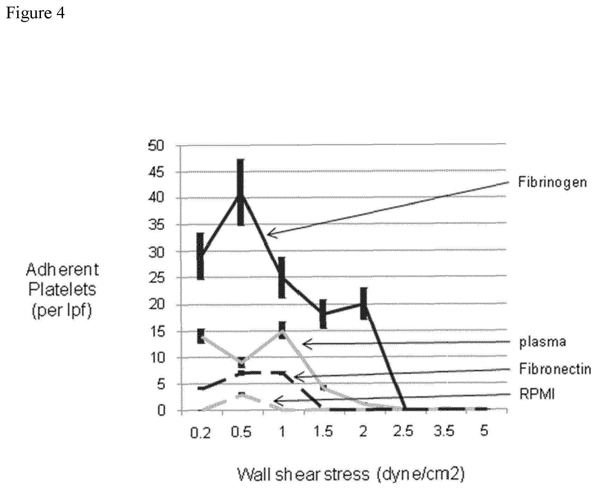

- Figure 4: Plasma protein influence on platelet adhesion to plates. Platelets were passed through plates coated with fibrinogen, plasma, fibronectin, or RMPI at the shear stress level indicated by the x-axis. Platelets in flow adhered optimally to fibronectin. For all proteins, platelet adhesion occurred maximally between 0.5 and 1.0 dyne/cm² lpf, low power field. Data shown are the means (+/- SD) of at least 2 independent experiments.

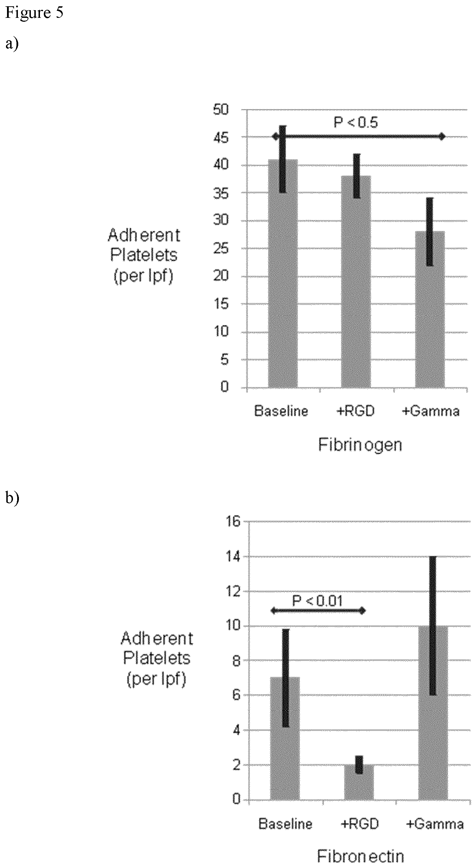

- Figure 5: Plasma protein influence on platelet adhesion to plates coated with Fibrinogen (A) or Fibronectin (B). Platelets were either untreated (baseline), or pretreated with either RGD fragments (+RGD) or gamma fragments (+Gamma) and their subsequent adhesion to fibrinogen (left panel) and fibronectin (right panel) was assessed. Platelet binding to fibrinogen was decreased by gamma fragments (p < 0.05), while binding to fibronectin was decreased by RGD peptides (p < 0.001). lpf, low power field. Data shown are the means (+/- SD) of at least 2 independent experiments.

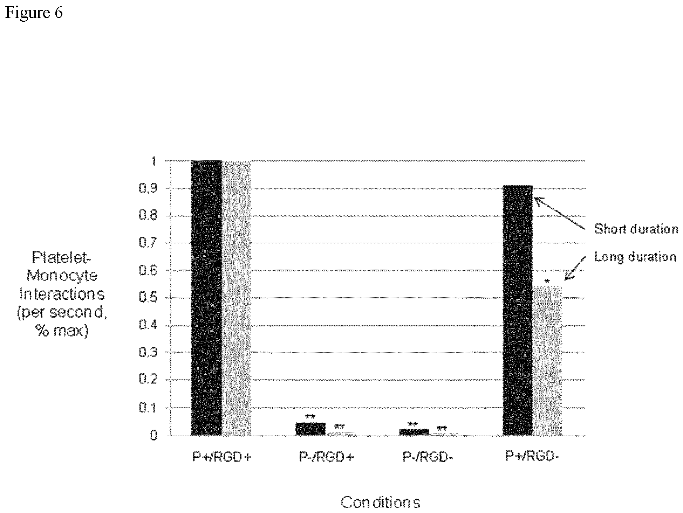

- Figure 6: Proteins involved in monocyte-platelet interactions. Monocytes were passed between platelet-coated plates at a wall shear stress of 0.5 dyne/cm2 under the conditions indicated by the x-axis: platelets were either pretreated with anti-P-selectin (P-) or an isotype control (P+); monocytes were either pretreated with RGD peptides (RGD-) or a control fragment (RGD+). Monocyte-platelet interactions were quantified under each set of conditions using digital microscopy, and are expressed in the figure as a fraction of the maximum seen under conditions of P+/RGD+. Interactions were divided into those lasting less than 3 second (short duration, black bars) and those lasting greater than 3 seconds, including stable binding (long-duration, gray bars). All conditions which involved blocking with anti-P-selectin (P-) resulted in a significant decrease in both short and long duration interactions (**, p<0.01); Blocking only RGD (RGD-) resulted in a significant decrease in long-duration interactions (*, p<0.05) but no change in short-duration interactions. Data shown are the means (+/- SD) of 3 independent experiments.

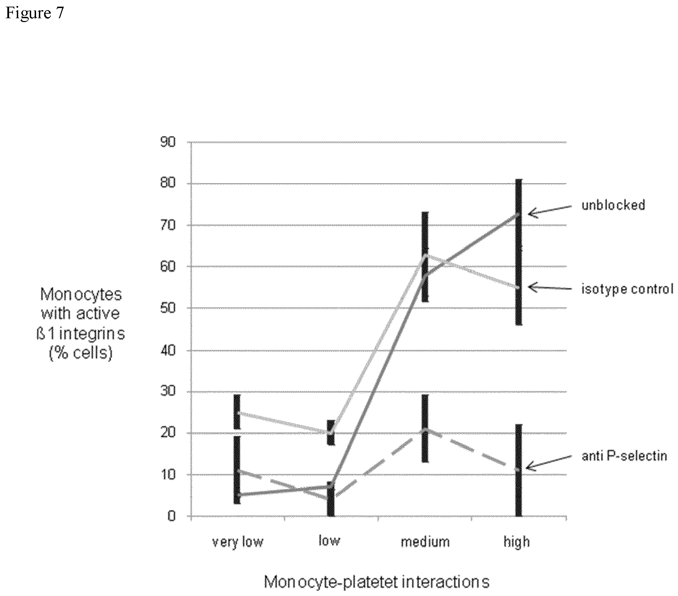

- Figure 7: Effect of p-Selectin exposure on monocyte integrins. Plastic plates were coated with platelets at the relative density indicated by the x-axis. Platelets were then pretreated with anti p-selectin (dashed line) or an isotype control (gray line), or received no pretreatment (black line). Monocytes were passed through the plates at 0.5 dyne/cm2 and then immediately assessed by flow cytometry for expression of active β1 integrins. The y-axis indicates the percent of monocyte which bound an antibody directed at an epitope only exposed when the integrin is in the open confirmation. Data shown are the means (+/- SD) of 3 independent experiments.

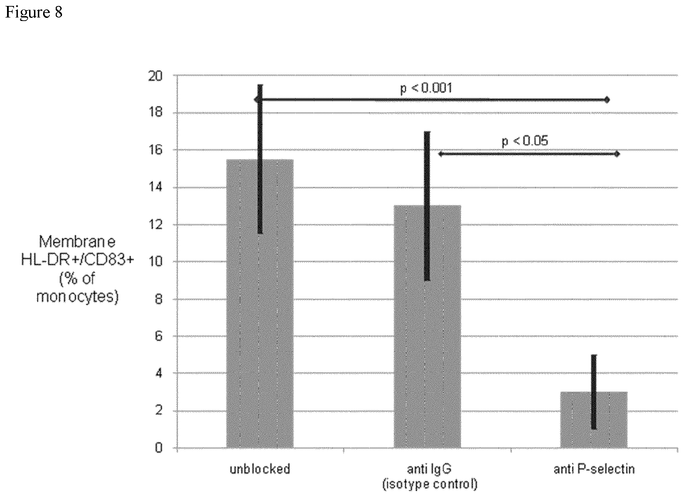

- Figure 8: Effect of P-selectin exposure on monocyte phenotype after overnight incubation. Platelet-coated plates were either untreated (first column), or pretreated with an isotype control (second column) or anti-P-selectin (third column). Monocytes were passed through the plates at 0.5 dyne/cm2 then incubated overnight. The y-axis indicates the percent of monocytes which developed a phenotype consistent with DC differentiation, i.e., membrane HLA-DR+/CD83+. Data shown are the means (+/- SD) of 3 independent experiments.

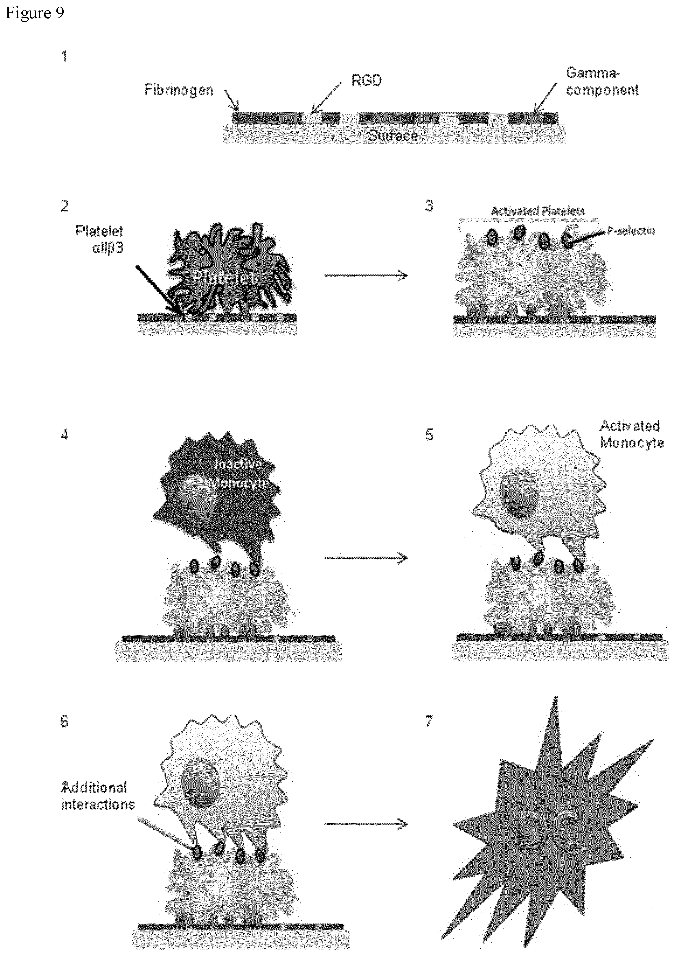

- Figure 9: Proposed mechanism for induction of monocyte-to-DC differentiation. Based on data presented in this manuscript, the following sequence of events is postulated: (1) plasma fibrinogen coats the plastic surface of the flow chamber; (2) through their αIIbβ3 receptor, unactivated platelets bind to the gamma-component of immobilized fibrinogen; (3) platelets become activated and instantaneously express preformed P-selectin and other surface proteins; (4) passaged monocytes transiently bind P-selectin via PSGL-1, causing partial monocyte activation and integrin receptor conformational changes; (5) partially-activated monocytes, now capable of further interactions, bind additional platelet-expressed ligands, including those containing RGD domains; (6) finally, so influenced, monocytes efficiently enter the DC maturational pathway within 18 hours. Note that, in-vivo, step (1) above may be replaced physiologically by inflammatory signals from tissue acting on local endothelium, causing it to recruit and activate platelets in a similar manner.

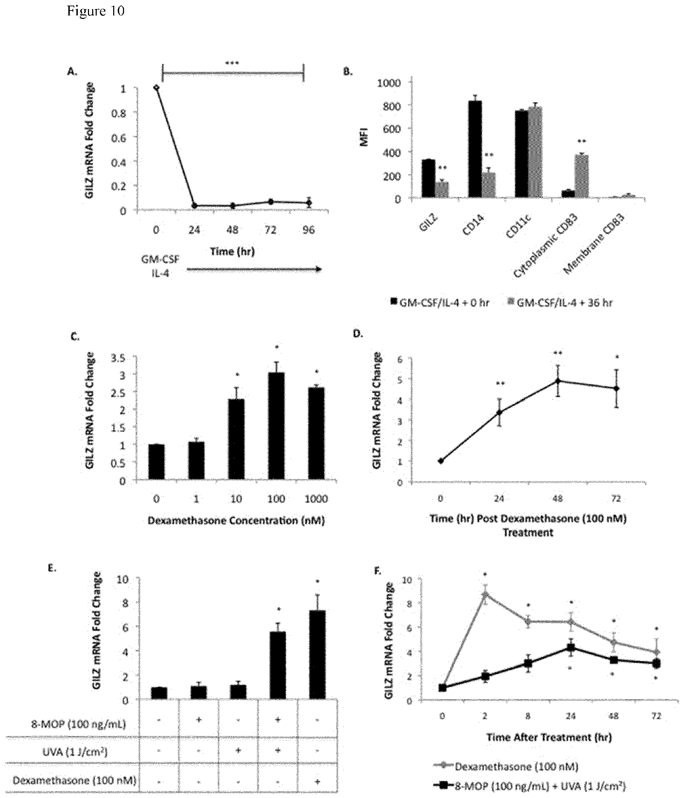

- Figure 10:: Expression of GILZ is rapidly down-regulated as monocytes differentiate into immature MoDC, and up-regulated after exposure to dexamethasone. A.) GILZ mRNA expression in CD11c+ MoDC is presented as a fold change relative to freshly isolated monocytes. B.) Median fluorescence intensities for intracellular and cell surface markers after 0 and 36 hr. C.) GILZ mRNA expression in CD11c+ MoDC after 24 hr is presented as a fold change relative to MoDC receiving no dexamethasone. D.) GILZ mRNA expression in CD11c+ MoDC is presented as a fold change relative to MoDC at time 0 hr. E.) GILZ mRNA expression in CD11c+ MoDC after 24 hr is presented as a fold change relative to untreated MoDC. F.) GILZ mRNA expression in CD11c+ MoDC is presented as a fold change relative to untreated MoDC. All data are expressed as mean ± standard deviation for a minimum of 3 independent experiments. For differential gene expression: *≥2.5-fold change and p <0.05, **≥2.5-fold change and p <0.01, ***≥2.5-fold change and p < 0.001

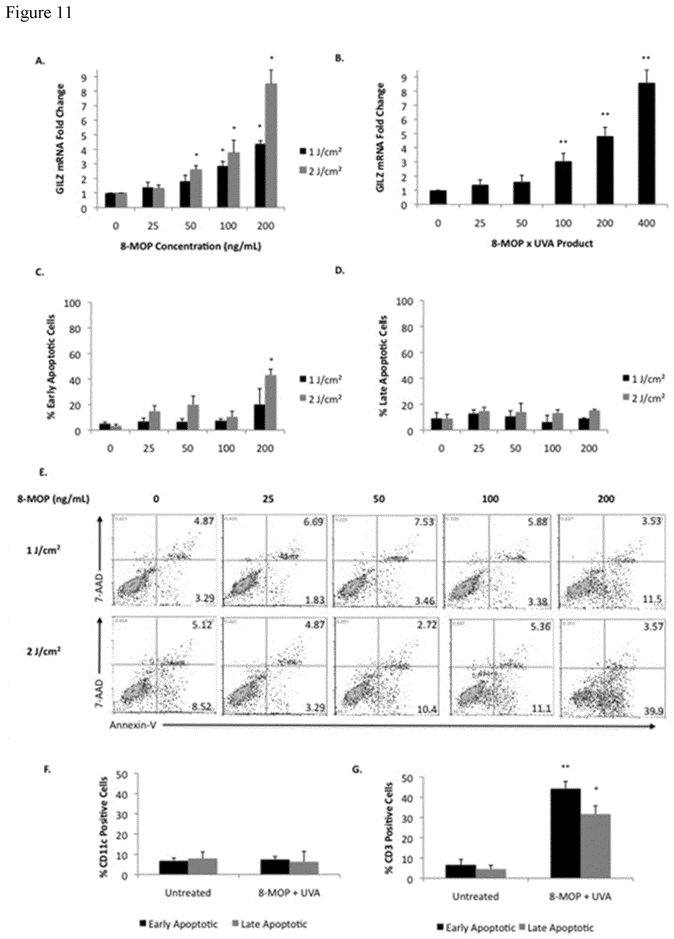

- Figure 11:: 8-MOP plus UVA light up-regulates GILZ in immature MoDC in a dose-dependent fashion. A.) GILZ expression is presented as a function of the 8-MOP concentration at 1 J/cm2 and 2 J/cm2 of UVA light. GILZ mRNA expression in CD11c+ MoDC 24 hr after PUVA treatment is presented as a fold change relative to MoDC receiving no 8-MOP. B.) GILZ expression is presented as a function of the 8-MOP concentration multiplied by the UVA dose. C.) The percentage of early apoptotic CD11c+ cells after 24 hr. D.) The percentage of late apoptotic CD11c+ cells after 24 hr E.) Dot plots of CD11c+-gated cells for UVA doses of 1 J/cm2 and 2 J/cm2 are shown for 1 representative experiment of 4. The percentage of CD11c+ cells displaying Annexin-V+/7-AAD- or Annexin-V+/7-AAD+ phenotypes are indicated. The percentage of F.) CD11c+ cells and G.) CD3+ cells expressing early and late apoptotic markers were quantified 24 hr after treatment with 8-MOP (100 ng/mL) and UVA light (1 J/cm2). All data represent mean ± standard deviation of at least 4 independent experiments. For differential gene expression: * ≥ 2.5-fold change and p < 0.05, ** ≥2.5-fold change and p < 0.01

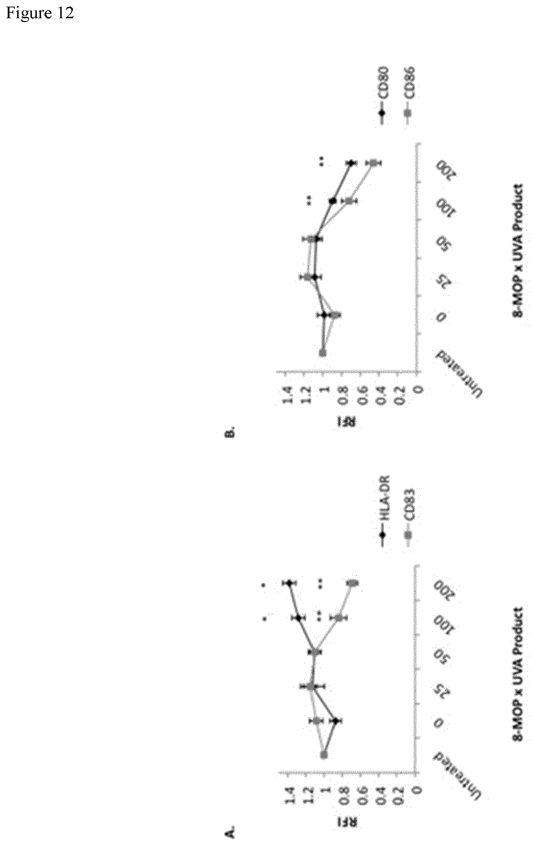

- Figure 12:: 8-MOP plus UVA light down-regulates CD83, CD80 and CD86 and up-regulates HLA-DR in immature MoDC in a dose-dependent manner. Relative fluorescence intensities for membrane expression of A.) HLA-DR and CD83, and B.) CD80 and CD86 are presented as a function of the 8-MOP concentration (0 to 200 ng/mL) multiplied by the UVA dose (1 or 2 J/cm2) 24 hr after PUVA treatment. Untreated MoDC served as controls and were assigned an RFI value of 1. Data represent mean ± standard deviation of 4 independent experiments. *p<0.05, **p < 0.01

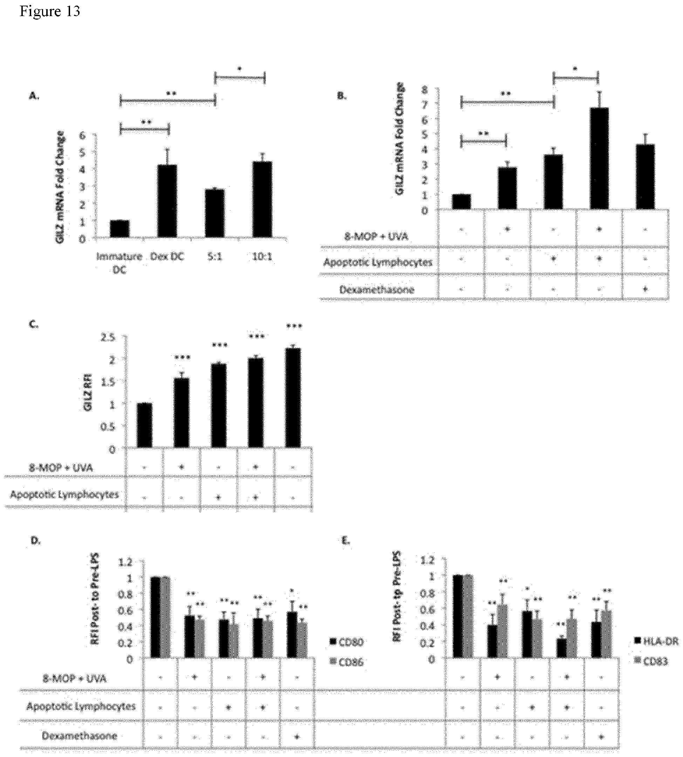

- Figure 13:: Immature MoDC exposed to apoptotic lymphocytes up-regulate GILZ. A.) GILZ mRNA expression in CD11c+ MoDC 24 hr after co-culture is presented as a fold change relative to untreated MoDC that were cultured alone. B.) GILZ mRNA expression in CD11c+ MoDC 24 hr after co-culture is presented as a fold change relative to untreated MoDC that were cultured alone. C.) Relative fluorescence intensity for intracellular GILZ 24 hr after co-culture. Relative fluorescence intensities post- to pre-LPS stimulation for D.) CD80 and CD86 and E.) HLA-DR and CD83 were calculated as follows: (MFItreated after LPS - MFItreated before LPS) / (MFIuntreated after LPS - MFIuntreated before LPS). Data represent mean ± standard deviation for at least 4 independent experiments. For differential gene expression: * ≥ 2.5-fold change and p < 0.05

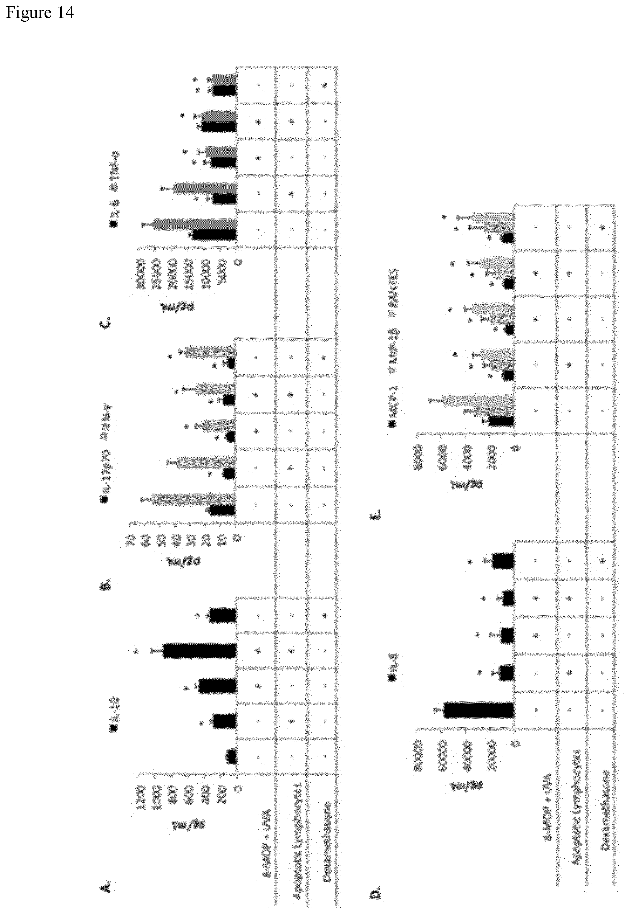

- Figure 14:: MoDC expressing GILZ increase production of IL-10, and decrease production of various pro-inflammatory cytokines and chemokines. 24 hr after LPS stimulation, culture supernatants were harvested for cytokine quantification by magnetic bead multiplex immunoassays for A.) IL-10, and the pro-inflammatory cytokines B.) IL-12p70 and IFN-γ, C.) IL-6 and TNF-α. The same analysis was performed for the pro-inflammatory chemokines D.) IL-8, and E.) MCP-1, MIP-1β and RANTES. Data are presented as mean ± standard deviation of 3 independent experiments. * p<0.05 compared to the untreated MoDC group.

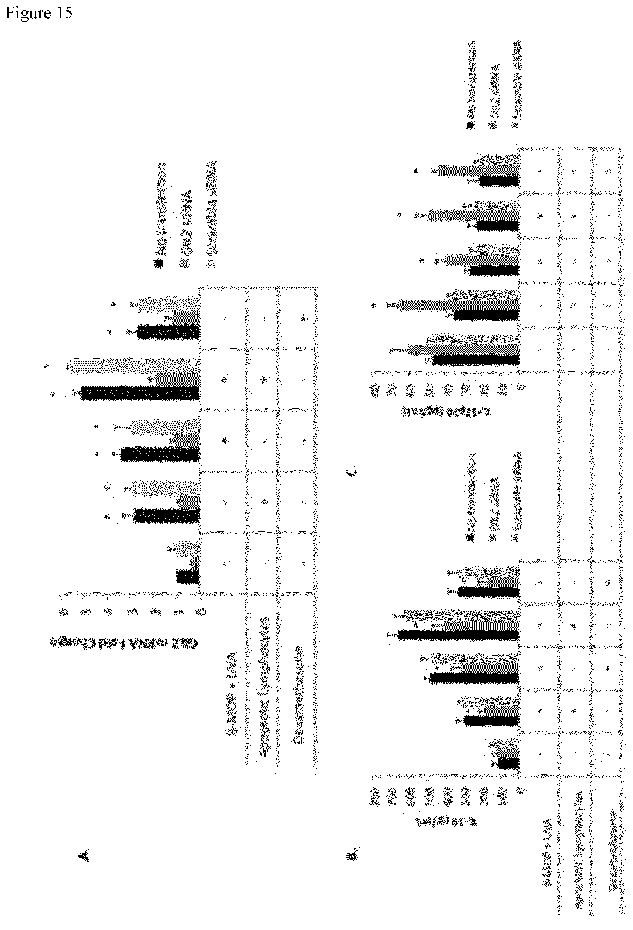

- Figure 15:: siRNA-mediated knockdown of GILZ abolishes the increased IL-10 to IL-12p70 ratio characteristic of tolerogenic dendritic cells. A.) GILZ mRNA expression is presented as fold change compared to untreated MoDC that were cultured alone. * ≥ 2.5-fold change and p < 0.05. B.) Quantification of IL-10 and IL-12p70 protein levels in culture supernatants after LPS stimulation. Data represent mean ± standard deviation of 3 independent experiments. *p < 0.05, compared to identically treated MoDC not transfected with siRNA.

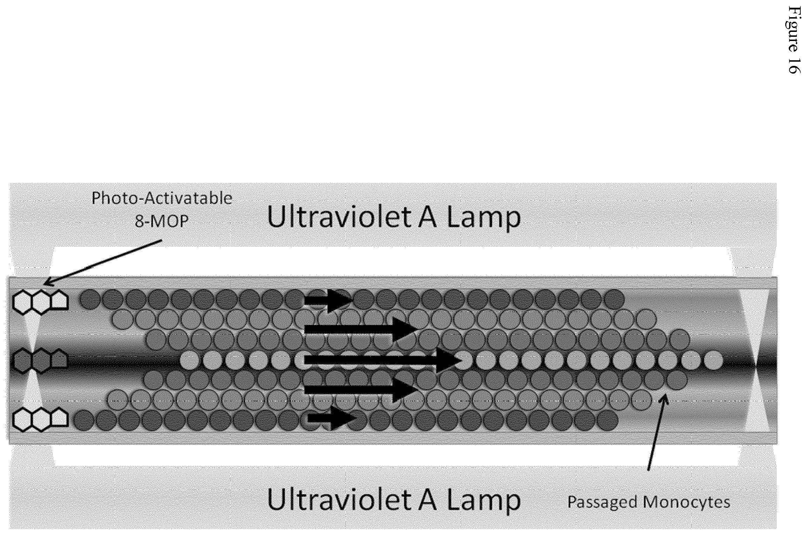

- Figure 16:: depicts the flow of monocytes in a classical ECP process in the presence of UVA and 8-MOP. The monocytes in the middle experience lower UVA exposure than the monocytes towards the surfaces of the channels.

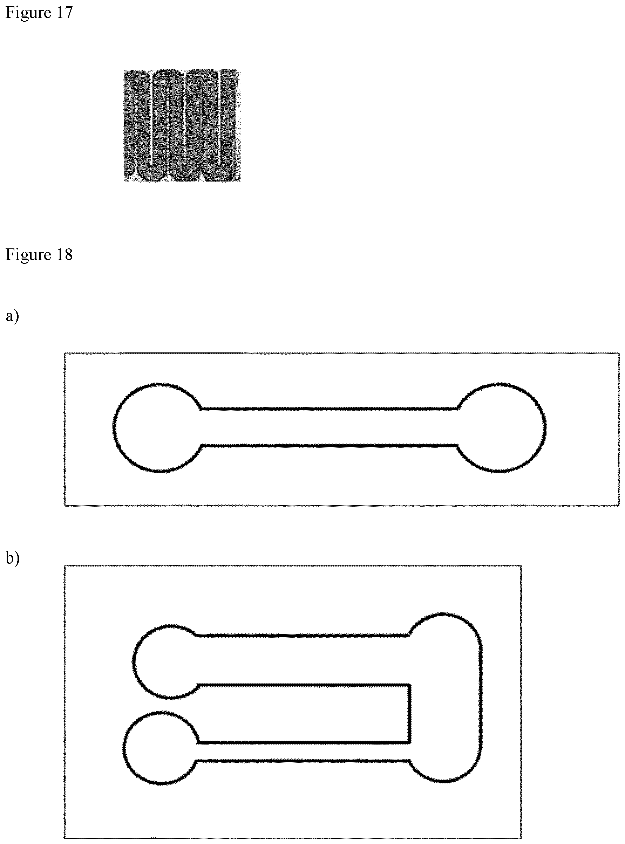

- Figure 17:: depicts the design of the channels of the device used in a classical ECP process.

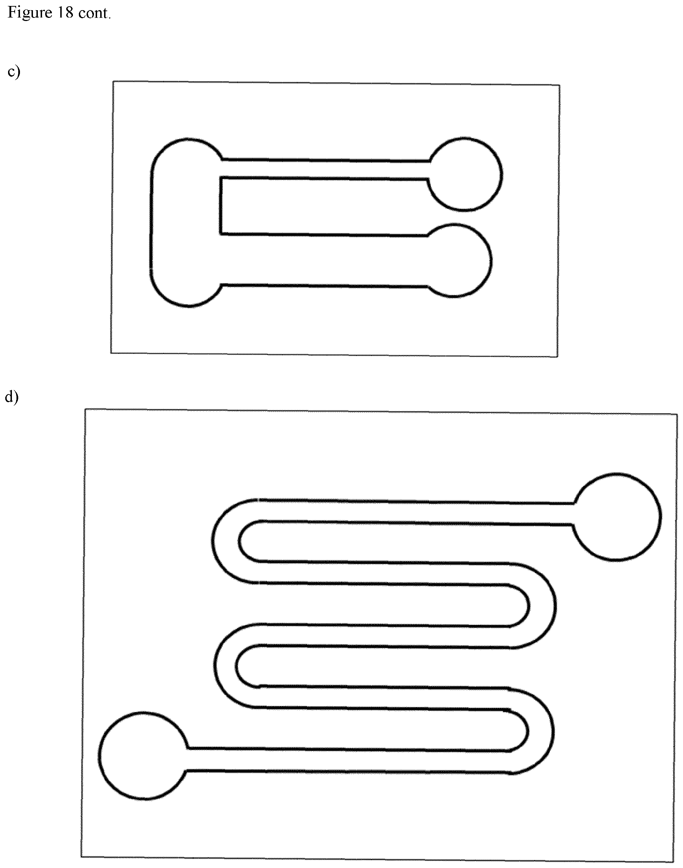

- Figure 18:: a) to d) depict different geometries of the flow chamber of a device that may be used for the methods of the invention.

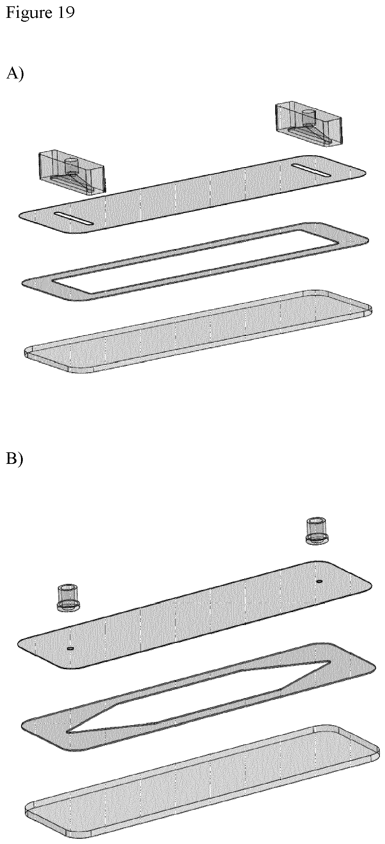

- Figure 19:: A) depicts the geometry of a device used in some of the examples. B) depicts the geometry of an alternative device.

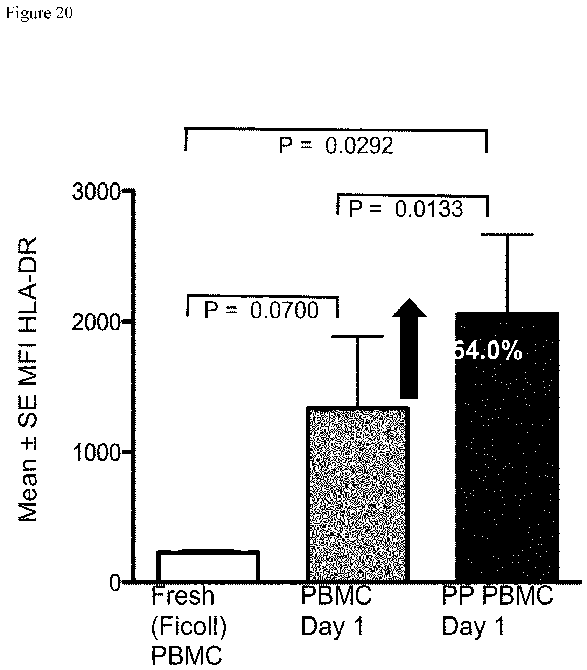

- Figure 20:: depicts increase of expression of HLA-DR upon physical activation of monocytes through a device of Figure 19

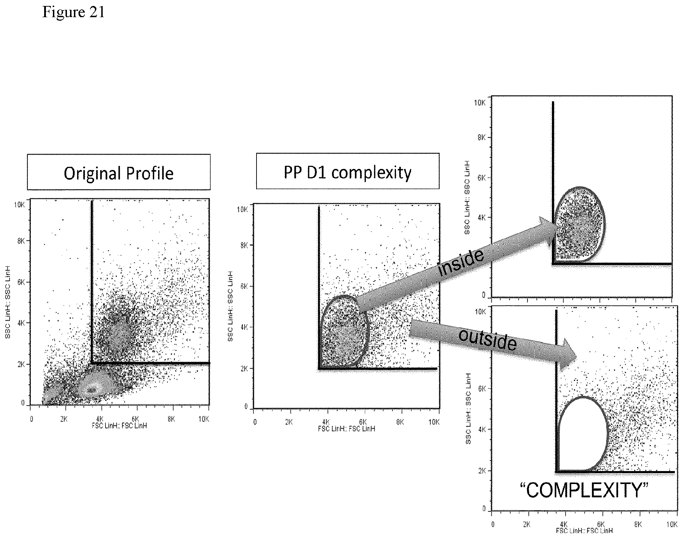

- Figure 21:: depicts increase of FSC/SSC complexity upon physical activation of monocytes through a device of Figure 19

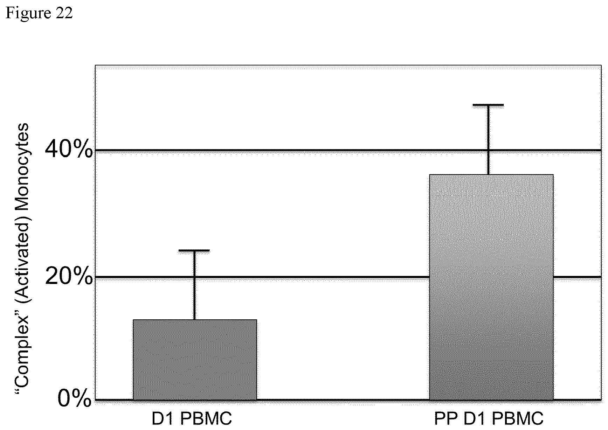

- Figures 22:: depicts increase of FSC/SSC complexity upon physical activation of monocytes by passing through a device of Figure 19

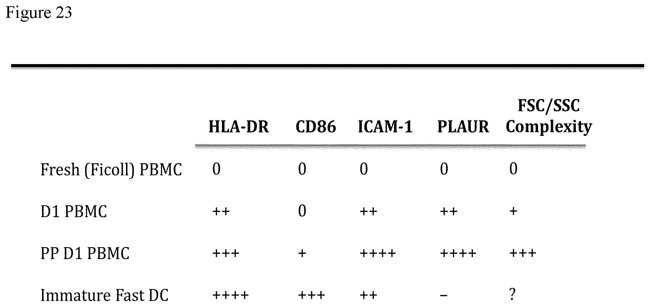

- Figure 23:: depicts increase of expression of HLA-DR, CD86, ICAM-1, PLAUR and or FSC/SSC complexity upon physical activation of monocytes through a device of Figure 19
- Figure 24:: depicts increase of expression of GILZ upon physical activation of monocytes through a device of Figure 19 and application of 8-MOP and UVA

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of' is considered to be a preferred embodiment of the term "comprising of'. If hereinafter a group is defined to comprise at least a certain number of embodiments, this is also to be understood to disclose a group, which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody, which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±20 %, preferably ±15 %, more preferably ±10%, and even more preferably ±5 %.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)" etc. relate to steps of a method or use or assay there is no time or time interval coherence between the steps unless indicated otherwise, i.e. the steps may be carried out simultaneously or there may be time intervals of seconds, minutes, hours, days, weeks, months or even years between such steps, unless otherwise indicated in the application as set forth herein above or below.

Technical terms are used by their common sense. If a specific meaning is conveyed to certain terms, definitions of terms will be given in the following in the context of which the terms are used.

As already mentioned, the present invention is based to some extent on data presented hereinafter, which for a miniaturized device allowed (i) to mimic some aspects of the classical ECP procedure and (ii) to elucidate the cellular and molecular mechanism of induction of differentiation of monocytes into immuno-stimulatory dendritic cells in an extracorporeal amount of blood. As is shown by the experiments described hereinafter, these immuno-stimulatory autologous dendritic cells can be characterized by expression of molecular markers indicative of immuno-stimulatory autologous dendritic cells. The data also shows that conditions that lead to an increased expression of Glucocorticoid-induced Leucine Zipper (GILZ) will favorably allow monocytes to differentiate into immuno-suppressive dendritic cells. For the purposes of the present invention such immuno-suppressive dendritic cells are also designated as immuno-inhibiting autologous dendritic cells, tolerogenic autologous dendritic cells or truncated autologous dendritic cells. This data shows that e.g. the sequential activation of platelets and binding of monocytes to such activated platelets under conditions of shear force is essential for differentiation of monocytes into dendritic cells and, depending on the specific conditions chosen, in particular into immuno-stimulatory or immuno-suppressive dendritic cells. Further, these findings immediately allow for a rationalized approach to obtain immuno-stimulatory and immuno-suppressive dendritic cells. Given that one can mimic and dissect the series of molecular events leading to the formation of immuno-stimulatory autologous dendritic cells and immuno-suppressive dendritic cells obtained in the classical ECP process, one can now design devices and more particularly flow chambers, which allow to further dissect the molecular events leading to differentiation of monocytes into immuno-stimulatory autologous dendritic cells and immuno-suppressive dendritic cells on a scale suitable for research purposes, but also which allow to obtain immuno-stimulatory autologous dendritic cells and immuno-suppressive dendritic cells for therapeutic purposes. This will be explained in further detail.

In the classical ECP procedure, 2.5 L to 6 L blood is typically obtained from patients suffering from CTLC by apheresis such as leukaphereses. This extracorporeal amount of blood, which typically is processed by apheresis such as leukaphereses to give a final volume of about 200 ml to 500 ml comprising leukocytes including monocytes as well as plasma components, platelets and cancerous T-cells, is then passed under shear stress through a photopheresis device having transparent plastic channels together with the photoactivatable drug 8-MOP. This extracorporeal amount of blood comprising 8-MOP is then irradiated by exposing the transparent channels to UV-A having a wavelength of 315 to 380 nm. The irradiated extracorporeal amount of blood is then re-introduced into the patient. The beneficial effects of this procedure on the course of CTLC in some of the treated patients was originally hypothesized to result from the destruction of cancerous T-cells. Based on this hypothesis, it was assumed that patients would have to undergo repeated cycles of ECP. However, for some of the patients beneficial long-term effects were observed making repeated treatment superfluous and, in the following, interesting and partially non-reconcilable effects were found, which could explain some of the positive outcomes of ECP for CTLC treatment.

For example, as is described in US 6,524,855 induction of DC was observed in the extracorporeal amount of blood and it was hypothesized that some of the beneficial effects of ECP on CTLC resulted from cancer-specific antigens that were shed by cancerous T-cells as a consequence of the 8-MOP induced apoptosis of these cells and loading of DC, which had started to differentiate, with these antigens. The re-introduction of the extracorporeal amount of blood comprising such cancer-antigen loaded autologous DC was assumed to provide a vaccination-like long-term lasting therapeutic effect. However, at the same time it was observed that so-called "truncated" DC were formed during the ECP procedure, which did not provide an immuno-stimulatory effect, but rather the opposite, namely an immuno-suppressant effect. The induction of such different types of DC with opposing effects by the same process was puzzling and, from a practical perspective, posed hurdles as to a rationalized use of ECP for obtaining immuno-stimulatory or immuno-suppressant DC. Further, the need for apheresis such as leukaphereses to obtain a sufficient amount of extracorporeal blood is another factor negatively affecting treatment quality for patients.

The data presented hereinafter suggest that shear stress is in principle responsible for the induction of DC. By using the miniaturized model device as described hereinafter, it was shown that induction of immuno-stimulatory DC occurs even if substantially lower amounts of extracorporeal blood, which has not been obtained by apheresis such as leukaphereses, are used, and if 8-MOP is not added to the extracorporeal amount of blood and even if no irradiation with UV-A takes place. Thus, induction of DC occurred despite omission of central steps of the classical ECP procedure. However, shear stress seems to be one factor that is crucial for obtaining DC as such. Other crucial steps for the induction of dendritic cell formation seem to be the activation of platelets by plasma components and the activation of monocytes by such activated platelets. The data further suggests that, if shear-stress induced induction of dendritic cell formation takes place in the presence of 8-MOP and irradiation with UVA, expression of the Glucocorticoid-induced Leucine Zipper (GILZ) is increased, which in turn activates a pathway leading to formation of truncated, i.e. immuno-suppressive, tolerogenic DC (see Example 2). The fact that shear-stress induced induction of immuno-stimulatory DC could be achieved by applying shear stress without the addition of 8-MOP and without irradiation with UV-A further suggests that in the classical ECP procedure due to the dimensions of the plastic channels some of the initially shear-stress induced DC were not effectively irradiated (maybe because they were moving in the middle of the channels of the ECP device) with the consequence that these DC could further develop into immuno-stimulatory DC (see Figure 16). This previous data was obtained using a device having the general architecture of Figure 17. However, in the classical ECP and ECP-like procedures, mixtures of immuno-stimulatory autologous and immuno-suppressive autologous DC were obtained. Based on the data presented hereinafter, it is now possible to e.g. dispense with some of the requirements of the ECP and ECP-like processes of the prior art, e.g. to use large amounts of blood which needs to be processed by apheresis such as leukaphereses. Further, one can now deliberately adapt the process parameters and the design of the device, which is used to exert a physical force on monocytes, to deliberately obtain either immuno-stimulatory autologous or immuno-suppressive autologous DC. For example, by selecting the design and dimensions of the flow chamber, one can make sure that basically all DC are contacted with 8-MOP and irradiated with UVA such that immuno-suppressive DC will form. Moreover, as the molecular markers being indicative of immuno-suppressive DC and of immuno-stimulatory autologous DC as they are obtainable by the methods described herein have been deciphered, one can further purify dendritic cell populations, which depending on the chosen method parameters will be substantially already only immuno-suppressive DC or immuno-stimulatory autologous DC, by e.f. FACS analysis to homogeneity.

The methods as described hereinafter may be performed without the need of molecular cocktails to achieve maturation and differentiation of monocytes into immuno-suppressive dendritic cells. Further, as the invention is based on inducing differentiation of monocytes contained in an extracorporeal quantity of mammalian subject's blood sample, the differentiation process is not limited to the molecular events which can be triggered by typical cytokine cocktails. Rather, dendritic cells as obtainable with the methods described hereinafter seem to have more complex molecular patterns, which seem representative of a broader functionality of these dendritic cells.

In a first aspect, the invention thus relates to a method for inducing differentiation of monocytes contained in an extracorporeal quantity of a mammalian subject's blood sample into immuno-suppressive dendritic cells, said method comprising at least the steps of:
a) subjecting said extracorporeal quantity of said mammalian subject's blood sample to a physical force such that said monocytes are activated and induced to differentiate into immuno-suppressive dendritic cells, which are identifiable by at least one molecular marker, wherein said at least one molecular marker is indicative of immuno-suppressive dendritic cells.

As has already been mentioned, the methods described hereinafter have been shown to produce immune-suppressive cells, which due to their molecular markers seem to be related to if not correspond to cells that are commonly named immune-suppressive or tolerogenic dendritic cells. Thus the immune-suppressive cells according to the invention have been named immune-suppressive dendritic cells. However, dendritic cells are representatives of a broader class of cells, which may be designated as antigen-presenting cells. Thus, the methods as described hereinafter generally refer to the production of immune-suppressive antigen-presenting cells with immune-suppressive dendritic cells being preferred.

The term "immuno-suppressive dendritic cells", which is used synonymous to tolerogenic, immuno-inhibiting, or truncated dendritic cells, thus refers to cells derivable from monocytes by treating the monocytes contained in an extracorporeal quantity of said mammalian subject's blood sample as it is described herein and identifiable by molecular markers as described in the following. These molecular markers include GILZ, IDO (Indoleamine), KMO (kynurenine 3-hydroxylase), transforming growth factor beta (TGFβ), and/or IL-10 (Interleukin 10), with GILZ being a preferred molecular marker. Thus, dendritic cells as they are obtainable by the methods described herein, which show an increased expression of GILZ, IDO, KMO, TGFβ, and/or IL-10, preferably of GILZ are considered to be immuno-suppressive dendritic cells. It is to be understood that an increased expression is to be determined in comparison to the monocytes before they are subjected to the methods described herein. Such immuno-suppressive dendritic cells may also show an GILZ-induced increased IL-10 to IL-12p70 ratio or a decreased production of various pro-inflammatory cytokines and chemokines such as IL-12, TNF-α, and IL-6. The preferred molecular marker, which is considered indicative for immune-suppressive dendritic cells, is currently GILZ. As will be explained further below in more detail the immuno-suppressive dendritic cells may be autologous and/or allogenic immuno-suppressive dendritic cells. Autologous immuno-suppressive dendritic cells may be produced by the methods described herein for treatment of e.g. autoimmune diseases. Allogenic immuno-suppressive dendritic cells may be produced by the methods described herein for treatment of e.g. Graft-versus-Host disease. It needs to be understood that where immuno-suppressive antigen-presenting cells such as dendritic cells are mentioned herein, this refers to immuno-suppressive antigen-presenting cells such as dendritic cells which have the capacity of displaying e.g. disease-specific antigens in their surfaces after these cells have been contacted with such antigens. It is to be further understood that immuno-suppressive dendritic cells as obtainable by the methods described herein and identifiable by the molecular markers described herein in one embodiment may be considered as dendritic cells which have already differentiated enough and internalized and even display e.g. antigens from e.g. cells being involved in an autoimmune disease or antigens of cells from a recipient in e.g. a Graft-versus-Host disease application, such that they can be considered more generally to be immuno-suppressive autologous and/or allogenic antigen-presenting cells. These immuno-suppressive autologous and/or allogenic antigen-presenting cells will nevertheless be of a tolerogenic type and thus suppress an immune answer against the displayed antigens. The term "immuno-suppressive autologous and/or allogenic dendritic cells" in one embodiment thus encompasses immuno-suppressive autologous and/or allogenic antigen-presenting dendritic cells. However, the process can also be conducted in a way such that the dendritic cells express molecular markers indicative of immuno-suppressive autologous and/or allogenic dendritic cells, which have not yet internalized and displayed antigens, because e.g. the cells were obtained from a sample of a donor that does not suffer from an auto-immune disease or Graft-versus-host disease. However, the insight provided by the data and conclusions of the present invention allows to make immuno-suppressive antigen-presenting cells such as dendritic cells, which then can be loaded with antigens characteristic an auto-immune disease or with antigens from a donor in a transplantation setting, which have been obtained separately and not concurrently with the immuno-suppressive antigen-presenting cells such as dendritic cells. Such antigens may then be co-incubated with immuno-suppressive antigen-presenting cells such as dendritic cells to effect their presentation in the subsequent therapeutic setting.

That dendritic cells, which overexpress GILZ, IDO, KMO, PDL1, PDL2, TGFβ and/or IL-10, preferably GILZ and which have been obtained by activating monocytes using processes described herein, show properties reminiscent of tolerogenic dendritic cells can be taken *inter alia* from the observation that such dendritic cells expressing GILZ are resistant to full maturation as it can be induced by e.g. LPS and be determined by increased expression of HLA-DR, CD83, CD80 and CD86. Further, the upregulation of GILZ and the decreased production of various pro-inflammatory cytokines and chemokines such as IL-12, TNF-α, and IL-6 are reminiscent of the known effects of dexamethasone, which can be used to obtain tolerogenic dendritic cells (Cohen et al., Blood (2006), 107(5), 2037-2044).

Thus, immuno-suppressive dendritic cells can additionally be distinguished from immuno-stimulatory autologous dendritic cells in that they do not show an increased expression of molecular markers, which are indicative of immuno-stimulatory autologous dendritic cells. It is to be understood that an expression is to be determined in comparison to the monocytes before they are subjected to the methods described herein.

The term "immuno-stimulatory autologous dendritic cells" refers to dendritic cells, which can be obtained by activating monocyte using the methods described herein and which show an increased expression of molecular markers, which are indicative of immuno-stimulatory autologous dendritic cells. These molecular markers, which are indicative of immuno-stimulatory autologous dendritic cells, have been discussed in the literature for dendritic cells, which can present antigens by way of MHC I and MHC II. It is to be understood that immuno-stimulatory autologous dendritic cells as obtainable by the methods described herein and identifiable by the molecular markers described herein may be considered as dendritic cells which have already differentiated enough and internalized and even display e.g. tumor-specific antigens from apoptotic cells such as cytotoxic T-cells, which are contained in the extracorporeal quantity of a respective mammalian subject's blood sample, or e.g. viral or bacterial antigens, which are contained in the extracorporeal quantity of a respective mammalian subject's blood sample, such that they can be considered to be immuno-stimulatory autologous antigen-presenting dendritic cells. However, the process can also be conducted in a way such that the dendritic cells express molecular markers indicative of immuno-stimulatory dendritic cells, which have not yet internalized and display antigens. The term "immuno-stimulatory autologous dendritic cells" in one embodiment thus encompasses immuno-stimulatory autologous antigen-presenting dendritic cells. It is to be understood that the designation of immuno-stimulatory dendritic cells as immuno-stimulatory autologous dendritic cells depends on whether the obtained immuno-stimulatory dendritic cells are later re-introduced into the same donor. This can be done in a continous or dis-continous fashion where the dendritic cells are cultivated for extended periods of time before they are re-introduced into the donor. All of the issues discussed hereinafter for immuno-stimulatory dendritic cells refer to immuno-stimulatory autologous dendritic cells. It is, however, to be understood that the discussion of such embodiments always includes the scenario where the processes are used to make immuno-stimulatory dendritic cells as such and where only the later administration of these cells will make them potentially immuno-stimulatory autologous dendritic cells.

The present invention allows preferential production of immuno-suppressive DC relative to immuno-stimulatory DC. The preferential production of immuno-suppressive dendritic cells relative to immuno-stimulatory dendritic cells means that starting from an extracorporeal amount of blood sample, more immuno-suppressive dendritic cells than immuno-stimulatory DC may be selectively obtained compared to a situation where e.g. the same extracorporeal amount of blood sample was subjected to a classical ECP procedure. Even though production of immuno-suppressive DC will be produced preferentially relative to immuno-stimulatory DC, immuno-stimulatory DC may be still present after the methods in accordance with the invention have been performed. Nevertheless, the present invention provides the parameters and variables that can be manipulated to skew production of one dendritic cell population over the other. Further, the eventually remaining immuno-stimulatory dendritic cells may be removed by e.g. affinity purification against molecular markers that are indicative of immuno-stimulatory dendritic cells.

As is described in the examples, molecular marker which are indicative of immuno-stimulatory autologous dendritic cells obtainable by the methods described herein were identified by subjecting monocytes contained in the extracorporeal quantity of mammalian subjects' blood samples derived either from healthy volunteers to the process using a miniaturized device (see markers 88 to 99 of Table 1). Further, as is also described in the example, molecular markers, which are indicative of immuno-stimulatory autologous dendritic cells, were identified by subjecting monocytes contained in the extracorporeal quantity of mammalian subjects' blood samples derived either from healthy volunteers or from patients suffering from CTCL to an ECP process. It is also described in the example, how molecular markers, which are indicative of immuno-suppressive dendritic cells, were identified by subjecting monocytes contained in the extracorporeal quantity of mammalian subjects' blood samples derived either from healthy volunteers or from patients suffering from GvH disease (GvHD)to an ECP process (see markers 1 to 87 of Table 1). The dendritic cells were then isolated and up-regulated expression of molecular markers, which are known or suspected to play a role in immuno-stimulatory dendritic cells, was analyzed.

Some of the markers identified for the ECP process, which is assumed to lead to a complex mixture of immune-stimulatory and immune-suppressive dendritic cells, are the same as they were observed for the dendritic cells obtained by the process with the miniaturized device, which should lead to immune-stimulatory dendritic cells only. Thus to the extent that the ECP process leads to up-regulation of molecular markers, which can be associated with dendritic cell function, it seems justified to assume that these markers will also be suitable to identify immune-stimulatory dendritic cells as they are obtainable by the processes described herein such as with the miniaturized device. A set of overall 99 molecular markers was identified as being upregulated for immuno-stimulatory autologous dendritic cells obtainable by methods described herein. This set may be extended by further molecular markers in the future through comparable analysis

Thus, the data of examples 1 and 3 lead to a set of 99 markers, which are considered indicative of immuno-stimulatory autologous dendritic cells. These markers are summarized in Table 1.

**Table 1**

| No. | Marker | NCBI Gene ID No. | mRNA REF | SEQ ID No. |
|---|---|---|---|---|
| 1 | ABCA1 | 19 | NM_005502.3 | 1 |
| 2 | ACVR1 B | 91 | NM_004302.4 | 2 |
| 3 | ANPEP | 290 | NM_001150.2 | 3 |
| 4 | AQP9 | 366 | NM_020980.3 | 4 |
| 5 | ATP6V0B | 533 | NM_001039457.1 | 5 |
| 6 | BASP1 | 10409 | NM_001271606.1 | 6 |
| 7 | BEST1 | 7439 | NM_001139443.1 | 7 |
| 8 | CD63 | 967 | NM_001257389.1 | 8 |
| 9 | CD68 | 968 | NM_001040059.1 | 9 |
| 10 | CDCP1 | 64866 | NM_022842.3 | 10 |
| 11 | CPM | 1368 | NM_001005502.2 | 11 |
| 12 | CRK | 1398 | NM_005206.4 | 12 |
| 13 | CSF2RA | 1438 | NM_001161529.1 | 13 |
| 14 | CTNND1 | 1500 | NM_001085458.1 | 14 |
| 15 | CTSB | 1508 | NM_001908.3 | 15 |
| 16 | CXCL16 | 58191 | NM_001100812.1 | 16 |
| 17 | EMP1 | 2012 | NM_001423.2 | 17 |
| 18 | ENG | 2022 | NM_000118.2 | 18 |
| 19 | EPB41L3 | 23136 | NM_012307.2 | 19 |
| 20 | FLOT1 | 10211 | NM_005803.2 | 20 |
| 21 | GNA15 | 2769 | NM_002068.2 | 21 |
| 22 | GPNMB | 93695 | NM_053110.4 | 22 |
| 23 | GPR137B | 83924 | NM_031999.2 | 23 |
| 24 | GPR157 | 269604 | NM_177366.3 | 24 |
| 25 | HEXB | 3074 | NM_000521.3 | 25 |
| 26 | HOMER3 | 9454 | NM_001145721.1 | 26 |
| 27 | ICAM1 | 3383 | NM_000201.2 | 27 |
| 28 | IL1R1 | 3554 | NM_000877.2 | 28 |
| 29 | IRAK1 | 3654 | NM_001025242.1 | 29 |
| 30 | ITGA5 | 3678 | NM_002205.2 | 30 |
| 31 | ITGB8 | 3696 | NM_002214.2 | 31 |
| 32 | KCTD11 | 147040 | NM_001002914.2 | 32 |
| 33 | LAMP2 | 3920 | NM_001122606.1 | 33 |
| 34 | LEPROT | 54741 | NM_001198681.1 | 34 |
| 35 | LGALS3 | 3958 | NM_001177388.1 | 35 |
| 36 | LILRB4 | 11006 | NM_001081438.1 | 36 |
| 37 | MARCKSL1 | 65108 | NM_023009.6 | 37 |
| 38 | MCOLN1 | 57192 | NM_020533.2 | 38 |
| 39 | MFAP3 | 4238 | NM_001135037.1 | 39 |
| 40 | MGAT4B | 11282 | NM_014275.4 | 40 |
| 41 | MR1 | 3140 | NM_001194999.1 | 41 |
| 42 | MRAS | 22808 | NM_001085049.2 | 42 |
| 43 | MSR1 | 4481 | NM_002445.3 | 43 |
| 44 | NEU1 | 4758 | NM_000434.3 | 44 |
| 45 | NPC1 | 4864 | NM_000271.4 | 45 |
| 46 | OLR1 (LOX1) | 4973 | NM_001172632.1 | 46 |
| 47 | OMG | 4974 | NM_002544.4 | 47 |
| 48 | P2RX4 | 5025 | NM_001256796.1 | 48 |
| 49 | PI4K2A | 55361 | NM_018425.2 | 49 |
| 50 | PLAUR | 5329 | NM_001005376.2 | 50 |
| 51 | PMP22 | 5376 | NM_000304.2 | 51 |
| 52 | PPAP2B | 8613 | NM_003713.4 | 52 |
| 53 | PSEN1 | 5663 | NM_000021.3 | 53 |
| 54 | PVRL2 | 5819 | NM_001042724.1 | 54 |
| 55 | RAB13 | 5872 | NM_002870.2 | 55 |
| 56 | RAB8B | 51762 | NM_016530.2 | 56 |
| 57 | RAB9A | 9367 | NM_001195328.1 | 57 |
| 58 | RALA | 5898 | NM_005402.3 | 58 |
| 59 | RHEB | 6009 | NM_005614.3 | 59 |
| 60 | RNASE1 | 6035 | NM_002933.4 | 60 |
| 61 | SC5DL | 6309 | NM_001024956.2 | 61 |
| 62 | SDC2 | 6383 | NM_002998.3 | 62 |
| 63 | SEMA6B | 10501 | NM_032108.3 | 63 |
| 64 | SIRPA | 140885 | NM_001040022.1 | 64 |
| 65 | SLC17A5 | 26503 | NM_012434.4 | 65 |
| 66 | SLC1A4 | 6509 | NM_001193493.1 | 66 |
| 67 | SLC22A4 | 6583 | NM_003059.2 | 67 |
| 68 | SLC31A1 | 1317 | NM_001859.3 | 68 |
| 69 | SLC35E3 | 55508 | NM_018656.2 | 69 |
| 70 | SLC39A6 | 25800 | NM_001099406.1 | 70 |
| 71 | SLC6A6 | 6533 | NM_001134367.1 | 71 |
| 72 | SLC6A8 | 6535 | NM_001142805.1 | 72 |
| 73 | SLC7A11 | 23657 | NM_014331.3 | 73 |
| 74 | STX3 | 6809 | NM_001178040.1 | 74 |
| 75 | STX6 | 10228 | NM_005819.4 | 75 |
| 76 | TM9SF1 | 10548 | NM_001014842.1 | 76 |
| 77 | TMBIM1 | 64114 | NM_022152.4 | 77 |
| 78 | TMEM33 | 55161 | NM_018126.2 | 78 |
| 79 | TNFRSF10B | 8795 | NM_003842.4 | 79 |
| 80 | TNFRSF11A | 8792 | NM_001270949.1 | 80 |
| 81 | TNFRSF1A | 7132 | NM_001065.3 | 81 |
| 82 | TNFRSF1B | 7133 | NM_001066.2 | 82 |
| 83 | TNFSF14 | 8740 | NM_003807.3 | 83 |
| 84 | TNFSF9 | 8744 | NM_003811.3 | 84 |
| 85 | TRIP10 | 9322 | NM_004240.2 | 85 |
| 86 | TRIP6 | 7205 | NM_003302.2 | 86 |
| 87 | YKT6 | 10652 | NM_006555.3 | 87 |
| 88 | DC-LAMP (LAMP3) | 27074 | NM_014398.3 | 88 |
| 89 | CLEC5A | 23601 | NM_013252.2 | 89 |
| 90 | SPC2 (PCSK2) | 5126 | NM_002594.3 | 90 |
| 91 | THBS1 | 7057 | NM_003246.2 | 91 |
| 92 | CD14 | 929 | NM_000591.3 | 92 |
| 93 | CD40 | 958 | NM_001250.4 | 93 |
| 94 | CD80 | 941 | NM_005191.3 | 94 |
| 95 | CCR7 | 1236 | NM_001838.3 | 95 |
| 96 | CD83 | 9308 | NM_001251901.1 | 96 |
| 97 | ADAM Decysin | 27299 | NM_014479.3 | 97 |
| 98 | FPRL2 (FPR3) | 2359 | NM_002030.3 | 98 |
| 99 | CD86 | 942 | NM_006889.4 | 99 |

Of the 87 genes (markers 1 to 87) that represent surface markers /functional mediators of immunostimulatory DC function, 66 were found to be uniquely identified in the ECP-induced process (plate passaged, overnight cultured, see example) dendritic cells, after comparison to expression databases for "classical" dendritic cells. These are: ABCA1 , ACVR1B, ATP6V0B, BASP1, BEST1, CPM, CRK, CSF2RA, CTNND1, CTSB, CXCL16, ENG, FLOT1, GNA15, GPR137B, GPR157, HEXB, HOMER3, ICAM1, IRAK1, ITGA5, ITGB8, KCTD11, LAMP2, LEPROT, MARCKSL1, MCOLN1, MFAP3, MGAT4B, MR1, MRAS, MSR1, NEU1, OLR1, OMG, PI4K2A, PLAUR, PMP22, PVRL2, RAB13, RAB8B, RAB9A, RALA, RNASE1, SC5DL, SEMA6B, SIRPA, SLC1A4, SLC22A4, SLC31A1, SLC35E3, SLC39A6, SLC6A6, SLC6A8, STX3, STX6, TM9SF1, TMBIM1, TMEM33, TNFRSF10B, TNFRSF11A, TNFRSF1A, TNFRSF1B, TNFSF14, TNFSF9, YKT6.

Immuno-suppressive dendritic cells can thus be distinguished from immune-stimulatory dendritic cells by determining expression of at least one molecular marker for the immuno-stimulatory autologous dendritic cells obtainable by the methods described herein and by comparing its expression for monocytes contained within the extracorporeal quantity of a mammalian subject's blood sample. If no increased expression of these markers is observed for presumed immuno-suppressive dendritic cells vs. monocytes, this is indicative of the differentiation of monocytes to immuno-suppressive dendritic cells.

Immuno-suppressive dendritic cells, in addition to increased expression of GILZ (SEQ ID No.: 100), , IDO (SEQ ID No.: 101), KMO (SEQ ID No.: 102), TGFβ (SEQ ID No.: 103) and/or IL-10 (SEQ ID No.: 104), preferably of GILZ are thus identifiable by determining expression of at least one molecular marker for the immuno-stimulatory autologous dendritic cells obtainable by the methods described herein and by comparing its expression for monocytes contained within the extracorporeal quantity of a mammalian subject's blood sample. If no increased expression of such molecular markers for dendritic cells vs. monocytes is observed, this is indicative of the differentiation of monocytes to immuno-suppressive autologous and/or allogenic dendritic cells.

Immuno-stimulatory autologous dendritic cells are identifiable by determining expression for at least 2, 3, 4, 5, 6, 7, 8, 9, 10, 15, 20, 25, 30, 35, 40, 45, 50 or more molecular markers selectable from Table 1. For example, one may identify immuno-stimulatory autologous dendritic cells by determining expression for at least 1, 2, 3, 4, 5, 6, 7, 8, 9, 10, 11, 12, 13, 14, 15, 16, 17, 18, 19, 20, 21 or 22 molecular markers selectable from the group comprising PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB and/or CD86. More preferably, one may identify immuno-stimulatory autologous dendritic cells by determining expression for at least 1, 2, 3, 4, 5, 6, 7, 8, 9, or 10 molecular markers selectable from the group comprising PLAUR, NEU1, CD80, CCR7, LOX1, CD83, ADAM Decysin, FPRL2, GPNMB and/or CD86. The most preferred markers which are considered indicative of immuno-stimulatory autologous dendritic cells are PLAUR, NEU1, CD80, CD83, and/or CD86. The data and conclusions presented herein suggest that the process of obtaining immuno-stimulatory dendritic cells seems to include a global monocyte activation step and a monocyte to immuno-stimulatory antigen-presenting cell (e.g. dendritic cell) differentiation step. These different steps seem to be traceable by molecular markers as described above and by Forward Scattering/Side Scattering Complexity (FSC/SSC Complexity) which is determinable by FACS analysis. The molecular markers may moreover be be grouped according to their know function as e.g. molecular markers of antigen-presentation, molecular markers of cellular adhesion etc.. HLA-DR, CD86, and CD 80 may be considered to representative of antigen-presentation. PLAUR, and ICAM-1 may be considered to representative of cell adhesion. Markers like HLA-DR, PLAUR and ICAM-1 as well as FSC/SSC complexity may be moreover considered to be indicative of global monocyte activation while increased expression of e.g. CD83, ADAM-Decysin, CD40, CD80, LAMP-3, and CCR7 seems indicative of monocyte to dendritic cell differentiation.

The immuno-stimulatory suppressive dendritic cells as they are obtainable by the methods described herein can thus not only be positively identified by molecular markers, which are indicative of immune-suppressive dendritic cells such as GILZ, IDO, KMO, TGFβ, and/or IL-10, but also by the absence of up-regulation of molecular markers, which are indicative of immune-stimulatory dendritic cells such as PLAUR, CD80 and CD83. Further both of these cell types, i.e. immune-suppressive and immune-stimulatory dendritic cells can be distinguished from the monocytes, which are subjected to a physical force to induce the differentiation process thereof, by determining the expression of molecular markers which are considered indicative of monocytes such as CD33 , CD36, and/or FCGR1a (Receptor for IgGFc fragment 1A). If it is found that the expression of these factors is down-regulated compared to expression of the monocytes, before they have been subjected to a physical force and process as described herein, then this is considered indicative that the monocytes have entered the maturation and differentiation pathway towards immune-stimulatory and/or immuno-suppressive dendritic cells. The distinction between these later two dendritic cell population can then be made by determining expression of molecular markers such as PLAUR, CD80, CD83 and GILZ.

As mentioned above, the method as described hereinafter may be performed without the need of molecular cocktails to achieve maturation and differentiation of monocytes into immuno-suppressive autologous and/or allogenic dendritic cells. Such cocktails may comprise factors such as e.g. dexamethasone IL-10, IL-4 or TGF-β. However, in one embodiment it is considered to add such maturation cocktails to the immuno-suppressive autologous and/or allogenic dendritic cells as they are obtainable by methods in accordance with the invention, e.g. to push the differentiation towards a certain dendritic cell profile that can be achieved with such cocktails.

Given that one now has the understanding and correspondingly the tools, e.g. the molecular markers at hand to distinguish between immuno-suppressive autologous and/or allogenic dendritic cells and immuno-stimulatory autologous dendritic cells, one can now deliberately vary both the design of the device and the flow chamber through which the extracorporeal quantity of a mammalian subject's blood sample and thus the monocytes are passed to experience a physical force, and the parameters at which the process of inducing differentiation of monocytes into immuno-suppressive autologous and/or allogenic dendritic cells is performed, to purposively enable differentiation of monocytes into immuno-suppressive autologous and/or allogenic dendritic cells.

As mentioned above, an extracorporeal quantity of a mammalian subject's blood sample is passed through a flow chamber of a device, such that a shear force is applied to said monocytes contained within said mammalian subject's blood sample. Alterations of the design of the device and the flow chamber which have an influence on the differentiation of monocytes into immuno-suppressive autologous and/or allogenic dendritic cells include variation of flow forces, variation of the geometry of the flow path of the flow chamber, variation of the dimensions of the flow chamber, the possibility to adjust temperature, the possibility of exposure of the extracorporeal quantity of the mammalian subject's blood sample in the flow chamber to visible or UV light, etc.. Application of a physical force may not only be achieved by e.g. passing an extracorporeal amount of blood sample through a flow chamber, but also by placing such an extracorporeal amount of blood sample in e.g. an EVA plastic bag as obtainable from Macopharma and gently moving or shaking this blood sample-filled bag (see e.g. Andreu et al., (1994), Trans. Sci., 15(4), 443-454)

As also mentioned above and shown hereinafter, activation of monocytes and induction of differentiation into immuno-suppressive autologous and/or allogenic dendritic cells is dependent on interaction of monocytes with activated platelets and/or specific plasma components in a situation where the monocytes experience physical force which may be provided by a device as described hereinafter. Variation of process parameters thus include varying the nature, purity and concentrations of plasma components; the nature, purity and concentration of platelets; the order of steps by which plasma components and/or platelets are passed through and/or disposed on the flow chamber; the density by which the flow chamber is coated with plasma components and/or platelets, the flow forces of the extracorporeal quantity of the mammalian subject's blood sample and in particular the platelets and/or the monocytes are passed through the flow chamber of such a flow chamber, the temperature and/or time at which the extracorporeal quantity of the mammalian subject's blood sample and in particular the platelets and/or the monocytes are passed through the flow chamber of such a device, etc., the nature, purity and concentrations of additional factors such as 8-MOP and/or cytokines are added to the extracorporeal quantity of the mammalian subject's blood sample and in particular to the monocytes, etc.

Factors relating to the design of the device and the flow chamber as well as to process parameter will now be discussed in more detail as regards their relevance for the differentiation of monocytes into immuno-suppressive autologous and/or allogenic dendritic cells. It is to be understood that for any of the embodiments discussed in the following differentiation of monocytes into immuno-suppressive autologous and/or allogenic dendritic cells is achieved wherein immuno-suppressive autologous and/or allogenic dendritic cells are identifiable by determining expression of at least GILZ, IDO, KMO, TGFβ, and/or IL-10, preferably of GILZ and/or by determining expression of molecular markers of Table 1. If e.g. expression of GILZ is increased and if expression of molecular markers of Table 1 is not increased, this is considered indicative that monocytes have differentiated into immuno-suppressive autologous and/or allogenic dendritic cells. Further, for all embodiments discussed in the following it is to be understood that monocytes that are contained in an extracorporeal quantity of a mammalian subject's blood sample are subjected to a physical force such as shear stress in order to allow them to differentiate into dendritic cells, e.g. upon interaction with activated platelets and/or plasma components.

In one embodiment of the first aspect, the invention relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample.

In another embodiment of the first aspect, the invention relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said flow chamber of said device has a design allowing to apply a shear force to said monocytes contained within said mammalian subject's blood sample.

In another embodiment of the first aspect, the invention relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and wherein said device additionally allows for adjustment of at least one parameter selected from the group comprising temperature, and light exposure.

In another embodiment of the first aspect, the invention relates to a method of inducing differentiation of monocytes contained in an extracorporeal quantity of a mammalian subject's blood sample into immuno-suppressive autologous and/or allogenic dendritic cells, wherein said extracorporeal quantity of said mammalian subject's blood sample is subjected to a physical force by passing said extracorporeal quantity of said mammalian subject's blood sample through a flow chamber of a device as mentioned before and wherein said monocytes are activated and induced to differentiate into immuno-suppressive autologous and/or allogenic dendritic cells through interaction with activated platelets and/or plasma components.

For all of these embodiments and for all of the embodiment discussed in the following, it is preferred to subject the monocyte contained within the extracorporeal amount of a mammalian's blood sample to a physical force such as shear stress in the presence of DNA-cross linking agent such as 8-MOP and exposing the monocytes to light, preferably to UVA light such that an increased expression of GILZ is effected. The design and dimensions of the flow chamber for all of these embodiments should be preferably selected such that substantially all of the monocytes are in contact with a DNA-cross linking agents such as 8-MOP and that substantially all of the monocytes are exposed to light, preferably to UVA.

For example, in one embodiment of the first aspect, the invention relates to a method of inducing differentiation of monocytes contained in an extracorporeal quantity of a mammalian subject's blood sample into immuno-suppressive autologous and/or allogenic dendritic cells, wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) activating platelets, which may be comprised within said extracorporeal quantity of said mammalian subject's blood or which may be provided separate from said mammalian subject's blood sample comprising at least monocytes,
c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells by binding to said activated platelets obtained in step b).

In another embodiment of the first aspect, the invention relates to a method of inducing differentiation of monocytes contained in an extracorporeal quantity of a mammalian subject's blood sample into immuno-suppressive autologous and/or allogenic dendritic cells, wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) passing plasma components, which may be comprised within said extracorporeal quantity of said mammalian subject's blood sample or which may be provided separate from said mammalian subject's blood sample, c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells by binding to said plasma components obtained in step b).

In yet another embodiment of the first aspect, the invention relates to a method of inducing differentiation of monocytes contained in an extracorporeal quantity of a mammalian subject's blood sample into immuno-suppressive autologous and/or allogenic dendritic cells, wherein said method comprises at least the steps of:
a) applying said extracorporeal quantity of said mammalian subject's blood sample comprising at least monocytes to a device, which is configured to provide for a flow chamber through which said extracorporeal quantity of said mammalian subject's blood sample can be passed,
b) passing plasma components, which may be comprised within said extracorporeal quantity of said mammalian subject's blood or which may be provided separate from said mammalian subject's blood sample,
c) activating platelets, which may be comprised within said extracorporeal quantity of said mammalian subject's blood sample or which may be provided separate from said mammalian subject's blood sample comprising at least monocytes,
d) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells by binding to said activated platelets and/or plasma components obtained in steps b) and c).

In yet another embodiment of the first aspect, the invention relates to a method of inducing differentiation of monocytes contained in an extracorporeal quantity of a mammalian subject's blood sample into immuno- suppressive autologous and/or allogenic dendritic cells, wherein said method comprises at least the steps of:
a) optionally passing platelets-rich plasma through a a device, which is configured to provide for a flow chamber through which said extracorporeal quantity of said mammalian subject's blood sample can be passed,
b) applying said extracorporeal quantity of said mammalian subject's blood sample comprising at least monocytes to a device, which is configured to provide for a flow chamber through which said extracorporeal quantity of said mammalian subject's blood sample can be passed,
c) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-suppressive autologous and/or allogenic dendritic cells optionally by binding to said platelets-rich plasma obtained of step a).

As can be taken from the experiment described herein, the method for inducing differentiation of monocytes contained in an extracorporeal quantity of a mammalian subject's blood into immuno-suppressive autologous and/or allogenic dendritic cells works optimal, if platelets which are comprised within said extracorporeal quantity of said mammalian subject's blood are activated and if the extracorporeal quantity of said mammalian subject's blood comprising at least monocytes in said device is treated by applying a physical force to the monocytes contained within said extracorporeal quantity of said mammalian subject's blood such that said monocytes are activated and induced to differentiate into dendritic cells by binding to said activated platelets. However, activation of monocytes may also be achieved by direct interaction with plasma components, i.e. without interaction with activated platelets. These dendritic cells can then be made embarking on differentiation into immuno-suppressive autologous and/or allogenic dendritic cells by exposing the monocytes additionally to DNA-cross linking agents such as 8-MOP and light such as UVA light.

The steps of activating platelets and the subsequent activation and differentiation of monocytes into dendritic cells will be discussed in the following for the embodiment that (i) plasma components such as plasma proteins are passed through the flow chamber of the device so that these components adhere to the walls of the flow chamber, that (ii) platelets are passed through the flow chamber and are activated by binding to the plasma components and that (iii) monocytes-containing fractions such as an extracorporeal quantity of said mammalian subject's blood comprising at least monocytes are passed through the flow chamber and are activated for differentiation into dendritic cells by binding to the activated platelets. It is, however, to be understood that these activities also occur if the plasma fraction or plasma proteins or fragments thereof, the platelet fraction and the monocytes-containing fraction are passed simultaneously through the channels or channel-like structures as is the case for a whole blood fraction if obtained from the extracorporeal amount of blood as described below. It is further to be understood that the process may be performed even though not with same effectiveness by adhering only plasma components to the walls of the flow chamber and letting monocytes interact with the plasma components. Nevertheless, in the following these aspect will be discussed for a preferred embodiment, i.e. where steps (i), (ii), and (iii) are realized.

As regards the first step, plasma components including proteins like fibrinogen or fibronectin, or fragments thereof like the gamma component of fibrinogen may be provided either as fractions obtained from the extracorporeal amount of blood sample or in purified form from other resources e.g. in the form of recombinantly expressed proteins. Even though it seems that activation of platelets by plasma proteins such as fibrinogen and fibronectin is sufficient so that recombinantly expressed forms of these proteins are sufficient, it can be preferred to use plasma fractions which are obtained from the extracorporeal amount of blood sample and comprise these proteins as these plasma fractions have a more complex composition and may comprise all plasma components, which provide for an optimal activation of platelets.

Plasma protein fractions, plasma proteins or fragments thereof may be passed through the flow chamber, which may be made of plastic or non-plastic materials such as glass in order to adhere to the walls of the channels or channel-like structures. There is no requirement that the plasma fractions or plasma proteins are passed through the flow chamber at a specific physical force such as e.g. a specific pressure. However, in order to streamline the process, it is envisaged to pass the plasma fractions or plasma proteins through the flow chamber at a shear stress which is comparable if not identical to the shear stress required for monocyte activation being described in more detail below. In general, the plasma fractions or plasma proteins are first pumped through the flow chamber to coat the surfaces thereof with plasma proteins, including fibronectin and fibrinogen. The flow rate of the plasma protein fractions, plasma proteins or fragments thereof through the flow chamber is controlled to obtain a desired level of protein adherence to the plastic surfaces. If desired, the flow can be stopped for a period of time and the plasma component can "soak" the surfaces of the flow chamber. By controlling the speed and timing of the pump that propels the plasma components through the flow chamber, the degree of coating of can be controlled. In one approach, the plasma fractions or plasma proteins are exposed to the surfaces of the flow chamber structures for a period between about 1 to 60 min, between about 1 to about 30 min, between about 1 to about 20 min, or between about 1 to about 10 min. To enhance plasma protein adherence to the surfaces of the flow chamber, the flow may be temporarily discontinued (for up to about 60 min), before resumption, or the flow rate may be slowed from the filling rate (up to 100 ml/minute) to as low as 5 ml/minute, during this phase of the procedure.

One can also envisage a scenario, where a device with a flow chamber is used for which the surfaces of the flow chamber have been pre-coated with e.g. purified plasma proteins or fragments thereof such as the gamma component of fibrinogen. Such pre-coated devices may be used if the whole process s conducted in a handheld device comprising a cartridge providing the flow chamber which is configured for e.g. one time use. One can also envisage a scenario, where a device with a flow chamber is used for which the surfaces of the flow chamber have been pre-coated with e.g. platelets-rich plasma.

After the plasma fractions or plasma proteins or fragments thereof have been passed through the channels or channel-like structures and the surfaces thereof have been coated with plasma proteins, the platelet fraction is passed by e.g. pumping into and through the flow chamber. The flow rate and residence time of the platelets within the flow chamber is selected to allow the platelets to bind to the plasma components or proteins or fragments thereof which have adhered before to the surfaces of the flow chamber and to thereby become activated.

The data presented herein suggest that activation of platelets by plasma components is a sequential process in which inactivated platelets first bind to the gamma component of fibronectin, get activated thereby and can then bind to the RGD motif (Arginine, Glycine, Aspartic Acid) which is found in many plasma proteins such as fibronectin or fibrinogen. If purified and/or recombinantly expressed plasma proteins or fragments thereof are used for activation of platelets, it can therefore be envisaged to pre-coat channels or channel-like structures with at least the gamma-component of fibrinogen and optionally additionally with RGD peptides. These plasma protein fragments and peptides may allow for efficient activation of platelets and at the same time for an optimal control of the coating process of the surfaces of the channels or channel-like structures. Of course, all of these components are present if a plasma fraction obtained from the extracorporeal amount of blood is used for coating and activation.

For efficient binding of the platelets to the plasma components and activation thereby, the flow rate may be adjusted upward or downward compared to the coating step of the plasma components, or flow may be stopped for a period of time, to obtain the desired level of platelets bound to the plasma components. The flow rates for plasma activation will typically be in the range of about 5 ml/min to about 200 ml/min, of about 10 ml/min to about 150 ml/min, of about 10 ml/min to about 100 ml/min, or of about 5 ml/min to about 50 ml/min. Typically, it will be desirable to allow between about 1 to 60 min, between about 1 to about 30 min, between about 1 to about 20 min, or between about 1 to about 10 min for the platelets to bind to the plasma components.

Even though shear stress does not seem to of the same importance for activation of platelets as for activation of monocytes, it can be preferred to pass the platelets fraction through the flow chamber under a shear force of about 0.1 to about 20.0 dynes/cm², of about 0.2 to about 15.0 dynes/cm², of about 0.3 to about 10.0 dynes/cm² such as from about 0.2 to about 0.4, to about 0.5, to about 0.6, to about 0.7, to about 0.8, to about 0.9, to about 1, to about 2, to about 3, to about 4, to about 5, or to about 6 dynes/cm². Typical flow rates of the platelets-containing fraction may be in the range of about 5 ml/min to about 200 ml/min, of about 10 ml/min to about 150 ml/min, of about 10 ml/min to about 100 ml/min, or of about 5 ml/min to about 50 ml/min. The flow rates will depend to some extent on the size and geometry of the flow chamber and can particularly be used if flow chamber of the below-mentioned dimensions are used. In general, one will select flow rates to achieve the afore-mentioned shear stress values.

Thus, it is contemplated to pass the platelets-containing fraction through the channels or channel-like structures with a flow rate of about 10 ml/minute to about 200 ml/minute to produce a shear force of about 0.1 to about 10.0 dynes/cm².

After the platelets have been passed through the channels or channel-like structures and have been activated by the plasma proteins or fragments thereof, which have been disposed on the surfaces of the channels or channel-like structures thereof, the monocytes-containing fraction, e.g. the extracorporeal quantity of said mammalian subject's blood sample or the below-mentioned leukocyte or buffy coat fraction, which have been obtained from the extracorporeal amount of blood sample, is passed by e.g. pumping into and through the channels or channel-like structures, by applying a physical force. It is to be understood that activation of platelets through interaction with plasma components will lead to adherence of platelets to plasma components.

It is also to be understood that the same events as described above will happen if an extracorporeal quantity of a mammalian subject's blood sample comprising platelets and plasma components is passed through the flow chamber. In this case, plasma components will adhere to the walls to the flow chamber and then activate platelets. However, in this scenario the process may be less controllable and account may be taken of this by increasing the residence time of the extracorporeal quantity of a mammalian subject's blood sample comprising platelets and plasma components in the flow chamber.

It is further to be noticed that instead of activated platelets, factors derived from platelets may be used, which are sufficient to monocytes. These factors include e.g. fibronectin and may also include factors such as P-selectin, Integrin α5β1the C-type lectin receptor, CD61, CD36, CD47 and complement inhibitors such as CD55 and CD59, or TREM-like transcipt-1. Such platelet-derived factors may also be disposed directly on the surfaces of the flow chamber either as e.g. mixtures of purified components or mixtures of components obtained by e.g. lysis of platelets contained within the extracorporeal quantity of a mammalian subject's blood sample. In this case, the need for e.g. coating the surfaces of the flow chamber with plasma components may be bypassed.

The data presented herein suggest that once platelets have been activated, proteins such as P-selectin and RGD-containing ligands are expressed by the activated platelets, which can then interact with monocytes and activate their differentiation into dendritic cells. Moreover, it was found monocyte activation and dendritic cell induction by activated platelets do not occur under static conditions. Rather monocytes need to be passed through the channels or channel-like structures under application of a physical force. Given that platelets upon activation need about 60 to about 120 min to express factors such as P-selectin, which then activates monocytes, passing of monocytes may be delayed until platelets have started to express these factors, e.g. for about 60 to about 120 min. If an extracorporeal quantity of a mammalian subject's blood sample comprising monocytes, platelets and plasma components is passed through the flow chamber, this time period may have to be adjusted to longer times.

It is to be understood that interaction of monocytes with activated platelets, platelet-derived factors or plasma components is not sufficient for activation and differentiation of monocytes without the application of a physical force at the same time.

Application of a physical force for moving the monocytes-containing fraction through the flow chamber preferably may mean that a monocytes-containing fraction such as the extracorporeal quantity of a mammalian subject's blood sample is moved through the flow chamber under shear stress. Typically, monocytes-containing fraction may be passed through the flow chamber under a shear force of about 0.1 to about 20.0 dynes/cm², of about 0.2 to about 10.0 dynes/cm², such as from about 0.2 to about 0.3, to about 0.4, to about 0.5, to about 0.6, to about 0.7, to about 0.8, to about 0.9, to about 1, to about 1.5, or to about 2 dynes/cm². Typical flow rates of the monocytes-containing fraction may be in the range of about 5 ml/min to about 200 ml/min, of about 10 ml/min to about 150 ml/min, of about 10 ml/min to about 100 ml/min, or of about 5 ml/min to about 50 ml/min. The flow rates will depend to some extent on the size and geometry of the flow chamber and can particularly be used if channels or channel-like structures of the below-mentioned dimensions are used. In general, one will select flow rates to achieve the afore-mentioned shear stress values.

Thus, it is contemplated to pass the monocytes-containing fraction through the channels or channel-like structures with a flow rate of about 10 ml/minute to about 200 ml/minute to produce a shear force of about 0.1 to about 0.5 dynes/cm². In any case it must be made sure that a shear force is generated that allows binding of monocytes to activated platelets and differentiation of such activated monocytes into dendritic cells.

The data presented herein suggests that monocyte-platelet interaction can be divided into short-acting interactions which are arbitrarily defined as contact occurring for less than 3 seconds by detection with a light microscope and long-acting interactions, defined as contact longer than 3 seconds by detection with a light microscope. It seems that the initial short-acting interactions are mediated by P-selectin which is expressed on activated platelets. These initial contacts can then subsequently trigger long-acting interactions mediated by RGD-containing proteins expressed by the activated platelets.

The activation of monocytes and differentiation into dendritic cells may be positively influenced by allowing the monocytes to establish long-acting contacts with platelets, e.g. by giving the monocytes and platelets enough time to interact. As the monocytes flow through the channels or channel-like structures, they alternately bind to and disadhere from the platelets by the shearing force induced by the flow through the flow chamber. The residence time of the monocyte/platelet interaction may be controlled by varying the flow rate, e.g. by controlling the speed of the pump. For example, the pump may initially be operated at a slow speed/low flow rate to enhance monocyte/platelet interaction, and the speed/flow rate may then be increased to facilitate disadherence and collection of the treated monocytes from the treatment device. It seems that adherence of the monocytes to the platelets may be best accomplished at about 0.1 to about 2 dynes/cm², at about 0.1 to about 1 dynes/cm², and preferably at about 0.1 to about 0.5 dynes/cm², while disadherence and collection of the monocytes may be best accomplished at increased shear levels.

It is to be understood that activation of monocytes leads to immobilization, e.g. by interacting with activated platelets, platelets-derived factors or plasma components. In order to harvest the induced immuno-suppressive autologous and/or allogenic dendritic cells, one may increase the shear stress to e.g. 20 Dynes/cm² and/or may treat the immuno-suppressive autologous and/or allogenic dendritic cells with factors allowing disadherence from activated platelets, platelets-derived factors or plasma components by adding factors such as Plavic, Aspirin or other blood thinners. Temperature is another factor to influence activation of monocytes and their differentiation into dendritic cells. The methods in accordance with the invention may be performed in a range of about 18°C to about 42°C, preferably in a range of about 22°C to about 41°C and more preferably in a range of about 37°C to about 41°C.

One parameter that can also be varied to tune activation of monocytes is the density by which the flow chamber is coated with plasma components and thus with platelets that bind to the plasma components. In general, the denser the surfaces of the flow chamber are coated with plasma components and platelets, the more efficient will be the monocyte activation.

It has been mentioned above, that platelets are activated by binding to plasma components. The term "activated platelets" in accordance with the invention is used to refer to platelets which show an increased expression of P-selectin, αIIb-β3 integrin and/or RGD-containing proteins such as fibronectin, fibrinogen or vitronectin as a consequence of binding of platelets to plasma components such as fibronectin and/or fibrinogen. Expression may be determined by conventional methods such as RT-PCR, Western-Blotting or FACS analysis. The term "unactivated platelets" in accordance with the invention is used to refer to platelets for which binding to plasma proteins such as fibronectin or fibrinogen cannot be reduced by pre-incubating platelets with the gamma component of fibrinogen.

It has been mentioned above, that monocytes are activated and start to differentiate into dendritic cells by binding to activated platelets under shear stress conditions. The term "activated monocytes" in accordance with the invention is used to refer to monocytes which upon binding to activated platelets under shear stress conditions express increased levels of the open confirmation of β1-integrin and start expressing markers of maturing dendritic cells such as HLA-DR⁺/CD83⁺. As a control to determine whether interaction of monocytes with activated platelets leads to activation and differentiation of dendritic cells one can compare expression of HLA-DR⁺/CD83⁺ after binding of monocytes to activated platelets under shear stress condition either in the absence of anti-P-selectin antibodies (activation) or presence of anti-P-selectin antibodies (control). Expression may be determined by conventional methods such as RT-PCR, Western-Blotting or FACS analysis. If one the additionally applies DNA-cross linking agents such as 8-MOP and exposes the activated monocytes to light such as UVA, one can direct the differentiation process towards immuno-suppressive autologous and/or allogenic dendritic cells and thus to increase expression of GILZ. As one can take from the data of example 2, such cells even if treated with LPS will be resistant to differentiation into immuno-stimulatory autologous dendritic cells and will thus not continue to express HLA-DR, CD83, CD80 and CD86 at high levels, but rather will maintain the tolerogenic state.

After monocytes have been activated by a method in accordance with the invention, they start differentiating into immuno-suppressive autologous and/or allogenic dendritic cells. The term "immuno-suppressive autologous and/or allogenic dendritic cells s" in accordance with the invention is used as mentioned above. These immuno-suppressive autologous and/or allogenic dendritic cells can be identified by increase expression of e.g. GILZ and preferably additionally no increased expression of molecular markers of Table 1.

The experimental findings described herein further immediately suggest various embodiments of this first aspect that can provide for different advantages.

The finding, that activation of monocytes and subsequent induction of differentiation of these monocytes into immuno-suppressive autologous and/or allogenic dendritic cells can be achieved in a miniaturized device, allows to conduct the process with smaller amounts of an extracorporeal blood sample. As mentioned above, the classical ECP procedure requires processing of 2.5L to 6L blood, which is typically obtained from patients by apheresis such as leukaphereses, to obtain a final volume of about 200 ml to 500 ml comprising leukocytes including monocytes as well as plasma components and platelets.

However, the methods in accordance with the invention may require substantial lower amount of blood samples thus bypassing the need of apheresis such as leukaphereses or other processes, which are a considerable burden to patients.

Thus, the present invention can be performed without the need for apheresis such as leukaphereses and the whole process of obtaining such immuno-suppressive autologous dendritic cells may be performed in a handheld device.

Thus, in one embodiment of the first aspect of the invention, which may be combined with the above described embodiments, it is contemplated to perform the method in accordance with the first aspect, wherein said extracorporeal quantity of said mammalian subject's blood is not obtained by apheresis such as leukaphereses.

Said extracorporeal quantity of said mammalian subject's blood may be between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal blood of said mammalian subject to give a final volume between about 1 ml to about 100 ml, between about 1 ml to about 50 ml, between about 1 ml to about 40 ml, or between about 1 ml to about 30 ml an extracorporeal amount of a mammalian's blood sample.

The quantity of extracorporeal blood withdrawn and applied to the device may be whole blood. Alternatively, said extracorporeal quantity of said mammalian subject's blood may be obtained by isolating leukocytes from between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal whole blood of said mammalian subject.

Said extracorporeal quantity of said mammalian subject's blood may also be obtained by isolating buffy coats from between about 5 ml to about 500 ml, between about 10 ml to about 450 ml, between about 20 ml to about 400 ml, between about 30 ml to about 350 ml, between about 40 ml to about 300 ml, or between about 50 ml to about 200 ml or between about 50 ml to about 100 ml of extracorporeal whole blood of said mammalian subject.

In all of the afore-mentioned cases (whole blood, leukocyte fraction, buffy coats), said extracorporeal amount of blood will typically comprise between about 1x10⁴ to about 1x10⁸ such as about 5x10⁶ mononuclear cells/ml.

The person skilled in the art is familiar how to obtain whole blood, a leukocyte fraction thereof or a buffy coat fraction thereof (see e.g. Bruil et al., Transfusion Medicine Reviews (1995), IX (2), 145-166) an include filtration, differential centrifugation. A preferred method relies on filters, as they are available from e.g. Pall. Such filters may be incorporated into the device such that processing of the extracorporeal sample can be done in the handheld device. As a source one can also use e.g. blood of the umbilical cord.

If one uses centrifugation, one may obtain whole blood through a syringe with e.g. a 17 or 18 gauge-gauge needle. Such a whole blood sample may be centrifuged to remove debris and other components. The whole blood sample may then be filtered through common filters, as they are available from Pall.

For obtaining a mononuclear leukocyte fraction, one may obtain a whole blood sample as described and then layer such a sample on e.g. Ficoll-Hypaque. Subsequently a centrifugation step is performed at e.g. about 100g to about 200 g such as 180 g and the mononuclear leukocyte fraction can then be collected from the interface and washed with common buffers such as HBSS. The washed mononuclear leukocyte fraction can then be resuspended in serum-free cell culture medium such as RPMI-1640 medium (GIBCO). Other methods for obtaining mononuclear leukocyte fractions include elutriation, filtration, density centrifugation, etc..

As pointed out above, crucial steps for the induction of dendritic cells formation seem to involve the activation of platelets by plasma components and the activation of monocytes by such activated platelets. In principle, one could pass a whole blood sample through the device under shear stress. The plasma components of such a sample will then bind to the surfaces of the flow chamber and allow for adherence and activation of platelets within such a sample by plasma-components. The monocytes of such a sample will then bind to the activated platelets and be activated themselves.

Similarly one may obtain combinations of the various components such as a platelet-rich plasma containing fraction which may be obtained by centrifuging a whole blood sample which has been obtained as described above at about 100 g to about 180 g such as about 150 g for about 10 min to about 20 min such as about 15 min to separate the debris of the whole blood sample. The platelet-rich plasma layer is then collected and recentrifuged at about 700 g to about 1000 g such as about 900 g for about 3 min to about 10 min such as about 5 min. The resultant pellet is then resuspended in serum-free cell culture medium.

However, in order to have the best control over the process, it may be desirable to first pass plasma components through the flow chamber and let them adhere, then platelets and then the monocytes-containing fraction. For this approach, it may be desirable to obtain a leukocyte fraction comprising a monocytes- or buffy-coat fraction comprising monocytes, which does not comprise plasma components and which does not comprise platelets. Such plasma- and platelet-free monocytes-containing fractions may be obtained as is described in the art. If leukocyte or buffy-coat fractions are obtained as described above, they will be sufficiently free of plasma or platelets for the purposes of the invention. For this approach, it may also be desirable to have platelet- and/or plasma-fractions.

Thus, the invention contemplates to use platelets which have been separated from the extracorporeal quantity of said mammalian subject's blood before said extracorporeal quantity of said mammalian subject's blood is applied to said device. These platelets may then be passed through the flow chamber, which has been coated with plasma components such as fibronectin.

In another embodiment, the invention considers to use plasma components, which have been separated from the extracorporeal quantity of said mammalian subject's blood before said extracorporeal quantity of said mammalian subject's blood is applied to said device. These plasma components may then be passed through flow chamber so that they can adhere.

Instead of using plasma components which have been obtained from the extracorporeal amount of blood, one may also use plasma components, which have been isolated from other sources such as e.g. by recombinant protein expression. Such plasma components include fibrinogen, fibronectin, P-selectin, and fragments thereof such as the gamma component of fibrinogen.

Even though it may be preferred to use an extracorporeal amount of blood, which has not been obtained by apheresis such as leukaphereses, using an extracorporeal amount of blood, which was obtained by apheresis such as leukaphereses is not excluded by the invention.

Thus, in another embodiment of the first aspect of the invention it is contemplated to perform the method as described above, wherein said extracorporeal quantity of said mammalian subject's blood is obtained by apheresis such as leukaphereses.

Apheresis such as leukaphereses may be performed as is known in the art. Thus, an extracorporeal quantity of blood such as 2.5 L to 6 L may be obtained from a subject and treated by conventional leukaphereses to obtain three fractions, namely the plasma, the platelets and the buffy coats. The plasma, which contains proteins such as fibronectin and fibrinogen, is the lightest blood fraction, and therefore is the first portion of the blood selectively removed from the centrifuge and passaged through channels or channel-like structures. After the plasma has been pumped through the channels or channel-like structures and the surfaces thereof have been coated with plasma proteins, the second lightest component in the leukaphereses centrifuge, the platelet fraction, is pumped into and through the channels or channel-like structures. The third lightest fraction to be eluted from the leukaphereses centrifuge is the buffy coat, which contains the white blood cells, including the blood monocytes. The buffy coat including the monocytes is then pumped through the channels or channel-like structures. Blood sample may be obtained using the Therakos device, the Spectra cell separator (see Andreu et al., (1994), Transf. Sci., 15(4), 443-454), or the Theraflex device from Macopharma.

Thus, the invention in one embodiment the invention considers to use platelets which have been separated from the extracorporeal quantity of said mammalian subject's blood obtained by apheresis such as leukaphereses before said extracorporeal quantity of said mammalian subject's blood comprising monocytes is applied to said device.

In another embodiment the invention considers to use plasma components, which have been separated from the extracorporeal quantity of said mammalian subject's blood obtained by apheresis such as leukaphereses before said extracorporeal quantity of said mammalian subject's blood comprising monocytes and/or platelets is applied to said device.

Instead of using plasma components which have been obtained from the extracorporeal amount of blood, one may use also either plasma components which have been isolated from other sources such as e.g. by recombinant protein expression. Such plasma components include fibrinogen, fibronectin, or P-selectin. One can also use fragments of plasma proteins such as the gamma component of fibrinogen which corresponds to amino acids 400-411 (SEQ ID NO.: 105, His-His-Leu-Gly-Gly-Ala-Lys-Gln-Ala-Gly-Asp-Val). This gamma component is shown by the data presented herein to be able to activate platelets. It can therefore be preferred to use plasma fractions which at least, if not predominantly comprise fibronectin. Similarly, it can be preferred to use e.g. recombinantly expressed and/or purified fibronectin or the gamma component thereof to activate platelets.

For both embodiments of the first aspect of the invention where the extracorporeal amount of blood is obtained or not obtained by apheresis such as leukaphereses, it may be considered to pass all three fractions, namely plasma components, platelets and the monocytes-containing fraction at once, e.g. even in the form of a whole blood sample or by using only pre-purified fractions of whole blood, through the flow chamber even though the afore-described sequential passing of these fractions through the flow chamber may provide for better control over the process. Pre-purified fractions of whole blood may be obtained by e.g. centrifuging a blood bag and squeezing out the supernatant, which would be enriched in white blood cells and platelets.

As mentioned the flow rate through flow chamber and thus the resulting shear stress will effect the differentiation of the monocytes into dendritic cells. Further, application of photo-activatable DNA-cross linking agents such as 8-MOP and exposure to light such as UVA light can be used to induce increased expression of GILZ. Aside from the flow rate, the design and the dimensions of the flow chamber may be varied to manipulate and even improve the application of a physical force to the monocytes as well as to make sure that substantially all monocytes can be contacted with DNA-cross linking agents such as 8-MOP and exposed to light such as UVA..

A device having a flow chamber with channels or channel-like structures may be suitable. Such a flow chamber having the general architecture, albeit at smaller dimensions, of a device, which is used for the classical ECP procedure is depicted in Fig. 17.

However, other geometries such as those depicted in Fig. 18 a) to d) may also be used. Thus, the findings described herein allow to consider flow chambers of significantly simplified geometry, which also allows having better control over the process in terms of turbulences and shear stress occurring during the process.

A device having a multiplicity of flow chambers may be suitable. Such a flow chamber having the general architecture, albeit at smaller dimensions, of a device, which is used for the classical ECP procedure is depicted in Fig. 17.

Typically, a flow gradient will be created in the flow chamber such as channels as the monocytes-containing fraction is passed through. The monocytes will alternately bind to and disengage from the platelets and/or plasma components. Maturation of monocytes into dendritic cells is greatly enhanced by this interaction, with increased exposure to the platelets and/or plasma components thereby providing increased signaling of this maturational process.

In order to obtain a homogenous population of immuno-suppressive autologous and/or allogenic dendritic cells as possible, it is therefore desirable that the design and the dimensions of the flow chamber, such as channels is selected avoid different flow zones in the flow chamber.

The flow chamber such as channels may in principle have any cross-sectional shape suitable for the above described purposes. They thus may have a rectangular, round, elliptical, or other cross-sectional form. Even though the dimensions of such flow chamber will be discussed in the following mainly with respect to a rectangular cross-section, it can be preferred that flow chamber such as channels with an elliptical or round cross-section are used as such cross-sections should allow for e.g. more homogenous coating with plasma components and/or more continuous flow properties with less turbulences.

If having a rectangular cross-sections, flow chamber such as channels may have dimensions of about 5 µm to up to about 500 µm of height and of about 5 µm to up to about 500 µm of width. The channels or channel-like structures may also have dimensions of about 10 µm to up to and including about 400 µm of height and of about 10 µm to up to and including about 400 µm of width, of about 10 µm to up to and including about 300 µm of height and of about 10 µm to up to and including about 300 µm of width, of about 10 µm to up to and including about 250 µm of height and of about 10 µm to up to and including about 250 µm of width, of about 10 µm to up to and including about 100 µm of height and of about 10 µm to up to and including about 100 µm of width, or of about 10 µm to up to and including about 50 µm of height and of about 10 µm to up to and including about 50 µm of width.

If flow chambers such as channels of elliptical cross-section are used, the aforementioned dimensions of height and width would have to be adapted correspondingly to allow for a comparable volume.

If flow chambers such as channels of round cross-sections are used, the diameter may typically be in the range of about 5 µm to up to and including about 500 µm, of about 10 µm to up to and including about 400 µm, of about 10 µm to up to and including about 300 µm, of about 10 µm to up to and including about 250 µm, of about 10 µm to up to and including about 100 µm, or of about 10 µm to up to and including about 50 µm.

Smaller dimensions are generally preferred for the flow chambers with a particular preference for height, widths or diameters of below 100 µm such as 50 µm the reason being that it is assumed that for such smaller dimensions interaction of monocytes with platelets is more efficient and uniform and flow properties at the surfaces and in the center of the flow chamber are more comparable.

The length of the flow chamber such as channels channel-like structures is usually selected such that the flow chamber allows for passage of the volume of extracorporeal blood. For example the flow chamber and the device may be configured to allow for passing of an overall volume of between about 1 ml to about 50 ml, between about 1 ml to about 40 ml, or between about 1 ml to about 30 ml.

The flow chamber may have internal sub structures to increase the surface area or to make the flow conditions less heterogeneous.

The flow chamber may be filled with particles to increase the surface area or to make the flow conditions less heterogeneous.

The material of the flow chamber may be plastic or non-plastic.

If non-plastic materials are considered, one may use glass.

The surface of the chamber may be coated covalently or via adsorption.

Materials for auxiliary tubing, chambers, valves etc. may be selected to for having reduced interactions with blood components.

Surfaces of auxiliary tubing, chambers, valves etc. may be treated/coated for having reduced interactions with blood components.

If plastic materials are considered, one may use acrylics, polycarbonate, polyetherimide, polysulfone, polyphenylsulfone, styrenes, polyurethane, polyethylene, teflon or any other appropriate medical grade plastic. In a preferred embodiment of the present invention, the flow chamber is made from an acrylic plastic.

The flow chamber may be made of a material that provides a degree of transparency such that the sample within the flow chamber such as the monocytes-containing fractions can be irradiated with visible or UV light, preferably with UV-A. As is shown by the experiments, exposure to UV-A and 8-MOP leads to increased expression of GILZ and thus to activation and differentiation of monocytes into immuno-suppressive autologous and/or allogenic dendritic cells. Thus exposure to light such as UV-A and DNA-cross linking agents such as 8-MOP should be optimized when producing immuno-suppressive autologous and/or allogenic dendritic cells.

However, once monocytes have embarked on the maturation pathway long enough such that immuno-suppressive autologous and/or allogenic dendritic cells have formed as can be determined by the molecular markers mentioned above, one can envisage to stop adding DNA-cross linking agents such as 8-MOP and to stop exposing the immuno-suppressive autologous and/or allogenic dendritic cells to e.g. UV-A. As shown in Example 2, once the immuno-suppressive autologous and/or allogenic dendritic cells have formed as is determinable by increased expression of GILZ, they are resistant to e.g. LPS-induced maturation. However, by contacting autologous and/or allogenic leukocytes, which are contained within the extracorporeal amount of a mammalian's blood sample and which have become apoptotic by e.g. the UVA administration, GILZ expression of the immuno-suppressive autologous and/or allogenic dendritic cells can be further increased. Thus, one can e.g. incubate immuno-suppressive autologous and/or allogenic dendritic cells with such apoptotic leukocytes such that that the immuno-suppressive autologous and/or allogenic dendritic cells will take up and display antigens from the apoptotic leukocytes to obtain immuno-suppressive autologous and/or allogenic antigen-presenting cells. Given that the immuno-suppressive autologous and/or allogenic dendritic cells have already embarked on a tolerogenic state, such immuno-suppressive autologous and/or allogenic antigen-presenting cells can then be used for treatment of autoimmune diseases, hypersensitivity diseases, rejection in solid-organ transplantation or treatment of Graft-versus-Host disease.

A typical flow chamber may have the geometry depicted in Fig. 19A). The flow path has dimensions of 20 mm by 80 mm. The chamber is made of polystyrene, PET (polyethylenteherephtalate), PMMA (poly (methyl mathacrylate)) and silicon. A blood sample may be spun at low speed through a Ficoll gradient to obtain e.g. 8 ml of sample with a concentration of white blood cells of e.g. 10¹⁰ cells/ml. The chamber may be pre-coated with platelets-rich plasma. The sample may be passed through the chamber at about 0.028 Pa for about... min. The chamber may then be washed with about 3 ml RPMI at 0.028 Pa. A second wash with 30-55 ml RPMI may be performed at about 1.2 Pa. The collected activated monocytes will then be combined and used for further analysis.

In this way immuno-suppressive autologous and/or allogenic dendritic cells and immuno-suppressive autologous and/or allogenic antigen-presenting cells can be obtained without the need for rather expensive cocktails of cytokines, which moreover use unphysiologically high concentrations and require cell incubation for a couple of days, for induction of monocyte to dendritic cell differentiation. Even though not necessary, it can be considered to cultivate such immuno-suppressive autologous and/or allogenic dendritic cells and immuno-suppressive autologous and/or allogenic antigen-presenting cells in a buffered culture medium with one or more cytokines and/or chemokines, such as IL-10, IL-4 or TGF-ß during the incubation period, to further enhance differentiation into the tolerogenic state. Cultivation, if considered, may be performed under standard conidtions, e.g. at 37° C and 5% CO₂ in standard mediums for culturing of human cells such as in RPMI-1640 medium (obtainable e.g. from GIBCO), supplemented with 15% AB serum (obtainable from e.g. Gemini Bio-Products).

Further, immuno-suppressive autologous and/or allogenic dendritic cells can then be manipulated ex vivo, prior to re-administration to the subject, in order to tailor them for the desired therapeutic purpose.

Thus, prior to re-administration to the subject, such immuno-suppressive autologous and/or allogenic dendritic cells can e.g. be processed ex vivo, such as by loading them with antigens, being derived from cells involved in autoimmune diseases, hypersensitivity diseases, solid-organ transplantation rejection and Graft-versus-Host disease. Such antigens may e.g. provided in the form of apoptotic cells comprising these antigens. This will lead to immuno-suppressive autologous and/or allogenic antigen-presenting cells. One of the advantages of the invention provided by the insight presented herein is that obtaining immuno-suppressive autologous and/or allogenic dendritic cells can be separated from loading with antigens, being derived from cells involved in autoimmune diseases, hypersensitivity diseases, solid-organ transplantation rejection and Graft-versus-Host disease. In this way, immuno-suppressive autologous and/or allogenic dendritic cells can be isolated and tailored for specific purposes.

The immuno-suppressive autologous and/or allogenic dendritic cells may in particular be loaded with disease antigens to produce antigen presenting dendritic cells which upon re-introduction into the subject will suppress an immune response against the antigens.

For treatment of autoimmune diseases the antigens may be selected for diseases such as wherein the autoimmune disease is selected from the group comprising rheumatoid arthritis, psoriasis, multiple sclerosis, type 1 diabetes and systemic lupus erythematosus.

The immuno-suppressive autologous and/or allogenic dendritic cells obtained in accordance with the invention and disease effector agents are incubated for a period of time sufficient to maximize the number of functional antigen presenting dendritic cells in the incubated cell population. Typically, the treated blood cell concentrate and possibly antigens are incubated for a period of from about 1 to about 24 hours, with the preferred incubation time extending over a period of from about 12 to about 24 hours. Additional incubation time may be necessary to fully mature the loaded immuno-suppressive antigen-presenting cells prior to reintroduction to the subject. Preferably, the blood cell concentrate and disease effector agents are incubated at a temperature of between 35° C and 40° C. In a particularly preferred embodiment, the incubation is performed at about 37°C.

As mentioned above, by loading immune-suppressive dendritic cells obtainable by the methods described herein with antigens allows producing immuno-suppressive antigen-presenting cells.

In order to avoid e.g. protein degradation of delivered antigen and inefficient processing of soluble antigens by dendritic cells, it is contemplated to enhance formation of immuno-suppressive antigen-presenting cells by encapsulation of antigens in polymeric nanoparticles (NPs), which may be made from biodegradable polymers such as polylactic acid (Waeckerle-Men et al., Adv Drug Deliv Rev (2005), 57:475-82). Such NPs may be further modified with targeting moieties for DEC-205 such as an DEC-205 antibody to improve receptor-mediated endocytosis and antigen presentation.

Thus the invention contemplates to use encapsulation of antigens in polymeric nanoparticles to optionally further enhance formation of immuno-suppressive antigen-presenting cells.

Inducing monocyte differentiation according to the method described above provides immuno-suppressive autologous and/or allogenic dendritic cells which equal or exceed the numbers of dendritic cells that are obtained by expensive and laborious culture of leukocytes in the presence of cytokines such as IL-10, IL4 or TGF-ß for a couple of days. The large numbers of functional dendritic cells generated by the method described above provide a ready means of presenting selected material, such as, for example, apoptotic cells, disease antigens, antigens, plasmids, DNA or a combination thereof, and are thereby conducive to efficient immunotherapy.

As mentioned above, immuno-suppressive autologous and/or allogenic dendritic cells can be preferably obtained by a method in accordance with the invention in the presence of a photoactivatable DNA-cross linking agent such as 8-MOP and by exposure to light such as UV-A.

The present invention thus aims at obtaining individual-specific functionally and maturationally synchronized immuno-suppressive autologous and/or allogenic dendritic cells.

In a second aspect, the present invention relates to immuno-suppressive autologous and/or allogenic dendritic cells obtainable by a method described herein, preferably for use in treating autoimmune diseases, hypersensitivity diseases, solid-organ transplantation rejection and Graft-versus-Host disease. The autoimmune disease may be selected from the group comprising rheumatoid arthritis, psoriasis, multiple sclerosis, type 1 diabetes and systemic lupus erythematosus.

In a third aspect, the present invention relates to a method of treating autoimmune diseases, hypersensitivity diseases, solid-organ transplantation rejection or Graft-versus-Host disease by administering to a patient in need thereof immuno-suppressive autologous and/or allogenic dendritic cells obtainable by a method described herein.

The invention is now described with respect to some specific examples which, however, are for illustrative purposes and not to be construed in a limiting manner.

### Experiments

### Experiment 1- Shear stress and platelet activation for inducing monocyte activation

### Materials and Methods

### Procurement of leukocytes and platelets

All samples were acquired from young, healthy subjects not taking medications, including aspirin, known to influence platelet function. Samples were obtained under the guidelines of the Yale Human Investigational Review Board, and informed consent was provided according to the Declaration of Helsinki. Peripheral blood specimens were collected through a 19-gauge needle from the antecubital vein into syringes containing heparin, then layered on Ficoll-Hypaque (Gallard-Schlessinger, Carle Place, N.Y.). Following centrifugation at 180g, the interface containing the mononuclear leukocyte fraction was collected and washed twice in HBSS, then resuspended in RPMI-1640 medium (GIBCO) to a final concentration of 5 x 10⁶ mononuclear cells/ml. Cells were utilized within one hour of being acquired.

### Preparation of Platelet-rich-Plasma

Whole blood was centrifuged at 150 g for 15 min at room temperature. The platelet-rich-plasma (PRP) layer was collected and centrifuged at 900 g for 5 min, and the platelet pellet resuspended in RPMI 1640 to the desired concentration.

### Preparation of Parallel-Plates

Two likes of parallel-plate flow chambers were used to model the flow dynamics of ECP. Experiments involving the assessment of cell phenotype post-flow were conducted using the larger Glycotech system (Glycotech, Rockville, MD). This system consisted of a volumetric flow path measuring 20000 x 10000 x 254 microns (length x width x height). The bottom plate in this system was composed of a 15mm petri dish (BD Biosciences, Durham, NC) separated by a gasket and vacuum-connected to an acrylic flow deck, which formed the upper plate. For experiments requiring the plates to be pre-coated with platelets, prior to assembling the flow chamber, 20 drops of the desired concentration of PRP was placed in the center of the petri dish and platelets allowed to settle for 20 minutes at room temperature. The petri dish was washed twice with 2ml of RPMI, and the flow chamber then assembled.

For experiments not involving the collection and phenotyping of cells post-flow, Vena8 biochips (Cellix Ltd, Dublin, Ireland) were used to generate laminar flow. The volumetric flow path for a channel of the Vena8 biochips measured 20000 x 400 x 100 microns (length x width x height). Protein coating of these chips is described in the appropriate section below.

### Experiments using Parallel-Plates

The parallel-plate flow chamber was mounted on the stage of a phase contrast optical microscope (CK40, Olympus, Japan) with a 10x objective. All runs were performed at room temperature. A uniform laminar flow field was simulated by use of a syringe pump (KD Scientific, New Hope, PA) capable of generating near-constant volumetric flow rates. The components of the configuration were devised to minimize tubing. Prior to infusing cell suspensions through the plates, the system was washed with 5 ml of RPMI at a flow rate producing a wall shear stress of approximately 1 dyne/cm². Cell suspensions of interest were then passed through the chamber at a fixed flow rate and wall shear stress.

All experiments were viewed in real time, recorded at 15.2 frames per second using a DP 200 digital camera and software (DeltaPix, Maalov, Denmark), and analyzed using Image J software (NIH).

### Overnight culture

When overnight culture was required, cells were centrifuged and resuspended in RPMI-1640 medium (GIBCO), supplemented with 15% AB serum (Gemini Bio-Products) to a final concentration of 5 x 106 cells/ml. Cells were cultured overnight for 18 hours in 12-well polystyrene tissue culture plates (2 ml per well) at 37° C in 5% CO2.

### Immunophenotyping

Monoclonal antibodies for immunophenotyping included CD 14 (LPS receptor; monocytes), CD11c (integrin subunit; monocytes and DC), HLA- DR (class II MHC molecule), CD83 (DC marker), CD62p (P-selectin; activated platelets), and CD61 (integrin subunit; platelets). Antibodies were obtained from Beckman Coulter (CD14, CD11c, HLADR, CD83) or Sigma (CD62p, CD61) and used at their pre-determined optimal dilutions. Background staining was established with appropriate isotype controls, and immunofluorescence was analyzed using a FC500 flow cytometer (Beckman Coulter). Two-color membrane staining was performed by adding the pre-determined optimal concentrations of both antibodies directly conjugated to FITC or PE and incubating for 20 min at 4°C, followed by washing to remove unbound antibodies. Combined membrane and cytoplasmic staining was performed following manufacturer's instructions for cell fixation and permeabilization (Intraprep kit, Beckman Coulter).

### Quantitative real-time PCR

Gene expression was compared between cells exposed during flow through the parallel plates to low (10 + 5/low power field [lpf]) versus high (102 + 32/lpf) levels of platelets, followed by overnight culture. Cell RNA was isolated using RNeasy Mini Kit columns with on-column DNase I treatment (QIAGEN). RNA yield and purity were measured using a NanoDrop ND-1000 Spectrophotometer and an Agilent 2100 Bioanalyzer. RNA was reverse transcribed to cDNA using the High Capacity cDNA Reverse Transcription Kit (Applied Biosystems). Reverse transcription was carried out in a 96-well thermocycler (MJ Research PTC-200) in the following conditions: 25°C, 10 minutes, 37°C, 120 minutes, 85°C, 5 seconds. TaqMan real-time PCR was used to detect transcripts of DC-LAMP, CD40, ADAM Decysin, Lox1, CCR7, CD80, CD83, CD86, FPRL2, and GPNMB. Primers and probes for each sequence were obtained as inventoried Taqman Gene Expression Assays (Applied Biosystems). HPRT1 was used as a reference gene.

### Co-cultures of Platelets with Monocytes

Experiments involving co-cultures of monocytes with additional platelets were performed as described in the Overnight Culture section, with a few necessary modifications. Following Ficoll-Hypaque separation, mononuclear cells were resuspended in 30% AB serum/RMPI to a final concentration of 10 x 106 cells/ml, of which 1 ml was allocated to each well of a 16- well plate. An additional 1ml of platelets (suspended in RPMI, at 2x the desired final concentration) or RPMI without platelets was then added to each well. To activate platelets, 500 µl containing 2 units of thrombin was added to half the wells, and 500 µl of RPMI was added to the others to balance the volume. Cells were then incubated as described previously.

### Platelet Adhesion Studies

Platelet adhesion experiments were performed using the Vena8 flow chamber described above. Fibrinogen and fibronectin (Sigma) were dissolved in PBS to a final concentration of 200 mcg/ml. Channels of the Vena8 chips were incubated at room temperature in a humidified chamber for 2 hours with the protein solution, autologous plasma, or PBS alone. The channels were washed with 5x the volume RPMI. Platelet-rich-plasma was then perfused through the protein-coated channel at the indicated shear-stress, held constant. For each channel, still images were acquired exactly 90 seconds into the experiment at 4 pre-defined low power fields located along the flow path (fields were centered at 2500, 7500, 12500, and 17500 microns from the start point of infusion).

Some experiments involved pre-treating platelet-rich-plasma with protein fragments prior to infusion through the channels. Small RGD peptides, containing the amino-acid sequence Arg-Gly-Asp-Ser; DRG peptides, contain the amino-acid sequence Ser-Asp- Gly-Arg; or fragment 400-411 of fibrinogen, containing the amino-acid sequence His- His-Leu-Gly-Gly-Ala-Lys-Gln-Ala-Gly-Asp-Val, were incubated at a concentration of 2mM with PRP for 20 minutes at room temperature. The PRP was then perfused through the channels as previously described.

### Receptor-Ligand Studies

Platelet-coated Vena8 channels were pre-treated with either 40 µg/ml anti-P-selectin (R&D Systems) or 40 µg/ml of an isotype control for 30 minutes at room temperature, then washed with 5x the volume RPMI. Mononuclear cell suspensions were pre-treated with either RGD or DGR peptides at a concentration of 2.5 mM. Video samples lasting 400 frames (26.3 seconds) were recorded 60 seconds after commencement of flow using a lower power field of view spanning 400 microns and centered at 7500 microns from the flow start point.

β-1 integrin conformation was assessed using the Glycotech flow chamber. 15mm platelet-coated petri dishes (described above) were pre-treated with 40 µg/ml anti-P-selectin or an isotype control for 20 minutes at room temperature, then washed with 5x the volume RPMI. Immediately following perfusion through the platelets, cells were immunophenotyped with anti-CD29 HUTS-21 (BD Biosciences), an antibody that specifically binds to the active (open) conformation of β1 integrins.

### Results

### Monocytes in flow transiently interact with immobilized platelets

ECP was initially developed as a means to enable extracorporeal chemotherapeutic exposure of pathogenic leukocytes to ultraviolet A (UVA)-activated 8-methoxypsoralen (8-MOP), a DNA-cross-linking drug. Therefore, ECP involves the flow of leukapheresed blood between large transparent plastic parallel-plates separated by 1 mm. To permit detailed analysis of the flow dynamics involved during ECP, independent of UVA/8-MOP exposure, the flow conditions of ECP were reproduced using miniature parallel plates with surface area of only 0.8 mm², separated by 100 microns. This model permitted visualization using digital microscopy. Studies using the model revealed the following sequence (determined by video analysis): initial adherence of platelets from the flow stream to the plate, followed by transient binding of passaged monocytes to the immobilized platelets.

### DC induction correlates with the number of monocyte-platelet interactions

Based on the initial qualitative observations described above, platelets were hypothesized to induce monocyte-to-DC differentiation under conditions of flow. To test the influence of platelets on monocyte-to-DC differentiation, monocytes were passed between parallel plates pre-coated with autologous platelets at low (10 + 5/low power field [lpf]), medium (44 + 20/lpf), and high (102 + 32/lpf) densities. Cells were passed through the plates at a flow rate producing a wall shear stress of 0.5 dyne/cm², analogous to the wall shear stress in post-capillary venules. The number of monocyte-platelet interactions per unit time increased in proportion to augmented density of platelets (determined by video analysis). An average of 52.3 + 15 monocyte-platelet interactions per lpf per second were observed with the high-density plate, dropping to 18.3 + 14 and 3.4±1 interactions per second with the medium and low-density plates, respectively (Figure 1a).

Following overnight incubation, a correlation was found between the percentage of cells which developed a DC phenotype and the frequency of monocyte-platelet physical interactions observed the previous day (Figure 1b). An increasing number of monocyte-platelet interactions correlated with increasing proportion of cells expressing markers consistent with DC differentiation, membrane HLA-DR and CD83. An average of 14.2% of monocytes exposed to the high-density platelet-coated plate were HLA-DR+/ CD83+ after overnight incubation, compared to 4.9% and 0.8% of monocytes exposed to plates coated with medium and low levels of platelets, respectively.

### Monocyte exposure to platelets results in changes in gene expression

To supplement the described changes in monocyte phenotype observed following platelet exposure, RT-PCR was performed to assess for changes in gene expression. Monocytes were passed through parallel plates coated with high or low densities of platelets as described in the previous section. Following overnight incubation, RNA was extracted and RT-PCR performed to determine level of expression for 10 genes associated with DC (Figure 2). CD40, a costimulatory molecule with known expression on mature dendritic cells (Cella et al., 1996, see reference list), was found to be upregulated by over 567% in monocytes exposed to high densities of platelets relative to monocytes exposed to low levels. LAMP3, a marker specific to DC differentiation (de Saint-Vis at al., 1998, see reference list), was upregulated by 398%. CD80 is a costimulatory molecule known to be upregulated upon APC activation (Slavik et al., 1999, see reference list) upregulated by 220% in monocytes exposed to high levels of platelets. CCR7, a chemokine receptor known to play a role in DC migration to lymphoid organs, was upregulated by 376%. LOX1, CD83, CCR7, and ADAM Decysin, all genes associated with DC (Berger et al., 2010, see reference list), were also upregulated in the monocytes exposed to high levels of platelets. FPRL2, GPNMB, and CD86 were all downregulated in monocytes exposed to high levels of platelets. FPRL2 is a receptor that when activated is known to inhibit DC maturation (Kang et al., 2005, see reference list) GPNMB is a protein involved in decreasing cytokine production (Ripoll et al., 2007, see reference list); CD86 is a costimulatory molecule expressed by APCs.

### DC induction in the presence of platelets does not occur under static conditions

Platelets could potentially influence monocytes through direct receptor-ligand interaction, or via cytokines and other secreted mediators. To determine whether the platelet induction of monocyte-to-DC differentiation requires flow dynamics, we tested the role of platelets under static conditions. Monocytes were co-cultured with low (<50,000/mm³), medium (100-200,000/mm³) and high (>400,000/mm³) concentrations of platelets, with platelets in either an inactive or active state (induced by the addition of thrombin). After overnight incubation in static conditions (shear stress = 0), we found that neither activated nor non-activated platelets were capable of inducting DC differentiation of monocytes in the absence of flow (see Figure 3).

### Platelets suspended in flow bind to serum proteins adsorbed onto the plate

Several proteins abundantly present in plasma, including fibronectin and fibrinogen, are well known adsorb onto glass and plastic surfaces; the contribution of adherent plasma proteins on platelet adhesion and activation was therefore assessed. Parallel plates were pre-coated with either fibrinogen, fibronectin, plasma, or saline. Unactivated platelets were then passed through at shear rates producing wall shear stresses ranging from 0.2 to 6.0 dyne/cm². The highest concentrations of platelets adhered to plates coated with fibrinogen (Figure 4). Adhesion to fibronectin-coated, plasma-coated, and uncoated plates was observed as well, but to a significantly lower extent (p < 0.05). In the absence of flow, platelet adherence was equivalent on all protein substrates.

Both fibrinogen and fibronectin contain segments with the amino acid sequence arginine(R)-gylcine(G)-aspartate(D), RGD. RGD segments are well-known to interact with many integrin receptors, particularly the I/A domain of beta subunits, which are exposed when the integrins are in the active conformation (Xiong et al., 2002, see references). In experiments using fibrinogen-coated plates, platelet adhesion was not significantly altered by pre- incubation of platelets with RGD peptides; however, adhesion was significantly decreased (p < 0.05) by pre-incubation of platelets with peptide fragments corresponding to amino acids 400-411 of fibrinogen, the gamma component of the protein (Figure 5 a). In experiments using fibronectin-coated plates, pre-incubating platelets with RGD peptides decreased adhesion significantly, while pre-incubating platelets with peptide fragments corresponding to amino acids 400-411 of fibrinogen had no effect, (Figure 5b). Interestingly, it should be noted that unlike the I/A domain of integrins, which is known to interact with RGD domains of proteins, the region of the integrin found to interact with the gamma component of fibrinogen is exposed in the integrin's inactive state (Weisel et al., 1992, see references). Therefore, this data suggests that unactivated platelets in flow bind to the gamma-component of fibrinogen-coated plates. The potential for platelets in the unactivated state to bind fibrinogen may explain the greater level of platelet adhesion seen on fibrinogen-coated plates explained in the previous paragraph.

### Platelets are activated by adhesion to the plate

Platelets physiologically circulate in an inactive state, with an array of proteins stored in intracellular granules. Upon encountering stimuli such as damaged endothelium or thrombin, platelets become activated and almost instantaneously translocate these intracellular proteins to the plasma membrane (Kaplan et al., 1979, see references). It was postulated that platelet adhesion to the plastic plate/absorbed proteins caused platelet activation similar to that caused by well-known stimuli. To test this hypothesis, surface expression of P-selectin, a well-known marker of platelet activation, was assessed before and after adhesion. Prior to adhesion, 6 + 3% of platelets were found to express P-selectin, with a mean fluorescence intensity (MFI) of 12.4 + 6.9; following adhesion, P-selectin positivity increased to 64 + 13% (MFI 98.2 + 14). The positive control, platelets activated with thrombin, was 71 + 18 % P-selectin positive (MFI 108.3 + 23). Expression of P-selectin was further assessed at 30, 60, and 90 minutes following platelet adhesion; P-selectin expression remained stable at all time points, with 72 + 11% of platelets P-selectin positive 90 minutes after adhesion, indicating that platelets remain in an active state for the duration of the procedure. Similar trends were found in assessment of αIIb-β3, a fibrinogen-binding integrin, with surface expression of this protein increasing from 4 + 3% prior to adhesion, to 49 + 18% post-adhesion.

### Monocytes interact with P-selectin and RGD-containing ligands expressed on activated platelets

The monocyte-platelet interactions observed on video were divided into two categories: (1) short-acting, arbitrarily defined as contact occurring for less than 3 seconds (46 frames), and (2) long-acting, defined as contact longer than 3 seconds, including stable binding. Since it had been previously determined that the platelets in the ECP system were in an activated state, and that activated platelets express an array of proteins including P- selectin and RGD containing proteins (e.g. fibronectin, fibrinogen, and vitronectin), it was sought to determine the involvement, if any, of these proteins in either short or long- duration interactions. Plates were pre-coated with platelets, and four conditions tested: (1) platelets pre-treated with an irrelevant isotype control, and monocytes untreated (P+RGD+); (2) platelets pre-treated with an irrelevant isotype control, and monocytes pre-incubated with RGD peptides (P+RGD-); (3) platelets pre-treated with anti-P-selectin, and monocytes untreated (P-RGD+); (4) platelets pre-treated with anti- P-selectin, and monocytes pre-treated with RGD peptides (P-RGD-). It was assumed that pre-treating monocytes with RGD peptides should result in a decreased in the number of free RGD- recognizing receptors available to interact with RGD-containing proteins expressed by the platelets. Thus, the four conditions tested represent every permutation of potential interaction with two platelet ligands, P-selectin and RGD-containing-proteins. As shown by Figure 6, both short-acting and long-acting interactions were maximal when neither RGD nor P-selectin were blocked (P+RGD+); the level of interaction in all other conditions was expressed as a percentage of this maximum. Blocking with anti-P-selectin alone (P-RGD+) resulted in a decrease of both short and long monocyte- platelet interactions to almost zero (p < 0.01; Figure 6, also confirmed by video analysis). In contrast, blocking RGD alone (P+RGD-) did not significantly alter the number of short-duration interactions, but decreased the long-duration monocyte-platelet interactions by 44% (p < 0.05; Figure 6). Blocking both P-selectin and RGD simultaneously (P-RGD-) resulted in a pattern similar to that seen when only P-selectin was blocked, with both long and short duration interactions reduced to near zero. The most appropriate conclusions, based on the pattern of interactions observed in each of the four conditions, are as follows: (1) P-selectin is predominantly responsible for the short-duration interactions; (2) RGD-containing proteins expressed by the platelet are involved in long-duration interactions, but not short-duration interactions; (3) monocyte interaction with P-selectin must occur upstream of monocyte interaction with RGD-containing proteins expressed by platelets. This last conclusion is based on the observation that conditions of P-RGD+ decreased both short and long duration interactions to near zero, while P+RGD-conditions only decreased long-duration interactions. If the interactions were not sequential, conditions of P- RGD+ should have produced similar results to P+RGD+ in terms of long-duration interactions. Furthermore, the ordering of the interactions, i.e. that P-selectin acts upstream of RGD-interactions, is apparent by the finding that conditions of P+RGD- only influenced long duration interactions, while conditions of P-RGD+ produced similar results to those of P-RGD-.

### Monocyte exposure to P-selectin results in downstream monocyte integrin-activation

Integrin receptors, in their open conformation, are known to interact with RGD-containing ligands (Ruoslathi et al., 1996, see references). Using an antibody that recognizes an epitope exposed only when the β1 integrin is in its open conformation, we assessed the conformation of monocyte integrins before and after flow through the model. Figure 7 shows that as the number of short-acting monocyte-platelet interactions increased, there was corresponding increase in the percentage of monocytes expressing integrins in their open conformation immediately post-flow. The black line shows that an average of 71% of monocytes which had received a high number of platelet-interactions (> 61+ 19/lpf x sec) expressed β1 in the active form, compared to 9% of monocytes which had received a low number of platelet interactions (<5.1+2/lpf x sec). These results were not significantly affected by pre-treating the adherent platelets with an irrelevant isotype control (gray line). In contrast, pre-treating platelets with anti-P-selectin reduced the monocyte-platelet interactions to near zero, and monocytes emerging from flow in these conditions (dashed line) displayed low levels of active β1 integrins, irrespective of the density of platelets to which they were exposed. It is noteworthy that all cell populations prior to passage through the plates demonstrated similar low levels of baseline integrin activation (<10%); therefore, differences seen in short-duration monocyte-platelet interactions were not the result of differences in integrin conformation preflow.

### Monocyte exposure to P-selectin is required for DC differentiation

Given the dependence of monocyte-platelet interactions on platelet P-selectin, we set out to determine if there was a relationship between monocyte exposure to P-selectin at time 0, and the phenotype later developed by the monocyte after overnight incubation, time 18-hours (Figure 8). Monocytes were passed though parallel plates coated with high densities (108 + 36/lpf) of platelets that were either untreated (unblocked), or pretreated with either anti-P-selectin or an isotype control. 15.5 + 4 % of monocytes exposed to unblocked platelets became membrane HLA-DR+/CD83+ (markers of maturing DC) after overnight incubation, and 13 + 4 % of the those exposed to platelets blocked with the irrelevant isotype control. In contrast, only 3 + 2% of the monocytes exposed to platelets blocked with anti-P-selectin became HLA-DR+/CD83+ after overnight incubation.

### Experiment 2 - Identification of further molecular markers for immuno-suppressive dendritic cells

### Materials and Methods

### Sample Collection and Monocyte Enrichment

Peripheral blood specimens were acquired from healthy subjects under the guidelines of the Yale Human Investigational Review Board, and informed consent was provided according to the Declaration of Helsinki. PBMC were isolated by centrifugation over a Ficoll-Hypaque gradient (Isolymph, CTL Scientific). Monocytes were enriched from freshly isolated PBMC by: 1) plastic adherence for dexamethasone dose-titration experiments (purity: 71.6 ± 5.6% CD14⁺); 2) CD14 magnetic bead positive selection (Miltenyi Biotec) for PUVA dose-titration experiments (purity: 88.1 ± 3.5% CD14⁺), and; 3) Monocyte Isolation Kit II (Miltenyi Biotec) for LPS stimulation experiments (purity: 83.8 ± 3.8% CD14⁺).

### Generation of Monocyte-Derived Dendritic cells (MoDC)

Monocytes were cultured at a density of 5 x 10⁶ cells/mL in 6- and 12-well polystyrene tissue culture plates at 37°C and 5% CO₂ in RPMI-1640 (Gibco) supplemented with heat-inactivated 15% AB serum (Gemini) and 1% penicillin/streptomycin (now referred to as complete media). 800 IU/mL recombinant human GM-CSF (R&D Systems) and 1000 IU/mL recombinant human IL-4 (R&D Systems) were added to cultures for 36 hr to induce monocyte to DC differentiation as described.

### 8-MOP and UVA Light Treatment

Cultures were incubated with 8-MOP (Uvadex, 20 µg/mL) for 30 min in the dark, and then irradiated with a desktop UVA light box containing a series of 12 linear fluorescent tubes. The tubes emitted UVA light ranging from 320 to 400 nm. The UVA irradiance (power, W/m²) was measured using a photodiode. Given a measured irradiance and the absorption properties of the various components of the system, it was possible to determine the time (sec) needed to expose the cells to deliver a given dose of UVA radiation (J/cm²).

### MoDC/Lymphocyte Co-Cultures

Non-adherent cells (purity: 66.0 ± 4.5% CD3⁺) removed during plastic adherence will now be generally referred to as lymphocytes. Lymphocytes were treated with 8-MOP (100 ng/mL) and UVA (1 J/cm²), washed with PBS, and co-cultured in complete media at 37°C and 5% CO₂ with either PUVA-treated or untreated-MoDC in a ratio of 5 or 10 lymphocytes to 1 MoDC. MoDC treated for 24 hr with 100 nM dexamethasone (Sigma) served as the positive control group. After 24 hr, cells were harvested and MoDC were re-purified. To ensure that RNA was not isolated in significant amounts from lymphocytes, it was critical to re-purify MoDC from all cultures using CD11c magnetic bead (Miltenyi Biotec) positive selection (purity: 96.4 ± 1.0% CD11c⁺). CD11c⁺ MoDC were re-plated at 0.5-1.0 x 10⁶ cells/mL in complete media and stimulated with 100 ng/mL LPS (Sigma). 24 hr after LPS stimulation, cells were harvested for RNA isolation and immunophenotyping, and supernatants were collected for cytokine quantification. As negative controls, parallel groups did not receive LPS.

### siRNA Experiments

Silencer select pre-designed and validated GILZ siRNA (Invitrogen), with off-target prediction algorithms, was used to knockdown GILZ expression. Mo-dendritic cells were transfected using Lipofectamine RNAiMAX Reagent (Invitrogen). RNAᵢ duplex and lipofectamine reagent were incubated together for 20 min, then added to MoDC cultures and incubated for 2 hr at 37°C and 5% CO₂.Transfected MoDC were treated in an identical fashion as described for the MoDC/lymphocyte co-cultures. MoDC were also transfected with scramble siRNA.

### Immunophenotyping

Monoclonal antibodies included HLA-DR, CD80, CD83, CD3, CD86, CD14, CD11c and GILZ. Antibodies were obtained from Beckman-Coulter and eBioscience and were used at their pre-determined optimal dilutions. Apoptosis was assessed using the Annexin-V Apoptosis Detection Kit (eBioscience), with Annexin-V recognizing phosphatidylserine (PS) on the surface of apoptotic cells. 7-AAD substituted for PI as the cell viability dye. Cells displaying an Annexin-V⁺/7-AAD⁻phenotype were classified as early apoptotic cells, and cells displaying an Annexin-V⁺/7-AAD⁺ phenotype were classified as late apoptotic cells. Dual membrane and intracytoplasmic staining was performed using the IntraPrep fix and permeabilization kit (Beckman-Coulter). Background staining was established with appropriate isotype and fluorescence minus one controls. Immunofluorescence was analyzed using a FACSCalibur L (BD Biosciences) within 2 hr of fixation with 2% paraformaldehyde. A minimum of 10,000 events were collected for each group.

### Quantitative Real-Time PCR

RNA was isolated from CD1 1c⁺MoDC using QIAShredder columns (QIAGEN) and the RNeasy Mini Kit (QIAGEN) with on-column DNase I treatment (QIAGEN). RNA yield and purity were assessed using a NanoDrop ND-1000 spectrophotometer. cDNA was obtained using the High Capacity cDNA Reverse Transcription Kit (Applied Biosystems) in a 96-well thermocycler (MJ Research PTC-200). TaqMan real-time PCR was used to detect transcripts of GILZ, CD80, and CD86. Primers and probes were obtained as pre-designed and validated Taqman Gene Expression Assays (Applied Biosystems). SYBR green real-time PCR (Applied Biosystems) was used to detect transcripts of IL-12, IL-10, IL-6, TNF-alpha, and TGF-β. Primers were designed to span intron junctions using Primer3Plus. Primer melting curves were obtained to confirm a single product. HPRT-1 and GAPDH were used as reference genes. Samples were run in triplicate on a 7500 Real Time PCR System (Applied Biosystems). The delta-delta C(t) method was used to calculate the fold change.

### Cytokine Quantification

Culture supernatants were analyzed in a multiplex format utilizing magnetic beads to IL-6, IL-8, IL-10, IL-12p70, IFN-γ, TNF-α, RANTES, MCP-1, and MIP-1β (BioRad Laboratories). For siRNA experiments, supernatants were analyzed with enzyme-linked immunosorbent assay (ELISA) kits for IL-10 (R&D Systems) and IL-12p70 (Enzo Life Science). All samples and standards were run in duplicate and analyzed using the LUMINEX 200 (LUMINEX), or the BioTek EL800 (BioTek).

### Statistical Analysis

Student's *t*-tests were used for statistical comparisons between groups, with *p*-values < 0.05 considered statistically significant. Differential gene expression was considered statistically significant with a ≥ 2.5-fold change and a *p*-value < 0.05.

### Results

### Expression of GILZ is rapidly down-regulated as monocytes differentiate into immature MoDC

Freshly isolated CD14⁺ monocytes express GILZ, but rapidly down-regulate GILZ by more than 99% as they differentiate into immature MoDC (Figure 10A). A reduction in GILZ mRNA was confirmed by a 61% decrease in GILZ protein levels (Figure 10B). GILZ down-regulation correlated with reduced expression of CD14 (monocyte-specific marker, see Zhou et al., references), and increased expression of cytoplasmic CD83, (immature MoDC marker, see Klein et al., references). Importantly, MoDC remained immature, expressing low membrane CD83 (mature DC marker, see Renzo et al., references, *p* = 0.16). MoDC up-regulate GILZ after treatment with dexamethasone (dex) in a dose-dependent manner (Figure 10C). Treatment with 100 nM dex for 24 hr was selected as the positive control for inducing GILZ expression in MoDC (Dex-dendritic cells) (Figure 10D).

8-MOP or UVA treatment alone did not effect GILZ expression (Figure 10E). However, when MoDC were treated with the combination of 8-MOP and UVA light (PUVA-dendritic cells), GILZ expression increased 5.5-fold. The induction of GILZ exhibited a slow time course, peaking 24 hr after treatment, and remaining significantly elevated for 72 hr (Figure 10F). In comparison, Dex-dendritic cells up-regulated GILZ as little as 2 hr after treatment.

### Immature MoDC treated with the combination of 8-MOP and UVA light up-regulate GILZ and assume a tolerogenic, immuno-suppressive phenotype

It was next examined if there was a PUVA dose-dependent effect on GILZ expression. MoDC treated with 1 J/cm² UVA and 100 or 200 ng/mL 8-MOP up-regulated GILZ 2.9- and 4.4-fold respectively (Figure 11A). A similar dose-dependent phenomenon was observed with 2 J/cm², starting at an 8-MOP concentration of 50 ng/mL. Treatment with 0.5 J/cm² had no effect on GILZ expression until the 8-MOP concentration reached 200 ng/mL, and treatment with 4 J/cm² resulted in high levels of non-specific cell death (data not shown). The number of photo-adducts formed per 10⁶ base pairs is directly related to the product of the 8-MOP concentration and UVA dose, see Gasparro et al., references. As the product of 8-MOP and UVA reached 100, GILZ was up-regulated 3-fold, and as the product increased to 200 and 400, GILZ was up-regulated 4.8- and 8.6-fold respectively (Figure 11B).

The percentage of early apoptotic CD11c⁺ cells was minimally (*p* > 0.05) higher at 2 J/cm² as compared to 1 J/cm² for all doses of 8-MOP tested (Figure 11C). At 2 J/cm² and 200 ng/mL, there was an increase in the percentage of early apoptotic CD11c⁺ cells as compared to untreated MoDC (Figure 11C). The percentage of late apoptotic CD11c⁺ cells remained less than 13% at 1 J/cm², and less than 16% at 2 J/cm² for all doses of 8-MOP tested (Figure 11D). Moreover, dot plots highlight the relative resistance of MoDC to the pro-apoptotic effect of escalating doses of PUVA (Figure 11E). The number of cells recovered from cultures did not statistically differ in any group treated with 1 or 2 J/cm² (data not shown), and greater than 90% CD11c⁺ cells (range 91.0-97.5%) were harvested after treatment.

In contrast, lymphocytes display Annexin-V as early as 2 hr after treatment with 1 J/cm² and 100 ng/mL (data not shown). In contrast to MoDC treated with 100 ng/mL and 1 J/cm² (Figure 11F), 24 hr after treatment with the same dose of PUVA, the percentage of early apoptotic lymphocytes increased from 6.6% in untreated MoDC to 44.3% in PUVA-dendritic cells, and the percentage of late apoptotic lymphocytes increased from 4.5% to 33.7% (Figure 11G). Given that 64.3 ± 3.2% of lymphocytes were Annexin-V⁺ 24 hr after treatment, PUVA-treated lymphocytes are subsequently referred to as apoptotic lymphocytes (ApoL).

The PUVA dose-dependent induction of GILZ correlated with a decrease in cell surface expression of CD80, CD86, and CD83 (Figure 12A, 3B). Down-regulation of these markers paralleled the induction of GILZ (see Figure 11B), beginning at 8-MOP concentrations of 100 ng/mL for both 1 and 2 J/cm². As the product of 8-MOP and UVA exceeded 100, CD83, CD80 and CD86 expression were reduced by 31%, 30% and 54% respectively, and HLA-DR expression increased by 38%.

### MoDC exposed to apoptotic lymphocytes up-regulate GILZ and are resistant to LPS-induced full maturation

To dissect the individual contributions of PUVA and exposure to apoptotic cells, MoDC were first co-cultured with varying ratios of ApoL. GILZ was up-regulated in an ApoL dose-dependent fashion (Figure 13A). When PUVA-dendritic cells were exposed to ApoL, GILZ was expressed at higher levels than in PUVA-dendritic cells cultured alone (Figure 13B). PUVA-dendritic cells exposed to ApoL also expressed GILZ at higher levels than in untreated MoDC exposed to ApoL (6.7-fold and 3.6-fold higher, respectively). There was a corresponding 1.5-fold increase in the GILZ protein level in all groups in which GILZ mRNA was up-regulated (Figure 13C). Induction of GILZ was not related to an increase in the number of early or late apoptotic CD11c⁺ cells, as there were < 12% early apoptotic (range 3.8-11.4%) and late apoptotic (range 6.3-11.5%) CD11c⁺ cells in all groups demonstrating up-regulation of GILZ.

MoDC expressing GILZ greater than 2.5-fold above untreated MoDC were resistant to full maturation by LPS and exhibited a semi-mature, tolerogenic phenotype. LPS stimulation increased CD80 expression in MoDC up-regulating GILZ to only 50% of the levels seen after LPS stimulation in untreated MoDC (Figure 13D, range 0.48-0.57%), and increased CD86 expression to only 45% of untreated MoDC (Figure 13D, range 0.42-0.47%). Similar results were obtained for HLA-DR and CD83 (Figure 14E, range 47-65% and 23-57% of untreated MoDC after LPS respectively). In addition, MoDC up-regulating GILZ expressed 6% of the CD80 mRNA of untreated MoDC (range 4.5-7.5%), and expressed 50% of the CD86 mRNA of untreated MoDC (range 12.4-85.1%), as assessed by qRT-PCR.

### MoDC expressing GILZ display a tolerogenic cytokine profile, and knockdown of GILZ reduces the IL-10 to IL-12p70 ratio

Supernatants were harvested from co-cultures as described in Figure 13B. Dex-dendritic cells up-regulated GILZ 4.29-fold (see Figure 13B), increased production of IL-10 (Figure 14A), and decreased production of all pro-inflammatory cytokines (Figure 14B, 14C) and chemokines (Figure 14D, 14E) tested. In comparison, PUVA-dendritic cells up-regulated GILZ 2.78-fold (see Figure 13B), increased production of IL-10, and decreased production of all pro-inflammatory cytokines and chemokines tested, except TNF-α and IFN-γ. PUVA-dendritic cells or untreated MoDC, exposed to ApoL expressed GILZ at higher levels that PUVA-dendritic cells cultured alone (3.6- and 6.7-fold higher, respectively; see Figure 13B). These two groups increased production of IL-10, and decreased production of all pro-inflammatory cytokines and chemokines tested. Cytokine levels were confirmed at the RNA level, with MoDC that up-regulated GILZ also demonstrating up-regulation of IL-10 mRNA 8-fold above untreated MoDC (range 5.5-11.8, *p* < 0.01). Reductions in IL-12, TNF-α, and IL-6 were also confirmed at the RNA level (data not shown). TGF-β was up-regulated 2.5-fold in MoDC up-regulating GILZ (data not shown). TGF-β was not included in the multiplex analysis and therefore was only analyzed at the mRNA level.

The IL-10 to IL-12p70 ratio is a useful indicator of tolerogenicity, since tolerogenic dendritic cells are characterized by an increased IL-10 to IL-12p70 ratio, see Steinman et al., references). The ratio of IL-10 to IL-12p70 increased from 6.7 in untreated MoDC to 67.7 in Dex-dendritic cells. Similarly, the IL-10 to IL-12p70 ratio increased to 38.7 in PUVA-dendritic cells, and to 89.4 and 114.9 in untreated MoDC and PUVA-dendritic cells exposed to ApoL, respectively (p < 0.05).

To assess whether induction of GILZ was mediating the tolerogenic cytokine profile, MoDC were transfected with siRNA to knockdown GILZ expression. Transfection with GILZ siRNA reduced GILZ expression in Mo-dendritic cells by 68% (Figure 15A, range 59-79%). Transfection with scramble siRNA did not significantly change GILZ expression. There was also no significant difference in the number of cells recovered from any groups transfected with siRNA as compared to non-transfected groups (data not shown).

Treated MoDC up-regulating GILZ 2.5-fold higher than untreated MoDC produced higher levels of IL-10 (Figure 15B), and knockdown of GILZ reduced IL-10 production by 39% (range 34-48%, *p* < 0.05). Treated MoDC up-regulating GILZ 2.5-fold higher than untreated MoDC also produced lower amounts of IL-12p70 (Figure 15C), and knockdown of GILZ increased IL-12p70 production by 188% (range 149-214%, *p* < 0.05). Treatment with scramble siRNA had no appreciable effect on the production of IL-10 or IL-12p70. Knockdown of GILZ reduced the IL-10 to IL-12p70 ratio that had been elevated after GILZ induction. Dex-dendritic cells treated with GILZ siRNA demonstrated a reduction in the IL-10 to IL-12p70 ratio from 15.3 in non-transfected MoDC to 3.9 in transfected Dex-dendritic cells. In PUVA-dendritic cells the ratio decreased from 8.4 in non-transfected MoDC to 2.9 in PUVA-dendritic cells, and in untreated MoDC and PUVA-dendritic cells exposed to ApoL, reductions in the ratio from 18.1 to 7.8 and 28.4 to 8.3, respectively, were observed.

These results demonstrates that like other immunosuppressive mediators, PUVA induces the expression of GILZ and generates tolerogenic immuno-suppressive dendritic cells, characterized by low expression of the co-stimulatory molecules CD80 and CD86, and the maturation marker CD83. GILZ induction is necessary for the polarization towards a tolerogenic cytokine profile, characterized by increased IL-10 production, and decreased pro-inflammatory cytokine and chemokine production, including IL-12p70. These results further implicate GILZ as the molecular switch mediating the immunosuppressive effects of apoptotic cells.

### Experiment 3 - Identification of further molecular markers for immuno-stimulatory dendritic cells

### Patient samples

Leukocytes from patients undergoing ECP using the UVAR XTS Photopheresis System (Therakos) were obtained under the guidelines of the Yale Human Investigational Review Board. Informed consent was provided according to the Declaration of Helsinki. Aliquots were procured at 3 time points: before treatment (Pre ECP), immediately after 8-MOP/ultraviolet A (UVA) exposure (ECP Day 0) or after 18-hour incubation of treated blood mononuclear leukocytes (ECP Day 1) in a 1-L platelet storage bag (PL-2410; Baxter).

### Normal subjects

To determine whether ECP induces monocytes from healthy subjects to convert to DC, mononuclear leukocytes from normal subjects were examined in 2 ways. Leukapheresed leukocytes from normal subjects (N = 3) were studied pretreatment (pre-ECP), immediately after ECP (ECP Day 0), and 18 hours after ECP (ECP Day 1). A desktop apparatus, incorporating a UVA light source and a plastic exposure plate, enabled laboratory reproduction of the clinical ECP system and sample access for parallel RNA isolation, immunophenotyping, and functional studies. Alternatively, a unit of blood from normal subjects was drawn into a transfer bag and passed through the ECP treatment apparatus in an identical fashion to that of treated patients (N = 3). The cells obtained from the unit of normal blood were used for microarrays and antigen presentation assays.

### Psoralen addition

As is routinely done during ECP, the standard 8-MOP concentrated solution (Therakos) was added directly to the clinical ECP apparatus and to the laboratory model system. That mode of introduction enabled precise 100-200 ng/mL concentrations throughout the clinical procedures and experimentation.

### Overnight culture

In ECP, it is not possible to examine phenotypic and functional changes in treated monocytes, because those cells are immediately reinfused into patients. Therefore, after ECP, cells were cultured for 18 hours (RPMI 1640/15% autologous serum) to study induced monocyte gene activation, maturation and function. Prior to (pre-ECP) and immediately after ECP (ECP Day 0), patient and normal subject samples were isolated by centrifugation over a Ficoll-Hypaque gradient. The cells were resuspended in RPMI-1640 medium (Gibco), supplemented with 7.5% AB serum, 7.5% autologous serum (Gemini Bio-Products) and cultured (for patients) in 6-well polystyrene tissue-culture plates at a density of 5*10⁶ cells/mL and in Baxter platelet storage bags (for normal subjects 37°C, 5% CO2). After overnight culture (ECP Day 1), cells were harvested before undergoing monocyte enrichment. To generate DC for comparative phenotypic analysis, cells were cultured in RPMI 1640 15% serum in the presence of 1 mL of GMCSF and IL4 (25 ng/mL; R&D Systems) for 6 days.

### Magnetic bead enrichment of the monocyte population

To enable determination of whether ECP activates genes directing monocytes into the dendritic cell maturational pathway, it was necessary to develop a gentle negative monocyte enrichment method that eliminates contribution of lymphocytes to the transcriptome analysis while minimizing monocyte physical or cell membrane perturbation. Monocytes were enriched from the mononuclear cell pool by single passage through affinity columns. This negative selection method limited physical perturbation, whereas lymphocytes adherent to magnetic microbeads (Miltenyi Biotec), conjugated to relevant monoclonal antibodies (anti-CD4, CD8, CD19), were depleted. However, enrichment of ECP Day 1 monocytes beyond 60%-80% proved challenging, because diminished surface display of lymphocyte markers by ECP-damaged lymphocytes permitted a fraction of T and B cells to escape retention in the columns. Repetitive passes through the affinity column, to further enhance monocyte purity, was not an option because that approach compounds the physical perturbation of passively filtered monocytes. Fortuitously, a series of analyses revealed that ECP's preferential damage of lymphocytes precluded the necessity of full purification of monocytes for accurate assessment of level of DC gene activation. Due to their extreme sensitivity to UVA-activated 8-MOP, 99% of ECP-processed lymphocytes were apoptotic after overnight incubation (as determined by staining with APO2-PE, Trypan blue, and/or annexin-fluorescein isothiocynate FITC/ propidium idodide). Because ECP causes global lymphocyte apoptosis, 90%-95% of viable mononuclear leukocytes in the ECP day 1 fraction were monocytes. This phenomenon accounts for the observation that multiple step magnetic bead removal of apoptotic lymphocytes, performed as follows and yielding monocyte purity of greater than 95%, does not alter levels of observed gene expression in the studied cell populations. To accomplish that comparison we modified the monocyte purification procedure by adapting a negative selection protocol using magnetic beads and the EasySep magnet. Peripheral blood mononuclear cells were centrifuged at low speed (120g for 10 minutes) to remove platelets. Cells were then labeled using the Monocyte Isolation Kit II (Miltenyi Biotec) following the manufacturers procedure with the follow- ing modifications: (1) buffer consisted of ice-cold phosphate-buffered saline containing 2% autologous serum and ImM EDTA (ethylenediaminetetraacetic acid); (2) blocking time was increased to 10 minutes; (3) labeling with the Biotin-Antibody Cocktail was increased to 20 minutes; and (4) cells were washed once between labeling with the Biotin-Antibody Cocktail and the Anti-Biotin Microbeads. To avoid stimulating the monocytes by passing them over a column, the magnetically labeled cells were instead separated from the unlabeled monocytes using the EasySep magnet (StemCell Technologies). Cells, in 2 mL of buffer in a 5-mL polystyrene tube, were placed in the magnet for 10 minutes, and then the unlabeled cells were carefully poured off into a new tube. This procedure was repeated 2x, to maximally enhance monocyte purity. At this point, because the purity was still insufficient, cells were relabeled with the Monocyte Isolation Kit II reagents and placed in the EasySep magnet for an additional 10 minutes, and the unlabeled monocytes were eluted. Final purity (X=96%+4.5) was assessed by flow cytometric analysis of CD14 staining.

### Immunophenotyping

Monoclonal antibodies specific for monocytes and dendritic cells, included: CD14 (lipopolysaccharide [LPS] receptor, monocytes); CD36 (receptor for apoptotic cells, monocytes); human leukocyte antigen DR-1 (HLA-DR; class II major histocompatibility complex [MHC] molecule); CD83 (dendritic cell marker); cytoplasmic dendritic cell-lysosome-associated membrane protein (DC-LAMP; dendritic cell marker); and CD80 and CD86 (B7.1 and B7.2 costimulatory molecules). Antibodies were obtained from Beckman Coulter and used at their predetermined optimal dilutions. Background staining was established with appropriate isotype controls, and immunofluorescence was analyzed using a FC500 flow cytometer (Beckman Coulter). Combined membrane and cytoplasmic staining was performed following manufacturer's instruc- tions for cell fixation and permeabilization (Intraprep kit; Beckman Coulter).

### Antigen presentation assay

Volunteer freshly isolated, magnetic bead-enriched, antigen-experienced CD4⁺ populations (2*10⁶/mL, 50 µL/well) were added to monocytes (2*10⁶/mL, 50 µL/well) in the presence of tetanus toxoid (10 µg/mL, 100 µL/well) and RPMI medium 1640/15% autologous serum. After 5 days of culture, the cells received 1 µCi of [³H]-thymidine and were incubated overnight, harvested, and counted in a Beta liquid scintillation counter (PerkinElmer). Results are presented as the mean and standard deviation of 5 replicate cultures.

### MLR/CML assay

To assess whether ECP-processed monocytes are functionally capable of stimulating MHC class I-restricted cytotoxicity by CD8 T cells, mononuclear leukocytes from 3 normal subjects were studied. One unit of anti-coagulated blood, freshly procured from each of 3 HLA-A2-positive volunteers, served as sources of stimulator monocyte/dendritic cells, before and after being processed through the clinical ECP apparatus in a manner identical to the actual ECP procedure. Mononuclear fractions were isolated from the blood immediately prior to ECP processing (pre-ECP) and immediately after ECP (ECP D0). After gamma irradiation (3000 rad, Cesium source) to ensure unidirectional T-cell stimulation, the Pre ECP fraction was serially diluted in RPMI 1640/15% autologous serum, and 100 µL containing from 25 000 to 250 cells was plated in round-bottom microtiter plate wells, in 5 replicates. The ECP D0 fraction was incubated for 18 hours in large well plates and harvested by scraping the wells to free adherent cells. The re-suspended cells were then serially diluted and plated as above. An A-2-negative normal donor served as the source of responder CD4 and CD8 T cells, purified by positive selection on Miltenyi magnetic bead columns (average purity 98%). Responder T cells (50 000/well in 100 µL) were then added to the wells containing either Pre-ECP or ECP-DO stimulators, and the plates were cultured for 7 days at 37°C in a CO2 incubator. For target cells, the A-2-positive T-B hybridoma lymphoblast line, 174 x CWM.T1, was labeled with ⁵¹Cr and added to the MLR cultures at 10⁴ cells/well. After 4-hour incubation, plates were centrifuged, and 100 µL of supernatant was removed from each well for counting in a gamma counter. "Percent-specific lysis" was defined as 100 times the following fraction:
Mean cpm (sample) - Mean cpm (T cell only)
Mean cpm (detergent maximum release) - mean cpm (T cells only)

### RNA isolation and microarray hybridization

Total RNA was isolated using RNeasy Mini Kit columns with on-column DNase I treatment (QIAGEN). RNA yield and purity were measured using the NanoDrop ND-1000 Spectrophotometer and the Agilent 2100 Bioana- lyzer. Fragmented cRNAs were hybridized on Affymetrix HG U133 Plus 2.0 human chips, and screening for approximately 47 400 human genes and ESTs was performed by the Yale University W. M. Keck Resource Laboratory. The microarray results are available on Gene Expression Omnibus under accession number GSE23604.

### Data analysis

Raw data without normalization generated from Affymetrix GeneChip Operating Software Version 1.2 (GCOS 1.2; Affymetrix) were analyzed using GeneSpring software 7.2 (Agilent Technologies-Silicon Genetics). Data were normalized using Robust Multi-Array. Only probe sets with a minimal fold change of >2.0 combined with an average signal intensity of 500 or higher in either apheresis such as leukaphereses or treated samples were included in the analysis. Differential gene expression was considered as a ≥2-fold change and P≤.05. Principal component analysis (PCA) of the induced transcriptomes was performed by standard methodology. Signal transduction pathway involvement was identified with MetaCore Software Version 1.0 (GeneGo).

### Quantitative real-time PCR

Microarray expression of selected genes was confirmed in aliquots of the same RNA samples, using quantitative real-time polymerase chain reaction (PCR). RNA was reverse transcribed to cDNA using the High Capacity cDNA Reverse Transcription Kit (Applied Biosystems). Reverse transcription was carried out in a 96-well thermocycler (MJ Research PTC-200) in the following conditions: 25°C, 10 minutes, 37°C, 120 minutes, 85°C, 5 seconds. TaqMan real-time PCR was used to detect transcripts of DC-LAMP, CCR7, CD80, CD86, and CD14. Primers and probes for each sequence were obtained as inventoried Taqman Gene Expression Assays (Applied Biosystems). HPRT1 was used as a reference gene.

### Results

### Large ECP-induced changes in individual gene expressions

The stimulation by ECP of individual gene activation in monocytes was expressed as the ratio of ECP Day 1 to pre-ECP expression for the relevant gene. To preclude inadvertent gene induction during monocyte enrichment, a negative column purification method was used, whereby lymphocytes were retained, and monocytes were passively filtered. The results revealed that the ECP-processed monocytes from both patients and normal subjects remain sufficiently viable to reproducibly express a shared transcriptome signature.

Genes were considered significantly up- or down-regulated by ECP if fold change was >2 and significance was P ≤ .05 compared with pre ECP. Levels of RNA transcripts from approximately 3000 genes were significantly changed in each patient group and in normal subjects (Table 2). Overall, 1129 genes were up- or down-regulated in common by ECP-processed monocytes from both CTCL and GVHD patients and from normal subjects, indicating commonality in ECP-induced gene activation.

**Table 2: Number of Monocyte Genes with Altered Expression after ECP.**

| **Monocyte Source** | **Total** | **Up-regulated** | **Down-regulated** |
|---|---|---|---|
| Normal Subjects (alone): N=6 | 3,666 | 1494 (41%) | 2172 (59%) |
| CTCL (alone): N=3 | 4,315 | 2613 (61%) | 1702 (38%) |
| GVHD (alone): N=3 | 4,350 | 2658 (61%) | 1692 (39%) |

Number of genes significantly induced or suppressed by ECP.

Increased expression of numerous genes associated with dendritic cell differentiation, adhesion, and function (Table 3) further support ECP stimulation of entry of monocytes into that pathway.

**Table 3: : ECP-Enhanced Expression of DC Marker Genes, Ratio* of Post-ECP/Pre-ECP Levels**

| Gene | Attributes | CTCL and GVHD (N-6) Induced Expression Ratio | Normal Subjects (N - 6) Induced Expression Ratio |
|---|---|---|---|
| DC-LAMP | DC Lysomal Protein | 27.6 | 17.2 |
| | | p=1.2x10⁻⁰⁹ | p=1.4 x10⁻⁰⁷ |
| GPNMB | Transmembrane glycoprotein | 205.7 | 123.3 |
| | | p=9.6x10¹⁵ | p=2.8x10¹⁴ |
| CD80 | Co-stimulatory molecule, B7.1 | 13.4 | NC |
| | | p=2.3x10⁻¹³ | |
| CD86 | Co-stimulatory molecule, B7.2⁸ | NC | 5.0 |
| | | | p=1.4x10⁻⁰⁵ |
| CD40 | Involved in DC survival | 2.3 | NC |
| | | p=5.7⁻⁰⁴ | |
| Decysin | ADAM-like, Expressed in LPS matured DC | 26.5 | 7.1 |
| | | p=1.0x10⁻⁰⁹ | p=5.6x10⁻⁰⁴ |
| CCR7 | Lymph node homing molecule | 2.6 | NC |
| | | p=7.0x10⁻⁰³ | |
| CD83 | DC maturation molecule | NC | 2.3 |
| | | | p=0.03 |
| OLR1 | Lox1, lectin-like receptor | 13.6 | 100.1 |
| | | p=3.3x10⁻⁰⁵ | p=8.3x10⁻⁰⁸ |
| CLEC5A | MDL-1 | 10.9 | 45.5 |
| | | P=9.5x10⁻⁰⁷ | p=1.6x10⁻⁰⁸ |
| FPRL2 | Formyl peptide receptor-like-2 | 33.9 | 43.2 |
| | | p=2.1x10⁻⁰⁸ | p=1.9x10⁻⁰⁸ |
| SDC2 | Syndecan, cell surface proteoglycan | 21.7 | 98.9 |
| | | p=9.3x10⁻⁰⁸ | p=3.3x10⁻⁰⁹ |
| THBS1 | Thrombospondin 1 | 6.2 | 10.4 |
| | | p=7.8x10⁻⁰⁸ | p=4.7x10⁻⁰⁹ |

| | | | |
|---|---|---|---|
| *Ratio = (Pre-ECP Gene Expression) to (Post-ECP Gene Expression), Fold increase in expression of multiple genes involved in DC maturation and function induced by ECP. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. | | | |

Further genes, the expression of which was found to be increased and which can be considered to be molecular markers of immune-stimulatory dendritic cells are depicted in Table 1.

As would be expected during monocyte-to-dendritic cell maturation, CD14 (monocyte marker) expression was diminished, as assessed by measuring the mean fluorescence intensity on the monocyte populations of all patients and normal subjects, after overnight culture of ECP-processed monocytes. This result was confirmed in RT-PCR studies of the patients' post-ECP cells (results not shown). Further factors, the expression of which was reduced indicating monocyte-to-dendritic cell maturation are shown in Table 4.

**Table 4: ECP-Reduced Expression of Monocyte Marker Genes , Ratio* of Post-ECP/Pre-ECP Levels**

| Gene | Attributes | CTCL and GVHD (N=6) | Normal Subjects (N=6) |
|---|---|---|---|
| | | Induced Expression Ratio | Induced Expression Ratio |
| CD33 | Cell surface protein expressed on monocytes | -2.2 | NC |
| | | p=4.5x10⁻⁰⁴ | |
| CD36 | Receptor for apoptotic cells | -7.4 | NC |
| | | p=7.9x10⁻⁰⁵ | |
| FCGR1A | Receptor for IgGFc fragment 1A | -6.9 | -4.4 |
| | | p=6.6x10⁻⁰⁵ | p=2.1 x10⁻⁰³ |

| | | | |
|---|---|---|---|
| *Ratio = (Pre-ECP Gene Expression) to (Post-ECP Gene Expression), Fold decrease in expression of genes distinctive of monocytes induced by ECP, as the monocytes differentiate into DC. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. | | | |

Further factors, the expression of which was reduced and thus indicating monocyte-to-immuno suppressive dendritic cell maturation are shown in Table 5.

**Table 5: ECP-Enhanced Expression of Immunosuppression-Associated Genes, Ratio* of Post-ECP/Pre-ECP Levels.**

| Gene | Attributes Normal | CTCL and GVHD (N-6) | Normal Subjects (N-6) |
|---|---|---|---|
| | | Induced Expression Ratio | Induced Expression Ratio |
| IDO | Indoleamine | 27.8 | 9.4 |
| | | p=4.0x10⁻¹⁰ | p=1.1x10⁻⁰⁶ |
| KMO | kynurenine 3-hydroxylase | 6.0 | NC |
| | | p=2.5x10⁻⁰⁶ | |
| IL10 | Interleukin 10 | 6.3 | 8.6 |
| | | p=9.2x10⁻⁰⁶ | =5.7x10⁻⁰⁶ |

| | | | |
|---|---|---|---|
| *Ratio = (Pre-ECP Gene Expression) to (Post-ECP Gene Expression), ECP-induced fold increase in expression of genes which contribute to DC capacity suppress T cell-mediated immunologic reactions. Impact of treatment on gene expression is displayed as an *Induced Expression Ratio* (ratio of post-ECP to pre-ECP expression for the relevant gene). RNA was isolated from 3 CTCL patients and 3 GVHD patients and 6 normal subjects at the relevant time points. | | | |

### Experiment 4 - Surface molecule markers and functional mediators of immuno-stimulatory DC.

Further analysis of the ECP-induced dendritic cells transcriptome was performed to identify a subset of surface molecule gene products as markers and functional mediators of immuno-stimulatory dendritic cells. Of 466 genes upregulated in ECP-induced dendritic cells were cross referenced to approximately 2000 known or presumed full-length human transmembrane genes to identify 87 shared surface proteins.

### Materials and Methods

### Procurement of leukocytes and platelets

All samples were acquired from young, healthy subjects not taking medications, including aspirin, known to influence platelet function. Samples were obtained under the guidelines of the Yale Human Investigational Review Board, and informed consent was provided according to the Declaration of Helsinki. Peripheral blood specimens were collected through a 19-gauge needle from the antecubital vein into syringes containing heparin, then layered on Ficoll-Hypaque (Gallard-Schlessinger, Carle Place, N.Y.). Following centrifugation at 180g, the interface containing the mononuclear leukocyte fraction was collected and washed twice in HBSS, then resuspended in RPMI-1640 medium (GIBCO) to a final concentration of 5 x 10⁶ mononuclear cells/ml. Cells were utilized within one hour of being acquired.

### Preparation of Platelet-rich-Plasma

Whole blood was centrifuged at 150 g for 15 min at room temperature. The platelet-rich-plasma (PRP) layer was collected and centrifuged at 900 g for 5 min, and the platelet pellet resuspended in RPMI 1640 to the desired concentration.

### Preparation of Plates

Plate passage was conducted using a Glycotech system (Glycotech, Rockville, MD). This system consisted of a volumetric flow path measuring 20000 x 10000 x 254 microns (length x width x height). The bottom plate in this system was composed of a 15mm petri dish (BD Biosciences, Durham, NC) separated by a gasket and vacuum-connected to an acrylic flow deck, which formed the upper plate. For precoating with platelets, prior to assembling the flow chamber, 20 drops of the desired concentration of PRP was placed in the center of the petri dish and platelets allowed to settle for 20 minutes at room temperature. The petri dish was washed twice with 2ml of RPMI, and the flow chamber then assembled.

### Overnight culture

When overnight culture was required, cells were centrifuged and resuspended in RPMI-1640 medium (GIBCO), supplemented with 15% AB serum (Gemini Bio-Products) to a final concentration of 5 x 106 cells/ml. Cells were cultured overnight for 18 hours in 12-well polystyrene tissue culture plates (2 ml per well) at 37° C in 5% CO2.

### Immunophenotyping

Monoclonal antibodies for immunophenotyping included CD 14 (LPS receptor; monocytes), CD11c (integrin subunit; monocytes and DC), HLA-DR (class II MHC molecule), CD83 (DC marker), CD62p (P-selectin; activated platelets), and CD61 (integrin subunit; platelets). Antibodies were obtained from Beckman Coulter (CD14, CD11c, HLADR, CD83) or Sigma (CD62p, CD61) and used at their pre-determined optimal dilutions. Background staining was established with appropriate isotype controls, and immunofluorescence was analyzed using a FC500 flow cytometer (Beckman Coulter). Two-color membrane staining was performed by adding the pre-determined optimal concentrations of both antibodies directly conjugated to FITC or PE and incubating for 20 min at 4°C, followed by washing to remove unbound antibodies. Combined membrane and cytoplasmic staining was performed following manufacturer's instructions for cell fixation and permeabilization (Intraprep kit, Beckman Coulter).

### Results

Plate-passed and/or PBMC D1 populations showed significant upregulation of analyzed surface expression of SIRPa, ICAM1, CXCL16, LIGHT, PLAUR (CD87, plasminogen activator, urokinase receptor), MSR1, Neul (sialidase), CD137L, and CATB (CTSB, cathepsin B).

### Experiment 5 - Determining expression of molecular markers and FSC/SSC complexity after passing monocytes through flow chamber

### Materials and Methods

Monocytes were passed through a device depicted in Fig. 19. In brief, a blood sample was spun at low speed through a Ficoll gradient to obtain e.g. 8 ml of sample with a concentration of peripheral blood mononuclear cells (PBMC) of e.g. 10¹⁰ cells/ml. The chamber was pre-coated with platelets. The sample was passed through the chamber at about 0.028 Pa. The chamber and then washed with about 3 ml RPMI at 0.028 Pa. A second wash with 30-55 ml RPMI was performed at about 1.2 Pa. The collected activated monocytes were combined, incubated for a day and used for further analysis (PP D1 PBMC). As a control PBMCs were not passed through the device and incubated for a day (D1 PBMC). As another control immature fast DC were obtained by directly cultivating PBMC in the presence of GM-CSF and IL-4 (immature Fast DC). Further, PBMC were analyzed directly after harvest through a Ficoll gradient (Fresh (Ficoll) PBMC).

The cells and controls were then analyzed for expression of HLA-DR, CD86, ICAM-1, and PLAUR. They were further analyzed for FSC/SSC complexity. The results are depicted for HLA-DR in Figure 20 and for FSC/SSC complexity in Figure 21 and 22. A summary is shown in Figure 23.

### Results

The results show that cells subjected to centrifugation through a Ficoll gradient alreads seem to experience enough physical forces to start differentiating as becomes apparent from incubating these cells for one day (D1 PBMC). However, activation and differentiation is more pronounced upon plate passage through the device (PP D1 PDMC). The dendritic cells obtained by methods in accordance with the invention in the absence of e.g. 8-MOP and UV-A moreover have a more complex and distinct pattern than immature Fast DC obtained with cytokine cocktails.

### Experiment 6 - Determining inlfuence of 8-MOP and UVA on activated monocytes

### Materials and Methods

Monocytes were passed through a device derived from the Therakos system. The dimensions of the flow chamber were 30 mm width, 1600 mm length, and 1 mm height. In brief, a blood sample was spun at low speed through a Ficoll gradient to obtain e.g. 80 ml of sample with a concentration of peripheral blood mononuclear cells (PBMC) of e.g. 1.5^{∗}10⁶ cells/ml. The chamber was pre-coated with platelets. The sample was passed through the chamber for 60 min at a flow rate of about 24 ml/min corresponding to a shear stress of about 0.13 Pa. The collected activated monocytes were spun at low speed and resuspended in a small volume of medium comprising 8-MOP and irradiated with combined, incubated for a day and used for further analysis (Day 1PBMC + PP). As a control PBMCs were not passed through the device and incubated for a day (Day 1 PBMC). Further, PBMC were analyzed directly after harvest through a Ficoll gradient (Day0 PBMC). Day 1 PBMC + PP were then further treated with 8-MOP and UVA (PUVA).

The cells and controls were then analyzed for expression of GILZ. The results are depicted for GILZ in Figure 24.

### Results

The results show that cells that activated monocytes that would differentiate into immuno-stimulatory dendritic cells can be channeled into immuno-suppressive dendritic cells by application of 8-MOP and UVA.

### References:

1. Berger C, Hoffmann K, Vasquez JG, Mane S, Lewis J, Filler R et al. Rapid generation of maturationally synchronized human dendritic cells: contribution to the clinical efficacy of extracorporeal photochemotherapy. Blood 2010; 116(23): 4838-4847.
2. Cella M, Scheidegger D, PalmerLehmann K, Lane P, Lanzavecchia A, Alber G. Ligation of CD40 on dendritic cells triggers production of high levels of interleukin-12 and enhances T cell stimulatory capacity: T-T help via APC activation. Journal of Experimental Medicine 1996; 184(2): 747-752.
3. de Saint-Vis B, Vincent J, Vandenabeele S, Vanbervliet B, Pin JJ, Ait-Yahia S et al. A novel lysosome-associated membrane glycoprotein, DC-LAMP, induced upon DC maturation, is transiently expressed in MHC class II compartment. Immunity 1998; 9(3): 325-336.
4. Slavik JM, Hutchcroft JE, Bierer BE. CD80 and CD86 are not equivalent in their ability to induce the tyrosine phosphorylation of CD28. Journal of Biological Chemistry 1999; 274(5): 3116-3124.
5. Kang HK, Lee HY, Kim MK, Park KS, Park YM, Kwak JY et al. The synthetic peptide Trp- Lys-Tyr-Met-Val-D-Met inhibits human monocyte-derived dendritic cell maturation via formyl peptide receptor and formyl peptide receptor-like 2. Journal of Immunology 2005; 175(2): 685-692.
6. Ripoll VM, Irvine KM, Ravasi T, Sweet MJ, Hume DA. Gpnmb is induced in macrophages by IFN-gamma and lipopolysaccharide and acts as a feedback regulator of proinflammatory responses. Journal of Immunology 2007; 178(10): 6557-6566.
7. Chen SQ, Springer TA. Selectin receptor-ligand bonds: Formation limited by shear rate and dissociation governed by the Bell model. Proceedings of the National Academy of Sciences of the United States of America 2001; 98(3): 950-955.
8. Thomas WE. Understanding the counterintuitive phenomenon of catch bonds. Current Nanoscience 2007; 3: 63-83.
9. Xiong JP, Stehle T, Zhang RG, Joachimiak A, Frech M, Goodman SL et al. Crystal structure of the extracellular segment of integrin alpha V beta 3 in complex with an Arg-Gly-Asp ligand. Science 2002; 296(5565): 151-155.
10. Weisel JW, Nagaswami C, Vilaire G, Bennett JS. Examination of the Platelet Membrane Glycoprotein-IIB-IIIA Complex and its Interaction with Fibrinogen and Other Ligands by Electron-Microscopy. Journal of Biological Chemistry 1992; 267(23): 16637-16643.
11. Kaplan KL, Broekman MJ, Chernoff A, Lesznik GR, Drillings M. Platelet alpha-granule proteins - studies on release and subcellular-localization. Blood 1979; 53(4): 604-618.
12. Ruoslahti E. RGD and other recognition sequences for integrins. Annual Review of Cell and Developmental Biology 1996; 12: 697-715.
13. Zhou LJ, Tedder TF. CD14+ blood monocytes can differentiate into functionally mature CD83+ dendritic cells. Proc. Natl. Acad. Sci. U.S.A. 1996;93(6):2588-2592.
14. Klein E. CD83 localization in a recycling compartment of immature human monocyte-derived dendritic cells. International Immunology. 2005;17(4):477-487.
15. Renzo MD, Rubegni P, Pasqui AL, et al. Extracorporeal photopheresis affects interleukin (IL)-10 and IL-12 production by monocytes in patients with chronic graft-versus-host disease. Br J Dermatol. 2005;153(1):59-65.
16. Gasparro FP, Bevilacqua PM, Goldminz D, et al. Repair of 8-MOP photoadducts in human lymphocyte. In DNA Damage and Repair in Human Tissues (edited by B. M. Sutherland and A.D. Woodhead). Plenum Press, New York.
17. Steinman RM, Hawiger D, Nussenzweig MC. Tolerogenic dendritic cells. Annu. Rev. Immunol. 2003;21:685-711.

### Embodiments of the invention

1. A method for inducing differentiation of monocytes contained in an extracorporeal quantity of a mammalian subject's blood sample into immuno-suppressive antigen-presenting cells, said method comprising at least the steps of:
   a) subjecting said extracorporeal quantity of said mammalian subject's blood sample to a physical force such that said monocytes are activated and induced to differentiate into immuno-suppressive antigen-presenting cells, which are identifiable by at least one molecular marker, wherein said at least one molecular marker is indicative of immuno-suppressive antigen-presenting cells.
2. Method according to embodiment 1,
   wherein said immuno-suppressive antigen-presenting cells are immuno-suppressive dendritic cells which are identifiable by an increased expression of GILZ IDO, KMO, and/or IL-10.
3. Method according to any of embodiments 1 or 2,
   wherein said immuno-suppressive antigen-presenting or dendritic cells are identifiable by a GILZ induced increased IL-10 to IL-12p70 ratio.
4. Method according to any of embodiments 1, 2, or 3,
   wherein said immuno-suppressive antigen-presenting or dendritic cells are identifiable by determining expression of at least 1, 2, 3, 4, 5, 6, 7, 8, 9, 10, 15, 20 , 25, 30, 35, 40, 45, 50, 55, or 60 molecular markers, which are indicative of immuno-stimulatory antigen-presenting or dendritic cells
5. Method according to any of embodiments 1, 2, 3, or 4,
   wherein said at least 1, 2, 3, 4, 5, 6, 7, 8, 9, 10, 15, 20 , 25, 30, 35, 40, 45, 50, 55, or 60 molecular markers are selectable from table 1 do not show an increased expression.
6. Method according to embodiment 5,
   wherein said at least 1, 2, 3, 4, 5, 6, 7, 8, 9, 10, 15, 20 , 25, 30, 35, 40, 45, 50, 55, or 60 molecular markers include PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB and/or CD86.
7. Method according to any of embodiments 1, 2, 3, 4, 5, or 6,
   wherein said monocytes are activated and induced to differentiate into said immuno-suppressive antigen-presenting or dendritic cells without the need for addition of a molecular cocktail comprising cytokines and/or chemokines.
8. Method according to any of embodiments 1, 2, 3, 4, 5, 6, or 7,
   wherein said extracorporeal quantity of said mammalian subject's blood sample is subjected to a physical force by passing said extracorporeal quantity of said mammalian subject's blood sample through a flow chamber of a device, which allows for fixed or tunable adjustment of the flow rate of said extracorporeal quantity of said mammalian subject's blood sample through said flow chamber of said device such that a shear force is applied to said monocytes contained within said mammalian subject's blood sample.
9. Method according to any of embodiments 1, 2, 3, 4, 5, 6, 7, or 8,
   wherein said extracorporeal quantity of said mammalian subject's blood sample is subjected to a physical force by passing said extracorporeal quantity of said mammalian subject's blood sample through a flow chamber of a device, which allows adjustment of the flow rate of said extracorporeal quantity of said mammalian subject's blood sample through said flow chamber of said device such that a shear force is applied to said monocytes contained within said mammalian subject's blood sample, and
   wherein said device additionally allows for adjustment of at least one parameter selected from the group comprising temperature, and light exposure.
10. Method according to any of embodiments 1, 2, 3, 4, 5, 6, 7, 8, or 9,
   wherein said monocytes are activated and induced to differentiate into immuno-suppressive antigen-presenting or dendritic cells through interaction with activated platelets and/or plasma components.
11. Method according to any of embodiments 1, 2, 3, 4, 5, 6, 7, 8, 9, or 10,
   wherein activation of said monocytes and differentiation into immuno-suppressive antigen-presenting or dendritic cells can be influenced by the design and dimensions of the flow chamber, the flow rate at which the monocytes are passed through the flow chamber, the light to which the monocytes are exposed to in the presence or absence of DNA cross-linking agents such as 8-MOP, the temperature at which the monocytes, platelets, platelets-derived factors and/or plasma components are passed through the flow chamber, the order by which the monocytes, platelets, platelets-derived factors and/or plasma components are passed through the flow chamber, the density by which plasma components are coated to the surfaces of the flow chamber, the density by which platelets and/or platelets derived factors adhere to the surfaces and or to the plasma components of the flow chamber, and/or the density by which monocytes adhere to the platelets and/or platelets derived factors and or plasma components adhered to the surfaces of the flow chamber.
12. Method according to any of embodiments 1, 2, 3, 4, 5, 6, 7, 8, 9, 10, or 11,
   wherein said method comprises at least the steps of:
   a) applying said extracorporeal quantity of said mammalian subject's blood sample comprising at least monocytes to a device, which is configured to provide for a flow chamber through which said extracorporeal quantity of said mammalian subject's blood sample can be passed,
   b) activating platelets, which may be comprised within said extracorporeal quantity of said mammalian subject's blood or which may be provided separate from said mammalian subject's blood sample comprising at least monocytes,
   c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive antigen-presenting or dendritic cells by binding to said activated platelets obtained in step b).
13. Method according to any of embodiments 1, 2, 3, 4, 5, 6, 7, 8, 9, 10, or 11,
   wherein said method comprises at least the steps of:
   a) applying said extracorporeal quantity of said mammalian subject's blood sample comprising at least monocytes to a device, which is configured to provide for a flow chamber through which said extracorporeal quantity of said mammalian subject's blood sample can be passed,
   b) passing plasma components, which may be comprised within said extracorporeal quantity of said mammalian subject's blood sample or which may be provided separate from said mammalian subject's blood sample,
   c) treating said extracorporeal quantity of said mammalian subject's blood sample comprising at least monocytes in said device by applying a physical force to the monocytes contained within said extracorporeal quantity of said mammalian subject's blood sample such that said monocytes are activated and induced to differentiate into immuno-suppressive antigen-presenting or dendritic cells by binding to said plasma components obtained in step b).
14. Method according to any of embodiments 1, 2, 3, 4, 5, 6, 7, 8, 9, 10, or 11,
   wherein said method comprises at least the steps of:
   a) applying said extracorporeal quantity of said mammalian subject's blood sample comprising at least monocytes to a device, which is configured to provide for a flow chamber through which said extracorporeal quantity of said mammalian subject's blood sample can be passed,
   b) passing plasma components, which may be comprised within said extracorporeal quantity of said mammalian subject's blood or which may be provided separate from said mammalian subject's blood sample,
   c) activating platelets, which may be comprised within said extracorporeal quantity of said mammalian subject's blood sample or which may be provided separate from said mammalian subject's blood sample comprising at least monocytes,
   d) treating said extracorporeal quantity of said mammalian subject's blood comprising at least monocytes in said device by applying a physical force to the monocytes contained within said extracorporeal quantity of said mammalian subject's blood sample such that said monocytes are activated and induced to differentiate into immuno-suppressive antigen-presenting or dendritic cells by binding to said activated platelets and/or plasma components obtained in steps b) and c).
15. Method of any of embodiments 12, 13, or 14,
   wherein said extracorporeal quantity of said mammalian subject's blood sample is not obtained by .
16. Method of embodiment 15,
   wherein said extracorporeal quantity of said mammalian subject's blood sample is between about 10 ml to about 500 ml of extracorporeal whole blood of said mammalian subject.
17. Method of any of embodiments 15 to 16,
   wherein said extracorporeal quantity of said mammalian subject's blood sample is obtained by isolating leukocytes from about 10 ml to about 500 ml of extracorporeal whole blood of said mammalian subject.
18. Method of any of embodiments 15, 16, or 17,
   wherein said extracorporeal quantity of said mammalian subject's blood sample is obtained by isolating buffy coats from about 10 ml to about 500 ml of extracorporeal whole blood of said mammalian subject.
19. Method of any of embodiments 15, 16, 17, or 18,
   wherein said extracorporeal quantity of said mammalian subject's blood sample does not comprise plasma components.
20. Method of any of embodiments 15, 16, 17, 18, or 19,
   wherein said extracorporeal quantity of said mammalian subject's blood sample does not comprise platelets.
21. Method of embodiment 20,
   wherein said platelets have been separated from said extracorporeal quantity of said mammalian subject's blood before said extracorporeal quantity of said mammalian subject's blood said is applied to said device.
22. Method of any of embodiments 12, 13, or 14,
   wherein said extracorporeal quantity of said mammalian subject's blood is obtained by .
23. Method of embodiment 22,
   wherein said extracorporeal quantity of said mammalian subject's blood is obtained by isolating leukocytes by .
24. Method of any of embodiments 22 to 23,
   wherein said extracorporeal quantity of said mammalian subject's blood is obtained by isolating buffy coats by .
25. Method of any of embodiments 22, 23, to 24,
   wherein said extracorporeal quantity of said mammalian subject's blood does not comprise plasma components.
26. Method of any of embodiments 22, 23, 24, or 25,
   wherein said extracorporeal quantity of said mammalian subject's blood does not comprise platelets.
27. Method of embodiment 26,
   wherein said platelets have been separated from said extracorporeal quantity of said mammalian subject's blood before said extracorporeal quantity of said mammalian subject's blood said is applied to said device.
28. Method of any of embodiments 12, 13, 14, 15, 16, 17, 18, 19, 20, 21, 22, 23, 24, 25, 26, or 27, wherein said flow chamber has dimensions of about 1 µm to up to about 400 µm of height and of about 1 µm to up to about 400 µm of width.
29. Method of embodiment 28, wherein said flow chamber has dimensions of about 5 µm to up to and including about 300 µm of height and of about 5 µm to up to and including about 300 µm of width.
30. Method of embodiment 29, wherein said flow chamber has dimensions of about 10 µm to up to and including about 250 µm of height and of about 10 µm to up to and including about 250 µm of width.
31. Method of embodiment 30, wherein said flow chamber has dimensions of about 50 µm to up to and including about 200 µm of height and of about 50 µm to up to and including about 200 µm of width.
32. Method of embodiment 31, wherein said flow chamber has dimensions of about 50 µm to up to and including about 100 µm of height and of about 50 µm to up to and including about 100 µm of width.
33. Method of any of embodiments 28, 29, 30, 31, or 32, wherein said flow chamber is configured to take up a volume of between about 1 ml to about 50 ml of said extracorporeal amount of said mammalian subject's blood sample.
34. Method of any of embodiments 8, 9, 10, 11, 12, 13, 14, 15, 16, 17, 18, 19, 20, 21, 22, 23, 24, 25, 26, 27, 28, 29, 30, 31, 32, or 33, wherein the material of said flow chamber is not plastic.
35. Method of embodiment 34, wherein said non-plastic material is selected from the group consisting of glass.
36. Method of any of embodiments 8, 9, 10, 11, 12, 13, 14, 15, 16, 17, 18, 19, 20, 21, 22, 23, 24, 25, 26, 27, 28, 29, 30, 31, 32, or 33, wherein the material of said flow chamber is plastic.
37. Method of embodiment 36, wherein said plastic material is selected from the group consisting of acrylics, polycarbonate, polyetherimide, polysulfone, polyphenylsulfone, styrenes, polyurethane, polyethylene, teflon or any other appropriate medical grade plastic.
38. Method of any of embodiments 8, 9, 10, 11, 12, 13, 14, 15, 16, 17, 18, 19, 20, 21, 22, 23, 24, 25, 26, 27, 28, 29, 30, 31, 32, 33, 34, 35, 36, or 37, wherein said flow chamber is configured to allow for transmittance of light.
39. Method of embodiment 38, wherein said flow chamber is configured to allow for transmittance of UV light.
40. Method of any of embodiments 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39, wherein activation of said platelets is achieved by disposing plasma components, which are comprised within said extracorporeal quantity of said mammalian subject's blood sample, on the surface of said flow chamber such that at least some of said platelets can interact with said plasma components and are immobilized on the surface of said flow chamber.
41. Method of any of embodiments 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, wherein activation of said platelets is achieved by disposing proteins selected from the group comprising fibrinogen, fibronectin, and the gamma component of fibrinogen on the surface of said flow chamber such that at least some of said platelets can interact with said proteins and are immobilized on the surface of said flow chamber.
42. Method of embodiment 41, wherein activation of said platelets is achieved by disposing fibronectin on the surface of said flow chamber such that at least some of said platelets can interact with said fibronectin and are immobilized on the surface of said flow chamber.
43. Method of any of embodiments 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42, wherein said platelets are passed through said flow chamber under a shear force of about 0.1 to about 10.0 dynes/cm², preferably in a range of about 0.1 to about 2.0 dynes/cm²
44. Method of any of embodiments 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42 or 43, wherein said monocytes are passed through said flow chamber with a flow rate of about 10 ml/minute to about 200 ml/minute to produce a shear force of about 0.1 to about 20.0 dynes/cm².
45. Method of any of embodiments 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or 44, wherein activation of platelets can be monitored by expression of P-selectin and/or αIIb-β3 integrin.
46. Method of any of embodiments 8, 9, 10, 11, 12, 13, 14, 15, 16, 17, 18, 19, 20, 21, 22,23,24,25,26,27,28,29,30,31,32,33,34,35,36,37,38,39,40,41,42,43, 44, or 45, wherein said monocytes are activated and induced to differentiate into immuno-suppressive antigen-presenting or dendritic cells by passing said monocytes through said flow chamber under a shear force of about 0.1 to about 10.0 dynes/cm², preferably force of about 0.1 to about 1.0 dynes/cm² such that said monocytes can bind to said activated platelets.
47. Method of any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or 46, further comprising the step of incubating the activated monocytes to allow the formation of immuno-suppressive dendritic cells.
48. Method of any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, or 47 for obtaining individual-specific functionally and maturationally synchronized immuno-suppressive antigen-presenting or dendritic cells.
49. Method of any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48,
   wherein said monocytes are exposed to UV light in the presence of a DNA-cross linking agent to effect increased expression of GILZ.
50. Method of embodiment 49,
   wherein said monocytes are exposed to UVA light in the presence of 8-MOP to effect increased expression of GILZ.
51. Method of any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50, for obtaining immuno-suppressive autologous antigen-presenting or dendritic cells.
52. Method of any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50, for obtaining immuno-suppressive allogenic antigen-presenting or dendritic cells.
53. Immuno-suppressive autologous dendritic cells obtainable by a method in accordance with any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51 for use in treating autoimmune diseases.
54. Immuno-suppressive autologous dendritic cells for use of embodiment 53,
   wherein the autoimmune disease is selected from the group comprising rheumatoid arthritis, psoriasis, multiple sclerosis, type 1 diabetes and systemic lupus erythematosus.
55. Immuno-suppressive autologous dendritic cells obtainable by a method in accordance with any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51 for use in treating an hypersensitivity diseases.
56. Immuno-suppressive allogenic dendritic cells obtainable by a method in accordance with any of embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 52 for use in treating rejection in solid-organ transplantation and/or Graft-versus-Host disease.

## Claims

1. A method for inducing differentiation of monocytes contained in an extracorporeal quantity of a mammalian subject's blood sample into immuno-suppressive dendritic cells, said method comprising at least the steps of:
a) providing a flow chamber of a device;
b) passing the extracorporeal quantity of said mammalian subject's blood sample through the flow chamber, whereby a shear force is applied to said monocytes; and
c) exposing said monocytes to UV light in the presence of a DNA-cross linking agent, preferably 8-MOP, which activates and induces the monocytes to differentiate into immuno-suppressive dendritic cells.

2. Method according to claim 1,
wherein said immuno-suppressive dendritic cells are identifiable
i) by increased expression of GILZ, IDO, KMO, TGFβ, and/or IL-10;
ii) by a GILZ induced increased IL-10 to IL-12p70 ratio; and/or
iii) by determining expression of at least 1, 2, 3, 4, 5, 6, 7, 8, 9, 10, 15, 20 , 25, 30, 35, 40, 45, 50, 55, or 60 molecular markers, which are indicative of immuno-stimulatory dendritic cells, wherein preferably said at least 1, 2, 3, 4, 5, 6, 7, 8, 9, 10, 15, 20 , 25, 30, 35, 40, 45, 50, 55, or 60 molecular markers are selectable from table 1 and do not show an increased expression; wherein more preferably said at least 1, 2, 3, 4, 5, 6, 7, 8, 9, 10, 15, 20 , 25, 30, 35, 40, 45, 50, 55, or 60 molecular markers include PLAUR, NEU1, CTSB, CXCL16, ICAM1, MSR1, OLR1, SIRPA, TNFRSF1A, TNFSF14, TNFSF9, PMB22, CD40, LAMP3, CD80, CCR7, LOX1, CD83, ADAM Decysin, FPRL2, GPNMB and/or CD86; and/or
iv) full resistance to maturation by LPS stimulation.

3. Method according to any of claims 1 or 2,
wherein
i) said device allows for fixed or tunable adjustment of the flow rate; and/or
ii) said device allows for adjustment of at least one parameter selected from the group comprising temperature and light exposure; and/or
iii) wherein said monocytes are activated and induced to differentiate into immuno-suppressive dendritic cells through interaction with activated platelets and/or plasma components; and/or
iv) wherein activation of said monocytes and differentiation into immuno-suppressive dendritic cells can be influenced by the design and dimensions of the flow chamber, the flow rate at which the monocytes are passed through the flow chamber, the light to which the monocytes are exposed to in the presence or absence of DNA cross-linking agents such as 8-MOP, the temperature at which the monocytes, platelets, platelets-derived factors and/or plasma components are passed through the flow chamber, the order by which the monocytes, platelets, platelets-derived factors and/or plasma components are passed through the flow chamber, the density by which plasma components are coated to the surfaces of the flow chamber, the density by which platelets and/or platelets derived factors adhere to the surfaces and or to the plasma components of the flow chamber, and/or the density by which monocytes adhere to the platelets and/or platelets derived factors and or plasma components adhered to the surfaces of the flow chamber.

4. Method according to any of claims 1 to 3,
wherein said method additionally comprises
(i) before method step b), the step of passing platelets through the flow chamber, which may be comprised within said extracorporeal quantity of said mammalian subject's blood or which may be provided separate from said mammalian subject's blood sample comprising at least monocytes; or
(ii) before method step b), the step of passing plasma components through the flow chamber, which may be comprised within said extracorporeal quantity of said mammalian subject's blood sample or which may be provided separate from said mammalian subject's blood sample; or
(iii) before method step b), the steps of (i) and (ii).

5. Method of claim 4, wherein said extracorporeal quantity of said mammalian subject's blood sample has not been obtained by apheresis; wherein
i) said extracorporeal quantity of said mammalian subject's blood sample is between about 10 ml to about 500 ml of extracorporeal whole blood of said mammalian subject;
ii) wherein said extracorporeal quantity of said mammalian subject's blood sample is obtained by isolating leukocytes from about 10 ml to about 500 ml of extracorporeal whole blood of said mammalian subject; and/or
iii) wherein said extracorporeal quantity of said mammalian subject's blood sample is obtained by isolating buffy coats from about 10 ml to about 500 ml of extracorporeal whole blood of said mammalian subject.

6. Method of claim 5, wherein
i) said extracorporeal quantity of said mammalian subject's blood sample does not comprise plasma components; and/or
ii) wherein said extracorporeal quantity of said mammalian subject's blood sample does not comprise platelets, wherein preferably said platelets have been separated from said extracorporeal quantity of said mammalian subject's blood before said extracorporeal quantity of said mammalian subject's blood said is applied to said device.

7. Method of claim 4, wherein said extracorporeal quantity of said mammalian subject's blood has been obtained by apheresis; and
i) wherein preferably i) said extracorporeal quantity of said mammalian subject's blood is obtained by isolating leukocytes by leukapheresis; wherein preferably said extracorporeal quantity of said mammalian subject's blood is obtained by isolating buffy coats by leukapheresis; and/or
ii) said extracorporeal quantity of said mammalian subject's blood does not comprise plasma components; and/or
iii) said extracorporeal quantity of said mammalian subject's blood does not comprise platelets, wherein preferably said platelets have been separated from said extracorporeal quantity of said mammalian subject's blood before said extracorporeal quantity of said mammalian subject's blood said is applied to said device.

8. Method of any of claims 1 to 7, wherein
i) said flow chamber has dimensions of about 1 µm to up to about 400 µm of height and of about 1 µm to up to about 400 µm of width, wherein preferably said flow chamber has dimensions of about 5 µm to up to and including about 300 µm of height and of about 5 µm to up to and including about 300 µm of width, wherein more preferably said flow chamber has dimensions of about 10 µm to up to and including about 250 µm of height and of about 10 µm to up to and including about 250 µm of width, and wherein even more preferably said flow chamber has dimensions of about 50 µm to up to and including about 200 µm of height and of about 50 µm to up to and including about 200 µm of width; and wherein even more preferably said flow chamber has dimensions of about 50 µm to up to and including about 100 µm of height and of about 50 µm to up to and including about 100 µm of width; and/or
ii) said flow chamber is configured to take up a volume of between about 1 ml to about 50 ml of said extracorporeal amount of said mammalian subject's blood sample.

9. Method of any of claims 1 to 8, wherein
i) the material of said flow chamber is not plastic, and wherein preferably said non-plastic material is selected from the group consisting of glass; or
ii) the material of said flow chamber is plastic, wherein preferably said plastic material is selected from the group consisting of acrylics, polycarbonate, polyetherimide, polysulfone, polyphenylsulfone, styrenes, polyurethane, polyethylene, teflon or any other appropriate medical grade plastic; and/or
iii) said flow chamber is configured to allow for transmittance of light.

10. Method of any of claims 3 to 9, wherein
i) activation of said platelets is achieved by disposing plasma components, which are comprised within said extracorporeal quantity of said mammalian subject's blood sample, on the surface of said flow chamber such that at least some of said platelets can interact with said plasma components and are immobilized on the surface of said flow chamber;
ii) activation of said platelets is achieved by disposing proteins selected from the group comprising fibrinogen, fibronectin, and the gamma component of fibrinogen on the surface of said flow chamber such that at least some of said platelets can interact with said proteins and are immobilized on the surface of said flow chamber, wherein preferably activation of said platelets is achieved by disposing fibronectin on the surface of said flow chamber such that at least some of said platelets can interact with said fibronectin and are immobilized on the surface of said flow chamber; and/or
iii) activation of platelets can be monitored by expression of P-selectin and/or αIIb-β3 integrin.

11. Method of any of claims 3 to 10, wherein
said platelets are passed through said flow chamber under a shear force of about 0.1 to about 10.0 dynes/cm2, preferably in a range of about 0.1 to about 2.0 dynes/cm2, and/or

12. Method of any of claims 3 to 11, wherein
i) said monocytes are passed through said flow chamber with a flow rate of about 10 ml/minute to about 200 ml/minute to produce a shear force of about 0.1 to about 20.0 dynes/cm2; and/or
ii) wherein said monocytes are passed through said flow chamber under a shear force of about 0.1 to about 10.0 dynes/cm², preferably a shear force of about 0.1 to about 1.0 dynes/cm² such that said monocytes can bind to said activated platelets.

13. Method of any of claims 3 to 12,
wherein the method further comprises the step of incubating the activated monocytes to allow the formation of immuno-suppressive dendritic cells.

14. Method of any of claims 1 to 13 for obtaining individual-specific functionally and maturationally synchronized immuno-suppressive dendritic cells.

15. Method of any of claims 1 to 13 for obtaining immuno-suppressive autologous dendritic cells or immuno-suppressive allogenic dendritic cells.


SEQUENCE LISTING

<110>  TRANSIMMUNE AG, YALE UNIVERSITY

<120>  Method for obtaining immuno-suppressive dendritic cells

<130>  T 7757-WO

<150>  GB1300052.6
<151>  2013-01-03

<150>  USP 61/748,550
<151>  2013-01-03

<160>  105

<170>  PatentIn version 3.5

<210>  1
<211>  10515
<212>  DNA
<213>  Homo sapiens

<400>  1
ggaggaggga gagcacaggc tttgaccgat agtaacctct gcgctcggtg cagccgaatc      60

tataaaagga actagtcccg gcaaaaaccc cgtaattgcg agcgagagtg agtggggccg     120

ggacccgcag agccgagccg acccttctct cccgggctgc ggcagggcag ggcggggagc     180

tccgcgcacc aacagagccg gttctcaggg cgctttgctc cttgtttttt ccccggttct     240

gttttctccc cttctccgga aggcttgtca aggggtagga gaaagagacg caaacacaaa     300

agtggaaaac agttaatgac cagccacggc gtccctgctg tgagctctgg ccgctgcctt     360

ccagggctcc cgagccacac gctgggggtg ctggctgagg gaacatggct tgttggcctc     420

agctgaggtt gctgctgtgg aagaacctca ctttcagaag aagacaaaca tgtcagctgc     480

tgctggaagt ggcctggcct ctatttatct tcctgatcct gatctctgtt cggctgagct     540

acccacccta tgaacaacat gaatgccatt ttccaaataa agccatgccc tctgcaggaa     600

cacttccttg ggttcagggg attatctgta atgccaacaa cccctgtttc cgttacccga     660

ctcctgggga ggctcccgga gttgttggaa actttaacaa atccattgtg gctcgcctgt     720

tctcagatgc tcggaggctt cttttataca gccagaaaga caccagcatg aaggacatgc     780

gcaaagttct gagaacatta cagcagatca agaaatccag ctcaaacttg aagcttcaag     840

atttcctggt ggacaatgaa accttctctg ggttcctgta tcacaacctc tctctcccaa     900

agtctactgt ggacaagatg ctgagggctg atgtcattct ccacaaggta tttttgcaag     960

gctaccagtt acatttgaca agtctgtgca atggatcaaa atcagaagag atgattcaac    1020

ttggtgacca agaagtttct gagctttgtg gcctaccaag ggagaaactg gctgcagcag    1080

agcgagtact tcgttccaac atggacatcc tgaagccaat cctgagaaca ctaaactcta    1140

catctccctt cccgagcaag gagctggctg aagccacaaa aacattgctg catagtcttg    1200

ggactctggc ccaggagctg ttcagcatga gaagctggag tgacatgcga caggaggtga 1260

tgtttctgac caatgtgaac agctccagct cctccaccca aatctaccag gctgtgtctc 1320

gtattgtctg cgggcatccc gagggagggg ggctgaagat caagtctctc aactggtatg 1380

aggacaacaa ctacaaagcc ctctttggag gcaatggcac tgaggaagat gctgaaacct 1440

tctatgacaa ctctacaact ccttactgca atgatttgat gaagaatttg gagtctagtc 1500

ctctttcccg cattatctgg aaagctctga agccgctgct cgttgggaag atcctgtata 1560

cacctgacac tccagccaca aggcaggtca tggctgaggt gaacaagacc ttccaggaac 1620

tggctgtgtt ccatgatctg gaaggcatgt gggaggaact cagccccaag atctggacct 1680

tcatggagaa cagccaagaa atggaccttg tccggatgct gttggacagc agggacaatg 1740

accacttttg ggaacagcag ttggatggct tagattggac agcccaagac atcgtggcgt 1800

ttttggccaa gcacccagag gatgtccagt ccagtaatgg ttctgtgtac acctggagag 1860

aagctttcaa cgagactaac caggcaatcc ggaccatatc tcgcttcatg gagtgtgtca 1920

acctgaacaa gctagaaccc atagcaacag aagtctggct catcaacaag tccatggagc 1980

tgctggatga gaggaagttc tgggctggta ttgtgttcac tggaattact ccaggcagca 2040

ttgagctgcc ccatcatgtc aagtacaaga tccgaatgga cattgacaat gtggagagga 2100

caaataaaat caaggatggg tactgggacc ctggtcctcg agctgacccc tttgaggaca 2160

tgcggtacgt ctgggggggc ttcgcctact tgcaggatgt ggtggagcag gcaatcatca 2220

gggtgctgac gggcaccgag aagaaaactg gtgtctatat gcaacagatg ccctatccct 2280

gttacgttga tgacatcttt ctgcgggtga tgagccggtc aatgcccctc ttcatgacgc 2340

tggcctggat ttactcagtg gctgtgatca tcaagggcat cgtgtatgag aaggaggcac 2400

ggctgaaaga gaccatgcgg atcatgggcc tggacaacag catcctctgg tttagctggt 2460

tcattagtag cctcattcct cttcttgtga gcgctggcct gctagtggtc atcctgaagt 2520

taggaaacct gctgccctac agtgatccca gcgtggtgtt tgtcttcctg tccgtgtttg 2580

ctgtggtgac aatcctgcag tgcttcctga ttagcacact cttctccaga gccaacctgg 2640

cagcagcctg tgggggcatc atctacttca cgctgtacct gccctacgtc ctgtgtgtgg 2700

catggcagga ctacgtgggc ttcacactca agatcttcgc tagcctgctg tctcctgtgg 2760

cttttgggtt tggctgtgag tactttgccc tttttgagga gcagggcatt ggagtgcagt 2820

gggacaacct gtttgagagt cctgtggagg aagatggctt caatctcacc acttcggtct 2880

ccatgatgct gtttgacacc ttcctctatg gggtgatgac ctggtacatt gaggctgtct 2940

ttccaggcca gtacggaatt cccaggccct ggtattttcc ttgcaccaag tcctactggt 3000

ttggcgagga aagtgatgag aagagccacc ctggttccaa ccagaagaga atatcagaaa 3060

tctgcatgga ggaggaaccc acccacttga agctgggcgt gtccattcag aacctggtaa 3120

aagtctaccg agatgggatg aaggtggctg tcgatggcct ggcactgaat ttttatgagg 3180

gccagatcac ctccttcctg ggccacaatg gagcggggaa gacgaccacc atgtcaatcc 3240

tgaccgggtt gttccccccg acctcgggca ccgcctacat cctgggaaaa gacattcgct 3300

ctgagatgag caccatccgg cagaacctgg gggtctgtcc ccagcataac gtgctgtttg 3360

acatgctgac tgtcgaagaa cacatctggt tctatgcccg cttgaaaggg ctctctgaga 3420

agcacgtgaa ggcggagatg gagcagatgg ccctggatgt tggtttgcca tcaagcaagc 3480

tgaaaagcaa aacaagccag ctgtcaggtg gaatgcagag aaagctatct gtggccttgg 3540

cctttgtcgg gggatctaag gttgtcattc tggatgaacc cacagctggt gtggaccctt 3600

actcccgcag gggaatatgg gagctgctgc tgaaataccg acaaggccgc accattattc 3660

tctctacaca ccacatggat gaagcggacg tcctggggga caggattgcc atcatctccc 3720

atgggaagct gtgctgtgtg ggctcctccc tgtttctgaa gaaccagctg ggaacaggct 3780

actacctgac cttggtcaag aaagatgtgg aatcctccct cagttcctgc agaaacagta 3840

gtagcactgt gtcatacctg aaaaaggagg acagtgtttc tcagagcagt tctgatgctg 3900

gcctgggcag cgaccatgag agtgacacgc tgaccatcga tgtctctgct atctccaacc 3960

tcatcaggaa gcatgtgtct gaagcccggc tggtggaaga catagggcat gagctgacct 4020

atgtgctgcc atatgaagct gctaaggagg gagcctttgt ggaactcttt catgagattg 4080

atgaccggct ctcagacctg ggcatttcta gttatggcat ctcagagacg accctggaag 4140

aaatattcct caaggtggcc gaagagagtg gggtggatgc tgagacctca gatggtacct 4200

tgccagcaag acgaaacagg cgggccttcg gggacaagca gagctgtctt cgcccgttca 4260

ctgaagatga tgctgctgat ccaaatgatt ctgacataga cccagaatcc agagagacag 4320

acttgctcag tgggatggat ggcaaagggt cctaccaggt gaaaggctgg aaacttacac 4380

agcaacagtt tgtggccctt ttgtggaaga gactgctaat tgccagacgg agtcggaaag 4440

gattttttgc tcagattgtc ttgccagctg tgtttgtctg cattgccctt gtgttcagcc 4500

tgatcgtgcc accctttggc aagtacccca gcctggaact tcagccctgg atgtacaacg 4560

aacagtacac atttgtcagc aatgatgctc ctgaggacac gggaaccctg gaactcttaa 4620

acgccctcac caaagaccct ggcttcggga cccgctgtat ggaaggaaac ccaatcccag 4680

acacgccctg ccaggcaggg gaggaagagt ggaccactgc cccagttccc cagaccatca 4740

tggacctctt ccagaatggg aactggacaa tgcagaaccc ttcacctgca tgccagtgta 4800

gcagcgacaa aatcaagaag atgctgcctg tgtgtccccc aggggcaggg gggctgcctc 4860

ctccacaaag aaaacaaaac actgcagata tccttcagga cctgacagga agaaacattt 4920

cggattatct ggtgaagacg tatgtgcaga tcatagccaa aagcttaaag aacaagatct 4980

gggtgaatga gtttaggtat ggcggctttt ccctgggtgt cagtaatact caagcacttc 5040

ctccgagtca agaagttaat gatgccatca aacaaatgaa gaaacaccta aagctggcca 5100

aggacagttc tgcagatcga tttctcaaca gcttgggaag atttatgaca ggactggaca 5160

ccaaaaataa tgtcaaggtg tggttcaata acaagggctg gcatgcaatc agctctttcc 5220

tgaatgtcat caacaatgcc attctccggg ccaacctgca aaagggagag aaccctagcc 5280

attatggaat tactgctttc aatcatcccc tgaatctcac caagcagcag ctctcagagg 5340

tggctctgat gaccacatca gtggatgtcc ttgtgtccat ctgtgtcatc tttgcaatgt 5400

ccttcgtccc agccagcttt gtcgtattcc tgatccagga gcgggtcagc aaagcaaaac 5460

acctgcagtt catcagtgga gtgaagcctg tcatctactg gctctctaat tttgtctggg 5520

atatgtgcaa ttacgttgtc cctgccacac tggtcattat catcttcatc tgcttccagc 5580

agaagtccta tgtgtcctcc accaatctgc ctgtgctagc ccttctactt ttgctgtatg 5640

ggtggtcaat cacacctctc atgtacccag cctcctttgt gttcaagatc cccagcacag 5700

cctatgtggt gctcaccagc gtgaacctct tcattggcat taatggcagc gtggccacct 5760

ttgtgctgga gctgttcacc gacaataagc tgaataatat caatgatatc ctgaagtccg 5820

tgttcttgat cttcccacat ttttgcctgg gacgagggct catcgacatg gtgaaaaacc 5880

aggcaatggc tgatgccctg gaaaggtttg gggagaatcg ctttgtgtca ccattatctt 5940

gggacttggt gggacgaaac ctcttcgcca tggccgtgga aggggtggtg ttcttcctca 6000

ttactgttct gatccagtac agattcttca tcaggcccag acctgtaaat gcaaagctat 6060

ctcctctgaa tgatgaagat gaagatgtga ggcgggaaag acagagaatt cttgatggtg 6120

gaggccagaa tgacatctta gaaatcaagg agttgacgaa gatatataga aggaagcgga 6180

agcctgctgt tgacaggatt tgcgtgggca ttcctcctgg tgagtgcttt gggctcctgg 6240

gagttaatgg ggctggaaaa tcatcaactt tcaagatgtt aacaggagat accactgtta 6300

ccagaggaga tgctttcctt aacaaaaata gtatcttatc aaacatccat gaagtacatc 6360

agaacatggg ctactgccct cagtttgatg ccatcacaga gctgttgact gggagagaac 6420

acgtggagtt ctttgccctt ttgagaggag tcccagagaa agaagttggc aaggttggtg 6480

agtgggcgat tcggaaactg ggcctcgtga agtatggaga aaaatatgct ggtaactata 6540

gtggaggcaa caaacgcaag ctctctacag ccatggcttt gatcggcggg cctcctgtgg 6600

tgtttctgga tgaacccacc acaggcatgg atcccaaagc ccggcggttc ttgtggaatt 6660

gtgccctaag tgttgtcaag gaggggagat cagtagtgct tacatctcat agtatggaag 6720

aatgtgaagc tctttgcact aggatggcaa tcatggtcaa tggaaggttc aggtgccttg 6780

gcagtgtcca gcatctaaaa aataggtttg gagatggtta tacaatagtt gtacgaatag 6840

cagggtccaa cccggacctg aagcctgtcc aggatttctt tggacttgca tttcctggaa 6900

gtgttctaaa agagaaacac cggaacatgc tacaatacca gcttccatct tcattatctt 6960

ctctggccag gatattcagc atcctctccc agagcaaaaa gcgactccac atagaagact 7020

actctgtttc tcagacaaca cttgaccaag tatttgtgaa ctttgccaag gaccaaagtg 7080

atgatgacca cttaaaagac ctctcattac acaaaaacca gacagtagtg gacgttgcag 7140

ttctcacatc ttttctacag gatgagaaag tgaaagaaag ctatgtatga agaatcctgt 7200

tcatacgggg tggctgaaag taaagaggaa ctagactttc ctttgcacca tgtgaagtgt 7260

tgtggagaaa agagccagaa gttgatgtgg gaagaagtaa actggatact gtactgatac 7320

tattcaatgc aatgcaattc aatgcaatga aaacaaaatt ccattacagg ggcagtgcct 7380

ttgtagccta tgtcttgtat ggctctcaag tgaaagactt gaatttagtt ttttacctat 7440

acctatgtga aactctatta tggaacccaa tggacatatg ggtttgaact cacacttttt 7500

tttttttttt tgttcctgtg tattctcatt ggggttgcaa caataattca tcaagtaatc 7560

atggccagcg attattgatc aaaatcaaaa ggtaatgcac atcctcattc actaagccat 7620

gccatgccca ggagactggt ttcccggtga cacatccatt gctggcaatg agtgtgccag 7680

agttattagt gccaagtttt tcagaaagtt tgaagcacca tggtgtgtca tgctcacttt 7740

tgtgaaagct gctctgctca gagtctatca acattgaata tcagttgaca gaatggtgcc 7800

atgcgtggct aacatcctgc tttgattccc tctgataagc tgttctggtg gcagtaacat 7860

gcaacaaaaa tgtgggtgtc tccaggcacg ggaaacttgg ttccattgtt atattgtcct 7920

atgcttcgag ccatgggtct acaggtcatc ccttatgaga ctcttaaata tacttagatc 7980

ctggtaagag gcaaagaatc aacagccaaa ctgctggggc tgcaagctgc tgaagccagg 8040

gcatgggatt aaagagattg tgcgttcaaa cctagggaag cctgtgccca tttgtcctga 8100

ctgtctgcta acatggtaca ctgcatctca agatgtttat ctgacacaag tgtattattt 8160

ctggcttttt gaattaatct agaaaatgaa aagatggagt tgtattttga caaaaatgtt 8220

tgtacttttt aatgttattt ggaattttaa gttctatcag tgacttctga atccttagaa 8280

tggcctcttt gtagaaccct gtggtataga ggagtatggc cactgcccca ctatttttat 8340

tttcttatgt aagtttgcat atcagtcatg actagtgcct agaaagcaat gtgatggtca 8400

ggatctcatg acattatatt tgagtttctt tcagatcatt taggatactc ttaatctcac 8460

ttcatcaatc aaatattttt tgagtgtatg ctgtagctga aagagtatgt acgtacgtat 8520

aagactagag agatattaag tctcagtaca cttcctgtgc catgttattc agctcactgg 8580

tttacaaata taggttgtct tgtggttgta ggagcccact gtaacaatac tgggcagcct 8640

tttttttttt ttttttaatt gcaacaatgc aaaagccaag aaagtataag ggtcacaagt 8700

ctaaacaatg aattcttcaa cagggaaaac agctagcttg aaaacttgct gaaaaacaca 8760

acttgtgttt atggcattta gtaccttcaa ataattggct ttgcagatat tggatacccc 8820

attaaatctg acagtctcaa atttttcatc tcttcaatca ctagtcaaga aaaatataaa 8880

aacaacaaat acttccatat ggagcatttt tcagagtttt ctaacccagt cttatttttc 8940

tagtcagtaa acatttgtaa aaatactgtt tcactaatac ttactgttaa ctgtcttgag 9000

agaaaagaaa aatatgagag aactattgtt tggggaagtt caagtgatct ttcaatatca 9060

ttactaactt cttccacttt ttccagaatt tgaatattaa cgctaaaggt gtaagacttc 9120

agatttcaaa ttaatctttc tatatttttt aaatttacag aatattatat aacccactgc 9180

tgaaaaagaa aaaaatgatt gttttagaag ttaaagtcaa tattgatttt aaatataagt 9240

aatgaaggca tatttccaat aactagtgat atggcatcgt tgcattttac agtatcttca 9300

aaaatacaga atttatagaa taatttctcc tcatttaata tttttcaaaa tcaaagttat 9360

ggtttcctca ttttactaaa atcgtattct aattcttcat tatagtaaat ctatgagcaa 9420

ctccttactt cggttcctct gatttcaagg ccatatttta aaaaatcaaa aggcactgtg 9480

aactattttg aagaaaacac aacattttaa tacagattga aaggacctct tctgaagcta 9540

gaaacaatct atagttatac atcttcatta atactgtgtt accttttaaa atagtaattt 9600

tttacatttt cctgtgtaaa cctaattgtg gtagaaattt ttaccaactc tatactcaat 9660

caagcaaaat ttctgtatat tccctgtgga atgtacctat gtgagtttca gaaattctca 9720

aaatacgtgt tcaaaaattt ctgcttttgc atctttggga cacctcagaa aacttattaa 9780

caactgtgaa tatgagaaat acagaagaaa ataataagcc ctctatacat aaatgcccag 9840

cacaattcat tgttaaaaaa caaccaaacc tcacactact gtatttcatt atctgtactg 9900

aaagcaaatg ctttgtgact attaaatgtt gcacatcatt cattcactgt atagtaatca 9960

ttgactaaag ccatttgtct gtgttttctt cttgtggttg tatatatcag gtaaaatatt 10020

ttccaaagag ccatgtgtca tgtaatactg aaccactttg atattgagac attaatttgt 10080

acccttgtta ttatctacta gtaataatgt aatactgtag aaatattgct ctaattcttt 10140

tcaaaattgt tgcatccccc ttagaatgtt tctatttcca taaggattta ggtatgctat 10200

tatcccttct tataccctaa gatgaagctg tttttgtgct ctttgttcat cattggccct 10260

cattccaagc actttacgct gtctgtaatg ggatctattt ttgcactgga atatctgaga 10320

attgcaaaac tagacaaaag tttcacaaca gatttctaag ttaaatcatt ttcattaaaa 10380

ggaaaaaaga aaaaaaattt tgtatgtcaa taactttata tgaagtatta aaatgcatat 10440

ttctatgttg taatataatg agtcacaaaa taaagctgtg acagttctgt tggtctacag 10500

aaaaaaaaaa aaaaa 10515

<210> 2

<211>  4564
<212>  DNA
<213>  Homo sapiens

<400>  2
ggggaggcgc cgggggcgcg cgcgcgcgcg ctgggcgctg ctgggctgcg gcggcggcgg      60

cggcggcggt ggttactatg gcggagtcgg ccggagcctc ctccttcttc ccccttgttg      120

tcctcctgct cgccggcagc ggcgggtccg ggccccgggg ggtccaggct ctgctgtgtg      180

cgtgcaccag ctgcctccag gccaactaca cgtgtgagac agatggggcc tgcatggttt      240

ccattttcaa tctggatggg atggagcacc atgtgcgcac ctgcatcccc aaagtggagc      300

tggtccctgc cgggaagccc ttctactgcc tgagctcgga ggacctgcgc aacacccact      360

gctgctacac tgactactgc aacaggatcg acttgagggt gcccagtggt cacctcaagg      420

agcctgagca cccgtccatg tggggcccgg tggagctggt aggcatcatc gccggcccgg      480

tgttcctcct gttcctcatc atcatcattg ttttccttgt cattaactat catcagcgtg      540

tctatcacaa ccgccagaga ctggacatgg aagatccctc atgtgagatg tgtctctcca      600

aagacaagac gctccaggat cttgtctacg atctctccac ctcagggtct ggctcagggt      660

tacccctctt tgtccagcgc acagtggccc gaaccatcgt tttacaagag attattggca      720

agggtcggtt tggggaagta tggcggggcc gctggagggg tggtgatgtg gctgtgaaaa      780

tattctcttc tcgtgaagaa cggtcttggt tcagggaagc agagatatac cagacggtca      840

tgctgcgcca tgaaaacatc cttggattta ttgctgctga caataaagat aatggcacct      900

ggacacagct gtggcttgtt tctgactatc atgagcacgg gtccctgttt gattatctga      960

accggtacac agtgacaatt gaggggatga ttaagctggc cttgtctgct gctagtgggc     1020

tggcacacct gcacatggag atcgtgggca cccaagggaa gcctggaatt gctcatcgag     1080

acttaaagtc aaagaacatt ctggtgaaga aaaatggcat gtgtgccata gcagacctgg     1140

gcctggctgt ccgtcatgat gcagtcactg acaccattga cattgccccg aatcagaggg     1200

tggggaccaa acgatacatg gcccctgaag tacttgatga aaccattaat atgaaacact     1260

ttgactcctt taaatgtgct gatatttatg ccctcgggct tgtatattgg gagattgctc     1320

gaagatgcaa ttctggagga gtccatgaag aatatcagct gccatattac gacttagtgc     1380

cctctgaccc ttccattgag gaaatgcgaa aggttgtatg tgatcagaag ctgcgtccca     1440

acatccccaa ctggtggcag agttatgagg cactgcgggt gatggggaag atgatgcgag     1500

agtgttggta tgccaacggc gcagcccgcc tgacggccct gcgcatcaag aagaccctct     1560

cccagctcag cgtgcaggaa gacgtgaaga tctaactgct ccctctctcc acacggagct     1620

cctggcagcg agaactacgc acagctgccg cgttgagcgt acgatggagg cctacctctc     1680

gtttctgccc agccctctgt ggccaggagc cctggcccgc aagagggaca gagcccggga     1740

gagactcgct cactcccatg ttgggtttga gacagacacc ttttctattt acctcctaat 1800

ggcatggaga ctctgagagc gaattgtgtg gagaactcag tgccacacct cgaactggtt 1860

gtagtgggaa gtcccgcgaa acccggtgca tctggcacgt ggccaggagc catgacaggg 1920

gcgcttggga ggggccggag gaaccgaggt gttgccagtg ctaagctgcc ctgagggttt 1980

ccttcgggga ccagcccaca gcacaccaag gtggcccgga agaaccagaa gtgcagcccc 2040

tctcacaggc agctctgagc cgcgctttcc cctcctccct gggatggacg ctgccgggag 2100

actgccagtg gagacggaat ctgccgcttt gtctgtccag ccgtgtgtgc atgtgccgag 2160

gtgcgtcccc cgttgtgcct ggttcgtgcc atgcccttac acgtgcgtgt gagtgtgtgt 2220

gtgtgtctgt aggtgcgcac ttacctgctt gagctttctg tgcatgtgca ggtcgggggt 2280

gtggtcgtca tgctgtccgt gcttgctggt gcctcttttc agtagtgagc agcatctagt 2340

ttccctggtg cccttccctg gaggtctctc cctcccccag agcccctcat gccacagtgg 2400

tactctgtgt ctggcaggct actctgccca ccccagcatc agcacagctc tcctcctcca 2460

tctcagactg tggaaccaaa gctggcccag ttgtccatga caaaagaggc ttttgggcca 2520

aaatgtgagg gtggtgggtg ggatgggcag ggaaggaatc ctggtggaag tcttgggtgt 2580

tagtgtcagc catgggaaat gagccagccc aagggcatca tcctcagcag catcgaggaa 2640

gggccgagga atgtgaagcc agatctcggg actcagattg gaatgttaca tctgtctttc 2700

atctcccaga tcctggaaac agcagtgtat attttggtgg gtggtgggtt tggggtgggg 2760

aagggaaggg cgggcaagga gtggggaggg agtctggggt gggagggagg catctgcatg 2820

ggtcttcttt tactggactg tctgatcagg gtggagggaa ggtgagaggt ttgcatccac 2880

ttcaggagcc ctactgaagg gaacagcctg agccgaacat gttatttaac ctgagtatag 2940

tatttaacga agcctagaag cacggctgtg ggtggtgatt tggtcagcat atcttaggta 3000

tataataact ttgaagccat aacttttaac tggagtggtt tgatttcttt ttttaatttt 3060

attgggaggg tttggatttt aacttttttt aatgttgtta aatattaagt ttttgtaaaa 3120

ggaaaaccat ctctgtgatt acctctcaat ctatttgttt ttaaagaaat ccctaaaaaa 3180

aaaaattatc caattgaacg cacatagctc aatcacactg gaaatgtttg tccttgcacc 3240

tgagcctgtt cccactcagc agtgagagtt cctctttgcc ctgaggctca gtctctctcg 3300

tattttgtcc ccacccccaa ttccttgagt ggtttttgct ctagggccct ttcttgcact 3360

gtccagctgg ttgtaccctc tccaggcatt tattcaacaa atgtgggtga agtgcctgct 3420

gggtgccagg tgctgggaat acatctgtgg acaagacatg cttgggtcct actcctggag 3480

cactgtaaaa agagctgatt caagtaagta gatgcctgtt ttgagaccag aaggtttcat 3540

aattggttct acgacccttt tgagcctaga attattgttc ttatataaga tcactgaaga 3600

aagaggaacc cccacaaccc cctccacaaa gagaccaggg gcgggtgatg agacctgggg 3660

tttagaaccc caggtgagac ctcaaatcac tgcattcatt ctgagccccc ttcctgtccc 3720

caggggaggt gtattgtgta tgtagcctta gagcatctct gcctccaacc cagcagttct 3780

ctgccaaagc ttgtggagga gggagagccc tgtccctgcc ctcaggctcc ccagtgctcc 3840

tggcccttct atttatttga ctgattattg cttctttcct tgcattaaag gagatcttcc 3900

cctaaccttt gggccaattt actggccact aatttcgttt aaataccatt gtgtcattgg 3960

ggggaccgtc tttacccctg ctgacctccc acctatccgc cctgcagcag aaccttggcg 4020

gtttataggt aatgatggaa cttagactcc tcttcccaga gtcacaagta gcctctggga 4080

tctgccaaca cacgtccact cccaagccac tagcccactc cccagttggc ccttctgccc 4140

ttaccccaca cacagtccaa ctcttccacc tctggggaag atggagcagg tctttgggaa 4200

gctcccacac ccacctctgc cactcttaac actaagtgag agttggggag aaactgaagc 4260

cgtgtttttg gccccccgag gctaaccctg atccatagtg ctacctgcac ctctggattc 4320

tggattcaca gaccaagtcc aagcccgttc ttacgtcgcc ataaaggccc ccgaacggca 4380

ttctcggtac ttctgtttgt ttttgtacat tttattagaa aggactgtaa aatagccact 4440

tagacacttt acctcttcag tatgcaaatg taaataaatt gtaatatagg aaatcttttg 4500

ttttaatata agaatgagcc tgtccaattt ctgctgtaca ttattaaaag ttttattcac 4560

agag 4564

<210> 3
<211> 3740
<212> DNA
<213> Homo sapiens

<400> 3
gggacggcgg cggcgcagct cggaacccgc cagggtccag ggtccaggtt ccagcgcccg 60

gcggcccagg caccccccga gcccagctcc acacaccgtt cctggatctc ctctccccag 120

gcggagcgtg cccctgccca gtccagtgac cttcgcctgt tggagccctg gttaattttt 180

gcccagtctg cctgttgtgg ggctcctccc ctttggggat ataagcccgg cctggggctg 240

ctccgttctc tgcctggcct gaggctccct gagccgcctc cccaccatca ccatggccaa 300

gggcttctat atttccaagt ccctgggcat cctggggatc ctcctgggcg tggcagccgt 360

gtgcacaatc atcgcactgt cagtggtgta ctcccaggag aagaacaaga acgccaacag 420

ctcccccgtg gcctccacca ccccgtccgc ctcagccacc accaaccccg cctcggccac 480

caccttggac caaagtaaag cgtggaatcg ttaccgcctc cccaacacgc tgaaacccga 540

ttcctaccgg gtgacgctga gaccgtacct cacccccaat gacaggggcc tgtacgtttt 600

taagggctcc agcaccgtcc gtttcacctg caaggaggcc actgacgtca tcatcatcca 660

cagcaagaag ctcaactaca ccctcagcca ggggcacagg gtggtcctgc gtggtgtggg 720

aggctcccag ccccccgaca ttgacaagac tgagctggtg gagcccaccg agtacctggt 780

ggtgcacctc aagggctccc tggtgaagga cagccagtat gagatggaca gcgagttcga 840

gggggagttg gcagatgacc tggcgggctt ctaccgcagc gagtacatgg agggcaatgt 900

cagaaaggtg gtggccacta cacagatgca ggctgcagat gcccggaagt ccttcccatg 960

cttcgatgag ccggccatga aggccgagtt caacatcacg cttatccacc ccaaggacct 1020

gacagccctg tccaacatgc ttcccaaagg tcccagcacc ccacttccag aagaccccaa 1080

ctggaatgtc actgagttcc acaccacgcc caagatgtcc acgtacttgc tggccttcat 1140

tgtcagtgag ttcgactacg tggagaagca ggcatccaat ggtgtcttga tccggatctg 1200

ggcccggccc agtgccattg cggcgggcca cggcgattat gccctgaacg tgacgggccc 1260

catccttaac ttctttgctg gtcattatga cacaccctac ccactcccaa aatcagacca 1320

gattggcctg ccagacttca acgccggcgc catggagaac tggggactgg tgacctaccg 1380

ggagaactcc ctgctgttcg accccctgtc ctcctccagc agcaacaagg agcgggtggt 1440

cactgtgatt gctcatgagc tggcccacca gtggttcggg aacctggtga ccatagagtg 1500

gtggaatgac ctgtggctga acgagggctt cgcctcctac gtggagtacc tgggtgctga 1560

ctatgcggag cccacctgga acttgaaaga cctcatggtg ctgaatgatg tgtaccgcgt 1620

gatggcagtg gatgcactgg cctcctccca cccgctgtcc acacccgcct cggagatcaa 1680

cacgccggcc cagatcagtg agctgtttga cgccatctcc tacagcaagg gcgcctcagt 1740

cctcaggatg ctctccagct tcctgtccga ggacgtattc aagcagggcc tggcgtccta 1800

cctccacacc tttgcctacc agaacaccat ctacctgaac ctgtgggacc acctgcagga 1860

ggctgtgaac aaccggtcca tccaactccc caccaccgtg cgggacatca tgaaccgctg 1920

gaccctgcag atgggcttcc cggtcatcac ggtggatacc agcacgggga ccctttccca 1980

ggagcacttc ctccttgacc ccgattccaa tgttacccgc ccctcagaat tcaactacgt 2040

gtggattgtg cccatcacat ccatcagaga tggcagacag cagcaggact actggctgat 2100

agatgtaaga gcccagaacg atctcttcag cacatcaggc aatgagtggg tcctgctgaa 2160

cctcaatgtg acgggctatt accgggtgaa ctacgacgaa gagaactgga ggaagattca 2220

gactcagctg cagagagacc actcggccat ccctgtcatc aatcgggcac agatcattaa 2280

tgacgccttc aacctggcca gtgcccataa ggtccctgtc actctggcgc tgaacaacac 2340

cctcttcctg attgaagaga gacagtacat gccctgggag gccgccctga gcagcctgag 2400

ctacttcaag ctcatgtttg accgctccga ggtctatggc cccatgaaga actacctgaa 2460

gaagcaggtc acacccctct tcattcactt cagaaataat accaacaact ggagggagat 2520

cccagaaaac ctgatggacc agtacagcga ggttaatgcc atcagcaccg cctgctccaa 2580

cggagttcca gagtgtgagg agatggtctc tggccttttc aagcagtgga tggagaaccc 2640

caataataac ccgatccacc ccaacctgcg gtccaccgtc tactgcaacg ctatcgccca 2700

gggcggggag gaggagtggg acttcgcctg ggagcagttc cgaaatgcca cactggtcaa 2760

tgaggctgac aagctccggg cagccctggc ctgcagcaaa gagttgtgga tcctgaacag 2820

gtacctgagc tacaccctga acccggactt aatccggaag caggacgcca cctctaccat 2880

catcagcatt accaacaacg tcattgggca aggtctggtc tgggactttg tccagagcaa 2940

ctggaagaag ctttttaacg attatggtgg tggctcgttc tccttctcca acctcatcca 3000

ggcagtgaca cgacgattct ccaccgagta tgagctgcag cagctggagc agttcaagaa 3060

ggacaacgag gaaacaggct tcggctcagg cacccgggcc ctggagcaag ccctggagaa 3120

gacgaaagcc aacatcaagt gggtgaagga gaacaaggag gtggtgctcc agtggttcac 3180

agaaaacagc aaatagtccc cagcccttga agtcacccgg cccccatgca aggtgcccac 3240

atgtgtccat cccagcggct ggtgcagggc ctccattcct ggagcccgag gcaccagtgt 3300

cctcccctca aggacaaagt ctccagccca cgttctctct gcctgtgagc cagtctagtt 3360

cctgatgacc caggctgcct gagcacctcc cagcccctgc ccctcatgcc aaccccgccc 3420

taggcctggc atggcacctg tcgcccagtg ccctggggct gatctcaggg aagcccagct 3480

ccagggccag atgagcagaa gctctcgatg gacaatgaac ggccttgctg ggggccgccc 3540

tgtaccctct ttcacctttc cctaaagacc ctaaatctga ggaatcaaca gggcagcaga 3600

tctgtatatt tttttctaag agaaaatgta aataaaggat ttctagatga aaaaaaaaaa 3660

aaaaaaaaaa aaaaaaaaaa aaaaaaaaaa aaaaaaaaaa aaaaaaaaaa aaaaaaaaaa 3720

aaaaaaaaaa aaaaaaaaaa 3740

<210> 4
<211> 3033
<212> DNA
<213> Homo sapiens

<400> 4
aacacaactg gcacatctct tttctcatct cttgaaaaaa accaacagag aaaaaagtac 60

cttgagaata aaggtaatga ttaatctgtc aggcacaaaa gggattgttt tggggatttc 120

gggttctaag tcgcagattc aaacaaatag cagcgaacag ggaatgacag ttccaccaga 180

agacgattaa gccacagcct ctaattggaa cggcatttgt acagtcagag actcttacca 240

gacatctcca ggaatctgtg agccattgtc aaaacgtcca ttttcatctg gctgtgaaag 300

tgaggaccac aacaggtagg tattggtaga aacaggagtc ctcagagaag ccccaagatg 360

cagcctgagg gagcagaaaa gggaaaaagc ttcaagcaga gactggtctt gaagagcagc 420

ttagcgaaag aaaccctctc tgagttcttg ggcacgttca tcttgattgt ccttggatgt 480

ggctgtgttg cccaagctat tctcagtcga ggacgttttg gaggggtcat cactatcaat 540
gttggatttt caatggcagt tgcaatggcc atttatgtgg ctggcggtgt ctctggtggt 600
cacatcaacc cagctgtgtc tttagcaatg tgtctctttg gacggatgaa atggttcaaa 660
ttgccatttt atgtgggagc ccagttcttg ggagcctttg tgggggctgc aaccgtcttt 720
ggcatttact atgatggact tatgtccttt gctggtggaa aactgctgat cgtgggagaa 780
aatgcaacag cacacatttt tgcaacatac ccagctccgt atctatctct ggcgaacgca 840
tttgcagatc aagtggtggc caccatgata ctcctcataa tcgtctttgc catctttgac 900
tccagaaact tgggagcccc cagaggccta gagcccattg ccatcggcct cctgattatt 960
gtcattgctt cctccctggg actgaacagt ggctgtgcca tgaacccagc tcgagacctg 1020
agtcccagac ttttcactgc cttggcaggc tgggggtttg aagtcttcag agctggaaac 1080
aacttctggt ggattcctgt agtgggccct ttggttggtg ctgtcattgg aggcctcatc 1140
tatgttcttg tcattgaaat ccaccatcca gagcctgact cagtctttaa gacagaacaa 1200
tctgaggaca aaccagagaa atatgaactc agtgtcatca tgtagtggca tgctcagctc 1260
tggatttgca gtcagtttgg gattctcttc agaaagatgg catctaagtg tctgtgttct 1320
tgtaagcctg aggtggaatc cacccagttt tgtctgctag ccatatggga catctaattg 1380
gaaaagcatc tgcataaaag tttggaaaca atgaccactt ctctaccatt gtcccccacc 1440
cccacccccc agaataacgc tgactgtccc ctgaaacagc cttctctcct gccctgttta 1500
tttcatcctc gatgggaatt cttgctaggt aagcactaat aactcggcat cttgacgata 1560
gtcccatttg ggtggtttca gctgcactat ctgtatgaaa tggtgtcacc aaaacccttt 1620
tcttcagtat cgacaaagat tacattctga gtaccaacca aaccctaaat tgaaagacaa 1680
aactatggtt tcagtcaaca tattcatgaa ttagggagct aatgggttaa gcttccagtt 1740
cccgctatgc tactggattt gtataaatac tgatattctc caaacctagt ggtgtaggga 1800
gcaagagaat gcagctggaa ggcacaaggg gaggacattg tggcattcag aaactgcagg 1860
agacaagatg aatttgagaa gccaaatgga atttttaatg gaaaccattt atcagattaa 1920
tctcttgctc tcctgcattt tagaggacac caattaattt cctggtcttt agtatataat 1980
aacctaaaat accattgtaa cctcagtcat gaaaaataca tcactctgtc tttttagctc 2040
aaatgtattt tcctaattgc ccacttgaga acagacattt gacaagttat atcaacgact 2100
gtgcttgtcc attattttac acatgcccta gaagccaaaa ctgaaagcca ctggatcctg 2160
gtctagctga atcttcagag tgggaggtct ccaaaaagat attaccttat tgggcttaac 2220
aattcacaag gcactttcac acccattatc taatttaatc ctcataatga ctatgtgagg 2280
caaatgccac attgcccatt tttcagataa agaaacaaaa tcttagggaa gataagttga 2340
gttgtccaag agcacactga aagttgaatg ttatctaatg cattcctcta cctttcagaa 2400

gatcagtagc tggctgagaa tctttgccaa atcttccttg ctagccagaa gtggaattgg 2460

cagcttctag aatatgtaca cctctggaca aaatgttcct caatcttaag atacaaagac 2520

cctcattgtc tgggtctatt cccacactta ctgagtacag atgaaggaaa gtggtagcaa 2580

tttaatcata actttcattt gctgaaaaac attatgagaa ggcctccctt cctaagccac 2640

ctctggtctt gctaagtctt gatcttgctt cctgccagca ccaaacatta cattcagggg 2700

atttcctctg gctcagtctt ttccccttga agttctctaa tagatgttac ttttgacaaa 2760

agatcgccta tgagttacaa gcaccagggg atgctctaca tcaagggatg caccttcagt 2820

caaactgtca aaaagcccag aattcccaaa ggcattaggt ttcccaactg ctttgtgctg 2880

atatcagaac agcagaaatt aaatgtgaaa tgtttctgat gacttatgtt ctacaatcta 2940

tggacatacg ggattttttt ttcttgcttt gaagctacct ggatatttcc tatttgaaat 3000

aaaattgttc ggtcattgtt gaaaaaaaaa aaa 3033

<210> 5
<211> 979
<212> DNA
<213> Homo sapiens

<400> 5
gagttaggtg acgctgcggg gcgggcggac agactgcggg acggacggtg gacgctggga 60

cgcgtttgta gctccggccc cgccgttccg acccccgccg ccgtcgccgc catgacgggg 120

ctagcactgc tctactccgg ggtcttcgtg gccttctggg cctgcgcgct ggccgtgggt 180

tcctgacgga gacttcgccc ttcatgtggt ccaacctggg cattggccta gctatctccc 240

tgtctgtggt tggggcagcc tggggcatct atattaccgg ctcctccatc attggtggag 300

gagtgaaggc ccccaggatc aagaccaaga acctggtcag catcatcttc tgtgaggctg 360

tggccatcta cggcatcatc atggcaattg tcattagcaa catggctgag cctttcagtg 420

ccacagaccc caaggccatc ggccatcgga actaccatgc aggctactcc atgtttgggg 480

ctggcctcac cgtaggcctg tctaacctct tctgtggagt ctgcgtgggc atcgtgggca 540

gtggggctgc cctggccgat gctcagaacc ccagcctctt tgtaaagatt ctcatcgtgg 600

agatctttgg cagcgccatt ggcctctttg gggtcatcgt cgcaattctt cagacctcca 660

gagtgaagat gggtgactag atgatatgtg tgggtggggc cgtgcctcac ttttatttat 720

tgctggtttt cctgggacag ctggagctgt gtcccttagc ctttcagagg cttggtgttc 780

agggccctcc ctgcactccc ctcttgctgc gtgttgattt ggaggcactg cagtccaggc 840

cgagtcctca gtgcggggag caggctgctg ctgctgactc tgtgcagctg cgcacctgtg 900

tcccccacct ccaccctcaa cccatcttcc tagtgtttgt gaaataaact tggtatttgt 960

ctgggtcagt gcaaaaaaa 979

<210>  6
<211>  1727
<212>  DNA
<213>  Homo sapiens

<400>  6
gcgcaactcg tttgcagcgg cgcagcccag acgcgcctgc agctggggct caccccaacc      60

tcgctgccag ccgagaactc caagatggga ggcaagctca gcaagaagaa gaagggctac     120

aatgtgaacg acgagaaagc caaggagaaa gacaagaagg ccgagggcgc ggcgacggaa     180

gaggagggga ccccgaagga gagtgagccc caggcggccg cagagcccgc cgaggccaag     240

gagggcaagg agaagcccga ccaggacgcc gagggcaagg ccgaggagaa ggagggcgag     300

aaggacgcgg cggctgccaa ggaggaggcc ccgaaggcgg agcccgagaa gacggagggc     360

gcggcagagg ccaaggctga gcccccgaag gcgcccgagc aggagcaggc ggcccccggc     420

cccgctgcgg gcggcgaggc ccccaaagct gctgaggccg ccgcggcccc ggccgagagc     480

gcggcccctg ccgccgggga ggagcccagc aaggaggaag gggaacccaa aaagactgag     540

gcgcccgcag ctcctgccgc ccaggagacc aaaagtgacg gggccccagc ttcagactca     600

aaacccggca gctcggaggc tgccccctct tccaaggaga cccccgcagc cacggaagcg     660

cctagttcca cacccaaggc ccagggcccc gcagcctctg cagaagagcc caagccggtg     720

gaggccccgg cagctaattc cgaccaaacc gtaaccgtga aagagtgaca aggacagcct     780

ataggaaaaa caataccact taaaacaatc tcctctctct ctctctctct ctctctctat     840

ctctctctct atctcctctc tctctctcct ctcctatctc tcctctctct ctctcctata     900

ctaacttgtt tcaaattgga agtaatgata tgtattgccc aaggaaaaat acaggatgtt     960

gtcccatcaa gggagggagg gggtgggaga atccaaatag tatttttgtg gggaaatatc    1020

taatatacct tcagtcaact ttaccaagaa gtcctggatt tccaagatcc gcgtctgaaa    1080

gtgcagtaca tcgtttgtac ctgaaactgc cgccacatgc actcctccac cgctgagagt    1140

tgaatagctt ttcttctgca atgggagttg ggagtgatgc gtttgattct gcccacaggg    1200

cctgtgccaa ggcaatcaga tctttatgag agcagtattt tctgtgtttt ctttttaatt    1260

tacagccttt cttattttga tattttttta atgttgtgga tgaatgccag ctttcagaca    1320

gagcccactt agcttgtcca catggatctc aatgccaatc ctccattctt cctctccaga    1380

tatttttggg agtgacaaac attctctcat cctacttagc ctacctagat ttctcatgac    1440

gagttaatgc atgtccgtgg ttgggtgcac ctgtagttct gtttattggt cagtggaaat    1500

gaaaaaaaaa aaaaaaaaaa gtctgcgttc attgcagttc cagtttctct tccattctgt    1560

gtcacagaca ccaacacacc actcattgga aaatggaaaa aaaaaacaaa aaaaaaacaa    1620

aaaaatgtac aatggatgca ttgaaattat atgtaattgt ataaatggtg caacagtaat    1680

aaagttaaac aattaaaaag aagtaataaa gacaaaaaaa aaaaaaa 1727

<210> 7
<211> 2852
<212> DNA
<213> Homo sapiens

<400> 7
caaggctggg accagaaacc aggactgttg actgcagccc ggtattcatt ctttccatag 60

cccacagggc tgtcaaagac cccagggcct agtcagaggc tcctccttcc tggagagttc 120

ctggcacaga agttgaagct cagcacagcc ccctaacccc caactctctc tgcaaggcct 180

caggggtcag aacactggtg gagcagatcc tttagcctct ggattttagg gccatggtag 240

agggggtgtt gccctaaatt ccagccctgg tctcagccca acaccctcca agaagaaatt 300

agaggggcca tggccaggct gtgctagccg ttgcttctga gcagattaca agaagggact 360

aagacaagga ctcctttgtg gaggtcctgg cttagggagt caagtgacgg cggctcagca 420

ctcacgtggg cagtgccagc ctctaagagt gggcaggggc actggccaca gagtcccagg 480

gagtcccacc agcctagtcg ccagaccttc tgtgggatca tcggacccac ctggaacccc 540

acctgctggc cctcacggaa gaacaacagc tgatgtttga gaaactgact ctgtattgcg 600

acagctacat ccagctcatc cccatttcct tcgtgctggg cttctacgtg acgctggtcg 660

tgacccgctg gtggaaccag tacgagaacc tgccgtggcc cgaccgcctc atgagcctgg 720

tgtcgggctt cgtcgaaggc aaggacgagc aaggccggct gctgcggcgc acgctcatcc 780

gctacgccaa cctgggcaac gtgctcatcc tgcgcagcgt cagcaccgca gtctacaagc 840

gcttccccag cgcccagcac ctggtgcaag caggctttat gactccggca gaacacaagc 900

agttggagaa actgagccta ccacacaaca tgttctgggt gccctgggtg tggtttgcca 960

acctgtcaat gaaggcgtgg cttggaggtc gaatccggga ccctatcctg ctccagagcc 1020

tgctgaacga gatgaacacc ttgcgtactc agtgtggaca cctgtatgcc tacgactgga 1080

ttagtatccc actggtgtat acacaggtgg tgactgtggc ggtgtacagc ttcttcctga 1140

cttgtctagt tgggcggcag tttctgaacc cagccaaggc ctaccctggc catgagctgg 1200

acctcgttgt gcccgtcttc acgttcctgc agttcttctt ctatgttggc tggctgaagg 1260

tggcagagca gctcatcaac ccctttggag aggatgatga tgattttgag accaactgga 1320

ttgtcgacag gaatttgcag gtgtccctgt tggctgtgga tgagatgcac caggacctgc 1380

ctcggatgga gccggacatg tactggaata agcccgagcc acagcccccc tacacagctg 1440

cttccgccca gttccgtcga gcctccttta tgggctccac cttcaacatc agcctgaaca 1500

aagaggagat ggagttccag cccaatcagg aggacgagga ggatgctcac gctggcatca 1560

ttggccgctt cctaggcctg cagtcccatg atcaccatcc tcccagggca aactcaagga 1620

ccaaactact gtggcccaag agggaatccc ttctccacga gggcctgccc aaaaaccaca 1680

aggcagccaa acagaacgtt aggggccagg aagacaacaa ggcctggaag cttaaggctg 1740

tggacgcctt caagtctgcc ccactgtatc agaggccagg ctactacagt gccccacaga 1800

cgcccctcag ccccactccc atgttcttcc ccctagaacc atcagcgccg tcaaagcttc 1860

acagtgtcac aggcatagac accaaagaca aaagcttaaa gactgtgagt tctggggcca 1920

agaaaagttt tgaattgctc tcagagagcg atggggcctt gatggagcac ccagaagtat 1980

ctcaagtgag gaggaaaact gtggagttta acctgacgga tatgccagag atccccgaaa 2040

atcacctcaa agaacctttg gaacaatcac caaccaacat acacactaca ctcaaagatc 2100

acatggatcc ttattgggcc ttggaaaaca ggtctgtcct ccacctgaac caggggcact 2160

gcattgccct gtgccccacc ccagcttccc ttgctctgag cctacccttc ctccacaatt 2220

tcctagggtt ccatcactgc cagagcacac tggacctacg cccagcactg gcttggggta 2280

tatacttggc caccttcaca gggatcctag ggaagtgttc gggacctttt ctcacttcac 2340

cctggtatca cccggaagac ttcttgggac caggtgaagg aagatgaggt tgtgctgacc 2400

agaatgctgc tggagaactg ccccagggct gacaggccag gcttagctga gcagatgtta 2460

tcactggccc caacttactt tgagcaaggg tggctgaccc aaaaccatga ggtggcagtc 2520

agctggatga cagatgaaca cttcccccat aactatttag ggtagtaccc aagcactaca 2580

ggaaagggtg gcaggaactg cctcactcct aggaactggt agatggtgag gttgagggtg 2640

tccagcgccc ttaggtcatt ttctcactgc ctgggaacct caccaaaata cttcttgctt 2700

ccttggggtc agcccaaagc tgtcacaaaa tcagatattt ccctttattc cagatttcct 2760

ggacactttc acccaattat aaacacccca cttcagcccc aatcacgtgg gaggaagtgt 2820

aacttccctt ttaaaaaaaa aaaaaaaaaa aa 2852

<210> 8
<211> 1238
<212> DNA
<213> Homo sapiens

<400> 8
agactaggat ccctggaaaa tggagaagct gtgctaatag agggggccca gaaatcccca 60

ctctagaatg ctgtagaatg ttgggagaca cccaggatgt gagccaggga ctttctggaa 120

gtgtttgttc tggccccacc cgaccccagg cagtccccag ctgtctgcac agtcggatgg 180

ggagggggct tgcacagagt tggagccaga ggagagagct ggctcatccc ctacggtagg 240

atggggaaac ctcacagacc acattgtcac ccggcctcag ctctccgccc cggcgctcag 300

agggtaactc tcacccacct cgtccgcttc tctgaaccag agtgacccag gctgcgctcc 360

gccccgctct cctaccccga gttggcacgg aggcccggca gccatggcgg tggaaggagg 420

aatgaaatgt gtgaagttct tgctctacgt cctcctgctg gccttttgcg cctgtgcagt 480

gggactgatt gccgtgggtg tcggggcaca gcttgtcctg agtcagacca taatccaggg 540

ggctacccct ggctctctgt tgccagtggt catcatcgca gtgggtgtct tcctcttcct 600

ggtggctttt gtgggctgct gcggggcctg caaggagaac tattgtctta tgatcacgtt 660

tgccatcttt ctgtctctta tcatgttggt ggaggtggcc gcagccattg ctggctatgt 720

gtttagagat aaggtgatgt cagagtttaa taacaacttc cggcagcaga tggagaatta 780

cccgaaaaac aaccacactg cttcgatcct ggacaggatg caggcagatt ttaagtgctg 840

tggggctgct aactacacag attgggagaa aatcccttcc atgtcgaaga accgagtccc 900

cgactcctgc tgcattaatg ttactgtggg ctgtgggatt aatttcaacg agaaggcgat 960

ccataaggag ggctgtgtgg agaagattgg gggctggctg aggaaaaatg tgctggtggt 1020

agctgcagca gcccttggaa ttgcttttgt cgaggttttg ggaattgtct ttgcctgctg 1080

cctcgtgaag agtatcagaa gtggctacga ggtgatgtag gggtctggtc tcctcagcct 1140

cctcatctgg gggagtggaa tagtatcctc caggtttttc aattaaacgg attatttttt 1200

cagaccgaaa agagatggtc tgagtttgtc ttagagtg 1238

<210> 9
<211> 1790
<212> DNA
<213> Homo sapiens

<400> 9
taattacaaa aactaatgac taagagagag gtggctagag ctgaggcccc tgagtcaggc 60

tgtgggtggg atcatctcca gtacaggaag tgagactttc atttcctcct ttccaagaga 120

gggctgaggg agcagggttg agcaactggt gcagacagcc tagctggact ttgggtgagg 180

cggttcagcc atgaggctgg ctgtgctttt ctcgggggcc ctgctggggc tactggcaga 240

gagcactgga acaaccagcc acaggactac caagagccac aaaaccacca ctcacaggac 300

aaccaccaca ggcaccacca gccacggacc cacgactgcc actcacaacc ccaccaccac 360

cagccatgga aacgtcacag ttcatccaac aagcaatagc actgccacca gccagggacc 420

ctcaactgcc actcacagtc ctgccaccac tagtcatgga aatgccacgg ttcatccaac 480

aagcaacagc actgccacca gcccaggatt caccagttct gcccacccag aaccacctcc 540

accctctccg agtcctagcc caacctccaa ggagaccatt ggagactaca cgtggaccaa 600

tggttcccag ccctgtgtcc acctccaagc ccagattcag attcgagtca tgtacacaac 660

ccagggtgga ggagaggcct ggggcatctc tgtactgaac cccaacaaaa ccaaggtcca 720

gggaagctgt gagggtgccc atccccacct gcttctctca ttcccctatg gacacctcag 780

ctttggattc atgcaggacc tccagcagaa ggttgtctac ctgagctaca tggcggtgga 840

gtacaatgtg tccttccccc acgcagcaca gtggacattc tcggctcaga atgcatccct 900

tcgagatctc caagcacccc tggggcagag cttcagttgc agcaactcga gcatcattct 960

ttcaccagct gtccacctcg acctgctctc cctgaggctc caggctgctc agctgcccca 1020

cacaggggtc tttgggcaaa gtttctcctg ccccagtgac cggtccatct tgctgcctct 1080

catcatcggc ctgatccttc ttggcctcct cgccctggtg cttattgctt tctgcatcat 1140

ccggagacgc ccatccgcct accaggccct ctgagcattt gcttcaaacc ccagggcact 1200

gagggggttg gggtgtggtg ggggggtacc cttatttcct cgacacgcaa ctggctcaaa 1260

gacaatgtta ttttccttcc ctttcttgaa gaacaaaaag aaagccgggc atgacggctc 1320

atgcctgtaa tcccagcact ttgggaggct gaggcaggtg gatcactgga ggtcaggagt 1380

ttgagaccag cctggccaac atggtgaaac cctgtctcta ctaaaaatac aattagccag 1440

gtgtggcggc gtaatcccag ctggcctgta atcccagcta cttgggaggc tgaggcagaa 1500

ctgcttgaac ccaggaggtg gaggttgcag tgagccgtca tcgcgccact aagccaagat 1560

cgcgccactg cactccagcc tgggcgacag agccagactg tctcaaataa ataaatatga 1620

gataatgcag tcgggagaag ggagggagag aattttatta aatgtgacga actgcccccc 1680

cccccccccc agcaggagag cagcaaaatt tatgcaaatc tttgacgggg ttttccttgt 1740

cctgccagga ttaaaagcca tgagtttctt gtcaaaaaaa aaaaaaaaaa 1790

<210> 10
<211> 6017
<212> DNA
<213> Homo sapiens

<400> 10
gggcggggct cgggccggtc cgcccgcgcg caggtgagtg agccagggcg gagcgcagct 60

gcgccgggct tgggcgcctg gggccgccgc tccccaccgt cgttttcccc accgaggccg 120

aggcgtcccg gagtcatggc cggcctgaac tgcggggtct ctatcgcact gctaggggtt 180

ctgctgctgg gtgcggcgcg cctgccgcgc ggggcagaag cttttgagat tgctctgcca 240

cgagaaagca acattacagt tctcataaag ctggggaccc cgactctgct ggcaaaaccc 300

tgttacatcg tcatttctaa aagacatata accatgttgt ccatcaagtc tggagaaaga 360

atagtcttta cctttagctg ccagagtcct gagaatcact ttgtcataga gatccagaaa 420

aatattgact gtatgtcagg cccatgtcct tttggggagg ttcagcttca gccctcgaca 480

tcgttgttgc ctaccctcaa cagaactttc atctgggatg tcaaagctca taagagcatc 540

ggtttagagc tgcagttttc catccctcgc ctgaggcaga tcggtccggg tgagagctgc 600

ccagacggag tcactcactc catcagcggc cgaatcgatg ccaccgtggt caggatcgga 660

accttctgca gcaatggcac tgtgtcccgg atcaagatgc aagaaggagt gaaaatggcc 720

ttacacctcc catggttcca ccccagaaat gtctccggct tcagcattgc aaaccgctca 780

tctataaaac gtctgtgcat catcgagtct gtgtttgagg gtgaaggctc agcaaccctg 840

atgtctgcca actacccaga aggcttccct gaggatgagc tcatgacgtg gcagtttgtc 900

gttcctgcac acctgcgggc cagcgtctcc ttcctcaact tcaacctctc caactgtgag 960

aggaaggagg agcgggttga atactacatc ccgggctcca ccaccaaccc cgaggtgttc 1020

aagctggagg acaagcagcc tgggaacatg gcggggaact tcaacctctc tctgcaaggc 1080

tgtgaccaag atgcccaaag tccagggatc ctccggctgc agttccaagt tttggtccaa 1140

catccacaaa atgaaagcaa taaaatctac gtggttgact tgagtaatga gcgagccatg 1200

tcactcacca tcgagccacg gcccgtcaaa cagagccgca agtttgtccc tggctgtttc 1260

gtgtgtctag aatctcggac ctgcagtagc aacctcaccc tgacatctgg ctccaaacac 1320

aaaatctcct tcctttgtga tgatctgaca cgtctgtgga tgaatgtgga aaaaaccata 1380

agctgcacag accaccggta ctgccaaagg aaatcctact cactccaggt gcccagtgac 1440

atcctccacc tgcctgtgga gctgcatgac ttctcctgga agctgctggt gcccaaggac 1500

aggctcagcc tggtgctggt gccagcccag aagctgcagc agcatacaca cgagaagccc 1560

tgcaacacca gcttcagcta cctcgtggcc agtgccatac ccagccagga cctgtacttc 1620

ggctccttct gcccgggagg ctctatcaag cagatccagg tgaagcagaa catctcggtg 1680

acccttcgca cctttgcccc cagcttccaa caagaggcct ccaggcaggg tctgacggtg 1740

tcctttatac cttatttcaa agaggaaggc gttttcacgg tgacccctga cacaaaaagc 1800

aaggtctacc tgaggacccc caactgggac cggggcctgc catccctcac ctctgtgtcc 1860

tggaacatca gcgtgcccag agaccaggtg gcctgcctga ctttctttaa ggagcggagc 1920

ggcgtggtct gccagacagg gcgcgcattc atgatcatcc aggagcagcg gacccgggct 1980

gaggagatct tcagcctgga cgaggatgtg ctccccaagc caagcttcca ccatcacagc 2040

ttctgggtca acatctctaa ctgcagcccc acgagcggca agcagctaga cctgctcttc 2100

tcggtgacac ttaccccaag gactgtggac ttgactgtca tcctcatcgc agcggtggga 2160

ggtggagtct tactgctgtc tgccctcggg ctcatcattt gctgtgtgaa aaagaagaaa 2220

aagaagacaa acaagggccc cgctgtgggt atctacaatg acaacatcaa tactgagatg 2280

ccgaggcagc caaaaaagtt tcagaaaggg cgaaaggaca atgactccca tgtgtatgca 2340

gtcatcgagg acaccatggt atatgggcat ctgctacagg attccagcgg ctccttcctg 2400

cagccagagg tggacaccta ccggccgttc cagggcacca tgggggtctg tcctccctcc 2460

ccacccacca tatgctccag ggccccaact gcaaagttgg ccactgagga gccacctcct 2520

cgctcccctc ctgagtctga gagtgaaccg tacaccttct cccatcccaa caatggggat 2580

gtaagcagca aggacacaga cattccctta ctgaacactc aggagcccat ggagccagca 2640

gaataacttg atccattcca gacgctttgc tgagtttcat aaagcagggc actgagacac 2700

ccgtccgtgt tcctaaccag aaatcctaaa gaagaggaat tatacagaag gaacagcagg 2760

aggttttcct ggacaccgcc aacttcacat tgctcagtgg actcattcta agggcaagac 2820

attgaaaatg atgaattcca atctggatac agtcatgaca gctcatgtgc tcctcaactt 2880

aggctgtgcg gttagccagc ctgtaatgag aggagagagg cctgagtcac ctagcatagg 2940

gttgcagcaa gccctggatt cagagtgtta aacagaggct tgccctcttc aggacaacag 3000

ttccaattcc aaggagccta cctgaggtcc ctactctcac tggggtcccc aggatgaaaa 3060

cgacaatgtg cctttttatt attatttatt tggtggtcct gtgttattta agagatcaaa 3120

tgtataacca cctagctctt ttcacctgac ttagtaataa ctcatactaa ctggtttgga 3180

tgcctgggtt gtgacttcta ctgaccgcta gataaacgtg tgcctgtccc ccaggtggtg 3240

ggaataattt acaatctgtc caaccagaaa agaatgtgtg tgtttgagca gcattgacac 3300

atatctgctt tgataagaga cttcctgatt ctctaggtcg gttcgtggtt atcccattgt 3360

ggaaattcat cttgaatccc attgtcctat agtcctagca ataagagaaa tttcctcaag 3420

tttccatgtg cggttctcct agctgcagca atactttgac atttaaagag aaatttagag 3480

aatattctca tcctctaaaa atgtttaaat atataccaaa cagtggcccc ctgcattagt 3540

tttctgttgc cactgcaacc tattacttgg tagcttaaaa acaacacatt agcttatagt 3600

cctggggatc agaattccaa aatggatgtc cctgaatgaa aatcaaggtg tcagcagagc 3660

tgtgctcctt ctgaaggctc tagggagaag ccggttcctt gccatttcaa gcttctagag 3720

gctggctgca ttcccaggct ccagtggctg gtcaagcttt tctcacatgg catcactgtg 3780

acactggccc tcccacttcc ctctttgact tacaaagccc accaggaaga tccaggataa 3840

tctctccatc taaagttcct tcatcatcct ggaagagcct tttgccatgc aagacaacat 3900

agccacaggt ggggattagg accagaacat ctttggggtg ctgttattct gcctaccaca 3960

ccttcctgcc actgactccc acaggagagg ctacaaaatg atctggcgca cagggatgtt 4020

ttgtttagct tgcggactct aacacttaaa aaaaaaccca gatcagaaga tctggccatg 4080

ctggggctca cattctcacc tagcaacaac tggctggagc tgggcaccag ctctgccttt 4140

agaaggggtg tccacttcac caggtcacca cagcccacac tacgccctat cacttcccac 4200

aatgaggctg agtgtttgtt tctactgatc aatgcccctg caggttgcat ttattgtaat 4260

gaaaaagaaa gactgggatt aatctctaat caggtgagta gaccatgaga ccaatgtgtg 4320

ctcacattac cctttttctt ttttttcttt ttctttttct tttttttttt aatgtgagac 4380

aggatctcat tctgttgcct aggctggagt gcagtggcgc aatctcggct cactgcaacc 4440

tctgcctcct gggctcaagc aattctccca cctcagcctc ccaaatagct gggatcactg 4500

gcacaaacca ccatgcccag ctaattttgt attttttgta gagacagggt ttcaccatgt 4560

tgcccaggct ggtctcaacc tcctgggctc aagcaatcct cctgcctcgg cctcccaaag 4620

tgctgggatt acagatgtga gccaccgcat ccagccccac accctcattt ataccaatta 4680

cctgcccagt aactgtggac ttttgcttcc tcacccctgc tctgatctgg aaggagaggg 4740

attatgttat agcttgtcag cacagtccca agttcaatat ttctgcggca aaaacttcct 4800

tcaaaaaata aatgtacttc attgtattca atgaattcac cttggaaatg caccgcctca 4860

acttgttcac atggcataaa tgaaaggaat tttatagtct cctaaatggc gtgtactgca 4920

agacctcttg aacactttcc agaggatagg atatttaagt catgcccttg gcgttgccta 4980

tggcaccttt cccttctgaa agtctggttc ctgcccagtg acccttggcc ttgtgagccg 5040

agatgctgac cctgcataaa gggccaaagg agggctgcgg cttccttccc tcactgaaga 5100

gcccttattt gaattcactg tgtggagccc tagccctcca ttctcgacat tccccaacct 5160

cccagcccct tccaagcagg actaggtgcc ctgcattcca cccaaggtgg gattggcctt 5220

ccttaggctg gctacttgtc accatcaccg acatcactgt tgcctgcaag gacaccacgt 5280

ggccattttc cttcaactga gggctcaaaa ctcctggaca agttgctggc tcctgagacc 5340

agtatttcct ggagctgtgc ctcagtgaag gggcccagcc tgaggaaccc tggctctttt 5400

ctttaaagcc caggccccac ttacgtaaaa catttcaggg tcactggaaa cagtgaagtg 5460

ccatttgttg aagcctactg catgccagcc cactgctcat ccacgtggtc tgccatgcct 5520

acgaggaagg ccagcgcatg caggactggt ctctaatgct gtggtcattg cacagaaggg 5580

aaaggtctca aggaagagtc aactggaaca agcacaagcc caccggacat ggccttggta 5640

aaggttagca gactggtgtg tgtggatctg cagtgcttca ctggaaataa tttattcatt 5700

gcagatactt tttaggtggc attttattca tttcctgtgc tttaaataaa caaatgtacc 5760

aaaaaacaag tatcaagctg tttaagtgct tcggctactt gtcccctggt tcagtagagg 5820

ccccggtttc ccagttgttg actgtgacag gctcagcatg ggctcagcag atgctgtctt 5880

aatttgtgga tgatacagaa agccaggctt tgggatacaa gttctttcct cttcatttga 5940

tgccgtgcac tgtgtgaagc agatgttttt gtccggaaat aaaaataata gtcttggagt 6000

ctcgccaaaa aaaaaaa 6017

<210> 11
<211> 6651
<212> DNA
<213> Homo sapiens

<400> 11
ataacacccg gccccgccgg gcggccgcgg gtgggtagag aacatggact tcccgtgcct 60

ctggctaggg ctgttgctgc ctttggtagc tgcgctggat ttcaactacc accgccagga 120

agggatggaa gcgtttttga agactgttgc ccaaaactac agttctgtca ctcacttaca 180

cagtattggg aaatctgtga aaggtagaaa cctgtgggtt cttgttgtgg ggcggtttcc 240

aaaggaacac agaattggga ttccagagtt caaatacgtg gcaaatatgc atggagatga 300

gactgttggg cgggagctgc tgctccatct gattgactat ctcgtaacca gtgatggcaa 360

agaccctgaa atcacaaatc tgatcaatag tacccggata cacatcatgc cttccatgaa 420

cccagatgga tttgaagccg tcaaaaagcc tgactgttat tacagcatcg gaagggaaaa 480

ttataaccag tatgacttga atcgaaattt ccccgatgct tttgaatata ataatgtctc 540

aaggcagcct gaaactgtgg cagtcatgaa gtggctgaaa acagagacgt ttgtcctctc 600

tgcaaacctc catggtggtg ccctcgtggc cagttaccca tttgataatg gtgttcaagc 660

aactggggca ttatactccc gaagcttaac gcctgatgat gatgtttttc aatatcttgc 720

acatacctat gcttcaagaa atcccaacat gaagaaagga gacgagtgta aaaacaaaat 780

gaactttcct aatggtgtta caaatggata ctcttggtat ccactccaag gtggaatgca 840

agattacaac tacatctggg cccagtgttt tgaaattacg ttggagctgt catgctgtaa 900

atatcctcgt gaggagaagc ttccatcctt ttggaataat aacaaagcct cattaattga 960

atatataaag caggtgcacc taggtgtaaa gggtcaagtt tttgatcaga atggaaatcc 1020

attacccaat gtaattgtgg aagtccaaga cagaaaacat atctgcccct atagaaccaa 1080

caaatatgga gagtattatc tccttctctt gcctgggtct tatataataa atgttacagt 1140

ccctggacat gatccacaca tcacaaaggt gattattccg gagaaatccc agaacttcag 1200

tgctcttaaa aaggatattc tacttccatt ccaagggcaa ttggattcta tcccagtatc 1260

aaatccttca tgcccaatga ttcctctata cagaaatttg ccagaccact cagctgcaac 1320

aaagcctagt ttgttcttat ttttagtgag tcttttgcac atattcttca aataaagtaa 1380

aatgtgaaac tcaacccaca tcaccacctg gaatcaggga ttgctcactc caggttactg 1440

caaccctaac tcactctagt gggaccttga ctggagaaac tccacgatct tcctgaagaa 1500

gagaaatgga tgtttccaaa ttccacaata agcaatatgt ggtgataatg aaaagaatga 1560

ttcagtcttg acggtgaatg gaagacactt acctaacaag tactgctcat ttacactcaa 1620

attaatcttg aagtagtctt aaaatgtgta agaagttaaa acttgagaag caaaaaaatg 1680

cctgcaaaaa gaagatcatt ttgtatacag agaaccggat gaatataagc aatgaagatg 1740

aacatttatt gatcttctac atacaagact tcaccataag gccaggagca gtggctcaca 1800

ccttgtaatc ccagcacttt gggaggccaa ggtgggcgga tcaccctgag gtcaggagtt 1860

caaaaccagc ctgaccaaca tggtgaaacc ctgtctctac taaatattag cggggtgtgg 1920

tggcgggcac ctgtaatcgc agcctttcag gaggctgaga caggagaatc gcttgaaccc 1980

tagaggcgga gtttgcagtg agccgagata gtgccattgt actccagctt gggcaacaga 2040

gtaagactct gtctcaaaaa aaaaaaaaca aaaacaaaca aacaaaaaaa acacctcacc 2100

atgagtgcta catgtgaata gatattaagt gccatatata attagttctc agaagaaggg 2160

agaaatgatc ataggactgg gaattgtttt gcaaacgttc taggagatgt gagagaaaat 2220

atgtaaccac atcttagtgg cccaagaaaa tacaggcctg aagggataag attgtgtctc 2280

tatagagctt caaagcatac aggtcaatta agaaagcccc tctctctcca gagccgtttc 2340

cctagctttt ggcacctgga tgccacagtc ctccattagg ctgatgactc caaagatgta 2400

actctagcct cttgcctgag cttcagactc gcgtcccact gcccacagga cacatccacc 2460

tggatgtgac tcacaggtac ctccaaccca tcatgtggag atactcatcc tgttccccct 2520

agagctgctc ttcctgctgc attctctctc tcaattactg ggaccaccaa gctaggaacc 2580

tgggagtcat ccttgatact ttctcttcct ccttaatcct gtgtattcag caagtaacta 2640

aaggttggtg ttggccaggc atggtggctc atgcctgtaa tcccagcatt ttgggaggcc 2700

aaggcgggcg gatcacttga ggtcaggagc tcaagaccag cctggccaac atggtgaaac 2760

cccatctcta ctaaaaaaaa aaaaaaaatt agtcgggcgt ggtggtgcat gcctgtaatc 2820

ccagctactg gggaggctga ggcaggagaa tcgcttgaac ctgggaggca gaggttgcag 2880

tgagccggga ttgcgccatt gtactccagc ctgggtgaag aagtgagact ctgtcttaaa 2940

aaaaaaaatt ggtgctgata aatattgatg aattctgctc tctgctctct atggttgtca 3000

acactgcaga gttgaggcct catatctcac ctgcactgct gcaacagctt actggtccct 3060

tgctcccagc cttctcctct tcagtccatc gtccacacag cactggggaa ggggagccac 3120

ttgaaacaaa agtcaacaac tggttgtagt tcataaacac agagctgttt gtgtcccctg 3180

tatctggaat gccattatga cccactacat tttttctttc ctacccctct taaaactcag 3240

ttcaggtagc agctccacta ggaagccttg gctgaccata atcccattca attccatttc 3300

acctcttcgc aggcagtctg gggttaggga ccctttctct ttgctcccca aaataaactg 3360

gttatctcta ctattggatt tacaacattg tattataatc ttctccatgt gtgccttctc 3420

tagtagaatg tgagctcttt gaggccaagg tctatttaat ttgtttgaaa aattcattgt 3480

tatatcctca aagcctagca catagtaggt actgaatgaa tgaatgaaca aggggtgcca 3540

ggagactgct actcccagtc cttcccagaa actgcctagg gctttgagtc attttatgaa 3600

gctaggtctt aatgcgtagg caacctccca gctcactatg aacgctgaca gaagagtgtt 3660

ttcatgtcta taatcaagaa ttccagatac attcctttta ctgaaccttg aattgatcct 3720

aagattggta gtaaaggtat tatgttacct cctaacagca ctacaaagta ccttttttta 3780

tcagaaaaaa attttaccat taggactcaa tttgaagtac taatgcttct caagttctcc 3840

actatgagag ttaccctgta ttagaccgtt acctataaga attaaggggt aaagcactaa 3900

acagaaaaga aaaaaaaaat agcaactctg gtgagcagat ttctttcctt tcttccttcc 3960

ttctcctctt cctaccttcc tccctccttt ccctctcctc cccttctctc cctttccctc 4020

cccttccctt ccttttcttc tttcctccgc tcccctcccc ttccctcccc ttcccatcct 4080

tctttctctt ttttttactt aatccccagt gtgacagtaa tataggctga tttctagaag 4140

tgtggtgtat tactcatgga aagtgagttg ccttggttat tactttcaat tgaaagttct 4200

atgggatcta gaaatgagac atactggcat ggagagtgag aacgacaaag gaatgaagag 4260

ctacaggagc atttaggcca tttctatgcc aagcttattc tacatgcaca aaatcataca 4320

tgttaataaa tataaacaaa ttggaggctt atttaaacca attatgaaat ctggtaattt 4380

gtgcagcagc aatagatgat aaccaaaaaa aactcataat aatctgaata tcttgatcat 4440

ttgtatttaa agaagcagta attatatact tgaaagtaca taatatagta ttgcaaaaat 4500

gactttggta tattacaaat taaaagtata taagatgaaa cttgatttgc tatcaagccc 4560

caagcaattt ttcaactggg cattgaattc taacttttct aagatagcaa tttttgaaga 4620

gacacgaaca aaaatctgaa ttagttcatg agccttaatg taaatctctt gctgaaatag 4680

tttttaaaat cagaatttag ttatctatca gactcaaaat catttaaaga ctaacaaaac 4740

acaatcatga tattctaact gtggtcaaac caggtaccca agccacctcc ctgcccaacg 4800

cctttccggc ttttcccctc cctcttgggc tggtggttat gctcctccag ctctagttca 4860

gctataattc cttttataga gaaaccaacc tgatacacac tttcatgatg ggagaaaaat 4920

gtgggagtga aatggtattt agaaagcagc agtcaggcac ggtggctcat gcctgtaatc 4980

ccagcacttt gggaggctga ggcaggcgga tcacttgagg tcaggagctc gagaccagcc 5040

tggccaacac ggtgaaaccc catctctact aaaaaaaaat acaaaaatta gccgggcgtg 5100

gtggcaggca cctgtaatcc cagctacttg ggaggctgag gcaggagaaa tcgcctgaac 5160

ccagaaggca gaggttgcag tgagccaaga tcacatcact gcactgcact ccagccgggg 5220

tgacagagcg aacctctgtc tcaaaaaaaa aaaaagaaaa aagaaagaaa gaaaaaaggc 5280

agaagccctg gattcaaatc cgccacacat tcagtttctt tatctgtaaa atggagacca 5340

ccccccgcca cgctgaacgg tgattctgtg actggtaaga gatgctacat ttttggtgct 5400

tgttcaggtg gaggaaagat gatagttaac actcaggtaa taagtatttt gaaggcagta 5460

taatatacct tcttaaagag tatacctact caaatgttgg taaatgttga catgattgaa 5520

tctaaatggc aaagagtatt tttagaaaac attaagtccc tgcagataaa tgacagtgtt 5580

gatttggatg cttaattaca ttcagacatg aactgttgga tgtatctgaa atgttaaaag 5640

ctttttctca acatttccaa aagtctttcc aagaaatcaa tgttatgttt tgttccagaa 5700

gcaaatttgc atttgtgatc tgtttctaaa aatggtacaa gttagctctg tttagaaagt 5760

aaaaatatct gatgttagat tggaagtatc tcttcctggg gaatccagaa agataagcat 5820

agcatattgt cttactgcaa tagataagtt gcttattgag aagtctggtt gttattctat 5880

atggtaacaa tacagttgat gtatatttta tgatagatcc tttatatttt cctcatgact 5940

ttagaagggg gaagggggag aaaattatga tgaccagact agttaaagag cattgaaagt 6000

ccacagtact gtagctaaag tagaagtttg ggtttgttat agactttaca ttatatcaac 6060

taataagcag atactgtaca gtattgctca ccattttatc atacttttgc atatgaacta 6120

ctccattgcc ttttatagat gttttatagc tgatcttacc agttttcctg gtaacttttt 6180

ttatttcttt tttttttttt tgagacggag tctcgcccta acacccaggt tggagtgcag 6240

tgccgtgatc tcggctcact gcaacctctg cctcccgggt tcaagcaatt ctcctgtctc 6300

agcctcccga gtacctggga ctaccggtgc ctgtctccac gcccggctaa ttttttgtat 6360

ttgtagtaga gacggggttt caccgtgtta gccaggatgg tctcgatctc ctgacctcat 6420

gatctgcctg cctctgcctg gacctcccaa agtgctggga ttacaggcgt gagcccccgc 6480

gcccagccac tttctttaat actataacta agaatttatt aaaatgcaca aattgtctaa 6540

gactgtaaag tttattgggg agaggccatg actacctctg aatttagtaa atttaaaata 6600

tttctgattc tcaataaaga actaatatcc atataaaaaa aaaaaaaaaa a 6651

<210> 12
<211> 3055
<212> DNA
<213> Homo sapiens

<400> 12
atttccggag gggaggcccc gcggctgccg ccgccatttc gggcgctgct gtgaagctga 60

aaccggagcc ggtccgctgg gcggcgggcg ccgggggccg gaggggcgcg cgcggcggcg 120

gcaccccagc gtttaggcgc ggaggcagcc atggcgggca acttcgactc ggaggagcgg 180

agtagctggt actgggggag gttgagtcgg caggaggcgg tggcgctgct gcagggccag 240

cggcacgggg tgttcctggt gcgggactcg agcaccagcc ccggggacta tgtgctcagc 300

gtctcagaga actcgcgcgt ctcccactac atcatcaaca gcagcggccc gcgcccgccg 360

gtgccaccgt cgcccgccca gcctccgccc ggggtgagcc cctccagact ccgaatagga 420

gatcaagagt ttgattcatt gcctgcttta ctggaattct acaaaataca ctatttggac 480

actacaacgt tgatagaacc agtttccaga tccaggcagg gtagtggagt gattctcagg 540

caggaggagg cggagtatgt gcgagccctc tttgacttta atgggaatga tgaggaagat 600

cttcccttta agaaaggaga catcttgaga atccgggaca agcctgaaga gcagtggtgg 660

aatgcggagg acagcgaagg caagagaggg atgattccag tcccttacgt cgagaagtat 720

agacctgcct ccgcctcagt atcggctctg attggaggtc ggtgagctgg taaaggttac 780

gaagattaat gtgagtggtc agtgggaagg ggagtgtaat ggcaaacgag gtcacttccc 840

attcacacat gtccgtctgc tggatcaaca gaatcccgat gaggacttca gctgagtata 900

gttcaacagt tttgctgaca gatgggaaca atcttttttt tttttttcca actgccatct 960

atacaatttt cttacagatg tcaaaagcag tctagtttat ataagcattc tgttacctgt 1020

gatatttttt agactgaact gctccattcc tagtcttaat taccatattc agggtacgaa 1080

ctggagggct tgtgtgttag cttctgaatt ggcaattgga ggcggtagtg gtcgtgcctg 1140

tgtgtatcag aagggatagg tatcttgcct cctttctctc aggcagtgca aatcaccctg 1200

tggaaaaccg atggacagga aggagtgtta cacactgctt accctgattt attcagtggt 1260

tttgttttca ttctggaacc atactatcaa atggcgacag actgttccgt tccacccccg 1320

tgaagtaatc atgcaccgtg tgaatagtat caagcaggat tgctttcatt gtatggagca 1380

tgaccagcgt gtgactcatt ctgacatttc agatcctaag aattctaaga acactactag 1440

aagcatttgt tccctcctag tcaatgcttc atactttttc ttgggattct tttagccctt 1500

gacattcttg tcccccaaac ctgtaagtag gtgaattcct aagataagtg tgtattttca 1560

ttccaggtga aaagcaggat gtaccgagca ctttattcag tgcatagctt taagccagtg 1620

ttggattcac taagtggaca gccagtctcc cagctctctg ccttccccaa aagggtcgta 1680

gtaggtcacc cttctacagc agctaactag agtcctaact aatgggatcc agcagggcca 1740

tttctccaga gggccagtat cctattagga gactcttgga attcttaggt tctactcaag 1800

agtggaagga ccaatcacct ctgatattct gtggaaggtt ttggggtcaa attctgccct 1860

ctgcattctg tgcaacttgt ataaaagtca agttagtatt acatgaattt ggggtagggt 1920

tagtgctttg aaaaaatgtt gaaccggctg ggcgcggtgg ctcacgtctg taatcccagc 1980

actttgggag gccgaggcgg gtggatcatg aggtcaggag ttcgagacca gcctggccaa 2040

catagtgaaa ccccatctct gctaaagata taaaaaatta gcccggcgtg gtggtgcacg 2100

cctgtaatcc cagctactcg ggaggctgag gcaggagaat tgcttcaacc tgggaggtgg 2160

aggctgcagt gagccgagat cgcaccactg cgttccagcc tgagcgacag ggcaagactc 2220

agtctcaaaa aaaaaaaaaa ggaaaaaaaa aagaaaaaaa aatgttgaac caattgtgaa 2280

ttacttatgt attattcatt tctcatgggg agagtaatgc tgttgaagaa cattacattg 2340

taaactgcct tcatttttgg ctctttgttt atgttcaggt ttagtttaca aacccattta 2400

agtatggaat gatttatatg gggtcaggtg ctccacaaaa tagacctatg agaccaaaaa 2460

tgacctaggc tatttagacg acagcatgaa acttccacgt tagttctcag tctataaagg 2520

cacttaccgg tctctggtgt ggtatgacca atagaaacac cttatagttt gctttggacc 2580

tcattttgga aaaataatct gcctttctaa ttgttctgca taggttaaaa tgataaattt 2640

acattctttg aacctatacc agattgtggt gtccgagtga ccggcacact gtctgacaca 2700

cagtcagtgt gcacgtattt gtctgagtga atgaggagac ctgagaaacc ggtgacgtgg 2760

cacagggaag ccagctggcc caggattccg tacatggccg caagcagact aacgcgttga 2820

cgctaattta atgtatttta cctcacacta aggtcatgct tgataaagac gttaaactca 2880

acttgtaaaa tggtagccca gtgctatgca cagagtgggt gctcattagt gttgaatgaa 2940

cacatttgta atactacatg taattccatc tgactgcttt gttaaatttt cagttagaac 3000

gtagatactg taaagtccac acacacatta aatcttgttt tcctgaaagt atggc 3055

<210> 13
<211> 1983
<212> DNA
<213> Homo sapiens

<400> 13
ctctgaaggg agctactcag aagcgggagt ctccgagaga agaaaagcag gtggaaggag 60

aggaagcgga tgccgtgggg tttacagcag gaaaatccgt ggagacagca gatccgagaa 120

gcggcgatgt ttgcgtagaa ccctgtcagc tgagccatga cccatgaacc atggaagctt 180

gactctagat tgaccatctt gagatgccaa agatgtccac gtcctaatcc catgtgggag 240

acagaataat ggccctgcag accttcccag ctggccatga cccctcattt gaccagctct 300

tcccttctct ctgaccagca ccatgcttct cctggtgaca agccttctgc tctgtgagtt 360

accacaccca gcattcctcc tgatcccaga gaaatcggat ctgcgaacag tggcaccagc 420

ctctagtctc aatgtgaggt ttgactccag gacgatgaat ttaagctggg actgccaaga 480

aaacacaacc ttcagcaagt gtttcttaac tgacaagaag aacagagtcg tggaacccag 540

gctcagtaac aacgaatgtt cgtgcacatt tcgtgaaatt tgtctgcatg aaggagtcac 600

atttgaggtt cacgtgaata ctagtcaaag aggatttcaa cagaaactgc tttatccaaa 660

ttcaggaagg gagggtaccg ctgctcagaa tttctcctgt ttcatctaca atgcggattt 720

aatgaactgt acctgggcga ggggtccgac ggccccccgt gacgtccagt attttttgta 780

catacgaaac tcaaagagaa ggagggagat ccggtgtcct tattacatac aagactcagg 840

aacccatgtg ggatgtcacc tggataacct gtcaggatta acgtctcgca attactttct 900

ggttaacgga accagccgag aaattggcat ccaattcttt gattcacttt tggacacaaa 960

gaaaatagaa cgattcaacc ctcccagcaa tgtcaccgta cgttgcaaca cgacgcactg 1020

cctcgtacgg tggaaacagc ccaggaccta tcagaagctg tcgtacctgg actttcagta 1080

ccagctggac gtccacagaa agaataccca gcctggcacg gaaaacctac tgattaatgt 1140

ttctggtgat ttggaaaata gatacaactt tccaagctct gagcccagag caaaacacag 1200

tgtgaagatc agagctgcag acgtccgcat cttgaattgg agctcctgga gtgaagccat 1260

tgaatttggt tctgacgacg ggaacctcgg ctctgtgtac atttatgtgc tcctaatcgt 1320

gggaaccctt gtctgtggca tcgtcctcgg cttcctcttt aaaaggttcc ttaggataca 1380

gcggctgttc ccgccagttc cacagatcaa agacaaactg aatgataacc atgaggtgga 1440

agacgagatc atctgggagg aattcacccc agaggaaggg aaaggctacc gcgaagaggt 1500

cttgaccgtg aaggaaatta cctgagaccc agagggtgta ggaatggcat ggacatctcc 1560

gcctccgcga cacgggggaa ctgttttctt gatgatgctg tgaaccttta tatcattttc 1620

tatgttttta tttaaaaaca tgacatttgg ggccaggcgc ggtggctcac gcctgtaatc 1680

ccagcacttt gggaggccaa ggcaggcgga tcacctgagg tcaggagttc aagaccagcc 1740

tgcccaacat ggtgaaaccc catctggact aaaaatgcag aaatttaccc aggcacggcg 1800

gcggacgccc atcatcccag ctacttggga ggctgaggca ggagaattgc ttgaacccgt 1860

gaggcggagg ttgtagtgag ccaagatcgc accattgcac accaacctgc gtgacagagc 1920

aagattgcat ctcaaaacaa acaataataa taaataataa aaacctgata tttggctggg 1980

caa 1983

<210> 14
<211> 6363
<212> DNA
<213> Homo sapiens

<400> 14
ggctgacatc acttaggaaa gcgaaggggg tagggctgcc agatcagttt gtcaccaccc 60

aggctccctt gcctttggct gggtgcaact tccattttag gtgttggatc tgagggggaa 120

aaaaaagaga gagggagaga gagagaaaga agagcaggaa agatcccgaa aggaggaaga 180

ggtggcgaaa aatcaactgc cctgctggat ttgtctttct cagcaccttg gcgaagcctt 240

gggtttcttt cttaaaggac tgatttttag aactccacat ttgaggtgtg tggcttttga 300

agaaaatgta tgtactgacg ggaaaaggag gataagcaag tcgaattttt gtcttacgct 360

ctctccttcc tgcttcctcc ttgctgtggt ggctgggatg cttcttccat gattttttga 420

atctagactg ggctgttctc tgtgttaaac caatcagttg cgaccttctc ttaacagtgt 480

gaagtgaggg ggtctctctc cctccttctc cttcctctgt gattcacctt cctttttacc 540

ctgccctgcg gcggctccgc cccttacctt catggacgac tcagaggtgg agtcgaccgc 600

cagcatcttg gcctctgtga aggaacaaga ggcccagttt gagaagctga cccgggcgct 660

ggaggaggaa cggcgccacg tctcggcgca gctggaacgc gtccgggtct caccacaaga 720

tgccaaccca ctcatggcca acggcacact cacccgccgg catcagaacg gccggtttgt 780

gggcgatgct gaccttgaaa gacagaaatt ttcagatttg aaactcaacg gaccccagga 840

tcacagtcac cttctatata gcaccatccc caggatgcag gagccggggc agattgtgga 900

gacctacacg gaggaggatc ctgagggagc catgtctgta gtctctgtgg agacctcaga 960

tgatgggacc actcggcgca cagagaccac ggtcaagaaa gtagtgaaga ctgtgacaac 1020

acggacagta cagccagtcg ctatgggacc agacgggttg cctgtggatg cttcatcagt 1080

ttctaacaac tatatccaga ctttgggtcg tgatttccgc aagaatggca atgggggacc 1140

tggtccctat gtggggcaag ctggcactgc tacccttcct aggaacttcc actaccctcc 1200

tgatggttat agtcgccact atgaagatgg ttatccaggt ggcagtgata actatggcag 1260

tctgtcccgg gtgacccgca ttgaggagcg gtataggccc agcatggaag gctaccgggc 1320

acctagtaga caggatgtgt atgggcccca accccaggtt cgggtaggtg ggagcagcgt 1380

ggatctgcat cgctttcatc cagagcctta tgggctagag gatgaccagc gtagtatggg 1440

ctatgatgac ctggattatg gtatgatgtc tgattatggc actgcccgtc ggactgggac 1500

accctctgac cctcgtcggc gcctcaggag ctatgaagac atgattggtg aggaggtgcc 1560

atcggatcaa tactactggg ctcctttggc ccagcatgag cgaggaagtt tagcaagctt 1620

ggatagcctg cgcaaaggag ggcctccacc tcctaattgg agacagccag agctgccaga 1680

ggtgatcgcc atgcttggat tccgcttgga tgctgtcaag tccaatgcag ctgcatacct 1740

gcaacactta tgctaccgca atgacaaggt gaagactgac gtgcggaagc tcaagggcat 1800

cccagtactg gtgggattgt tagaccatcc caaaaaggaa gtgcaccttg gagcctgtgg 1860

agctctcaag aatatctctt ttggacgtga ccaggataac aagattgcca taaaaaactg 1920

tgatggtgtg cctgcccttg tgcgattgct tcgaaaggct cgtgatatgg accttactga 1980

agttattacc ggaaccctgt ggaatctttc atcccatgac tcaatcaaaa tggagattgt 2040

ggaccatgca ctgcatgcct tgacagatga agtgatcatt cctcattctg gttgggagcg 2100

ggaacctaat gaagactgta agccacgcca tattgagtgg gaatcggtgc tcaccaacac 2160

agctggctgc cttaggaatg taagctcaga gaggagtgaa gctcgccgga aacttcggga 2220

atgtgatggt ttagttgatg ccctcatttt cattgttcag gctgagattg ggcagaagga 2280

ttcagacagc aagcttgtag agaactgtgt ttgccttctt cggaacttat catatcaagt 2340

tcaccgggag atcccacagg cagagcgtta ccaagaggca gctcccaatg ttgccaacaa 2400

tactgggcca catgctgcca gttgctttgg ggccaagaag ggcaaagatg agtggttctc 2460

cagagggaaa aaacctatag aggatccagc aaacgataca gtggatttcc ctaaaagaac 2520

gagtccagct cgaggctatg agctcttatt tcagccagag gtggttcgga tatacatctc 2580

acttcttaag gagagcaaga ctcctgccat cctagaagcc tcagctggag ctatccagaa 2640

cttgtgtgct gggcgctgga cgtatggtcg atacatccgc tctgctctgc gtcaagagaa 2700

ggctctttct gccatagctg acctcctgac taatgaacat gaacggtggt tgaaagctgc 2760

atctggagca ctgagaaacc tggctgtgga tgctcgcaac aaagaattaa ttggtaaaca 2820

tgctattcct aacttggtaa agaatctgcc aggaggacag cagaactcct cttggaattt 2880

ctctgaggac actgtcatct ctattttgaa cactatcaac gaggttatcg ctgagaactt 2940

ggaggctgcc aaaaagcttc gagagacaca gggtattgag aagctggtgt tgatcaacaa 3000

atcagggaac cgctcagaaa aagaagttcg agcagcagca cttgtattac agacaatctg 3060

gggatataag gaactgcgga agccactgga aaaagaagga tggaagaaat cagactttca 3120

ggtgaatcta aacaatgctt cccgaagcca gagcagtcat tcatatgatg atagtactct 3180

ccctctcatt gaccggaacc aaaaatcaga taagaaacct gatcgggaag aaattcagat 3240

gagcaatatg ggatcaaaca caaaatcact agataacaac tattccacac caaatgagag 3300

aggagaccac aatagaacac tggatcgatc gggggatcta ggcgacatgg agccattgaa 3360

gggaacaaca cccttgatgc aggacgaggg gcaggaatct ctggaggaag agttggatgt 3420

gttggttttg gatgatgagg ggggccaagt gtcttacccc tccatgcaga agatttagca 3480

ccactatctc cgttccatct gggcttatat gtacttttat tttttggtgg tgaaattgac 3540

tgatgatttt ccttttcttt cgctggacta ttgtgccaac tgccaggctg cctcctgccc 3600

ttacagccct aagtggctgc cttctttcca tcaactccca acttcttcct gtgaagttta 3660

attgtctcaa cgcctccccc tcccccattc cctccatttt tctcccaaga aacctgactc 3720

aattatttgc atattttgag aaactgctgc agattagttc tttttgccag ttttccctgg 3780

aactcctggc cttttgtgga ggggagggat ggagagaata ggaatcttca ctagaagccg 3840

tgggaagaat tggaagttac atgctgtata tgcaatgtcc agcagtctga taaactgacg 3900

attcttaatc aagatttttt tcctgatggg gaaggacttt ttattttctt ttagagaggg 3960

gaaagtgtga gctcttccct tattcctaat ggctattttt gaagcaaaga aggccagcaa 4020

cattggcaca tgccacctgg caaaggaccc ttgagtaagt gaaggtctcc taaaactggg 4080

attaagaaac cttgctctcc tcatctccaa ggcagggacc atcaagaacc tacagactcc 4140

atctcttctg caagcctcat gccaaccctg ggctattgct gctgcccctt aaacacaggc 4200

tgtccttaac ccacctctcc tgccctgtga tatgtctgct gagttggcct ggccatttcc 4260

aagaggctgt agaaagggga gaatgtcaag gaagactttt ggtagagaag gagcagaaag 4320

atgtgttttt gggaagaaga agacctctag gaggagctag taggaatgta catgaagcaa 4380

ttagtctgaa actggcttcc ccactccccc gtttctcctt ttcctatcct tataggcctg 4440

tcccttgcct ctgccctgga ttggttggca aactaaagga cttgatgtac ataactcctg 4500

tcccttttcc cttacaaggt ggggattgcc cctggctttg cctcttcttt gtgcctttgg 4560

cctggggtgc atctcctccc gcccttccat gtgcctttct ttgcctctgc agtctcattt 4620

ctcataattt tgcaaattat attttgttgc tttcttacct actattggcc ctaaatagca 4680

gaaagaagag aagtgaccga gagaacctca gattcttcat tgaggattgg tatagccatg 4740

atttcagtca tagcaagctt ttgctcaaca gcatatgggt gggattttgc aaaaatccta 4800

ttctgatgaa tctcaaagta aggctggtaa gagaagtgag tggtgtgact cttactcctt 4860

aggtgcccag aatttaccat catctctgaa ggagttacag ggaagtggtc tccccaattc 4920

tcccctccct ccagtattgc cccctctcac tttagcatat attaattagc aggttgggct 4980

agagaaatca gctgctatgc gggttgatta ttattattat ttctaatcct tttccttatt 5040

tgccttctac tccccttaat ctaatctaaa agctctgttc catgcaactg gagttcctta 5100

tccctctctt ccccttccct tatatattga ggctatgggg taggagaaaa gtgcacaacc 5160

caccaccccc tttactcgtg cattaaaatt tcttatttac ccttttcccc cttcccattt 5220

cttcccactt tcatctacct tttctggcaa aaaggagcct tttgctctct gtgaccctaa 5280

gagcacactg cacagggaaa attgccccat ccagacctgg ctccactctt gatctctctt 5340

gtcctcttct gctcttttcc tggtgctctt ttttctcggt ggggtgtggg taatagaaca 5400

gccgtgggct tttggggacc tttaactttt ttttctctct tttgtttata aaaaacacta 5460

aacattcaat tccagagaac caaaaatccc accttcccac cgaacactac taaggggctt 5520

gtgttctgct ccataccttt tctcttttct ttctgtcttg ttaatgcttt taaaaacaaa 5580

tgagtttttt atataaataa agtttttaaa gtgtgtatgt ggggggtctg tgtcatttct 5640

tcacttcaag ctgttatttc ttccctgctt tgcatctttg ttacttcctt atgtatcagt 5700

gtcctttcca gagcaaccag aaggaggtta taccaggatt tattttgagc tcagccccaa 5760

ctctttatca agcaacattc ttgttaacta tatgtgaaac attttttctt ctgaagattc 5820

ttaaaaattg aatgtggctg aagttgaaca tgggagctta ttgctaattt agagatagga 5880

aactgaagca taaagaatta atgacttact ttaattactg gaattcttct gcaacatttg 5940

acaaaactaa ccttgaataa ggcccactgt aatacgtagc tctcttaaat ataacactta 6000

ggactagaag attagaaact accaatccca actacgtaat aggaaaatgt aggatcaaaa 6060

ggcccatgta tataagtact gaccactggg ccataatgtt gcttctcagg ctatatgcag 6120

tcctttagtc agaagtcaat aggcctattt attaatattt tacagaccat attacctgga 6180

ttaccaggga ctatctttgc tgcagagatc aagggttaag atctatggga agatacttat 6240

ttttctgagg tccttatgtc ctgtcatata attaaagact caagagaatt tatgtgaaat 6300

gctttctgta tgcccaaatc tttagattaa aattatatag ctgctcctga aaaaaaaaaa 6360

aaa 6363

<210> 15
<211> 3783
<212> DNA
<213> Homo sapiens

<400> 15
ggggcggggc cgggagggta cttagggccg gggctggccc aggctacggc ggctgcaggg 60
ctccggcaac cgctccggca acgccaaccg ctccgctgcg cgcaggctgg gctgcaggct 120
ctcggctgca gcgctgggtg gatctaggat ccggcttcca acatgtggca gctctgggcc 180
tccctctgct gcctgctggt gttggccaat gcccggagca ggccctcttt ccatcccctg 240
tcggatgagc tggtcaacta tgtcaacaaa cggaatacca cgtggcaggc cgggcacaac 300
ttctacaacg tggacatgag ctacttgaag aggctatgtg gtaccttcct gggtgggccc 360
aagccacccc agagagttat gtttaccgag gacctgaagc tgcctgcaag cttcgatgca 420
cgggaacaat ggccacagtg tcccaccatc aaagagatca gagaccaggg ctcctgtggc 480
tcctgctggg ccttcggggc tgtggaagcc atctctgacc ggatctgcat ccacaccaat 540
gcgcacgtca gcgtggaggt gtcggcggag gacctgctca catgctgtgg cagcatgtgt 600
ggggacggct gtaatggtgg ctatcctgct gaagcttgga acttctggac aagaaaaggc 660
ctggtttctg gtggcctcta tgaatcccat gtagggtgca gaccgtactc catccctccc 720
tgtgagcacc acgtcaacgg ctcccggccc ccatgcacgg gggagggaga tacccccaag 780
tgtagcaaga tctgtgagcc tggctacagc ccgacctaca aacaggacaa gcactacgga 840
tacaattcct acagcgtctc caatagcgag aaggacatca tggccgagat ctacaaaaac 900
ggccccgtgg agggagcttt ctctgtgtat tcggacttcc tgctctacaa gtcaggagtg 960
taccaacacg tcaccggaga gatgatgggt ggccatgcca tccgcatcct gggctgggga 1020
gtggagaatg gcacacccta ctggctggtt gccaactcct ggaacactga ctggggtgac 1080
aatggcttct ttaaaatact cagaggacag gatcactgtg gaatcgaatc agaagtggtg 1140
gctggaattc cacgcaccga tcagtactgg gaaaagatct aatctgccgt gggcctgtcg 1200
tgccagtcct gggggcgaga tcggggtaga aatgcatttt attctttaag ttcacgtaag 1260
atacaagttt cagacagggt ctgaaggact ggattggcca aacatcagac ctgtcttcca 1320
aggagaccaa gtcctggcta catcccagcc tgtggttaca gtgcagacag gccatgtgag 1380
ccaccgctgc cagcacagag cgtccttccc cctgtagact agtgccgtag ggagtacctg 1440
ctgccccagc tgactgtggc cccctccgtg atccatccat ctccagggag caagacagag 1500
acgcaggaat ggaaagcgga gttcctaaca ggatgaaagt tcccccatca gttcccccag 1560
tacctccaag caagtagctt tccacatttg tcacagaaat cagaggagag acggtgttgg 1620
gagccctttg gagaacgcca gtctcccagg ccccctgcat ctatcgagtt tgcaatgtca 1680
caacctctct gatcttgtgc tcagcatgat tctttaatag aagttttatt ttttcgtgca 1740
ctctgctaat catgtgggtg agccagtgga acagcgggag acctgtgcta gttttacaga 1800
ttgcctcctt atgacgcggc tcaaaaggaa accaagtggt caggagttgt ttctgaccca 1860

ctgatctcta ctaccacaag gaaaatagtt taggagaaac cagcttttac tgtttttgaa 1920

aaattacagc ttcaccctgt caagttaaca aggaatgcct gtgccaataa aagttttctc 1980

caacttgaag tctactctga tgggatctca gatcctttgt cactgcctat agacttgtag 2040

ctgctgtctc tctttgtccc tgcagagaat cacgtcctgg aactgcatgt tcttgcgact 2100

cttgggactt catcttaact tctcgctgcc ccagccatgt tttcaaccat ggcatccctc 2160

ccccaattag ttccctgtca tcctcgtcaa ccttctctgt aagtgcctgg taagcttgcc 2220

cttgcttaag aactcaaaac atagctgtgc tctatttttt tgttgttgtt gtgactgaca 2280

gagtgagatt ccgtctccca ggctggagtg cagtggcgcc ttctcagctc actgcaacct 2340

gcagcctcct agattcaagc gattctcctg cttcagcctt ccgagtagct gggatgacag 2400

gcactcacca atatgcctgg gtaatttttg tatttttaag tacatacagg atttcaccat 2460

gttggccagg ctagtttcaa actcccggcc tcaggtggtc tgcctgcctc agcctcccaa 2520

agtgttggga ttacaggcgt gagccactgg gccctgcctg tattttttat cagccacaaa 2580

tccagcaaca agctgaggat tcagctcata aaacaggctt ggtgtcttgg tgatctcaca 2640

taaccaagat gctaccccgt ggggaaccac atccccctgg atgccctcca gccttggttt 2700

gggctggagt cagggcctgt atacagtatt ttgaatttgt atgccactgg tttgcattgc 2760

tggtcaggaa ctctagtgct ttgcatagcc ctggtttaga aacatgttat agcagttctt 2820

ggtatagagc aaactagaag aaccagcaat cattccactg tcctgccaag gtacacctca 2880

gtactcccct tcccaactga agtggtatga ggctagctct ttccaaaagc attcaagttt 2940

ggcttctgat gtgactcaga atttaggaac cagatgctag atcaaataag ctctgaaaat 3000

ctgaggaaca ttgtaggaaa ggtttgttaa gcatctctta agtgccatga tgagcataac 3060

agccggccgt cgtggctcac gcctgtaatc ccagcacttt gggaggccaa ggtgggagga 3120

tgacaaggtc aggagttcaa gaccagcctg gccaacatgc tgaaacctca cctctactaa 3180

aaatacaaaa attagctggg catggtggca catgcctgta atcccagcta cttgggaggc 3240

tgaggcagga gaatcgcttg aacccgggag gcggaggttg cagtgagcca agacagtgcc 3300

agtgcactcc agcctcggtg acagcgcaag gctccgtctc aataattaaa aaaaaaaaaa 3360

aaaaaaaaaa ggccgggcgc agtggctcaa gcctgtaatc ccagcacttt gggaggctga 3420

ggcgggcaga tcacctgagg tcaggagttt tgagatcagc cttggcaaca cggtgaaacc 3480

ccatctctac taaaaataca aaattagcca agcatgctgg cacatgcctg taatcccagc 3540

tactcgggag gctgaggtac gagaatcgct tgaacctggg aggcagagga tgcagtgagc 3600

cgagatcacg ccattgcact ccagcctggg ggacaagagt gaatctgtgt ctcaccaaaa 3660

aaaaaaagaa aaagaaagat gcttaacaaa ggttaccata agccacaaat tcataaccac 3720

ttatccttcc agtttcaagt agaatatatt cataacctca ataaagttct ccctgctccc 3780

aaa                                                                 3783

<210>  16
<211>  1466
<212>  DNA
<213>  Homo sapiens

<400>  16
ggtgcgtccg cgggtggctg ccccgcaggt gcgcgcggcc ggggctggcg gcgactctct      60

ccaccgggcc gcccgggagg ctcatgcagc gcggctgggt cccgcggcgc ccggatcggg     120

gaagtgaaag tgcctcggag gaggagggcc ggtccggcag tgcagccgcc tcacaggtcg     180

gcggacgggc caggcgggcg gcctcctgaa ccgaaccgaa tcggctcctc gggccgtcgt     240

cctcccgccc ctcctcgccc gccgccggag ttttctttcg gtttcttcca agattcctgg     300

ccttccctcg acggagccgg gcccagtgcg ggggcgcagg gcgcgggagc tccacctcct     360

cggctttccc tgcgtccaga ggctggcatg gcgcgggccg agtactgagc gcacggtcgg     420

ggcacagcag ggccgggggg tgcagctggc tcgcgcctcc tctccggccg ccgtctcctc     480

cggtccccgg cgaaagccat tgagacacca gctggacgtc acgcgccgga gcatgtctgg     540

gagtcagagc gaggtggctc catccccgca gagtccgcgg agccccgaga tgggacggga     600

cttgcggccc gggtcccgcg tgctcctgct cctgcttctg ctcctgctgg tgtacctgac     660

tcagccaggc aatggcaacg agggcagcgt cactggaagt tgttattgtg gtaaaagaat     720

ttcttccgac tccccgccat cggttcagtt catgaatcgt ctccggaaac acctgagagc     780

ttaccatcgg tgtctatact acacgaggtt ccagctcctt tcctggagcg tgtgtggggg     840

caacaaggac ccatgggttc aggaattgat gagctgtctt gatctcaaag aatgtggaca     900

tgcttactcg gggattgtgg cccaccagaa gcatttactt cctaccagcc ccccaatttc     960

tcaggcctca gagggggcat cttcagatat ccacacccct gcccagatgc tcctgtccac    1020

cttgcagtcc actcagcgcc ccaccctccc agtaggatca ctgtcctcgg acaaagagct    1080

cactcgtccc aatgaaacca ccattcacac tgcgggccac agtctggcag ctgggcctga    1140

ggctggggag aaccagaagc agccggaaaa aaatgctggt cccacagcca ggacatcagc    1200

cacagtgcca gtcctgtgcc tcctggccat catcttcatc ctcaccgcag ccctttccta    1260

tgtgctgtgc aagaggagga gggggcagtc accgcagtcc tctccagatc tgccggttca    1320

ttatatacct gtggcacctg actctaatac ctgagccaag aatggaagct tgtgagggta    1380

aactgtggct tattcttaca aaaagtgtaa taaaggagac tgacccctga caacatggta    1440

ggcactgtat aaaaaaaaaa aaaaaa                                          1466

<210>  17
<211>  2804

<212>  DNA
<213>  Homo sapiens

<400>  17
aaagccagac tgattcatag aaactccttt aaaacacggt gaaaagaaac cgcccattac      60

acaccccagt acaccagcag aggaaactta taacctcggg aggcaggtcc ttcccctcag     120

tgcggtcaca tacttccaga agagcggacc agggctgctg ccagcacctg ccactcagag     180

cgcctctgtc gctgggaccc ttcagaactc tctttgctca caagttacca aaaaaaaaag     240

agccaacatg ttggtattgc tggctggtat ctttgtggtc cacatcgcta ctgttattat     300

gctatttgtt agcaccattg ccaatgtctg gttggtttcc aatacggtag atgcatcagt     360

aggtctttgg aaaaactgta ccaacattag ctgcagtgac agcctgtcat atgccagtga     420

agatgccctc aagacagtgc aggccttcat gattctctct atcatcttct gtgtcattgc     480

cctcctggtc ttcgtgttcc agctcttcac catggagaag ggaaaccggt tcttcctctc     540

aggggccacc acactggtgt gctggctgtg cattcttgtg ggggtgtcca tctacactag     600

tcattatgcg aatcgtgatg gaacgcagta tcaccacggc tattcctaca tcctgggctg     660

gatctgcttc tgcttcagct tcatcatcgg cgttctctat ctggtcctga gaaagaaata     720

aggccggacg agttcatggg gatctggggg gtggggagga ggaagccgtt gaatctggga     780

gggaagtgga ggttgctgta caggaaaaac cgagataggg gaggggggag ggggaagcaa     840

aggggggagg tcaaatccca aaccattact gaggggattc tctactgcca agcccctgcc     900

ctggggagaa agtagttggc tagtactttg atgctccctt gatggggtcc agagagcctc     960

cctgcagcca ccagacttgg cctccagctg ttcttagtga cacacactgt ctggggcccc    1020

atcagctgcc acaacaccag ccccacttct gggtcatgca ctgaggtcca cagacctact    1080

gcactgagtt aaaatagcgg tacaagttct ggcaagagca gatactgtct ttgtgctgaa    1140

tacgctaagc ctggaagcca tcctgccctt ctgacccaaa gcaaaacatc acattccagt    1200

ctgaagtgcc tactgggggg ctttggcctg tgagccattg tccctctttg gaacagatat    1260

ttagctctgt ggaattcagt gacaaaatgg gaggaggaaa gagagtttgt aaggtcatgc    1320

tggtgggtta gctaaaccaa gaaggagacc ttttcacaat ggaaaacctg ggggatggtc     1380

agagcccagt cgagacctca cacacggctg tccctcatgg agacctcatg ccatggtctt    1440

tgctaggcct cttgctgaaa gccaaggcag ctcttctgga gtttctctaa agtcactagt    1500

gaacaattcg gtggtaaaag taccacacaa actatgggat ccaaggggca gtcttgcaac    1560

agtgccatgt tagggttatg tttttaggat tcccctcaat gcagtcagtg tttcttttaa    1620

gtatacaaca ggagagagat ggacatggct cattgtagca caatcctatt actcttcctc    1680

taacattttt gaggaagttt tgtctaatta tcaatattga ggatcagggc tcctaggctc    1740

agtggtagct ctggcttaga caccacctgg agtgatcacc tcttggggac cctgcctatc    1800

ccacttcaca ggtgaggcat ggcaattctg gaagctgatt aaaacacaca taaaccaaaa 1860

ccaaacaaca ggcccttggg tgaaaggtgc tatataattg tgaagtatta agcctaccgt 1920

atttcagcca tgataagaac agagtgcctg cattcccagg aaaatacgaa aatcccatga 1980

gataaataaa aatataggtg atgggcagat cttttcttta aaataaaaaa gcaaaaactc 2040

ttgtggtacc tagtcagatg gtagacgagc tgtctgctgc cgcaggagca cctctataca 2100

ggacttagaa gtagtatgtt attcctggtt aagcaggcat tgctttgccc tggagcagct 2160

attttaagcc atctcagatt ctgtctaaag gggttttttg ggaagacgtt ttctttatcg 2220

ccctgagaag atctacccca gggagaatct gagacatctt gcctactttt ctttattagc 2280

tttctcctca tccatttctt ttataccttt cctttttggg gagttgttat gccatgattt 2340

ttggtattta tgtaaaagga ttattactaa ttctatttct ctatgtttat tctagttaag 2400

gaaatgttga gggcaagcca ccaaattacc taggctgagg ttagagagat tggccagcaa 2460

aaactgtggg aagatgaact ttgtcattat gatttcatta tcacatgatt atagaaggct 2520

gtcttagtgc aaaaaacata cttacatttc agacatatcc aaagggaata ctcacatttt 2580

gttaagaagt tgaactatga ctggagtaaa ccatgtattc ccttatcttt tacttttttt 2640

ctgtgacatt tatgtctcat gtaatttgca ttactctggt ggattgttct agtactgtat 2700

tgggcttctt cgttaataga ttatttcata tactataatt gtaaatattt tgatacaaat 2760

gtttataact ctagggatat aaaaacagat tctgattccc ttca 2804

<210> 18
<211> 3196
<212> DNA
<213> Homo sapiens

<400> 18
ctctacccgg ttggcaggcg gcctggccca gccccttctc taaggaagcg catttcctgc 60

ctccctgggc cggccgggct ggatgagccg ggagctccct gctgccggtc ataccacagc 120

cttcatctgc gccctggggc caggactgct gctgtcactg ccatccattg gagcccagca 180

ccccctcccc gcccatcctt cggacagcaa ctccagccca gccccgcgtc cctgtgtcca 240

cttctcctga cccctcggcc gccaccccag aaggctggag cagggacgcc gtcgctccgg 300

ccgcctgctc ccctcgggtc cccgtgcgag cccacgccgg ccccggtgcc cgcccgcagc 360

cctgccactg gacacaggat aaggcccagc gcacaggccc ccacgtggac agcatggacc 420

gcggcacgct ccctctggct gttgccctgc tgctggccag ctgcagcctc agccccacaa 480

gtcttgcaga aacagtccat tgtgaccttc agcctgtggg ccccgagagg ggcgaggtga 540

catataccac tagccaggtc tcgaagggct gcgtggctca ggcccccaat gccatccttg 600

aagtccatgt cctcttcctg gagttcccaa cgggcccgtc acagctggag ctgactctcc 660

aggcatccaa gcaaaatggc acctggcccc gagaggtgct tctggtcctc agtgtaaaca 720

gcagtgtctt cctgcatctc caggccctgg gaatcccact gcacttggcc tacaattcca 780

gcctggtcac cttccaagag cccccggggg tcaacaccac agagctgcca tccttcccca 840

agacccagat ccttgagtgg gcagctgaga ggggccccat cacctctgct gctgagctga 900

atgaccccca gagcatcctc ctccgactgg gccaagccca ggggtcactg tccttctgca 960

tgctggaagc cagccaggac atgggccgca cgctcgagtg gcggccgcgt actccagcct 1020

tggtccgggg ctgccacttg gaaggcgtgg ccggccacaa ggaggcgcac atcctgaggg 1080

tcctgccggg ccactcggcc gggccccgga cggtgacggt gaaggtggaa ctgagctgcg 1140

cacccgggga tctcgatgcc gtcctcatcc tgcagggtcc cccctacgtg tcctggctca 1200

tcgacgccaa ccacaacatg cagatctgga ccactggaga atactccttc aagatctttc 1260

cagagaaaaa cattcgtggc ttcaagctcc cagacacacc tcaaggcctc ctgggggagg 1320

cccggatgct caatgccagc attgtggcat ccttcgtgga gctaccgctg gccagcattg 1380

tctcacttca tgcctccagc tgcggtggta ggctgcagac ctcacccgca ccgatccaga 1440

ccactcctcc caaggacact tgtagcccgg agctgctcat gtccttgatc cagacaaagt 1500

gtgccgacga cgccatgacc ctggtactaa agaaagagct tgttgcgcat ttgaagtgca 1560

ccatcacggg cctgaccttc tgggacccca gctgtgaggc agaggacagg ggtgacaagt 1620

ttgtcttgcg cagtgcttac tccagctgtg gcatgcaggt gtcagcaagt atgatcagca 1680

atgaggcggt ggtcaatatc ctgtcgagct catcaccaca gcggaaaaag gtgcactgcc 1740

tcaacatgga cagcctctct ttccagctgg gcctctacct cagcccacac ttcctccagg 1800

cctccaacac catcgagccg gggcagcaga gctttgtgca ggtcagagtg tccccatccg 1860

tctccgagtt cctgctccag ttagacagct gccacctgga cttggggcct gagggaggca 1920

ccgtggaact catccagggc cgggcggcca agggcaactg tgtgagcctg ctgtccccaa 1980

gccccgaggg tgacccgcgc ttcagcttcc tcctccactt ctacacagta cccataccca 2040

aaaccggcac cctcagctgc acggtagccc tgcgtcccaa gaccgggtct caagaccagg 2100

aagtccatag gactgtcttc atgcgcttga acatcatcag ccctgacctg tctggttgca 2160

caagcaaagg cctcgtcctg cccgccgtgc tgggcatcac ctttggtgcc ttcctcatcg 2220

gggccctgct cactgctgca ctctggtaca tctactcgca cacgcgtgag taccccaggc 2280

ccccacagtg agcatgccgg gcccctccat ccacccgggg gagcccagtg aagcctctga 2340

gggattgagg ggccctggcc aggaccctga cctccgcccc tgcccccgct cccgctccca 2400

ggttccccca gcaagcggga gcccgtggtg gcggtggctg ccccggcctc ctcggagagc 2460

agcagcacca accacagcat cgggagcacc cagagcaccc cctgctccac cagcagcatg 2520

gcatagcccc ggccccccgc gctcgcccag caggagagac tgagcagccg ccagctggga 2580

gcactggtgt gaactcaccc tgggagccag tcctccactc gacccagaat ggagcctgct 2640

ctccgcgcct acccttcccg cctccctctc agaggcctgc tgccagtgca gccactggct 2700

tggaacacct tggggtccct ccaccccaca gaaccttcaa cccagtgggt ctgggatatg 2760

gctgcccagg agacagacca cttgccacgc tgttgtaaaa acccaagtcc ctgtcatttg 2820

aacctggatc cagcactggt gaactgagct gggcaggaag ggagaacttg aaacagattc 2880

aggccagccc agccaggcca acagcacctc cccgctggga agagaagagg gcccagccca 2940

gagccacctg gatctatccc tgcggcctcc acacctgaac ttgcctaact aactggcagg 3000

ggagacagga gcctagcgga gcccagcctg ggagcccaga gggtggcaag aacagtgggc 3060

gttgggagcc tagctcctgc cacatggagc cccctctgcc ggtcgggcag ccagcagagg 3120

gggagtagcc aagctgcttg tcctgggcct gcccctgtgt attcaccacc aataaatcag 3180

accatgaaac cagtga 3196

<210> 19
<211> 4446
<212> DNA
<213> Homo sapiens

<400> 19
cgcaccgccg ccgaggacgc gcgcccgagc ctagtcccca cgccgcggcg cgcccgggct 60

ccctgctgat cccagaacaa tcaaccatga cgaccgaatc tggatcagac tcggaatcca 120

agccggacca ggaggccgag ccccaggagg cggcgggggc gcaggggcgc gcgggggcgc 180

ccgtgccgga gccgcccaag gaggagcagc agcaggccct ggagcagttc gccgccgctg 240

cagcgcacag caccccggtg cggagggagg tcactgacaa ggaacaggag tttgctgcca 300

gggctgcaaa acagctcgaa tatcagcaat tagaagacga taaactttct cagaaatcat 360

ctagcagtaa actctctcgg tctccattaa agattgtcaa aaagcctaaa agcatgcagt 420

gcaaagtgat acttctcgat ggatcagaat atacctgtga tgtagagaaa cgctccagag 480

gacaagtgct gtttgataaa gtgtgtgaac acttgaactt gctagagaaa gactactttg 540

ggcttacgta tcgagatgct gaaaaccaga agaattggtt ggaccctgct aaggaaataa 600

aaaaacaggt tcgaagtggt gcttggcact tttcatttaa tgtgaaattt tatccaccag 660

accctgccca actatctgaa gatatcacca ggtactacct ctgcttgcag ttgcgagatg 720

acatcgtgtc cggaaggctg ccctgctcct ttgttaccct ggccttgctg ggctcctaca 780

ctgtccagtc agagctcgga gactatgacc cagatgaatg tgggagcgat tacattagtg 840

agttccgctt tgcaccaaac cacactaaag aactggaaga caaagtgatc gagctgcaca 900

agagccacag aggaatgacg ccagcagaag cagagatgca tttcttggaa aatgccaaaa 960

aattatcaat gtatggggta gatttacatc atgctaagga ctcagaaggg gtagaaatta 1020

tgttaggagt ttgtgcaagt ggtctgttga tatatcgcga ccggctgcga ataaacagat 1080

ttgcctggcc caaggttcta aagatttcat acaaacggaa caacttttac attaagatcc 1140

ggccgggaga gtttgaacaa tttgaaagca ccattgggtt taagctgcca aaccatcgag 1200

ctgccaagcg tttatggaaa gtatgtgttg agcatcatac atttttcaga ctactgttac 1260

cagaagcacc tcccaagaaa ttcctaacct tgggttccaa gtttcgttat agtggcagga 1320

cacaagcgca aacgagaaga gccagtgcgt tgatagatcg cccagcacct tactttgaac 1380

gctcatccag caaacgttat accatgtctc gcagcttgga tggagaggtt ggtactggcc 1440

agtacgccac aacaaaaggc atctctcaga ccaacttgat caccactgtg actccggaga 1500

agaaggctga ggaggagcgg gacgaggaag aggacaaacg gaggaagggg gaagaagtca 1560

cgcccatctc ggccatccgg cacgagggaa agtcacctgg gcttggcact gactcatgtc 1620

ccttgtcacc cccatccacc cattgtgccc ccacatctcc cacagagctc cgtaggaggt 1680

gtaaggagaa tgactgcaaa ctgccaggtt atgagccgtc cagagctgag cacctgcctg 1740

gagagcccgc cttggactct gatggcccag ggaggcctta cctaggggat caagatgtgg 1800

cttttagcta cagacagcaa actggcaagg ggaccaccct gttctccttc tccttgcagc 1860

tccctgagtc attcccctcc ctcctagatg atgatggata cctctctttc cccaaccttt 1920

ctgaaaccaa cctcctgccc cagagcttgc agcattacct cccgatccgc tcaccgtccc 1980

ttgtgccctg tttcctcttc atctttttct ttctgctgtc tgcctccttc tcagtgccat 2040

acgctctcac tctctccttc cctctggctc tgtgcctctg ctacctggag cccaaggcgg 2100

cctccttgag cgcctcccta gacaatgacc cgagtgacag ttcagaggaa gagactgaca 2160

gtgagcgcac ggacaccgca gccgacgggg agaccaccgc cactgagtcg gaccaggagg 2220

aagatgcaga gctcaaggca caggagctag aaaaaactca agatgacctg atgaaacatc 2280

aaaccaacat tagcgagctg aaaagaacct tcttagaaac ctcaacagac actgccgtaa 2340

cgaatgaatg ggagaagagg ctttccacct cccccgtgcg actggccgcc aggcaggagg 2400

atgcccccat gatcgaacca cttgtccctg aagagactaa gcagtcttct ggggaaaagc 2460

tcatggatgg ctctgaaatc ttcagtttat tagagtctgc gcgaaaacca acagaattca 2520

taggaggggt tacttctact tctcaaagct gggttcagaa aatggaaacc aagacggagt 2580

ccagtggaat agagacggaa cccaccgtgc accacctgcc gcttagcact gagaaggtgg 2640

tgcaggagac cgtgttggtg gaggagcggc gtgtggtgca cgcgagtggg gatgcttctt 2700

actcggcggg agacagcggg gatgctgcag cacagcccgc attcacaggc attaaaggga 2760

aagagggctc tgccttgacg gagggggcta aagaggaagg aggggaggag gtcgctaaag 2820

ctgtcctgga acaggaagag acagccgctg cttcccgtga gcgacaagag gagcagagtg 2880

cagccatcca catttcagaa actttggaac aaaaacctca ttttgagtcc tcaacggtga 2940

agacggaaac catcagtttt ggcagtgttt caccgggagg agtaaagcta gaaatttcca 3000

cgaaggaagt gccagtagtt cacaccgaaa ccaaaaccat cacatatgaa tcatcacagg 3060

tcgatccagg cacagatctg gagccaggcg tgctgatgag tgcacagacg atcacatctg 3120

aaaccaccag taccaccacc actacgcaca tcaccaaaac tgtgaaaggg ggcatttcag 3180

agacaagaat tgagaagcga atagtcatca cgggggatgc agacattgac catgaccagg 3240

cgctggctca ggcaattaaa gaggccaaag agcagcaccc tgacatgtca gtgaccaaag 3300

tagtggtcca taaagagaca gagatcacac cagaagatgg agaggattga ccagaggaat 3360

aacttagctt gcacatgaat gcagtcatgc aaaccgttag gaaaaccaga gcctatatgg 3420

agttccctct tctaacccaa ctgacttgta tctgtccgtg gaaaatttca gtccagaaga 3480

attgaccttg accattaata aagacactgg cagagagatc ttcccataat aaagcaatct 3540

gattcagcat cactaaaccg ataatgcatg aagcaacgat aaaattacaa aagagcagca 3600

tttttaattt tcacaaaatg tctcagtttt cagctatacc tgcacgttca taaccaacaa 3660

tataaaccgt ggtctcatgt aacacataaa caattcatgc ctttcatagt ttattattat 3720

taaagtctaa acaaaattgc aatttcttag gtaaccttat atttacaata aatgaagatt 3780

accctcaaat gctagaagct gtctaggtcc gtccggtgtg tcagattttc ctcagattag 3840

atgtgccaat aaccaagttt attcagtaaa caacttgtac ttgtttcatc tggttttatt 3900

actctcaccc ataaacagta atgactctct gaccctctgg aaatatgtaa tgcttccaat 3960

cttgctttgt gtatctcatt taatttgtta taaggtagta ctgattttag catattaatg 4020

cgatttcttc cttgttgttt gctttggtct gtgttcaatc cagagagctt aaattgtcat 4080

tattttggga agaaaacctg tatttttgtt agtttacaat attatgaaat ttcacttcag 4140

gagaaactgc tgggcttcct gtggctttgt tttcttagtt actttttccg tgccgtgtat 4200

tttttaattg atttttcttc ttttacttga aaagaaagtg ttttattttc aaatctggtc 4260

catatttaca ttctagttca gagccaagcc ttaaactgta cagaatttcc actgtaatta 4320

aaactattta gtgttagtta taaatagcct tcaaaaagag agattctcca ttacacgatc 4380

acctgcatca cagcccatgg tgaatgtatg tttctgcata gcgaaataaa aatggcaaat 4440

gcactg 4446

<210> 20
<211> 1839
<212> DNA
<213> Homo sapiens

<400> 20
agcaacgggg tgcggcaggg tggggaacgc gggagcgggg ccagctccca ggaaagctgg 60

tctgcgagcg gcccctgccc ggctcccagg tccctgcgcg accccgccct tcccgagacc 120

ccagccgggc tgccgcccgc gtcccggaag ctccagcctg aaccatgttt ttcacttgtg 180

gcccaaatga ggccatggtg gtctccgggt tctgccgaag ccccccagtc atggtggctg 240

gagggcgtgt ctttgtcctg ccctgcatcc aacagatcca gaggatctct ctcaacacac 300

tgaccctcaa tgtcaagagt gaaaaggttt acactcgcca tggggtcccc atctcagtca 360

ctggcattgc ccaggtaaaa atccaggggc agaacaagga gatgttggcg gccgcctgtc 420

agatgttcct ggggaagacg gaggctgaga ttgcccacat tgccctggag acgttagagg 480

gccaccagag ggccatcatg gcccacatga ctgtggagga gatctataag gacaggcaga 540

aattctcaga acaggttttc aaagtggcct cctcagacct ggtcaacatg ggcatcagtg 600

tggttagcta cactctgaag gacattcacg atgaccagga ctatttgcac tctttgggga 660

aggctcgaac agctcaagtc caaaaagatg cacggattgg agaagcagag gccaagagag 720

atgctgggat ccgggaagct aaagccaagc aggaaaaggt gtctgctcag tacctgagtg 780

agatcgagat ggccaaggca cagagagatt acgaactgaa gaaggccgcc tatgacatcg 840

aggtcaacac ccgccgagca caggctgacc tggcctatca gcttcaggtg gccaagacta 900

agcagcagat tgaggagcag cgggtgcagg tgcaggtggt ggagcgggcc cagcaggtgg 960

cagtgcagga gcaggagatc gcccggcggg agaaggagct ggaggcccgg gtgcggaagc 1020

cagcggaagc ggagcgctac aagctggagc gcctagccga ggcagagaag tcccaactaa 1080

ttatgcaggc ggaggcagaa gccgcgtctg tgcggatgcg tggggaagct gaggcctttg 1140

ccataggggc ccgagcccga gccgaggctg agcagatggc caagaaggca gaagccttcc 1200

agctgtacca agaggctgct cagctggaca tgctgctaga gaagctgccc caggtggcag 1260

aggagatcag tggtcccttg acttcagcca ataagatcac actggtgtcc agcggcagtg 1320

ggaccatggg ggcagccaaa gtgactgggg aagtactgga cattctaact cgcctgccag 1380

agagtgtgga aagactcaca ggcgtgagca tctcccaggt gaatcacaag cctttgagaa 1440

cagcctgagc cttcagccct cacagatgcc cagcctcata gctgaagttg cctgaatgat 1500

cctcctgttg catgtaaccc actggcctcc ctgagcatgt ccattgacag tgaggtccca 1560

cccctcatct ctccttgcca aatagtttgt gccttgtctt gaagggggtt gctccccttg 1620

ccaacctcac actgctatga ttgccaactc cagcggtccc atgtcagcct tctgatgatc 1680

ccactccacc ccacctcaac ttatttaact tcctaattaa atcagactgt ttgagcctgt 1740

tgtctagaat attttcctga ccaagactga gggatgggct ggaggttttc aactttgcta 1800

cccaaataaa ttgctgtaag taagtactaa aaaaaaaaa 1839

<210> 21

<211>   2132
<212>   DNA
<213>   Homo sapiens

<400>   21
ggggagccct ggcctcccca cctcctcccg tccccaccct gttcccagca ctcaagcctt      60

gccaccgccg agccgggctt cctgggtgtt tcaggcaagg aagtctaggt ccctgggggg     120

tgacccccaa ggaaaaggca gcctccctgc gcacccggtt gcccggagcc ctctccaggg     180

ccggctgggc tgggggttgc cctggccagc aggggcccgg gggcgatgcc acccggtgcc     240

gactgaggcc accgcaccat ggcccgctcg ctgacctggc gctgctgccc ctggtgcctg     300

acggaggatg agaaggccgc cgcccgggtg gaccaggaga tcaacaggat cctcttggag     360

cagaagaagc aggaccgcgg ggagctgaag ctgctgcttt tgggcccagg cgagagcggg     420

aagagcacct tcatcaagca gatgcggatc atccacggcg ccggctactc ggaggaggag     480

cgcaagggct tccggcccct ggtctaccag aacatcttcg tgtccatgcg ggccatgatc     540

gaggccatgg agcggctgca gattccattc agcaggcccg agagcaagca ccacgctagc     600

ctggtcatga gccaggaccc ctataaagtg accacgtttg agaagcgcta cgctgcggcc     660

atgcagtggc tgtggaggga tgccggcatc cgggcctact atgagcgtcg gcgggaattc     720

cacctgctcg attcagccgt gtactacctg tcccacctgg agcgcatcac cgaggagggc     780

tacgtcccca cagctcagga cgtgctccgc agccgcatgc ccaccactgg catcaacgag     840

tactgcttct ccgtgcagaa aaccaacctg cggatcgtgg acgtcggggg ccagaagtca     900

gagcgtaaga aatggatcca ttgtttcgag aacgtgatcg ccctcatcta cctggcctca     960

ctgagtgaat acgaccagtg cctggaggag aacaaccagg agaaccgcat gaaggagagc    1020

ctcgcattgt ttgggactat cctggaacta ccctggttca aaagcacatc cgtcatcctc    1080

tttctcaaca aaaccgacat cctggaggag aaaatcccca cctcccacct ggctacctat    1140

ttccccagtt tccagggccc taagcaggat gctgaggcag ccaagaggtt catcctggac    1200

atgtacacga ggatgtacac cgggtgcgtg gacggccccg agggcagcaa gaagggcgca     1260

cgatcccgac gcctcttcag ccactacaca tgtgccacag acacacagaa catccgcaag    1320

gtcttcaagg acgtgcggga ctcggtgctc gcccgctacc tggacgagat caacctgctg    1380

tgacccaggc cccacctggg gcaggcggca ccggcgggcg ggtgggaggt gggagtggct    1440

gcagggaccc ctagtgtccc tggtctatct ctccagcctc ggcccacacg caagggagtc     1500

gggggacgga cggcccgctg ctggccgctc tcttctctgc ctctcaccag gacagccgcc    1560

ccccagggta ctcctgccct tgcttgactc agtttccctc ctttgaaagg gaaggagcaa    1620

aacggccatt tgggatgcca gggtggatga aaaggtgaag aaatcagggg attgaggact    1680

tgggtgggtg ggcatctctc aggagcccca tctccgggcg tgtcacctcc tgggcagggt    1740

tctgggaccc tctgtgggtg acgcacaccc tgggatgggg ctagtagagc cttcaggcgc 1800

cttcgggcgt ggactctggc gcactctagt ggacaggaga aggaacgcct tccaggaacc 1860

tgtggactag gggtgcaggg acttcccttt gcaaggggta acagaccgct ggaaaacact 1920

gtcactttca gagctcggtg gctcacagcg tgtcctgccc cggtttgcgg acgagagaaa 1980

tcgcggccca caagcatccc cccatccctt gcaggctggg ggctgggcat gctgcatctt 2040

aaccttttgt atttattccc tcaccttctg cagggctccg tgcgggctga aattaaagat 2100

ttcttagagg ctgcgtcgcc agcgtcctgt tt 2132

<210> 22
<211> 3798
<212> DNA
<213> Homo sapiens

<400> 22
ggggcaagtg actcatgagc acgagttgtt aagcgatgga atttgggaga tcaagccaca 60

ctgcttaaaa catcacatga tctccctctg gccccgtatt tcataaaaca gagcggatcg 120

caggaggccg acactgtgac tcctggtgga tgggactggg gagtcagagt caagccctga 180

ctggttgcag gcgctcggag tcagcatgga aagtctctgc ggggtcctgg gatttctgct 240

gctggctgca ggactgcctc tccaggctgc caagcgattt cgtgatgtgc tgggccatga 300

acagtatccc gatcacatga gagagcacaa ccaattacgt ggctggtctt cggatgaaaa 360

tgaatgggat gaacacctgt atccagtgtg gaggagggga gacggcaggt ggaaggactc 420

ctgggaagga ggccgtgtgc aggcagtcct gaccagtgac tcaccggctc tggtgggttc 480

caatatcact tttgtggtga acctggtgtt ccccagatgc cagaaggaag atgctaatgg 540

caatatcgtc tatgagaaga actgcaggaa tgatttggga ctgacatctg acctgcatgt 600

ctacaactgg actgcagggg cagatgatgg tgactgggaa gatggcacca gccgaagcca 660

gcatctcagg ttcccggaca ggaggccctt ccctcgcccc catggatgga agaaatggag 720

ctttgtctac gtctttcaca cacttggcca gtatttccaa aaactgggtc ggtgttcagc 780

acgggtttct ataaacacag tcaacttgac agctggccct caggtcatgg aagtgactgt 840

ctttcgaaga tacggccggg catacattcc catctcgaag gtgaaagatg tgtatgtgat 900

aacagatcag atccctgtat tcgtgaccat gtcccagaag aatgacagga acttgtctga 960

tgagatcttc ctcagagacc tccccatcgt cttcgatgtc ctcattcatg atcccagcca 1020

cttcctcaac gactctgcca tttcctacaa gtggaacttt ggggacaaca ctggcctgtt 1080

tgtctccaac aatcacactt tgaatcacac ttatgtgctc aatggaacct tcaaccttaa 1140

cctcaccgtg caaactgcag tgcccgggcc atgccctccc ccttcgcctt cgactccgcc 1200

tccaccttca actccgccct cacctccgcc ctcacctctg cccacattat caacacctag 1260

cccctcttta atgcctactg gttacaaatc catggagctg agtgacattt ccaatgaaaa 1320

ctgccgaata aacagatatg gctacttcag agccaccatc acaattgtag aggggatcct 1380

ggaagtcagc atcatgcaga tagcagatgt ccccatgccc acaccgcagc ctgccaactc 1440

cctgatggac ttcactgtga cctgcaaagg ggccaccccc atggaagcct gtacgatcat 1500

ctccgacccc acctgccaga tcgcccagaa ccgggtctgc agccctgtgg ctgtggatgg 1560

gctgtgcctg ctgtctgtga gaagagcctt caatgggtct ggcacctact gtgtgaattt 1620

cactctggga gatgatgcaa gcctggccct caccagcacc ctgatctcta tccctggcaa 1680

agacccagac tcccctctga gagcagtgaa tggtgtcctg atctccatcg gctgcctggc 1740

tgtgcttgtc accatggtta ccatcttgct gtacaaaaaa cacaaggcgt acaagccaat 1800

aggaaactgc cccaggaaca cggtcaaggg caagggcctg agtgttctcc tcagtcacgc 1860

gaaagccccg ttcttccgag gagaccagga gaaggatcca ttgctccagg acaagccaag 1920

gacactctaa gtctttggcc ttccctctga ccaggaaccc actcttctgt gcatgtatgt 1980

gagctgtgca gaagtatgtg gctgggaact gttgttctct aaggattatt gtaaaatgta 2040

tatcgtggct tagggagtgt ggttaaatag cattttagag aagacatggg aagacttagt 2100

gtttcttccc atctgtattg tggtttttac actgttcgtg gggtggacac gctgtgtctg 2160

aaggggaggt ggggtcactg ctacttaagg tcctaggtta actgggggag ataccacaga 2220

tgcctcagct ttccacataa catgggcatg aacccagcta atcaccacct gaaggccatg 2280

cttcatctgc cttccaactc actgagcatg cctgagctcc tgacaaaatt ataatgggcc 2340

cgggctttgt gtatggtgcg tgtgtgtaca tattctactc attaaaaagg cagtctaata 2400

agctgtgtga ttattatatt ggggagaaaa ttttccctgt gtagttcagt gaactaccca 2460

tccattcatt tatccatgga cacttataaa gcatgtggtg ggctgcactt gacctatgag 2520

accttgtcta ttctatacca gcagaccctt aatagcaacc aagtgagatc tgagagtcca 2580

gactgtatct accttgatga aggtagacaa ctggataaat atgatggtat tatagaatga 2640

ctgctcattt ttgaggcatt gtggaggacc aacattcagt ctggtacttt gacacccccc 2700

ccccccacta ggtggcctag aagcaaaaag aagctttttg tttggaaatt gtctccaaag 2760

aatgtaaaaa ttgctgacca cttaggacag ggcctctcag ccaggagtgt ttggcaatgt 2820

ttggagagat ttgtggttgt caaaactagg gtggggtgct actggcatct agtggggaga 2880

gcccagggct gctgaacacc ctgtgcctgc gggacagccc cacagccaag gagcccagag 2940

caactgaagg agtcctgggc acaggcatcc caacagcagc cttgtatctg aaccgagccc 3000

tgacatctaa agctttgtga cctagtgaca tggcatgtta ggagtctttt ctgcctctgt 3060

ctcagctgta gaaggggtat gctgtccctc tctgactcac ttcacatggc cgctactgct 3120

cctgatggaa gaatgagtgc aggtacagcc ttgtgttaca gtctcagcct ggggacagaa 3180

acacgtgaag aagcttgatt gattgattga ttgattgatt gattgattga ttgattgatt 3240

gattgatttt tacatactaa ggagcccgtt cactagtacc caaaacagga cattgatttc 3300

aggttgctgc agaatccaca gaccgcttag actccacccc atggttccaa acactgtctt 3360

gtggaggtgg gaccccataa tttggatttc tgagaaacca acagagaatc cttattctgc 3420

tggttccagg acaagaatga gcaaggctgg gcttgggatc acttccttgc tcaggtcaac 3480

agggggcagc agagaacagc agatacagaa cagccaccaa agcggcaggt ccatgacttt 3540

gtttatctag cttcatctaa acaaatttta atctagtagt aggaaagaag tctgaaatag 3600

taaattgtgt cgatttatat tttccaagtg accctggtaa gggaactgtc tgcagaatgg 3660

aagaaatagc ctaagagaca gggatggctt gattgatgtt ggctgagaat taaaacttct 3720

cacgaaaagt acatgtgtat gtaacaaatt attccaggaa ttgcttattt ctagggcttt 3780

tataaatgtc tgcatcct 3798

<210> 23
<211> 2996
<212> DNA
<213> Homo sapiens

<400> 23
atgtggtgcg gcgggaggaa gtgcggcttg ttttcccggc taggctctgg agcggcgggc 60

gcggcgcgat gcgcgggtac ccgggagcga acggctgcga gccctgatga agctcgagca 120

gccccagcct gatggaggcg cctccgtggg agccggtgcg caatgactcc ctgcctccca 180

cgctgagccc cgcggtgccg ccctacgtga agctcggcct caccgcggtc tacaccgtct 240

tctacgcgct cctcttcgtg ttcatctatg cgcagctctg gctggtgctg cgctaccgtc 300

acaagcggct cagctaccag agcgtcttcc tcttcctttg cctcttctgg gcctcgctgc 360

gtaccgtgct cttctccttt tacttccgag acttcgtggc agccaactcg ttcagcccct 420

tcgtcttctg gctgctctac tgcttccccg tgtgtctaca gttcttcacc ctcacgctca 480

tgaacttgta cttcacgcag gtgattttca aggccaagtc aaaatattct ccagagctac 540

tcaaataccg gttacccctc tacctggcct cacttttcat cagcctcgtt ttcctgttgg 600

tgaatctgac ctgtgctgtg ctggtgaaga cgggagactg ggacaggaag gttatcgtct 660

ctgtgagagt ggccatcaat gacacactct ttgtgctgtg tgctatctct ctctccatct 720

gcctctacaa aatctccaag atgtccctgg ccaacatcta cttggagtca aagggctcat 780

cagtgtgtca ggtaactgcc attggtgtca ccgtcatctt gctctacacc tctcgggcct 840

gctacaacct gttcatcttg tcattttctc agatcaagaa cgtccattcc tttgattatg 900

actggtacaa tgtatccgac caggcagatc tgaagagcca gctgggtgac gccggctacg 960

tagtgtttgg cgtggtgctt ttcgtgtggg agctcctacc caccaccttg gtggtttatt 1020

tcttccgagt cagaaatccc acgaaggatc ttaccaatcc tgggatggtc cccagccatg 1080

gattcagtcc cagatcttac ttctttgaca acccccgaag atatgacagt gatgatgacc 1140

ttgcctggaa cattgcccct cagggacttc agggaagttt tgctccagac tactatgatt 1200

ggggacaaca aaataacagc ttcctggcac aagcaggaac tttgcatcaa gactccactt 1260

tggatccaga caaagcaagc caagggtagc agcagctgac acagccctat ggaagagttc 1320

tctgttgaaa gccttcagcc agacagaccg gatgacagct gagttgctaa ggcagttttc 1380

cttaggaaac agaactctag tttttgctat agctttctca tggctccaca gggctaagca 1440

ataatttaga gcaataaact ctttagtact agcagagaat ctggctattt cagtgggtat 1500

aatttaaact tataaaagag gttctgtact tttataaaga tgtattttat ataacttaaa 1560

tactaatgct aaagtatact aggtttttcc ttgattgtta attgcaacgt atgttgtagt 1620

ttgcacagac tttcatgcat aattcacttt aaaacgtata gaatacgtgg tctaatagtt 1680

taaagctttg gggaaagttt ccacaaatct tacctctgaa ggtccctctt gtgagtgcca 1740

cgtggtgggc tcctttcacc gccactcaag cattccaagt tcaggagaag cagagtacca 1800

tggtctgtac gtaacaggct caacagcagc agcagcagca gcagcagcag cagcaatttc 1860

tcgtaaactc tgtcctaagc ctggtccttc ttcatctgaa agcactacta caagcactcc 1920

agtaacaagt ggatactgtt aagatgtagt tgctgacact attaaacctc ctctgctgtg 1980

tgtgtagcca ttttgtagag ttttcttcag cccggtgtaa ctgaatactc actacctcag 2040

tacctcagta ggatgcaagg actgtgcctt ctttgactca gccagtgctt gctatagtca 2100

ggctacaagc caagaggtcc ccacagagta ttaacaataa atacttctgg ccttcaagct 2160

ctaaaggatt gcagactcct gacagctttt ctgtaagaca tgcctgtcat ttgtatgagg 2220

ctgacacggg gctcactgcc tgttattttt agatagtgtt ctattaaaag ctatgtgtat 2280

gagaaagtag gctctgccta cgtggcggca gcatccccat atcagccaga gagtgttcca 2340

gacagtggtc ctcttgtgcc atctccgtgc tgtcctggga aaacgctgga acaacgtggc 2400

cctcctggag gccggatgct ctggcttctt ctggtcctat aggtcacaga gccacgccca 2460

ctttctccca ttttgttata tttaagattg ggagcccagt ttccagtggg ttgtaatggg 2520

gtcttcgttg tacagaggac gacgtggagg aacttgcgca gccccgcccc acaccctgcc 2580

tttgggtttg agtaaacatc tgggttgcaa gccatttaaa attgcttctt tgccagggtg 2640

aattctggca aagcctacac aaatcgcctg tacgatagca ctgtataaaa gtttatctgt 2700

caccatacct gcaatgattt ggctgttgca gccagtgccc acagctcctg tgtccttgtc 2760

tctagcaggg cggtacctgt cagcagccaa gggcaaggct tgctggaaag cacagcctca 2820

ttaaggctgt tctgtttgca cccattttgt agcacatgca cactttacag ttggtgaatg 2880

ctgggcgtct gttttcttac agtaacaagc aagctatcat ccatttttac aataaagttg 2940

tcagcattca tgtcagcaat aaaaagacta cagctcttaa aaaaaaaaaa aaaaaa 2996

<210> 24
<211> 4762
<212> DNA
<213> Homo sapiens

<400> 24
aagggaggga cctggaggac tcagggtagg attgtagagc tcagggacag ggagtggagg 60

gcgccgcggt gagaccgctg ttgtgtagtt gttagactgg gcggggctgg gacgcaggcg 120

ggacccgtgg ggacgcgagc ccgggcgcca aatgcccaga cagaggcgac cgcgggctcg 180

gcttggccac gacctctcgg cacccttagg cctgtgcacc ctggctgtct gcccttggtc 240

cccaacccac catgccctca cccgcgcctc ccaccgagct gctgccgtgg gagcgcgcgg 300

tggtgctgct gtcgtgtgcg ctgtcagctc tgggctcggg cctcctggtg gccacgcacg 360

ccctgtggcc tgacctgcgt agccgggcgc ggcgcctgct actcttcctg tcgctagcgg 420

acctgctctc ggctgcctcg tacttctacg gagtgctgca ggactttgcg ggcacttcgt 480

gggattgcgt ccttcagggc gctctctcta ctttcgccaa caccagctcc ttcttctgga 540

cggtggccat cgccctctac ctatacctca gcatcgtccg aactacgcgc gggccctcca 600

cggaccacct aatctgggct tttcacctca tcagctgggg tgtcccgttg gccatcacag 660

tggcagccgt ctctctaaag aagataggct atgatgcctc ggatgtgtct gtgggctggt 720

gctggatcaa cctggaggct gaggaccgtg tcctgtggat gctactgacc gggaagctgt 780

gggagatgct ggcttatatc ttgctacctc tgctgtacct tttggtcaga aagcacatca 840

acagggcgca ccaggcgctc tcggagtacc ggcccatctg cgaggggcgc cagctgcagc 900

gaggctcctc cacttccacg gcagataaga agttggtcct cattccgctc atattcatct 960

gcctccgcgt ctggagcacc gtgcgctttg tcctgacgct ctgtggttcc ccggctgtac 1020

agacacccgt gctggttgtt ctgcatggca ttggaaacac cttccaggga ggggccaact 1080

gcatcatgtt cgtcctctgt acccgggcag tccgcacaag gctcttttct ctttgctgct 1140

gctgtcctcg gccctccacc cagagccctc cgggggctcc tacgcccccc aagataggag 1200

aatctcagga atccagacgg accccagaag tgcccagcac ttgagcggtt ggctttcctc 1260

cctgtccgta ctggcgctgc cttcctggtt cctgcttcag gattaggaga ccaagcatgc 1320

tgtcagcctg gcctaaagtg aagatcggag cccagtggag ggacagccat cagtatggac 1380

tcttctacct cccagacttg caggcaggca gtgtgttcct tgcacactca tgtcctggtt 1440

cagtggggtt tgtttgtgta agcacaaaga ccgtgagact cagctatgcg ggataccgcc 1500

agggcattgg ttctcagtgt ccccggccac caacgttgtt tacagttgga gggatccgta 1560

cctgtggacc catcccaggc ttgactgaat aataatgaac accatcttgg atcccagaca 1620

tcgcagtatg gagtccgggc ttgccagggc gttgagaatc cctagagacc ctgggggtaa 1680

ttagcagtgc tggtcagctg agggtaatat ccttaaacta ggtcccaagt ccacggaagt 1740

cacaggagac tgccatcatt gatgaaaacc acccgggcac acactcatgg gcatctcacg 1800

gaatctctgc gtgggtcatc gcccctcagt gtttattaca tctgaatttc agcacaggcc 1860

agagtccaga gtcctgtcca ttagtcaatc acttaacatc ctcgttccct ccaggtacca 1920

ggacagaagg cagctcagct ccagccccta tggacaatta ccagttcacc tactgttctc 1980

acagagccct ctcctcacgg tgactcatac agggggaggg ggtctggctc tgcactttgc 2040

ttaggaaagg tgaccagtga atggatctga ttccaccctg aaggtgaatg tttgaagagt 2100

cagcctcggg ctggcctgta gctccgtgta gtttccaggt tggtcctagc accccaaata 2160

cttaaaaggc ctttgtgttt tgtagttatc tgggggatat agaaatagga ttctgagatg 2220

attacatttt attttgtttt tttgagacag ggtctcatgt ggacatgctg gcctggaact 2280

tgttatgtag atcaggctgg cctccaactc acagagatcc acctgcctct gcctcctgat 2340

agttacattt ttaaaaaaat gttgtgttgt gttgtgcctg ttaggcaggt gctccgccac 2400

tgagttcaat cccatgcccc tccccactcc cttttttttg agacataatc ttactaagtt 2460

gcctagactg gccttcaact ctcagtcgac caggctggtc ttgaacttaa gattttccta 2520

cctcaacttc ccaggaagct agaattgtcg cctttggcta gcaagcctgg ccagagcggg 2580

gcttaactga gatctgctca gtggctcttc cacccacaca tccctcattt cagaaagcag 2640

tgtgtgccga cgtgcaggct ctcggtgtgg gaagtgtgcc cgccaccttg cagtcaaggt 2700

gaggtagcgc ccttggattc tgtcaatacc ctcacaaaac cgagaggatg ctcagagcct 2760

ggctcaagat ggaagcagag tcacacgggg catcagcgcc catctctatc aggaaaggaa 2820

tagtcgcgca gagggctgtg tctgtatgct cctcttctgg cttcctaaag catttcttta 2880

ggaaactcta caagaggtgg gttccctttt gggagtctaa gaaagctcct tggcttggta 2940

ctctgtctgc ttacaaaacc agtctgtgta gggtgtcctg agatcctgtg aagatattac 3000

agccaatgtg tgcactgtca gcgtgtagcg cagcggtaat ggatctcctg gcttctaaaa 3060

tggaacagaa attctaggaa tttgtctcct ggaggggaga aaataaacca ccaaatacta 3120

cggaggaaat gatgttggat aaacccagct ggacagagca gtgtgaaacc ctggctgttt 3180

ggggctttgt tcaaggaaag gcaggcaggg ggcatccgaa agccctgctc ctggagcctg 3240

gggcttaccc aagttttctc tgatgccact ggccaggatt gctgtccgta aaacccccta 3300

caaagtctac acttggctct ttccatctgt ttcccatgct gccctgtcac cgaaggtggc 3360

tggtggtgac ccgaggcccg gtgcctgacc tctagcccta cgtgctcaca gacatcaacc 3420

ttagctcagc cggtggccaa gtgggtcttc taggcagcca gggttcagag ctgaagagga 3480

atagaagaat ccttggggcc aggaggggcc ttagcaccgg tgaggaccag ctcccctttg 3540

ctttgctcag cctcgtgggt tcctatctct actaggaagt gtcattaata tctgtctgtc 3600

cgtgttcctc tgtcacccaa atgtgacctc agccttaatg ggtgctacag cccccacacc 3660

cttcttaagc actttatgaa tttttttttt ccgagacagg gtttctctgt atagccctgg 3720

ctgtcctgga actcactctg tagaccaggc tggcctcgaa ctcagaaatc cgcctgcctc 3780

tgcctcctga gtgctgggat taaagacgtg tgtcaccacc gcctggtaca ctttgtgaat 3840

cttgttctgc aaatccgttg ccagctgctt cctgcctttg actttctgag gcagagagag 3900

agagagggag agagggagag agggagagag agagggagag aatgtgtgtg tgcgtgagat 3960

attatgctct agcatttatc tctcttcatt tttttttttt gttttgtttt ttaagtttcg 4020

aaacagggtt ctcaggctgg agagatggct cagcggctaa gagcactgac tgctcttccg 4080

aaggtcctga gttcaaattc cagcaaccac atggtggctc acaaccattt gtaatgagat 4140

ctgatgccct cttctggtgt gtctgaggac agatacagtg tacttatata taataaataa 4200

ataaatcgtt aatttttttt ttaaaaaaga atcagggttc tcactacttg tccaggcagg 4260

ccttaagcag ttttactgtt tctgggatta caggcctatg ccaccccgag ccggcttttt 4320

agtttgttct ctctgtctct ctgtctctct gtctctctct ctctctctct ctctctctct 4380

ctctctctct ctctctctct ctttctctct ctatgtgtgt ctctttctct ctctgcctct 4440

ctgtctgtct gtctctctct ctgcttctct ctctgaaaat aaaaataaaa actattatgt 4500

gtccaaagta caaatgagac catatgtgtt tgtttgtatg tgtacgggta ttttgtgtgt 4560

acgcagttcc cttagaggcc agagggactc tgatcccctg aatctggagt catagctgtg 4620

aactctgggc aaagggaact aagcctgggt cctttgcagg aacagcaacg ctcttatctg 4680

ttgagctagc tctccagccc tctctgcaaa tgtttgtgtg aactcacagt tgtggtataa 4740

ttaaacctgg catgagattt tt 4762

<210> 25
<211> 1919
<212> DNA
<213> Homo sapiens

<400> 25
gcagtcatct gactcggtga ctcacccgcg gccgcgcttc ctctgatccg ggccgggcgg 60

gaagtcgggt cccgaggctc cggctcggca gaccgggcgg aaagcagccg agcggccatg 120

gagctgtgcg ggctggggct gccccggccg cccatgctgc tggcgctgct gttggcgaca 180

ctgctggcgg cgatgttggc gctgctgact caggtggcgc tggtggtgca ggtggcggag 240

gcggctcggg ccccgagcgt ctcggccaag ccggggccgg cgctgtggcc cctgccgctc 300

tcggtgaaga tgaccccgaa cctgctgcat ctcgccccgg agaacttcta catcagccac 360

agccccaatt ccacggcggg cccctcctgc accctgctgg aggaagcgtt tcgacgatat 420

catggctata tttttggttt ctacaagtgg catcatgaac ctgctgaatt ccaggctaaa 480

acccaggttc agcaacttct tgtctcaatc acccttcagt cagagtgtga tgctttcccc 540

aacatatctt cagatgagtc ttatacttta cttgtgaaag aaccagtggc tgtccttaag 600

gccaacagag tttggggagc attacgaggt ttagagacct ttagccagtt agtttatcaa 660

gattcttatg gaactttcac catcaatgaa tccaccatta ttgattctcc aaggttttct 720

cacagaggaa ttttgattga tacatccaga cattatctgc cagttaagat tattcttaaa 780

actctggatg ccatggcttt taataagttt aatgttcttc actggcacat agttgatgac 840

cagtctttcc catatcagag catcactttt cctgagttaa gcaataaagg aagctattct 900

ttgtctcatg tttatacacc aaatgatgtc cgtatggtga ttgaatatgc cagattacga 960

ggaattcgag tcctgccaga atttgatacc cctgggcata cactatcttg gggaaaaggt 1020

cagaaagacc tcctgactcc atgttacagt agacaaaaca agttggactc ttttggacct 1080

ataaacccta ctctgaatac aacatacagc ttccttacta catttttcaa agaaattagt 1140

gaggtgtttc cagatcaatt cattcatttg ggaggagatg aagtggaatt taaatgttgg 1200

gaatcaaatc caaaaattca agatttcatg aggcaaaaag gctttggcac agattttaag 1260

aaactagaat ctttctacat tcaaaaggtt ttggatatta ttgcaaccat aaacaaggga 1320

tccattgtct ggcaggaggt ttttgatgat aaagcaaagc ttgcgccggg cacaatagtt 1380

gaagtatgga aagacagcgc atatcctgag gaactcagta gagtcacagc atctggcttc 1440

cctgtaatcc tttctgctcc ttggtactta gatttgatta gctatggaca agattggagg 1500

aaatactata aagtggaacc tcttgatttt ggcggtactc agaaacagaa acaacttttc 1560

attggtggag aagcttgtct atggggagaa tatgtggatg caactaacct cactccaaga 1620

ttatggcctc gggcaagtgc tgttggtgag agactctgga gttccaaaga tgtcagagat 1680

atggatgacg cctatgacag actgacaagg caccgctgca ggatggtcga acgtggaata 1740

gctgcacaac ctctttatgc tggatattgt aaccatgaga acatgtaaaa aatggagggg 1800

aaaaaggcca cagcaatctg tactacaatc aactttattt tgaaatcatg taaaataaga 1860

tattagactg ttttttgaat aaaatatttt tattgattga aaaaaaaaaa aaaaaaaaa 1919

<210> 26
<211> 1428
<212> DNA
<213> Homo sapiens

<400> 26
ggccggcggc atccgaggcg catgactagt tggggccaaa ccagtgctcc tgccacctct 60

ctggctgccc cctagagcct gcccatccca gcctgaccaa tgtccacagc cagggagcag 120

ccaatcttca gcacacgggc gcacgtgttc caaattgacc cagccaccaa gcgaaactgg 180

atcccagcgg gcaagcacgc actcactgtc tcctatttct acgatgccac ccgcaatgtg 240

taccgcatca tcagcatcgg aggcgccaag gccatcatca acagcactgt cactcccaac 300

atgaccttca ccaaaacttc ccagaagttc gggcagtggg ccgacagtcg cgccaacaca 360

gtctacggcc tgggctttgc ctctgaacag catctgacac agtttgccga gaagttccag 420

gaagtgaagg aagcagccag gctggccagg gagaaatctc aggatggcgg ggagctcacc 480

agtccagccc tggggctcgc ctcccaccag gtgcccccga gccctctcgt cagtgccaac 540

ggccccggcg aggaaaaact gttccgcagc cagagcgctg atgcccccgg ccccacagag 600

cgcgagcggc taaagaagat gttgtctgag ggctccgtgg gcgaggtaca gtgggaggcc 660

gagtttttcg cactgcagga cagcaacaac aagctggcag gcgccctgcg agaggccaac 720

gccgccgcag cccagtggag gcagcagctg gaggctcagc gtgcagaggc cgagcggctg 780

cggcagcggg tggctgagct ggaggctcag gcagcttcag aggtgacccc caccggtgag 840

aaggaggggc tgggccaggg ccagtcgctg gaacagctgg aagctctggt gcaaaccaag 900

gaccaggaga ttcagaccct gaagagtcag actgggggcc cccgcgaggc cctggaggct 960

gccgagcgtg aggagactca gcagaaggtg cagacccgca atgcggagtt ggagcaccag 1020

ctgcgggcga tggagcgcag cctggaggag gcacgggcag agcgggagcg ggcgcgggct 1080

gaggtgggcc gggcagcgca gctgctggac gtcagcctgt ttgagctgag tgagctgcgt 1140

gagggcctgg cccgcctggc tgaggctgcg ccctgagccg ggctggtttt tctatgaacg 1200

attccggcct gggatgcggg ccaggctgca ggcggcatag ttgggcccat tcgtcctgga 1260

aagggactgg ggggtcccaa cttagccctg ggtgggccgg gccgggctgg gctggggtgg 1320

gccccagtcg gctctggttg ttggcagctt tggggctgtt tttgagcttc tcattgtgta 1380

gaatttctag atcccccgat tacatttcta agcgtggcaa aaaaaaaa 1428

<210> 27
<211> 3249
<212> DNA
<213> Homo sapiens

<400> 27
caagcttagc ctggccggga aacgggaggc gtggaggccg ggagcagccc ccggggtcat 60

cgccctgcca ccgccgcccg attgctttag cttggaaatt ccggagctga agcggccagc 120

gagggaggat gaccctctcg gcccgggcac cctgtcagtc cggaaataac tgcagcattt 180

gttccggagg ggaaggcgcg aggtttccgg gaaagcagca ccgccccttg gcccccaggt 240

ggctagcgct ataaaggatc acgcgcccca gtcgacgctg agctcctctg ctactcagag 300

ttgcaacctc agcctcgcta tggctcccag cagcccccgg cccgcgctgc ccgcactcct 360

ggtcctgctc ggggctctgt tcccaggacc tggcaatgcc cagacatctg tgtccccctc 420

aaaagtcatc ctgccccggg gaggctccgt gctggtgaca tgcagcacct cctgtgacca 480

gcccaagttg ttgggcatag agaccccgtt gcctaaaaag gagttgctcc tgcctgggaa 540

caaccggaag gtgtatgaac tgagcaatgt gcaagaagat agccaaccaa tgtgctattc 600

aaactgccct gatgggcagt caacagctaa aaccttcctc accgtgtact ggactccaga 660

acgggtggaa ctggcacccc tcccctcttg gcagccagtg ggcaagaacc ttaccctacg 720

ctgccaggtg gagggtgggg caccccgggc caacctcacc gtggtgctgc tccgtgggga 780

gaaggagctg aaacgggagc cagctgtggg ggagcccgct gaggtcacga ccacggtgct 840

ggtgaggaga gatcaccatg gagccaattt ctcgtgccgc actgaactgg acctgcggcc 900

ccaagggctg gagctgtttg agaacacctc ggccccctac cagctccaga cctttgtcct 960

gccagcgact cccccacaac ttgtcagccc ccgggtccta gaggtggaca cgcaggggac 1020

cgtggtctgt tccctggacg ggctgttccc agtctcggag gcccaggtcc acctggcact 1080

gggggaccag aggttgaacc ccacagtcac ctatggcaac gactccttct cggccaaggc 1140

ctcagtcagt gtgaccgcag aggacgaggg cacccagcgg ctgacgtgtg cagtaatact 1200

ggggaaccag agccaggaga cactgcagac agtgaccatc tacagctttc cggcgcccaa 1260

cgtgattctg acgaagccag aggtctcaga agggaccgag gtgacagtga agtgtgaggc 1320

ccaccctaga gccaaggtga cgctgaatgg ggttccagcc cagccactgg gcccgagggc 1380

ccagctcctg ctgaaggcca ccccagagga caacgggcgc agcttctcct gctctgcaac 1440

cctggaggtg gccggccagc ttatacacaa gaaccagacc cgggagcttc gtgtcctgta 1500

tggcccccga ctggacgaga gggattgtcc gggaaactgg acgtggccag aaaattccca 1560

gcagactcca atgtgccagg cttgggggaa cccattgccc gagctcaagt gtctaaagga 1620

tggcactttc ccactgccca tcggggaatc agtgactgtc actcgagatc ttgagggcac 1680

ctacctctgt cgggccagga gcactcaagg ggaggtcacc cgcaaggtga ccgtgaatgt 1740

gctctccccc cggtatgaga ttgtcatcat cactgtggta gcagccgcag tcataatggg 1800

cactgcaggc ctcagcacgt acctctataa ccgccagcgg aagatcaaga aatacagact 1860

acaacaggcc caaaaaggga cccccatgaa accgaacaca caagccacgc ctccctgaac 1920

ctatcccggg acagggcctc ttcctcggcc ttcccatatt ggtggcagtg gtgccacact 1980

gaacagagtg gaagacatat gccatgcagc tacacctacc ggccctggga cgccggagga 2040

cagggcattg tcctcagtca gatacaacag catttggggc catggtacct gcacacctaa 2100

aacactaggc cacgcatctg atctgtagtc acatgactaa gccaagagga aggagcaaga 2160

ctcaagacat gattgatgga tgttaaagtc tagcctgatg agaggggaag tggtggggga 2220

gacatagccc caccatgagg acatacaact gggaaatact gaaacttgct gcctattggg 2280

tatgctgagg ccccacagac ttacagaaga agtggccctc catagacatg tgtagcatca 2340

aaacacaaag gcccacactt cctgacggat gccagcttgg gcactgctgt ctactgaccc 2400

caacccttga tgatatgtat ttattcattt gttattttac cagctattta ttgagtgtct 2460

tttatgtagg ctaaatgaac ataggtctct ggcctcacgg agctcccagt cctaatcaca 2520

ttcaaggtca ccaggtacag ttgtacaggt tgtacactgc aggagagtgc ctggcaaaaa 2580

gatcaaatgg ggctgggact tctcattggc caacctgcct ttccccagaa ggagtgattt 2640

ttctatcggc acaaaagcac tatatggact ggtaatggtt acaggttcag agattaccca 2700

gtgaggcctt attcctccct tccccccaaa actgacacct ttgttagcca cctccccacc 2760

cacatacatt tctgccagtg ttcacaatga cactcagcgg tcatgtctgg acatgagtgc 2820

ccagggaata tgcccaagct atgccttgtc ctcttgtcct gtttgcattt cactgggagc 2880

ttgcactatg cagctccagt ttcctgcagt gatcagggtc ctgcaagcag tggggaaggg 2940

ggccaaggta ttggaggact ccctcccagc tttggaagcc tcatccgcgt gtgtgtgtgt 3000

gtgtatgtgt agacaagctc tcgctctgtc acccaggctg gagtgcagtg gtgcaatcat 3060

ggttcactgc agtcttgacc ttttgggctc aagtgatcct cccacctcag cctcctgagt 3120

agctgggacc ataggctcac aacaccacac ctggcaaatt tgattttttt tttttttcca 3180

gagacggggt ctcgcaacat tgcccagact tcctttgtgt tagttaataa agctttctca 3240

actgccaaa 3249

<210> 28
<211> 4909
<212> DNA
<213> Homo sapiens

<400> 28
tagacgcacc ctctgaagat ggtgactccc tcctgagaag ctggacccct tggtaaaaga 60

caaggccttc tccaagaaga atatgaaagt gttactcaga cttatttgtt tcatagctct 120

actgatttct tctctggagg ctgataaatg caaggaacgt gaagaaaaaa taattttagt 180

gtcatctgca aatgaaattg atgttcgtcc ctgtcctctt aacccaaatg aacacaaagg 240

cactataact tggtataaag atgacagcaa gacacctgta tctacagaac aagcctccag 300

gattcatcaa cacaaagaga aactttggtt tgttcctgct aaggtggagg attcaggaca 360

ttactattgc gtggtaagaa attcatctta ctgcctcaga attaaaataa gtgcaaaatt 420

tgtggagaat gagcctaact tatgttataa tgcacaagcc atatttaagc agaaactacc 480

cgttgcagga gacggaggac ttgtgtgccc ttatatggag ttttttaaaa atgaaaataa 540

tgagttacct aaattacagt ggtataagga ttgcaaacct ctacttcttg acaatataca 600

ctttagtgga gtcaaagata ggctcatcgt gatgaatgtg gctgaaaagc atagagggaa 660
ctatacttgt catgcatcct acacatactt gggcaagcaa tatcctatta cccgggtaat 720
agaatttatt actctagagg aaaacaaacc cacaaggcct gtgattgtga gcccagctaa 780
tgagacaatg gaagtagact tgggatccca gatacaattg atctgtaatg tcaccggcca 840
gttgagtgac attgcttact ggaagtggaa tgggtcagta attgatgaag atgacccagt 900
gctaggggaa gactattaca gtgtggaaaa tcctgcaaac aaaagaagga gtaccctcat 960
cacagtgctt aatatatcgg aaattgaaag tagattttat aaacatccat ttacctgttt 1020
tgccaagaat acacatggta tagatgcagc atatatccag ttaatatatc cagtcactaa 1080
tttccagaag cacatgattg gtatatgtgt cacgttgaca gtcataattg tgtgttctgt 1140
tttcatctat aaaatcttca agattgacat tgtgctttgg tacagggatt cctgctatga 1200
ttttctccca ataaaagctt cagatggaaa gacctatgac gcatatatac tgtatccaaa 1260
gactgttggg gaagggtcta cctctgactg tgatattttt gtgtttaaag tcttgcctga 1320
ggtcttggaa aaacagtgtg gatataagct gttcatttat ggaagggatg actacgttgg 1380
ggaagacatt gttgaggtca ttaatgaaaa cgtaaagaaa agcagaagac tgattatcat 1440
tttagtcaga gaaacatcag gcttcagctg gctgggtggt tcatctgaag agcaaatagc 1500
catgtataat gctcttgttc aggatggaat taaagttgtc ctgcttgagc tggagaaaat 1560
ccaagactat gagaaaatgc cagaatcgat taaattcatt aagcagaaac atggggctat 1620
ccgctggtca ggggacttta cacagggacc acagtctgca aagacaaggt tctggaagaa 1680
tgtcaggtac cacatgccag tccagcgacg gtcaccttca tctaaacacc agttactgtc 1740
accagccact aaggagaaac tgcaaagaga ggctcacgtg cctctcgggt agcatggaga 1800
agttgccaag agttctttag gtgcctcctg tcttatggcg ttgcaggcca ggttatgcct 1860
catgctgact tgcagagttc atggaatgta actatatcat cctttatccc tgaggtcacc 1920
tggaatcaga ttattaaggg aataagccat gacgtcaata gcagcccagg gcacttcaga 1980
gtagagggct tgggaagatc ttttaaaaag gcagtaggcc cggtgtggtg gctcacgcct 2040
ataatcccag cactttggga ggctgaagtg ggtggatcac cagaggtcag gagttcgaga 2100
ccagcccagc caacatggca aaaccccatc tctactaaaa atacaaaaat gagctaggca 2160
tggtggcaca cgcctgtaat cccagctaca cctgaggctg aggcaggaga attgcttgaa 2220
ccggggagac ggaggttgca gtgagccgag tttgggccac tgcactctag cctggcaaca 2280
gagcaagact ccgtctcaaa aaaagggcaa taaatgccct ctctgaatgt ttgaactgcc 2340
aagaaaaggc atggagacag cgaactagaa gaaagggcaa gaaggaaata gccaccgtct 2400
acagatggct tagttaagtc atccacagcc caagggcggg gctatgcctt gtctggggac 2460
cctgtagagt cactgaccct ggagcggctc tcctgagagg tgctgcaggc aaagtgagac 2520

tgacacctca ctgaggaagg gagacatatt cttggagaac tttccatctg cttgtatttt 2580

ccatacacat ccccagccag aagttagtgt ccgaagaccg aattttattt tacagagctt 2640

gaaaactcac ttcaatgaac aaagggattc tccaggattc caaagttttg aagtcatctt 2700

agctttccac aggagggaga gaacttaaaa aagcaacagt agcagggaat tgatccactt 2760

cttaatgctt tcctccctgg catgaccatc ctgtcctttg ttattatcct gcattttacg 2820

tctttggagg aacagctccc tagtggcttc ctccgtctgc aatgtccctt gcacagccca 2880

cacatgaacc atccttccca tgatgccgct cttctgtcat cccgctcctg ctgaaacacc 2940

tcccaggggc tccacctgtt caggagctga agcccatgct ttcccaccag catgtcactc 3000

ccagaccacc tccctgccct gtcctccagc ttcccctcgc tgtcctgctg tgtgaattcc 3060

caggttggcc tggtggccat gtcgcctgcc cccagcactc ctctgtctct gctcttgcct 3120

cgacccttcc tcctcctttg cctaggaggc cttctcgcat tttctctagc tgatcagaat 3180

tttaccaaaa ttcagaacat cctccaattc cacagtctct gggagacttt ccctaagagg 3240

cgacttcctc tccagccttc tctctctggt caggcccact gcagagatgg tggtgagcac 3300

atctgggagg ctggtctccc tccagctgga attgctgctc tctgagggag aggctgtggt 3360

ggctgtctct gtccctcact gccttccagg agcaatttgc acatgtaaca tagatttatg 3420

taatgcttta tgtttaaaaa cattccccaa ttatcttatt taatttttgc aattattcta 3480

attttatata tagagaaagt gacctatttt ttaaaaaaat cacactctaa gttctattga 3540

acctaggact tgagcctcca tttctggctt ctagtctggt gttctgagta cttgatttca 3600

ggtcaataac ggtcccccct cactccacac tggcacgttt gtgagaagaa atgacatttt 3660

gctaggaagt gaccgagtct aggaatgctt ttattcaaga caccaaattc caaacttcta 3720

aatgttggaa ttttcaaaaa ttgtgtttag attttatgaa aaactcttct actttcatct 3780

attctttccc tagaggcaaa catttcttaa aatgtttcat tttcattaaa aatgaaagcc 3840

aaatttatat gccaccgatt gcaggacaca agcacagttt taagagttgt atgaacatgg 3900

agaggacttt tggtttttat atttctcgta tttaatatgg gtgaacacca acttttattt 3960

ggaataataa ttttcctcct aaacaaaaac acattgagtt taagtctctg actcttgcct 4020

ttccacctgc tttctcctgg gcccgctttg cctgcttgaa ggaacagtgc tgttctggag 4080

ctgctgttcc aacagacagg gcctagcttt catttgacac acagactaca gccagaagcc 4140

catggagcag ggatgtcacg tcttgaaaag cctattagat gttttacaaa tttaattttg 4200

cagattattt tagtctgtca tccagaaaat gtgtcagcat gcatagtgct aagaaagcaa 4260

gccaatttgg aaacttaggt tagtgacaaa attggccaga gagtgggggt gatgatgacc 4320

aagaattaca agtagaatgg cagctggaat ttaaggaggg acaagaatca atggataagc 4380

gtgggtggag gaagatccaa acagaaaagt gcaaagttat tccccatctt ccaagggttg 4440

aattctggag gaagaagaca cattcctagt tccccgtgaa cttcctttga cttattgtcc 4500

ccactaaaac aaaacaaaaa acttttaatg ccttccacat taattagatt ttcttgcagt 4560

ttttttatgg cattttttta aagatgccct aagtgttgaa gaagagtttg caaatgcaac 4620

aaaatattta attaccggtt gttaaaactg gtttagcaca atttatattt tccctctctt 4680

gcctttctta tttgcaataa aaggtattga gccatttttt aaatgacatt tttgataaat 4740

tatgtttgta ctagttgatg aaggagtttt ttttaacctg tttatataat tttgcagcag 4800

aagccaaatt ttttgtatat taaagcacca aattcatgta cagcatgcat cacggatcaa 4860

tagactgtac ttattttcca ataaaatttt caaactttgt actgttaaa 4909

<210> 29
<211> 3499
<212> DNA
<213> Homo sapiens

<400> 29
cgcggacccg gccggcccag gcccgcgccc gccgcggccc tgagaggccc cggcaggtcc 60

cggcccggcg gcggcagcca tggccggggg gccgggcccg ggggagcccg cagcccccgg 120

cgcccagcac ttcttgtacg aggtgccgcc ctgggtcatg tgccgcttct acaaagtgat 180

ggacgccctg gagcccgccg actggtgcca gttcgccgcc ctgatcgtgc gcgaccagac 240

cgagctgcgg ctgtgcgagc gctccgggca gcgcacggcc agcgtcctgt ggccctggat 300

caaccgcaac gcccgtgtgg ccgacctcgt gcacatcctc acgcacctgc agctgctccg 360

tgcgcgggac atcatcacag cctggcaccc tcccgccccg cttccgtccc caggcaccac 420

tgccccgagg cccagcagca tccctgcacc cgccgaggcc gaggcctgga gcccccggaa 480

gttgccatcc tcagcctcca ccttcctctc cccagctttt ccaggctccc agacccattc 540

agggcctgag ctcggcctgg tcccaagccc tgcttccctg tggcctccac cgccatctcc 600

agccccttct tctaccaagc caggcccaga gagctcagtg tccctcctgc agggagcccg 660

cccctttccg ttttgctggc ccctctgtga gatttcccgg ggcacccaca acttctcgga 720

ggagctcaag atcggggagg gtggctttgg gtgcgtgtac cgggcggtga tgaggaacac 780

ggtgtatgct gtgaagaggc tgaaggagaa cgctgacctg gagtggactg cagtgaagca 840

gagcttcctg accgaggtgg agcagctgtc caggtttcgt cacccaaaca ttgtggactt 900

tgctggctac tgtgctcaga acggcttcta ctgcctggtg tacggcttcc tgcccaacgg 960

ctccctggag gaccgtctcc actgccagac ccaggcctgc ccacctctct cctggcctca 1020

gcgactggac atccttctgg gtacagcccg ggcaattcag tttctacatc aggacagccc 1080

cagcctcatc catggagaca tcaagagttc caacgtcctt ctggatgaga ggctgacacc 1140

caagctggga gactttggcc tggcccggtt cagccgcttt gccgggtcca gccccagcca 1200

gagcagcatg gtggcccgga cacagacagt gcggggcacc ctggcctacc tgcccgagga 1260

gtacatcaag acgggaaggc tggctgtgga cacggacacc ttcagctttg gggtggtagt 1320

gctagagacc ttggctggtc agagggctgt gaagacgcac ggtgccagga ccaagtatct 1380

gaaagacctg gtggaagagg aggctgagga ggctggagtg gctttgagaa gcacccagag 1440

cacactgcaa gcaggtctgg ctgcagatgc ctgggctgct cccatcgcca tgcagatcta 1500

caagaagcac ctggacccca ggcccgggcc ctgcccacct gagctgggcc tgggcctggg 1560

ccagctggcc tgctgctgcc tgcaccgccg ggccaaaagg aggcctccta tgacccagga 1620

gaactcctac gtgtccagca ctggcagagc ccacagtggg gctgctccat ggcagcccct 1680

ggcagcgcca tcaggagcca gtgcccaggc agcagagcag ctgcagagag gccccaacca 1740

gcccgtggag agtgacgaga gcctaggcgg cctctctgct gccctgcgct cctggcactt 1800

gactccaagc tgccctctgg acccagcacc cctcagggag gccggctgtc ctcaggggga 1860

cacggcagga gaatcgagct gggggagtgg cccaggatcc cggcccacag ccgtggaagg 1920

actggccctt ggcagctctg catcatcgtc gtcagagcca ccgcagatta tcatcaaccc 1980

tgcccgacag aagatggtcc agaagctggc cctgtacgag gatggggccc tggacagcct 2040

gcagctgctg tcgtccagct ccctcccagg cttgggcctg gaacaggaca ggcaggggcc 2100

cgaagaaagt gatgaatttc agagctgatg tgttcacctg ggcagatccc ccaaatccgg 2160

aagtcaaagt tctcatggtc agaagttctc atggtgcacg agtcctcagc actctgccgg 2220

cagtgggggt gggggcccat gcccgcgggg gagagaagga ggtggccctg ctgttctagg 2280

ctctgtgggc ataggcaggc agagtggaac cctgcctcca tgccagcatc tgggggcaag 2340

gaaggctggc atcatccagt gaggaggctg gcgcatgttg ggaggctgct ggctgcacag 2400

acccgtgagg ggaggagagg ggctgctgtg caggggtgtg gagtagggag ctggctcccc 2460

tgagagccat gcagggcgtc tgcagcccag gcctctggca gcagctcttt gcccatctct 2520

ttggacagtg gccaccctgc acaatggggc cgacgaggcc tagggccctc ctacctgctt 2580

acaatttgga aaagtgtggc cgggtgcggt ggctcacgcc tgtaatccca gcactttggg 2640

aggccaaggc aggaggatcg ctggagccca gtaggtcaag accagccagg gcaacatgat 2700

gagaccctgt ctctgccaaa aaatttttta aactattagc ctggcgtggt agcgcacgcc 2760

tgtggtccca gctgctgggg aggctgaagt aggaggatca tttatgcttg ggaggtcgag 2820

gctgcagtga gtcatgattg tatgactgca ctccagcctg ggtgacagag caagaccctg 2880

tttcaaaaag aaaaaccctg ggaaaagtga agtatggctg taagtctcat ggttcagtcc 2940

tagcaagaag cgagaattct gagatcctcc agaaagtcga gcagcaccca cctccaacct 3000

cgggccagtg tcttcaggct ttactgggga cctgcgagct ggcctaatgt ggtggcctgc 3060

aagccaggcc atccctgggc gccacagacg agctccgagc caggtcaggc ttcggaggcc 3120

acaagctcag cctcaggccc aggcactgat tgtggcagag gggccactac ccaaggtcta 3180

gctaggccca agacctagtt acccagacag tgagaagccc ctggaaggca gaaaagttgg 3240

gagcatggca gacagggaag ggaaacattt tcagggaaaa gacatgtatc acatgtcttc 3300

agaagcaagt caggtttcat gtaaccgagt gtcctcttgc gtgtccaaaa gtagcccagg 3360

gctgtagcac aggcttcaca gtgattttgt gttcagccgt gagtcacact acatgccccc 3420

gtgaagctgg gcattggtga cgtccaggtt gtccttgagt aataaaaacg tatgttgcaa 3480

taaaaaaaaa aaaaaaaaa 3499

<210> 30
<211> 4267
<212> DNA
<213> Homo sapiens

<400> 30
attcgcctct gggaggttta ggaagcggct ccgggtcggt ggccccagga cagggaagag 60

cgggcgctat ggggagccgg acgccagagt cccctctcca cgccgtgcag ctgcgctggg 120

gcccccggcg ccgacccccg ctgctgccgc tgctgttgct gctgctgccg ccgccaccca 180

gggtcggggg cttcaactta gacgcggagg ccccagcagt actctcgggg cccccgggct 240

ccttcttcgg attctcagtg gagttttacc ggccgggaac agacggggtc agtgtgctgg 300

tgggagcacc caaggctaat accagccagc caggagtgct gcagggtggt gctgtctacc 360

tctgtccttg gggtgccagc cccacacagt gcacccccat tgaatttgac agcaaaggct 420

ctcggctcct ggagtcctca ctgtccagct cagagggaga ggagcctgtg gagtacaagt 480

ccttgcagtg gttcggggca acagttcgag cccatggctc ctccatcttg gcatgcgctc 540

cactgtacag ctggcgcaca gagaaggagc cactgagcga ccccgtgggc acctgctacc 600

tctccacaga taacttcacc cgaattctgg agtatgcacc ctgccgctca gatttcagct 660

gggcagcagg acagggttac tgccaaggag gcttcagtgc cgagttcacc aagactggcc 720

gtgtggtttt aggtggacca ggaagctatt tctggcaagg ccagatcctg tctgccactc 780

aggagcagat tgcagaatct tattaccccg agtacctgat caacctggtt caggggcagc 840

tgcagactcg ccaggccagt tccatctatg atgacagcta cctaggatac tctgtggctg 900

ttggtgaatt cagtggtgat gacacagaag actttgttgc tggtgtgccc aaagggaacc 960

tcacttacgg ctatgtcacc atccttaatg gctcagacat tcgatccctc tacaacttct 1020

caggggaaca gatggcctcc tactttggct atgcagtggc cgccacagac gtcaatgggg 1080

acgggctgga tgacttgctg gtgggggcac ccctgctcat ggatcggacc cctgacgggc 1140

ggcctcagga ggtgggcagg gtctacgtct acctgcagca cccagccggc atagagccca 1200

cgcccaccct taccctcact ggccatgatg agtttggccg atttggcagc tccttgaccc 1260

ccctggggga cctggaccag gatggctaca atgatgtggc catcggggct ccctttggtg 1320

gggagaccca gcagggagta gtgtttgtat ttcctggggg cccaggaggg ctgggctcta 1380

agccttccca ggttctgcag cccctgtggg cagccagcca caccccagac ttctttggct 1440

ctgcccttcg aggaggccga gacctggatg gcaatggata tcctgatctg attgtggggt 1500

cctttggtgt ggacaaggct gtggtataca ggggccgccc catcgtgtcc gctagtgcct 1560

ccctcaccat cttccccgcc atgttcaacc cagaggagcg gagctgcagc ttagagggga 1620

accctgtggc ctgcatcaac cttagcttct gcctcaatgc ttctggaaaa cacgttgctg 1680

actccattgg tttcacagtg gaacttcagc tggactggca gaagcagaag ggaggggtac 1740

ggcgggcact gttcctggcc tccaggcagg caaccctgac ccagaccctg ctcatccaga 1800

atggggctcg agaggattgc agagagatga agatctacct caggaacgag tcagaatttc 1860

gagacaaact ctcgccgatt cacatcgctc tcaacttctc cttggacccc caagccccag 1920

tggacagcca cggcctcagg ccagccctac attatcagag caagagccgg atagaggaca 1980

aggctcagat cttgctggac tgtggagaag acaacatctg tgtgcctgac ctgcagctgg 2040

aagtgtttgg ggagcagaac catgtgtacc tgggtgacaa gaatgccctg aacctcactt 2100

tccatgccca gaatgtgggt gagggtggcg cctatgaggc tgagcttcgg gtcaccgccc 2160

ctccagaggc tgagtactca ggactcgtca gacacccagg gaacttctcc agcctgagct 2220

gtgactactt tgccgtgaac cagagccgcc tgctggtgtg tgacctgggc aaccccatga 2280

aggcaggagc cagtctgtgg ggtggccttc ggtttacagt ccctcatctc cgggacacta 2340

agaaaaccat ccagtttgac ttccagatcc tcagcaagaa tctcaacaac tcgcaaagcg 2400

acgtggtttc ctttcggctc tccgtggagg ctcaggccca ggtcaccctg aacggtgtct 2460

ccaagcctga ggcagtgcta ttcccagtaa gcgactggca tccccgagac cagcctcaga 2520

aggaggagga cctgggacct gctgtccacc atgtctatga gctcatcaac caaggcccca 2580

gctccattag ccagggtgtg ctggaactca gctgtcccca ggctctggaa ggtcagcagc 2640

tcctatatgt gaccagagtt acgggactca actgcaccac caatcacccc attaacccaa 2700

agggcctgga gttggatccc gagggttccc tgcaccacca gcaaaaacgg gaagctccaa 2760

gccgcagctc tgcttcctcg ggacctcaga tcctgaaatg cccggaggct gagtgtttca 2820

ggctgcgctg tgagctcggg cccctgcacc aacaagagag ccaaagtctg cagttgcatt 2880

tccgagtctg ggccaagact ttcttgcagc gggagcacca gccatttagc ctgcagtgtg 2940

aggctgtgta caaagccctg aagatgccct accgaatcct gcctcggcag ctgccccaaa 3000

aagagcgtca ggtggccaca gctgtgcaat ggaccaaggc agaaggcagc tatggcgtcc 3060

cactgtggat catcatccta gccatcctgt ttggcctcct gctcctaggt ctactcatct 3120

acatcctcta caagcttgga ttcttcaaac gctccctccc atatggcacc gccatggaaa 3180

aagctcagct caagcctcca gccacctctg atgcctgagt cctcccaatt tcagactccc 3240

attcctgaag aaccagtccc cccaccctca ttctactgaa aaggaggggt ctgggtactt 3300

cttgaaggtg ctgacggcca gggagaagct cctctcccca gcccagagac atacttgaag 3360

ggccagagcc aggggggtga ggagctgggg atccctcccc cccatgcact gtgaaggacc 3420

cttgtttaca cataccctct tcatggatgg gggaactcag atccagggac agaggcccca 3480

gcctccctga agcctttgca ttttggagag tttcctgaaa caacttggaa agataactag 3540

gaaatccatt cacagttctt tgggccagac atgccacaag gacttcctgt ccagctccaa 3600

cctgcaaaga tctgtcctca gccttgccag agatccaaaa gaagccccca gctaagaacc 3660

tggaacttgg ggagttaaga cctggcagct ctggacagcc ccaccctggt gggccaacaa 3720

agaacactaa ctatgcatgg tgccccagga ccagctcagg acagatgcca cacaaggata 3780

gatgctggcc cagggcccag agcccagctc caaggggaat cagaactcaa atggggccag 3840

atccagcctg gggtctggag ttgatctgga acccagactc agacattggc acctaatcca 3900

ggcagatcca ggactatatt tgggcctgct ccagacctga tcctggaggc ccagttcacc 3960

ctgatttagg agaagccagg aatttcccag gaccctgaag gggccatgat ggcaacagat 4020

ctggaacctc agcctggcca gacacaggcc ctccctgttc cccagagaaa ggggagccca 4080

ctgtcctggg cctgcagaat ttgggttctg cctgccagct gcactgatgc tgcccctcat 4140

ctctctgccc aacccttccc tcaccttggc accagacacc caggacttat ttaaactctg 4200

ttgcaagtgc aataaatctg acccagtgcc cccactgacc agaactagaa aaaaaaaaaa 4260

aaaaaaa 4267

<210> 31
<211> 8787
<212> DNA
<213> Homo sapiens

<400> 31
ggcgggtgct tctagggcgc tcccagagcc gcctccccct gttgctggca tcccgagctt 60

cctcccttgc cagccaggac gctgccgact tgtctttgcc cgctgctccg cagacggggc 120

tgcaaagctg caactaatgg tgttggcctc cctgcccacc tgtggaagca actgcgctga 180

ttgatgcgcc acagactttt ttcccctcga cctcgccggc gtcccctccc acagatccag 240

catcacccag tgaatgtaca ttagggtggt ttccccccca gcttcgggct ttgtttgggt 300

ttgattgtgt ttggctcttc gctaagctga tttatgcagc agaagcccca ccggctggag 360

agaaacaaaa gctcttttct ttgtcccgga gcaggctgcg gagcccttgc agagccctct 420

ctccagtcgc cgccggggcc cttggccgtc gaaggaggtg cttctcgcgg agaccgcggg 480

acccgccgtg ccgagccggg agggccgcag gggccctgag atgccgagcg gtgcccgggc 540

ccgcttacct gcaccgcttg ctccgagccg cggggtccgc ctgctaggcc tgcggaaaac 600

gtcctagcga cactcggccc gcgggccccg aggtgcgccc gggaggcgcg agcccgcgtc 660

cggaaggcag tcaggcggcg ggcgcggggc gggctgtttt gcattatgtg cggctcggcc 720

ctggcttttt ttaccgctgc atttgtctgc ctgcaaaacg accggcgagg tcccgcctcg 780

ttcctctggg cagcctgggt gttttcactt gttcttggac tgggccaagg tgaagacaat 840

agatgtgcat cttcaaatgc agcatcctgt gccaggtgcc ttgcgctggg tccagaatgt 900

ggatggtgtg ttcaagagga tttcatttca ggtggatcaa gaagtgaacg ttgtgatatt 960

gtttccaatt taataagcaa aggctgctca gttgattcaa tagaataccc atctgtgcat 1020

gttataatac ccactgaaaa tgaaattaat acccaggtga caccaggaga agtgtctatc 1080

cagctgcgtc caggagccga agctaatttt atgctgaaag ttcatcctct gaagaaatat 1140

cctgtggatc tttattatct tgttgatgtc tcagcatcaa tgcacaataa tatagaaaaa 1200

ttaaattccg ttggaaacga tttatctaga aaaatggcat ttttctcccg tgactttcgt 1260

cttggatttg gctcatacgt tgataaaaca gtttcaccat acattagcat ccaccccgaa 1320

aggattcata atcaatgcag tgactacaat ttagactgca tgcctcccca tggatacatc 1380

catgtgctgt ctttgacaga gaacatcact gagtttgaga aagcagttca tagacagaag 1440

atctctggaa acatagatac accagaagga ggttttgacg ccatgcttca ggcagctgtc 1500

tgtgaaagtc atatcggatg gcgaaaagag gctaaaagat tgctgctggt gatgacagat 1560

cagacgtctc atctcgctct tgatagcaaa ttggcaggca tagtggtgcc caatgacgga 1620

aactgtcatc tgaaaaacaa cgtctatgtc aaatcgacaa ccatggaaca cccctcacta 1680

ggccaacttt cagagaaatt aatagacaac aacattaatg tcatctttgc agttcaagga 1740

aaacaatttc attggtataa ggatcttcta cccctcttgc caggcaccat tgctggtgaa 1800

atagaatcaa aggctgcaaa cctcaataat ttggtagtgg aagcctatca gaagctcatt 1860

tcagaagtga aagttcaggt ggaaaaccag gtacaaggca tctattttaa cattaccgcc 1920

atctgtccag atgggtccag aaagccaggc atggaaggat gcagaaacgt gacgagcaat 1980

gatgaagttc ttttcaatgt aacagttaca atgaaaaaat gtgatgtcac aggaggaaaa 2040

aactatgcaa taatcaaacc tattggtttt aatgaaaccg ctaaaattca tatacacaga 2100

aactgcagct gtcagtgtga ggacaacaga ggacctaaag gaaagtgtgt agatgaaact 2160

tttctagatt ccaagtgttt ccagtgtgat gagaataaat gtcattttga tgaagatcag 2220

ttttcttctg agagttgcaa gtcacacaag gatcagcctg tttgcagtgg tcgaggagtt 2280

tgtgtttgtg ggaaatgttc atgtcacaaa attaagcttg gaaaagtgta tggaaaatac 2340

tgtgaaaagg atgacttttc ttgtccatat caccatggaa atctgtgtgc tgggcatgga 2400

gagtgtgaag caggcagatg ccaatgcttc agtggctggg aaggtgatcg atgccagtgc 2460

ccttcagcag cagcccagca ctgtgtcaat tcaaagggcc aagtgtgcag tggaagaggc 2520

acgtgtgtgt gtggaaggtg tgagtgcacc gatcccagga gcatcggccg cttctgtgaa 2580

cactgcccca cctgttatac agcctgcaag gaaaactgga attgtatgca atgccttcac 2640

cctcacaatt tgtctcaggc tatacttgat cagtgcaaaa cctcatgtgc tctcatggaa 2700

caacagcatt atgtcgacca aacttcagaa tgtttctcca gcccaagcta cttgagaata 2760

tttttcatca ttttcatagt tacattcttg attgggttgc ttaaagtcct gatcattaga 2820

caggtgatac tacaatggaa tagtaataaa attaagtcct catcagatta cagagtgtca 2880

gcctcaaaaa aggataagtt gattctgcaa agtgtttgca caagagcagt cacctaccga 2940

cgtgagaagc ctgaagaaat aaaaatggat atcagcaaat taaatgctca tgaaactttc 3000

aggtgcaact tctaaaaaaa gatttttaaa cacttaatgg gaaactggaa ttgttaataa 3060

ttgctcctaa agattataat tttaaaagtc acaggaggag acaaattgct cacggtcatg 3120

ccagttgctg gttgtacact cgaacgaaga ctgacaagta tcctcatcat gatgtgactc 3180

acatagctgc tgactttttc agagaaaaat gtgtcttact actgtttgag actagtgtcg 3240

ttgtagcact ttactgtaat atataactta tttagatcag catagaatgt agatcctctg 3300

aagagcactg attacacttt acaggtacct gttatcccta cgcttcccag agagaacaat 3360

gctgtgagag agtttagcat tgtgtcacta caaggtacag gtaatccctg cactggacat 3420

gtgaggaaaa aaataatctg gcaagtatat tctaaggttg ccaaacactt caacagttgg 3480

tggttgaata gacaagaaca gctagatgaa taaatgattc gtgtttcact ctttcaagag 3540

gtgaacagat acaaccttaa tcttaaaaga ttattgcttt ttaaagtgtg tagttttatg 3600

catgtgtgtt tatggtttgc ttatttttgc aagatggata ctaattccag cattctctcc 3660

tctttgcctt tatgttttgt tttctttttt acaggataag tttatgtatg tcacagatga 3720

ctggattaat taagtgctaa gttactactg ccataaaaaa ctaataatac aatgtcactt 3780

tatcagaata ctagttttaa aagctgaatg ttaatagggg acactgtaaa gtatcatcaa 3840

aacctgaata gcttcattgt gcacaagtgt ggagttttgt atcctcttac ctggtaaact 3900

gaagggattg tttggccatt tcatttatct tatcattaat tcacaagata gttagaaatt 3960

ctgcctcaag caaagtacca cattttgaat gttttcttag attttgattg caagtagata 4020

tcagcatttt ttaaatgaaa agctatatta tcttctccct tcaaggcagc ctaaggatgt 4080

tctttcccag aatcactcca acccttcttg ccagaattca taaaagtaca aaattggaga 4140

atagatgata tcttagaaat aagctttttt tttttttttt tttttttttg agacggagtt 4200

tcactcttgt cacccaggct gaagtgcaat ggcgcaatta gggttcactg caacctctgc 4260

ctcccgggtt caagcagttc tcctgcctca gcctcctgag tagctgggat tacaggcatc 4320

caccaccgtg cccagctaat ttttgtattt ttagtagaga cggggttttg ccatgttgga 4380

caggttgatc tcaaactcct gacctcaggt gatctaccct cctcggcctc ccagagtgtt 4440

gggattacag gcatgagcca ccatgccagg ctgctaattc tcctttttag tgagttaggg 4500

aactgagcct cagaaaactt aaacgatttc tcagaaaaca ctcaagtgat aaagtggcca 4560

cattggaaag gagtttttat cttctcattg tcaggccagt gttcattgca caatatcatg 4620

ctacctcttg aatctttaaa atattcaatt ggcaaatgtt tttcaatgtg atttactcat 4680

gtcttaagtg tatgaggaaa gttcaaagca aaatagaaag gaataattca aactgaattg 4740

tccataatca gcttccagtc tttcatgcta atcagcttct taagagactg aagtatggca 4800

tacctacagg ggaattcctt cgcaccatag cctgtatgaa cagtgttccc tggagttctc 4860

cagtgctcag cttgagacct tgatacacgg gccatgagcc ctgtcttccc caatggaaat 4920

ttatttacac ttaccttatc cctatggact tagtctgatt ttattggcta ggagtctaac 4980

agtcctgtgt ggatatacag ttttgcccat gacaacaaag gaatctatcc gaaatatctt 5040

tttttttata ataaacttcc aagatttgct gtcttccagc acttgagtta aagtactaga 5100

tactgcattt tgatgaagac taaccccatc tcatattcta ccctaaagag aactgaaaaa 5160

cctataataa gttgttctgg agccaataaa cacagcagct ctgttagatg tcctctacag 5220

ccaagcactt tcaatgctaa cttgaactgc atttccttcc tcaaatgaga gattgacata 5280

attcagtact gtgagtcact tgtataagaa acctttgatc actaaaaata atgtaaaaat 5340

tgggtttagt agcctaatac acataacgtt cttcttaaaa aggaaaatgg atggatgcct 5400

gacaaccctc caaaagaaaa aagtgtaaga tagccattaa gatgatgaca atttttgaaa 5460

tgaacattat gatatttatg aacaataaac aaatttccgt atggaatgaa ttatccaaaa 5520

agagtataac aaaatgaaat ccttaaaaat ccagagttta tatttttttt ataccctcac 5580

ttgtttgcac taactttata gtggaccaag gctgttacca taggaaggga caaacttcct 5640

tgtaggcaac tcagtgttag acgatgattg tggttatgct tgcaaagtct tgtgcttatc 5700

ttttttgttt ttacttaaaa agctaatttt taaagattgt agggcttgta ttttacttga 5760

ataattgata tcttcctgtg taatgatttg tgagatgaga attaatattt gactagttag 5820

aattaattaa atggtaaggg aacacagggt actcttaggt taaataatgt atgcaaatag 5880

agtctatttt caactaatat ggccacagga gccttttgag attcattgat attaaacaca 5940

attaatgaaa ttttaaattg ttaacagaat tgagaacttg aacaacactt ttagtactgc 6000

agcatttttg tgccctaaag tatgtaatga tttataaatg tgccatacat acactacaac 6060

ataacatttg ctttgttatg cattttattt ctctggggac accattgcac tgcagtgcac 6120

acgtatttat aaacatttgt tatatttttg gaaacttgct aatatttatt aagtcataga 6180

cttttctgga ggacttaaaa attcactaaa aatctgatta tgtcttaaat gttcagttta 6240

tctttggttt attaaaataa aaaaaaaatc taagattaaa cacagtagat atctctggag 6300

gcaattttcc aaaactcaac attaaaattt gtggatgcat gagatgcaat ccttcaaaga 6360

atgaatctga aatatatttt taatatttac ttaatatcca ctgaagatat ctttatgcaa 6420

gacaagagtc agccatcaga cactgaaata tattatgata gattatgaag aattttctct 6480

gtagaattat attcttcctg gaacctggta gagtagatta gactcaaagg ctttttcttc 6540

cttttcttac tcctgttttt tccactcact cttcccaaga gatttcctaa agcttcaagc 6600

ttaataagcc taatagtgaa aaataactga atttaatggt ataatgaagt tcttcatttc 6660

cagacatctt taattgatct taaagctcat ttgagtcttt gcccctgaac aaagacagac 6720

ccattaaaat ctaagaattc taaattttca caactgtttg agcttctttt cattttgaag 6780

gatttggaat atatatgttt tcataaaagt atcaagtgaa atatagttac atgggagctc 6840

aatcatgtgc agattgcatt ctgttatgtt gactcaatat ttaatttaca actatcctta 6900

tttatattga cctcaagaac tccattttat gcaatgcaga ccactgagat atagctaaca 6960

ttctttcaaa taattttcct tttcttttat aattcctcta tagcaaattt ttatgtataa 7020

ctgattatac atatccatat ttatatttca ttgattccaa gacatcactt tttcaattta 7080

acatctctga aattgtgaca tttcttgcaa ctgttggcac ttcagatgca gtgtttaaaa 7140

ttatgcttga ataaatatta cactaatcca actttaccta aatgtttatg catctaggca 7200

aattttgttt tcttataaag atttgagagc ccatttatga caaaatatga aggcgaaatt 7260

taaggacaac tgagtcacgc acaactcaac atggagccta actgattatc agctcagatc 7320

ccgcatatct tgagtttaca aaagctcttt caggtcccca tttatacttt acgtgagtgc 7380

gaatgatttc agcaaaccct aacttaacta acaagaatgg gtaggtatgt ctacgtttca 7440

ttaacaaatt tttattattt ttattctatt atatgagatc cttttatatt atcatctcac 7500

ttttaaacaa aattaactgg aaaaatatta catggaactg tcatagttag gttttgcagc 7560

atcttacatg tcttgtatca atggcaggag aaaaatatga taaaaacaat cagtgctgtg 7620

aaaaacaact ttcttctaga gtcctcttac tttttattct tctttatcat ttgtgggttt 7680

ttcccccttg gctctgatca ctttaacttc aagcttatgt aacgactgtt ataaaactgc 7740

atatttaaat tatttgaatt atatgaaata attgttcagc tatctgggca gctgttaatg 7800

taaacctgag agtaataaca ctactctttt atctacctgg aatacttttc tgcataaaat 7860

ttatctttgt aagctaactc tattaatcag gtttcttcta gcctctgcaa cctacttcag 7920

ttagaattgt ctaatactgc tctattaatc aggtttctag cctctacaac ctacttcagt 7980

taaaattgtc taatacagca atatttaaaa aaaaaacact gcaattgtca aggatggaaa 8040

atgtgtgatt tgtgtaaaca atttttacca actttacatt ttcctacaga taaatgtgaa 8100

attttgataa gaagtctacg caatgacaag tatggtacat aaattttatt aagaatattg 8160

agtataaagt actttaattc taaattataa gaaaatatac atttgcacat attaatatag 8220

aaattcattt tgtgtatatt taacatagct tttaaactat tttacattag ctacttcatt 8280

atggtttctt gaacttctga aaaaaattag aaatgtatta aacttatcag taacataaaa 8340

acttattttg tttcacctaa cgaatactgc gtttgtaaaa ataaatttaa tatagaatat 8400

atttttaaat taaatatttg aatataaaat agctctaaga aagaagcaaa ttatcactga 8460

acatatttct tattatttct ggctttgaat tatacgtaac ttaaattgtc ttaaatgata 8520

cagaatattg gagaatatga tactttcaca taatatacta tgaacctgtt catataactc 8580

tgattgacta ctaacttctg ttttatgtat ttattaaaga gctgacactg tagtttgtgg 8640

tgagatgttt atttttctaa cagagcttat aacagttagg acaaggcatt taattaatgc 8700

atcattctgt ttagtagtag gtgttaatca atatgaaatt ctctgtttta aaataaaaat 8760

gtaaaaatct aagaataaaa aaaaaaa 8787

<210> 32
<211> 3081
<212> DNA
<213> Homo sapiens

<400> 32
attagaggct ccagccccgc cgacttgcag acgtgagatc gggcacacct gagcggcggc 60

ggggcggtcg tggccacatc cggggcgacg tgcctgagtc accccgtccc gccagcgtct 120

gccagtccag ccagtccgcc cagtctctcg cgtccgagac tcgcctccag cctcccacct 180

ccgcccgggc cgcgcgagcc tcgcgggggc gggggcgggg cgccaagggg cggggctgtc 240

tcttaaaggg ccccgggccg ctgcccttag gccacttcct gggggcggag aggacctcag 300

cggctgcggc gacacccagg gaaggcggcg cggccgggtc ccgaaactcc tggctgtttc 360

catcagagcc ctcggacact cccagcccgg gctgagcacg catcgtcgct ccccggcgga 420

tacaaggggg ctccgccatc cgctcccgtc agttcggcct ccatctcctg ggacccgcgc 480

cggcagccag gccaggcctc tgagtggccc cagagccctg gctggactcg tccacggcgg 540

cagcgatctg cccggggtct cggaggccat cccttcagag tcggccctgt gctcgccacc 600

gtcacctgct ggttggattc cggaaaccca ctgtctgaag accacagagg ggtgtcgctg 660

accaccccaa atcggatacg tccagacctc aagctccctt cccctctctg gctgccctct 720

gctcttttca tctcttctct caaccttttg gggatttctg tgtcctgaca ccacctcccc 780

atccaccacc aaagtagccg gggtgagccc caaaccttac tgggtgtgct ccacctgtgc 840

ctccaaccca gcgaatctga cagcttcgac ccaattctgc acacacccag gaagttctgc 900

cttttctttt ctttcggtgt ctcctgtact tcccaaaatt tctcctcctc ctgtgccctc 960

ttcgcccccc tcctttgggg gccccgtgac cctgaatgtg gggggcacac tatattccac 1020

cactttggag accctgaccc gcttcccaga ctctatgctg ggggccatgt ttagggccgg 1080

cacccccatg ccccccaacc tcaattccca aggaggcggc cactacttca tcgaccggga 1140

tggcaaggcc ttccggcaca tcctcaattt cctgaggctg ggccgcctgg acctgccccg 1200

tgggtacgga gagacagcgc tgctcagggc agaggctgac ttctaccaga tccggcccct 1260

cctggacgcg ctgcgggaac tggaggcctc tcaggggacc cctgcaccca cagctgccct 1320

gctccacgca gatgtagatg tcagcccccg cctggtgcac ttctctgctc gccggggacc 1380

ccatcactat gagctgagct ccgtccaggt ggacaccttc cgagccaacc ttttctgcac 1440

cgactctgag tgtctaggtg ctttgcgggc ccgatttggt gtggccagtg gggatagggc 1500

agaggggagc ccacattttc atctggagtg ggccccccgc cccgtggaac tccccgaggt 1560

ggagtatggg agactggggc tgcagccgct gtggactggg gggccaggag agcggcggga 1620

ggtggtgggc accccaagct tcctggagga ggtgctgcgg gtggctctcg agcacggctt 1680

ccgactagac tctgtcttcc ccgaccccga agacctgctc aactccaggt ctctgcgctt 1740

tgtccggcac tgaggatgct gttctcagtt tgactgtggg gaggagagag aatggggtac 1800

tagcacccct gaagcctctt tccagctctg cttcaggagc tatgagagtc gggactctcc 1860

tgcacctgac tggagctcag atgtgggcag gaattcccaa acctgagccc accaaggact 1920

cacaagtggt ccagaaggtc tcaacctgtg ctgaccctgg gaggggtagg gaaggttctc 1980

tcagcttgtt cttgcctaag gctgagcacc tccagtctct ccttgatttg gagctcagtg 2040

tttaagggct tggaaaaggg gggaacatct ctttacccag actagaccta gcaaaaccct 2100

ggaaggatat tgaggtctgg ggaaaaggga ggactttgca ttttcccaat gcggtctctt 2160

ggaccatggc ttctactcct gaagctgggt ggcctggcct ggcctgacca atgagaggcc 2220

agaacactct ggaacatcgg aagaggagtt ctttgctatg ttccaagcca tctactgagg 2280

gaggcagaaa ggccacaacc caccctaggt tgatgtatgg gagctaggac agtccccatg 2340

gcaatggggc tggagcatcc ctcatctgga agaatcccat actgatggca gggctggcca 2400

gggggaagag ggtagtatct gtgggtcctg gcctttcttc atgtgtgcgt gcatatcagc 2460

ccgtgtggct gactgatgta taggtccctg gcatcctggt tcatatctgt gttgctgact 2520

acagtgtctg tgatgtccgc atgtccaggc ctgtttgggg ttgcctagcg actcttctgg 2580

cacagggtgt gtctgtggta tacctgtgag gtggttgaca attagtagtt taatcacagg 2640

gtgtgtgtgt gtgtgtgtgt gtgtgtgttt atgtgcacgc atgtatatgc atcaccacgt 2700

agccaggagg ggcctgttgg ggtttgagtc actgggatct tcctggtgag aggtaagaga 2760

agtcactggg cttagctggg cctctgaggc ctgtatggaa ctcttggttg ctgaggcaac 2820

catggacctg ttgctaggag atagctgggg aaggcccaag gccgcccagg gcagagagag 2880

gagacgaaga gtttgggaca gtgggggagg agatgggaag ggatgggatt tctgggtccc 2940

agagcgggtg ggatactcac gcacagcttc ttcactggtg gggggtgggg cacacattat 3000

ttctcactgg tcatgattta caagaagaaa aataaaactg cttttggaac cacaaaaaaa 3060

aaaaaaaaaa aaaaaaaaaa a 3081

<210> 33
<211> 3767
<212> DNA
<213> Homo sapiens

<400> 33
aagaaagagc cccgccccta gtcttatgac tcgcactgaa gcgccgattc ctggcttttg 60

caaggctgtg gtcggtggtc atcagtgctc ttgacccagg tccagcgagc cttttccctg 120

gtgttgcagc tgttgttgta ccgccgccgt cgccgccgtc gccgcctgct ctgcggggtc 180

atggtgtgct tccgcctctt cccggttccg ggctcagggc tcgttctggt ctgcctagtc 240

ctgggagctg tgcggtctta tgcattggaa cttaatttga cagattcaga aaatgccact 300

tgcctttatg caaaatggca gatgaatttc acagtacgct atgaaactac aaataaaact 360

tataaaactg taaccatttc agaccatggc actgtgacat ataatggaag catttgtggg 420

gatgatcaga atggtcccaa aatagcagtg cagttcggac ctggcttttc ctggattgcg 480

aattttacca aggcagcatc tacttattca attgacagcg tctcattttc ctacaacact 540

ggtgataaca caacatttcc tgatgctgaa gataaaggaa ttcttactgt tgatgaactt 600

ttggccatca gaattccatt gaatgacctt tttagatgca atagtttatc aactttggaa 660

aagaatgatg ttgtccaaca ctactgggat gttcttgtac aagcttttgt ccaaaatggc 720

acagtgagca caaatgagtt cctgtgtgat aaagacaaaa cttcaacagt ggcacccacc 780

atacacacca ctgtgccatc tcctactaca acacctactc caaaggaaaa accagaagct 840

ggaacctatt cagttaataa tggcaatgat acttgtctgc tggctaccat ggggctgcag 900

ctgaacatca ctcaggataa ggttgcttca gttattaaca tcaaccccaa tacaactcac 960

tccacaggca gctgccgttc tcacactgct ctacttagac tcaatagcag caccattaag 1020

tatctagact ttgtctttgc tgtgaaaaat gaaaaccgat tttatctgaa ggaagtgaac 1080

atcagcatgt atttggttaa tggctccgtt ttcagcattg caaataacaa tctcagctac 1140

tgggatgccc ccctgggaag ttcttatatg tgcaacaaag agcagactgt ttcagtgtct 1200

ggagcatttc agataaatac ctttgatcta agggttcagc ctttcaatgt gacacaagga 1260

aagtattcta cagctgaaga atgttctgct gactctgacc tcaactttct tattcctgtt 1320

gcagtgggtg tggccttggg cttccttata attgttgtct ttatctctta tatgattgga 1380

agaaggaaaa gtcgtactgg ttatcagtct gtgtaatcag ttaaatctag tgtttgtttg 1440

tttttttcaa ttagaagtta cgtttccatt ggctaaaagc caggacatgc tgtgcaatag 1500

attgtttaag atatgcagac taacttcagt gagttcctag ctaacttggg catgagtaca 1560

cttatttaag acaaaatata ttaggaccaa tttttttctg ttttttttct tcctttgtta 1620

aagtataatt aaaagaaaaa ttgtggctta gaatttttta agtaaataat gattttaagc 1680

ccctggatcc aattatgaaa gcatttttgc tgatgtgtaa ttttatatgt tacagttact 1740

tatattttac tactttgatg ttatttgcaa aatcaaaggt gttaaagaat ttaacttgct 1800

tcaggaaata aattcaagaa catagtggat tcattttcat tggtggcaga cacgaaattt 1860

ggttcatgat aagacttcct ttccccacct cctgatcagc attatttaaa tctgtatttt 1920

tctgttagtt aagaaagaaa tggcttcatg atattgtatt taatagcaaa agtttggctg 1980

tcttcttcat tactgttaat agctactata ttttaacaag gagatttctt tttttgttgt 2040

tgttgttcta gagtttggaa tatactgatt atctcagact tgacatttat actgaaggat 2100

gaagtaagac ctccagcttt ttttaaaaaa ggtgttgatt tggaacacct gtatgggtta 2160

tggtttatta aggttatggt ttagaaagtt tttttccctc agagccttaa cttgttaaga 2220

aggttcattt atcctgcact gaaaacaaaa actctatata ctttgtttgt gtgcctcctg 2280

cactctccca ttccctatgt gaatatgctc tagttgatat ttttaatata ttgatttctt 2340

ttttctcaca gcaacaagtg cttactctag aggttagtgg gccctgatat gtcatcagtc 2400

agatgcctgc ctagccaaag ctggactaag attattctgt acatttgttg atcttgatat 2460

agacttatat ccctgtaggg actgctaatg gctccggctt ctggagtaag gtactggaga 2520

ccactcatcc ctgtgtctgc ttgattggtt cagctgttga attgcccttt tatttggaag 2580

cagtgttgaa gttgtctagg gttcaaatgg ctgctttgta cacctgtcat tagtataagg 2640

cagatgttta ttttatcaag ctattttatc tctacattta actaaaaaca aaagttccca 2700

aagatctgcc ttcacttcag aaattttttt tggattaaaa aaattaagcc tgaaccttaa 2760

ataaagtgag ttggttattc attccaagga ttaagtccca atctacctct cagcacaatg 2820

cagaagctca ccactgtatt gctgccatta actcatgcca gaaccctttg ccaataactg 2880

gaattacaaa tttttgttaa agaaaattta tcaagatctt tctttactgc cttctctata 2940

tgtacatctc aaaaacatgt acatctcaaa aactggagta gaaagttaga ttgctcaact 3000

acaactcctc tagaactcta tagctctgac atacagattc acactctcct ctatttgcta 3060

agtatgtaaa gaatgttttc ttttaaaatg ttctcttttg agaacaactg cttatttgtt 3120

ataaaagcat ttggttaaaa tgatgtcatc ataaaaaaca gtggctttgt ttcaatacat 3180

atttttgaga tgattatcta gaagccagat taataaaatc agcttgtgac cttgctaagc 3240

atataaactg gaaattcaga tacattcaaa attatgggtt catttaaaag tgttctacct 3300

tttgggtatg agactaatat cactaattcc tcaatagtta tcatggctct atcttaatta 3360

attagaaaat atgtgtgttt aattctttga gaattaaaat agagaatatt aacagagggt 3420

taaaaactgc ttcaactcca ataagataaa ggaagctcaa aatctatgag ctgagtgttc 3480

aattagcttt gcctactgag ttcaatttta tgtcaataca acagtggatc agacagtacg 3540

actttgaact ggtgaatgta aacaattgtt tttcacctaa gctgctttgg aagaactgat 3600

gcttgctgct aactaaagtt ttggatgtat cgatttagag aaccaattaa tacctgcaaa 3660

ataaagcata ctgtggtact tctgtttgat ctagtatgtg tgattttaga ttgatggatt 3720

aaaaattaat aaagatcata cattccatac caaaaaaaaa aaaaaaa 3767

<210> 34
<211> 4625
<212> DNA
<213> Homo sapiens

<400> 34
tcgggagaca tggcgggcgt taaagagaag aaaccagtgt gtgtgtagta tgtgtttttt 60

gcatggggca gttggtaaaa acaccgcgtc ccttatctgt atggcttcag agcaatgcga 120

gacggaaaag gttttttgca aggcttcctg tattttgctc tcgtggcatt atccttcagt 180

ggggctattg gactgacttt tcttatgctg ggatgtgcct tagaggatta tggcgtttac 240

tggcccttat tcgtcctgat tttccacgcc atctccccca tcccccattt cattgccaaa 300

agagtcacct atgactcaga tgcaaccagt agtgcctgtc gggaactggc atatttcttc 360

actactggaa ttgttgtttc tgcctttgga tttcctgtta ttcttgctcg tgtggctgtg 420

atcaaatggg gagcctgcgg ccttgtgttg gcaggcaatg cagtcatttt ccttacaatt 480

caagggtttt tccttatatt tggaagagga gatgatttta gctgggagca gtggtagcac 540

tttattctga ttacagtgca ttgaatttct tagaactcat actatctgta tacatgtgca 600

catgcggcat tttactatga aatttaatat gctgggtttt ttaatacctt tatatatcat 660

gttcacttta agaaagactt cataagtagg agatgagttt tattctcagc aaatagacct 720

gtcaaattta gattatgtta ctcaaattat gttacttgtt tggctgttca tgtagtcacg 780

gtgctctcag aaaatatatt aacgcagtct tgtaggcagc tgccacctta tgcagtgcat 840

cgaaaccttt tgcttgggga tgtgcttgga gaggcagata acgctgaagc aggcctctca 900

tgacccagga aggccggggt ggatccctct ttgtgttgta gtccatgcta ttaaaagtgt 960

ggcccacaga ccaagagcct caacatttcc tagagcctta ttagaaatgc agaatctgaa 1020

gccccactct ggacccagga cattttgatg agatccaaag gagttgtatg cacatgaaag 1080

tttgagaagc atcatcatag agaagtaaac atcacaccca acttccttat ctttccagtg 1140

gctaaaccac ttaacctctc tgggtgttac ctgctcattt gtttaaaaaa aaaaaaaaag 1200

tctcacctgc tttcatgctg aggacaagtt cagatgttca agcctataat atttaggcag 1260

ttcctcaaat ttatgaaaag tgttctcaga attgggagac agtcaaaggg tacaaagcct 1320

cagttaggag gaataagtgt gatttttttt taaagatcac ttgcacagca tgctaaatat 1380

aggaataatt gaatgtatat ttcaatattg ctaagagagt aaatttctaa tgttctcata 1440

aaaaagttaa atatttgaga tcatatgtta attagtgtaa tcattccacc ttatattcaa 1500

aaatcataaa accgtattgt accctataaa aatatacaat aatttgtcaa tatataatca 1560

aaataaaaaa caaaacatac tctctccccc aaaaaaacat ctcagtgggg aacagatgta 1620

tcttttcatc tgaaagacaa tgctggggga agagctccac tgagatgcgg gcagggaggc 1680

tgggctcgag ccagcccctg cgttagcagg agggggagaa cagataggta actcttttac 1740

atttccttta tgatctggca cttctcccca gctccttccc tctgcccccc acccctactc 1800

ctcaacagtt ctggtttgcc ctgacttctc tacggctctg gcttcttccc gaagagatat 1860

aggagccatg taagcacgca gtgggtgaac tgcttaattt cactacgtgt tgatgtactt 1920

gtcttccgtc ctgtaggtct tttctatata actttatgcc acccttaaat gaatcattgg 1980

gtatacctgt catgttggat cctgtaatca cagttttccc tgctcacctt tttgtctaag 2040

atctattgag aaagggaaat atgggaagga gaaccatttg atcagaatac aaccaatagt 2100

ctttaagcat tgttaaagta tgaaactgaa atacattcaa aacacttaat ccttgaggct 2160

tgtgatctga gtaattagca ggtatgatgc tgggactgga aaatagaaag taataactaa 2220

agggttaatg tgcaacgtta ttttttggcc ttgttcatga ttttatgttt tcagtgtcct 2280

gtgtacatat agaattgtta aagttgtcat ttccaatatt tatattagaa aaattattta 2340

gatactttat aattttaacc ggcattttta ataatgacac ttgcatttat tgtattgtaa 2400

taaatttcac ttttaacttt aaaagtttaa ctttaaaatt tttttgtgat gttgccttgc 2460

ctgaaaagat aacaaaaatg agagaatttc ttgatgtttt aaaatgggca gttttgagca 2520

ataatctgtc ctaacagaac agtagcaata agttttagga taccatcttg aatgtctagt 2580

tggtgtgcaa tagcttttct ttctaagatg gcaataatga ttcatttcta ctacattttg 2640

caaaagtgtt tttgttgctt atacacattt tcaataacca aggtagcctt catatgtagc 2700

cttaaagcat tacctcttga ttgtatcttt agattgatat aaagtacttg catatagagt 2760

atttgaagtg atagattatt agatttgctc tatgtctgaa aagagagcta ttctgcagtg 2820

cctaaatatc atttaaacag taaatattaa taggaaatat tgctatatct gaatatataa 2880

tacaaaagtt tgatcatggt gacacaaatg ttggacattt ttttccttat aaaaggctct 2940

ttttttatat attgtacaat atatttggag attcagagca tagtgactat agtcgaaaac 3000

tgagattgca cttccaaaat tggccacaag taaataatct tatgaaggga ttctttatca 3060

tgtttcaaac aagtgggtta caagcagact ttgagacact tttccacaga aacaatacta 3120

tgaattggtg attgagttcc caggccaagc ctccctcaac aggttcaact ctaatatacc 3180

taacctgtga tactgaaggt gcctgcctga gttttgggtc tctgagacag ggtagtgtga 3240

gtagtttgga ggaaggacag tgcaactttc cacccctttt cctaagaaca aggtctttct 3300

ccttttaatt tttccactca ttttcacctc ctaatgccct tgagatccag gtacactcct 3360

gggagttttg ttcacctctc ccaactgaga accttcccac tgggctccat cctccctcct 3420

gaggttcttc atattccaga gtcacccacc cttctcctcc cattagtcag ttctctaagt 3480

acagctgatg tcatgtggtg ctgagaagaa agcagatcac acttcatcac agaaagaatg 3540

ccttgtgatt atcttctcca catctgaaat tccttttgac acctgcattg ggccgactgc 3600

cattcccatg actgctgcac ctgcgttttt agagaatgcc tcataaccca ctgattctca 3660

ttcacagaga atgggagaac ggaatgaaga aagattccag cagcttatag aaggatagca 3720

atattttggg acagggaaaa tcctgtcata cctcatctct tcctcaggag gagttctgag 3780

ctggtcctgc ttttcatagt tgtttctttt cttccactta agaactcata gatttttctt 3840

actgtcctaa ggaagtcctt acctctgagg tatctcctca atgaatactg ttttcaaggc 3900

tgaaatagtt cattatgtta ataaccttct ttatgttctc agggaaatgc ttaggtggtg 3960

tcacaaaatg tgccttttct tttctttttt tttttttttt tttgaggcag agtctcgctc 4020

tgttgcccag gctggagtgc agtggtgcga tcttggctca ctgcaagctc cgcctcccgg 4080

gttcacgcca ttctcctgcc tcagcctccc aaggagctgg gactacaggc tcccgccacc 4140

acgcctggct aatttttttg tatttttagt aaagacgggt ttcatcgtgt tagccaggat 4200

ggtctcgatc tcctgacctc atgatccgcc cgcctctgcc tcccaaagtg ctgggattac 4260

aggcctgagc cactgtgccc agccaaaatg tgcctttgca aagtttgcga aatcagattt 4320

tgtatcccaa tagaaccaaa atatttatga ggatgctagc attttccaag catagtaatt 4380

agttcacaac tgagaaatat tatgtctgta gtagataaat attagttgtg cattttaatt 4440

taattctcct ttttccattt tgtctcatga agtaccttat tgcaaaaatc ccactgagta 4500

atagctcata aattataatc tttcaaatag ccatgctacc agcgtacaac agtgatacat 4560

gtaaccccaa atgtgatgtg agaggacgat tactttgtaa ataaaacttg ttattgacat 4620

tttaa 4625

<210> 35
<211> 744
<212> DNA
<213> Homo sapiens

<400>  35
gagtatttga ggctcggagc caccgccccg ccggcgcccg cagcacctcc tcgccagcag      60

ccgtccggag ccagccaacg agcggaaaat ggcagacaat ttttcgctcc atgatgcgtt     120

atctgggtct ggaaacccaa accctcaagg atggcctggc gcatggggga accagcctgc     180

tggggcaggg ggctacccag gggcttccta tcctggggcc taccccgggc aggcaccccc     240

aggggcttat cctggacagg cacctccagg cgcctaccct ggagcacctg gagcttatcc     300

cggagcacct gcacctggag tctacccagg gccacccagc ggccctgggg cctacccatc     360

ttctggacag ccaagtgcca ccggagccta ccctgccact ggcccctatg gcgcccctgc     420

tgggccactg attgtgcctt ataacctgcc tttgcctggg ggagtggtgc ctcgcatgct     480

gataacaatt ctgggcacgg tgaagcccaa tgcaaacaga attgctttag atttccaaag     540

agggaatgat gttgccttcc actttaaccc acgcttcaat gagaacaaca ggagagtcat     600

tgtttgcact tacatgtgta aaggtttcat gttcactgtg agtgaaaatt tttacattca     660

tcaatatccc tcttgtaagt catctactta ataaatatta cagtgaatta cctgtctcaa     720

tatgtcaaaa aaaaaaaaaa aaaa                                            744

<210>  36
<211>  2094
<212>  DNA
<213>  Homo sapiens

<400>  36
cacttgttca atgatgtacc cccagtgtca ggcgctttgc aaacacacga tacatacggg      60

ttgatgtttg gtcaagagag gaattaagac caggcagaca gcaggctggg atcagagaga     120

ccccatttct gtctgaaatg tctgcagaga acctggtgcc tgcctcagcc ctagctctgg     180

ggaaatgaaa gccaggctgg ggttcaaatg agggcagttt cccttcctgt gggctgctga     240

tggaacaacc ccatgacgag aaggacccag cctccaagcg gccacaccct gtgtgtctct     300

ttgtcctgcc ggcactgagg actcatccat ctgcacagct ggggcccctg ggaggagacg     360

ccatgatccc caccttcacg gctctgctct gcctcgggct gagtctgggc cccaggaccc     420

acatgcaggc agggcccctc cccaaaccca ccctctgggc tgagccaggc tctgtgatca     480

gctgggggaa ctctgtgacc atctggtgtc aggggaccct ggaggctcgg gagtaccgtc     540

tggataaaga ggaaagccca gcaccctggg acagacagaa cccactggag cccaagaaca     600

aggccagatt ctccatccca tccatgacag aggactatgc agggagatac cgctgttact     660

atcgcagccc tgtaggctgg tcacagccca gtgaccccct ggagctggtg atgacaggag     720

cctacagtaa acccaccctt tcagccctgc cgagtcctct tgtgacctca ggaaagagcg     780

tgaccctgct gtgtcagtca cggagcccaa tggacacttt ccttctgatc aaggagcggg     840

cagcccatcc cctactgcat ctgagatcag agcacggagc tcagcagcac caggctgaat     900

tccccatgag tcctgtgacc tcagtgcacg gggggaccta caggtgcttc agctcacacg 960

gcttctccca ctacctgctg tcacacccca gtgaccccct ggagctcata gtctcaggat 1020

ccttggagga tcccaggccc tcacccacaa ggtccgtctc aacagctgca ggccctgagg 1080

accagcccct catgcctaca gggtcagtcc cccacagtgg tctgagaagg cactgggagg 1140

tactgatcgg ggtcttggtg gtctccatcc tgcttctctc cctcctcctc ttcctcctcc 1200

tccaacactg gcgtcaggga aaacacagga cattggccca gagacaggct gatttccaac 1260

gtcctccagg ggctgccgag ccagagccca aggacggggg cctacagagg aggtccagcc 1320

cagctgctga cgtccaggga gaaaacttct gtgctgccgt gaagaacaca cagcctgagg 1380

acggggtgga aatggacact cggagcccac acgatgaaga cccccaggca gtgacgtatg 1440

ccaaggtgaa acactccaga cctaggagag aaatggcctc tcctccctcc ccactgtctg 1500

gggaattcct ggacacaaag gacagacagg cagaagagga cagacagatg gacactgagg 1560

ctgctgcatc tgaagccccc caggatgtga cctacgccca gctgcacagc tttaccctca 1620

gacagaaggc aactgagcct cctccatccc aggaaggggc ctctccagct gagcccagtg 1680

tctatgccac tctggccatc cactaatcca ggggggaccc agaccccaca agccatggag 1740

actcaggacc ccagaaggca tggaagctgc ctccagtaga catcactgaa ccccagccag 1800

cccagacccc tgacacagac cactagaaga ttccgggaac gttgggagtc acctgattct 1860

gcaaagataa ataatatccc tgcattatca aaataaagta gcagacctct caattcacaa 1920

tgagttaact gataaaacaa aacagaagtc agacaatgtt ttaaattgaa tgatcatgta 1980

aatattacac atcaaaccaa tgacatggga aaatgggagc ttctaatgag gacaaacaaa 2040

aaatagagaa aaattaataa agtcaaaatg tttattcttg aaaaaaaaaa aaaa 2094

<210> 37
<211> 1562
<212> DNA
<213> Homo sapiens

<400> 37
gcgggcggca ttctggcgcg gagcggagcg gcggcgggcg cagctagcgg gtcggccgcg 60

gagcggaggt gcagctcggc ttcccccggc acccctcccc ctcgggcgcc agccccaccc 120

ctccgccggc cgggccgacc ccgccgtact atcccctgcg gcgcgagccc ggggcggctc 180

caagcgcccc ccagcagacc cccatcatgg gcagccagag ctccaaggct ccccggggcg 240

acgtgaccgc cgaggaggca gcaggcgctt cccccgcgaa ggccaacggc caggagaatg 300

gccacgtgaa aagcaatgga gacttatccc ccaagggtga aggggagtcg ccccctgtga 360

acggaacaga tgaggcagcc ggggccactg gcgatgccat cgagccagca ccccctagcc 420

agggtgctga ggccaagggg gaggtccccc ccaaggagac ccccaagaag aagaagaaat 480

tctctttcaa gaagcctttc aaattgagcg gcctgtcctt caagagaaat cggaaggagg 540

gtgggggtga ttcttctgcc tcctcaccca cagaggaaga gcaggagcag ggggagatcg 600

gtgcctgcag cgacgagggc actgctcagg aagggaaggc cgcagccacc cctgagagcc 660

aggaacccca ggccaagggg gcagaggcta gtgcagcctc agaagaagag gcagggcccc 720

aggctacaga gccatccact ccctcggggc cggagagtgg ccctacacca gccagcgctg 780

agcagaatga gtagctaggt aggggcaggt gggtgatctc taagctgcaa aaactgtgct 840

gtccttgtga ggtcactgcc tggacctggt gccctggctg ccttcctgtg cccagaaagg 900

aaggggctat tgcctcctcc cagccacgtt ccctttcctc ctctccctcc tgtggattct 960

cccatcagcc atctggttct cctcttaagg ccagttgaag atggtccctt acagcttccc 1020

aagttaggtt agtgatgtga aatgctcctg tccctggccc tacctccttc cctgtcccca 1080

cccctgcata aggcagttgt tggttttctt ccccaattct tttccaagta ggttttgttt 1140

accctactcc ccaaatccct gagccagaag tggggtgctt atactcccaa accttgagtg 1200

tccagccttc ccctgttgtt tttagtctct tgtgctgtgc ctagtggcac ctgggctggg 1260

gaggacactg ccccgtctag gtttttataa atgtcttact caagttcaaa cctccagcct 1320

gtgaatcaac tgtgtctctt ttttgacttg gtaagcaagt attaggcttt ggggtggggg 1380

gaggtctgta atgtgaaaca acttcttgtc tttttttctc ccactgttgt aaataacttt 1440

taatggccaa accccagatt tgtacttttt ttttttttct aactgctaaa accattctct 1500

tccacctggt tttactgtaa catttggaaa aggaataaat gtcgtccctt tagtggtgct 1560

tt 1562

<210> 38
<211> 2118
<212> DNA
<213> Homo sapiens

<400> 38
cacgtgaccg aggcacagat cagctgatgc cggagggttt gaagccgcgc cgcgagggag 60

cgaggtcgca gtgacagcgg cgggcgatcg gacccaggct gccccgccgt acccgcctgc 120

gtcccgcgct cccgccccag catgacagcc ccggcgggtc cgcgcggctc agagaccgag 180

cggcttctga cccccaaccc cgggtatggg acccaggcgg ggccttcacc ggcccctccg 240

acacccccag aagaggaaga ccttcgccgt cgtctcaaat actttttcat gagtccctgc 300

gacaagtttc gagccaaggg ccgcaagccc tgcaagctga tgctgcaagt ggtcaagatc 360

ctggtggtca cggtgcagct catcctgttt gggctcagta atcagctggc tgtgacattc 420

cgggaagaga acaccatcgc cttccgacac ctcttcctgc tgggctactc ggacggagcg 480

gatgacacct tcgcagccta cacgcgggag cagctgtacc aggccatctt ccatgctgtg 540

gaccagtacc tggcgttgcc tgacgtgtca ctgggccggt atgcgtatgt ccgtggtggg 600

ggtgaccctt ggaccaatgg ctcagggctt gctctctgcc agcggtacta ccaccgaggc 660

cacgtggacc cggccaacga cacatttgac attgatccga tggtggttac tgactgcatc 720

caggtggatc cccccgagcg gccccctccg ccccccagcg acgatctcac cctcttggaa 780

agcagctcca gttacaagaa cctcacgctc aaattccaca agctggtcaa tgtcaccatc 840

cacttccggc tgaagaccat taacctccag agcctcatca ataatgagat cccggactgc 900

tataccttca gcgtcctgat cacgtttgac aacaaagcac acagtgggcg gatccccatc 960

agcctggaga cccaggccca catccaggag tgtaagcacc ccagtgtctt ccagcacgga 1020

gacaacagct tccggctcct gtttgacgtg gtggtcatcc tcacctgctc cctgtccttc 1080

ctcctctgcg cccgctcact ccttcgaggc ttcctgctgc agaacgagtt tgtggggttc 1140

atgtggcggc agcggggacg ggtcatcagc ctgtgggagc ggctggaatt tgtcaatggc 1200

tggtacatcc tgctcgtcac cagcgatgtg ctcaccatct cgggcaccat catgaagatc 1260

ggcatcgagg ccaagaactt ggcgagctac gacgtctgca gcatcctcct gggcacctcg 1320

acgctgctgg tgtgggtggg cgtgatccgc tacctgacct tcttccacaa ctacaatatc 1380

ctcatcgcca cactgcgggt ggccctgccc agcgtcatgc gcttctgctg ctgcgtggct 1440

gtcatctacc tgggctactg cttctgtggc tggatcgtgc tggggcccta tcatgtgaag 1500

ttccgctcac tctccatggt gtctgagtgc ctgttctcgc tcatcaatgg ggacgacatg 1560

tttgtgacgt tcgccgccat gcaggcgcag cagggccgca gcagcctggt gtggctcttc 1620

tcccagctct acctttactc cttcatcagc ctcttcatct acatggtgct cagcctcttc 1680

atcgcgctca tcaccggcgc ctacgacacc atcaagcatc ccggcggcgc aggcgcagag 1740

gagagcgagc tgcaggccta catcgcacag tgccaggaca gccccacctc cggcaagttc 1800

cgccgcggga gcggctcggc ctgcagcctt ctctgctgct gcggaaggga cccctcggag 1860

gagcattcgc tgctggtgaa ttgattcgac ctgactgccg ttggaccgta ggccctggac 1920

tgcagagacc cccgcccccg accccgctta tttatttgta gggtttgctt ttaaggatcg 1980

gctccctgtc gcgcccgagg agggcctgga cctttcgtgt cggacccttg ggggcgggga 2040

gactgggtgg ggagggtgtt gaataaaagg gaaaataaat gtgtcgtttt catttttaaa 2100

aaaaaaaaaa aaaaaaaa 2118

<210> 39
<211> 4588
<212> DNA
<213> Homo sapiens

<400> 39
aaacttccga gttaagccgc cgctgaggcc ggaaggagct agacggcggt cgggtggaaa 60

gtggttggtt tctgataact tcctaaacat caccaatgta gcttttgatg accgtgggct 120

ctatacctgt ttcgtcacct ctccaattcg tgcctcctac tctgtcaccc tacgtgttat 180

cttcacctcg ggagacatga gtgtctatta catgattgtt tgcctgattg cctttacaat 240

cacactcatc ttgaatgtca cacggctgtg catgatgagc agccatcttc gcaagactga 300

gaaggctatc aatgagttct ttagaactga aggggctgag aaacttcaga aggcctttga 360

gattgcaaaa cgtatcccca tcattacctc agccaaaact ctggagctcg ccaaagtcac 420

acaatttaag accatggagt ttgctcgtta tattgaagaa ctggcaagaa gtgtccctct 480

tccacctctt attctaaact gtcgagcctt tgttgaggag atgtttgagg ctgtgcgagt 540

ggacgaccct gatgacctgg gtgaaagaat taaagagaga cctgccttga atgctcaagg 600

tggcatctat gtcattaacc cagagatggg acggagtaat tcaccaggag gagattcaga 660

tgatggctct ctgaatgaac aaggccagga aatagcagtt caggtttctg tccaccttca 720

gtcagaaacc aaaagtattg atacagagtc tcaaggcagc agtcatttca gtccacctga 780

tgatatagga tctgcagaat ctaactgtaa ctacaaagat ggggcatatg aaaactgtca 840

gctgtaacct acaatgctgt aacccagtac ctacaaaatc agctcgctct cagaaaagga 900

acctgtttct tagaagaagt aacatttttg ccaaaagatg actggggttt tccgtttgtt 960

aatattaagc acatcagaac gtgaattgcc aaagtcttca ttagaaggca gcatttttcc 1020

tctctgatac ttttcagtca ttttccttag agctttatta aattatgcat gctaagattt 1080

aaaggagccc cagataaaca tgatggggaa aagcactgaa ctaagagtcc catggtttct 1140

cttctggtca cagttcttcc attggttgga tctgatactt atcttgggac ttcagttttt 1200

ccgtcaataa gatgagggat taggtgagat ctgaagttgt ttctagctct atagcttctt 1260

aacccatcac ctttaaatta ccagaatctt cactcatctc taagtaaacc tttcccggga 1320

cttttactcg cttcttttgg aaaggattaa gctgagatct aaatttccac accaatgtca 1380

taatgcacag aggtttttga aaaacatctg agtgtttttc agatgttttg ccatgtggag 1440

catataatga tatgtgcaag attgaatctt ttcaatgtag cacatgtctg tagggttata 1500

cagatgtcag agagctaact gctctgtaaa ctactttcca tgagtaaatt ggtccttggt 1560

gggggtgtat catattttta acttactgag atatcatttt agttcattga ggttggcagg 1620

gattgcctaa ctgatcttcc aaagtgagca gtttatttct aaggtataac gtctttgatg 1680

cttttagaat aagaacagtg tcaaatcatc tgtcttctgg aaaatcatgg attttcatat 1740

ttctgttaac agaatttctc aggctttccc tttttaaaag tattggactc tacaaatagg 1800

ttctatattt gggatctcat cctaggagaa aacccaaaac tggattcctc ttaagacctc 1860

tgtcaaccct cctgcccttt gtggtcttag gtatggtgct ataggttgca tgcgtcttta 1920

tcttgtttgt ttggttaata ttttgttgtt gaggttttta tttttgttta cttcagatac 1980

ataattctga gctatggctg cttttgtagc ctttccaaga agcactaacc tgaaataaga 2040

ttagataatt gtgagggtgt gttactatat taaaatacac acacaactga tctagacatc 2100

aagaggaaga aaaatgatgg atgatgccac ctgcttcaac tgtatataat aaacacttat 2160

gatgacattt cttgcctggc tgagatctga tataatggaa ttgtaaatac tcttcagaac 2220

aatttcttca gctacaggaa gcgtggtgcc atataatttt aagaacttgg cagtggagct 2280

ccatttgggg cttcacgttt cttcatatga cttctgatta ttgaagcatt acttcagcta 2340

gaggctcctg agggaaatgt tcagaggagg ttttatcagg attttaattt aaggtttcaa 2400

acaggggaag acaggaaatt caaagcgtga cgggataaaa ctcatgcctc cctttgtcca 2460

ggcttatcag aagtaataca tctgctctga atagcatgaa tgaatcaatg tgcagtttta 2520

tcagatggca tcatggataa agatgataat gctgcctttt ctgtctctca ggctgtttcc 2580

atggaaacct ctagagccaa ataaaagcta accaatcatt tagccaaagc ttgccttggc 2640

tcatagactg gtatttcttt aaggaaaatt gtttttatat atttgactat aagagcaaag 2700

gctcttgaac atatcctaga ttatggaaca ctttttccct tccctttctt ctggaaaaat 2760

taaatttttt ttctgtccac tgagactgga gtgcagtggc aagatcatag ctcactgcag 2820

cctccaactc ctggggtcaa gcgatcgtcc tgcttcagcc tcccaagcag ctaggactac 2880

aggtgtgcac cacctttcct ggctaatttt tttttatttt ttgtagagat gggggtctca 2940

ctttgttgcc caggctgacc ttgtacttgt ggcttcaaat gatcctcctg ccttacctcc 3000

caaagtgttg agattacagg catgagccac tgtgcctggc caaaaaacat tttaaatccc 3060

ttgtctgggg gtcagtcctc taaacatccc tcagatttca gatagtactt tcaaccctgt 3120

cctaacatag caggtttgca gtaatctttt agaatatagc ttgtcaaatt agagaatggt 3180

tttaccccac atgttctcat aggagacatt attatttaga accctaaggt agacatgttt 3240

aaaatcaaag tcctaagaaa cagaactttg gaaaaatgga ggaaatgttt ttaaagtctg 3300

taagtttgca cgatactgta tagtaactaa atgcatgcta ctccgttgta tcctagttat 3360

tttagaaaca gaggtggcct aatttggtgg ccaaagtaac tggtttactt tgagtgtacc 3420

agcttatggt gccattgatg aggaattaaa gtaggtcaaa atttaattgg agttggtatt 3480

acttcgtaaa gctagttttc aagaggaaga aaaccacact tactaatgtt ttctgtatct 3540

aatcaaatac tcttcatata taattaagcc tcatgttatc ttttttttaa atcaaccttt 3600

tgaacttcaa ctacagtcta aaagtcttga tggtaactat agtgtaatta tctttttgtc 3660

tcactggatt ttatagttag tggaaaatgc ctttacaaaa tgtatttaaa atagctgtca 3720

tctcatttgt aaattttgtc gtgtattgtg atatagtgaa ccttattgtc cttatgaaat 3780

ggtagctttg tgaaatacat tcaccaaaaa tcaaaatttg aacatcttta tgattcctta 3840

ccagctgaag ccagatagac aggtattaat tgagctgatg ccccacttga gtttatagac 3900

tgtttgataa ctgcctgtcc tccaaattgt gtatgtatat gttacgatgg tttaattctt 3960

gagtcagggc cagcacgcat ttatattttc taccaattac cttgatagaa atatcttaga 4020

aattgctgac ctggaacggt tgtgagaaga ctcctggctt ctttcttgcc tcacttaaca 4080

aatattttaa ggtcaaagca atatctgtgc acggctttcc ttttgctcct ccagacaagt 4140

gaggctgttg gtatagtcct cttcaccctc tgcatgtagc ttcaccctag atcagacttt 4200

tgtctcttgg gtcccagatg gcacaggagc actgcatgct tgttttctag agcccagcca 4260

gtcatgggtg ctagcctagt ctccacacac cagcaagtag aacccaagtg tattgtataa 4320

atatttcctg agtaccagta agagaatgca ttcttttctc atctaggcca ggaatgttga 4380

aaatgctcag ccttacatag aaactcctag attttcacta acgcatttca caaaagtaaa 4440

taagtatttc atataattca gaggatgttt aaattgtcag cattttaata aatacttgca 4500

ttataatttt gtctcttttt taaagaaagt catacttgaa tataatttat taaacgttca 4560

atggagtata tagtctattt gaaatttt 4588

<210> 40
<211> 2482
<212> DNA
<213> Homo sapiens

<400> 40
cccagatcca ggccgggccg cggctctcgc cgcccagccc agcccagccc ggcccggccc 60

ggccctgccg cggaggcgag gccgccagtg tcccgcgccc ctgatatctg cagtgagcct 120

gatacctgcc tctgcccttc tgagcctgtt cctcttccct gagtacaggg cacaaagctt 180

gcgccctgag gggcggccgg cgcgctccct ggcccggtcc ccgcccggcc ccgggccccc 240

cgcccctccc cgacccgggg ccggggcccc tgccgccgcc gccgccgcct tccgacccct 300

gcgccccggc cccggtcccc cgggccatgc agcctcggcc ccgcgggcgc ccgccgcgca 360

cccgaggaga tgaggctccg caatggcacc ttcctgacgc tgctgctctt ctgcctgtgc 420

gccttcctct cgctgtcctg gtacgcggca ctcagcggcc agaaaggcga cgttgtggac 480

gtttaccagc gggagttcct ggcgctgcgc gatcggttgc acgcagctga gcaggagagc 540

ctcaagcgct ccaaggagct caacctggtg ctggacgaga tcaagagggc cgtgtcagaa 600

aggcaggcgc tgcgagacgg agacggcaat cgcacctggg gccgcctaac agaggacccc 660

cgattgaagc cgtggaacgg ctcacaccgg cacgtgctgc acctgcccac cgtcttccat 720

cacctgccac acctgctggc caaggagagc agtctgcagc ccgcggtgcg cgtgggccag 780

ggccgcaccg gagtgtcggt ggtgatgggc atcccgagcg tgcggcgcga ggtgcactcg 840

tacctgactg acactctgca ctcgctcatc tccgagctga gcccgcagga gaaggaggac 900

tcggtcatcg tggtgctgat cgccgagact gactcacagt acacttcggc agtgacagag 960

aacatcaagg ccttgttccc cacggagatc cattctgggc tcctggaggt catctcaccc 1020

tccccccact tctaccctga cttctcccgc ctccgagagt cctttgggga ccccaaggag 1080

agagtcaggt ggaggaccaa acagaacctc gattactgct tcctcatgat gtacgcgcag 1140

tccaaaggca tctactacgt gcagctggag gatgacatcg tggccaagcc caactacctg 1200

agcaccatga agaactttgc actgcagcag ccttcagagg actggatgat cctggagttc 1260

tcccagctgg gcttcattgg taagatgttc aagtcgctgg acctgagcct gattgtagag 1320

ttcattctca tgttctaccg ggacaagccc atcgactggc tcctggacca tattctgtgg 1380

gtgaaagtct gcaaccccga gaaggatgcg aagcactgtg accggcagaa agccaacctg 1440

cggatccgct tcaaaccgtc cctcttccag cacgtgggca ctcactcctc gctggctggc 1500

aagatccaga aactgaagga caaagacttt ggaaagcagg cgctgcggaa ggagcatgtg 1560

aacccgccag cagaggtgag cacgagcctg aagacatacc agcacttcac cctggagaaa 1620

gcctacctgc gcgaggactt cttctgggcc ttcacccctg ccgcggggga cttcatccgc 1680

ttccgcttct tccaacctct aagactggag cggttcttct tccgcagtgg gaacatcgag 1740

cacccggagg acaagctctt caacacgtct gtggaggtgc tgcccttcga caaccctcag 1800

tcagacaagg aggccctgca ggagggccgc accgccaccc tccggtaccc tcggagcccc 1860

gacggctacc tccagatcgg ctccttctac aagggagtgg cagagggaga ggtggaccca 1920

gccttcggcc ctctggaagc actgcgcctc tcgatccaga cggactcccc tgtgtgggtg 1980

attctgagcg agatcttcct gaaaaaggcc gactaagctg cgggcttctg agggtaccct 2040

gtggccagcc ctgaagccca catttctggg ggtgtcgtca ctgccgtccc cggagggcca 2100

gatacggccc cgcccaaagg gttctgcctg gcgtcgggct tgggccggcc tggggtccgc 2160

cgctggcccg gaggccctag gagctggtgc tgcccccgcc cgccgggccg cggaggaggc 2220

aggcggcccc cacactgtgc ctgaggcccg gaaccgttcg cacccggcct gccccagtca 2280

ggccgtttta gaagagcttt tacttgggcg cccgccgtct ctggcgcgaa cactggaatg 2340

catatactac tttatgtgct gtgtttttta ttcttggata catttgattt tttcacgtaa 2400

gtccacatat acttctataa gagcgtgact tgtaataaag ggttaatgaa gtgtgtgcct 2460

caaaaaaaaa aaaaaaaaaa aa 2482

<210> 41
<211> 7725
<212> DNA
<213> Homo sapiens

<400> 41
agaatcttct gtaggccttt ctcttgcctt cttttattca caactgatga cactgcatat 60

cttcccctgt tcttattggg agaaggcctt gtgtgtcacc aagaggttct cagaagggac 120

ctgtcagttt ttggttaaaa gaacccggaa agagaaggac tatgggggaa ctgatggcgt 180

tcctgttacc tctcatcatt gtgttaatgg tgaagcacag cgattcccgg acgcactctc 240

tgagatattt tcgcctgggc gtttcggatc ccatccatgg ggtccctgaa tttatttcgg 300

ttgggtacgt ggactcgcac cctatcacca catatgacag tgtcactcgg cagaaggagc 360

cacgggcccc atggatggca gagaacctcg cgcctgatca ctgggagagg tacactcagc 420

tgctgagggg ctggcagcag atgttcaagg tggaactgaa gcgcctacag aggcactaca 480

atcactcaga taatgtggct cacaccatca agcaggcatg ggaggccaat cagcatgagt 540

tgctgtatca aaagaattgg ctggaagaag aatgtattgc ctggctaaag agattcctgg 600

agtatgggaa agacacccta caaagaacag agcccccact ggtcagagta aatcgcaaag 660

aaacttttcc aggggttaca gctctcttct gcaaagctca tggcttttac cccccagaaa 720

tttacatgac atggatgaaa aacggggaag aaattgtcca agaaattgat tatggagaca 780

ttcttcccag tggggatgga acctatcagg cgtgggcatc aattgagctt gatcctcaga 840

gcagcaacct ttactcctgt catgtggagc actgcggtgt ccacatggtt cttcaggtcc 900

cccaggaatc agaaactatc cctcttgtga tgaaagctgt ctctgggtcc attgtccttg 960

tcattgtgct ggctggagtt ggtgttctag tctggagaag aaggccccga gagcaaaatg 1020

gagccatcta ccttccaaca ccagatcgat gattgcagat ccctcttttc cagttctcct 1080

tcctctagga gccatgttat cctctgtccc ccatagagtc aagcctagtg cttgaaggtc 1140

ctgacgacac ccacaacata catgagagta atgggattga gcatttatgg cagcaacaga 1200

ggagccacaa aatgttcttt gttctttggc tccaaaaaga ctgtcagctt tcagtctctt 1260

ttgatggact gttttatcag agttgacttt aaatacagct tgtctcatga cacaacgctt 1320

ccctacattc tatttgtcaa tgatgatttg caactagttg gagattctca gagcaggaag 1380

gaatcttttc aaccagagca ggaactgtct tctgcaatgc cttggacttg agcctccagc 1440

ctccacttga acaccatgtg aagggaacct cagtacttca taaaatggcc tttctcattc 1500

atctttcatg ggaacattta ttgtacaagc gctttgaata tcatgggcac catgactgtg 1560

accctacagg taggattgga tcactccatg agagtagccg gcaggtttct acaatggcct 1620

gggaatggac tgattatttt tatacatttt ctggcctgag agaaagccaa agtcccctgc 1680

tgttcacagc aaccctgcct gggagcttgg aatcttggta atctgcccgg ttggatctat 1740

ggaggtagtc tcaccctttt tgtcttttgt gggaaattaa gagaaataat tatcagacat 1800

atcatcacct ccagtggaac tacagagacc tggacccagc tgcactattt taatgtaaaa 1860

ataacagtat ggccaggtgc agtggctcac gcctgtaatc ccatcacttt gagcagccaa 1920

ggcgggcgga tcacgaggtc aggagattaa gaccatcctg gccaatatgg tgaaaccctg 1980

tctctactaa aatacaaaaa attagctggg catggtgttg cgtgcctgta gtcccagcta 2040

cttgggaggc tgagacaggg gaattgcttg aacccgggag gcagagattg cagtgagccg 2100

agatcacgcc actgcactcc agcctggcga cagagtgaga ctctatctca aaataataat 2160

aataataata ataataataa taataataat aacagtatat ttggtgtcag gagagggctc 2220

aattctcatt tctgcctttc ctgtgctggc tcatggtagc tgggcatgac ttgccttcct 2280

acataggttg tcttcataca tatgcactgg gaatcaataa aagcccatgg tgagaatgaa 2340

catcccctta atgttcctta ctatccccaa cccctgaggc ctcacctact gccctgccat 2400

gtggagctac ttgccctggg gctgccagtc acacattcct cggtcctact tctctgaccc 2460

cgtttgactc tgcacctgag ccctaatgct tacttcagtg acctgaactt tgacaagtgg 2520

cttttgtcct gcacctcagg tttgacctct gctctccctt gaccttgact gtgacatttg 2580

acctttggct ttaatcatta cagcctcaga taaaggtacc ttcagcccgg gcacagtggc 2640

tcacgcctgt aatcctagca ctttgggagg ctgaggcagg cggattccct gagctcagga 2700

gttcaagacc agcctgggca acacggtgaa accctgtctc tactaaaata caaataatta 2760

gctgggcatg gtggcatgtg cctatagtcc cagctacttg ggaggctaag gcaggagaat 2820

cacttgaatc tataaggcag aggtggcagt gagccgagat cacaccactg cactccagcc 2880

tgggcaacgg agcaagactc tgtctccaaa aaaaaaaaga aagataccct cagtgtgcca 2940

ggcctctaag agctcacctg ccaggcttcc tccttgctcc actgtcccat gtaattccat 3000

atatgaagct accactgtac atctctcttt tccggtgcct gttgagttgc atagaagcac 3060

agttgtgttt attttgtttt tagggttgcc atgggcaatt tccgtgccac ttttaagcag 3120

tgttgcactg tgaagagaat gtaggcaagt ttatttctgg aatggtttct tcttacaatc 3180

agaatagtta ggatgtaata tatttttggg tgggcattta aagtgaaaag gtacatattt 3240

acatagacac aggtgataat gtatctatgt aaatgccttt tgattctgca actgcaggat 3300

actctcatca aagacacaga taaaaagcct ctgtgtttcc aaggccttgc cctacaccta 3360

acacataata tgtccaaatg gatgaagagg aggcaaggac aaggatgtga tgacaaaaca 3420

ttctgttatg cacttgtagc atttatgttt cttcctgggg gattttataa tactaaaaga 3480

atcataatat aaagagatga ttaaaaaaaa aatactgccg ggcacggtgg ctcatgcctg 3540

taatcccagc attttgggag gccgaggtgg gcagatcacc tgaggtcggg agttcgagac 3600

cagcctgacc aacatggaga aaccctgtct ctaccaaaca tacaaaatta gccgggcatg 3660

gtggcgcatg cctataatcc cagctactcg ggagtctgag gcagaagaac cgcttgaacc 3720

cgggaggcag aggttgtggt gagccgagat cgcgccatcg cactctagcc tgggcaacaa 3780

gagtgaaact ccatctcaaa aaaataaaaa taaaataagt aagtaatacc taaaattctg 3840

caaccttcat tttactatag atggttgaag attatattac ttcttaattg ttttagcctt 3900

gttattgctt cattacttca tggttgttga gtacagatgc tcagtaatca taacctatga 3960

aatatttgac accatgatct aaacattaaa aacaaataac tgtgctattg ccacagctat 4020

gttttggctt tgaattttct ttactgaata ttttggatca agaacactag atgagaaacc 4080

tgttcaactc tgttcttttt ttttttttta actccactgt atttattacc tgtttttgtt 4140

ttttttgttt gtttgttttt gatgtgcata tagaccaaga tgtggtaaat ttaaatggca 4200

gatgtttttg atgtggtaca tttaagggag agaaagcagt ttaaagagca gggtgaaaaa 4260

tccaacaaga ctccatcgag agtttctgag ctctcccatc aggggccagt cctcctttcc 4320

tctctcctct tactcccatg atttccaagt tgtgatcctt tccttatttc tgaggaatga 4380

cttggtttct cctcttcttt ttttggcctg agagaagatg tttttgcact tgtagctatg 4440

aggaacagat tgtccactag ggaggccagc tgatcatttt ctgccagagt cacacagagc 4500

agtcacacct tattttgaaa accactgtct ggggtctttg tcctcacata tgcaggtcta 4560

gtgtccccac aaagtgatca gatggatata taaagtggag tgccaatgta ttaatttact 4620

gtgagaaaca caattgctaa gtgggtcaga tatctgtctc agctggtcag tacaccttcc 4680

agcaggaaaa tctacataag aacaactaaa tcacaatctg tagagtgctt gatgacccca 4740

gaattggtgc aaggggacac attcttgctt gttagcacct gctctctgga gtttgctatt 4800

ttctcacaca cagtgtatta gttcacagaa tgttctccaa ggaggacagg gggctttgcc 4860

catagccatg tgctgtgggc agcagagcta ggaagaagca caggcatctc ccagcccagg 4920

tgtttcccac ttaactgcat tgcccttttc atcttttttt tttccccaat agcttcagga 4980

cattcagtac attgtgcttt ttagaggttg atgattccac tgcctgagct gccttcacct 5040

ctctttcttt ggaaattgcc atctttgagc attgtatgtc tctgtaacat ctctgcatct 5100

ccttttcact ctggcctccc tttctccatt gtccttctag cttctggttg ccccaaaccc 5160

cacagactgt gtaacaaaac ccaaaaccta ttggtttaaa acaataccca ttttattttc 5220

tttcatagtt tctatgggtc aggaatttgg atatggcttg ggtaagcagt tctggcttct 5280

catggagttg gaggcaagtg gtggctggaa cagaaatggg gcagccaggg gtggtggcca 5340

ggcatcagct acatctcctc catgtagtct caggaactct ccatgtaatc tctctgtgtg 5400

ggttgggctt cctcacaaca tggtggcctc agggcagtca ctgcttacat ggcagctagc 5460

ttccctcaga gtgagtatcc caagagatca cagtcaaagt gcatagcatt tatataatct 5520

aatcttggaa gtcacaaaat gtcattcttg tgatactctc attggtgaag aagtcataaa 5580

atcctgctca ggttcaaaaa gagggtgtat agaacccaac actcaatagg aagattgtca 5640

gagttacatt gtaagaagag catgtagttg ggagatatca ttgtggccat tttgggaaag 5700

atgcaacatg tcacaatgtc atgttctcca tcgtctcccc gttccacctt gtactggttt 5760

ccaagggtag tcgtttaaaa gtaccacaaa aactgggtgg cttggaacaa cagaaattta 5820

ttgtttcaca gttctggagg ccagaagttc aaaatgaagt gttggcagaa acatgctgcc 5880

tctaaaggga ctgggaaagg atctgttcca ggcttccctc ctatcttctg gttgttcctt 5940

gccttctggc tgcgtaactc tagtcttcat ttggtgttct ccctgcatgc atggctttgt 6000

gtccaaattt ccttttttca tgaggccatc agtcatactg gattagggac ccactctact 6060

actgcaggat gacctcacct tcccaaatta catctgcaac aaccctattt ccaaagaagg 6120

ccacattctg aggtactggg ggttataact tcaacatgaa ttttttggag gaaacaattc 6180

aactcctaac acatctctac ccaacctctg taggttccct caatccacca aaattttttg 6240

gctccactgg attatctagc agttaggaaa ccaagatcat taaatgaatt caccgcaact 6300

acagtgagac tgactacctt agcttacctc tgtgaaagga gttaagccaa aggaatctgt 6360

ggaattttgg agagtttggg agcatttggg ggcaggaggc aaatgttctt cctttaaatc 6420

acaacactta gttctcttcc atttataaga ctcacccctc catcccaacc cctgcaccac 6480

aggacaagga agtgttcttg gtcttcaact ttcatccctg atggtgaaag cagttgctcc 6540

tgacctattt gcccaccagc ttctcctctg gagcctgagg cttctgatgc ctgcctggct 6600

ggttctcagt aagaaggtca agttcaacca gaggggagat gctgatgcct ttcagtactt 6660

aaatatgagt tcagaccctg gggcctggac ataagatttg gggtcccctg gatataagat 6720

ttctgaaaac actcagactg tggagacccc tgctgaggga gaagccccaa actgtggctt 6780

caggggaatg caccaaggct ctcattgagg ccaccttctc caacaagctc ccctcctgct 6840

tccccatggc tggcatggct gaggaaaaag gacactgagc acagcccgtg catgagcggc 6900

ttgccatgca acaggataaa acccataatg ccactcagca agccttggtt gtaaatctag 6960

tttgattaca tttgtaatca aatgatggcc atttgttctg tttctggttt gtgaaccaac 7020

tgaagacata agcagggcct cagctaaccc acaaatagca catgtgtgca aactggaaaa 7080

atgaaccctt cttctgggag gacgccagcc caggccaggt cacccggctt ggccagcaga 7140

acacagagta gattttggtc ccgtttgttc cccagtgggg tatctatcct tgtgcagggc 7200

acaagcctac atggtggctc tggtcatatc attagaaaat agacagaaat gggctgcaca 7260

ccagaatgaa tgaattgaat tgaaagggag gagtgatggt ggaaaaaaaa acaagtcaat 7320

tcatttagac tggtagaacc agaaccactg tgtagtacat ccaaacggtt aaaattccct 7380

ggaagatgtt acataatcct atcatggtgt ttatttatgg aaatctattt taaaaatttt 7440

atgtaatact gcacagtctg tttgcatgat gccttgtacg tagtagcaac tcagtaaata 7500

ctttttgaat gaactagtat agtattttaa ttagctagtc ttcatgtact ggtacaaaag 7560

aacagtgtca tcttacagct gaagtcatag aggggaaata tcccactcaa gatcatataa 7620

cattccaggt actcaggatg aatggtttga ggactggtct gaattcttca aaggtttcag 7680

ctgtattaac attctccatc taataaactt tatcttgtca ttgca 7725

<210> 42
<211> 4033
<212> DNA
<213> Homo sapiens

<400> 42
cggcggccgc ggggcccggc gggcgcgacg ctgcctcctc accggcgcag gctaggaggg 60

ggcggcctga gtgccgtagc cgagccgggg ctggagcgcg cggtctgacc tacgagaaac 120

atggcaacca gcgccgtccc cagtgacaac ctccccacat acaagctggt ggtggtgggg 180

gatgggggtg tgggcaaaag tgccctcacc atccagtttt tccagaagat ctttgtgcct 240

gactatgacc ccaccattga agactcctac ctgaaacata cggagattga caatcaatgg 300

gccatcttgg acgttctgga cacagctggg caggaggaat tcagcgccat gcgggagcaa 360

tacatgcgca cgggggatgg cttcctcatc gtctactccg tcactgacaa ggccagcttt 420

gagcacgtgg accgcttcca ccagcttatc ctgcgcgtca aagacaggga gtcattcccg 480

atgatcctcg tggccaacaa ggtcgatttg atgcacttga ggaagatcac cagggagcaa 540

ggaaaagaaa tggcgaccaa acacaatatt ccgtacatag aaaccagtgc caaggaccca 600

cctctcaatg tcgacaaagc cttccatgac ctcgttagag taattaggca acagattccg 660

gaaaaaagcc agaagaagaa gaagaaaacc aaatggcggg gagaccgggc cacaggcacc 720

cacaaactgc aatgtgtgat cttgtgacag gcctgaggcc ctgggcacag tgacggtggc 780

ctggccagcc ctcgggaccc ctccccacct aactgcactg aaaccatttc taaccacaac 840

ccttggccca aggacttggt acaggaaggg agaagggcag gtgggcaggg agcagacagg 900

gtctggcttt gcccagaggg cacgggcttt cccacctctc aaagagacaa ggaagccacc 960

tgtaagcaga agcagcatcc aagtgcccct ggccccccca tgtgttgatt caacccggtt 1020

cctccccctc tctcggtggg tgtgttgttt attgtaacta catagtgttg gtttgatgtg 1080

gaagtgttta tccacataca aagtacaaaa caagccatga acaagcttct ttcccttacc 1140

ccccatccac aatgtctgag cttggatgtc ttttatagat ttttaaatta ttttagtgat 1200

tattatttta ttaaaggggt ctgggctcac tgcctggtga agtttcaagt gttcagcaga 1260

cctctctggt aacatatctg gaatattgtt gttgtttttt aaccgagttt tcccatcagt 1320

gccaaaactc aactcaatct gaaagtagag tgtctgagag gacagaaggt aatgggaact 1380

gtagctggag gcctcaggcc atgggtcaaa cctgggaggg aaagagaccc tacacatggc 1440

ctagaaatga gagaagagag aggtatttac ccagaggatt ttcctatggt tggggatgca 1500

aatattagaa aacagattgt attttgctga ggggagtggc tgtcatgagc atgtcagttc 1560

taaaaggggt tttcattatc ctggaaatgt ataaactaaa gtaagctgat tggctttgca 1620

aacatgttca tttgtttttc agacagtatg ggttaagttc tctgccctcc ccaggggtct 1680

gaggaggctc tgggtttctc agatctgtct cttgctgcgt tttcacatca gctgtgctgc 1740

ttggtgcctc tctgatacga atacactgac acgtcaaagt aacctaatgt ggacaccatc 1800

cagaaaactc cagttcatgc tggatcttaa ccaaaaatga ttcaatactg ttatcactaa 1860

aacagcacca agacctgaag ccatcttccc ttggagtcaa ctgactacca cctctataag 1920

cctagtcaat gagcagaccc cttccagtat ttgtaaaagt agtactaggt tgcctttttg 1980

gcaattttta ttgacctgtt gaatcttgac tataaaatga tctgagaagt aaggaaggct 2040

gggctgatgt gtggctctca tataccttct gcaagggggc agtctcccca gctccctgat 2100

gatgctcacc cccgcccccc cacctcaggt gctgctggtg tgagccaaag actggagttt 2160

ttccagctgg ggtgggagtg gagagacaac aggaacaacg ctgcaccaaa gaaaaggtca 2220

gaataaaagg cagcacagct ggtgacctta ttttctagat gttacaaatc aggtcactat 2280

gcaaactaga atatcctcag caggtggcct ggccactctg gagaaagaaa cccaaggaaa 2340

gtgagcaccc aactggatgc caagacaccc gggttctgaa aatgtgctgt gttcctacct 2400

cggcaagatc accagcactg aggggcccag ctggagaatg attctgctac aaaaggagac 2460

agttgagact tttgcttgtt ggaaatcaaa cttcttattt gtctaaattg cccctttttc 2520

tgttcctaaa aggaaggata agagagaaca ttccaggtga ggcacttcaa agtttcctta 2580

gaccctatag tgttaagagg tattttaaac actaaaagga caaagctctt cccaatcctt 2640

atgcttccct aagtggtatc tgcagcagtt tgttgtgtgc agtttgatgg cagctgcaaa 2700

ctggaggtga ggcggaggaa aggcaggtag gaaggagtaa ggatggagat gctcagaatc 2760

aagagcatgg cggagtagga gaagaagccc tgcacacagg gcagtgtcca cagccagaaa 2820

actcctgctg ggcaccaacc actacgagca taccccatgc ccaccgtgga gctgcaactc 2880

ctcgacagca ctgagtttga tagtctcact ggaagcagat cagctgatgt agaacagaga 2940

cctcggccat aaaggtgaga agacataggg atttcaacca cacagttggg acagaaggga 3000

cagtgcatct gttcatccat cctgcacttg gcccacgttg aactccatgg tgcctgagag 3060

agactagtta agggttggtc ttctgtatcc tctgctgttg agcctctggt aagctttcat 3120

ctcccatgaa ctcatttccc cataaatgaa atgggtaaat aatgccccat ttgtagaagt 3180

gggccctcat gactgaggta gcttccagat aggccagagt agagtgtaga gtgtgccccg 3240

tgacatccct ccatcttctc ctccattatc atctagcagg gtcagactgg gaaacctggt 3300

tggccacgcc acaccatgac cgaggagcca actgggactt ctggctgttt gacatcctca 3360

tgttcccgtt ggtcttccgg agaatagtgc taccctcaca tcccctggag cacagccttc 3420

ctgaaatgcc ctcaccccat gcctttgcca ttgtgtgctc tcagatttct tccactgttt 3480

gacaccctcc ttagagggct gctctttttt ttccagagat aatcctagcc atcctctcca 3540

ctcccacggc tggggacaat ggccacttac tacctgtgca ctttgccact cgggacacct 3600

ggatggtttc tcttaggact ttgcccacct ccttctcatg gcacttgctg tggaaaatgc 3660

ctggctggcc tcgtggggcc tgtctcactt ttccaggaga catgacccac taacgtggca 3720

actttaaccc aaaggcccct cagacatgtt acagcaaatc tggagccaca gacaggttcc 3780

ctccattggc agcccattgt gtttgaaatt ccatgtcggg tttacttgga atgaaagata 3840

cttgaattat tgtgcgcctg tgagcgccca gcttctgttt catagtctta acaggtggcc 3900

attgtcgtga aacgagtgat gcctgaagat ctcagtgatg tttgaacctt ctgtgtaact 3960

ttttattaag tctttgtatc tctcgactga ttaataaaga agagaaacac gtaaaaaaaa 4020

aaaaaaaaaa aaa 4033

<210> 43
<211> 2960
<212> DNA
<213> Homo sapiens

<400> 43
aaatttagat tttgcaaacc tgtgcattga tgagagtgct attgaaacac attaagaaag 60

attttcaacg caggaatgtg tcatttcctt tcttcatgta ccagatgctg aaatactatg 120

agataaagat tttaggtttc aattgtaaag agagagaagt ggataaatca gtgctgcttt 180

ctttaggacg aaagaagtat ggagcagtgg gatcactttc acaatcaaca ggaggacact 240

gatagctgct ccgaatctgt gaaatttgat gctcgctcaa tgacagcttt gcttcctccg 300

aatcctaaaa acagcccttc ccttcaagag aaactgaagt ccttcaaagc tgcactgatt 360

gccctttacc tcctcgtgtt tgcagttctc atccctctca ttggaatagt ggcagctcaa 420

ctcctgaagt gggaaacgaa gaattgctca gttagttcaa ctaatgcaaa tgatataact 480

caaagtctca cgggaaaagg aaatgacagc gaagaggaaa tgagatttca agaagtcttt 540

atggaacaca tgagcaacat ggagaagaga atccagcata ttttagacat ggaagccaac 600

ctcatggaca cagagcattt ccaaaatttc agcatgacaa ctgatcaaag atttaatgac 660

attcttctgc agctaagtac cttgttttcc tcagtccagg gacatgggaa tgcaatagat 720

gaaatctcca agtccttaat aagtttgaat accacattgc ttgatttgca gctcaacata 780

gaaaatctga atggcaaaat ccaagagaat accttcaaac aacaagagga aatcagtaaa 840

ttagaggagc gtgtttacaa tgtatcagca gaaattatgg ctatgaaaga agaacaagtg 900

catttggaac aggaaataaa aggagaagtg aaagtactga ataacatcac taatgatctc 960

agactgaaag attgggaaca ttctcagacc ttgagaaata tcactttaat tcaaggtcct 1020

cctggacccc cgggtgaaaa aggagatcga ggtcccactg gagaaagtgg tccacgagga 1080

tttccaggtc caataggtcc tccgggtctt aaaggtgatc ggggagcaat tggctttcct 1140

ggaagtcgag gactcccagg atatgccgga aggccaggaa attctggacc aaaaggccag 1200

aaaggggaaa aggggagtgg aaacacatta agaccagtac aactcactga tcatattagg 1260

gcagggccct cttaagatca ggtgggttgg gcgggacatc ctctgctacc atctcattaa 1320

aaggcccttc acctctggac aagtcatctg cacaactgac ttccaagatc cttttgtgac 1380

tcctccaaat gactttggtt cccgtgttgt acctgacttc cacatggcct tctctcctgg 1440

tccctggtgc tgtttgggcc tctgctccca tgctcatacc tcttcttact ccaattactc 1500

caccatcacc tctctcccct atcaccccca gcctggacac ctctcatgca cggactggag 1560

ggctgctcca accagtcctc agttctctgc cacccattga cctagagtct tgaacccaat 1620

ttaatttatt gggttctagg agaactgctg tgttctcacc ctaacttgga agagtgatgt 1680

ttcagtcaag caaagcgatt cctaccatac aatataacac ttgtgtgagg ctctgtccta 1740

aatatctcaa ttaccaatat gtggtttggt agtatttctc gccatgcttt gctcatgcgc 1800

aatgagacta caactagggt gtaaatttta agtatcccat ctaaaactca tacaatgata 1860

ggaaaaatcc atttgttttt catttgattt ttactgagga atcagctcaa tcttcaatga 1920

atactggtct ctttccaaag catttttgat caaagtaaag actgagtcaa gggctttttt 1980

tttttctttt tcttgtttta agagacagag ccttgttcta ttgcacaggc tggactacac 2040

gcattcacct agagtctaga acacaattta atttattggg ttctaggaga actgtcatga 2100

gtattgataa tatgagagtt ctttatattc aaacattatt ctcaaccaga gatagggatg 2160

tcatagaaga aaatccattc attcaatcat taattcacat gtccattatg tacctccatg 2220

agctggacat aacagctaat aagagataat tgtctctggt tttacagagc taattgtccc 2280

taagagatgt agacaaatga acaagcaatt acaatacatc taagctatac tgggggagga 2340

acagggctgg ataggtatgc agaggagata aaaaaatttt aattccttag aatatttttt 2400

aaaaattgat tcttatttta ccttctcatc ttcttatttt ccaaattaca gcatatatat 2460

atatatatat atatatatat atatatatat atatatatat attttttttt tttttttttt 2520

ttttttttta agttttgaag tgtagtcgag cttgggcaat ttatccaacc catttaaacc 2580

aaaaataaaa cttttcatgt attacctggt catttcaaac aaaaatattt tgatcatgaa 2640

aaagaatacc aatattcttt tgttctaaaa atctcttatg ggattacatg ttatattttt 2700

ggtttctctc tactgatcaa cagactacat tttcacaact cttctttcct ttacgtttta 2760

acacacagac ccaagattca tactattaag attctagtag aactctagat ggtatgcctc 2820

tgtgtatctc agcattttta ttcccactct tgtataatga acatgttaac acctacctca 2880

cagggttgtt gtgaggatca agtaagatat tgtgtgtgtg aagatgctct gtgaaatcat 2940

aaagtccttt aaagatgtaa                                                2960

<210>  44
<211>  2088
<212>  DNA
<213>  Homo sapiens

<400>  44
gagctacttg aagaccaatt agagtccggg aagcgcggcg gggcctccag accggggcgg      60

gcttaagggt gacatctgcg ctttaaaggg tccgggtcag ctgactcccg actctgtgga     120

gtctagctgc cagggtcgcg gcagctgcgg ggagagatga ctggggagcg acccagcacg     180

gcgctcccgg acagacgctg ggggccgcgg attctgggct tctggggagg ctgtagggtt     240

tgggtgtttg ccgcgatctt cctgctgctg tctctggcag cctcctggtc caaggctgag     300

aacgacttcg gtctggtgca gccgctggtg accatggagc aactgctgtg ggtgagcggg     360

agacagatcg gctcagtgga caccttccgc atcccgctca tcacagccac tccgcggggc     420

actcttctcg cctttgctga ggcgaggaaa atgtcctcat ccgatgaggg ggccaagttc     480

atcgccctgc ggaggtccat ggaccagggc agcacatggt ctcctacagc gttcattgtc     540

aatgatgggg atgtccccga tgggctgaac cttggggcag tagtgagcga tgttgagaca     600

ggagtagtat ttcttttcta ctccctttgt gctcacaagg ccggctgcca ggtggcctct     660

accatgttgg tatggagcaa ggatgatggt gtttcctgga gcacaccccg gaatctctcc     720

ctggatattg gcactgaagt gtttgcccct ggaccgggct ctggtattca gaaacagcgg     780

gagccacgga agggccgcct catcgtgtgt ggccatggga cgctggagcg ggacggagtc     840

ttctgtctcc tcagcgatga tcatggtgcc tcctggcgct acggaagtgg ggtcagcggc     900

atcccctacg gtcagcccaa gcaggaaaat gatttcaatc ctgatgaatg ccagccctat     960

gagctcccag atggctcagt cgtcatcaat gcccgaaacc agaacaacta ccactgccac    1020

tgccgaattg tcctccgcag ctatgatgcc tgtgatacac taaggccccg tgatgtgacc    1080

ttcgaccctg agctcgtgga ccctgtggta gctgcaggag ctgtagtcac cagctccggc    1140

attgtcttct tctccaaccc agcacatcca gagttccgag tgaacctgac cctgcgatgg    1200

agcttcagca atggtacctc atggcggaaa gagacagtcc agctatggcc aggccccagt    1260

ggctattcat ccctggcaac cctggagggc agcatggatg gagaggagca ggccccccag    1320

ctctacgtcc tgtatgagaa aggccggaac cactacacag agagcatctc cgtggccaaa    1380

atcagtgtct atgggacact ctgagctgtg ccactgccac aggggtattc tgccttcagg    1440

actctgcctt caggaacacg ggtctgtaga gggtctgctg gagacgcctg aaagacagtt    1500

ccatcttcct ttagactcca gccttggcaa aatcaccttc cctttaccag ggaaatcact    1560

tcctttagga ctgaaagcta ggcgtcctct cccacaaaaa agtcctgccc tcatctgaga    1620

atactgtctt tccatatggc taagtgtggc cccaccaccc tctctgccct cccgggacat 1680

tgattggtcc tgtcttgggc aggtctagtg agctgtagaa ttgaatcaat gtgaactcag 1740

ggaactgggg aaggctgagc ctcctctttg gtgttgcggt aagataaccg acagggctgg 1800

tgaaagtccc cagatggcag gatatttggt ttcagagtaa ggactaggtg caccaccatg 1860

actgactatc aatcaaaatg tttgtaactt aaaattttta atgaaggata atgaatattt 1920

gtagagtctc tatggttctg tcaatgcaca tcttcgtgtc tgttttcctc atgtatcctt 1980

gtgagcctgg gtgagttctg gggagagacc tgatgtgcgt actgcctgtg aaaatctgac 2040

tttggcaaat caaatcctct tttcctttg aaaaaaaaa aaaaaaa 2088

<210> 45
<211> 4827
<212> DNA
<213> Homo sapiens

<400> 45
gaagggcaac acggggacct tgaagcgggg tcgcggcggc gccccagccc gggccaggga 60

gtcccggcag cggcacctcc cagaaagggc ggagccgacg acgccttctt ccttcctgac 120

cggcgcgcgc agcctgctgc cgcggtcagc gcctgctcct gctcctccgc tcctcctgcg 180

cggggtgctg aaacagcccg gggaagtaga gccgcctccg gggagcccaa ccagccgaac 240

gccgccggcg tcagcagcct tgcgcggcca cagcatgacc gctcgcggcc tggcccttgg 300

cctcctcctg ctgctactgt gtccagcgca ggtgttttca cagtcctgtg tttggtatgg 360

agagtgtgga attgcatatg gggacaagag gtacaattgc gaatattctg gcccaccaaa 420

accattgcca aaggatggat atgacttagt gcaggaactc tgtccaggat tcttctttgg 480

caatgtcagt ctctgttgtg atgttcggca gcttcagaca ctaaaagaca acctgcagct 540

gcctctacag tttctgtcca gatgtccatc ctgtttttat aacctactga acctgttttg 600

tgagctgaca tgtagccctc gacagagtca gtttttgaat gttacagcta ctgaagatta 660

tgttgatcct gttacaaacc agacgaaaac aaatgtgaaa gagttacaat actacgtcgg 720

acagagtttt gccaatgcaa tgtacaatgc ctgccgggat gtggaggccc cctcaagtaa 780

tgacaaggcc ctgggactcc tgtgtgggaa ggacgctgac gcctgtaatg ccaccaactg 840

gattgaatac atgttcaata aggacaatgg acaggcacct tttaccatca ctcctgtgtt 900

ttcagatttt ccagtccatg ggatggagcc catgaacaat gccaccaaag gctgtgacga 960

gtctgtggat gaggtcacag caccatgtag ctgccaagac tgctctattg tctgtggccc 1020

caagccccag cccccacctc ctcctgctcc ctggacgatc cttggcttgg acgccatgta 1080

tgtcatcatg tggatcacct acatggcgtt tttgcttgtg ttttttggag cattttttgc 1140

agtgtggtgc tacagaaaac ggtattttgt ctccgagtac actcccatcg atagcaatat 1200

agctttttct gttaatgcaa gtgacaaagg agaggcgtcc tgctgtgacc ctgtcagcgc 1260

agcatttgag ggctgcttga ggcggctgtt cacacgctgg gggtctttct gcgtccgaaa 1320

ccctggctgt gtcattttct tctcgctggt cttcattact gcgtgttcgt caggcctggt 1380

gtttgtccgg gtcacaacca atccagttga cctctggtca gcccccagca gccaggctcg 1440

cctggaaaaa gagtactttg accagcactt tgggcctttc ttccggacgg agcagctcat 1500

catccgggcc cctctcactg acaaacacat ttaccagcca tacccttcgg gagctgatgt 1560

accctttgga cctccgcttg acatacagat actgcaccag gttcttgact tacaaatagc 1620

catcgaaaac attactgcct cttatgacaa tgagactgtg acacttcaag acatctgctt 1680

ggcccctctt tcaccgtata acacgaactg caccattttg agtgtgttaa attacttcca 1740

gaacagccat tccgtgctgg accacaagaa aggggacgac ttctttgtgt atgccgatta 1800

ccacacgcac tttctgtact gcgtacgggc tcctgcctct ctgaatgata caagtttgct 1860

ccatgaccct tgtctgggta cgtttggtgg accagtgttc ccgtggcttg tgttgggagg 1920

ctatgatgat caaaactaca ataacgccac tgcccttgtg attaccttcc ctgtcaataa 1980

ttactataat gatacagaga agctccagag ggcccaggcc tgggaaaaag agtttattaa 2040

ttttgtgaaa aactacaaga atcccaatct gaccatttcc ttcactgctg aacgaagtat 2100

tgaagatgaa ctaaatcgtg aaagtgacag tgatgtcttc accgttgtaa ttagctatgc 2160

catcatgttt ctatatattt ccctagcctt ggggcacatg aaaagctgtc gcaggcttct 2220

ggtggattcg aaggtctcac taggcatcgc gggcatcttg atcgtgctga gctcggtggc 2280

ttgctccttg ggtgtcttca gctacattgg gttgcccttg accctcattg tgattgaagt 2340

catcccgttc ctggtgctgg ctgttggagt ggacaacatc ttcattctgg tgcaggccta 2400

ccagagagat gaacgtcttc aaggggaaac cctggatcag cagctgggca gggtcctagg 2460

agaagtggct cccagtatgt tcctgtcatc cttttctgag actgtagcat ttttcttagg 2520

agcattgtcc gtgatgccag ccgtgcacac cttctctctc tttgcgggat tggcagtctt 2580

cattgacttt cttctgcaga ttacctgttt cgtgagtctc ttggggttag acattaaacg 2640

tcaagagaaa aatcggctag acatcttttg ctgtgtcaga ggtgctgaag atggaacaag 2700

cgtccaggcc tcagagagct gtttgtttcg cttcttcaaa aactcctatt ctccacttct 2760

gctaaaggac tggatgagac caattgtgat agcaatattt gtgggtgttc tgtcattcag 2820

catcgcagtc ctgaacaaag tagatattgg attggatcag tctctttcga tgccagatga 2880

ctcctacatg gtggattatt tcaaatccat cagtcagtac ctgcatgcgg gtccgcctgt 2940

gtactttgtc ctggaggaag ggcacgacta cacttcttcc aaggggcaga acatggtgtg 3000

cggcggcatg ggctgcaaca atgattccct ggtgcagcag atatttaacg cggcgcagct 3060

ggacaactat acccgaatag gcttcgcccc ctcgtcctgg atcgacgatt atttcgactg 3120

ggtgaagcca cagtcgtctt gctgtcgagt ggacaatatc actgaccagt tctgcaatgc 3180

ttcagtggtt gaccctgcct gcgttcgctg caggcctctg actccggaag gcaaacagag 3240

gcctcagggg ggagacttca tgagattcct gcccatgttc ctttcggata accctaaccc 3300

caagtgtggc aaagggggac atgctgccta tagttctgca gttaacatcc tccttggcca 3360

tggcaccagg gtcggagcca cgtacttcat gacctaccac accgtgctgc agacctctgc 3420

tgactttatt gacgctctga agaaagcccg acttatagcc agtaatgtca ccgaaaccat 3480

gggcattaac ggcagtgcct accgagtatt tccttacagt gtgttttatg tcttctacga 3540

acagtacctg accatcattg acgacactat cttcaacctc ggtgtgtccc tgggcgcgat 3600

atttctggtg accatggtcc tcctgggctg tgagctctgg tctgcagtca tcatgtgtgc 3660

caccatcgcc atggtcttgg tcaacatgtt tggagttatg tggctctggg gcatcagtct 3720

gaacgctgta tccttggtca acctggtgat gagctgtggc atctccgtgg agttctgcag 3780

ccacataacc agagcgttca cggtgagcat gaaaggcagc cgcgtggagc gcgcggaaga 3840

ggcacttgcc cacatgggca gctccgtgtt cagtggaatc acacttacaa aatttggagg 3900

gattgtggtg ttggcttttg ccaaatctca aattttccag atattctact tcaggatgta 3960

tttggccatg gtcttactgg gagccactca cggattaata tttctccctg tcttactcag 4020

ttacataggg ccatcagtaa ataaagccaa aagttgtgcc actgaagagc gatacaaagg 4080

aacagagcgc gaacggcttc taaatttcta gccctctcgc agggcatcct gactgaactg 4140

tgtctaaggg tcggtcggtt taccactgga cgggtgctgc atcggcaagg ccaagttgaa 4200

caccggatgg tgccaaccat cggttgtttg gcagcagctt tgaacgtagc gcctgtgaac 4260

tcaggaatgc acagttgact tgggaagcag tattactaga tctggaggca accacaggac 4320

actaaacttc tcccagcctc ttcaggaaag aaacctcatt ctttggcaag caggaggtga 4380

cactagatgg ctgtgaatgt gatccgctca ctgacactct gtaaaggcca atcaatgcac 4440

tgtctgtctc tccttttagg agtaagccat cccacaagtt ctataccata tttttagtga 4500

cagttgaggt tgtagataca ctttataaca ttttatagtt taaagagctt tattaatgca 4560

ataaattaac tttgtacaca tttttatata aaaaaacagc aagtgatttc agaatgttgt 4620

aggcctcatt agagcttggt ctccaaaaat ctgtttgaaa aaagcaacat gttcttcaca 4680

gtgttcccct agaaaggaag agatttaatt gccagttaga tgtggcatga aatgagggac 4740

aaagaaagca tctcgtaggt gtgtctactg ggttttaact tatttttctt taataaaata 4800

cattgttttc ctaaaaaaaa aaaaaaa 4827

<210> 46
<211> 2393

<212>  DNA
<213>  Homo sapiens

<400>  46
attcttctat tagataacag tagctattta aatacttctg cagaagctca catattttta      60

gtttgttgaa gttcgtgact gcttcactct ctcattctta gcttgaattt ggaaatgact     120

tttgatgacc taaagatcca gactgtgaag gaccagcctg atgagaagtc aaatggaaaa     180

aaagctaaag gtcttcagtt tctttactct ccatggtggt gcctggctgc tgcgactcta     240

ggggtccttt gcctgggatt agtagtgacc attatggtgc tgggcatgca attatcccag     300

gtgtctgacc tcctaacaca agagcaagca aacctaactc accagaaaaa gaaactggag     360

ggacagatct cagcccggca acaagcagaa gaagcttcac aggagtcaga aaacgaactc     420

aaggaaatga tagaaaccct tgctcggaag ctgaatgaga aatccaaaga gcaaatggaa     480

cttcaccacc agaatctgaa tctccaagaa acactgaaga gagtagcaaa ttgttcagga     540

cttcatccag caagcaattt cctattccag ttttccattc tggatggggc tgtctcggag     600

gaaccccagc tacccatggc tctgggagga cggttctcct ttgatgcccc acttatttag     660

agtccgaggc gctgtctccc agacataccc ttcaggtacc tgtgcatata tacaacgagg     720

agctgtttat gcggaaaact gcattttagc tgccttcagt atatgtcaga agaaggcaaa     780

cctaagagca cagtgaattt gaaggctctg gaagaaaaga aaaaagtctt tgagttttat     840

tctggaattt aagctattct ttgtcacttg ggtgccaaac atgagagccc agaaaactgt     900

catttagctg gctgcagaac tcctttgcag aaactggggt tccaggtgcc tggcaccttt     960

atgtcaacat ttttgattct agctacctgt attatttcac ctagcttgtc ccaagcttcc    1020

ctgccagcct gaagtccatt ttcccctttt tattttaaaa tttgactcct cttcaagctt    1080

gaaaaccctc tgaactcagt cttctttacc tcattatcac cttcccctca cactcctaaa    1140

attgcatgaa agacagaaca tggagaactt gctcaagtgc aggcagagag caaaaagggg    1200

aaatatgtct gggaaaaagt gcacgtgaag aaacaaagaa ggacagaggc cattccgaaa    1260

tcaagaaact catgttctta actttaaaaa aggtatcaat ccttggtttt taaactgtgg    1320

tccatctcca gactctacca cttacggaca gacagacaga cagacacaca cacacacaca    1380

cacacacatt ttgggacaag tggggagccc aagaaagtaa ttagtaagtg agtggtcttt    1440

tctgtaagct aatccacaac ctgttaccac ttcctgaatc agttattatt tcttcatttt    1500

tttttctacc agaggacaga ttaatagatt taacccttca caacagttct tgttagaatc    1560

atgggatgtg tggcccagag gtaagaatag aatttctttc cctaaagaac ataccttttg    1620

tagatgaact cttctcaact ctgttttgct atgctataat tccgaaacat acaagacaaa    1680

aaaaatgaag acactcaatc tagaacaaac taagccaggt atgcaaatat cgctgaatag    1740

aaacagatgg aattagaaat atatcttcta tttttaggct tctatttcct ttccacccac    1800

tcttcacagg ctattctact ttaaaggaag cctttttatt ttgctgcaca caatctagca 1860

ggaatctttt ttttttttta agagctgtgt catccttatg taggcaagag atgtttgctt 1920

ttgttaaaag ctttattgag atataattaa cataaaataa actgaacata tttaaagtgt 1980

actatttgat aagttttcac accttgtgga gaacatgcat actacaatta agagagtgaa 2040

catatccatc atccctcaaa gtgtcacaat gctcctcctg atgactcctc cccagaaaac 2100

caccaatcgg ctttcatttt gcattttgta gttttatgtg aatggaatca tatagtatgt 2160

cttttttttt tgtctggctt ctttcacttt gcataattat tttgagattc atatgtctcc 2220

atcttgatgc tcgtatgaat tcattctttt aaatgttgaa tattcccttg tatggatata 2280

ccacaattca tttacccatt tacttgttga tgacatttgg gttgttttag ttttgggata 2340

ttacaaataa agctgctgtg aacatttgtg tacaagaaaa aaaaaaaaaa aaa 2393

<210> 47
<211> 1945
<212> DNA
<213> Homo sapiens

<400> 47
ataaatttga gtcagcacca gcgacagctc tgcagtcctc ctatgtggta ctgatcaggt 60

ggttgcagag cttcagctca cagcaacaca atgcagctga gcaggcaagc acagcccaca 120

gccagaaaca gttccgactc tacagaacaa gacgaccttt aagtttccca gagaaaatga 180

gatgctgatg ttgaagacga caccacggct ttgatggaat atcagatatt gaaaatgtct 240

ctctgcctgt tcatccttct gtttctcaca cctggtattt tatgcatttg tcctctccaa 300

tgtatatgca cagagaggca caggcatgtg gactgttcag gcagaaactt gtctacatta 360

ccatctggac tgcaagagaa tattatacat ttaaacctgt cttataacca ctttactgat 420

ctgcataacc agttaaccca atataccaat ctgaggaccc tggacatttc aaacaacagg 480

cttgaaagcc tgcctgctca cttacctcgg tctctgtgga acatgtctgc tgctaacaac 540

aacattaaac ttcttgacaa atctgatact gcttatcagt ggaatcttaa atatctggat 600

gtttctaaga acatgctgga aaaggttgtc ctcattaaaa atacactaag aagtctcgag 660

gttctcaacc tcagtagtaa caaactttgg acagttccaa ccaacatgcc ctccaaacta 720

catatcgtgg acctgtctaa taattctttg acacaaattc ttccaggtac attaataaac 780

ctgacaaatc tcacacatct ttacctgcac aacaataagt tcacattcat tccagaccaa 840

tcttttgacc aactctttca gttgcaagag ataacccttt acaataacag gtggtcatgt 900

gaccacaaac aaaacattac ttacttactg aagtggatga tggaaacaaa agcccatgtg 960

atagggactc catgttctac ccaaatatca tctttaaagg aacataacat gtatcccaca 1020

ccttctggat ttacctcaag cttattcact gtaagtggga tgcagacagt ggacaccatt 1080

aactctctga gtgtggtaac tcaacccaaa gtgaccaaaa tacccaaaca atatcgaaca 1140

aaggaaacaa cgtttggtgc cactctaagc aaagacacca cctttactag cactgataag 1200

gcttttgtgc cctatccaga agatacatcc acagagacta tcaattcaca tgaagcagca 1260

gctgcaactc taactattca tctccaagat ggaatggtca caaacacaag cctcactagc 1320

tcaacaaaat catccccaac acccatgacc ctaagtatca ctagtggcat gccaaataat 1380

ttctctgaaa tgcctcaaca aagcacaacc cttaacttat ggagggaaga gacaaccaca 1440

aatgtaaaga ctccattacc ttctgtggca aatgcttgga aagtaaatgc ttcatttctc 1500

ttattgctca atgttgtggt catgctggct gtctgagggt ctgcattttc tgaaactaat 1560

gaaagcactc ctccctgatg tacagttggg aaaatatgtc catatctaac cagtgattcg 1620

agctatattt aagtattcaa gaaagccagt cttaacattt ctaactctga tgtaaatgaa 1680

gtaacttgtc ttaaataaaa gaaatgcaca atgtcttggt acttgctgct attttactgt 1740

cttaattaag taaactaatg agtttctttt ataaaaaaaa tgaaatgttt taaggcttca 1800

atttattgca caaaatataa agcatctaaa ctttaatatg tattttatgt atgtttacac 1860

tgtcaaacat ctggaaaata aaaggtctat gctcaaaaaa aaaaaaaaaa aaaaaaaaaa 1920

aaaaaaaaaa aaaaaaaaaa aaaaa 1945

<210> 48
<211> 2091
<212> DNA
<213> Homo sapiens

<400> 48
aagtgctggg atgacaggtg tgagccaccg cccccggccc ctcgcccgcc ttttgaagga 60

gcctttcgtc ctcaagggcg aggccactcc ccccccgcga gttccatgcc ccctagaggg 120

tcatcgttcc cgacggggag gtggcgccct cccccgggcc ccgggccccg accgcccgtg 180

ctgcctcctt ccgggccctc ctccgcgatg acggcgccgc cagcaggcca ggcggactgg 240

gcggggctcc gagcggggac tgggacccag accgactagg ggactgggag cgggcggcgc 300

ggccatggcg ggctgctgcg ccgcgctggc ggccttcctg ttcgagtacg acacgccgcg 360

catcgtgctc atccgcagcc gcaaagtggg gctcatgaac cgcgccgtgc aactgctcat 420

cctggcctac gtcatcgggt gctaccatcc ccatttggca gaagtggaaa tggagtcccc 480

tagaaggtgg gtgtttgtgt gggaaaaggg ctaccaggaa actgactccg tggtcagctc 540

cgttacgacc aaggtcaagg gcgtggctgt gaccaacact tctaaacttg gattccggat 600

ctgggatgtg gcggattatg tgataccagc tcaggaggaa aactccctct tcgtcatgac 660

caacgtgatc ctcaccatga accagacaca gggcctgtgc cccgagattc cagatgcgac 720

cactgtgtgt aaatcagatg ccagctgtac tgccggctct gccggcaccc acagcaacgg 780

agtctcaaca ggcaggtgcg tagctttcaa cgggtctgtc aagacgtgtg aggtggcggc 840

ctggtgcccg gtggaggatg acacacacgt gccacaacct gcttttttaa aggctgcaga 900

aaacttcact cttttggtta agaacaacat ctggtatccc aaatttaatt tcagcaagag 960

gaatatcctt cccaacatca ccactactta cctcaagtcg tgcatttatg atgctaaaac 1020

agatcccttc tgccccatat tccgtcttgg caaaatagtg gagaacgcag gacacagttt 1080

ccaggacatg gccgtggagg gaggcatcat gggcatccag gtcaactggg actgcaacct 1140

ggacagagcc gcctccctct gcttgcccag gtactccttc cgccgcctcg atacacggga 1200

cgttgagcac aacgtatctc ctggctacaa tttcaggttt gccaagtact acagagacct 1260

ggctggcaac gagcagcgca cgctcatcaa ggcctatggc atccgcttcg acatcattgt 1320

gtttgggaag gcagggaaat ttgacatcat ccccactatg atcaacatcg gctctggcct 1380

ggcactgcta ggcatggcga ccgtgctgtg tgacatcata gtcctctact gcatgaagaa 1440

aagactctac tatcgggaga agaaatataa atatgtggaa gattacgagc agggtcttgc 1500

tagtgagctg gaccagtgag gcctacccca cacctgggct ctccacagcc ccatcaaaga 1560

acagagagga ggaggaggga gaaatggcca ccacatcacc ccagagaaat ttctggaatc 1620

tgattgagtc tccactccac aagcactcag ggttccccag cagctcctgt gtgttgtgtg 1680

caggatctgt ttgcccactc ggcccaggag gtcagcagtc tgttcttggc tgggtcaact 1740

ctgcttttcc cgcaacctgg ggttgtcggg ggagcgctgg cccgacgcag tggcactgct 1800

gtggctttca gggctggagc tggctttgct cagaagcctc ctgtctccag ctctctccag 1860

gacaggccca gtcctctgag gcacggcggc tctgttcaag cactttatgc ggcaggggag 1920

gccgcctggc tgcagtcact agacttgtag caggcctggg ctgcaggctt ccccccgacc 1980

attccctgca gccatgcggc agagctggca tttctcctca gagaagcgct gtgctaaggt 2040

gatcgaggac cagacattaa agcgtgattt tcttaaaaaa aaaaaaaaaa a 2091

<210> 49
<211> 4185
<212> DNA
<213> Homo sapiens

<400> 49
cggccgcgag cgcagtggtg tggagcgcgc cgggtcccgg agccggctgt ctgagggatg 60

gacgagacga gcccactagt gtcccccgag cgggcccaac ccccggacta caccttcccg 120

tcgggctcgg gcgctcactt tccgcaggtg cccgggggcg cggtccgagt ggcggcggcg 180

gccggctcgg gcccctctcc gccaggctcg ccgggccacg accgcgagcg gcagccactg 240

ttggatcggg cccggggcgc ggcggcccag ggccagaccc aaaccgtggc ggcgcaggcc 300

caggctctgg ccgctcaggc cgcggcggca gcccacgccg ctcaggccca ccgcgagcgg 360

aacgagttcc cggaggatcc tgagttcgag gcggtggtgc ggcaggccga gctggccatc 420

gagcgctgca tctttcccga gcgcatctac cagggctcca gcggaagcta cttcgtcaag 480

gaccctcagg ggaggatcat tgctgtcttc aaacccaaga atgaagagcc ctatgggcat 540

cttaatccta agtggaccaa gtggctgcag aagctgtgct gtccttgctg ctttggccgt 600

gactgccttg tccttaacca gggctatctc tcagaagcag gggccagcct ggtggaccaa 660

aaactggaac tcaacattgt tccccgtaca aaggtagtat acctggccag tgagaccttc 720

aactatagtg ccattgaccg agtgaagtcc aggggcaagc ggcttgcact agagaaagtg 780

ccaaaagttg gacagcggtt taaccgcatc gggctaccac caaaggttgg ttcattccag 840

ctctttgttg aaggctacaa agatgcagac tattggctgc ggcgttttga agcagaacct 900

cttcctgaga acactaaccg gcaactactg ctccagtttg agcggttggt ggtgctggat 960

tacatcatcc gcaacactga tcgaggcaat gacaactggc tgattaaata tgactgtcca 1020

atggatagtt ctagctctcg ggacacagac tgggtggtgg tgaaggagcc tgttatcaag 1080

gtggctgcca tagacaatgg gctggccttc ccactgaagc atcctgactc ctggagggca 1140

tatccttttt actgggcctg gttgccccag gcgaaagtcc cattttctca ggagatcaaa 1200

gatctgatcc ttccaaagat atcggaccct aacttcgtca aggacttgga agaggaccta 1260

tatgaactct tcaagaaaga tcctggtttc gacaggggcc agttccataa gcagattgct 1320

gtcatgcggg gccagatctt aaatctgacc caggccttga aagacaacaa gagtcccctg 1380

cacctcgtcc agatgccacc tgtgattgtc gagacggccc gttcccacca gcggtcttct 1440

agcgagtcct acacacagag ctttcagagc cggaagccct tcttttcatg gtggtagctc 1500

cagaggcagg cagaggaaat attgtcagag actggtggga ggaagcctgg ggagtggggt 1560

gcaggaaaag ccagagaagc cggtggagag cagcaccttt aagagccctc tctctctgct 1620

tgccaccctg ctcagagctt tccacccaca gggagaagca caatcaggaa cagtgagtgc 1680

tcctcgccct tctgatgtgg gggaggctgg agctccatgc acgtagtcca gatgcctggg 1740

aaggaacatc tcccttccag catctgctgg tagcaggctg ggacagtccc ttccttccct 1800

gaaaccctgc tctattgcaa ttccctatta tattctgcat cagaaaaaca aacaaaacaa 1860

aaacaacttt aaatgcttgt agcagaaccc cgggtcatct catgtcagaa acctttaatc 1920

caggcctaaa tttgcataga cctgacattc agctgccttg cagttgcttc ctcccatgag 1980

ccaaggtggt gtcagagggc aactggatga ctcgcagtac cacagcactg ggacagacag 2040

aagccacacc tttcttttgg gtttttgcca agcctcctcc atctcccatc agtgctgtgg 2100

gctggctgca agcctcgaaa cagttctcct ggaagggagg tttttgcttt acccccgcca 2160

gcacttccgc acacaatcat agagaacctc tctgctctct gctggcctac agcttgtctg 2220

tttctcaagc agaggcagga agagctagtc ttagcattta tattttaata ggaagttgac 2280

tcccagcatg taaaagtgat ccacgcagcc ggagtgtatg ccgggagcta agtggtctat 2340

gggtgaacat atcccacctt gcttcctgag tccttggtcc caatcttctc atttgttcct 2400

ctcgttttaa attttttccc cccaactctt ttgatgtaag agttcagttt gtcttcggga 2460

gtgggtctct gcaagggctc tgggatgagt cttggcttcc aagaggacag gctattaggt 2520

tcttggactt ttttctgtgc taccgctgct gcttggtgga agtaacagga cgtggattct 2580

gcctcataag tggcagtttc ccttttctct ctgacttgtc ctaggccgat ttctctatgg 2640

cttccctgag aaaggtgagg cccaaaggag agaggccttc aaactgtccc aggtcctgcg 2700

cagctcagtg cgtatcttct tgcttccatg tgtcttttcc cctgctgcct cactccccac 2760

ccccacttgc caggtgtttg agccatttct acaccaaagc aaagtacggc ctcaggaggg 2820

agtaaaaagg gtgccatctg tgtctggagg ggcagctgtg ttcatgccct gtgctactgg 2880

acatttcaca attctggcac cttgcgattg gtcagtcaac ctcagaaagt aactatcttg 2940

aaggtttgaa aaacaaccaa agaaagggag tgaggactat ggctgcatgt cctctgcttg 3000

cccggctgca gagcagagat gtgcagccct ctggtcagct ggtccaggct ggtccccgcc 3060

ggtccccttc cagtccagcc accaagagtc cacttgtccc gggcttccac ctggctgaca 3120

ggaagaattt ctgagagctg gatgtgcatg ccctgtggac gaaggtacag ctcgcctgcc 3180

tgccccaatc ccagccccga caatcacatg cagctgactc ggacactggc cttgggaaca 3240

atgttcgaga gaacacttgc cccttgactg taggagccag aaggggaccc aggtgtgcat 3300

agctctctgt agacattttt acccaaacct gttggtaaag tgcccatctg gtgctcaaga 3360

gagcctgggg gtctaacagg gagcccggct gcctcacctg gccacagcct ccacaccaga 3420

tctccacatt gtcttgatcc agaccagctc tgtgatcaga aggaaattgg gtccagtgta 3480

ggagagagct ggtcctgggc ctggcaggca agagtgtggg catcctttcc tggcctttct 3540

ccactctccc tcaagcctgt gctcaggttg ccttgaatgt ggactctgga agagccaggg 3600

gcccagaatg ccgggggagg cttctgagtg gcactcatgg aacaccgtcc ctctgccagc 3660

cataggccct gcctccagtg tcagggaatg gaggctgggc tgcgagagtg ttgctgcccc 3720

ctgtgtcatt cttctaatcc aatgtagaaa ttgtacgtaa tgtatttaaa tcaacgcaaa 3780

tgtatgaata acaaatacag ttctgacctt ttttgtccag tttctttggg ggaaggaaga 3840

caaagaaggt aggaacggaa ttttgagggc aaagaaacct gtgtttccat ggaattgctg 3900

agacgtggct cctggggcta tttctcccta ataaaggatg atccaggtcc tcatttccaa 3960

agtcccaatg ctctgaaaac caaaagtatt ttcataaccc atttgaaacc aaacctgacc 4020

tgaacttaca ctgataggaa gctatgggta attatgatgt gttcctttta gtgtgattct 4080

ttgttgcaga aatgtcaata tattttatga catggttccc tactagggat tatacagtat 4140

ttgctgacta cttcctaaga gccaaaaata aaaaatctga attcc 4185

<210> 50
<211> 1455
<212> DNA
<213> Homo sapiens

<400> 50
gccgagccag ccccttcacc accagccggc cgcgccccgg gaagggaagt ttgtggcgga 60

ggaggttcgt acgggaggag ggggaggcgc ccacgcatct ggggctgact cgctctttcg 120

caaaacgtct gggaggagtc cctggggcca caaaactgcc tccttcctga ggccagaagg 180

agagaagacg tgcagggacc ccgcgcacag gagctgccct cgcgacatgg gtcacccgcc 240

gctgctgccg ctgctgctgc tgctccacac ctgcgtccca gcctcttggg gcctgcggtg 300

catgcagtgt aagaccaacg gggattgccg tgtggaagag tgcgccctgg gacaggacct 360

ctgcaggacc acgatcgtgc gcttgtggga agaaggagaa gagctggagc tggtggagaa 420

aagctgtacc cactcagaga agaccaacag gaccctgagc tatcggactg gcttgaagat 480

caccagcctt accgaggttg tgtgtgggtt agacttgtgc aaccagggca actctggccg 540

ggctgtcacc tattcccgaa gccgttacct cgaatgcatt tcctgtggct catcagacat 600

gagctgtgag aggggccggc accagagcct gcagtgccgc agccctgaag aacagtgcct 660

ggatgtggtg acccactgga tccaggaagg tgaagaaggg cgtccaaagg atgaccgcca 720

cctccgtggc tgtggctacc ttcccggctg cccgggctcc aatggtttcc acaacaacga 780

caccttccac ttcctgaaat gctgcaacac caccaaatgc aacgagggcc caatcctgga 840

gcttgaaaat ctgccgcaga atggccgcca gtgttacagc tgcaagggga acagcaccca 900

tggatgctcc tctgaagaga ctttcctcat tgactgccga ggccccatga atcaatgtct 960

ggtagccacc ggcactcacg aacgctcact ctggggaagc tggttgccat gtaaaagtac 1020

tactgccctg agaccaccat gctgtgagga agcccaagct actcatgtat aaatgccatg 1080

tggagataga gccccagatg tttcagccat ctcagcccag gcaccagaca agtgggtgaa 1140

gaagccacct tggacatgta gccccagcag atgtgatata gagaagaaac aggaaacttg 1200

gctatattag tttcctaggg ctgcctgtga taaattatta caaactttat aaactaacac 1260

attgtgtgcc tatatcaaaa catcatggaa ggacaggcac agtggctcat gcctgtagtc 1320

ctagcacttt gggagggtga gaaaggaaga tctcttgagc tcaggagttc aagatcagcc 1380

tgggcaacac agtgagacct catctccact aaaaataaaa aaaaattggc tggaaaaaaa 1440

aaaaaaaaaa aaaaa 1455

<210> 51
<211> 1828

<212>  DNA
<213>  Homo sapiens

<400>  51
cagttacagg gagcaccacc agggaacatc tcggggagcc tggttggaag ctgcaggctt      60

agtctgtcgg ctgcgggtct ctgactgccc tgtggggagg gtcttgcctt aacatccctt     120

gcatttggct gcaaagaaat ctgcttggaa gaaggggtta cgctgtttgg ccgggcagaa     180

actccgctga gcagaacttg ccgccagaat gctcctcctg ttgctgagta tcatcgtcct     240

ccacgtcgcg gtgctggtgc tgctgttcgt ctccacgatc gtcagccaat ggatcgtggg     300

caatggacac gcaactgatc tctggcagaa ctgtagcacc tcttcctcag gaaatgtcca     360

ccactgtttc tcatcatcac caaacgaatg gctgcagtct gtccaggcca ccatgatcct     420

gtcgatcatc ttcagcattc tgtctctgtt cctgttcttc tgccaactct tcaccctcac     480

caaggggggc aggttttaca tcactggaat cttccaaatt cttgctggtc tgtgcgtgat     540

gagtgctgcg gccatctaca cggtgaggca cccggagtgg catctcaact cggattactc     600

ctacggtttc gcctacatcc tggcctgggt ggccttcccc ctggcccttc tcagcggtgt     660

catctatgtg atcttgcgga aacgcgaatg aggcgcccag acggtctgtc tgaggctctg     720

agcgtacata gggaagggag gaagggaaaa cagaaagcag acaaagaaaa aagagctagc     780

ccaaaatccc aaactcaaac caaaccaaac agaaagcagt ggaggtgggg gttgctgttg     840

attgaagatg tatataatat ctccggttta taaaacctat ttataacact ttttacatat     900

atgtacatag tattgtttgc tttttatgtt gaccatcagc ctcgtgttga gccttaaaga     960

agtagctaag gaactttaca tcctaacagt ataatccagc tcagtatttt tgttttgttt    1020

tttgtttgtt tgttttgttt tacccagaaa taagataact ccatctcgcc ccttcccttt    1080

catctgaaag aagatacctc cctcccagtc cacctcattt agaaaaccaa agtgtgggta    1140

gaaaccccaa atgtccaaaa gcccttttct ggtgggtgac ccagtgcatc caacagaaac    1200

agccgctgcc cgaacctctg tgtgaagctt tacgcgcaca cggacaaaat gcccaaactg    1260

gagcccttgc aaaaacacgg cttgtggcat tggcatactt gcccttacag gtggagtatc    1320

ttcgtcacac atctaaatga gaaatcagtg acaacaagtc tttgaaatgg tgctatggat    1380

ttaccattcc ttattatcac taatcatcta aacaactcac tggaaatcca attaacaatt    1440

ttacaacata agatagaatg gagacctgaa taattctgtg taatataaat ggtttataac    1500

tgcttttgta cctagctagg ctgctattat tactataatg agtaaatcat aaagccttca    1560

tcactcccac atttttctta cggtcggagc atcagaacaa gcgtctagac tccttgggac    1620

cgtgagttcc tagagcttgg ctgggtctag gctgttctgt gcctccaagg actgtctggc    1680

aatgacttgt attggccacc aactgtagat gtatatatgg tgcccttctg atgctaagac    1740

tccagacctt ttgtttttgc tttgcatttt ctgattttat accaactgtg tggactaaga    1800

tgcattaaaa taaacatcag agtaactc 1828

<210> 52
<211> 3324
<212> DNA
<213> Homo sapiens

<400> 52
   gacctcgtga aataaaagtg cagaaaacaa acccaggcga tcacagcagc agccgccgcg 60
   gcagcagcac caacagcagg aggagcagga ggagccggag gaggaggagg aggaggaggc 120
   aaagttagag ttggggctgg cgctccggag ttgctgggct cagcgcagct cccattcatt 180
   aaggaaccag ctgcggagga aggtggccga gcgcccgcgc tgcccactcg ctcgctcgcg 240
   cactcagacg cgcgccacaa cagcgcgccc caagctgcgc agctctgcaa aagtttctgc 300
   tcgggatctg gctctcttcc ccttggactt tagaacgatt tagggttgac agaggaaagc 360
   agaggcgcgc aggaggagca gaaaacacca ccttctgcag ttggaggcag gcagccccgg 420
   ctgcactcta gccgccgcgc ccggagccgg ggccgacccg ccactatccg cagcagcctc 480
   ggccaggagg cgacccgggc gcctgggtgt gtggctgctg ttgcggacgt cttcgcgggg 540
   gcgggaggct cgcgccgcag ccagcgccat gcaaaactac aagtacgaca aagcgatcgt 600
   cccggagagc aagaacggcg gcagcccggc gctcaacaac aacccgagga ggagcggcag 660
   caagcgggtg ctgctcatct gcctcgacct cttctgcctc ttcatggcgg gcctcccctt 720
   cctcatcatc gagacaagca ccatcaagcc ttaccaccga gggttttact gcaatgatga 780
   gagcatcaag tacccactga aaactggtga gacaataaat gacgctgtgc tctgtgccgt 840
   ggggatcgtc attgccatcc tcgcgatcat cacgggggaa ttctaccgga tctattacct 900
   gaagaagtcg cggtcgacga ttcagaaccc ctacgtggca gcactctata agcaagtggg 960
   ctgcttcctc tttggctgtg ccatcagcca gtctttcaca gacattgcca aagtgtccat 1020
   agggcgcctg cgtcctcact tcttgagtgt ctgcaaccct gatttcagcc agatcaactg 1080
   ctctgaaggc tacattcaga actacagatg cagaggtgat gacagcaaag tccaggaagc 1140
   caggaagtcc ttcttctctg gccatgcctc cttctccatg tacactatgc tgtatttggt 1200
   gctatacctg caggcccgct tcacttggcg aggagcccgc ctgctccggc ccctcctgca 1260
   gttcaccttg atcatgatgg ccttctacac gggactgtct cgcgtatcag accacaagca 1320
   ccatcccagt gatgttctgg caggatttgc tcaaggagcc ctggtggcct gctgcatagt 1380
   tttcttcgtg tctgacctct tcaagactaa gacgacgctc tccctgcctg cccctgctat 1440
   ccggaaggaa atcctttcac ctgtggacat tattgacagg aacaatcacc acaacatgat 1500
   gtaggtgcca cccacctcct gagctgtttt tgtaaaatga ctgctgacag caagttcttg 1560
   ctgctctcca atctcatcag acagtagaat gtagggaaaa acttttgccc gactgatttt 1620
   taaaaaggaa aaaaaaaatg ttttactatg tggccttcca aaataggtag tgtttgccta 1680
   tgtggaaaca acagcaaact aacaccaagt gccagagtcc tggatgtgca attggtttaa 1740
   gtgttcatgt tctagtgaac acagcttgtt caggaacaaa cactaaaacg atttgagtag 1800
   aggctgctgg tcaccttttg tgacctgagg aatcccaggc ctgtgagaaa agcaaaaatt 1860
   cacattgcag cacatgatgc cagaaatagc actgaatcaa gaaaatagcc attgcggagc 1920
   tgccctcttg agtctttctg tccatcccat tctattctgt actgtgactt tagttcagga 1980
   agttttgttt tgtgttttaa ataaaaggaa agagcaagtt tgctcagtca agtgatcaga 2040
   tcccgaatct agattcccag ttctaaggcc ttatcacctc ccctgcccat aggccaacaa 2100
   ccatagttcc tcacattagt gattagcaga ctctttgtgg acgagtgaat ttcacaaaca 2160
   gcacaatttc agaagaaatc gagggcacag gccctcactt ttcttctttt gacgcatcat 2220
   cctgtgatgt tgaaatgtca attgcaggat gctgatgttg tgcacgtcaa tcaccgggca 2280
   cttgcatact cttagaaaca gttcgacttc ggttactgcc ttctcccttg aaatccttgc 2340
   tgcgtgccca ccaggatttc ctgtgagggc ccaggaatga gcaaggcatg gtctgccacc 2400
   agctgacgga aagcagcctt ctgtacaaca gatggagggt tgaagggggc agaatgaaaa 2460
   tcgaaccaac cttttagctg ttgcaaatca gaaggagcca gagaagcagg cagtctcatg 2520
   catgagaggt tacccttcag gatgacagag ctgagggtct ttgtaggagt tgctcttgct 2580
   gtgtaaagca ctattgtctt ggggttgagc cctagggcag ttcttggtag gttctgctgg 2640
   gcagaacata tgggttaaat ctcggtagag agtttccctc atcctctatc cgtaagtgtc 2700
   cttccatgca aggtcccact ctaggtgata gacaggaccc ccttctactg aacctttgag 2760
   gaaaggagga aggaagaaat gcgtttagat cttggatgca gacctttcaa agggttaaat 2820
   gtaaccatat ggatcaacca catgcacatc cttactacag aatccgtcct ttcatttcaa 2880
   cttatagcaa gctatgattt ttatatataa atattatata aataatgtat aaaacattaa 2940
   aagttaacta tgtaagatat tatttctgaa acaatttagc tatatccact atgattataa 3000
   actgtgtctc gacctgtgtt atttacatta gctgcttaaa aaagcattga gttaattttt 3060
   ttaaatatca actaaaatat catagttctg tggtagacat tgttttataa tgaaataact 3120
   gcaactagag aaaactgtat aaaaacatta aattgtcagt atttttgtaa ggttccattt 3180
   tgtaaagaga ataatattca aagacttttg tagcatacaa agtgaaaact tgtatctgcg 3240
   aaactatact tgtattaaat gtgcttttta aataaaagct cgtaacacaa ctaattaagg 3300
   acttgcaaaa aaaaaaaaaa aaaa 3324

<210> 53
<211> 6107
<212> DNA

<213>  Homo sapiens

<400>  53
aaatgacgac aacggtgagg gttctcgggc ggggcctggg acaggcagct ccggggtccg      60

cggtttcaca tcggaaacaa aacagcggct ggtctggaag gaacctgagc tacgagccgc     120

ggcggcagcg gggcggcggg gaagcgtata cctaatctgg gagcctgcaa gtgacaacag     180

cctttgcggt ccttagacag cttggcctgg aggagaacac atgaaagaaa gaacctcaag     240

aggctttgtt ttctgtgaaa cagtatttct atacagttgc tccaatgaca gagttacctg     300

caccgttgtc ctacttccag aatgcacaga tgtctgagga caaccacctg agcaatactg     360

tacgtagcca gaatgacaat agagaacggc aggagcacaa cgacagacgg agccttggcc     420

accctgagcc attatctaat ggacgacccc agggtaactc ccggcaggtg gtggagcaag     480

atgaggaaga agatgaggag ctgacattga aatatggcgc caagcatgtg atcatgctct     540

ttgtccctgt gactctctgc atggtggtgg tcgtggctac cattaagtca gtcagctttt     600

atacccggaa ggatgggcag ctaatctata ccccattcac agaagatacc gagactgtgg     660

gccagagagc cctgcactca attctgaatg ctgccatcat gatcagtgtc attgttgtca     720

tgactatcct cctggtggtt ctgtataaat acaggtgcta taaggtcatc catgcctggc     780

ttattatatc atctctattg ttgctgttct ttttttcatt catttacttg ggggaagtgt      840

ttaaaaccta taacgttgct gtggactaca ttactgttgc actcctgatc tggaattttg     900

gtgtggtggg aatgatttcc attcactgga aaggtccact tcgactccag caggcatatc     960

tcattatgat tagtgccctc atggccctgg tgtttatcaa gtacctccct gaatggactg    1020

cgtggctcat cttggctgtg atttcagtat atgatttagt ggctgttttg tgtccgaaag    1080

gtccacttcg tatgctggtt gaaacagctc aggagagaaa tgaaacgctt tttccagctc    1140

tcatttactc ctcaacaatg gtgtggttgg tgaatatggc agaaggagac ccggaagctc    1200

aaaggagagt atccaaaaat tccaagtata atgcagaaag cacagaaagg gagtcacaag    1260

acactgttgc agagaatgat gatggcgggt tcagtgagga atgggaagcc cagagggaca    1320

gtcatctagg gcctcatcgc tctacacctg agtcacgagc tgctgtccag gaactttcca    1380

gcagtatcct cgctggtgaa gacccagagg aaaggggagt aaaacttgga ttgggagatt    1440

tcattttcta cagtgttctg gttggtaaag cctcagcaac agccagtgga gactggaaca    1500

caaccatagc ctgtttcgta gccatattaa ttggtttgtg ccttacatta ttactccttg    1560

ccattttcaa gaaagcattg ccagctcttc caatctccat cacctttggg cttgttttct    1620

actttgccac agattatctt gtacagcctt ttatggacca attagcattc catcaatttt    1680

atatctagca tatttgcggt tagaatccca tggatgtttc ttctttgact ataacaaaat    1740

ctggggagga caaaggtgat tttcctgtgt ccacatctaa caaagtcaag attcccggct    1800

ggacttttgc agcttccttc caagtcttcc tgaccacctt gcactattgg actttggaag 1860

gaggtgccta tagaaaacga ttttgaacat acttcatcgc agtggactgt gtccctcggt 1920

gcagaaacta ccagatttga gggacgaggt caaggagata tgataggccc ggaagttgct 1980

gtgccccatc agcagcttga cgcgtggtca caggacgatt tcactgacac tgcgaactct 2040

caggactacc gttaccaaga ggttaggtga agtggtttaa accaaacgga actcttcatc 2100

ttaaactaca cgttgaaaat caacccaata attctgtatt aactgaattc tgaacttttc 2160

aggaggtact gtgaggaaga gcaggcacca gcagcagaat ggggaatgga gaggtgggca 2220

ggggttccag cttccctttg attttttgct gcagactcat cctttttaaa tgagacttgt 2280

tttcccctct ctttgagtca agtcaaatat gtagattgcc tttggcaatt cttcttctca 2340

agcactgaca ctcattaccg tctgtgattg ccatttcttc ccaaggccag tctgaacctg 2400

aggttgcttt atcctaaaag ttttaacctc aggttccaaa ttcagtaaat tttggaaaca 2460

gtacagctat ttctcatcaa ttctctatca tgttgaagtc aaatttggat tttccaccaa 2520

attctgaatt tgtagacata cttgtacgct cacttgcccc agatgcctcc tctgtcctca 2580

ttcttctctc ccacacaagc agtctttttc tacagccagt aaggcagctc tgtcgtggta 2640

gcagatggtc ccattattct agggtcttac tctttgtatg atgaaaagaa tgtgttatga 2700

atcggtgctg tcagccctgc tgtcagacct tcttccacag caaatgagat gtatgcccaa 2760

agacggtaga attaaagaag agtaaaatgg ctgttgaagc actttctgtc ctggtatttt 2820

gtttttgctt ttgccacaca gtagctcaga atttgaacaa atagccaaaa gctggtggtt 2880

gatgaattat gaactagttg tatcaacaca aagcaagagt tggggaaagc catatttaac 2940

ttggtgagct gtgggagaac ctggtggcag aaggagaacc aactgccaag gggaaagaga 3000

aggggcctcc agcagcgaag gggatacagt gagctaatga tgtcaaggag gagtttcagg 3060

ttattctcgt cagctccaca aatgggtgct ttgtggtctc tgcccgcgtt acctttcctc 3120

tcaatgtacc tttgtgtgaa ctgggcagtg gaggtgcctg ctgcagttac catggagttc 3180

aggctctggg cagctcagtc aggcaaaaca cacaaacagc catcagcctg tgtgggctca 3240

gggcacctct ggacaaaggc ttgtggggca taaccttctt taccacagag agcccttagc 3300

tatgctgatc agaccgtaag cgtttatgag aaacttagtt tcctcctgtg gctgaggagg 3360

ggccagcttt ttcttctttt gcctgctgtt ttctctccca atctatgata tgatatgacc 3420

tggtttgggg ctgtctttgg tgtttagaat atttgttttc tgtcccagga tatttcttat 3480

aagaacctaa cttcaagagt agtgtgcgag tactgatctg aatttaaatt aaaattggct 3540

tatattaggc agtcacagac aggaaaaata agagctatgc aaagaaaggg ggatttaaag 3600

tagtaggttc tatcatctca attcattttt ttccatgaaa tcccttcttc caagattcat 3660

tccctctctc agacatgtgc tagcatgggt attatcattg agaaagcaca gctacagcaa 3720

agccacctga atagcaattt gtgattggaa gcattcttga gggatcccta atctagagta 3780

atttatttgt gtaaggatcc caaatgtgtt gcacctttca tgatacattt cttctctgaa 3840

gagggtacgt ggggtgtgtg tatttaaatc catcctatgt attactgatt gtcctgtgta 3900

gaaagatggc aattattctg tctctttctc caagtttgag ccacatctca gccacattgt 3960

tagacagtgt acagagaacc tatctttcct tttttttttt ttaaaggaca ggattttgct 4020

gtgttgccca ggctagactt gaactcctgg gctcaagtaa tccacctcag cctgagtagc 4080

tgagactaca gcccatctta tttctttaaa tcattcatct caggcagaga acttttccct 4140

caaacattct ttttagaatt agttcagtca ttcctaaaac atccaaatgc tagtcttcca 4200

ccatgaaaaa tagattgtca ctggaaagaa cagtagcaat ttccataagg atgtgccttc 4260

actcacacgg gacaggcggt ggttatagag tcgggcaaaa ccagcagtag agtatgacca 4320

gccaagccaa tctgcttaat aaaaagatgg aagacagtaa ggaaggaaag tagccactaa 4380

gagtctgagt ctgactgggc tacagaataa agggtattta tggacagaat gtcattacat 4440

gcctatggga ataccaatca tatttggaag atttgcagat tttttttcag agaggaaaga 4500

ctcaccttcc tgtttttggt tctcagtagg ttcgtgtgtg ttcctagaat cacagctctg 4560

actccaaatg actcaatttc tcaattagaa aaagtagaag ctttctaagc aacttggaag 4620

aaaacagtca taagtaagca atttgttgat tttactacag aagcaacaac tgaagaggca 4680

gtgtttttac tttcagactc cgggattccc attctgtagt ctctctgctt ttaaaaaccc 4740

tccttttgca atagatgccc aaacagatga tgtttattac ttgttattta cgtggcctca 4800

gacagtgtat gtattctcga tataacttgt agagtgtgaa atataagttt aactaccaaa 4860

taaggtctcc cagggttaga tgactgcggg aagcctttga tcccaacccc caaggctttg 4920

tatatttgat catttgtgat ctaaccctgg aagaaaaaga gctcagaaac cactatgaaa 4980

aaatttgttc agtgttttct gtgttcccgt aggttctgga gtctgaggat gcaaagatga 5040

ataagataaa ttctcagaat gtagttataa tctcttgttt tctggtatat gccatctttc 5100

tttaacttct ctaaaatatt gggtatttgt caaataacca cttttaacag ttaccattac 5160

tgagggctta tacattggtg ttataaaagt gacttgattc agaaatcaat ccattcagta 5220

aagtactcct tctctaaatt tgctgttatg tctataagga acagtttgac ctgcccttct 5280

cctcacctcc tcacctgcct tccaacattg aatttggaag gagacgtgaa aattggacat 5340

ttggttttgc ccttgggctg gaaactatca tataatcata agtttgagcc tagaagtgat 5400

ccttgtgatc ttctcacctc tttaaattcc cacaacacaa gagattaaaa acagaggttt 5460

cagctcttca tagtgcgttg tgaaatggct ggccagagtg taccaacaaa gctgtcatcg 5520

ggctcacagc tcagagacat ctgcatgtga tcatctgcat agtcctctcc tctaacggga 5580

aacacctcag atttgcatat aaaaaagcac cctggtgctg aaatgaaccc ctttcttgaa 5640

catcaaagct gtctcccaca gccttgggca gcagggtgcc tcttagtgga tgtgctgggt 5700

ccaccctgag ccctgacatg tggtggcagc attgccagtt ggtctgtgtg tctgtgtagc 5760

agggacgatt tcccagaaag caattttcct tttgaaatac gtaattgttg agactaggca 5820

gtttcaaagt cagctgcata tagtagcaag tacaggactg tcttgttttt ggtgtccttg 5880

gaggtgctgg ggtgagggtt tcagtgggat catttactct cacatgttgt ctgccttctg 5940

cttctgtgga cactgctttg tacttaattc agacagactg tgaatacacc ttttttataa 6000

atacctttca aattcttggt aagatataat tttgatagct gattgcagat tttctgtatt 6060

tgtcagatta ataaagactg catgaatcca aaaaaaaaaa aaaaaaa 6107

<210> 54
<211> 2869
<212> DNA
<213> Homo sapiens

<400> 54
agaattcttt ggcaggggcg accttagaat cctggggagg agcgagaatg gaatcccggg 60

gaggaacagg ggtggaatcc ggggggcggg gtcagaacgc caggaggggg cggggccgga 120

gccagggtcg gcttgactcg ggggagcagc gggtggatcc tgtgacgtca gcgggttcga 180

accgccggag ctgagcgaga ggccgggggt gccgagccgg gcggggagag ctgggccggg 240

agagcagaac agggaggcta gagcgcagcg ggaaccggcc cggagccgga gccggagccc 300

cacaggcacc tactaaaccg cccagccgat cggcccccac agagtggccc gcgggcctcc 360

ggccgggccc agtcccctcc cgggccctcc atggcccggg ccgctgccct cctgccgtcg 420

agatcgccgc cgacgccgct gctgtggccg ctgctgctgc tgctgctcct ggaaaccgga 480

gcccaggatg tgcgagttca agtgctaccc gaggtgcgag gccagctcgg gggcaccgtg 540

gagctgccgt gccacctgct gccacctgtt cctggactgt acatctccct ggtgacctgg 600

cagcgcccag atgcacctgc gaaccaccag aatgtggccg ccttccaccc taagatgggt 660

cccagcttcc ccagcccgaa gcctggcagc gagcggctgt ccttcgtctc tgccaagcag 720

agcactgggc aagacacaga ggcagagctc caggacgcca cgctggccct ccacgggctc 780

acggtggagg acgagggcaa ctacacttgc gagtttgcca ccttccccaa ggggtccgtc 840

cgagggatga cctggctcag agtcatagcc aagcccaaga accaagctga ggcccagaag 900

gtcacgttca gccaggaccc tacgacagtg gccctctgca tctccaaaga gggccgccca 960

cctgcccgga tctcctggct ctcatccctg gactgggaag ccaaagagac tcaggtgtca 1020

gggaccctgg ccggaactgt cactgtcacc agccgcttca ccttggtgcc ctcgggccga 1080

gcagatggtg tcacggtcac ctgcaaagtg gagcatgaga gcttcgagga accagccctg 1140

atacctgtga ccctctctgt acgctaccct cctgaagtgt ccatctccgg ctatgatgac 1200

aactggtacc tcggccgtac tgatgccacc ctgagctgtg acgtccgcag caacccagag 1260

cccacgggct atgactggag cacgacctca ggcaccttcc cgacctccgc agtggcccag 1320

ggctcccagc tggtcatcca cgcagtggac agtctgttca ataccacctt cgtctgcaca 1380

gtcaccaatg ccgtgggcat gggccgcgct gagcaggtca tctttgtccg agagaccccc 1440

aacacagcag gcgcaggggc cacaggcggc atcatcgggg gcatcatcgc cgccatcatt 1500

gctactgctg tggctgccac gggcatcctt atctgccggc agcagcggaa ggagcagacg 1560

ctgcaggggg cagaggagga cgaagacctg gagggacctc cctcctacaa gccaccgacc 1620

ccaaaagcga agctggaggc acaggagatg ccctcccagc tcttcactct gggggcctcg 1680

gagcacagcc cactcaagac cccctacttt gatgctggcg cctcatgcac tgagcaggaa 1740

atgcctcgat accatgagct gcccaccttg gaagaacggt caggaccctt gcaccctgga 1800

gccacaagcc tggggtcccc catcccggtg cctccagggc cacctgctgt ggaagacgtt 1860

tccctggatc tagaggatga ggagggggag gaggaggaag agtatctgga caagatcaac 1920

cccatctatg atgctctgtc ctatagcagc cctctgattc cctaccaggg caaaggcttt 1980

gtcatgtccc gggccatgta tgtgtgagct gccatgcgcc tggcgtctca catctcacct 2040

gttgatccct tagctttctt gccaaggatc tagtgccccc tgacctctgg ccaggccact 2100

gtcagttaac acatatgcat tccatttgtg atgtctacct tggtggctcc actatgaccc 2160

ctaacccatg agcccagaga aattcaccgt gataatggaa tcctggcaac cttatctcat 2220

gaggcaggag gtggggaagg tgcttctgca caacctctga tcccaaggac tcctctccca 2280

gactgtgacc ttagaccata cctctcaccc cccaatgcct cgactccccc aaaatcacaa 2340

agaagaccct agacctataa tttgtcttca ggtagtaaat tcccaatagg tctgctggag 2400

tgggcgctga gggctccctg ctgctcagac ctgagccctc caggcagcag ggtcccactt 2460

accccctccc caccctgttc cccaaaggtg ggaaagaggg gattccccag cccaaggcag 2520

ggttttccca gcaccctcct gtaagcagaa gtctcagggt ccagaccctt ccctgagccc 2580

ccacccccac cccaattcct gcctaccaag caagcagccc cagcctaggg tcagacaggg 2640

tgagcctcat acagactgtg ccttgatggc cccagccttg ggagaagaat ttactgttaa 2700

cctggaagac tactgaatca ttttaccctt gcccagtgga ataggaccta aacatccccc 2760

ttccggggaa agtgggtcat ctgaattggg ggtagcaatt gatactgttt tgtaaactac 2820

atttcctaca aaatatgaat ttatactttg accaggaaaa aaaaaaaaa 2869

<210> 55
<211> 1211
<212> DNA
<213> Homo sapiens

<400>  55
ctgggctccg tgccgctctg tttgccaacc gtccagtccc gcctaccagt gccgggcgct      60

ccccacccct cccccggctc ccccggtgtc cgccatggcc aaagcctacg accacctctt     120

caagttgctg ctgatcgggg actcgggggt gggcaagact tgtctgatca ttcgctttgc     180

agaggacaac ttcaacaaca cttacatctc caccatcgga attgatttca agatccgcac     240

tgtggatata gaggggaaga agatcaaact acaagtctgg gacacggctg gccaagagcg     300

gttcaagaca ataactactg cctactaccg tggagccatg ggcattatcc tagtatacga     360

catcacggat gagaaatctt tcgagaatat tcagaactgg atgaaaagca tcaaggagaa     420

tgcctcggct ggggtggagc gcctcttgct ggggaacaaa tgtgacatgg aggccaagag     480

gaaggtgcag aaggagcagg ccgataagtt ggctcgagag catggaatcc gatttttcga     540

aactagtgct aaatccagta tgaatgtgga tgaggctttt agttccctgg cccgggacat     600

cttgctcaag tcaggaggcc ggagatcagg aaacggcaac aagcctccca gtactgacct     660

gaaaacttgt gacaagaaga acaccaacaa gtgctccctg ggctgaggac cctttcttgc     720

ctccccaccc cggaagctga acctgaggga gacaacggca gagggagtga gcaggggaga     780

aatagcagag gggcttggag ggtcacatag gtagatggta aagagaatga ggagaaaaag     840

gagaaaaggg aaaagcagaa aggaaaaaaa ggaagagaga ggaaggagaa agggagagga     900

atgaattgag gaagtgaaag aaggcaagga ggtaggaaga gagggaggag gaaaggaagg     960

agagatgcct caggcttcag accttacctg ggttttcagg gcaaacataa atgtaaatac    1020

actgatttat tctgttacta gatcaggttt tagggtcctg caaaaggcta gctcggcact    1080

acactaggga atttgctcct gttctgtcac ttgtcatggt ctttcttggt attaaaggcc    1140

accatttgca caaaaaaaaa aaaaaaaaaa aaaaaaaaaa aaaaaaaaaa aaaaaaaaaa    1200

aaaaaaaaaa a                                                         1211

<210>  56
<211>  4877
<212>  DNA
<213>  Homo sapiens

<400>  56
ggcggattgg cgggcacgcc ccctcgcccg cggccccctc cccgcctctc tccaccgcct      60

cctctggctc cccggtcaga gggccggagc gagaagatgg cgaagacgta cgattatctc     120

ttcaagctcc tgctgatcgg cgactcgggg gtaggcaaga cctgcctcct gttccgcttc     180

tcagaggacg ccttcaacac caccttcatc tccaccatcg gaattgattt taaaattaga     240

acgatagaac tagatggaaa gaaaattaag cttcagatat gggacacagc gggtcaggaa     300

agattccgaa caatcacgac agcgtactac agaggagcca tgggcattat gctggtctat     360

gacatcacaa atgaaaaatc ctttgacaat attaaaaatt ggatcagaaa cattgaagag 420

catgcctctt ccgatgtcga aagaatgatc ctgggtaaca aatgtgatat gaatgacaaa 480

agacaagtgt caaaagaaag aggggagaag ctagcaattg actatgggat taaattcttg 540

gagacaagcg caaaatccag tgcaaatgta gaagaggcat tttttacact tgcacgagat 600

ataatgacaa aactcaacag aaaaatgaat gacagcaatt cagcaggagc aggtggacca 660

gtgaaaataa cagaaaaccg atcaaagaag accagtttct ttcgttgctc gctactttga 720

tgaactcttt ctgagagact gcagcacacc tagaggcccc tttcctgctt ctctgaaagc 780

acaggtcacc cagcctcaga atcacacctc ccggctgctg ctgagagcac cactgaactt 840

agacctctca acacagtatg ccaagtggat tccagcctca tggcctagca aaagaacaga 900

ctcccttttt caaacatgga agcaatgaag tggagacaca tgcaggacct aactcgtttt 960

ttccttgttt tattacctgt tgcagaagcg gttatctttc ttttttactt tgcacatcag 1020

tgttagcctt tccctatttc agcacaatct tagactcata tttgcacact tttgtgtcgt 1080

gaagttctag acaaatttgt acatgtggca atgttaaaag agcatttaca gcagaggtta 1140

atatactaaa attaaagggt atttggtctg gttcatatgg tcaaatatta ctgccttggt 1200

agcatttatt taagggcttt ttcttaaata agaatcatta aagtcattaa aaaaatttac 1260

tgaaatgccc atcttgtcat caaaggccac aatttcttta tttcttcaga ttaagagctt 1320

tgcctcatcc ccgacctgtt ttccagagtc tgggtagctg aatgaatcac tttaaaatga 1380

ttacctctgc ctaatctata gaaactgcat ttggaaatca ccataatctc atttttccct 1440

ggggtttgta tttgctattc tttcccatgt ttgacttaag tgtaatcact cttaagtaat 1500

atttgaacat tattatctgt ttctatttgt gaacttcttg agctgaaatt ttacgtgggc 1560

tgagagatat accatttagg gttttagtgc agcatctaac tgtgattctg tcaataagga 1620

tatgtaatat atttttcttt aggttcactc cttagctggc tggtttagtt gtaataccaa 1680

attcctacca taatccctgt ctacaaaagt taggtttaga ttttagtttg cggaaacctt 1740

ccctatatag agacagatta acttgttgat ataaatttaa tagagctagc tcttggtaat 1800

ggtgaaaata atgagttttg gttggtttta tttggcagat gtttttagaa ataaaagtac 1860

ttagacctag tgcagcctct aggaaaagtc ttgccttttc attagagaaa acaggaccaa 1920

ggtttcagtt ttcaaacagc tgttgttgat tgtgtagaac ccagttccat ctgttttggt 1980

tcattgttac agaacttagt ccagtcattt gggctaaagc caaccaaaag cttagttgcc 2040

tttctcaaca aacactggta ctggtatact tttgtagatg aaaccatcac aaggtattta 2100

gtgttaactt gtgtgccaaa ttcagatcac tatgtcgttg ttgctctagc cttcagtgtc 2160

ataacacagg ggggataaaa cagaggggat gagggaaatg aattctgtta ataattattc 2220

ttcctggtat gcctgttttg cttcacaaag gctactatca tgctggatag ataagaacag 2280

gagatggcag tggaaaggga ttgcttgtta ccacagagaa ttctcttcaa attaagatat 2340

gtcattagaa tgcttggacc agtcaatctt ttgtacttat ttgaaaatat aggaacaatt 2400

tagcagctgc aaatatgccc aagctatttt taatagatat actaaactta ttgttgacaa 2460

atttcagcct ccttaatttt tttttttttg gtaattacct ataggcttaa aagtcattcg 2520

ttgctgttct agtataatta gtagttccgt gattgaaagt ttattgtaat tctactatct 2580

tcaaattaga tacattttca aaaggaaaag ataatttttt agaaacatct taatattcac 2640

tatttcctga aaaaatccaa gcagttaacg ctttctggat ggtaacaaag taccttctaa 2700

aagataagtg ctatgacacc atgtatgaat gtaattctct tagtaattta cctctgacta 2760

tttggtgtct taacgctttg gtttatataa tctcaggggt tgtctgcaaa ccaaaactga 2820

catattctgt ggttagcttt tattactttt ttttttaaca gaatgctttg ctttggttat 2880

atttcttctt tcttcttttg tcttatatgg attaataact agtctccatg aacttcactt 2940

gaaagagcct gtaaaagtta gatgagtcta aaagtgctct ttgaagtagc agcaaattgg 3000

gagtatatgc tctgttatat agaaataaat tgtccttgct attttcttac atttagcttt 3060

gctagattgt atatacattg agctaagacc ttaggaaatt cactttctgc atgataaaat 3120

gacccaataa atattccact ttgcttaata atgtacatac agtgcattat tttttctatt 3180

tgtagatgaa tttaatgaca gataattgtc tgttccccgc tgaaactgaa gaaatctagt 3240

ttttttgtga acatttttgt ggtcttatgg ataaggtaca tgaagatttt tgcagcagta 3300

ttagtggttc agtggctgca ctttattaat cagtgtgtta atttttgaca gtgattggac 3360

tagacctttt caaatagagt ctgagggtat cagacagtga aaatgtgctg taactaagta 3420

gcatgtaaat cagttgattg taaaacgttt cgctgggaac ttattttagc tatattttac 3480

ttcagacaga ttatgataca ataatctacc tgtgcatcag ttagtaggtg ctgcagggtt 3540

tcttactatt tacagaaaca ttttgtgcag tctttgttat aaattttcag aagactatac 3600

tctttacttt gaaggtctat tttttaatta tacctcattt agctaactag tattctaata 3660

cctggtagaa aaacagtgag ccagccttta agcatctaac aaaatttaga ctctttgttt 3720

tgttttgaac tgaagacatc attgggaaag gtaggaaata ttagtttagg atagagcata 3780

catgtcatat ccagtagcat aaaaaaagta ttatctccct gtctccatta ataaatttag 3840

ctgtgcaata taggtgcgtt tttgcagaag tttctagtaa gagattataa ctccatttta 3900

caggttctga atgctcagag tctatattaa ggcttataaa gtttttcctg tgatcagtaa 3960

gtgacacatt taagcagaca ttcttttcaa gttcatgact tagattcctt tacaaattta 4020

gttctcaatc tttaaaaacc acatttcatt atgttggtta attattataa attttaagca 4080

ctcatttctg caatcaggtt tctcagaatt tttttttttt aaacagaaga gacattcctt 4140

tttccttgtt acatccaagg ggggcacagg ggtgggtgga aaggaatgtc taaaaatgaa 4200

atcccttgat atagagggac ctagacggac aggatatgct aaaataattt caaattctag 4260

cacaatttta gagtagaata agtcattttt ttagactaat aaaattaatg gctgtcatgt 4320

tcactctgaa aaaaatctaa atgactgaaa tgtacagaaa taaaaattag caaacaatta 4380

ttctagggat attttcagat tttacttcat ttcttgaaat gcgtgtgcca tatgcaattg 4440

catttcttgt gccaagaaac taatagaact tatttcactt tacctttttt taaaatgtga 4500

atttagttat tatagttcaa ttttatggcc ttacagatgg cttttatttt gtttgcagct 4560

gacactgcag ttcctttcat gcaaaatacc ataaactgtt tgatgaaaat catgccccta 4620

atggaaactc tctagttttt ccatataact atcctactgt acatgtttaa acatatttta 4680

tttttgctcc aatggcttaa tgtgaaaagc tcctgcagat aagtggacct gtcatgtggt 4740

taatcttgtt taagccaatt cattaactgt gtactgatac tgatgctatg ttttttttaa 4800

atggatttta ttccaggtga actttttttt tataataatg ttcgtctaaa ataaaaacta 4860

cataatgaaa atgaaaa 4877

<210> 57
<211> 1288
<212> DNA
<213> Homo sapiens

<400> 57
cgtcgccatg ttgttccctc cgcgctggac gggagcagct ggagcgggag cctggctgcg 60

ctaccgcggc tgcctcctgc tgtgcaggtc cccgaccctc tctctgtcct cattgcgccc 120

agacgggccg gcccagagct cccgggtcgt ctttcgtgtg gccgcgaggg ttcttgaagc 180

ttttgagatt aacaatggca ggaaaatcat cactttttaa agtaattctc cttggagatg 240

gtggagttgg gaagagttca cttatgaaca gatatgtaac taataagttt gatacccagc 300

tcttccatac aataggtgtg gaatttttaa ataaagattt ggaagtggat ggacattttg 360

ttaccatgca gatttgggac acggcaggtc aggagcgatt ccgaagcctg aggacaccat 420

tttacagagg ttctgactgc tgcctgctta cttttagtgt cgatgattca caaagcttcc 480

agaacttaag taactggaag aaagaattca tatattatgc agatgtgaaa gagcctgaga 540

gctttccttt tgtgattctg ggtaacaaga ttgacataag cgaacggcag gtgtctacag 600

aagaagccca agcttggtgc agggacaacg gcgactatcc ttattttgaa acaagtgcaa 660

aagatgccac aaatgtggca gcagcctttg aggaagcggt tcgaagagtt cttgctaccg 720

aggataggtc agatcatttg attcagacag acacagtcaa tcttcaccga aagcccaagc 780

ctagctcatc ttgctgttga ttgttagatt gttgatgcat tctaaccaac tcacacatat 840

acacaaaatc aacatgggga tggagaagag aattagcgtt tgcagcagtg tatcatctac 900

taataaaatt aaactaatgt tgctgcttca ttagttggtg ggagaaggga cacatccact 960

cttggaggaa tatatttact caataatggc accttacatt tataaattgt aacagttgtc 1020

taataacgtt tctttaattt aaatatgtaa gttgcagagc taataaatga aatgaccaag 1080

actttaatta taataaaaat aagaaacttg actattctag aagttatact tggatttttt 1140

cctgggaaaa tggagaacta ctttttatat gtgtatgttt ttatgcaatt agcattgtat 1200

tcttggttca gggaaatact ttcctaaagc aataatgtta gatattaaag attaaaatct 1260

aatgtatttg caaaaaaaaa aaaaaaaa 1288

<210> 58
<211> 2828
<212> DNA
<213> Homo sapiens

<400> 58
agcgccccgg aagtgatctg tggcggctgc tgcagagccg ccaggaggag ggtggatctc 60

cccagagcaa agcgtcggag tcctcctcct ccttctcctc ctcctcctcc tcctcctcca 120

gccgcccagg ctcccccgcc acccgtcaga ctcctccttc gaccgctccc ggcgcggggc 180

cttccaggcg acaaggaccg agtaccctcc ggccggagcc acgcagccgc ggcttccgga 240

gccctcgggg cggcggactg gctcgcggtg cagattcttc ttaatccttt ggtgaaaact 300

gagacacaaa atggctgcaa ataagcccaa gggtcagaat tctttggctt tacacaaagt 360

catcatggtg ggcagtggtg gcgtgggcaa gtcagctctg actctacagt tcatgtacga 420

tgagtttgtg gaggactatg agcctaccaa agcagacagc tatcggaaga aggtagtgct 480

agatggggag gaagtccaga tcgatatctt agatacagct gggcaggagg actacgctgc 540

aattagagac aactacttcc gaagtgggga ggggttcctc tgtgttttct ctattacaga 600

aatggaatcc tttgcagcta cagctgactt cagggagcag attttaagag taaaagaaga 660

tgagaatgtt ccatttctac tggttggtaa caaatcagat ttagaagata aaagacaggt 720

ttctgtagaa gaggcaaaaa acagagctga gcagtggaat gttaactacg tggaaacatc 780

tgctaaaaca cgagctaatg ttgacaaggt attttttgat ttaatgagag aaattcgagc 840

gagaaagatg gaagacagca aagaaaagaa tggaaaaaag aagaggaaaa gtttagccaa 900

gagaatcaga gaaagatgct gcattttata atcaaagccc aaactccttt cttatcttga 960

ccatactaat aaatataatt tataagcatt gccattgaag gcttaattga ctgaaattac 1020

tttaacattt tggaaattgt tgtatatcac taaaagcatg aattggaact gcaatgaaag 1080

tcaaatttac tttaaaaaga aattaatatg gcttcaccaa gaagcaaagt tcaacttatt 1140

tcataattgc ctacatttat catggtcctg aatgtagcgt gtaagcttgt gtttcttggg 1200

cagtctttct tgaaattgaa gaggtgaaat gggggtgggg agtgggagga aaggtgactt 1260

cctctggtgt ttattataaa gcttaaattt tatatcattt taaaatgtct tggtcttcta 1320

ctgccttgaa aaatgacaat tgtgaacatg atagttaaac taccactttt tttaaccatt 1380

attatgcaaa atttagaaga aaagttattg gcatggttgt tgcatatagt taaactgaga 1440

gtaattcatc tgtgaatctg ctttaattac ctggtgagta acttagaaaa gtggtgtaaa 1500

cttgtacatg gaattttttg aatatgcctt aatttagaaa ctgaaaaata tctggttata 1560

tcattctggg tgtgttctta ctgacaccag gggtccgctg ccccatgtgt cctggtgaga 1620

aaatatatgc ctggcacagc ttttgtatag aaaattcttg agaagtaact gtccgctaga 1680

agtctgtcca aatttaaaat gtgtgccata ttctggttct tgaaaataag attccagagc 1740

tctttgatcg cttttaataa actgcaagtt cattttaaat gaagggccag catatatact 1800

tgcaagataa ttttcagctg caaggattca gcaccagtta tgtttgaatg aaccctcctt 1860

ttctctgaga ttctggtccc tggaaatccc tttctgctag tggtgagcat gtaagtgtta 1920

agtttttaat ctgggagcag ggcataggaa gaaaatgtca gtagtgctaa tgcattttgc 1980

actagaacgc ttcgggaaaa tattcatgct tgccatctgt tcatttctaa atttatattc 2040

ataaagttac agtttgatac aggaattatt aggagtaatt cttttctgtt tctgtttata 2100

atgaagaaca ctgtagctac attttcagaa gttaacatca agccatcaaa cctgggtata 2160

gtgcagaaaa cgtggcacac actgaccaca cattaggctg tgtcaccatt gtgtggtgta 2220

cctgctggaa gaattctagc atgctacttg gggacataat ttcagtggga aatatgccac 2280

tgaccgattt tttttttttc ctctttgcag tggggctagg acagttgatt caacaaagta 2340

tttttttctt ttttctcagt cctaatttga acaggtcaaa gatgtgttca ggcattccag 2400

gtaacaggtg tgtatgtaaa gttaaaaata ggctttttag gaactcactc tttagatatt 2460

tacatccagc ttctcatgtt aaatatttgt ccttaaaggg tttgagatgt acatctttca 2520

tttcgtattt ctcataggct atgccatgtg cggaattcaa gttaccaatg taacactggc 2580

cagcgggccc agcaatctcc atgtgtactt attacagtct tatttaacca ggggtcctaa 2640

ccactaacat tgtgactttg ctttgagacc tttcctctcc tgggtactga ggtgctatga 2700

agccaactga caaagatgca tcacgtgtct taggctgatg ccactacccg atttgtttat 2760

ttgcaatttg agccatttaa agaccaataa acttcctttt ttaaaatgtt aaaaaaaaaa 2820

aaaaaaaa 2828

<210> 59
<211> 2092
<212> DNA
<213> Homo sapiens

<400> 59
ggcgtaatta aaaagcggcg gaagaaggtg ggagggtcat gacgcagcga gtttcagtcg 60

tgacttttct gggggcatcg cggcgtcccc tttttttgcc tttaaagtaa aacgtcgccc 120

cgacgcaccc cccgcgtatt tcggggggcg gaggcggcgg gccacggcgc gaagaggggc 180

ggtgctgacg ccggccggtc acgtgggcgt gttgtggggg ggaggggcgc cgccgcgcgg 240

tcggttccgg gcggttggga gcgcgcgagc tagcgagcga gaggcagccg cgcccgccgc 300

cgcccctgct ctgtatgccg ctctctcccg gcgcggccgc cgccgatcac agcagcagga 360

gccaccgccg ccgcggttga tgtggttggg ccggggctga ggaggccgcc aagatgccgc 420

agtccaagtc ccggaagatc gcgatcctgg gctaccggtc tgtggggaaa tcctcattga 480

cgattcaatt tgttgaaggc caatttgtgg actcctacga tccaaccata gaaaacactt 540

ttacaaagtt gatcacagta aatggacaag aatatcatct tcaacttgta gacacagccg 600

ggcaagatga atattctatc tttcctcaga catactccat agatattaat ggctatattc 660

ttgtgtattc tgttacatca atcaaaagtt ttgaagtgat taaagttatc catggcaaat 720

tgttggatat ggtggggaaa gtacaaatac ctattatgtt ggttgggaat aagaaagacc 780

tgcatatgga aagggtgatc agttatgaag aagggaaagc tttggcagaa tcttggaatg 840

cagctttttt ggaatcttct gctaaagaaa atcagactgc tgtggatgtt tttcgaagga 900

taattttgga ggcagaaaaa atggacgggg cagcttcaca aggcaagtct tcatgctcgg 960

tgatgtgatt ctgctgcaaa gcctgaggac actgggaata tattctacct gaagaagcaa 1020

actgcccgtt ctccttgaag ataaactatg cttctttttt cttctgttaa cctgaaagat 1080

atcatttggg tcagagctcc cctcccttca gattatgtta actctgagtc tgtccaaatg 1140

agttcacttc cattttcaaa ttttaagcaa tcatattttc aatttatata ttgtatttct 1200

taatattatg accaagaatt ttatcggcat taatttttca gtgtagtttg ttgtttaaaa 1260

taatgtaatc atcaaaatga tgcatattgt tacactacta ttaactaggc ttcagtatat 1320

cagtgtttat ttcattgtgt taaatgtata cttgtaaata aaatagctgc aaacctcagt 1380

cctttgtgct acttgatgtg gctttcaaag aagagaagcc ttgtcctgag tttctcactt 1440

ggcttcagga aggccccagg ttggattcca gaaaccagtg aagatgtggc cacaggagga 1500

ggtgtgctga ggtggctgct gaccgtggac tccctgcgca gtggcctgca gatgttgggg 1560

ctgggttaca gctgattgaa gctgagtggc cctggggggt ctgtgagggg agttcctccc 1620

cagtgatgaa attctctcct tccaccctca aatccctaga ccttgactga aatgctccgt 1680

ggtcgggagc ctggtcaagg aggaggagct gctgagaggc attgttcgcc cttgctcata 1740

gcttagctcg atgtccgtgt cagacaggag atgattgaga acagccttgc ctgtcactgt 1800

cctagaacac cctggagttt agtgttctgt gtcagagtct tgggagcctc cttcagaccc 1860

agatgacggg cctccctctg tccaaggagc agctgtaaag gagaagaggg atttcatttg 1920

tttggtggct gttaccttgt ctgtaagtca aacttggagt tgagcagtgc tttttaaacg 1980

attccctttt gcagctaaaa tttcacaggg ctatttctaa tacgtaagca aatgttacca 2040

ttgactttat taataaaata tagttttgct ttgcaaaaaa aaaaaaaaaa aa 2092

<210> 60
<211> 820
<212> DNA
<213> Homo sapiens

<400> 60
gtataaggtc cacaccccgg gagctgagtg attgcagaaa ctggccttcc atctctctca 60

gacaccaagc tgcagatcca ggcttttctg ggaaagtgag gccaccatgg ctctggagaa 120

gtctcttgtc cggctccttc tgcttgtcct gatactgctg gtgctgggct gggtccagcc 180

ttccctgggc aaggaatccc gggccaagaa attccagcgg cagcatatgg actcagacag 240

ttcccccagc agcagctcca cctactgtaa ccaaatgatg aggcgccgga atatgacaca 300

ggggcggtgc aaaccagtga acacctttgt gcacgagccc ctggtagatg tccagaatgt 360

ctgtttccag gaaaaggtca cctgcaagaa cgggcagggc aactgctaca agagcaactc 420

cagcatgcac atcacagact gccgcctgac aaacggctcc aggtacccca actgtgcata 480

ccggaccagc ccgaaggaga gacacatcat tgtggcctgt gaagggagcc catatgtgcc 540

agtccacttt gatgcttctg tggaggactc tacctaaggt cagagcagcg agatacccca 600

cctccctcaa cctcatcctc tccacagctg cctcttccct cttccttccc tgctgtgaaa 660

gaagtaacta cagttagggc tcctattcaa cacacacatg cttccctttc ctgagtccca 720

tccctgcgtg atttgggggg tgaagagtgg gttgtgaggt gggccccatg ttaacccctc 780

cactctttct ttcaataaaa cgcagttgca aacacctgaa 820

<210> 61
<211> 7019
<212> DNA
<213> Homo sapiens

<400> 61
gggccggggg gcgatggaat aaaagaagat ggagagactt cagcgcctgg gactcgggtg 60

ggcgaggcgg aaggtgtcct cgcagcacgg cttttctccg cgccgcggtt ggttagcgag 120

tgccctctgg gtgctaggcg ttgggcggat ggtaggatcg cggtagcata cggatccgag 180

tcctgcgccg agtgagagga gaggctggca ggggctaagt gatggatctt gtactccgtg 240

ttgcagatta ctattttttt acaccatacg tgtatccagc cacatggcca gaagatgaca 300

tcttccgaca agctattagt cttctgattg taacaaatgt tggtgcttac atcctttatt 360

tcttctgtgc aacactgagc tattattttg tcttcgatca tgcattaatg aaacatccac 420

aatttttaaa gaatcaagtc cgtcgagaga ttaagtttac tgtccaggca ttgccatgga 480

taagtattct tactgttgca ctgttcttgc tggagataag aggttacagc aaattacatg 540

atgacctagg agagtttcca tatggattgt ttgaacttgt cgttagtata atatctttcc 600

tctttttcac tgacatgttc atctactgga ttcacagagg ccttcatcat agactggtat 660

ataagcgcct acataaacct caccatattt ggaagattcc tactccattt gcaagtcatg 720

cttttcaccc tattgatggc tttcttcaga gtctacctta ccatatatac ccttttatct 780

ttccattaca caaggtggtt tatttaagtc tgtacatctt ggttaatatc tggacaattt 840

ccattcatga cggtgatttt cgtgtccccc aaatcttaca gccatttatt aatggctcag 900

ctcatcatac agaccaccat atgttctttg actataatta tggacaatat ttcactttgt 960

gggataggat tggcggctca ttcaaaaatc cttcatcctt tgaggggaag ggaccgctca 1020

gttatgtgaa ggagatgaca gagggaaagc gcagcagcca ttcaggaaat ggctgtaaga 1080

atgaaaaatt attcaatgga gagtttacaa agactgaata gattattgcc cagttattct 1140

taagtaagga caaagaagga aatatcatcg tatttctttt ttttaataag gaaaaaataa 1200

tatccataca gtcaagatac atagtaaatg gtatcatttg gaaatcagca tcgtgggcac 1260

tgctgaggaa tgatcctagt ggtaggtcag aagaagatgc tgtgaacacc aggactttaa 1320

tcttatgctt aaaatgccag atgttgttcg ggggacaact tgtatctttc tagcagcaga 1380

tctgtagttt gtatagcctc aacaacaatt ttaaataaga tggagaataa attattgagg 1440

ggactaggct atatgcattt gccttcatcc acccatgttt attaagaatc attgtgctta 1500

ataataccaa gactaagcac cataaccaag aaatactaat gtaaagattg tttcttgttt 1560

caggaatggt taattcttca acgttggtat gataatgata acttgttttg acttgaataa 1620

agtactacat cagtgtggaa aaaaattctg atacattagc agctatgtaa atgacctaat 1680

tgatagcagg tgtaataaga ctatcgtctt cctacacata ggaggctcat tctctggaca 1740

cactatcacc tattacattt tactgattaa caaataaatt ggaatttaaa aatatcgata 1800

tcaccatgat ttaatccaga tctgggatta tgtagctaaa cattgtgatg attattattt 1860

aaaaccatta tttaataaga gtaaaaatat gtgaatctgg atatatttaa aaaaagaaat 1920

ttgatgccca gataatatat taggcactac tgatttttta gttaaattga tgcactacac 1980

ttttgatgtt tgaagttaca aacctgtaat ttttttgtaa aggaaataat tgccaaatac 2040

ctaggcccat tgctgacgat tagttctaaa atcttattcc tcctcttctc ccctcacttt 2100

tccctacttc ctctgcaaaa agatttaaca aatacattca taaggaaatg tgtgttgtaa 2160

caaatatatt gcaaaaacat agtttgtaaa ggcattctat aagctattta tgtaaaatca 2220

ataaaagttg atcataatta aactgtatca gttgagtatt atagcagcac aaagtattct 2280

ttgtacagat tttgtgccaa tttgaagcca cagaaatgat gtggattgtt aattgtgttt 2340

tagaacatcc ccggacactc agtgtcacag ggggaaagaa gtgggtacca cattctgttt 2400

atatttcaca ttttaactag atttgagtgt ttttagcaag aaatcagtct taaaatctaa 2460

tgtctgggat ccagaagaaa atgtctttaa tctgtgagtt attgtcacaa tgtcatctta 2520

tttaaatgta ccaattagca ttttgtaata ggcaaatgtc atttagtgct tttcaccaat 2580

cccactcacc cccggtgctc cgccttgcct aagaaaaaga aattaaggag aagtaaactt 2640

tatttcctaa tataatgtca gctgatattt attgagcttt tcctctttgc ccagagacta 2700

ggacccaaag aagttaagta actattccca ggtttatttc tctctcatat gatgtcccat 2760

gtggatgttt gtggtcagtg gacagctttc cacctagtct ttctgcgacc caggctcctt 2820

cctcttgggg ctctgccttt ctctcagtcc atagagccct ctttgttgaa agagcacata 2880

ggaaaagaag gaaaagtctg tgtggaaaat gtttctgggt caggcctgga agtggtgcat 2940

atctcttccg cccatgttcc tttggacaga actccgtcac atggcccacc tagagagatt 3000

ttgggaaatg tgtccagctg tgtgcctggg aggaaggggg caccattttc ttgagcagct 3060

agacagtttg ccgtatttgt ggtgttctcc tcttgttgat gttgaaatgg tgaatgagcc 3120

ataaagtatt tcaggttatc cacacactaa tcatctcagt gtctttaatt cttaactcca 3180

atatgaatgt ttaaagcttc ctctagattc ttattcctat ataactaata gagaagaaag 3240

gacagcttcc tatggggaag acagaggctt cctcatagat gttaggaata atcaaacttg 3300

cccctgccct ttcacccgtc tcaaattctg gtcttttaaa gcagcgttat gttaagtagt 3360

cctaacattg taatatacag tactgccaca ttctcctact ttctattaga ggaagtcaga 3420

gaatatttat ggaagtgagg acccaaatta ccttctacag atgactttta tagttacagg 3480

acagaaagtg aaaatcaagg ttacgttttc tacttttgtg gtagaaattg agaagtgggt 3540

ggatatggtt cgagaagacc tttcagaaac acagagactg agtctttgtc ttccatgctg 3600

tctctgcagt actgagtgaa tttccttata cccttgtatc atgttttcct cccatcttct 3660

agaagctggg gacagatttg gaagagaatt acacaagttc agttttttga tacatggatt 3720

ttacagtgca tgcaggttat ttatgtagag aggaggtctg ggagaaagat ggaaactagg 3780

gagatgactg agaacaaaga tatttgggat taacacagat agaagaaaag tttgaaacca 3840

tgagattgtc acagcatgaa aaaagatatt caaagacact aaccaacttg aggggtgcag 3900

tggtctgaat gtgtcctcca aaattcaaat gtggaaatgt atttgccagt gagatagtat 3960

gtagaggtgg ggccttgagg aagtgattaa gtcatgaggg ctctgggatt aatgacttaa 4020

aaagaggtgt gaggcagctg ttcagccctt ctgtctcctg tccctttttt tttttttttt 4080

tttgagatgg agtcttgctc tgttgcccca ggctggagtg cagtggcgtg atcttggttc 4140

actgcaagct ccacctcccg ggttcacgcc attctcctgc ctcagcctcc cgagtagctg 4200

ggactacagg tgcccgccac caccacgccc ggctaatttt ttatattttt agtagagatg 4260

gggtttcacc gtgttagcca ggatggtctt gatctcctga cctcatgatc tgcctgcctc 4320

ggcctcccaa agtgctggga ttataggtgt gagccaccat gcccagcccc ttctgtccct 4380

tctaacgtga ggacccagca acaaggtacc atcttgaaag cagcaactgg ggactcagca 4440

gacaccaaac caactggtgc ttaaccgtga cttcccagcc tccagagctg taagaaaata 4500

aatttctatt atttatagat tacccagttg aagatacttt gttaaggcag tacaaatgga 4560

ctaagacaag ggggaagaaa tttgcattcc tgatcttccc aacttcttca aattcacaac 4620

acttgaatga cagtcttatt tcagcactta ctgctatcac ctattacttt tatgtgtgtc 4680

ttacctattt gagatgccag attccttgga agtagagacc gtgtctgaat catcattgta 4740

ttaaaccact catccttaac aaatgcccaa gccatggtta aagttcaata aatactttgt 4800

tgaatttatt aataaaatgg cagaaatgtc attctcttcc atatatgttt aataaatccc 4860

tgataggtgc taagcactgc actaggtaaa aattctcttc tgatgctgtc tttttggcca 4920

accatttttt tatcatttat tcattagctg acatttgcta agtgctttgg aggggtcaaa 4980

aggggaagta atgagaaatc aaagatggtc cctacatcaa ggataaacta tcttttttta 5040

gtcactcaaa gtcataaccc tttggaaaca aaacccacca gtaccccaga ttttgaccac 5100

agatgaatca gtactacaag gactggttag agggttgaat gaatctgtat actcagcact 5160

taacacagca ctctgggtaa aagaaaaaag atcctcaaag atattagttg gttacatcaa 5220

gaaaggacaa acttaggtta atctataact tcatctcaga ggaacaggaa ctttggagat 5280

aaacagggct ctgccacttg caagttgcac catccctggt ttctccatct gtaaattgat 5340

taaaacactg cctatctaat aagattaaat aagttagaag cattcagtta aatgtcaact 5400

gaaactattg ttcatgtaaa ttgtgcttga tgctttttct ttctagattc aatgattatt 5460

gtcattttac ctccataggc cctcaataga aatcagttgc agagggcaga agcctagata 5520

ttttcacctt aaaattggag ggtgaaagac attgaggtga agtagagata gagggtacac 5580

agaaaaatcg tataagtaaa actaacatcg ttaacattat ttactgtaag ttatctttgt 5640

aagagtggta aaatacattg tgttgttaaa taatttcatt taaaaaatgc atcactttgt 5700

gtgtttttat attgctaaaa ccataaggcc agtctacaag gtttgtagat aaaatagaaa 5760

cataccttcc ttgaaaagca gaataaattt tttaaaggca ggaaggaagt gtttgaacca 5820

tgtgtcaaca agctttactg tcaaagcagg cttttggtat gggaagaaaa atacttataa 5880

atacttgttt taatatttgc tttattaaaa tacatttaaa atacagcatt tttaaatctc 5940

taagctcaac ttgaagatat aagaacagta aatttgataa aaatgagaaa ttacattccc 6000

atttctttaa caatttgtaa attccaatta tcctgaacat ttaataccat ttacatattt 6060

tattaatcac attttcttaa acatttgata agagatttaa tattttgatc caactaccaa 6120

aaaagcagac ttgtgtactt gacagatttt tctaaacact tcacaactca cgattcaaac 6180

aaagacaaaa tagcatatca aaagttaatc actcagttgg aaagcactca taccataggc 6240

ttttattcat ttcttgaata attttgttat atcttcctct tttaggctgc aatgagctat 6300

aattgcacta ctgcactcca cgctgggtga cagagcaaga ccctatctct aaaaataaaa 6360

aagtatatat atataaaaat atcttcctct attataattt aactcattaa gccatttatt 6420

tagatgtaaa cttgcccccc tgacatgtgg tatgaaacaa atagaaacct agaaatttag 6480

tgcatattca aatattaaga cagacactgg tgtggtgact tttgtctgtc gcttcattgg 6540

gacgtttttt ctttctgatc aacttaatga aattataatt tactataatt aagtgtagcc 6600

atttttactg tagagttcaa tgatctttga tgaacgtgta cacccatgta accaccaccc 6660

ccaatcaaag taaagaacat tttcttacca gaataaattt cctctccgtt tgcagtcatt 6720

ctccccagcc ctaggtcacc actgatccac cttctgttac tggaaggtta gttttcttcc 6780

ctgatttaga atttcatata aattaaatca gatagtatat actcttgtgt ttagtttctt 6840

tagcttaaca tgtttagaga tatttgctgt tgcctgtgtc tgtagctttt tgttttcatt 6900

gctgaatagt atttcattgt aatataccac agttggttta tgtatttgct gatgaatatt 6960

tgtgttattt ccagcgtggg attattatga ataaagttgc tacaaacatt tgtatacaa 7019

<210> 62
<211> 3485
<212> DNA
<213> Homo sapiens

<400> 62
agtcgcccag gggagcccgg agaagcaggc tcaggaggga gggagccaga ggaaaagaag 60

aggaggagaa ggaggaggac ccggggaggg aggcgcggcg cgggaggagg aggggcgcag 120

ccgcggagcc agtggccccg cttggacgcg ctgctctcca gatacccccg gagctccagc 180

cgcgcggatc gcgcgctccc gccgctctgc ccctaaactt ctgccgtagc tccctttcaa 240

gccagcgaat ttattcctta aaaccagaaa ctgaacctcg gcacgggaaa ggagtccgcg 300

gaggagcaaa accacagcag agcaagaaga gcttcagaga gcagccttcc cggagcacca 360

actccgtgtc gggagtgcag aaaccaacaa gtgagagggc gccgcgttcc cggggcgcag 420

ctgcgggcgg cgggagcagg cgcaggagga ggaagcgagc gcccccgagc cccgagcccg 480

agtccccgag cctgagccgc aatcgctgcg gtactctgct ccggattcgt gtgcgcgggc 540

tgcgccgagc gctgggcagg aggcttcgtt ttgccctggt tgcaagcagc ggctgggagc 600

agccggtccc tggggaatat gcggcgcgcg tggatcctgc tcaccttggg cttggtggcc 660

tgcgtgtcgg cggagtcgag agcagagctg acatctgata aagacatgta ccttgacaac 720

agctccattg aagaagcttc aggagtgtat cctattgatg acgatgacta cgcttctgcg 780

tctggctcgg gagctgatga ggatgtagag agtccagagc tgacaacatc tcgaccactt 840

ccaaagatac tgttgactag tgctgctcca aaagtggaaa ccacgacgct gaatatacag 900

aacaagatac ctgctcagac aaagtcacct gaagaaactg ataaagagaa agttcacctc 960

tctgactcag aaaggaaaat ggacccagcc gaagaggata caaatgtgta tactgagaaa 1020

cactcagaca gtctgtttaa acggacagaa gtcctagcag ctgtcattgc tggtggagtt 1080

attggctttc tctttgcaat ttttcttatc ctgctgttgg tgtatcgcat gagaaagaag 1140

gatgaaggaa gctatgacct tggagaacgc aaaccatcca gtgctgctta tcagaaggca 1200

cctactaagg agttttatgc gtaaaactcc aacttagtgt ctctatttat gagatcactg 1260

aacttttcaa aataaagctt ttgcatagaa taatgaagat ctttgttttt tgttttcatt 1320

aaagagccat tctggcactt taatgataaa atcccattgt atttaaaaca tttcatgtat 1380

ttctttagaa caacataaaa ttaaaattta acatctgcag tgttctgtga atagcagtgg 1440

caaaatatta tgttatgaaa accctcgatg ttcatggaat tggtttaaac ttttatgcgc 1500

aaatacaaaa tgattgtctt tttcctatga ctcaaagatg aaagctgttt catttgtgtc 1560

agcatgtctc agattgacct taccaagttg gtcttacttt gttaatttat ctgttgtccc 1620

cttcctctcc tctgccctcc cttcttgtgc ccttaaaacc aaaccctatg ccttttgtag 1680

ctgtcatggt gcaatttgtc tttggaaaat tcagataatg gtaatttagt gtatatgtga 1740

ttttcaaata tgtaaacttt aacttccact ttgtataaat ttttaagtgt cagactatcc 1800

attttacact tgctttattt ttcattacct gtagctttgg gcagatttgc aacagcaaat 1860

taatgtgtaa aattggatta ttactacaaa accgtttagt catatctatc taatcagatc 1920

ttcttttggg aggatttgat gtaagttact gacaagcctc agcaaaccca aagatgttaa 1980

cagtatttta agaagttgct gcagattcct ttggccactg tatttgttaa tttcttgcaa 2040

tttgaaggta cgagtagagg tttaaagaaa aatcagtttt tgttcttaaa aatgcattta 2100

agttgtaaac gtctttttaa gcctttgaag tgcctctgat tctatgtaac ttgttgcaga 2160

ctggtgttaa tgagtatatg taacagttta aaaaaaaagt tggtatttta taagcacaga 2220

caattctaat ggtaactttt gtagtcttat gaatagacat aaattgtaat ttgggaacat 2280

aaaaactact gaataaatca tgtggcctaa tattgaaaat gtcactgtta taaattttgt 2340

acatttttga tcaaatgtac atctcccctt tgctaacggc cgtctgctct caaggatgac 2400

gtgggtttga tttctaagtg tttcacagtg tctgtaaatc aagaccaaag agcctgtcga 2460

tgagactgtt tattaccaga ttcacttctg aattggccag aggaaatctg aatgtattat 2520

cctgtgtgtg tctaggtaga gatattggaa ggctgccagg ggatttcgaa gtttgcaacc 2580

tttataggat aactgatggc aatattaaga cagacgcctg cttttgcaaa taacttacaa 2640

gactgtaaat tccaaagatc tgaatggggc tttcctgatg ttggtatcta aggcttaggc 2700

ctatagattg atttaccttt ggaattgtgc tccaaatgtc tactgaagct taaccgaaga 2760

actaataaat ggactacagt agctcacgtt acaggaaggg agggtaggca gggaggctct 2820

gtgtgttaaa atgagggtct cactgcttta ggattgaagt ggctggaaag agtgatgcct 2880

ggggaaggag atggagttat gagggtactg tggctggtac tttctgtact aaacatttcc 2940

tttttctatt ttaccactaa ttttgtttta aactgtgagc cgtccaagtc agaagaagac 3000

agcaaaaaaa gcaacttttc caacatacaa tttactttta ataaagtatg aatatttcat 3060

tttgagaaca ttccctggaa ttgccacata attcattaaa aacatttttt taagcaacac 3120

ttggaacagt gtttacttta aatccttaat ggccttaatt aattctcaga ttcctgcccc 3180

atcacttaca gaaccaattc actttagagt gactaaaagg aaacgatagc ctagctttct 3240

aaagccacgc tgtgtccctc aattacagag ggtaggaatg ggtatacctc taactgtgca 3300

aagcagagtg aaattcaatt catagaataa caactgctgg gaatatccgt gccaggaaaa 3360

gaaaaatttc tggcaaatat tttgtcactg ctgtaaagca aaatatttgt gaaagtgcca 3420

aaataaagtc tgtcatgcca aaagtaaatc attgtataga ctgacatcca gttttcttca 3480

actgt 3485

<210> 63
<211> 3961
<212> DNA
<213> Homo sapiens

<400> 63
gaggtcttcc tgtttttgga gggggcaggc accccagggc cgattgtcgc gaatcacctt 60

cacctctggg ggctcagaat ccctgagcca gaaggccagg gcagggctgg gggatacccc 120

tgctgctcag acaggaaaat gggctcggag cttgcccaat acttcccaga aggacgaggc 180

tgctgaactt ctcattcggg gctccgggac ctggactgta cccctttctg gcgtcacctc 240

ctcctgtcgc ctggccctcg ccatgcagac cccgcgagcg tcccctcccc gcccggccct 300

gctgcttctg ctgctgctac tggggggcgc ccacggcctc tttcctgagg agccgccgcc 360

gcttagcgtg gcccccaggg actacctgaa ccactatccc gtgtttgtgg gcagcgggcc 420

cggacgcctg acccccgcag aaggtgctga cgacctcaac atccagcgag tcctgcgggt 480

caacaggacg ctgttcattg gggacaggga caacctctac cgcgtagagc tggagccccc 540

cacgtccacg gagctgcggt accagaggaa gctgacctgg agatctaacc ccagcgacat 600

aaacgtgtgt cggatgaagg gcaaacagga gggcgagtgt cgaaacttcg taaaggtgct 660

gctccttcgg gacgagtcca cgctctttgt gtgcggttcc aacgccttca acccggtgtg 720

cgccaactac agcatagaca ccctgcagcc cgtcggagac aacatcagcg gtatggcccg 780

ctgcccgtac gaccccaagc acgccaatgt tgccctcttc tctgacggga tgctcttcac 840

agctactgtt accgacttcc tagccattga tgctgtcatc taccgcagcc tcggggacag 900

gcccaccctg cgcaccgtga aacatgactc caagtggttc aaagagcctt actttgtcca 960

tgcggtggag tggggcagcc atgtctactt cttcttccgg gagattgcga tggagtttaa 1020

ctacctggag aaggtggtgg tgtcccgcgt ggcccgagtg tgcaagaacg acgtgggagg 1080

ctccccccgc gtgctggaga agcagtggac gtccttcctg aaggcgcggc tcaactgctc 1140

tgtacccgga gactcccatt tctacttcaa cgtgctgcag gctgtcacgg gcgtggtcag 1200

cctcgggggc cggcccgtgg tcctggccgt tttttccacg cccagcaaca gcatccctgg 1260

ctcggctgtc tgcgcctttg acctgacaca ggtggcagct gtgtttgaag gccgcttccg 1320

agagcagaag tcccccgagt ccatctggac gccggtgccg gaggatcagg tgcctcgacc 1380

ccggcccggg tgctgcgcag cccccgggat gcagtacaat gcctccagcg ccttgccgga 1440

tgacatcctc aactttgtca agacccaccc tctgatggac gaggcggtgc cctcgctggg 1500

ccatgcgccc tggatcctgc ggaccctgat gaggcaccag ctgactcgag tggctgtgga 1560

cgtgggagcc ggcccctggg gcaaccagac cgttgtcttc ctgggttctg aggcggggac 1620

ggtcctcaag ttcctcgtcc ggcccaatgc cagcacctca gggacgtctg ggctcagtgt 1680

cttcctggag gagtttgaga cctaccggcc ggacaggtgt ggacggcccg gcggtggcga 1740

gacagggcag cggctgctga gcttggagct ggacgcagct tcggggggcc tgctggctgc 1800

cttcccccgc tgcgtggtcc gagtgcctgt ggctcgctgc cagcagtact cggggtgtat 1860

gaagaactgt atcggcagtc aggaccccta ctgcgggtgg gcccccgacg gctcctgcat 1920

cttcctcagc ccgggcacca gagccgcctt tgagcaggac gtgtccgggg ccagcacctc 1980

aggcttaggg gactgcacag gactcctgcg ggccagcctc tccgaggacc gcgcggggct 2040

ggtgtcggtg aacctgctgg taacgtcgtc ggtggcggcc ttcgtggtgg gagccgtggt 2100

gtccggcttc agcgtgggct ggttcgtggg cctccgtgag cggcgggagc tggcccggcg 2160

caaggacaag gaggccatcc tggcgcacgg ggcgggcgag gcggtgctga gcgtcagccg 2220

cctgggcgag cgcagggcgc agggtcccgg gggccggggc ggaggcggtg gcggtggcgc 2280

cggggttccc ccggaggccc tgctggcgcc cctgatgcag aacggctggg ccaaggccac 2340

gctgctgcag ggcgggcccc acgacctgga ctcggggctg ctgcccacgc ccgagcagac 2400

gccgctgccg cagaagcgcc tgcccactcc gcacccgcac ccccacgccc tgggcccccg 2460

cgcctgggac cacggccacc ccctgctccc ggcctccgct tcatcctccc tcctgctgct 2520

ggcgcccgcc cgggcccccg agcagccccc cgcgcctggg gagccgaccc ccgacggccg 2580

cctctatgct gcccggcccg gccgcgcctc ccacggcgac ttcccgctca ccccccacgc 2640

cagcccggac cgccggcggg tggtgtccgc gcccacgggc cccttggacc cagcctcagc 2700

cgccgatggc ctcccgcggc cctggagccc gcccccgacg ggcagcctga ggaggccact 2760

gggcccccac gcccctccgg ccgccaccct gcgccgcacc cacacgttca acagcggcga 2820

ggcccggcct ggggaccgcc accgcggctg ccacgcccgg ccgggcacag acttggccca 2880

cctcctcccc tatggggggg cggacaggac tgcgcccccc gtgccctagg ccgggggccc 2940

cccgatgcct tggcagtgcc agccacggga accaggagcg agagacggtg ccagaacgcc 3000

ggggcccggg gcaactccga gtgggtgctc aagtcccccc cgcgacccac ccgcggagtg 3060

gggggccccc tccgccacaa ggaagcacaa ccagctcgcc ctccccctac ccggggccgc 3120

aggacgctga gacggtttgg gggtgggtgg gcgggaggac tttgctatgg atttgaggtt 3180

gaccttatgc gcgtaggttt tggttttttt tgcagttttg gtttcttttg cggttttcta 3240

accaattgca caactccgtt ctcggggtgg cggcaggcag gggaggcttg gacgccggtg 3300

gggaatgggg ggccacagct gcagacctaa gccctccccc acccctggaa aggtccctcc 3360

ccaacccagg cccctggcgt gtgtgggtgt gcgtgcgtgt gcgtgccgtg ttcgtgtgca 3420

aggggccggg gaggtgggcg tgtgtgtgcg tgccagcgaa ggctgctgtg ggcgtgtgtg 3480

tcaagtgggc cacgcgtgca gggtgtgtgt ccacgagcga cgatcgtggt ggccccagcg 3540

gcctgggcgt tggctgagcc gacgctgggg cttccagaag gcccgggggt ctccgaggtg 3600

ccggttagga gtttgaaccc cccccactct gcagagggaa gcggggacaa tgccggggtt 3660

tcaggcagga gacacgagga gggcctgccc ggaagtcaca tcggcagcag ctgtctaaag 3720

ggcttggggg cctggggggc ggcgaaggtg ggtggggccc ctctgtaaat acggccccag 3780

ggtggtgaga gagtcccatg ccacccgtcc ccttgtgacc tcccccctct gacctccagc 3840

tgaccatgca tgccacgtgg ctggctgggt cctctgccct ctctggagtt tgcctccccc 3900

agccccctcc ccatcaataa aactctgttt acaaccaccg gcaaaaaaaa aaaaaaaaaa 3960

a 3961

<210> 64
<211> 4201
<212> DNA
<213> Homo sapiens

<400> 64
tccggcccgc acccaccccc aagaggggcc ttcagctttg gggctcagag gcacgacctc 60

ctggggaggg ttaaaaggca gacgcccccc cgccccccgc gcccccgcgc cccgactcct 120

tcgccgcctc cagcctctcg ccagtgggaa gcggggagca gccgcgcggc cggagtccgg 180

aggcgagggg aggtcggccg caacttcccc ggtccacctt aagaggacga tgtagccagc 240

tcgcagcgct gaccttagaa aaacaagttt gcgcaaagtg gagcggggac ccggcctctg 300

ggcagccccg gcggcgcttc cagtgccttc cagccctcgc gggcggcgca gccgcggccc 360

atggagcccg ccggcccggc ccccggccgc ctcgggccgc tgctctgcct gctgctcgcc 420

gcgtcctgcg cctggtcagg agtggcgggt gaggaggagc tgcaggtgat tcagcctgac 480

aagtccgtgt tggttgcagc tggagagaca gccactctgc gctgcactgc gacctctctg 540

atccctgtgg ggcccatcca gtggttcaga ggagctggac caggccggga attaatctac 600

aatcaaaaag aaggccactt cccccgggta acaactgttt cagacctcac aaagagaaac 660

aacatggact tttccatccg catcggtaac atcaccccag cagatgccgg cacctactac 720

tgtgtgaagt tccggaaagg gagccccgat gacgtggagt ttaagtctgg agcaggcact 780

gagctgtctg tgcgcgccaa accctctgcc cccgtggtat cgggccctgc ggcgagggcc 840

acacctcagc acacagtgag cttcacctgc gagtcccacg gcttctcacc cagagacatc 900

accctgaaat ggttcaaaaa tgggaatgag ctctcagact tccagaccaa cgtggacccc 960

gtaggagaga gcgtgtccta cagcatccac agcacagcca aggtggtgct gacccgcgag 1020

gacgttcact ctcaagtcat ctgcgaggtg gcccacgtca ccttgcaggg ggaccctctt 1080

cgtgggactg ccaacttgtc tgagaccatc cgagttccac ccaccttgga ggttactcaa 1140

cagcccgtga gggcagagaa ccaggtgaat gtcacctgcc aggtgaggaa gttctacccc 1200

cagagactac agctgacctg gttggagaat ggaaacgtgt cccggacaga aacggcctca 1260

accgttacag agaacaagga tggtacctac aactggatga gctggctcct ggtgaatgta 1320

tctgcccaca gggatgatgt gaagctcacc tgccaggtgg agcatgacgg gcagccagcg 1380

gtcagcaaaa gccatgacct gaaggtctca gcccacccga aggagcaggg ctcaaatacc 1440

gccgctgaga acactggatc taatgaacgg aacatctata ttgtggtggg tgtggtgtgc 1500

accttgctgg tggccctact gatggcggcc ctctacctcg tccgaatcag acagaagaaa 1560

gcccagggct ccacttcttc tacaaggttg catgagcccg agaagaatgc cagagaaata 1620

acacaggaca caaatgatat cacatatgca gacctgaacc tgcccaaggg gaagaagcct 1680

gctccccagg ctgcggagcc caacaaccac acggagtatg ccagcattca gaccagcccg 1740

cagcccgcgt cggaggacac cctcacctat gctgacctgg acatggtcca cctcaaccgg 1800

acccccaagc agccggcccc caagcctgag ccgtccttct cagagtacgc cagcgtccag 1860

gtcccgagga agtgaatggg accgtggttt gctctagcac ccatctctac gcgctttctt 1920

gtcccacagg gagccgccgt gatgagcaca gccaacccag ttcccggagg gctggggcgg 1980

tgcaggctct gggacccagg ggccagggtg gctcttctct ccccacccct ccttggctct 2040

ccagcacttc ctgggcagcc acggccccct ccccccacat tgccacatac ctggaggctg 2100

acgttgccaa accagccagg gaaccaacct gggaagtggc cagaactgcc tggggtccaa 2160

gaactcttgt gcctccgtcc atcaccatgt gggttttgaa gaccctcgac tgcctccccg 2220

atgctccgaa gcctgatctt ccagggtggg gaggagaaaa tcccacctcc cctgacctcc 2280

accacctcca ccaccaccac caccaccacc accaccacta ccaccaccac ccaactgggg 2340

ctagagtggg gaagatttcc cctttagatc aaactgcccc ttccatggaa aagctggaaa 2400

aaaactctgg aacccatatc caggcttggt gaggttgctg ccaacagtcc tggcctcccc 2460

catccctagg ctaaagagcc atgagtcctg gaggaggaga ggacccctcc caaaggactg 2520

gagacaaaac cctctgcttc cttgggtccc tccaagactc cctggggccc aactgtgttg 2580

ctccacccgg acccatctct cccttctaga cctgagcttg cccctccagc tagcactaag 2640

caacatctcg ctgtggacgc ctgtaaatta ctgagaaatg tgaaacgtgc aatcttgaaa 2700

ctgaggtgtt agaaaacttg atctgtggtg ttttgttttg ttttttttct taaaacaaca 2760

gcaacgtgat cttggctgtc tgtcatgtgt tgaagtccat ggttgggtct tgtgaagtct 2820

gaggtttaac agtttgttgt cctggaggga ttttcttaca gcgaagactt gagttcctcc 2880

aagtcccaga accccaagaa tgggcaagaa ggatcaggtc agccactccc tggagacaca 2940

gccttctggc tgggactgac ttggccatgt tctcagctga gccacgcggc tggtagtgca 3000

gccttctgtg accccgctgt ggtaagtcca gcctgcccag ggctgctgag ggctgcctct 3060

tgacagtgca gtcttatcga gacccaatgc ctcagtctgc tcatccgtaa agtggggata 3120

gtgaagatga cacccctccc caccacctct cataagcact ttaggaacac acagagggta 3180

gggatagtgg ccctggccgt ctatcctacc cctttagtga ccgcccccat cccggctttc 3240

tgagctgatc cttgaagaag aaatcttcca tttctgctct caaaccctac tgggatcaaa 3300

ctggaataaa ttgaagacag ccagggggat ggtgcagctg tgaagctcgg gctgattccc 3360

cctctgtccc agaaggttgg ccagagggtg tgacccagtt accctttaac cccacccctt 3420

ccagtcgggt gtgagggcct gaccgggccc agggcaagca gatgtcgcaa gccctattta 3480

ttcagtcttc actataactc ttagagttga gacgctaatg ttcatgactc ctggccttgg 3540

gatgcccaag ggatttctgg ctcaggctgt aaaagtagct gagccatcct gcccattcct 3600

ggaggtccta caggtgaaac tgcaggagct cagcatagac ccagctctct gggggatggt 3660

cacctggtga tttcaatgat ggcatccagg aattagctga gccaacagac catgtggaca 3720

gctttggcca gagctcccgt gtggcatctg ggagccacag tgacccagcc acctggctca 3780

ggctagttcc aaattccaaa agattggctt gtaaaccttc gtctccctct cttttaccca 3840

gagacagcac atacgtgtgc acacgcatgc acacacacat tcagtatttt aaaagaatgt 3900

tttcttggtg ccattttcat tttattttat tttttaattc ttggaggggg aaataaggga 3960

ataaggccaa ggaagatgta tagctttagc tttagcctgg caacctggag aatccacata 4020

ccttgtgtat tgaaccccag gaaaaggaag aggtcgaacc aaccctgcgg aaggagcatg 4080

gtttcaggag tttattttaa gactgctggg aaggaaacag gccccatttt gtatatagtt 4140

gcaacttaaa ctttttggct tgcaaaatat ttttgtaata aagatttctg ggtaataatg 4200

a                                                                    4201

<210>  65
<211>  3333
<212>  DNA
<213>  Homo sapiens

<400>  65
gtggccgcgg ggcggtgtca tcgcccccgc cccgcccggt ccagccagct cggcccgggg      60

gcttcgggct gtcgggccgg cgctcccttc tctgccaggt ggcgagtaca cctgctcacg     120

taggcgtcat gaggtctccg gttcgagacc tggcccggaa cgatggcgag gagagcacgg     180

accgcacgcc tcttctaccg ggcgccccac gggccgaagc cgctccagtg tgctgctctg     240

ctcgttacaa cttagcaatt ttggcctttt ttggtttctt cattgtgtat gcattacgtg     300

tgaatctgag tgttgcgtta gtggatatgg tagattcaaa tacaacttta gaagataata     360

gaacttccaa ggcgtgtcca gagcattctg ctcccataaa agttcatcat aatcaaacgg     420

gtaagaagta ccaatgggat gcagaaactc aaggatggat tctcggttcc tttttttatg     480

gctacatcat cacacagatt cctggaggat atgttgccag caaaataggg gggaaaatgc     540

tgctaggatt tgggatcctt ggcactgctg tcctcaccct gttcactccc attgctgcag     600

atttaggagt tggaccactc attgtactca gagcactaga aggactagga gagggtgtta     660

catttccagc catgcatgcc atgtggtctt cttgggctcc ccctcttgaa agaagcaaac     720

ttcttagcat ttcatatgca ggagcacagc ttgggacagt aatttctctt cctctttctg     780

gaataatttg ctactatatg aattggactt atgtcttcta cttttttggt actattggaa     840

tattttggtt tcttttgtgg atctggttag ttagtgacac accacaaaaa cacaagagaa     900

tttcccatta tgaaaaggaa tacattcttt catcattaag aaatcagctt tcttcacaga     960

agtcagtgcc gtgggtaccc attttaaaat ccctgccact ttgggctatc gtagttgcac    1020

acttttctta caactggact ttttatactt tattgacatt attgcctact tatatgaagg    1080

agatcctaag gttcaatgtt caagagaatg ggtttttatc ttcattgcct tatttaggct    1140

cttggttatg tatgatcctg tctggtcaag ctgctgacaa tttaagggca aaatggaatt    1200

tttcaacttt atgtgttcgc agaattttta gccttatagg aatgattgga cctgcagtat    1260

tcctggtagc tgctggcttc attggctgtg attattcttt ggccgttgct ttcctaacta    1320

tatcaacaac actgggaggc ttttgctctt ctggatttag catcaaccat ctggatattg    1380

ctccttcgta tgctggtatc ctcctgggca tcacaaatac atttgccact attccaggaa    1440

tggttgggcc cgtcattgct aaaagtctga cccctgataa cactgttgga gaatggcaaa    1500

ccgtgttcta tattgctgct gctattaatg tttttggtgc cattttcttt acactattcg    1560

ccaaaggtga agtacaaaac tgggctctca atgatcacca tggacacaga cactgaagga    1620

accaataaat aatcctgcct ctattaatgt atttttattt atcatgtaac ctcaaagtgc 1680

cttctgtatt gtgtaagcat tctatgtctt tttttaattg tacttgtatt agatttttaa 1740

ggcctataat catgaaatat cactagttgc cagaataata aaatgaactg tgtttaatta 1800

tgaataatat gtaagctagg acttctactt taggttcaca tacctgcctg ctagtcgggc 1860

aacatgaagt aggacagttc tgttgatttt ttagggccat actaaaggga atgagctgaa 1920

acagacctcc tgataccttt gcttaattaa actagatgat aattctcagg tactgataaa 1980

cacctgttgt tgttcacttt cctcataaaa attgtcagct ctctctgaca cttagacctc 2040

aaactttagc atctctgtgg agctgccatc cactgtataa tttcgcctgg caactggact 2100

gaggggagtg tgcccaggca gctgccaagc actccctccc tggcttcagg gtcagagtgc 2160

ccagcgttta tcagaggcag catccaagcc cagagccagt gtcgactctt cggctggtgc 2220

ctttcctctg aggggctatc aatgtgtaga taaagccctg agtaggcaag agcagtgaga 2280

tccactgcta tggtcttgat acatcctcaa actttccctt cccagcacag aggaatattg 2340

gctggcatgc aacctgcaaa agaaaaatgc gaagcggccg ggcacggtgg ctcatgcctg 2400

taatcccagc actttggggg gctgaggtgg gcgaatcatg agatcaggag ttcgagacca 2460

gcctggccag catggtgaaa ccccatctct actaaaaata caaaaaatta gctgggcgtg 2520

gtgacgggcg cctgtaatcc cagatactca ggaggctgag gtaggagaat cacttgaacc 2580

tgggaggtgg aagttgcagt gaaccaagat cacgccactg cactccagcc tgggcgatgg 2640

agcgagactc caactcaaaa aaaaaaaaag aataaagaaa gaaaagtgcg atgcccagtc 2700

aatcacaaat aagatcatcc tggtttaaat ctactctcac atggatcaca gtataaattt 2760

ctatgtgctg tgttttgttc gtttgtattt tgtagagatg gggtctcgtt ttgtcgccca 2820

ggctggtttt gaactcctgg cttcaagcga tcctcctgtc tcggcctcac aaagtgttga 2880

gactacaggc atgagccact gtgcccagcc tgttctatgt ttttaagcta cacgagaatt 2940

ttttttttaa ttaattctca ctgtttgttc agtctgtctt catctaagtt tgtgttgcag 3000

tttaaagtta aagtgacttt taaaggccac atcacctgag actagggtaa tcatctttac 3060

ttctggttcc tgaaatcata tttttccagt ggaccatcct ccagtggctg tggttgttga 3120

gcatgctttc agaacaccta tgtggcttaa aacttagttt atgttttgtg ttcaacacta 3180

cgtgtaatat tttaaaactg tttaatgtga tgtgaataca tttatgtaca tttattttta 3240

aatttgtaaa tagctttaaa ttgctatggc aatgtttctt ttataaatca tcaaaataaa 3300

cctttgtgaa ttgaaaaaaa aaaaaaaaaa aaa 3333

<210> 66
<211> 3743
<212> DNA

<213>  Homo sapiens

<400>  66
tttcttctag aagagctttg cgtcttaaac gcccataccg cagacagctg cctggcgggc      60

gacccagccc catcctgatg gccccaggac acaagcccag gagcgccccg gtgcgcgcgg     120

ttccgcggcc caggggcgcc gggtttggtg gcacagcgag ccccctttct ctagcgaaac      180

ctgtttccct ccaatcttgt ggttgcagct ttccgtacgt atgcaaccga ttataaagtc     240

gtgacccaga acagcagctc tggaaatgta acccatgaaa agatccccat aggcactgag     300

atagaaggga tgaacatttt aggattggtc ctgtttgctc tggtgttagg agtggcctta     360

aagaaactag gctccgaagg agaagacctc atccgtttct tcaattccct caacgaggcg     420

acgatggtgc tggtgtcctg gattatgtgc tcagcgaccc ttccctctat gatgaagtgc     480

attgaagaga acaatggtgt ggacaagagg atcagcaggt ttattctccc catcggggcc     540

accgtgaaca tggacggagc agccatcttc cagtgtgtgg ccgcggtgtt cattgcgcaa     600

ctcaacaacg tagagctcaa cgcaggacag attttcacca ttctagtgac tgccacagcg     660

tccagtgttg gagcagcagg cgtgccagct ggaggggtcc tcaccattgc cattatcctg      720

gaggccattg ggctgcctac tcatgacctg cctctgatcc tggctgtgga ctggattgtg     780

gaccggacca ccacggtggt gaatgtggaa ggggatgccc tgggtgcagg cattctccac     840

cacctgaatc agaaggcaac aaagaaaggc gagcaggaac ttgctgaggt gaaagtggaa     900

gccatcccca actgcaagtc tgaggaggag acatcgcccc tggtgacaca ccagaacccc     960

gctggccccg tggccagtgc cccagaactg gaatccaagg agtcggttct gtgatggggc    1020

tgggctttgg gcttgcctgc cagcagtgat gtcccaccct gttcacccag ccgccagtca    1080

tggacacagg gcactgccct tgccaacttt taccctccca agcaatgctt tggcccagtc    1140

gctggcctga ggcttacctc tcggcactgg cattgggctc cccagccgga actggttacc    1200

aaggacaagg acactctgac attcggcttg atccatgtcc aggtgcaact gtgtgtacac    1260

cagggatctg tttggaaaca accccttgag ctgccaggct caagaaatca tggactcaca    1320

gggtcctgtg tggttacatc ttggaaaaaa tgcagatgta tttcactctc cccggtcagc    1380

tctgcatcag gtgttttctg agcaaaccaa gggggtttat agtcatctgt cgcattgcct    1440

cgagttgcag taattgaaaa aatgctcaaa ttcttagcca tggctggcct ttgctgagct    1500

gggactcagg tgtttaaaga gtttgtgcta tagctaggtg tggatagctt ctgatccctg    1560

ggttctggga gactgcaggt gccgcacatt gtcaagttag aaatactcca ggtgggtgtt    1620

agcactgtgg tggtctctgg tccacagcct taggtaaaca acttagattc tgaggtcaaa    1680

gaaaaaagga gagggaatgc agccttgtgg gggagaagcg gggcagaggg ttctctaatc    1740

taatcaggac aggacaggtt tcacatacaa ttgtcccagt tcgcatccca gccctggggc    1800

acttttctgc ttccttccag aggcctgggc ctctgataac actttggctt tttctccatt 1860

cacgctgatt tggcaaaagg ccagagatgg gcctccttcc ctggggaggt gtgatgtagt 1920

tatcacattc aggacccttg ttgatttatc atctattatt tgaattcaac tggacactct 1980

gtaaaatgct gcactgcagc aaaaacaaaa ccaccaccac cccagagaaa accatgtact 2040

aattggagtg gggtaccccc attcacaggt tcccaggtcc cctggctttg gctgatttca 2100

aaatatagag ccctttcttg ccagtacatc caagtttaaa attatcagcg aaatggtcca 2160

tgtttttcca attacctgct gacacggttc taagctaagt gaaggggaag atctgagagc 2220

gtgctgtttg tggctgttga tgcatattcg tgatgtaaca ggtcctgggg cctcacttta 2280

ccccatttgt aaaatggggc taatgtcacc tgcctcttac ctacctcaga gggatttggt 2340

gaagcaaact gttaatcttc gaaaacgacc atttcacttc ttggatatca agtgctaacc 2400

cagtatgttc ttctttttta tgtaagggac agctttctcc acagagtcct ttctgctggt 2460

gaggacagca tttctgagca gggctttgtt ctctatgtgc attaggactt ttatcatgcc 2520

cttgttctgt gtgtagttac ttgacagcat caaatgccgc ctcttcctaa tgtccttcaa 2580

gttttcatga actagcaacc ccaccttcca ccatggttct gggcgcctga ttttgctgtg 2640

actcccagac ccagccactg tttctgccac cctgtaacag gccattaaag ctccccagtg 2700

ttcagcctcc ttcactccct tgttttccct gttgctatgt gtcacctggg ccctacagac 2760

aggggcacac gcttatggat gtgtgtacca ttgagatgag aatgggtaga tggaacggag 2820

accatcaagc cacaccccct tcttaaaact ggggacatga gcctgagcag aaagggtgaa 2880

gaagagccat gggacacaga gttgacccag ccagggggaa agcccagctc tctttaaacc 2940

agctaagcca ttccagtctc ctgtgaagcc aaaagggacc aggaaccgtg caaaggaaac 3000

tggaaacttt tccccgctgg gtagagcatg ttgctgatac tcttctgttt tcaagggaaa 3060

caatcacatt gtttgattcc aaatggtaaa tgaacactca ctattcttca ggcttcagta 3120

aatctttttt tcttccttca tatatatata cacaacacac acacacatat gtatatctat 3180

acacacatgt gtgttgtgta tatgcatgtg tgtgtgtgcg tgtgtgtata gttttagctc 3240

caagccaagc aagtttgtgt ttggatagag gggaacttaa ctattaacta caagttgtat 3300

gtctgtggta tcttgatttt cccatttcta aagatgaatt tcacaaagcc ataaagcgtg 3360

aaattagagc tggacttaag actcattggc cgaccatcct gtgtcctggc ctggccctgc 3420

agtaagaagc gtgtctgggt ctggagaagg gtgcttccga gagtgtgcag gtggcccttc 3480

cccttggagg cgagaagaga gaatgtgctg tctatcttcc tggttttcag tccacagagt 3540

cggtagacca ggggttacgt gactggggaa aatctcacat ctccttgtct gaaaacattt 3600

cccctgctgt tctctttcta acatgttgtg gtaaatctgt tcagatactg ctcatctgac 3660

tgttttgtac atgtgacaat tgccttaaaa cctagcacag tcctcagaaa tgaataccgt 3720

gtttccactg gaaaaaaaaa aaa                                          3743

<210>  67
<211>  2214
<212>  DNA
<213>  Homo sapiens

<400>  67
cctgtttccc aggaacggtc cccggcttcg cgccccaatt tctaacagcc tgcctgtccc      60

ccgggaacgt tctaacatcc ttggggagcg ccccagctac aagacactgt cctgagaacg     120

ctgtcatcac ccgtagttgc aagtttcgga gcggcagtgg gaagcatgcg ggactacgac     180

gaggtgatcg ccttcctggg cgagtggggg cccttccagc gcctcatctt cttcctgctc     240

agcgccagca tcatccccaa tggcttcaat ggtatgtcag tcgtgttcct ggcggggacc     300

ccggagcacc gctgtcgagt gccggacgcc gcgaacctga gcagcgcctg gcgcaacaac     360

agtgtcccgc tgcggctgcg ggacggccgc gaggtgcccc acagctgcag ccgctaccgg     420

ctcgccacca tcgccaactt ctcggcgctc gggctggagc cggggcgcga cgtggacctg     480

gggcagctgg agcaggagag ctgcctggat ggctgggagt tcagccagga cgtctacctg     540

tccaccgtcg tgaccgagtg gaatctggtg tgtgaggaca actggaaggt gcccctcacc     600

acctccctgt tcttcgtagg cgtgctcctc ggctccttcg tgtccgggca gctgtcagac     660

aggtttggca ggaagaacgt tctcttcgca accatggctg tacagactgg cttcagcttc     720

ctgcagattt tctccatcag ctgggagatg ttcactgtgt tatttgtcat cgtgggcatg     780

ggccagatct ccaactatgt ggtagccttc atactaggaa cagaaattct tggcaagtca     840

gttcgtatta tattctctac attaggagtg tgcacatttt ttgcagttgg ctatatgctg     900

ctgccactgt ttgcttactt catcagagac tggcggatgc tgctgctggc gctgacggtg     960

ccgggagtgc tgtgtgtccc gctgtggtgg ttcattcctg aatctccccg atggctgata    1020

tcccagagaa gatttagaga ggctgaagat atcatccaaa aagctgcaaa aatgaacaac    1080

atagctgtac cagcagtgat atttgattct gtggaggagc taaatcccct gaagcagcag    1140

aaagctttca ttctggacct gttcaggact cggaatattg ccataatgac cattatgtct    1200

ttgctgctat ggatgctgac ctcagtgggt tactttgctc tgtctctgga tgctcctaat    1260

ttacatggag atgcctacct gaactgtttc ctctctgcct tgattgaaat tccagcttac    1320

attacagcct ggctgctatt gcgaaccctg cccaggcgtt atatcatagc tgcagtactg    1380

ttctggggag gaggtgtgct tctcttcatt caactggtac ctgtggatta ttacttctta    1440

tccattggtc tggtcatgct gggaaaattt gggatcacct ctgctttctc catgctgtat    1500

gtcttcactg ctgagctcta cccaaccctg gtcaggaaca tggcggtggg ggtcacatcc    1560

acggcctcca gagtgggcag catcattgcc ccctactttg tttacctcgg tgcttacaac    1620

agaatgctgc cctacatcgt catgggtagt ctgactgtcc tgattggaat cctcaccctt 1680

tttttccctg aaagtttggg aatgactctt ccagaaacct tagagcagat gcagaaagtg 1740

aaatggttca gatctgggaa aaaaacaaga gactcaatgg agacagaaga aaatcccaag 1800

gttctaataa ctgcattctg aaaaaatatc taccccattt ggtgaagtga aaaacagaaa 1860

aataagaccc tgtggagaaa ttcgttgttc ccactgaaat ggactgactg taacgattga 1920

caccaaaatg aaccttgcta tcaagaaatg ctcgtcatac agtaaactct ggatgattct 1980

tccagataat gtccttgctt tacaaaccaa ccatttctag agagtctcct tactcattaa 2040

ttcaatgaaa tggattggta agatgtcttg aaaacatgtt agtcaaggac tggtaaaata 2100

catataaaga ttaacactca tttccaatca tacaaatact atccaaataa aaataacatc 2160

attgtattaa cgcaaatatt aggtgacaac aaaaaaaaaa aaaaaaaaaa aaaa 2214

<210> 68
<211> 4797
<212> DNA
<213> Homo sapiens

<400> 68
ggcgggaggg ggcgggaaat cctcggcctc ggtggcggtg gtggacacgt cgagccgggt 60

agaagtggag gggccgttcg aagagtcgtg agggggtgac gggttaagat tcggagagag 120

aggtgctagt ggctggactt gacctggaaa gaatcttctg ctgactctca acttttcctg 180

gaaaaaatgg atcattccca ccatatgggg atgagctata tggactccaa cagtaccatg 240

caaccttctc accatcaccc aaccacttca gcctcacact cccatggtgg aggagacagc 300

agcatgatga tgatgcctat gaccttctac tttggcttta agaatgtgga actactgttt 360

tccggtttgg tgatcaatac agctggagaa atggctggag cttttgtggc agtgttttta 420

ctagcaatgt tctatgaagg actcaagata gcccgagaga gcctgctgcg taagtcacaa 480

gtcagcattc gctacaattc catgcctgtc ccaggaccaa atggaaccat ccttatggag 540

acacacaaaa ctgttgggca acagatgctg agctttcctc acctcctgca aacagtgctg 600

cacatcatcc aggtggtcat aagctacttc ctcatgctca tcttcatgac ctacaacggg 660

tacctctgca ttgcagtagc agcaggggcc ggtacaggat acttcctctt cagctggaag 720

aaggcagtgg tagtggatat cacagagcat tgccattgac atcaaactct atggcgtggc 780

cttatcgatt gcagtgggaa gttgttgaag acttgaagac gtgattcctg ctccaatcat 840

cccttcttgc tcctctttgt gcacgtacac acacacacac acacacacac acacacaccc 900

ctgctcaaca gaggtttagt ttacagtctc tgaactaaag tagtaacctc ccaaattgtt 960

ttttctaata agctgagatt cccatttctc ttaaggagaa gccacccatg agatgtcttt 1020

tccttctcca tcatcttaga gccaagttat atgttcttgt ctaatccatg tagctttttg 1080

ttcaatgact tgatcatctg cttccttttt gaatttttaa cagatagtaa gtaaatttgg 1140

tggttttttc ccctgggtca gtgatggaaa ggggttaact tcagccagga ttgatggcag 1200

ctgagggaaa ttcttgccca actaaaccca gaactcaaac ttaacattag aaaataaggt 1260

ccagggccgg acacagtggc ccatgcctgt aatcccagca ctttgggggg ccaaggcagg 1320

ctggatcacc tgaggacagg agttcgagac cagtctggcc aacatgggga aaccccgtct 1380

ctactaaaaa tacaaaaatt agccgggcat ggtggtgggc gcctgtaatc ccagctactc 1440

agaaggctga ggcaggagaa tcacttgaac ctaggaggcg gaggttgcag tgagccaaga 1500

tggcgccatt gcactccagc ctgggtgaca agagtgaaac tccatctcaa aaaaaaagaa 1560

aagaaggtcc agcttttgga ttcaatgagt gggaaataca ttgtgccttt ctctagatgt 1620

gatacgttat accaaaatct ttgtagtgtg cagagcggtg gtttgagact aaatacaggc 1680

ttagaacttg cagagtgtgt attcttggat ggctgatgca tcgacttgca ttcccactta 1740

acactttgat tagcatgaac ttgccaatca aaaaatgaca atcaatttga gaaaatagaa 1800

atagatattt ttaaataaaa ccattcacag tttactttgt cttgatacct tggtttgtcc 1860

cagctgaagt gaagcaagag agtttgaatt aatttttcca ttataatgtt ttcgcatgtc 1920

tgcctctaaa actgtgattt tcaagcttta gcgtgcatca gaatcacctg tagggcttgt 1980

taaaacacta attgctgggc ctcaacccaa aagcccccaa agtggcactt ctgagttcct 2040

gctgatgtta caaggaccca cacttttgag aatctgtgct ttaagctaag gaaatattgc 2100

ctggtgggtt ggctgcctgg tattgggcat ggaaatttga attgctgatt ggtagatggt 2160

gtgtctggac ttaactcacg tagtaaatac tgctgatcaa tacctaatca ttccacattt 2220

attgagctcc acctgtgtgt atgtgtaccc aagcacacat gtgtgaaggg ctatagccaa 2280

agtattttta ctagcctgta tgaaatcact agtccttatt tttaaaggtc tatggtttct 2340

tggaagtagt ttgattgttg agagagacct ttgatctgca gtgtaaatct accagtcatg 2400

ggccagaagg gcaaaagccc agctttctct ttggaaagac tcaggctgtg gtttgttgat 2460

ggccaggttt tcctcaggct ccaacaactg tgcttatacc aagcagatcc tcatcctcca 2520

tataatcatc tttgttattc gtgggggttt aattacatta caagtggcca aaacccctgt 2580

tctcagtgaa gaaccacatt ggatttgtat tctgttcagt tgtagtctac actgcagtct 2640

tattcctggt tcaaactacc tctttaaatt gatttgtctt gtgctggtct gttaaatcct 2700

gccctccttg gtgctagatc cagttgtgcc tcagggcaga ggaaacaaaa cacagctatc 2760

ctgttggcct gtgttgtggt tttgaagttt gtactttctc tgtgggtgcc agttaaatat 2820

tggagagcaa ggaatgtgga cttgtatggc tttgaaccaa gagaggttta tgagcctact 2880

ggattgaggt taaaatccaa gaaccaacat ttagagcttt gtgcttttct ctcattccat 2940

cactttgtaa tgatgatact taacatgagc agggtgaatg acaggtactg acgaagtcca 3000

acacaaaggt ataatacagc ctgttgtcta aagccaagga gtcataaaac catgagaaat 3060

aaataggaat caatagttag tagtgacatt ggtgctctct agaaatctca gcatgagctg 3120

ctatagaata ccctcccagc aacaaaacct aatcagtaag gccagctaga cccaatggct 3180

catgcctgta atcccaacac tttgggaggc caaggtggga ggatggcttg tgtccagaag 3240

ttcgagacca gcctggacaa catagtgaga tcctatctct ataaaaaatc aaaaattagc 3300

caggcatggt ggtgcatacc tatagtcctg gctatttggg aggctgaggc aggaggattg 3360

ctttagccct ggaggtcgag gctgcagtaa gccatgattg cgccactgca ctcagcccgg 3420

gtgacaaagc aagaccctgt ctcagaaaaa aagaaaattc aaggccagtt aagacaaaat 3480

gctatgactt tgaaattcac agaaagaaat aacagtttag attaggtctt caggtattca 3540

ggatagagat aatctcctga aaaacctgaa tttcagagat tcttagactg gctgccaaag 3600

gatgaagcta gtgaaggaga aaaagcttaa attccatctt gagctcttgg attgtgataa 3660

tacaatgatt tcattaactt ttcatttctg tatacctgtt catttggaat ttaatgcttg 3720

acttctttgt tcattttgga tctaaacttc tcttttcttc cttccccatt cacatctatt 3780

agaagactgc atcaccattt ctttggcccc cttactctgt tgtcctttcc cttttctttc 3840

agttttttta atcgcatgtc tagtatatta agtctccata gccctcctga tgcagtagac 3900

agtgctatgc tgtggatata ataccaacca gaaattggca tttataaacc tgttaagaga 3960

ctttaagcat gcttcaagag gcagttgacc cactggaatt tctataaggc tggtaccctt 4020

cccagagtta cagaatctta ggtgccgtct ctagtctgtg agggaggaac tcccagcatc 4080

cccattgccc acaaatggaa tcctcactgt atccactagg agattagaaa ttaaggtttc 4140

ttcactactt ctatggtagg gttgtctgaa attccctttc aggctgtggg tactggtctt 4200

gggttctagt cataaggggt tccttataag gagcaggcgg aggggagtac actttcatgt 4260

gatttaattt tgatcctgcc ctctccagct gctccttcaa aagatacatc aaaagataga 4320

aactctgggc tgggcacagt ggctcacaca ctttgggagg ccaagcgggg gtgcagatca 4380

cctgaggtca ggagtttgag accagcctgg ccaatgtggt gaaaccccat ctctactaaa 4440

aatacaaaaa ttagctgggc gtggtggtgc atgcctgtaa tcccagctac tcgggaggct 4500

gaggcaggag aatcgcttga acccaggagg cggaggttgc agtgagccaa gatggcgcca 4560

ttgcactcca gcctgggcga caagagcaaa actccgtctc aaaaaaaaaa aaaagataca 4620

aagtctgcat ttgatataat gccttaatta ctgggtctac aattaatgtt gactgtttta 4680

gattgtaagc tcctggagag cagtattgct gtagtaggaa tgttttaaca gtgtcatatg 4740

aaaaagaaca aaataaatat tttgattttg tgattctaaa aaaaaaaaaa aaaaaaa 4797

<210>  69
<211>  2347
<212>  DNA
<213>  Homo sapiens

<400>  69
ggcgtgggac gtgctgcggc gtcctagctg gcttacaggg cggcggcggg gtgtgtgtcc      60

tctgttaaga gtgctactcg cccggggttg atctgtgcat gccactcctg ggtcagacgg     120

tgaggtcggc gtctgcgagg acgcggcggt ggagtagaag ggcagccgga gacaggcccg     180

gcgccccttc cgaggctaga cggccccagc ttcgcgggga tcatggcatt gctggtggac     240

cgagtgcggg gccactggcg aatcgccgcc gggctcctgt tcaacctgct ggtgtccatc     300

tgcattgtgt tcctcaacaa atggatttat gtgtaccacg gcttccccaa catgagcctg     360

accctggtgc acttcgtggt cacctggctg ggcttgtata tctgccagaa gctggacatc     420

tttgccccca aaagtctgcc gccctccagg ctcctcctcc tggccctcag cttctgtggc     480

tttgtggtct tcactaacct ttctctgcag aacaacacca taggcaccta tcagctggcc     540

aaggccatga ccacgccggt gatcatagcc atccagacct tctgctacca gaaaaccttc     600

tccaccagaa tccagctcac gctgattcct ataactttag gtgtaatcct aaattcttat     660

tacgatgtga agtttaattt ccttggaatg gtgtttgctg ctcttggtgt tttagttaca     720

tccctttatc aagtgtgggt aggagccaaa cagcatgaat tacaagtgaa ctcaatgcag     780

ctgctgtact accaggctcc gatgtcatct gccatgttgc tggttgctgt gcccttcttt     840

gagccagtgt ttggagaagg aggaatattt ggtccctggt cagtttctgc tttgcttatg     900

gtgctgctat ctggagtaat agctttcatg gtgaacttat caatttattg gatcattggg     960

aacacttcac ctgtcaccta taacatgttc ggacacttca agttctgcat tactttattc    1020

ggaggatatg ttttatttaa ggatccactg tccattaatc aggcccttgg cattttatgt    1080

acattatttg gcattctcgc ctatacccac tttaagctca gtgaacagga aggaagtagg    1140

agtaaactgg cacaacgtcc ttaattgggt ttttgtggag aaaagaatgt tgtcccaaga    1200

agataaaaaa tattgttaag tgtgcaagtt attaaaaaaa aaaaattggg ccaggcacgg    1260

tggctcacgc ctgtaatccc agcactttgg gaggccaagg ccagcggatc acttgaggtc    1320

aggagttcga gaccagcctg accaacatgg agaaaccctg tctcaactaa taatacaaaa    1380

ttagccaggc gtggtggcgc atgcctgtaa tcccagctac tcgggaggct gaggcaggag    1440

aatcacttga acccgggagg cggcggttgc agtgagccga gatcgtacca ttgcactcca    1500

gcctgggcaa caagagcgaa actccatttc aaaaaaaaaa aattggtgac agactcaatg    1560

atggaatgat ttgtcggaat taacacaaag cagattttat tcatataatg actttttttt    1620

aagagtctct tttttaaaaa aacttaattc tctaaaaccg aaatggttca tgcttctttt    1680

taaaaatgat tgtataaaat gtatggaatg gttagcctgg tgtggtggtg cacacctgta    1740

atcccagcta cttgggagac tgagacatga gaatcgcttg agcctgggag gcggaggttg 1800

caatgagcca agatcgtacc actgcactcc agcctgggcg acagagcaag acactgtctc 1860

tctctctctc tccatatata tatgtgtgtg tgtgtatata tatatatatg tgtgtgtgtg 1920

tgtgtgtata catatataca catatataca cacacacata catatacatg tgtatatata 1980

taccatccca tatatatgtg ggatatatat atatatatat atggatatgg ttatatatat 2040

gggatggttt ggttggtccc agcaaagtat atgaaaatta aagttctgtg ataatgacaa 2100

aggaattgct gttactgtac tgcaaatatg ctgtgggttc tcggtgttca aactcttcta 2160

aggaaggaca cacagtagct ctctgcttgc tgatagatgg tttcccagtg tgagatttgt 2220

tattttgatc agagtattca aatcagaatt taaatctagt gtttctattt tagtttagct 2280

tcctgattta tataaatgaa atctcattta taaagtataa taaagatgac tgtaagacaa 2340

aatccaa 2347

<210> 70
<211> 1681
<212> DNA
<213> Homo sapiens

<400> 70
agtcctgggc gaagggggcg gtggttcccc gcggcgctgc gcgcggcggt aattagtgat 60

tgtcttccag cttcgcgaag gctaggggcg cggctgccgg gtggctgcgc ggcgctgccc 120

ccggaccgag gggcagccaa cccaatgaaa ccaccgcgtg ttcgcgcctg gtagagattt 180

ctcgaagaca ccagtgggcc cgttccgagc cctctggacc gcccgtgtgg aaccaaacct 240

gcgcgcgtgg ccgggccgtg ggacaacgag gccgcggaga ctgtttcaat gcatcaaagc 300

tactgacatc tcatggcatg ggcatccagg ttccgctgaa tgcaacagag ttcaactatc 360

tctgtccagc catcatcaac caaattgatg ctagatcttg tctgattcat acaagtgaaa 420

agaaggctga aatccctcca aagacctatt cattacaaat agcctgggtt ggtggtttta 480

tagccatttc catcatcagt ttcctgtctc tgctgggggt tatcttagtg cctctcatga 540

atcgggtgtt tttcaaattt ctcctgagtt tccttgtggc actggccgtt gggactttga 600

gtggtgatgc ttttttacac cttcttccac attctcatgc aagtcaccac catagtcata 660

gccatgaaga accagcaatg gaaatgaaaa gaggaccact tttcagtcat ctgtcttctc 720

aaaacataga agaaagtgcc tattttgatt ccacgtggaa gggtctaaca gctctaggag 780

gcctgtattt catgtttctt gttgaacatg tcctcacatt gatcaaacaa tttaaagata 840

agaagaaaaa gaatcagaag aaacctgaaa atgatgatga tgtggagatt aagaagcagt 900

tgtccaagta tgaatctcaa ctttcaacaa atgaggagaa agtagataca gatgatcgaa 960

ctgaaggcta tttacgagca gactcacaag agccctccca ctttgattct cagcagcctg 1020

cagtcttgga agaagaagag gtcatgatag ctcatgctca tccacaggaa gtctacaatg 1080

aatatgtacc cagagggtgc aagaataaat gccattcaca tttccacgat acactcggcc 1140

agtcagacga tctcattcac caccatcatg actaccatca tattctccat catcaccacc 1200

accaaaacca ccatcctcac agtcacagcc agcgctactc tcgggaggag ctgaaagatg 1260

ccggcgtcgc cactctggcc tggatggtga taatgggtga tggcctgcac aatttcagcg 1320

atggcctagc aattggtgct gcttttactg aaggcttatc aagtggttta agtacttctg 1380

ttgctgtgtt ctgtcatgag ttgcctcatg aattaggtga ctttgctgtt ctactaaagg 1440

ctggcatgac cgttaagcag gctgtccttt ataatgcatt gtcagccatg ctggcgtatc 1500

ttggaatggc aacaggaatt ttcattggtc attatgctga aaatgtttct atgtggatat 1560

ttgcacttac tgctggctta ttcatgtatg ttgctctggt tgatatggta agtttttaag 1620

aagtcttatt acattattga ccaacaataa aatagagaaa atattcaaaa aaaaaaaaaa 1680

a 1681

<210> 71
<211> 6512
<212> DNA
<213> Homo sapiens

<400> 71
gagagctgcc tgctcagaca acagacacgc gaggtcagga agaagccgct tataaattac 60

cgcttccttc gcgccgccgc caacgccgag ccccgaggac cgcaagccca gaggacaagc 120

tgcgccaaga gggagtgcgg agcgttcacc cagcgggtca gagagcgagc gggcaggcag 180

cccccggccg gcggaacccg gcacagccga gcagagcgcg ggcggcgccg cagccacccc 240

agatccagaa ccagaaccac agcccttctg aggagctccc aaaccaagga gatggccacc 300

aaggagaagc tgcagtgtct gaaagatttc cacaaggaca tcctgaagcc ctcaccaggg 360

aagagcccag gcacgcggcc tgaggacgag gctgagggaa aacctccgca gagggagaag 420

tggtctagca agatcgactt tgtgctctct gtggctggcg gcttcgtggg cttgggcaac 480

gtctggcgct tcccgtacct ctgctacaag aatggtggag gtgcgtttct cataccgtat 540

tttattttcc tgtttgggag cggcctgcct gtgtttttct tggagatcat cataggccag 600

tacacctctg aagggggcat cacctgctgg gaaaagatct gccccttgtt ctctggtatc 660

ggctatgcct ccgttgtaat tgtgtccctc ctgaatgtct actacatcgt catcctggcc 720

tgggccacat actacctgtt ccagtccttc cagaaggagc tgccctgggc acactgcaac 780

cacagctgga acacacctca ctgcatggag gacaccatgc gcaagaacaa gagtgtctgg 840

atcaccatca gctccaccaa cttcacctcc cctgtcatcg agttctggga gcgcaacgtg 900

ctgagcttgt cccctggaat cgaccaccca ggctctctga aatgggacct cgctctctgc 960

cttcttttag tctggctagt gtgtttcttc tgcatctgga agggcgtcag gtccactggg 1020

aaggtcgtct acttcacagc cacttttcca ttcgccatgc tcctggtgct gctggtccga 1080

gggctgacgc tgccgggcgc gggcgcaggc atcaagttct atctgtatcc tgacatcacc 1140

cgccttgagg acccacaggt gtggattgac gctgggactc agatattctt ctcttatgcc 1200

atctgcctgg gggctatgac ctcgctgggg agctacaaca agtacaagta taactcgtac 1260

agggactgta tgctgctggg atgcctgaac agtggtacca gttttgtgtc tggcttcgca 1320

attttttcca tcctgggctt catggcacaa gagcaagggg tggacattgc tgatgtggct 1380

gagtcaggtc ctggcctggc cttcattgcc tacccaaaag ctgtgacaat gatgccgctg 1440

cccacatttt ggtccattct tttttttatt atgcttctct tgcttggact ggatagccag 1500

tttgttgaag ttgaaggaca gatcacatcc ttggttgatc tttacccatc cttcctaagg 1560

aagggttatc gtcgggaaat cttcatcgcc ttcgtgtgta gcatcagcta cctgctgggg 1620

ctgacgatgg tgacggaggg tggcatgtat gtgtttcagc tctttgacta ctatgcagct 1680

agcggtgtat gccttttgtg ggttgcattc tttgaatgtt ttgttattgc ctggatatat 1740

ggaggtgata acctttatga tggtattgag gacatgattg gctatcggcc cgggccctgg 1800

atgaagtaca gctgggctgt gatcactcca gttctctgtg ttggatgttt catcttctcg 1860

ctcgtcaagt acgtacccct gacctacaac aaaacatacg tgtaccccaa ctgggccatt 1920

gggctgggct ggagcctggc cctttcctcc atgctctgcg ttcccttggt catcgtcatc 1980

cgcctctgcc agactgaggg gccgttcctt gtgagagtca agtacctgct gaccccaagg 2040

gaacccaacc gctgggctgt ggagcgcgag ggagccacac cttacaactc tcgcaccgtc 2100

atgaacggcg ctctcgtgaa accgacccac atcattgtgg agaccatgat gtgagctctc 2160

tcgggtcgac ggggccggcg gctttcctgc tgtttactaa cattagattc tcataggacc 2220

aggtttacag agctttatat ttgcactagg attttttttt ttttgtaatt gtcacagaaa 2280

atgtaattgt gggtatgtgt gcgtgcgtgt gtgtgtgtgt gtgtatcgtg tgtgtgtgtt 2340

ttgttttgat ttgggggata ttttgtacaa aaagaaaacc cacgggaaga tgtccgtgga 2400

gaggcagagc tttcatactg aattagatgt attttatggg aatttggtaa atttttcttt 2460

gtattttttt ttttacatat aagtatatat acacttagag attgtcatat acttttacca 2520

cttgaattga tcttcttgcc agcaatagat ctcattttca aaagcaattc ttcggtgctg 2580

tgtagctggc agaaagttct gtccagtaaa cgcaggatgg aattttcctg ggactctaca 2640

cccatcttaa ggtggtatac cttccaaatc ctggttcaga tggaagaaat agcaggagag 2700

aggacccatt agctggcaga cccaggggga agaaaggagg gctgtgagga gatacctcat 2760

taaacttggc ttagtgaaga agagagatgc caaaggaatg aaccaaccct tcacataaag 2820

gagactggct gaagctgaat gaggaggccc tatagcagaa gtctgattct aagagcagta 2880
gaaacttgta ccagaagcaa aatcccactt ttaattttga gatggtgagt ggatagtcag 2940
tagaccgtca gaaccactgg ccagagaggg agctgctaga gatccaagaa ggctggcagg 3000
agtgaggctc acaactcagc ctcgcaagag gtggcagagg cacaggaggc cacagtcctt 3060
cctggggcat tccaggcaga gaaggagcag aggctctccc ggcaggagct ggggtctcag 3120
ggctcagatg agtctgttgc atttgaatgg ggtcatagca ggttctggtc attccccaag 3180
caacatctca gcatctctta aagttgcctg caggaatgaa gcatgacata cctgttgagg 3240
gactagggga gtggtgggga ggtgagtgga ccaaaggata taggccccag gcatgcagat 3300
gggcccggtg tcggggaggg gtgctttctt tcctcatctc cccactcccc actctcagcc 3360
tgggagactc ctgccaagcc ctcattaaag atgccaccct gggctgccct ggcacctagc 3420
aaggcacacc aagaacagct tttgagtctg tatcctccac tgggggaagt gctcccagtt 3480
cagaacaagg gcagcccgtg gtgctgacct aggatataac aaagctcttc acttcaaaac 3540
ccctgcaata gctgggttta cagacattta ccacctgcgg acccaaaaga gaaggcctag 3600
gagagttttc tagaaggttg ggattgtcag ggtcctggcc cctcagaact ggcttgatca 3660
agggccttat gtggagcaga ggttgtctct gaaccaggag agaaggtact atacctttca 3720
aatccccagg gcagacacac ccccacccag cccctatttg gacctaaact gtgccatttg 3780
aacagtcact tccaagctca gtctaaatga aaccgaaacg tgaccacgca caaaggcagt 3840
cactgcctcg aggggtgcag accgcagaat tttcacagca ggggctcttg gaaccctgga 3900
aacccccttc ttaaatttgg gaggaggagt atgcctttgg tgtccccctc ccaaggggca 3960
attctgaacc ccatctttgg caggcataca tatttcactg tttccaaagc tatctactct 4020
gccaaacaac acccagtcct attccaaact ctcaacgatt ctatcttgtt cctgtttttc 4080
tatgtattta tggttgccgt ttgtgtctga tttgatttta ctgttttttc cctgatttta 4140
tggagtagca ttgtgacctg ttttcctttg tcttatataa ctttagtaaa ctaaccactg 4200
tcaatgattg agggcaggtg gcacgtgggg aagaggggac ttggcacgca gtggctacct 4260
gggcatttgt ggtcatttca gtttccatct ccccagcggg ggctccctgg gtgaaaggcc 4320
acagtatttt gggttggtag gcaaattgca acattctgga catggcctga ggaaggcctc 4380
ttcttataag attctcagac caaattctag accaaagaca caggcagacc aagtccccag 4440
gccccgcctg gaaggaagtc gttcctcaac tctccccaag gcacctgtct ccaatcagag 4500
ccctctcgcc cagccagccc tggctctgtg tgcagagcat agctctgcga gtacctgtgt 4560
aataatgctc aaccttcatg tctccgtata aacgaaactt tccatgagag ctcatgactc 4620
tggtccacct gtctatagag aatgggcaaa gtccttcacc tgctttctgc ttgggatggg 4680
tcagaaatgc tgatgcccgc acatagccca gccagccaga tctggaaagg aagcgagggg 4740

gttgtttaaa tcaatttttt aagatgaaga agtgggagac actgcgttga gatgggccat 4800

gctagggcca cagagatttc ctgacggtca gggagagaag ggcctccagg gtcccctaac 4860

ccaacgccct tgttgtaaat gaggtaactg aggctcaggg aggcactgtg agccaggaat 4920

ggattttctt gaaacagctc tagctgcagg ttctccgagg taggtgcagg gaatggtgag 4980

tgtctaacca gggctacatc cagcaacatc ctcaaggtct tcctgacaac caaagacaag 5040

cctttatgga aaaggaaatg cgctcccctc catgttcagg gatgagggga gcagcagcag 5100

ccacactccc accatcctca cagaattcct ggacccatgc ggtggctccg tgagctgggt 5160

gactccagcc tcacctgcac accccagccc tgcacggggc cctccttcct cccagcagcc 5220

cttggtgagc taggaattga gatccctgtt tgtgaaagag ggaactgagg tgcagagaag 5280

ccagaggtgt gccagatcct taggcaggat ttagatgaag tcgccctggc tccagactga 5340

ccccgaggct ctgcggggag tttccaggca gcaggaagtg gccttggatg ctctccttcc 5400

aggacagcat aacccctggg ccatgtgcag ctccttcact gccccctgga tccccagcat 5460

acccccaaag acagtgggga aacacaaggg gagagcacag catggcccct ccagcccact 5520

tcagggcact cttgtatcac ccgggtaccg ccacactggt cccccaccca gccagcatct 5580

cccagcacag cccctctccc tggggaaatg ctctgggtag ccagtctaaa ggcagaggca 5640

cctaactgct ccccgcagcc caccccaccc aagattcaga cacaagccag gaaaggaccc 5700

aagagaaaat ccttcaaggt ggcctgaggt cccatccctc cctcagaccc atgtggtccc 5760

aggccaggct gcctgggaca cggtaaatac cactgtgtgc aaaaatcgaa gtacaaaacc 5820

acaagactaa acaaaacaaa cccagagagc caaacttgta gaggtgggca gtccagaaag 5880

cagggggcag ccctccccct ttccttctct ccctgatcct cagaatatat attgttgtaa 5940

taggaagcat ttttgcattg ttctcttgtg ggtgtcacta cagacatgtt ctggcgtgtt 6000

ctccgaggga tggagcatcc tgttatatat ttgacttcaa attgagatgt tggcttcatt 6060

tttttttttt acccaattaa tctcccaatc cctagcaact gtgactctgt atttagcaca 6120

agagaaagct gagaatgtgg gtcttgcctc cttccagaaa tatgtctggc tcatcaggac 6180

atttttttaa aacttcaaaa tatttttaag atattttaaa cttttataaa aaaaaaatca 6240

accaacaaga gacttttctg aggaggaaca tttgtatttg aacaagatcc ttggtgtgta 6300

gttcagtctt gcagtataca agcttttgtg tataaatgtt ttatgatatg attccctgta 6360

ttttgcaggg gtttttttct cttttgcttt ttagataaat atgtatatca atattttaaa 6420

ttcatctttg ctttttttag aggagtttgt aatcacctta taacatgaaa ataaacattt 6480

cctttttaac atccaaaaaa aaaaaaaaaa aa 6512

<210> 72

<211>  3550
<212>  DNA
<213>  Homo sapiens

<400>  72
gccgggcccc gccgccgccc gcgcgccccc gggcccccga cacacatgag attcttcagg      60

ctcactttca agtgcttcgt ggactgcttc tgactgcgcc gcccgcgccc cgcaccccgc     120

cgcccgcccg ccgccccgtc ccccggcccg gccgcccccc ggcccccggc cggcccgcgc     180

cctcggggcc ctccccggtg ccgccggtgc cccccgcctg accgccgccc cccgtgaggc     240

gccgcgaccc cggcccggcc gtgcggcccg ccgaggccat ggcgaagaag agcgccgaga     300

acggcatcta tagcgtgtcc ggcgacgaga agaagggccc cctcatcgcg cccgggcccg     360

acggggcccc ggccaagggc gacggccccg tgggcctggg gacacccggc ggccgcctgg     420

ccgtgccgcc gcgcgagacc tggacgcgcc agatggactt catcatgtcg tgcgtgggct     480

tcgccgtggg cttgggcaac gtgtggcgct tcccctacct gtgctacaag aacggcggag     540

gtgtgttcct tattccctac gtcctgatcg ccctggttgg aggaatcccc attttcttct     600

tagagatctc gctgggccag ttcatgaagg ccggcagcat caatgtctgg aacatctgtc     660

ccctgttcaa aggcctgggc tacgcctcca tggtgatcgt cttctactgc aacacctact     720

acatcatggt gctggcctgg ggcttctatt acctggtcaa gtcctttacc accacgctgc     780

cctgggccac atgtggccac acctggaaca ctcccgactg cgtggagatc ttccgccatg     840

aagactgtgc caatgccagc ctggccaacc tcacctgtga ccagcttgct gaccgccggt     900

cccctgtcat cgagttctgg gagaacaaag tcttgaggct gtctggggga ctggaggtgc     960

caggggccct caactgggag gtgacccttt gtctgctggc ctgctgggtg ctggtctact    1020

tctgtgtctg gaagggggtc aaatccacgg gaaagatcgt gtacttcact gctacattcc    1080

cctacgtggt cctggtcgtg ctgctggtgc gtggagtgct gctgcctggc gccctggatg    1140

gcatcattta ctatctcaag cctgactggt caaagctggg gtcccctcag gtgtggatag    1200

atgcggggac ccagattttc ttttcttacg ccattggcct gggggccctc acagccctgg    1260

gcagctacaa ccgcttcaac aacaactgct acaatgggac cagcttcttt gctggcttcg    1320

tggtcttctc catcctgggc ttcatggctg cagagcaggg cgtgcacatc tccaaggtgg    1380

cagagtcagg gccgggcctg gccttcatcg cctacccgcg ggctgtcacg ctgatgccag    1440

tggccccact ctgggctgcc ctgttcttct tcatgctgtt gctgcttggt ctcgacagcc    1500

agtttgtagg tgtggagggc ttcatcaccg gcctcctcga cctcctcccg gcctcctact    1560

acttccgttt ccaaagggag atctctgtgg ccctctgttg tgccctctgc tttgtcatcg    1620

atctctccat ggtgactgat ggcgggatgt acgtcttcca gctgtttgac tactactcgg    1680

ccagcggcac caccctgctc tggcaggcct tttgggagtg cgtggtggtg gcctgggtgt    1740

acggagctga ccgcttcatg gacgacattg cctgtatgat cgggtaccga ccttgcccct 1800

ggatgaaatg gtgctggtcc ttcttcaccc cgctggtctg catgggcatc ttcatcttca 1860

acgttgtgta ctacgagccg ctggtctaca acaacaccta cgtgtacccg tggtggggtg 1920

aggccatggg ctgggccttc gccctgtcct ccatgctgtg cgtgccgctg cacctcctgg 1980

gctgcctcct cagggccaag ggcaccatgg ctgagcgctg gcagcacctg acccagccca 2040

tctggggcct ccaccacttg gagtaccgag ctcaggacgc agatgtcagg ggcctgacca 2100

ccctgacccc agtgtccgag agcagcaagg tcgtcgtggt ggagagtgtc atgtgacaac 2160

tcagctcaca tcaccagctc acctctggta gccatagcag cccctgcttc agccccaccg 2220

cacccctcca gggggcctgc ctttccctga cacttttggg gtctgcctgg gggaggaggg 2280

gagaaagcac catgagtgct cactaaaaca actttttcca tttttaataa aacgccaaaa 2340

atatcacaac ccaccaaaaa tagatgcctc tccccctcca gccctagccg agctggtcct 2400

aggccccgcc tagtgcccca cccccaccca cagtgctgca ctcctcctgc ccctgccacg 2460

cccaccccct gcccacctct ccaggctctg ctctgcagca cacccgtggg tgacccctca 2520

ccccagaagc agcagtggca gcttgggaaa tgtgaggaag ggaaggaggg agagacggga 2580

gggaggagag agaggagaag ggaggcaggg gaggggcagc agaaccaagg caaatatttc 2640

agctgggcta tacccctctc cccatccctg ttatagaagc ttagagagcc agccagcaat 2700

ggaaccttct ggttcctgcg ccaatcgcca ccagtatcaa ttgtgtgagc ttgggtgcga 2760

gtgcacgcgt gcgtgagtac ggagagtata tatagatctc tatctcttag caaaggtgaa 2820

tgccagatgt aaatggcgcc tctgggcaaa ggaggcttgt attttgcaca ttttataaaa 2880

acttgagaga atgagatttc tgcttgtata tttctaaaaa gaggaaggag cccaaaccat 2940

cctctcctta ccactcccat ccctgtgagc cctaccttac ccctctgccc ctagccaagg 3000

agtgtgaatt tatagatcta actttcatag gcaaaacaaa agcttcgagc tgttgcgtgt 3060

gtgagtctgt tgtgtggatg tgcgtgtgtg gtccccagcc ccagactgga ttggaaaagt 3120

gcatggtggg ggcctcgggg ctgtccccac gctgtccctt tgccacaagt ctgtggggca 3180

agaggctgca atattccgtc ctgggtgtct gggctgctaa cctggcctgc tcaggcttcc 3240

caccctgtgc ggggcacacc cccaggaagg gaccctggac acggctccca cgtccaggct 3300

taaggtggat gcacttcccg cacctccagt cttctgtgta gcagctttaa cccacgtttg 3360

tctgtcacgt ccagtcccga gacggctgag tgaccccaag aaaggcttcc ccgacaccca 3420

gacagaggct gcagggctgg ggctgggtga gggtggcggg cctgcgggga cattctactg 3480

tgctaaaaag ccactgcaga catagcaata aaaacatgtc attttccaaa gcaggaaaaa 3540

aaaaaaaaaa 3550

<210> 73
<211> 9648
<212> DNA
<213> Homo sapiens

<400> 73
ggtttgtaat gatagggcgg cagcagcagc agcagcagca gtggtggaac gaggaggtgg 60

agaattgaga gcacgatgca tacacaggtg tttctgagta gtaattagat cgctgtgaag 120

gaaaaagcac acctttgagt tttcacctgt gaacactata gcgctgagag agacagtctg 180

aaagcagagg aagacatcga tcagtaacac caagagacac caaagttgaa agttttgttt 240

tctttccctc tgttttattt ttcccccgtg tgtccctact atggtcagaa agcctgttgt 300

gtccaccatc tccaaaggag gttacctgca gggaaatgtt aacgggaggc tgccttccct 360

gggcaacaag gagccacctg ggcaggagaa agtgcagctg aagaggaaag tcactttact 420

gaggggagtc tccattatca ttggcaccat cattggagca ggaatcttca tctctcctaa 480

gggcgtgctc cagaacacgg gcagcgtggg catgtctctg accatctgga cggtgtgtgg 540

ggtcctgtca ctatttggag ctttgtctta tgctgaattg ggaacaacta taaagaaatc 600

tggaggtcat tacacatata ttttggaagt ctttggtcca ttaccagctt ttgtacgagt 660

ctgggtggaa ctcctcataa tacgccctgc agctactgct gtgatatccc tggcatttgg 720

acgctacatt ctggaaccat tttttattca atgtgaaatc cctgaacttg cgatcaagct 780

cattacagct gtgggcataa ctgtagtgat ggtcctaaat agcatgagtg tcagctggag 840

cgcccggatc cagattttct taaccttttg caagctcaca gcaattctga taattatagt 900

ccctggagtt atgcagctaa ttaaaggtca aacgcagaac tttaaagacg ccttttcagg 960

aagagattca agtattacgc ggttgccact ggctttttat tatggaatgt atgcatatgc 1020

tggctggttt tacctcaact ttgttactga agaagtagaa aaccctgaaa aaaccattcc 1080

ccttgcaata tgtatatcca tggccattgt caccattggc tatgtgctga caaatgtggc 1140

ctactttacg accattaatg ctgaggagct gctgctttca aatgcagtgg cagtgacctt 1200

ttctgagcgg ctactgggaa atttctcatt agcagttccg atctttgttg ccctctcctg 1260

ctttggctcc atgaacggtg gtgtgtttgc tgtctccagg ttattctatg ttgcgtctcg 1320

agagggtcac cttccagaaa tcctctccat gattcatgtc cgcaagcaca ctcctctacc 1380

agctgttatt gttttgcacc ctttgacaat gataatgctc ttctctggag acctcgacag 1440

tcttttgaat ttcctcagtt ttgccaggtg gctttttatt gggctggcag ttgctgggct 1500

gatttatctt cgatacaaat gcccagatat gcatcgtcct ttcaaggtgc cactgttcat 1560

cccagctttg ttttccttca catgcctctt catggttgcc ctttccctct attcggaccc 1620

atttagtaca gggattggct tcgtcatcac tctgactgga gtccctgcgt attatctctt 1680

tattatatgg gacaagaaac ccaggtggtt tagaataatg tcagagaaaa taaccagaac 1740

attacaaata atactggaag ttgtaccaga agaagataag ttatgaacta atggacttga 1800

gatcttggca atctgcccaa ggggagacac aaaataggga tttttacttc attttctgaa 1860

agtctagaga attacaactt tggtgataaa caaaaggagt cagttatttt tattcatata 1920

ttttagcata ttcgaactaa tttctaagaa atttagttat aactctatgt agttatagaa 1980

agtgaatatg cagttattct atgagtcgca caattcttga gtctctgata cctacctatt 2040

ggggttagga gaaaagacta gacaattact atgtggtcat tctctacaac atatgttagc 2100

acggcaaaga accttcaaat tgaagactga gatttttctg tatatatggg ttttgtaaag 2160

atggttttac acactataga tgtctatact gtgaaaagtg ttttcaattc tgaaaaaaag 2220

catacatcat gattatggca aagaggagag aaagaaattt attttacatt gacattgcat 2280

tgcttcccct tagataccaa tttagataac aaacactcat gctttaatgg attataccca 2340

gagcactttg aacaaaggtc agtggggatt gttgaataca ttaaagaaga gtttctaggg 2400

gctactgttt atgagacaca tccaggagtt atgtttaagt aaaaatcctt gagaatttat 2460

tatgtcagat gttttttcat tcattatcag gaagttttag ttatctgtca tttttttttt 2520

tcacatcagt ttgatcagga aagtgtataa cacatcttag agcaagagtt agtttggtat 2580

taaatcctca ttagaacaac cacctgtttc actaataact tacccctgat gagtctatct 2640

aaacatatgc attttaagcc ttcaaattac attatcaaca tgagagaaat caccaacaaa 2700

gaagatgttc aaaataatag tcccatatct gtaatcatat ctacatgcaa tgttagtaat 2760

tctgaagttt tttaaattta tggctatttt tacacgatga tgaattttga cagtttgtgc 2820

attttcttta tacattttat attcttctgt taaaatatct cttcagatga aactgtccag 2880

attaattagg aaaaggcata tattaacata aaaattgcaa aagaaatgtc gctgtaaata 2940

agatttacaa ctgatgtttc tagaaaattt ccacttctat atctaggctt tgtcagtaat 3000

ttccacacct taattatcat tcaacttgca aaagagacaa ctgataagaa gaaaattgaa 3060

atgagaatct gtggataagt gtttgtgttc agaagatgtt gttttgccag tattagaaaa 3120

tactgtgagc cgggcatggt ggcttacatc tgtaatccca gcactttggg aggctgaggg 3180

ggtggatcac ctgaggtcgg gagttctaga ccagcctgac caacatggag aaaccccatc 3240

tctactaaaa atacaaaatt agctgggcat ggtggcacat gctggtaatc tcagctattg 3300

aggaggctga ggcaggagaa ttgcttgaac ccgggaggcg gaggttgcag tgagccaaga 3360

ttgcaccact gtactccagc ctgggtgaca aagtcagact ccatctccaa aaaaaaaaga 3420

ttatatatat atatatatgt gtgtgtatgt gtgtgtgtgt gtgtgtgtgt atatatatat 3480

atatatatat acacacacac acacacactt tttatatata tatatatata tatatagtgg 3540

aacttacaaa tgagagtaat ataatgatga aattttgaac tgttatttat aaacatctaa 3600

ggtaaaatgg ttagtcatgg ccagagtatg tttcatcctt taatttttgt ccatttgaaa 3660

ataaggattt ttgaaagaat tataccaatt aaaattatta aaggcaaaca tagaattcat 3720

aaaaaattgt ccaaagtaga aatgatgacc tataatttgg agcatttcca attcagtaat 3780

ttcaattttg ctcttgaaaa catttaatat atatccaaga ctgacatttc tttagctgaa 3840

cctaacgttt gggtctctga gtgaatttat aataactcct tccttcctta gcatagggtt 3900

ttcaaaattt gatttataat tcctatttcc agtaaatatt gttcatttgt ccacatctct 3960

ccctatgata tgttgctgga ggtaagaatt tctttcatat tcctattttt tttttcccca 4020

tagactaggc tcatagaatt taaacaagca aattttcctg agctttttct tgccaaatga 4080

aagaagactg gtaaattctc atagagaggt ttgtgtagtt cttggctctt cctggggtta 4140

atgtgcttat attcacagtg gcaaattggt ctcagacttt aatttattta tttttgattt 4200

gaatttctct ttaaaagtat caatttaaaa ggtaactaga attattcttt ctcattttca 4260

aaagtgattt ttgcattatt aaatttccct gccattgtaa tgccatttca cgcagaaaaa 4320

aagtcagcca gtaattaaga aaaaaagtga tggagattaa gtagtatttt ggcttatttt 4380

taggactcat catgagaaga cacagttcct ttaatcagga aattaatatc cataattttc 4440

actcaaaatt gcagtatgta aagcagattc tcaaaaactc tcctgaacac ttatttatat 4500

atatgttttt atataagtaa aatttttctc atatttttat acgatatgca cacacacaca 4560

tacatgcaca tactacttac tacatgttct gtacttgtac tttgtaccat gcatattcaa 4620

atgtttatat acataagttt attataacat aaacagtaaa agtaatgaat actgtttaaa 4680

ataactaata tagtattttt taatttttgt ggggatggat tctcaaatac ttgtgatttt 4740

aaaagattct aaagctaaaa cacaacttga ttttaaaaag aatgattctc cttacacaat 4800

tataaatatt tgcagtaaat attttcctta taatactgtt ttgaccccat ttaaaaagta 4860

ttagattata ttcctttgat ccaatgaaaa ctgaacctta taaatggtta gctgaaagta 4920

gaccttattc ttgtccttct ttagaagagt aaagatttgt cctagggaag atggctgact 4980

tcggttccca acatgcgtat gcatttagac tgtagctcct cagccctgtg gacacaaaat 5040

ttggacagct tattaggtta cgttagcaat gcatgacggt ttctccaaca ctaagatatt 5100

cacgttgaaa cagatttcct gttcgtctta tgtgtctggt aaaattgttt ccccaattac 5160

aatttgacat atcaatagag ggttaacaag agtataatta cataacagaa ttcctcatga 5220

actgtaatca gtctacagga aaatcattat tttatcttga tttgcagatg aatatactgc 5280

taagaaaggg agcaactctg acctttgtta aagttgatct tttgtaattg aggtataagg 5340

tatgaaaaga taaaaaaccg aaggccagag aatcaggaaa tgaaagatag tatggactga 5400

aggtaacaat attttaatgt tatgcaatat agtcagagaa atattaaaaa ttagttgttt 5460

gctgtgcata ggtggatctc gcaggaagct aatgaaacct aagcttcagt gcctctcact 5520

tagacatgtt ccattcgagg tcctgaacct aactttgtat taggaattct gtactaattt 5580

tgttgaagaa gaccagcaaa gttgtgtaca cttctacccc cacaaaatct gcattgtcca 5640

tgtgagtaaa gtaaaataat tcctgttatt tttttctgtt agaaataagt atggaggata 5700

tgtttttaaa aatttatgag ttaattgaaa tatccatata taacaagtga ctttctcaca 5760

atatatatga tgtgatatat agggagatag tttcactttc atcatatttt atacgttgat 5820

tctgaactat agaaaaataa taaatgggat tttaattata gctcttagtt gggaaagaaa 5880

tatagagaga tgtgggattt gaatgcccat gaaagacatt ttattttact tgaatatatt 5940

cttgcttcac tttaccctcc ataatatgtt gtacattagt gctgatcaag tttacagagt 6000

tacattttgc tttcctaacc attcagtcag gaattaaaat atggcattgt ataacaactg 6060

ggaagaagct catagtggat ataaattaga gtagataatg ggtcaccttg atagcctctg 6120

tttacattac ttgtatatgg gcaaaataat tattacctat acgtgtattt aagcttaatt 6180

ttcatataaa cagtattttt aatctatgtt aaaatagata atatctaaaa gtgtgatctc 6240

taggtagtcc ttagtttatt agtactgtac ttcaaaaaga tttttaaata ggtccggcac 6300

ggtggctcat gcctgtaatc ccagcacttt gggaggctga ggcgggcgaa tcacctgagg 6360

tcaggagttc gagatcagcc tggccaacat ggtgaaaccc tgtctcaact aaaaatataa 6420

aaattagccg ggcgtggtgg caggcgcctg taatcccagc tactcgggag gctgaggcag 6480

gagaatcact tgaacccaag gggcagaagc tgcagttagc caagatcgca tcattgcact 6540

ccagcctagg ggacaagagc gcgagacttc atctcaaaaa aaaaaaaaaa aaaaaaaaaa 6600

gatttttaaa taatagctaa aggtatgctc tctaggtcat ccttagttta ttagtactgt 6660

acttaaaaat tattttttaa tagtcaattt tgggagataa ttatttcttt ccttatattt 6720

tccaattagt tggtgtctaa aaataaatgt tttgtctaat tttagatcag gtatacattc 6780

acaaaagcat aaatcatagt ctcacaggaa attcaccaat tttccatatg tcgtgagata 6840

actgtccttt ctacaacctc ataacaatga atttatataa ttacctagat tttcttagtg 6900

tgaatctacc cattagtttt attttcttgg tagttatttt tttccctcct ctctgttact 6960

attggcctta aaatacacag aggacggtta cagtgtccta atagctgtta catgtgtgtg 7020

tttcagcgta cttgaatcaa gtgtacattt atagtaccaa taaccgcctt tacagcttta 7080

cagttaacaa ttctctcaca aaactgtaga gcattaggca tctgagagcc atagagggcc 7140

aactttgttc cagagtgaac atgctttttt tcctcaacat atacactact gatttttttt 7200

aaaagtatga ctttcaagtg aattaatgta ttggttagga gaactgcttg ctaagtcctt 7260

attacctctt gttaaagcct cagaaggccg tgctgaaagc cagaggggaa aaaaagagta 7320

atgcacaggt atctcttttg cagtggtgac tgtattttga gtaccttgtg tgacagggta 7380

ttattacagc atcttgtggg aaaacctatt aggcctttgc atgttaaagc tgtataattt 7440

gttgggttgt gagtggtctg acttaaatgt gtattataaa atttagacat caaattttcc 7500

tactaactaa ctttattaga tgcatacttg gaagcacagt catatcacac tgggaggcaa 7560

tgcaatgtgg ttacctggtc ctaggtttga actgtcttat ttcaaaagat ttctgaatta 7620

atttttccct agaatttctc cttcattcca aagtacaaac atactttgaa gaatgaaaca 7680

gattgttccc atgaatgtat gctcatactc gactagaaac gatctatgtt aaatgactgt 7740

gtatatgaat tatttcaagt actaccccaa ataactttct tattgctctg aaagaagaaa 7800

agcaatgtaa atcactatga ttattgcaca aacaaccaga attctccaac aattttaagt 7860

aatctgatcc tcttcttgga gaaaattgtt acctaatagt ttttccttat gaatgttatt 7920

actactggta taaatcaaat ttctataaat ttcctactta agtcttaaga actgggttct 7980

tcctttgatg ttattcatgt tcagaaagga aacaacactt tactctttta ggacaattcc 8040

tagaatctat agtagtatca ggatatattt tgctttaaaa tatattttgg ttattttgaa 8100

tacagacatt ggctccaaat tttcatcttt gcacaatagt atgacttttc actagaactt 8160

ctcaacattt gggaactttg caaatatgag catcatatgt gttaaggctg tatcatttaa 8220

tgctatgaga tacattgttt tctccctatg ccaaacaggt gaacaaacgt agttgttttt 8280

tactgatact aaatgttggc tacctgtgat tttatagtat gcacatgtca gaaaaaggca 8340

agacaaatgg cctcttgtac tgaatacttc ggcaaactta ttgggtcttc attttctgac 8400

agacaggatt tgactcaata tttgtagagc ttgcgtagaa tggattacat ggtagtgatg 8460

cactggtaga aatggttttt agttattgac tcagaattca tctcaggatg aatcttttat 8520

gtctttttat tgtaagcata tctgaattta ctttataaag atggttttag aaagctttgt 8580

ctaaaaattt ggcctaggaa tggtaacttc attttcagtt gccaaggggt agaaaaataa 8640

tatgtgtgtt gttatgttta tgttaacata ttattaggta ctatctatga atgtatttaa 8700

atatttttca tattctgtga caagcattta taatttgcaa caagtggagt ccatttagcc 8760

cagtgggaaa gtcttggaac tcaggttacc cttgaaggat atgctggcag ccatctcttt 8820

gatctgtgct taaactgtaa tttatagacc agctaaatcc ctaacttgga tctggaatgc 8880

attagttatg accttgtacc attcccagaa tttcaggggc atcgtgggtt tggtctagtg 8940

attgaaaaca caagaacaga gagatccagc tgaaaaagag tgatcctcaa tatcctaact 9000

aactggtcct caactcaagc agagtttctt cactctggca ctgtgatcat gaaacttagt 9060

agaggggatt gtgtgtattt tatacaaatt taatacaatg tcttacattg ataaaattct 9120

taaagagcaa aactgcattt tatttctgca tccacattcc aatcatatta gaactaagat 9180

atttatctat gaagatataa atggtgcaga gagactttca tctgtggatt gcgttgtttc 9240

ttagggttcc tagcactgat gcctgcacaa gcatgtgata tgtgaaataa aatggattct 9300

tctatagcta aatgagttcc ctctggggag agttctggta ctgcaatcac aatgccagat 9360

ggtgtttatg ggctatttgt gtaagtaagt ggtaagatgc tatgaagtaa gtgtgtttgt 9420

tttcatctta tggaaactct tgatgcatgt gcttttgtat ggaataaatt ttggtgcaat 9480

atgatgtcat tcaactttgc attgaattga attttggttg tatttatatg tattatacct 9540

gtcacgcttc tagttgcttc aaccatttta taaccatttt tgtacatatt ttacttgaaa 9600

atattttaaa tggaaattta aataaacatt tgatagttta cataataa 9648

<210> 74
<211> 6384
<212> DNA
<213> Homo sapiens

<400> 74
agcttgtccc cgcctagcaa ggagtcggct aagaactgga tcctagcgag gagcccggca 60

cagacagcga atgaccgcag ccagacagtc gctcttgctc ttcctcggcc ctgcggcagg 120

atccgccggt gcaggggcct ctccccggac tccacgcgtg tctggagggc tctcgggtta 180

gggaaggggg ctttggagac gccccgggcg gccgggcggt ggcgggacgc gggcccttta 240

agaaggagcg aggggcgcgg ccaggtaggg gcgggtccag ggcggatcag cgctgcgccg 300

gcgccggccc gggagccgga tttggagcgc gaggcgccgg tgggggcgga gggggctgcg 360

cggcggaggc tcccgtggcc tcggacgctc ctcctagcta gcggccgccg cccgccgccg 420

cctgcgcctc cagctccttc gccccggcgg gcccggccgc cgcttccggc agctcacctg 480

ggaagcgctc acctgggacg cgctcacctg ggacgcgcta cctgcctccg ggcgcctggg 540

cttcaggatg aaggaccgtc tggagcagct gaaggccaag cagctgacac aggatgatga 600

tactgatgcg gttgagattg ctatcgacaa cacggctttt atggacgagt tcttttctga 660

gattgaggaa actcggctta acattgacaa gatctcagaa catgtagagg aggctaagaa 720

actctacagt atcattctct ctgcaccgat tccagagcca aaaaccaagg atgacctaga 780

gcagctcacg actgagatta agaaaagggc caacaacgtc cggaacaaac tgaagagcat 840

ggagaagcat attgaagaag atgaggtcag gtcatcggca gaccttcgga ttcggaaatc 900

ccagcactct gtcctttctc ggaagtttgt ggaggtgatg accaaataca atgaagctca 960

agtggacttc cgagaacgca gcaaagggcg aatccagcgg cagctcgaaa ttactggcaa 1020

aaagacaacc gatgaggagc tggaggagat gttggagagt ggcaacccgg ccatcttcac 1080

ttctgggatc attgactcac agatttccaa gcaagccctc agtgagattg agggacgaca 1140

caaggacatt gtgaggctgg agagcagcat caaggagctt cacgacatgt ttatggacat 1200

cgccatgctg gtggagaatc agggtgagat gttagataac atagagttga atgtcatgca 1260

cacagtggac cacgtggaga aggcacgaga tgaaacgaaa aaagctgtga aataccagag 1320

tcaggcccgg aagaaactga tttcactcca gactggtgtg gccacccttg tcttcagatg 1380

agaatggagt ctgaatggcc ttcctgagag cgagtgcgac ccgttccttt gtttccttgc 1440

aaccaccctt ggacctgact cagctaacaa tctagccctg ggggaatgtg atctacctga 1500

tgcgaccctg agttctcccc agagcctcct cctgccccac cagctctcaa gtaccttttc 1560

tcctggactg tgtggaccca cccagctttc ttcctccctg ttgtgtgtca gattatgcct 1620

tgcacttggg aaagctcttg tgagactctc ccaaggtgct gtatttttct acctcatgga 1680

gtattctccc agaaactgca atgtattttt ttaggggagt atctttaaca aagcagaatg 1740

attcttctaa gtttggcaac aagaaggctt ggatctgagt cttctacctg gcaggatgcc 1800

aatcctgttt gttgtccgta tgtcctgaaa acatgaggga ctggcagatg tcattttggt 1860

ctaaagagct gacttgtttg aaattcagcc ttaaattaag ctcttagttg ttcagcttgg 1920

ggggcaactt tgatttttct ctgtgttgta gtctctcata tttactcaag gagggaccag 1980

gatgatacag tcatctgagg ttatgctttg caaaaggctg acggtatgga atatgtttcc 2040

atgtctgagt cttagaaact ggctgctcat tgttagaaag tgatgctttg tgagactatt 2100

gtcttggggc caaaaataat caggattttt aaattgggca agggacaagg tgctagaatc 2160

ctaagctctg gaaatatttc atgacactgg tgtattcact catgtgttcc agatgtattc 2220

taattgtgta tgaaatgtat gtacacataa gtgtgtgtgt ctcaggaagt aggaaataaa 2280

aatggaagct attatgacct caaaaaaaaa aagccaactt tgagctagga taaaaattgg 2340

gtaaaggaca tttgcttacc tgcaaatgaa tcactgtgga aatgtgatct tcccatatca 2400

tcaagaaact tgttttctgg atgaatactg ggagaataaa atgagaactc tggagtgagc 2460

taaattgatc ccaattaagt ttttctgctt agcagacaga aggtataatt ttttgacacc 2520

ctttcccacc tggtgcctat gctaggcttg tcctgagaac atccctcagt aacttgatat 2580

tcacatgacc tacaggatgt cccatctgca gggctgagtc agttggggaa caccagaggc 2640

tacacagtag ctcttcctgc tactcggtta atgagcttgg caggttcttt gtctcactga 2700

attcttatca tggaaacagc agcagcagcc gctaggaaat cttcaagtgt agtgtctgtg 2760

ctaacccagt ggtaaatccc ttagatcccc tgctggtctc tggcagtctc cttgattttg 2820

ggtaccatgt atattttccg ctttgacttt aacgctttct aggatagggt aagcaccctt 2880

aattcaggca ctgtccatta gcttcctttg caaaggctac ttatggccgg tcacaatcca 2940

gcactcagac agagccaagg caatatcctc ttgcccatgg ctatgatgtc agacagtgga 3000

tgggctccag caacaagaga caaaataact aaaggccttt gctctcctct gacattgagg 3060

cctggggctt acagtttgga atacaacatg tgaaggtttt tgttgttgtt tgtatttttt 3120

agatgtaaac ttgattattt tattgctaat ttaaaaataa aaatgacttt gtattgattg 3180

tgaaacggtt ctggctctgt ctcgatgcag aaacacaatg atctggtgcc accatgtggt 3240

gatttttatt caggttttag aatgcagttc acaccttttt aagccatgtg ctggatcaga 3300

tggttcaaaa gtgcaatttt tgaacatggt ttaactccca cagaatgcag tgtaactatg 3360

tttgtgtttc agatttgagg tgttcccccc aaaagaattt ggttcagtcc ttgggagtat 3420

ctggctttag gaggaaatgg gggagatctg tcacgatgtt atctagaagg tggaatgacc 3480

ataccaaaca tccttttaat ctaacttgaa tgtctcacca aaaataacat ttctgttggc 3540

attctgggtc ctagaagcca gatccatctc ctttttcctt ctgttgctct cttccttcac 3600

accctcttcc atgtccacat gcacttatct ccctgcagaa tactttttgc gatgatgttt 3660

ctcatgtatt ctttctttcc ttgtctggat gagcagaaga agatcatgat catgatctgc 3720

tgtattatcc ttgcgatcat cttagcttcc accattggga gcatatttgc ctgaaaaagg 3780

tgagccatct gtggggaggg tcagaccttc tttcactgac ttgaaacctt tgtgtcttgg 3840

gggcactcta ggtgccttaa tctgggtggg attaggtgct aataatggtt agagaaaact 3900

aaagaaaggg atgtttcaga gacagaaaag tgagtgaaga atgaactgtt agtaggtagt 3960

ctgtgggagg agggggagga cagaaggtgg caatctgtcc tataacctgg tgtggcagaa 4020

tgctttgtac aggtgaagga tagtgattcc tgctaaacag tttgagcctt ggtatctgga 4080

agtgacaaaa agaacaagaa ttagttcttg cattaggtgc atcttgaact ttttggaaga 4140

gggccggcca cacagtaaat tcaaattaaa tttcttccct ttcaaggtta atgaaagtta 4200

acacagcctt gtatgtagtc ctttaccccg ggtaagaggg atttggtgat cccagccacg 4260

aacaccatgc tatataatct atgatttttt tcctccattt ttctgttatc tcccacagcc 4320

ctcacagatt gatcgactgg catttctaat cctccttcca cttctgtggt accatcactt 4380

ctccacgcag actcctcatc agcttctcct ctttccatta tgaaacttct taagaaacag 4440

ggcaccaatc aactacttat taagaattat gcaaagaata aacgtataca gaattgggcg 4500

gaggacaggg acagggagta cagatacata gctgattagg cagatggttt aaaggaggac 4560

tgcagggtag agaagcaagc agagtgggcg cctctttagg aagtgacaca gcctgcatgt 4620

gcagtatggc tgtgaagggg cagatatcat agcacaccta actcaacagg atcttacttg 4680

aactgctgtg agttggtcaa gtcagggcac ttctgctctt caggctcctc taggtcacac 4740

ctttgaccac cctacatctg tttcctcttt caggctccag tagtagtctt aaaagtgaag 4800

tttatctaag gataagcaca tgcctatctt gctcactgct gtgtccccac tttttagcac 4860

agcgcctgct gcttacaggt actcaaatat ctgctgactt aattacttat aattaagctc 4920

ttatttcagt catggacaaa tccttgggtt tgactcctaa actctttaag gtaccaatga 4980

gaacgtggtt tgttactgtc agaggctgtg taaagccgct tgggaatggg ctgatctgct 5040

tatgcaaaaa tgctaccaac ctttcaaacc ccattaccac aacatgaaaa tttaaagtgc 5100

tttttctatg ggtggtgatg gtggagttct agcaacagcc tcttaatctg tggagaagat 5160

ggctggctcc aggactgtgg ttcaataacg aaatacaggc ccacaaaata aaataggttt 5220

atagcatgac tgaactcaca catcagtaga acactctgtg aaccataaca aagaacagat 5280

aaaggtgtca agtgagaaag gtgaaatgag gttatcactc acttcacctc tcacctgatt 5340

tgtgttgcta gctgaaactg ctggccagta acgtatgtat aagaaactgt tataggccgg 5400

gcacggtggc tcatgcctgt aatcacagca ctttgggagg ccaaggcagg tggatcacca 5460

gactgaccaa catagtgaaa ccccctctct acaaaaaata caaaaattag ccaggcatgg 5520

tggcttgcac ctgtattccc agctacttgg gaggctgagg caggagaatt gcttgaatgc 5580

aagaggcgga agttgcagtg agccgagatc gcgccactgc actccagcct gggcaacaag 5640

agctaaattc catctaaaaa aaaaaaaaaa acaaaaaaaa aaaactgttc ttaatactta 5700

atactgtccc caattccatt caaggttcag ttgtgttcag ctttaaaacc agctatgtga 5760

atgtgagttc tagtgcagat tatttagtga ttatgtaact aaaattgatg aaaaaatcac 5820

actatacaac tgtgaggagc acacaactgc taagtttgta cttttgaaag taaattattc 5880

tttgtttgat accttatttt ttaagaaagt gggattaaaa atattttggt cagtgctgtt 5940

ttctacccac cttcaaaagc caatggtttg atatttctat taatttgtgc ttcctttagt 6000

tttaataggg gatagaagac tgcagctggt tgggtctgga aaacattaat ctgggcaaac 6060

ggagctggag ctatgaggat ctgagtccta gggggccctc attcactagc agtaagaatt 6120

taagtggtgc atggcacata gcactgtact agattctgca ggggcacaaa catagagcca 6180

aacactctcc ctcttggaga atttcagacc cagaaaaggc cacgcagttc taacttttgt 6240

catcggttcc ttttgctaaa aggcaaaggg tatgttcctt gcctattgtc caacataccc 6300

cttccaagat tgtgagaaga tgggtagctg ggcatcaata aatattgaat caattgacct 6360

aaaaaaaaaa aaaaaaaaaa aaaa 6384

<210> 75
<211> 4548
<212> DNA
<213> Homo sapiens

<400> 75
gggactggag gctgccgagg gggccggcgc ccgagtccgg gattcggcca gtggtgctga 60

gcgagtgctg gaccagcggc cgtcctgtgc acctggcctg tgcgcgtgcc cgctgctcgg 120

cttcacccag actaaggcgc gggcagctgc gggaacaggc ggggtgggcg gagggagacc 180

gggaggcacg ggcgccctgt gcgcggagga ggtgaaggcg gccggggccg ggacgccatg 240

tccatggagg accccttctt tgtggtgaaa ggagaggtac agaaagcagt caacactgcc 300

cagggattgt ttcagagatg gacagagctc ctccaggacc cctccacagc aacaagggaa 360

gaaatcgact ggaccaccaa cgagctgaga aataacctcc ggagcataga gtgggatcta 420

gaggaccttg atgaaaccat cagcatagtt gaagcaaatc ctagaaaatt taaccttgat 480

gcaactgaat tgagtataag aaaagccttc attacaagta ctcggcaagt tgtcagggac 540

atgaaagatc agatgtcaac ttcatctgtg caggcattag ctgaaagaaa aaatagacag 600

gcactgctgg gagacagtgg cagccagaac tggagcactg gaacaacaga taaatatggg 660

cgtctggacc gagagctcca gagagccaat tctcatttca ttgaggagca gcaggcacag 720

cagcagttga tcgtggaaca gcaggatgag cagttggagc tggtctctgg cagcatcggg 780

gtgctgaaga acatgtccca gcgcatcgga ggggagctgg aggaacaggc agttatgttg 840

gaagatttct ctcacgaatt ggagagcact cagtcccggc tggacaatgt gatgaagaaa 900

cttgcaaaag tatctcatat gaccagtgat cggcgccaat ggtgtgccat agccatcctc 960

tttgcagtcc tgttggttgt gctcatcctc ttcttagtgc tgtgacggcg gggcctctgg 1020

gtgcgagttc ctcctgcata tgaaccgagg ggaggaggag aagctgagca cgtgtgacat 1080

tgccgtctac tcacattcct atcctggaaa catactgctg cactgacttt tctccgtgtg 1140

accccacaat tgacatggct cctccatccc agcgctggaa gggccagtgg gaagaggaaa 1200

tagatgtctg cactcctggc tgcagctgga caacagaagc cccatgccgc ctgtccagtt 1260

cggaggagaa ctagctgctg ccttgccttc cgggacctcg tttgctgagg agggacttac 1320

agactccact ggtgttttgc tgttgctcat tccatgcatc tttggcagct cttttcttct 1380

gctcagaccc ttccccgtgc tcagacagtg caccgctgtc ccatctaaag aaacctgtca 1440

ggaatacgag cttctgggta tgtttcgttt cccattgctg tagcatttct tatcccctga 1500

gagctgatga ttattgagga cagaaggctc agaaacagtt tgtgacagaa aatgcagtgt 1560

ttcatttttc agggataaat gctaagataa aattgctttt ccaggtcatt tttttttgtg 1620

gtaagaataa ctaatggaaa ataatgaaac accctggggt ttgggggtgc taacaacttg 1680

tggctttaac tgacaggagc aattaaaaag agcaagaggg ttctgcattg gcatagctta 1740

gggaagggtt aatgatgtcg ccacaggtca gctcctgatc cttgccgact tgatgttgct 1800

gtaccagggc ttcctcccca gaggtgcagc ttgcgttttg agggtgattg ctacatatgt 1860

tgttgctaaa cagctcagta acacacttga atgaatttgg ataccagatt gtcctcatta 1920

cagttctttt actcttaggg cactctacac tgggggttgg ggttgggagt ggttagtaca 1980

tttattacat ttattaagaa acgtaatgac ataaaaggtt agctctgggc cagacttctc 2040

ttactctgtg ggtaatggca aggatgtgta ggtaaacttg gttctttttt ttccctaaga 2100

tgacagcttg attttatcat ctgcagtcaa ataactgagc caatccaaat ttaaatgata 2160

gatgctttaa ttgagtttaa gtagctgaaa ctgctgagac actaaacttt aaccttctga 2220

tgacttttta aaatgcctca aatgtgcaca tgtatatagg atatttttat aacttccctg 2280

atgaataatc tgatattaaa gtagtatttg gacccagagc cagaactcgg tggtggaggc 2340

tgctggtctc tcctcaccac cttcttttgc acttggaaag aacagcaaca tctggataga 2400

gttctggctt tgacttctca tttccttgtc tttttgggtg cattcctcag cacacttttt 2460

ttttaaacct ttttgttttg ttttgtttgt atttcatgtg gttttatttg ggggttttgg 2520

ttttttcacc cttttttgtg atttgcaatg atgtgcttgc ccagctaact tttgaattgc 2580

actttttaat aaatattctt aacaattttt gaagaaggat attttatctc atttgagatc 2640

atggtaggtt aagaaaatat gcctgttgat gaaagctaaa agcaaattta tgaaactaaa 2700

agggtgattg acatccatgt ttacactccg ctctaatgtt tgatatataa ataagttatt 2760

ttcaaattag gaaaaaacag tgagtattac aaagggcttc agatgtttag agtactaggt 2820

tatttatgtt ttacaaagtt tgaatcttct ataaactaag aaaggggatg atccttagat 2880

ttgcattaaa atatagaagt cttttaaagt aaatgtgaac cttgtctaag tactgtaatc 2940

cacacaacac attataagaa gcaaaccagc atcttaagga attataaaat taccctattt 3000

aaaagccatg ctattgttct gctattacca gatttattgt gccacacaaa aggatcatgt 3060

gtgtcagcag gggccgtttg gaacaaacct agtcattaat gagtaagata ctcctgttag 3120

ttcagggacc aagttttatg acccagaggc ttaatgatgt ttggatatat ttcaaatcgg 3180

cgtgcttacc tcactgattt aaattatttt ctaaatagtg gccattgtag acctgactca 3240

ggctgaagct aaatagagaa caatttagaa agttaactaa caatacagtg cattctaccc 3300

gtagggccac catgcccttc tgcccctggc tgatttgatc ctgtgtctga tcccattgca 3360

ccctgactgg gcagtcccta cagaaccagt gttaatttga agggcctcca ctcaggctcc 3420

aaatgtggca gccaaagaga acaatccagg gaacctacat ttatttttaa ggacaaatat 3480

ttcctcctca gtggtcctaa tgttcagggc tttagaggga acccaggtgg tctcttcacc 3540

ctgtgtccta gaatgggaga gtaagtagac agtggtgata accccacact gcttataagt 3600

gcatctttat agtatttggg gctttcctac ccctttagcc ttctgtacct agtaccatat 3660

tccagtttta aagaactggc agaatgtgat ggataacaga ggaagagctc aatttatgtt 3720

tattggaaga acattttact taaatgattt gaggggtggg agggagtgaa ctactgagtt 3780

tgccagagtg aaaatccatc tgaaaaactc agctaccttt agtttttagt cctcattttt 3840

ggtcttgtct ctgcggactg tgaagaatca caatgctcta tatgccctgg actgtgtggc 3900

aaatgcaggt tgcagcgtgt gtgttacatg aggatcttcc acaatttcag aatgcacgcc 3960

agagctgaag ggggaaactt ggtaacttgc ccattattct ctgcttttag ccagagttaa 4020

acagactgat gggtctggta gccaacaact tggcaacttc cactccttct cacctcgtga 4080

gattaagggc tgtgaaaaga aatctagtct aactccaaca gaaatctgtc tctgttaagt 4140

gttttacctt ctgtaagtag agatggtaga gccaagattt ttcttttggt aatttccctg 4200

tctataaagt gagaccaaag ggatatctgt tccctgttac ctttttggag aattcataac 4260

atttgaagat caaaaaattg aatgataaat atgaatggct tttcaattct gtggactttg 4320

taccatttgg cttcaccttg tactgcaaga tgaatttgta aacaaaacaa aattggactg 4380

tctggaaagc taaagttctg aaatatggaa tgtactgcct ctaatttttc tttgtcttcc 4440

tctcactggc atttttttct ctcccaggtt tcttaagaat aatgtttttt aaaggaggct 4500

ttttgcccat caagaataaa aagaaataaa accaaaaaaa aaaaaaaa 4548

<210> 76
<211> 2124
<212> DNA
<213> Homo sapiens

<400> 76
ggccgcgctg ccgatcgccg ggaggacccc cgcctcgccg aagacgggcg gggcaagccg 60

agcctcacgg ggtccccgga gctgggccgg gcctccagat ggagaaggcg caacggggag 120

ttcttgagta agccagagcg gtgtccagcg cggtgtagcc gcagccgccg ctgtcaggcg 180

cagcaacggg caaccccgta gaagtcggtc ggcaggtcct ctccaacccg ccgctaccgc 240

gccgctgtgg gagagacccc agcaggagcc caaaggcagc tacgggggcg cgaaggccgc 300

tggcgccgcc tcggccagcc cttcccgcgc ggttccactg ccttaaggat gacagtcgta 360

gggaaccctc gaagttggag ctgccagtgg ttgccaatcc tgatactgtt gctgggcaca 420

ggccatgggc caggggtgga aggcgtgaca cactacaagg ccggcgaccc tgttattctg 480

tatgtcaaca aagtgggacc ctaccataac cctcaggaaa cttaccacta ctatcagctt 540

ccagtctgct gccctgagaa gatacgtcac aaaagcctta gcctgggtga agtgctggat 600

ggggaccgaa tggctgagtc tttgtatgag atccgctttc gggaaaacgt ggagaagaga 660

attctgtgcc acatgcagct cagttctgca caggtggagc agctgcgcca ggccattgaa 720

gaactgtact actttgaatt tgtggtagat gacttgccaa tccggggctt tgtgggctac 780

atggaggaga gtggtttcct gccacacagc cacaagatag gactctggac ccatttggac 840

ttccacctag aattccatgg agaccgaatt atatttgcca atgtttcagt gcgggacgtc 900

aagccccaca gcttggatgg gttacgacct gacgagttcc taggccttac ccacacttat 960

agcgtgcgct ggtctgagac ttcagtggag cgtcggagtg acaggcgccg tggtgacgat 1020

ggtggtttct ttcctcgaac actggaaatc cattggttgt ccatcatcaa ctccatggtg 1080

cttgtgtttt tactggtggg ttttgtggct gtcattctaa tgcgtgtgct tcggaatgac 1140

ctggctcggt acaacttaga tgaggagacc acctctgcag gttctggtga tgactttgac 1200

cagggtgaca atggctggaa aattatccat acagatgtct tccgcttccc cccataccgt 1260

ggtctgctct gtgctgtgct tggcgtgggt gcccagttcc tggcccttgg cactggcatt 1320

attgtcatgg cactgctggg catgttcaat gtgcaccgtc atggggccat taactcagca 1380

gccatcttgt tgtatgccct gacctgctgc atctctggct acgtgtccag ccacttctac 1440

cggcagattg gaggcgagcg ttgggtgtgg aacatcattc tcaccaccag tctcttctct 1500

gtgcctttct tcctgacgtg gagtgtggtg aactcagtgc attgggccaa tggttcgaca 1560

caggctctgc cagccacaac catcctgctg cttctgacgg tttggctgct ggtgggcttt 1620

cccctcactg tcattggagg catctttggg aagaacaacg ccagcccctt tgatgcaccc 1680

tgtcgcacca agaacatcgc ccgggagatt ccaccccagc cctggtacaa gtctactgtc 1740

atccacatga ctgttggagg cttcctgcct ttcaggtatc ctccctttat tccatggcta 1800

ttactgtcag gttcctgacc tcaatttttc ctgtccctac tcatccagta ccctaaccca 1860

acccgttgat ccctggttca gtggtaccat tcagagatca ttaaatggtt cctcctatcc 1920

ccaagcagga ctgagcttga atgatatgag agtgtctcac ttataaagct ctccggagac 1980

atttccccct tcaccttcct ggtttctgac tttaatgcct atggacatca tgtggggttt 2040

aaagcccatt tgatgaccca tttactttgt tgaatacctc tttgtgccag gcaaagaata 2100

aagtggaata aaatggaaaa aaaa 2124

<210> 77
<211> 2375
<212> DNA
<213> Homo sapiens

<400> 77
gggcgcggag ccccagccga gcctagccct gcccggcccc ggaggacttg caacactccg 60

aggccaggaa cgctccgtct ggaacggcgc aggtcccagc agctggggtt ccccctcagc 120

ccgtgagcag ccatgtccaa ccccagcgcc ccaccaccat atgaagaccg caaccccctg 180

tacccaggcc ctccgccccc tgggggctat gggcagccat ctgtcctgcc aggagggtat 240

cctgcctacc ctggctaccc gcagcctggc tacggtcacc ctgctggcta cccacagccc 300

atgcccccca cccacccgat gcccatgaac tacggcccag gccatggcta tgatggggag 360

gagagagcgg tgagtgatag cttcgggcct ggagagtggg atgaccggaa agtgcgacac 420

acttttatcc gaaaggttta ctccatcatc tccgtgcagc tgctcatcac tgtggccatc 480

attgctatct tcacctttgt ggaacctgtc agcgcctttg tgaggagaaa tgtggctgtc 540

tactacgtgt cctatgctgt cttcgttgtc acctacctga tccttgcctg ctgccaggga 600

cccagacgcc gtttcccatg gaacatcatt ctgctgaccc tttttacttt tgccatgggc 660

ttcatgacgg gcaccatttc cagtatgtac caaaccaaag ccgtcatcat tgcaatgatc 720

atcactgcgg tggtatccat ttcagtcacc atcttctgct ttcagaccaa ggtggacttc 780

acctcgtgca caggcctctt ctgtgtcctg ggaattgtgc tcctggtgac tgggattgtc 840

actagcattg tgctctactt ccaatacgtt tactggctcc acatgctcta tgctgctctg 900

ggggccattt gtttcaccct gttcctggct tacgacacac agctggtcct ggggaaccgg 960

aagcacacca tcagccccga ggactacatc actggcgccc tgcagattta cacagacatc 1020

atctacatct tcacctttgt gctgcagctg atgggggatc gcaattaagg agcaagcccc 1080

cattttcacc cgatcctggg ctctcccttc caagctagag ggctgggccc tatgactgtg 1140

gtctgggctt taggcccctt tccttcccct tgagtaacat gcccagtttc ctttctgtcc 1200

tggagacagg tggcctctct ggctatggat gtgtgggtac ttggtgggga cggaggagct 1260

agggactaac tgttgctctt ggtgggcttg gcagggacta ggctgaagat gtgtcttctc 1320

cccgccacct actgtatgac accacattct tcctaacagc tggggttgtg aggaatatga 1380

aaagagccta ttcgatagct agaagggaat atgaaaggta gaagtgactt caaggtcacg 1440

aggttcccct cccacctctg tcacaggctt cttgactacg tagttggagc tatttcttcc 1500

cccagcaaag ccagagagct ttgtccccgg cctcctggac acataggcca ttatcctgta 1560

ttcctttggc ttggcatctt ttagctcagg aaggtagaag agatctgtgc ccatgggtct 1620

ccttgcttca atcccttctt gtttcagtga catatgtatt gtttatctgg gttagggatg 1680

ggggacagat aatagaacga gcaaagtaac ctatacaggc cagcatggaa cagcatctcc 1740

cctgggcttg ctcctggctt gtgacgctat aagacagagc aggccacatg tggccatctg 1800

ctccccattc ttgaaagctg ctggggcctc cttgcaggct tctggatctc tggtcagagt 1860

gaactcttgc ttcctgtatt caggcagctc agagcagaaa gtaaggggca gagtcatacg 1920

tgtggccagg aagtagccag ggtgaagaga gactcggtgc gggcagggag aatgcctggg 1980

ggtccctcac ctggctaggg agataccgaa gcctactgtg gtactgaaga cttctgggtt 2040

ctttccttct gctaacccag ggagggtcct aagaggaagg tgacttctct ctgtttgtct 2100

taagttgcac tgggggattt ctgacttgag gcccatctct ccagccagcc actgccttct 2160

ttgtaatatt aagtgccttg agctggaatg gggaaggggg acaagggtca gtctgtcggg 2220

tgggggcaga aatcaaatca gcccaaggat atagttagga ttaattactt aatagagaaa 2280

tcctaactat atcacacaaa gggatacaac tataaatgta ataaagttta tgtctagaag 2340

ttaaaaccca aaaaaaaaaa aaaaaaaaaa aaaaa 2375

<210> 78
<211> 7717
<212> DNA
<213> Homo sapiens

<400> 78
acggaaatga aaggagcact tccgggttcg gcaataacct ggagccggcg gcgtaggttg 60

gctctttagg gcttcacccc gaagctccac cttcgctccc gtctttctgg aaacaccgct 120

ttgatctcgg cggtgcggga caggtacctc ccggctgctg cgggtgccct ggatccagtc 180

ggctgcacca ggcgagcgag acccttccct ggtggaggct cagagttccg gcagggtgca 240

tccggcctgt gtgtggcgcg aggcagggaa gccggtaccc gggtcctggc cccagcgctg 300

acgttttctc tcccctttct tctctcttcg cggttgcggc gtcgcagacg ctagtgtgag 360

cccccatggc agatacgacc ccgaacggcc cccaaggggc gggcgctgtg caattcatga 420

tgaccaataa actggacacg gcaatgtggc tttctcgctt gttcacagtt tactgctctg 480

ctctgtttgt tctgcctctt cttgggttgc atgaagcagc aagcttttac caacgtgctt 540

tgctggcaaa tgctcttacc agtgctctga ggctgcatca aagattacca cacttccagt 600

taagcagagc attcctggcc caggctttgt tagaggacag ctgccactac ctgttgtatt 660

cactcatctt tgtaaattcc tatccagtta caatgagtat cttcccagtc ttgttattct 720

ctttgcttca tgctgccaca tatacgaaaa aggtccttga cgcaaggggc tcaaatagtt 780

tacctctgct gagatctgtc ttggacaaat taagtgctaa tcaacaaaat attctgaaat 840

tcattgcttg caatgaaata ttcctgatgc ctgcgacagt ttttatgctt tttagtggtc 900

aaggaagttt gctccaacct tttatatact atagatttct tacccttcga tattcgtctc 960

gaagaaaccc atattgtcgg accttattta atgaactgag gattgttgtt gaacacataa 1020

taatgaaacc tgcttgccca ctgtttgtga gaagactttg tctccagagc attgccttta 1080

taagcagatt ggcaccaaca gttccatagt ttaacatcta gttaagctac aaatatagta 1140

taagcattat tagcagctgg tacttctgct aggggttgta aattccaggt gttacactga 1200

cctcaatcca atttacataa tttacataaa tgcatctcgg tggaaaaata atcattttct 1260

tggcatgtta aatcaagctt aaaaagtttt gagaaaattt tactgcgctg tgttgctaat 1320

ggttaaagaa gtctgtatct agtgataaat ataccagttt ttttaaaaag atgctgttgt 1380

gcctatatca tgaagtacat taatttctca tgtaaaaaaa atagctctaa aatttgtttc 1440

aacctaattg gtaacctgag tttatatctg gcatgaattc attatggtga tacacatatg 1500

tgaattcagt acattttgag acagtattct accattcagt aattttggtt aatgatttta 1560

acacttctca gtgtatttaa tttcaaattg tttttttaat tggttttatg ctgctttgtt 1620

aggacagatg tgttttgaat gtaccattat aagaagaatt ctatgtatct taaactatga 1680

tcttctaaaa ttttatttcc gtaagtactt ctgtggcctt gagtattttt taaaaggctc 1740

aactgtaagc ctcttagcca gttggataaa tatttggggt cacctagcca ttgaaagcag 1800

aaagcagtag tgacacagct ttcccttcaa agagccattg agaaacattt ctcaaacagg 1860

aaatccttct tttactaatg tggacatata gattattcgt attatagttt gtagaactac 1920

ctagttcaga atcttgactg ccagttttct tggtttctta ggcttgaatt ttcatagaca 1980

attgcaacag tttagatgcc ttttgaaagg aatgtaatga agattcagca tctgactata 2040

tgtgtgtcta tcctgaaata ataatggaga gtatactgta gattacatgt ttacccatca 2100

aatctgactt aaaaggttaa atggaaggtt ttataggtaa ggtaattgat tgggaatggg 2160

gtagggggag gagttgtggg ggaataatgt gcatttcagt ctcaacgcat agataaattt 2220

aggggaattg gatgtattat tcaactttga tttgggttgt aaaatgtgtt aaatcctgtt 2280

cattgaactc ccatcaactc ttataaaatt catgctgatc ttcattaccg ttgcatgatt 2340

ggaaatgttt aaaacattgt acagttttag tatagagaaa tgtaatggtt tttgtgacca 2400

gtttctgtct gcatgtaatt tggatttctc aaatacattc attagtaatt tatcagtaac 2460

attagtttta tttttgttca tctccttatc tataaaaagg ggatattctt aggataaata 2520

catgaaaaat tatacttgat agcttaacta taatcagcta tttttgtatt tttgtaatat 2580

ttgtccacta agctggagaa gcagcctcat acagttgatt ttgtgtatgt ggctagtctt 2640

attgtcacta tgtaagtaat ccaatggttt tagaaactaa actttctaga gcaataaaat 2700

gactataatg ttaagtaaac ataatgttga tttctaatta tgttttaaaa aatgaagtct 2760

tgaattatat caagaaattt tggcagctga agtcatgttt attttgaagc tgttagtttt 2820

ttcctataat ttaaaaagat cttttagatt tatagaagag tcagaaatgt acaagagagt 2880

ttttttgttg ttgtttttgt tttttgagac agagtctgtc tctgtcgcca aggctggagt 2940

gcagtggcgc aatcctggct cactgtagcc tctgcctcct gggttcaagt gcttctcctg 3000

cctcagcctc ccgagtagct gggactacag gtgcacgcca ccacgcctgt agtcccagct 3060

gtattgtaaa aatacaaaat tttagtattt ttagtagaga cagggtttca ccatgttggc 3120

caggatggtc tcgatctcct gacctcgtga tctgcctgcc tcggcctccc aaagtgccgg 3180

gattacaggt gtgagccacc gcgccctgcc aagaagagtt cttttgcata ccctttactc 3240

aggtcctctc atgttaacgt tttacataac tgtagaacat ttatctaaag taagatatta 3300

gcccagaaca atactactaa ctgaagtata aaacttattt gaatttcaac agtttttttt 3360

tcatttctta ttttcctttt gtgtgctctg tttataccat gatccatgat ttttttaaaa 3420

tcatgattgt cttttaaaga tctgtgtgtc tctgttttga gtttttcctg tttattttga 3480

aaagtactgt tggtcaagat aattggtcaa taatccatgt tggttttaac aaaaagcatt 3540

ttaacattaa aaatattaca gtataaaata acactctgtg ctttaaattg aggttttatg 3600

tcattttagc agaattataa tatttctgat atactcatgt ttgacaagtt gaaacagatt 3660

tgtttcttaa aggaaggttt aatatacaaa aaaaggtaat cttaaactta cgaaaaagta 3720

aattttacaa tttgagcatt actagatgtt tagtttgcat gaactcatag ttagaaattc 3780

tgcaatagga atatctacaa ccggctgatt tggaatttga aattatagtg ttacatgtat 3840

acctatcaaa ttaaaattaa ggaaatacaa tagcaatata tagaatgaat gtagtaacag 3900

aaattaactc tttactgcat cattgaactt attgttagtt acaggtttaa aagaagttca 3960

tttaacatcc agtgtgtcta attcttctgg aagtggtgta gtaccattgt tcttctggca 4020

tttttaaata ttaaaccttt ttggatagat ggaagcctta tacaaaatct actttatttt 4080

agcaaggatt ctctgtcctt ttgtatagtt ggtaccttac taatttaaac tctaatatca 4140

atctaaagag aaatttatta tgcaatttgt atttaggttt tttttttttt ttttggaatg 4200

aagttcagag gtagatcctc ctggaagaaa gaaagcaagc gaacttttta aagaaaatta 4260

gacttgaata tttaagaatg tcccttacag agaaaaggcc aactataata ctaagctaaa 4320

agttatgaaa aattaatagg ttcttttata gagctaagaa tgatgaaacc atcaatactt 4380

ccttcttcct aaaaatccag atcaaaactt caggttaggt ttctaagttt aggacatgaa 4440

tattattttt ttctggaaaa gaagatgagt atatgtgtaa taagacaagt agaactgaga 4500

gatttagttt tttttttttt taagttttag ttcagaataa cattaatttt gagagattga 4560

ggtaaagaac cttaactaat gctaaggagt ttattttgat taacataggt tattctgacc 4620

accacctctt ccttccttaa tctccttaga atctgacagt ctcaaagctg tcacacaaat 4680

tagactaatt ttgacacttt gaaatgaaaa cttcaaggaa gaagtagcca cggacagtta 4740

tgtttataat cagtaggtgg cactctttcc tcaggtagcc ccccattttc acatgatgtg 4800

tttgaaggtt aaatgccacc aaaagtgctg agtcagctat aaaactaagt ccctgaattc 4860

catggccctt ttaaatatgt aatcattcaa gattgaaaaa aaaaattaag cattttttgt 4920

ttgtttgctt gtttgttttt gagacggagt ttcactcttg ttggccaggc tggagtgcaa 4980

tggcgccatc tcagctcact gcaacctctg cctcccggat tcaagcaatt ctccttcagc 5040

cctccaagta gctggggtta caggtgcccg ccaccatgcc cagctagttt ttgtattttt 5100

agtagagatg aggtttcacc atgttggcca ggctggtctt gaactcctga cctcgtgatc 5160

cccccacctc ggccttccaa agtgctggat tacaggcgtg agccactgtg cctggcttgc 5220

atttttaaaa tactgaatta ttcaaaagaa gtaccctgtc aatatgtgct ttctaggaaa 5280

acagtaaaat aggccacaat ttggagtgac accattcaga tcaaggtcta tccagttttt 5340

tcttttcatg ctaagtgcct acatcaccga aacacactaa tataaaatta tcctttctcc 5400

ttcattttca gatgtgtaaa aaatggtact taaagtgttt tcatgatcat tttgtaggta 5460

gactagatat agcccgttga acctctttta aaatttagac ttttgatagt aatataaaag 5520

catattgaaa tttgtagata ttatatgagg aatggcacct agatttgaaa attatgcttg 5580

gcttgtagag acaactagtt tctctcgctc tttttttttt tttttttttt ttttttgaga 5640

cagattctca ctcagttgcc caggctggag tgcagtggtg cagtcttggc tcactgcaac 5700

ctctgcctcc tgggttaaag cgattctcat gcctcagcct ccctagtagc tgagactaca 5760

ggcgtgcacc accacgccca gctaattttt gtatttttag tagagacagg atttcaccat 5820

gttcaccatg ttggtcaggc tggtcttgaa ctcctggcct caagtgatct gcccgcctcg 5880

acctcccaga gtgctgggat tataggtgtg agccactaag cctggctgag acaactagtt 5940

tcccttaact cattggaatt ctctaggatt aggagaattc cacagagcct atatgatatt 6000

atagctcaac atttagtata ccaaaggcat acccgtgtaa atctaggagt tatttccaga 6060

gattgtttta aggagcagtc ttatattcag ggtagaaagt tatgattgga tctgctgtta 6120

aggagaacaa aggagcttct aaaggtttgg gaggtttact ggtagtaact attctaggaa 6180

atatttatgt tttaaggtga tgttcacatg ggttctttag aaggaacata gtcaagtgtg 6240

atggattaac tctatatagt ctttctcctc ttgtgcgtgt aggaaatctg acctgcagtg 6300

tcagttgatg tgacaagaga taaagaaagc acagtatttt aaaatctaaa gcagattcct 6360

ttcttagaaa acaataggaa aaaattatag atggatgtct ttgctgaaat ctaacaatta 6420

gctcatattc catgagaaag agtggcctaa gaattatttc atgttaccta gccttctgaa 6480

gctactcact tgatgtgcct agcactttga aactaacctt ttctttcttt gttcatgaca 6540

gtttaattcc aaatatttac tattttctct tgtaactgtt agaacagttc cttttgacat 6600

taatttttgc ctacatatat atttttaagt tgagaccaaa tcggtgaagt gttgagcaag 6660

taacatttat gatgtgtgta tattggaaca aatgtaaaag ggttacaaag attagaaaca 6720

gagtcataaa aaatggcttg atttataaag gcattacttt tggtgcttta tataatggca 6780

tatattgaac taaaaatttg tatatacagt atgtcagcat ttcttagtaa cttctcttga 6840

atccattttt aatatctaat attgtacagg ttggggagtt acattcttca ggccaatact 6900

atccagacta tataaattta taaaataaat tgaaaaattc attcccctgt attcaagacc 6960

aaagcacata aatgctaatg tagggctcag aggggaaata cagttctcct gcatatttga 7020

gaaaatgtga agtcctttca agaaaatcta ataaacataa taatcatagc ctgctgacac 7080

taaggaaaaa ggacctcatt cactctttct tttatgcagt gatttactgg tccctactga 7140

tttccaaatt ggatcacgat agtaaattat ccatgctggt acctgtgaaa gtaagccctg 7200

ggatccatat ttgttttgtg ttctgcttaa atcagcaaga atgataaatt tgatggtgtg 7260

aaattggaag tatcaagggc tttctttggt gattgaggga aataatgtct ctacttgtaa 7320

tttattgtga ccctttttca ctgtatatgc tttgtatgtc taatatttat ttcaatgcaa 7380

attcaattgt tccttcatct gtattgttat atctaagatt ttattgatgt taaaatctaa 7440

ttgtggaata aaaatctctc tggaatttag cagatacaaa aatgttatct tgcaaaagaa 7500

ctaagaacat ttgtagttag aaatcagctt tcctttgagc ttaattgcct tttgttagaa 7560

ataaggtgaa tttgaacaca ctcctcttat cctcagccca tcacaaataa tagagatgcc 7620

atgattttga ggtctgatgt gaaactggta aaaatgtgat ctaaggtgta actggaaaaa 7680

aaaaggaaag aaaaattaca ttgatgcctc agctgtt 7717

<210> 79
<211> 4154
<212> DNA
<213> Homo sapiens

<400> 79
   acttggacgc gcttgcggag gattgcgttg acgagactct tatttattgt caccaacctg 60
   tggtggaatt tgcagttgca cattggatct gattcgcccc gccccgaatg acgcctgccc 120
   ggaggcagtg aaagtacagc cgcgccgccc caagtcagcc tggacacata aatcagcacg 180
   cggccggaga accccgcaat ctctgcgccc acaaaataca ccgacgatgc ccgatctact 240
   ttaagggctg aaacccacgg gcctgagaga ctataagagc gttccctacc gccatggaac 300
   aacggggaca gaacgccccg gccgcttcgg gggcccggaa aaggcacggc ccaggaccca 360
   gggaggcgcg gggagccagg cctgggcccc gggtccccaa gacccttgtg ctcgttgtcg 420
   ccgcggtcct gctgttggtc tcagctgagt ctgctctgat cacccaacaa gacctagctc 480
   cccagcagag agcggcccca caacaaaaga ggtccagccc ctcagaggga ttgtgtccac 540
   ctggacacca tatctcagaa gacggtagag attgcatctc ctgcaaatat ggacaggact 600
   atagcactca ctggaatgac ctccttttct gcttgcgctg caccaggtgt gattcaggtg 660
   aagtggagct aagtccctgc accacgacca gaaacacagt gtgtcagtgc gaagaaggca 720
   ccttccggga agaagattct cctgagatgt gccggaagtg ccgcacaggg tgtcccagag 780
   ggatggtcaa ggtcggtgat tgtacaccct ggagtgacat cgaatgtgtc cacaaagaat 840
   caggtacaaa gcacagtggg gaagtcccag ctgtggagga gacggtgacc tccagcccag 900
   ggactcctgc ctctccctgt tctctctcag gcatcatcat aggagtcaca gttgcagccg 960
   tagtcttgat tgtggctgtg tttgtttgca agtctttact gtggaagaaa gtccttcctt 1020
   acctgaaagg catctgctca ggtggtggtg gggaccctga gcgtgtggac agaagctcac 1080
   aacgacctgg ggctgaggac aatgtcctca atgagatcgt gagtatcttg cagcccaccc 1140
   aggtccctga gcaggaaatg gaagtccagg agccagcaga gccaacaggt gtcaacatgt 1200
   tgtcccccgg ggagtcagag catctgctgg aaccggcaga agctgaaagg tctcagagga 1260
   ggaggctgct ggttccagca aatgaaggtg atcccactga gactctgaga cagtgcttcg 1320
   atgactttgc agacttggtg ccctttgact cctgggagcc gctcatgagg aagttgggcc 1380
   tcatggacaa tgagataaag gtggctaaag ctgaggcagc gggccacagg gacaccttgt 1440
   acacgatgct gataaagtgg gtcaacaaaa ccgggcgaga tgcctctgtc cacaccctgc 1500
   tggatgcctt ggagacgctg ggagagagac ttgccaagca gaagattgag gaccacttgt 1560
   tgagctctgg aaagttcatg tatctagaag gtaatgcaga ctctgccatg tcctaagtgt 1620
   gattctcttc aggaagtcag accttccctg gtttaccttt tttctggaaa aagcccaact 1680
   ggactccagt cagtaggaaa gtgccacaat tgtcacatga ccggtactgg aagaaactct 1740
   cccatccaac atcacccagt ggatggaaca tcctgtaact tttcactgca cttggcatta 1800
   tttttataag ctgaatgtga taataaggac actatggaaa tgtctggatc attccgtttg 1860
   tgcgtacttt gagatttggt ttgggatgtc attgttttca cagcactttt ttatcctaat 1920
   gtaaatgctt tatttattta tttgggctac attgtaagat ccatctacac agtcgttgtc 1980
   cgacttcact tgatactata tgatatgaac cttttttggg tggggggtgc ggggcagttc 2040
   actctgtctc ccaggctgga gtgcaatggt gcaatcttgg ctcactatag ccttgacctc 2100
   tcaggctcaa gcgattctcc cacctcagcc atccaaatag ctgggaccac aggtgtgcac 2160
   caccacgccc ggctaatttt ttgtattttg tctagatata ggggctctct atgttgctca 2220
   gggtggtctc gaattcctgg actcaagcag tctgcccacc tcagactccc aaagcggtgg 2280
   aattagaggc gtgagccccc atgcttggcc ttacctttct acttttataa ttctgtatgt 2340
   tattatttta tgaacatgaa gaaactttag taaatgtact tgtttacata gttatgtgaa 2400
   tagattagat aaacataaaa ggaggagaca tacaatgggg gaagaagaag aagtcccctg 2460
   taagatgtca ctgtctgggt tccagccctc cctcagatgt actttggctt caatgattgg 2520
   caacttctac aggggccagt cttttgaact ggacaacctt acaagtatat gagtattatt 2580
   tataggtagt tgtttacata tgagtcggga ccaaagagaa ctggatccac gtgaagtcct 2640
   gtgtgtggct ggtccctacc tgggcagtct catttgcacc catagccccc atctatggac 2700
   aggctgggac agaggcagat gggttagatc acacataaca ataggtctca tgtcatatcc 2760
   caagtgaact tgagccctgt ttgggctcag gagatagaag acaaaatctg tctcccacgt 2820
   ctgccatggc atcaaggggg aagagtagat ggtgcttgag aatggtgtga aatggttgcc 2880
   atctcaggag tagatggccc ggctcacttc tggttatctg tcaccctgag cccatgagct 2940
   gccttttagg gtacagattg cctacttgag gaccttggcc gctctgtaag catctgactc 3000
   atctcagaaa tgtcaattct taaacactgt ggcaacagga cctagaatgg ctgacgcatt 3060
   aaggttttct tcttgtgtcc tgttctatta ttgttttaag acctcagtaa ccatttcagc 3120
   ctctttccag caaacccttc tccatagtat ttcagtcatg gaaggatcat ttatgcaggt 3180
   agtcattcca ggagtttttg gtcttttctg tctcaaggca ttgtgtgttt tgttccggga 3240
   ctggtttggg tgggacaaag ttagaattgc ctgaagatca cacattcaga ctgttgtgtc 3300
   tgtggagttt taggagtggg gggtgacctt tctggtcttt gcacttccat cctctcccac 3360
   ttccatctgg catcccacgc gttgtcccct gcacttctgg aaggcacagg gtgctgctgc 3420
   ctcctggtct ttgcctttgc tgggccttct gtgcaggacg ctcagcctca gggctcagaa 3480
   ggtgccagtc cggtcccagg tcccttgtcc cttccacaga ggccttccta gaagatgcat 3540
   ctagagtgtc agccttatca gtgtttaaga tttttctttt atttttaatt tttttgagac 3600
   agaatctcac tctctcgccc aggctggagt gcaacggtac gatcttggct cagtgcaacc 3660
   tccgcctcct gggttcaagc gattctcgtg cctcagcctc cggagtagct gggattgcag 3720
   gcacccgcca ccacgcctgg ctaatttttg tatttttagt agagacgggg tttcaccatg 3780
   ttggtcaggc tggtctcgaa ctcctgacct caggtgatcc accttggcct ccgaaagtgc 3840
   tgggattaca ggcgtgagcc accagccagg ccaagctatt cttttaaagt aagcttcctg 3900
   acgacatgaa ataattgggg gttttgttgt ttagttacat taggctttgc tatatcccca 3960
   ggccaaatag catgtgacac aggacagcca tagtatagtg tgtcactcgt ggttggtgtc 4020
   ctttcatgct tctgccctgt caaaggtccc tatttgaaat gtgttataat acaaacaagg 4080
   aagcacattg tgtacaaaat acttatgtat ttatgaatcc atgaccaaat taaatatgaa 4140
   accttatata aaaa 4154

<210> 80
<211> 3809
<212> DNA
<213> Homo sapiens

<400> 80
ggcagccgcg cccgctgggc cacagaggcc gctgaggccg cggcgcccgc cagcctgtcc 60

cgcgccatgg ccccgcgcgc ccggcggcgc cgcccgctgt tcgcgctgct gctgctctgc 120

gcgctgctcg cccggctgca ggtggctttg cagatcgctc ctccatgtac cagtgagaag 180

cattatgagc atctgggacg gtgctgtaac aaatgtgaac caggaaagta catgtcttct 240

aaatgcacta ctacctctga cagtgtatgt ctgccctgtg gcccggatga atacttggat 300

agctggaatg aagaagataa atgcttgctg cataaagttt gtgatacagg caaggccctg 360

gtggccgtgg tcgccggcaa cagcacgacc ccccggcgct gcgcgtgcac ggctgggtac 420

cactggagcc aggactgcga gtgctgccgc cgcaacaccg agtgcgcgcc gggcctgggc 480

gcccagcacc cgttgcagct caacaaggac acagtgtgca aaccttgcct tgcaggctac 540

ttctctgatg ccttttcctc cacggacaaa tgcagaccct ggaccaactg taccttcctt 600

ggaaagagag tagaacatca tgggacagag aaatccgatg cggtttgcag ttcttctctg 660

ccagctagaa aaccaccaaa tgaaccccat gtttacttgc ccggtttaat aattctgctt 720

ctcttcgcgt ctgtggccct ggtggctgcc atcatctttg gcgtttgcta taggaaaaaa 780

gggaaagcac tcacagctaa tttgtggcac tggatcaatg aggcttgtgg ccgcctaagt 840

ggagataagg aaatgtgact ggaaacagta actccacgtt catctccagc gggcaggtga 900

tgaacttcaa gggcgacatc atcgtggtct acgtcagcca gacctcgcag gagggcgcgg 960

cggcggctgc ggagcccatg ggccgcccgg tgcaggagga gaccctggcg cgccgagact 1020

ccttcgcggg gaacggcccg cgcttcccgg acccgtgcgg cggccccgag ggctgcgggg 1080

agccggagaa ggcctcgagg ccggtgcagg agcaaggcgg ggccaaggct tgagcgcccc 1140

ccatggctgg gagcccgaag ctcggagcca gggctcgcga gggcagcacc gcagcctctg 1200

ccccagcccc ggccacccag ggatcgatcg gtacagtcga ggaagaccac ccggcattct 1260

ctgcccactt tgccttccag gaaatgggct tttcaggaag tgaattgatg aggactgtcc 1320

ccatgcccac ggatgctcag cagcccgccg cactggggca gatgtctccc ctgccactcc 1380

tcaaactcgc agcagtaatt tgtggcacta tgacagctat ttttatgact atcctgttct 1440

gtgggggggg gggtctgttt tccccccata tttgtattcc ttttcataac ttttcttgat 1500

atctttcctc cctctttttt aatgtaaagg ttttctcaaa aattctccta aaggtgaggg 1560

tctctttctt ttctcttttc cttttttttt tctttttttg gcaacctggc tctggcccag 1620

gctagagtgc agtggtgcga ttatagcccg gtgcagcctc taactcctgg gctcaagcaa 1680

tccaagtgat cctcccacct caaccttcgg agtagctggg atcacagctg caggccacgc 1740

ccagcttcct ccccccgact ccccccccag agacacggtc ccaccatgtt acccagcctg 1800

gtctcaaact ccccagctaa agcagtcctc cagcctcggc ctcccaaagt actgggatta 1860

caggcgtgag cccccacgct ggcctgcttt acgtattttc ttttgtgccc ctgctcacag 1920

tgttttagag atggctttcc cagtgtgtgt tcattgtaaa cacttttggg aaagggctaa 1980

acatgtgagg cctggagata gttgctaagt tgctaggaac atgtggtggg actttcatat 2040

tctgaaaaat gttctatatt ctcatttttc taaaagaaag aaaaaaggaa acccgattta 2100

tttctcctga atctttttaa gtttgtgtcg ttccttaagc agaactaagc tcagtatgtg 2160

accttacccg ctaggtggtt aatttatcca tgctggcaga ggcactcagg tacttggtaa 2220

gcaaatttct aaaactccaa gttgctgcag cttggcattc ttcttattct agaggtctct 2280

ctggaaaaga tggagaaaat gaacaggaca tggggctcct ggaaagaaag ggcccgggaa 2340

gttcaaggaa gaataaagtt gaaattttaa tttgcatttt ttttgtctag ataagaatag 2400

cgtgaataga tcctctttta ttcgtaaata atcgtgcatc tgtgggttag ccttgtagaa 2460

gtggaaaaca ttccattttc caatgcattt aaatgtaaag ccaaatctgc atgttgtgaa 2520

tttaagaaaa cttattatcc taaaggtgcc tttctcttgg catcatcccg cttgtgagaa 2580

gcctagagga cgctccaggt ggaaggaaat cccctgggtg gttttatctt ttgttaccca 2640

gtgagcactg gttccccgca aatactgggg aaaagcaaaa atacacaagc aagttaaaat 2700

taattttgca catctgggag gttataaaag aaagcactaa tagtagtcac tgcccagact 2760

ttactggcca caaatgccca gctgaagagc atgactgtgg atcactggtt tttccctcct 2820

gctggaaatg ctggggtggt agcggtcgat taggattttc agtggagaag cacaggacag 2880

ttctgtaatt tatgggactc cttagccaac ataaagaact gcaggaaata actgcacagc 2940

caggaggatc cgttggtggg aatttaccgt cattcctgcc cttttattta acatcatcca 3000

cagagagatg ttatacaaat ggaggaaacc attatacctt ttgatatgga atatattaca 3060

gagttacagt tcacaaagta gaatgctgag ctgaaaaccc gagtttctgc tgtgactgtc 3120

atctcactga gcacttcgct tttctttgct tttatttttt ccttctataa aaaggcaata 3180

atgataattt ataatagttt ccctacccag agatattaga agtatgctac agtgaatgtt 3240

aaagtacctt gagatcctta aatcaaaggt gctatataca taagtaagac tctactttca 3300

gaaaaaggta atattatttc ctgcactgat ccctactaat tctatattga tccaaaggca 3360

actcaatgct aaaaaatgta tagaaaatat aagtctgtgt ctgtgtactg tagagatgta 3420

tgtgacaagt gtaaacaaaa tgaactgaag cagtaatgaa cagttattag ggggaacatg 3480

ataaagagat tatattaagc ttatgtttca ccataaaatc ctttttatgg cttactaaaa 3540

ccgagctcac tgtaaaatca tgatccaact tattgctaat ctttatgata tgcttattcc 3600

taatctttat ggtatggtgt caaccgttca tttgtatctt attgctcatt ccctggacca 3660

cagactaggg acagaaaata cttgctttaa taatatatat gctgttgatt tcacaaaaat 3720

ttattaaaat acagcctggg tacccagtga aagagctgaa atggaaatgg agtatcatgt 3780

ttcctactca cattttactc agctgtcgg 3809

<210> 81
<211> 2258
<212> DNA
<213> Homo sapiens

<400> 81
ctcctccagc tcttcctgtc ccgctgttgc aacactgcct cactcttccc ctcccacctt 60

ctctcccctc ctctctgctt taattttctc agaattctct ggactgaggc tccagttctg 120

gcctttgggg ttcaagatca ctgggaccag gccgtgatct ctatgcccga gtctcaaccc 180

tcaactgtca ccccaaggca cttgggacgt cctggacaga ccgagtcccg ggaagcccca 240

gcactgccgc tgccacactg ccctgagccc aaatggggga gtgagaggcc atagctgtct 300

ggcatgggcc tctccaccgt gcctgacctg ctgctgccac tggtgctcct ggagctgttg 360

gtgggaatat acccctcagg ggttattgga ctggtccctc acctagggga cagggagaag 420

agagatagtg tgtgtcccca aggaaaatat atccaccctc aaaataattc gatttgctgt 480

accaagtgcc acaaaggaac ctacttgtac aatgactgtc caggcccggg gcaggatacg 540

gactgcaggg agtgtgagag cggctccttc accgcttcag aaaaccacct cagacactgc 600

ctcagctgct ccaaatgccg aaaggaaatg ggtcaggtgg agatctcttc ttgcacagtg 660

gaccgggaca ccgtgtgtgg ctgcaggaag aaccagtacc ggcattattg gagtgaaaac 720

cttttccagt gcttcaattg cagcctctgc ctcaatggga ccgtgcacct ctcctgccag 780

gagaaacaga acaccgtgtg cacctgccat gcaggtttct ttctaagaga aaacgagtgt 840

gtctcctgta gtaactgtaa gaaaagcctg gagtgcacga agttgtgcct accccagatt 900

gagaatgtta agggcactga ggactcaggc accacagtgc tgttgcccct ggtcattttc 960

tttggtcttt gccttttatc cctcctcttc attggtttaa tgtatcgcta ccaacggtgg 1020

aagtccaagc tctactccat tgtttgtggg aaatcgacac ctgaaaaaga gggggagctt 1080

gaaggaacta ctactaagcc cctggcccca aacccaagct tcagtcccac tccaggcttc 1140

acccccaccc tgggcttcag tcccgtgccc agttccacct tcacctccag ctccacctat 1200

acccccggtg actgtcccaa ctttgcggct ccccgcagag aggtggcacc accctatcag 1260

ggggctgacc ccatccttgc gacagccctc gcctccgacc ccatccccaa cccccttcag 1320

aagtgggagg acagcgccca caagccacag agcctagaca ctgatgaccc cgcgacgctg 1380

tacgccgtgg tggagaacgt gcccccgttg cgctggaagg aattcgtgcg gcgcctaggg 1440

ctgagcgacc acgagatcga tcggctggag ctgcagaacg ggcgctgcct gcgcgaggcg 1500

caatacagca tgctggcgac ctggaggcgg cgcacgccgc ggcgcgaggc cacgctggag 1560

ctgctgggac gcgtgctccg cgacatggac ctgctgggct gcctggagga catcgaggag 1620

gcgctttgcg gccccgccgc cctcccgccc gcgcccagtc ttctcagatg aggctgcgcc 1680

cctgcgggca gctctaagga ccgtcctgcg agatcgcctt ccaaccccac ttttttctgg 1740

aaaggagggg tcctgcaggg gcaagcagga gctagcagcc gcctacttgg tgctaacccc 1800

tcgatgtaca tagcttttct cagctgcctg cgcgccgccg acagtcagcg ctgtgcgcgc 1860

ggagagaggt gcgccgtggg ctcaagagcc tgagtgggtg gtttgcgagg atgagggacg 1920

ctatgcctca tgcccgtttt gggtgtcctc accagcaagg ctgctcgggg gcccctggtt 1980

cgtccctgag cctttttcac agtgcataag cagttttttt tgtttttgtt ttgttttgtt 2040

ttgtttttaa atcaatcatg ttacactaat agaaacttgg cactcctgtg ccctctgcct 2100

ggacaagcac atagcaagct gaactgtcct aaggcagggg cgagcacgga acaatggggc 2160

cttcagctgg agctgtggac ttttgtacat acactaaaat tctgaagtta aagctctgct 2220

cttggaaaaa aaaaaaaaaa aaaaaaaaaa aaaaaaaa 2258

<210> 82
<211> 3682
<212> DNA
<213> Homo sapiens

<400> 82
gcgagcgcag cggagcctgg agagaaggcg ctgggctgcg agggcgcgag ggcgcgaggg 60

cagggggcaa ccggaccccg cccgcaccca tggcgcccgt cgccgtctgg gccgcgctgg 120

ccgtcggact ggagctctgg gctgcggcgc acgccttgcc cgcccaggtg gcatttacac 180

cctacgcccc ggagcccggg agcacatgcc ggctcagaga atactatgac cagacagctc 240

agatgtgctg cagcaaatgc tcgccgggcc aacatgcaaa agtcttctgt accaagacct 300

cggacaccgt gtgtgactcc tgtgaggaca gcacatacac ccagctctgg aactgggttc 360

ccgagtgctt gagctgtggc tcccgctgta gctctgacca ggtggaaact caagcctgca 420

ctcgggaaca gaaccgcatc tgcacctgca ggcccggctg gtactgcgcg ctgagcaagc 480

aggaggggtg ccggctgtgc gcgccgctgc gcaagtgccg cccgggcttc ggcgtggcca 540

gaccaggaac tgaaacatca gacgtggtgt gcaagccctg tgccccgggg acgttctcca 600

acacgacttc atccacggat atttgcaggc cccaccagat ctgtaacgtg gtggccatcc 660

ctgggaatgc aagcatggat gcagtctgca cgtccacgtc ccccacccgg agtatggccc 720

caggggcagt acacttaccc cagccagtgt ccacacgatc ccaacacacg cagccaactc 780

cagaacccag cactgctcca agcacctcct tcctgctccc aatgggcccc agccccccag 840

ctgaagggag cactggcgac ttcgctcttc cagttggact gattgtgggt gtgacagcct 900

tgggtctact aataatagga gtggtgaact gtgtcatcat gacccaggtg aaaaagaagc 960

ccttgtgcct gcagagagaa gccaaggtgc ctcacttgcc tgccgataag gcccggggta 1020

cacagggccc cgagcagcag cacctgctga tcacagcgcc gagctccagc agcagctccc 1080

tggagagctc ggccagtgcg ttggacagaa gggcgcccac tcggaaccag ccacaggcac 1140

caggcgtgga ggccagtggg gccggggagg cccgggccag caccgggagc tcagattctt 1200

cccctggtgg ccatgggacc caggtcaatg tcacctgcat cgtgaacgtc tgtagcagct 1260

ctgaccacag ctcacagtgc tcctcccaag ccagctccac aatgggagac acagattcca 1320

gcccctcgga gtccccgaag gacgagcagg tccccttctc caaggaggaa tgtgcctttc 1380

ggtcacagct ggagacgcca gagaccctgc tggggagcac cgaagagaag cccctgcccc 1440

ttggagtgcc tgatgctggg atgaagccca gttaaccagg ccggtgtggg ctgtgtcgta 1500

gccaaggtgg gctgagccct ggcaggatga ccctgcgaag gggccctggt ccttccaggc 1560

ccccaccact aggactctga ggctctttct gggccaagtt cctctagtgc cctccacagc 1620

cgcagcctcc ctctgacctg caggccaaga gcagaggcag cgagttgtgg aaagcctctg 1680

ctgccatggc gtgtccctct cggaaggctg gctgggcatg gacgttcggg gcatgctggg 1740

gcaagtccct gactctctgt gacctgcccc gcccagctgc acctgccagc ctggcttctg 1800

gagcccttgg gttttttgtt tgtttgtttg tttgtttgtt tgtttctccc cctgggctct 1860

gccccagctc tggcttccag aaaaccccag catccttttc tgcagagggg ctttctggag 1920

aggagggatg ctgcctgagt cacccatgaa gacaggacag tgcttcagcc tgaggctgag 1980

actgcgggat ggtcctgggg ctctgtgcag ggaggaggtg gcagccctgt agggaacggg 2040

gtccttcaag ttagctcagg aggcttggaa agcatcacct caggccaggt gcagtggctc 2100

acgcctatga tcccagcact ttgggaggct gaggcgggtg gatcacctga ggttaggagt 2160

tcgagaccag cctggccaac atggtaaaac cccatctcta ctaaaaatac agaaattagc 2220

cgggcgtggt ggcgggcacc tatagtccca gctactcaga agcctgaggc tgggaaatcg 2280

tttgaacccg ggaagcggag gttgcaggga gccgagatca cgccactgca ctccagcctg 2340

ggcgacagag cgagagtctg tctcaaaaga aaaaaaaaag caccgcctcc aaatgccaac 2400

ttgtcctttt gtaccatggt gtgaaagtca gatgcccaga gggcccaggc aggccaccat 2460

attcagtgct gtggcctggg caagataacg cacttctaac tagaaatctg ccaatttttt 2520

aaaaaagtaa gtaccactca ggccaacaag ccaacgacaa agccaaactc tgccagccac 2580

atccaacccc ccacctgcca tttgcaccct ccgccttcac tccggtgtgc ctgcagcccc 2640

gcgcctcctt ccttgctgtc ctaggccaca ccatctcctt tcagggaatt tcaggaacta 2700

gagatgactg agtcctcgta gccatctctc tactcctacc tcagcctaga ccctcctcct 2760

cccccagagg ggtgggttcc tcttccccac tccccacctt caattcctgg gccccaaacg 2820

ggctgccctg ccactttggt acatggccag tgtgatccca agtgccagtc ttgtgtctgc 2880

gtctgtgttg cgtgtcgtgg gtgtgtgtag ccaaggtcgg taagttgaat ggcctgcctt 2940

gaagccactg aagctgggat tcctccccat tagagtcagc cttccccctc ccagggccag 3000

ggccctgcag aggggaaacc agtgtagcct tgcccggatt ctgggaggaa gcaggttgag 3060

gggctcctgg aaaggctcag tctcaggagc atggggataa aggagaaggc atgaaattgt 3120

ctagcagagc aggggcaggg tgataaattg ttgataaatt ccactggact tgagcttggc 3180

agctgaacta ttggagggtg ggagagccca gccattacca tggagacaag aagggttttc 3240

caccctggaa tcaagatgtc agactggctg gctgcagtga cgtgcacctg tactcaggag 3300

gctgagggga ggatcactgg agcccaggag tttgaggctg cagcgagcta tgatcgcgcc 3360

actacactcc agcctgagca acagagtgag accctgtctc ttaaagaaaa aaaaagtcag 3420

actgctggga ctggccaggt ttctgcccac attggaccca catgaggaca tgatggagcg 3480

cacctgcccc ctggtggaca gtcctgggag aacctcaggc ttccttggca tcacagggca 3540

gagccgggaa gcgatgaatt tggagactct gtggggcctt ggttcccttg tgtgtgtgtg 3600

ttgatcccaa gacaatgaaa gtttgcactg tatgctggac ggcattcctg cttatcaata 3660

aacctgtttg ttttaaaaaa aa 3682

<210> 83
<211> 2894

<212>  DNA
<213>  Homo sapiens

<400>  83
cgagactcca tctcaaaaac aaaacaaata aacgaacaaa aaaacccaca acgtattatt      60

ttcttgttta cgaggtttct tgtctctctg gctccaccag aagaggagca gggacccttc     120

ttgctgttgt tcattgctgc atcccccaca ccgagagcag agcctggcat gggcagaaag     180

tcctcagtcg atatttggtg gccccaagcg aatgaagcat ccaagaaggg aaagctgggg     240

gctccccact gcacttgcca cctgagtcac attttcagaa gcctctggaa agtcgtgcac     300

agcccaggag tgttgagcaa tttcggtttc ctctgaggtt gaaggaccca ggcgtgtcag     360

ccctgctcca gacaccttgg gcatggagga gagtgtcgta cggccctcag tgtttgtggt     420

ggatggacag accgacatcc cattcacgag gctgggacga agccaccgga gacagtcgtg     480

cagtgtggcc cgggtgggtc tgggtctctt gctgttgctg atgggggccg ggctggccgt     540

ccaaggctgg ttcctcctgc agctgcactg gcgtctagga gagatggtca cccgcctgcc     600

tgacggacct gcaggctcct gggagcagct gatacaagag cgaaggtctc acgaggtcaa     660

cccagcagcg catctcacag gggccaactc cagcttgacc ggcagcgggg ggccgctgtt     720

atgggagact cagctgggcc tggccttcct gaggggcctc agctaccacg atggggccct     780

tgtggtcacc aaagctggct actactacat ctactccaag gtgcagctgg gcggtgtggg     840

ctgcccgctg ggcctggcca gcaccatcac ccacggcctc tacaagcgca caccccgcta     900

ccccgaggag ctggagctgt tggtcagcca gcagtcaccc tgcggacggg ccaccagcag     960

ctcccgggtc tggtgggaca gcagcttcct gggtggtgtg gtacacctgg aggctgggga    1020

gaaggtggtc gtccgtgtgc tggatgaacg cctggttcga ctgcgtgatg gtacccggtc    1080

ttacttcggg gctttcatgg tgtgaaggaa ggagcgtggt gcattggaca tgggtctgac    1140

acgtggagaa ctcagagggt gcctcagggg aaagaaaact cacgaagcag aggctggcgg    1200

tggtggctct cgcctgtaat cccagcactt tgggaggcca aggcaggcgg atcacctgag    1260

gtcaggagtt cgagaccagc ctggctaaca tggcaaaacc ccatctctac taaaaataca    1320

aaaattagcc ggacgtggtg gtgcctgcct gtaatccagc tactcaggag gctgaggcag    1380

gataattttg cttaaacccg ggaggcggag gttgcagtga gccgagatca caccactgca    1440

ctccaacctg ggaaacgcag tgagactgtg cctcaaaaaa aagaaaggaa gaaaaaagaa    1500

aactcaggaa acagatcttg ggggacactc cagggaaccc aaaactcaaa ggcggagagc    1560

tcagtgggca ccaccaaggc gagatgaagc cccagcaggc accttcagaa gacccacgta    1620

gactgcagac cctgccacgg acaatactaa ggacaaaaac ccagagactt ggggtctgtg    1680

ggcccccaaa catggggtaa agttgatttg cctgatattc aggaagaagg ggtgaggggt    1740

gggtatttat gcttttgatt cagaagaaag tggggcttgg gattccaggg acttggctgg    1800

gggtgggaaa cttcatccac ttccctactc tcatcatgag tacggacagg gtgggcggga 1860

gactgatcat cgggactcat catgaagagc ccagccccac cccacatact cagatcccac 1920

ccacagactg gtggccacac ctcagcctgg tcacaaagag ttacactcag atacatgagc 1980

acggcagcgt gctcataact gtttaacaac cagctgtcct gggaggggga cagctttgta 2040

atgtttgcca atttccatgg tgtaaatgct accaccatgg ctgatttcat cactgccaag 2100

catagacatc cctaatagga caccacggat ctgtccccgg catccggccc agggcctggc 2160

acaaagcatg ctctagggaa atgcttgctg attgaaagga aggaagaatg actctacagt 2220

cacacctatg gcatcccaca aaatctgtca catggctgca taatctcagc cactctttca 2280

caactataga ctcatacacg cgaagtgcca gattcatgca caaccacaca atcacatgga 2340

agtcacagac ggcatcacag acagtcacag cactgtgtgt atgttataac acaagcacac 2400

aaaactcaga cagcatccca gctacacagc cactcccaga ggtgtcaccg tcacacttgg 2460

taattaatac tcattacatt agacacagac agaccaagtt atagtcagac ctggttacac 2520

acatacacac acacaatatc accatgacaa atacacatta cacacacaca acatcacaat 2580

gacaaacaca cattacacac acaacatcac gatgacaaac acacattaca cacacaacat 2640

cacgatgaca aacacacatt acacacacat cacaatgaca aacacaacat tacacacaca 2700

caacatcaca atgacacaca catcacacac acatcacaat gacaaacaca caacattaca 2760

cacatataca cacagcctga gggccctccc cagcccagac taacacatct cggggtgagg 2820

accagacctt gttcataacc ctgggcctct taaccactga tctttgaaat aaatggcaaa 2880

tagttgtacc tgga 2894

<210> 84
<211> 1680
<212> DNA
<213> Homo sapiens

<400> 84
aaaaagcggc gcgctgtgtc ttcccgcagt ctctcgtcat ggaatacgcc tctgacgctt 60

cactggaccc cgaagccccg tggcctcccg cgccccgcgc tcgcgcctgc cgcgtactgc 120

cttgggccct ggtcgcgggg ctgctgctgc tgctgctgct cgctgccgcc tgcgccgtct 180

tcctcgcctg cccctgggcc gtgtccgggg ctcgcgcctc gcccggctcc gcggccagcc 240

cgagactccg cgagggtccc gagctttcgc ccgacgatcc cgccggcctc ttggacctgc 300

ggcagggcat gtttgcgcag ctggtggccc aaaatgttct gctgatcgat gggcccctga 360

gctggtacag tgacccaggc ctggcaggcg tgtccctgac ggggggcctg agctacaaag 420

aggacacgaa ggagctggtg gtggccaagg ctggagtcta ctatgtcttc tttcaactag 480

agctgcggcg cgtggtggcc ggcgagggct caggctccgt ttcacttgcg ctgcacctgc 540

agccactgcg ctctgctgct ggggccgccg ccctggcttt gaccgtggac ctgccacccg 600

cctcctccga ggctcggaac tcggccttcg gtttccaggg ccgcttgctg cacctgagtg 660

ccggccagcg cctgggcgtc catcttcaca ctgaggccag ggcacgccat gcctggcagc 720

ttacccaggg cgccacagtc ttgggactct tccgggtgac ccccgaaatc ccagccggac 780

tcccttcacc gaggtcggaa taacgtccag cctgggtgca gcccacctgg acagagtccg 840

aatcctactc catccttcat ggagacccct ggtgctgggt ccctgctgct ttctctacct 900

caaggggctt ggcaggggtc cctgctgctg acctcccctt gaggaccctc ctcacccact 960

ccttccccaa gttggacctt gatatttatt ctgagcctga gctcagataa tatattatat 1020

atattatata tatatatata tttctattta aagaggatcc tgagtttgtg aatggacttt 1080

tttagaggag ttgttttggg gggggggggg tcttcgacat tgccgaggct ggtcttgaac 1140

tcctggactt agacgatcct cctgcctcag cctcccaagc aactgggatt catcctttct 1200

attaattcat tgtacttatt tgcttatttg tgtgtattga gcatctgtaa tgtgccagca 1260

ttgtgcccag gctagggggc tatagaaaca tctagaaata gactgaaaga aaatctgagt 1320

tatggtaata cgtgaggaat ttaaagactc atccccagcc tccacctcct gtgtgatact 1380

tgggggctag cttttttctt tctttctttt ttttgagatg gtcttgttct gtcaaccagg 1440

ctagaatgca gcggtgcaat catgagtcaa tgcagcctcc agcctcgacc tcccgaggct 1500

caggtgatcc tcccatctca gcctctcgag tagctgggac cacagttgtg tgccaccaca 1560

cttggctaac tttttaattt ttttgcggag acggtattgc tatgttgcca aggttgttta 1620

catgccagta caatttataa taaacactca tttttcctcc ctctgaaaaa aaaaaaaaaa 1680

<210> 85
<211> 2029
<212> DNA
<213> Homo sapiens

<400> 85
ggcggcgggc ggcgggcggc ggggaccggg tgcggtggtg gctgcggcgg cggcggcggg 60

agcagcatgg attggggcac tgagctgtgg gatcagttcg aggtgctcga gcgccacacg 120

cagtgggggc tggacctgtt ggacagatat gtaaagttcg tgaaagaacg caccgaagtg 180

gaacaggctt acgccaaaca actgcggagc ctggtgaaaa aatatctgcc caagagacct 240

gccaaggatg atcctgagtc caaattcagc cagcaacagt ccttcgtaca gattctccag 300

gaggtgaatg actttgcagg ccagcgggag ctggtggctg agaacctcag tgtccgtgta 360

tgtcttgagc tgaccaagta ctcacaagag atgaaacagg agaggaagat gcacttccaa 420

gaagggcggc gggcccagca gcagctggaa aatggcttta aacagctgga gaatagtaag 480

cgtaaatttg agcgggactg ccgggaggca gagaaggcag cccagactgc tgaacggcta 540

gaccaggata tcaacgccac caaggctgat gtggagaagg ccaagcagca agcccacctt 600

cggagtcaca tggccgaaga aagcaaaaac gaatatgcgg ctcaactgca gcgcttcaac 660

cgagaccaag cccacttcta tttttcacag atgccccaga tattcgataa gctccaagac 720

atggatgaac gcagggccac ccgcctgggt gccgggtatg ggctcctgtc ggaggccgag 780

ctggaggtgg tgcccataat agccaagtgc ttggagggca tgaaggtggc tgcaaatgct 840

gtggatccca agaacgactc ccacgtcctt atagagctgc acaagtcagg ttttgcccgc 900

ccgggcgacg tggaattcga ggacttcagc cagcccatga accgtgcacc ctccgacagc 960

agtctgggca ccccctcgga tggacggcct gaactccgag gcccgggtcg cagccgcacc 1020

aagcgctggc cttttggcaa gaagaacaag acagtggtga ccgaggattt tagccacttg 1080

cccccagagc agcagcgaaa acggcttcaa cagcagttgg aagaacgcag tcgtgaactt 1140

cagaaggagg ttgaccagag ggaagcccta aagaaaatga aggatgtcta tgagaagaca 1200

cctcagatgg gggaccccgc cagcttggag ccccagatcg ctgaaaccct gagcaacatt 1260

gaacggctga aattggaagt gcagaagtat gaggcgtggc tggcagaagc tgaaagtcga 1320

gtccttagca accggggaga cagcctgagc cggcacgccc ggcctcccga cccccccgct 1380

agcgccccgc cagacagcag cagcaacagc gcatcacagg acaccaagga gagctctgaa 1440

gagcctccct cagaagagag ccaggacacc cccatttaca cggagtttga tgaggatttc 1500

gaggaggaac ccacatcccc cataggtcac tgtgtggcca tctaccactt tgaagggtcc 1560

agcgagggca ctatctctat ggccgagggt gaagacctca gtcttatgga agaagacaaa 1620

ggggacggct ggacccgggt caggcggaaa gagggaggcg agggctacgt gcccacctcc 1680

tacctccgag tcacgctcaa ttgaaccctg ccagagacgg gaagaggggg gctgtcggct 1740

gctgcttctg ggccacgggg agccccagga cctatgcact ttatttctga ccccgtggct 1800

tcggctgaga cctgtgtaac ctgctgcccc ctccaccccc aacccagtcc tacctgtcac 1860

accggacgga cccgctgtgc cttctaccat cgttccacca ttgatgtaca tactcatgtt 1920

ttacatcttt tctttctgcc gctcggctcc ggccattttg ttttatacaa aaatgggaaa 1980

aaaaaaaaag aaattatata aagttcctag aaaaaaaaaa aaaaaaaaa 2029

<210> 86
<211> 1762
<212> DNA
<213> Homo sapiens

<400> 86
gacgtcatcg gaggcgtggt cgtccccaaa attagggagg aagaggaaaa aaaaaagcca 60

gaaaaagttt tcttttctgg agtcccaaac gaggtgcggg acggaagagg gggtgaaggc 120

cagaggctcg gggcttcaag accgctgtct ggagtccccc tttccaggcc atgtcggggc 180

ccacctggct gcccccgaag cagccggagc ccgccagagc ccctcagggg agggcgatcc 240

cccgcggcac cccggggcca ccaccggccc acggagcagc actccagccc caccccaggg 300

tcaatttttg cccccttcca tctgagcagt gttaccaggc cccaggggga ccggaggatc 360

gggggccggc gtgggtgggg tcccatggag tactccagca cacgcagggg ctccctgcag 420

acaggggggg ccttcgccct ggaagcctgg acgccgagat agacttgctg agcagcacgc 480

tggccgagct gaatgggggt cggggtcatg cgtcacggcg accagaccga caggcatatg 540

agcccccgcc acctcctgcc taccgcacgg gctccctgaa gccaaatcca gcctcgccgc 600

tcccagcgtc tccctatggg ggccccactc cagcctctta cactaccgcc agcaccccgg 660

ctggcccagc cttccccgtg caagtgaagg tggcacagcc agtgaggggc tgcggcccac 720

ccaggcgggg agcctctcag gcctctgggc ccctcccggg cccccacttt cctctcccag 780

gccgaggtga agtctggggg cctggctata ggagccagag agagccaggg ccaggggcca 840

aagaggaagc tgctggggtc tctggccctg caggaagagg aagaggaggc gagcacgggc 900

cccaggtgcc cctgagccag cctccagagg atgagctgga taggctgacg aagaagctgg 960

ttcacgacat gaaccacccg cccagcgggg agtactttgg ccagtgtggt ggctgcggag 1020

aagatgtggt tggggatggg gctggggttg tggcccttga tcgcgtcttt cacgtgggct 1080

gctttgtatg ttctacatgc cgggcccagc ttcgcggcca gcatttctac gccgtggaga 1140

ggagggcata ttgcgagggc tgctacgtgg ccaccctgga gaaatgtgcc acgtgctccc 1200

agcccatcct ggaccggatc ctgcgggcta tggggaaggc ctaccaccct ggctgcttca 1260

cctgcgtggt gtgtcaccgc ggcctcgacg gcatcccctt cacagtggat gctacgagcc 1320

agatccactg cattgaggac tttcacagga agtttgcccc aagatgctca gtgtgcggtg 1380

gggccataat gcctgagcca ggtcaggagg agactgtgag aattgttgct ctggatcgaa 1440

gttttcacat tggctgttac aagtgcgagg agtgtgggct gctgctctcc tctgagggcg 1500

agtgtcaggg ctgctacccg ctggatgggc acatcttgtg caaggcctgc agcgcctggc 1560

gcatccagga gctctcagcc accgtcacca ctgactgctg agtcttccta gaagtacctg 1620

ctgggttctc agttccagtt cccatccttt gattgatcac tctccctgac atccacctgt 1680

atgactttgt caccaaatgc tgtcttctct ttctccaatc aagaaataat aatccctcga 1740

gtttacaaaa caaaaaaaaa aa 1762

<210> 87
<211> 2783
<212> DNA
<213> Homo sapiens

<400> 87
gattgcgagc caggaggagg aagccggcgg tggccccgtc agcagccggc tgctgagagg 60

ccggtaggcg gcggcggtcc cgaggggcgg cggccgcgct gctccctgag aacgggtccc 120

gcagctgggc aggcgggcgg cctgagggcg cggagccatg aagctgtaca gcctcagcgt 180

cctctacaaa ggcgaggcca aggtggtgct gctcaaagcc gcatacgatg tgtcttcctt 240

cagctttttc cagagatcca gcgttcagga attcatgacc ttcacgagtc aactgattgt 300

ggagcgctca tcgaaaggca ctagagcttc tgtcaaagaa caagactatc tgtgccacgt 360

ctacgtccgg aatgatagtc ttgcaggtgt ggtcattgct gacaatgaat acccatcccg 420

ggtggccttt accttgctgg agaaggtact agatgaattc tccaagcaag tcgacaggat 480

agactggcca gtaggatccc ctgctacaat ccattaccca gccctggatg gtcacctcag 540

tagataccag aacccacgag aagctgatcc catgactaaa gtgcaggccg aactagatga 600

gaccaaaatc attctgcaca acaccatgga gtctctgtta gagcgaggtg agaagctaga 660

tgacttggtg tccaaatccg aggtgctggg aacacagtct aaagccttct ataaaactgc 720

ccggaaacaa aactcatgct gtgccatcat gtgatgcagc ctgccagagg cccaatgctg 780

gaatggcacc atcattcaca tcagaactgc agcccctgga aaagaagaga cagccataga 840

cgaggagcca gagtgggggc agactggcca tttttatttt gaagttcctg cgagaaatgg 900

atggtggaag ggtggcgaat gttcaaattc atatgtgtgg tagtgattct tggaaagaat 960

ttgaggtccc caaaggtgta tttttgggca aatgaaacca taaactccga ctggcttctg 1020

tagatgccaa agggctcttt ttcagctaac cctgggaagg ctctgtggga gggaggtcgg 1080

agccagctgt ttctcgatct ttggtatatc tttggatctt atttgtacat taatgatatt 1140

aacactccag tggggggtgg ggagtccctg atgctagggc tggggtgggt ggagtttgaa 1200

gactcttggg aaagcctctc ctggggccac tgttgggggt gggagtgagc ccaccacaga 1260

ggccacaggc aggcccccac ttcaggccca aggcctgggg cggggggaac agtcactggg 1320

tctcagattc tgagactgtt gtttagctta cctttctgct aggattggct tcccgcagag 1380

ggcagggccc atcctaagca gcttccaagt cccacaaagg tggcttgtgg gaggatttgg 1440

aaggagctgc attgtgggcg gggagtgtgt gggttgggtt cgtaccagca agtagactag 1500

gaactgagcc caggaaaggg ggatgttttc ctggtgtttg gatggtcagc tgggagtgtc 1560

catcatcagg ggaagatcaa acacaggtgc actcagctgc ccagggcctc tgggacactt 1620

gccttgactt gcaacttgcc ttgaacatca cgatcaaagc agcaggtgct gtggtctctc 1680

aaaattgatt tttatttgac tctgtggctc taagactgcc ttgaaccgcc tgaggcctat 1740

gcatctgaac aagtgggtct ctcccttgag caccaggagt gggtgccagc cggccccgag 1800

gattcccagc accccaccta tggtcttgcc agcataggct tgctagttcc ttcttggtca 1860

gaggtagctg cagagggggg aggccaaggg tttggtctaa gctgtgccct gccacctggc 1920

aggaggccca ctcactgccc aagtcatggc aacaggctgg agcagcccag gagatgggcc 1980

taaaatgttc tggatccctt gggtcctagt gttatgttcc agtctgccca cctgtgctca 2040

ggatgcagcc ctgggatcca gcacccatgg aagcttctgc tgggatggtg tcacctatgg 2100

gttttgaacc agtgtggtat ggtccttggg agctctgctc tgagcttgcc acactgctga 2160

gagcacccac tgtcctgacc agagtctcag tggtcctgac ccccaatgtg ggcaggggct 2220

gggcaggagg gtggggtctg ctgtgggttc agaggactcc acctcctggc tggtttacct 2280

gctgctgccc attttctctg ggtactgctg gccagaggac tttagcctac ccctgaagag 2340

cctgtccatg tcattttcct actgccatag ataccctaag cccagggccc cttgaggccc 2400

agactcagcc tgcccactgg tgccggagac ggagtggagt gggcctggat ccgagggatg 2460

ctacctctcc ctttcccact tgaggaccct ggggagagat gggggcgggg aaaatggagg 2520

tatgaatttg gggtaagagg aagtgagatc tccgcttgca ggtcagcccc tgccttgcag 2580

ggcgggctgg cttgactcag gccctgtgag atagagggcc cagcccagcc ccacccacag 2640

atcccctgct cctgttgtgt tctgttgtaa atcatttggc gagactgtat tttagtaact 2700

gctgcctaac ttccctgtgt tctatttgag aggcgcctgt ctggataaag ttgtcttgaa 2760

atttcaaaaa aaaaaaaaaa aaa 2783

<210> 88
<211> 3341
<212> DNA
<213> Homo sapiens

<400> 88
ccgggccccg gcggctgcgc cgagtccccg cccctccctg ctccgtaggg gtaggagggg 60

gccggcggag tttccctccc cgcccagcgg ccctgggcgg gcttttcggc tgcttctcat 120

aagcaggtgg tttcgtttct ccggcacagg taggtttctc tggcaccgat tcggggcctg 180

cccggacttc gccgcacgct gcagaacctc gcccagcgcc caccatgccc cggcagctca 240

gcgcggcggc cgcgctcttc gcgtccctgg ccgtaatttt gcacgatggc agtcaaatga 300

gagcaaaagc atttccagaa accagagatt attctcaacc tactgcagca gcaacagtac 360

aggacataaa aaaacctgtc cagcaaccag ctaagcaagc acctcaccaa actttagcag 420

caagattcat ggatggtcat atcacctttc aaacagcggc cacagtaaaa attccaacaa 480

ctaccccagc gactacaaaa aacactgcaa ccaccagccc aattacctac accctggtca 540

caacccaggc cacacccaac aactcacaca cagctcctcc agttactgaa gttacagtcg 600

gccctagctt agccccttat tcactgccac ccaccatcac cccaccagct catacaactg 660

gaaccagttc atcaaccgtc agccacacaa ctgggaacac cactcaaccc agtaaccaga 720

ccacccttcc agcaacttta tcgatagcac tgcacaaaag cacaaccggt cagaagcctg 780

ttcaacccac ccatgcccca ggaacaacgg cagctgccca caataccacc cgcacagctg 840

cacctgcctc cacggttcct gggcccaccc ttgcacctca gccatcgtca gtcaagactg 900

gaatttatca ggttctaaac ggaagcagac tctgtataaa agcagagatg gggatacagc 960

tgattgttca agacaaggag tcggtttttt cacctcggag atacttcaac atcgacccca 1020

acgcaacgca agcctctggg aactgtggca cccgaaaatc caaccttctg ttgaattttc 1080

agggcggatt tgtgaatctc acatttacca aggatgaaga atcatattat atcagtgaag 1140

tgggagccta tttgaccgtc tcagatccag agacaattta ccaaggaatc aaacatgcgg 1200

tggtgatgtt ccagacagca gtcgggcatt ccttcaagtg cgtgagtgaa cagagcctcc 1260

agttgtcagc ccacctgcag gtgaaaacaa ccgatgtcca acttcaagcc tttgattttg 1320

aagatgacca ctttggaaat gtggatgagt gctcgtctga ctacacaatt gtgcttcctg 1380

tgattggggc catcgtggtt ggtctctgcc ttatgggtat gggtgtctat aaaatccgcc 1440

taaggtgtca atcatctgga taccagagaa tctaattgtt gcccgggggg aatgaaaata 1500

atggaattta gagaactctt tcatcccttc caggatggat gttgggaaat tccctcagag 1560

tgtgggtcct tcaaacaatg taaaccacca tcttctattc aaatgaagtg agtcatgtgt 1620

gatttaagtt caggcagcac atcaatttct aaatactttt tgtttatttt atgaaagata 1680

tagtgagctg tttattttct agtttccttt agaatatttt agccactcaa agtcaacatt 1740

tgagatatgt tgaattaaca taatatatgt aaagtagaat aagccttcaa attataaacc 1800

aagggtcaat tgtaactaat actactgtgt gtgcattgaa gattttattt tacccttgat 1860

cttaacaaag cctttgcttt gttatcaaat ggactttcag tgcttttact atctgtgttt 1920

tatggtttca tgtaacatac atattcctgg tgtagcactt aactcctttt ccactttaaa 1980

tttgtttttg ttttttgaga cggagtttca ctcttgtcac ccaggctgga gtacagtggc 2040

acgatctcgg cttatggcaa cctccgcctc ccgggttcaa gtgattctcc tgcttcagct 2100

tcccgagtag ctgggattac aggcacacac taccacgcct ggctaatttt tgtattttta 2160

ttatagacgg ggtttcacca tgttggccag actggtcttg aactcttgac ctcaggtgat 2220

ccacccacct cagcctccca aagtgctggg attacaggca tgagccattg cgcccggcct 2280

taaatgtttt ttttaatcat caaaaagaac aacatatctc aggttgtcta agtgttttta 2340

tgtaaaacca acaaaaagaa caaatcagct tatatttttt atcttgatga ctcctgctcc 2400

agaatcgcta gactaagaat taggtggcta cagatggtag aactaaacaa taagcaagag 2460

acaataataa tggcccttaa ttattaacaa agtgccagag tctaggctaa gcactttatc 2520

tatatctcat ttcattctca caacttatag gtgaatgagt aaactgagac ttaagggaac 2580

tgaatcactt aaatgtcacc tggctaactg atggcagagc cagagcttga attcatgttg 2640

gtctgacatc aaggtctttg gtcttctccc tacaccaagt tacctacaag aacaatgaca 2700

ccacactctg cctgaaggct cacacctcat accagcatac gctcacctta cagggaaatg 2760

ggtttatcca ggatcatgag acattagggt agatgaaagg agagctttgc agataacaaa 2820

atagcctatc cttaataaat cctccactct ctggaaggag actgaggggc tttgtaaaac 2880

attagtcagt tgctcatttt tatgggattg cttagctggg ctgtaaagat gaaggcatca 2940

aataaactca aagtattttt aaattttttt gataatagag aaacttcgct aaccaactgt 3000

tctttcttga gtgtatagcc ccatcttgtg gtaacttgct gcttctgcac ttcatatcca 3060

tatttcctat tgttcacttt attctgtaga gcagcctgcc aagaatttta tttctgctgt 3120

tttttttgct gctaaagaaa ggaactaagt caggatgtta acagaaaagt ccacataacc 3180

ctagaattct tagtcaagga ataattcaag tcagcctaga gaccatgttg actttcctca 3240

tgtgtttcct tatgactcag taagttggca aggtcctgac tttagtctta ataaaacatt 3300

gaattgtagt aaaggttttt gtaataaaaa cttactttgg a 3341

<210> 89
<211> 3524
<212> DNA
<213> Homo sapiens

<400> 89
caggaagagg tatttcttgg ggatgctacc aaggcagaga ctgtgaagaa ggaagaacgt 60

tgcttgggca aaaggagcat attctcagga gacggggccc ctgcctgcca caccaagcat 120

taggccacca ggaagacccc catctgcaag caagcctagc cttccaggga gaaagaggcc 180

cctgcagctc cttcatcatg aactggcaca tgatcatctc tgggcttatt gtggtagtgc 240

ttaaagttgt tggaatgacc ttatttctac tttatttccc acagattttt aacaaaagta 300

acgatggttt caccaccacc aggagctatg gaacagtctc acagattttt gggagcagtt 360

ccccaagtcc caacggcttc attaccacaa ggagctatgg aacagtctgc cccaaagact 420

gggaatttta tcaagcaaga tgttttttct tatccacttc tgaatcatct tggaatgaaa 480

gcagggactt ttgcaaagga aaaggatcca cattggcaat tgtcaacacg ccagagaaac 540

tgaagtttct tcaggacata actgatgctg agaagtattt tattggctta atttaccatc 600

gtgaagagaa aaggtggcgt tggatcaaca actctgtgtt caatggcaat gttaccaatc 660

agaatcagaa tttcaactgt gcgaccattg gcctaacaaa gacatttgat gctgcatcat 720

gtgacatcag ctaccgcagg atctgtgaga agaatgccaa atgatcacag ttccctgtga 780

caagaactat acttgcaact ctttttgaat ccatacaggt cgtctggcca atgattcttt 840

tacttaccta tctgtctacc agtagcggtc cttgcccatt tgggaaactg agcttctttc 900

ttctgcactg ggggactgga tgctagccat ctccaggaga caggatcagt tttacggaaa 960

caactcagtt agtatagaga tgaggtccgc ttctgtagta ctgagcattt ctgactgatc 1020

aaaaaggcct agtctgttga cagggtttgt tttattttag cctcagagta taccatacta 1080

ctagggagta actgtagagt gagaaattat aaacattatt tagggattac catggtggaa 1140

gagggataaa cataggtcct gtgacttcgt ctctgttctc aagggaaccc cattcacatg 1200

cccctcctaa ctccacaagc gagggtagca gaggctctcc tcagtctgaa ctaaggcttg 1260

gccttgggga gggctcctag tgctgagctt ggagcagcac ggacagcagc attgtttatg 1320

ggaatggaga gaggtctggg caggatagga accttcttgg agaccccttt gaagaaaacc 1380

aggcagccaa gggagccaaa cacactagat ttctgttctt cagcaaagcc ctgaagagac 1440

acttaagcta aaaattccct tgtcatattt ctgaaactcc attataacat atgtaactcc 1500

tttgtaacca aaatttaggt aagcaggctt cctttgctct gaaggttttg agtacctggc 1560

tgtatttgtt gagtattttt aaaattttgg atagtctctt aggcaacaat aatcacaata 1620

tattcatccc ttcagttctg gagaaagcct gataccaggc acagcctact gaccccaagg 1680

agcctggcac tgattggcat cacattgatc tagaactggt ccagccgacg aagagtagga 1740

aaagagaagg gctgctcagg gaaacattgg ctgggggcac ggaataagca catagtaaaa 1800

agggaacatc agggtcaaat ggaaatcacc tgagacagga aacagggagt tcatttggcc 1860

acactggaag aaaggcaaga aagaggaaga caagtcttgg agtaccctgg ctgttctcca 1920

cactcacaag acatcagcta tatactctgc ttggtgcata agaaagagaa aagagatgcc 1980

ttttgtgttt tgagtaagaa taattaaacc ataaggaaga ccatgtataa aactgatgga 2040

aataatagtc accaaagtac agcacatacc attttgtgtc taataacaat gtagcacagt 2100

aatgactgta catgtcattg tatgtatacc aaacaagatt gttgtaaatc atatttttta 2160

ttacaacact aagttctgct tctgcattcc taggtttcat catttttggc tccttagcat 2220

ggccacttac aattttttaa catgagataa cacatcaggt gtcagaactt gcttgaaggg 2280

aattaccaga agtaatttgt gtttgagatg gggtggaaat tggaattata ttagtagccg 2340

gtggagatac aagttctctg actgtgttgg gaaaggataa gtgctaccgt tgagaaggga 2400

agaaaggctg agtctaggtg gagaaaaata tcaacagaac tctagccaaa ggcaagcccc 2460

agaactcaga caacagaaag gaaatcctaa tccttctgtt ttgagaagag agaactgtag 2520

ttgcttcact tcctatttca tgacagaata actgcaaact tttaagatca ggaaatgtag 2580

acatctagtg atttctttag tagacagttt aatttccccc aagattagga gacacttctg 2640

tgcaggttct aaaaggagcc caatggcctg gggtgggagt ggggagtaga tagggaatat 2700

gtgggatttg gtttaagttc atcattggaa gagttcctgg atccttgcaa gcttagataa 2760

atgtgatctt tattagatag cagtggcatg ctttaaaaaa aaaaaaggca atgaaaattt 2820

agcaagccac tgaatttgag ttttcacttt gtttctaata tgctgtgtga atcagtacag 2880

ttttcttacc ctttcttggt cttaatttcc ttactgataa aatggggtag taatacctat 2940

ctcaaaaaat tattgcacat attaaataac attcctctat gtatctcaat ggcattagac 3000

attaggagaa gcattttgtg gaggatttga agttgagatc ttcatccaag aagtagcttt 3060

tcaatttgct agaagcttaa tgtaggcaag ccacttcatt tttcagaact tgtttactca 3120

tttataatat gggaataaaa atttgtgcaa gtcagagaag ggtgccttaa aaatgttgtg 3180

gccaagccac atgagatcaa agacacactt ttcatgacct caaatgtggg cccagcctag 3240

gtcagccaac ccccatccaa cccttagact cacgaacaaa tccacctgag atcagcagag 3300

ccaccctaga tcagctgaaa ctctaagcac aaaaataaaa acttatcact gtataccact 3360

ggagttttct ggttatctct cgtatagcaa aatctaactg atgcaatctc catctggcct 3420

tcatccttct ccctttattg tcctttcgtg tattgttcat ccagcaacca ggatgatctt 3480

gttaaaacat taaacagatt ctgtcactct taaaaaaaaa aaaa 3524

<210> 90
<211> 4787
<212> DNA
<213> Homo sapiens

<400> 90
gagctggtgc tctcccccag cccctaggga attggagctg aggaggagct gaaaatgcag 60

atttagcatc aagcacagac ctacactcgc tctttctctc cggtacacac agctccccac 120

attcgcaccc ctgcccgcgc gccgggccgc ctgactgcac ggcttcccct ccagccagat 180

gctggagaac acacactgat tcgctgcttt ccaagaccct gttcagtctc tttctctata 240

caaagatttt tttaaaaact atatataaga attctttatt tgcaccctcc ctccgagtcc 300

cctgctccgc cagcctgcgc gcctcctagc accacttttc actcccaaag aaggatgaag 360

ggtggttgtg tctcccagtg gaaggcggcc gccgggttcc tcttctgtgt catggttttt 420

gcatctgctg agcgaccggt cttcacgaat cattttcttg tggagttgca taaaggggga 480

gaggacaaag ctcgccaagt tgcagcagaa cacggctttg gagtccgaaa gcttcccttt 540

gctgaaggtc tgtaccactt ttatcacaat ggccttgcaa aggccaagag aagacgcagc 600

ctacaccaca agcagcagct ggagagagac cccagggtaa agatggcttt gcagcaggaa 660

ggatttgacc gaaaaaagcg aggttacaga gacatcaatg agatcgacat caacatgaac 720

gatcctcttt ttacaaagca gtggtatctg atcaatactg ggcaagctga tggcactcct 780

ggccttgatt tgaatgtggc tgaagcctgg gagctgggat acacagggaa aggtgttacc 840

attggaatta tggatgatgg gattgactat ctccacccgg acctggcctc caactataat 900

gccgaagcaa gttacgactt cagcagcaac gacccctatc cttaccctcg gtacacagat 960

gactggttta acagccacgg gacccgatgt gcaggagaag tttctgctgc cgccaacaac 1020

aatatctgtg gagttggagt agcatacaac tccaaggttg caggcatccg gatgctggac 1080

cagccattca tgacagacat catcgaggcc tcctccatca gtcatatgcc acagctgatt 1140

gacatctaca gcgccagctg gggccccaca gacaacggca agacagtgga tgggccccgg 1200

gagctcacgc tgcaggccat ggccgatggc gtgaacaagg gccgcggcgg caaaggcagc 1260

atctacgtgt gggcctccgg ggacggcggc agctatgacg actgcaactg cgacggctac 1320

gcctccagca tgtggaccat ctccatcaac tcagccatca acgacggcag gactgccctg 1380

tacgacgaga gctgctcttc caccttggct tccaccttca gcaacgggag gaaaaggaac 1440

cccgaggccg gtgtggcaac cacagatttg tacggcaact gcactctgag gcattctggg 1500

acatctgcag ctgcccccga ggcagctggt gtgtttgcac tggctctgga ggctaacctg 1560

ggtctgacct ggcgggacat gcagcatctg actgtgctca cctccaaacg gaaccagctt 1620

cacgacgagg tccatcagtg gcggcgcaat ggggtcggcc tggaatttaa tcacctcttt 1680

ggctacgggg tccttgatgc aggtgccatg gtgaaaatgg ctaaagactg gaaaaccgtg 1740

cctgagagat tccactgtgt gggaggctcc gtgcaggacc ctgagaaaat accatccact 1800

ggcaagttgg tgctgacact cacaaccgac gcctgtgagg ggaaggaaaa ttttgtccgc 1860

tacctggagc atgtccaggc tgtcatcacg gtcaacgcaa ccagaagagg agacctgaac 1920

atcaacatga cttcccctat gggcaccaag tccattttgc tgagccggcg tccaagggat 1980

gacgactcca aggtgggctt tgacaagtgg cctttcatga ccactcacac gtggggggaa 2040

gacgcccgag gcacctggac cctggagctg ggatttgtcg gcagcgcccc gcagaagggg 2100

gtgctgaagg agtggaccct gatgctgcat ggcactcaga gtgccccgta catcgaccag 2160

gtggtgcggg attaccagtc caagttggcc atgtccaaga aagaggagct ggaggaagag 2220

ctggacgaag ccgtggagag aagcctgaaa agcatcctta acaagaacta gcgctgcaca 2280

tccgcctttc ccaccgccct ccctccccag ctccgcctct gtcctcgctc cacgtttcag 2340

gcaggcacct agcaattcca tcacccgtac aggcaattcc gtcttcttaa tctgaagctt 2400

cactcactgt caatgattat tttcattaca atggaaacaa tcttttttac tctatgcccc 2460

aaatatagcg ttcccaacaa catccatgtc ctatgtgtga ctctaaattc tttatttctg 2520

tcattcaaat gggtgatatc ctgaaaaaaa aaaaaaaaaa aaaactggga cagctttccc 2580

ctcatttttt tttttgtttc tgagaaaaga acgtatttta aaagccacat agagtgactc 2640

caagaacaat tgtccatggt ctcaaacaag gggctgttac ataacaagaa aatcaaagct 2700

gaggacaggg tgtgagcgcc acatctctga aagcacagga gacactgtgc tataaatcct 2760

ttggggagcg atgttttgaa tttagtgaga tttaccaggg atgtagatta aggtgatgtg 2820

attcaaaaga tgccattcat agagagccct agttactgca tggggaaaga gatccaggaa 2880

gcatgagtgc tggatatttt actaccaatg ccaagataat tcactctact cagccggcgt 2940

ggcaaatata aaacttacag agcgtggctg tgctctcacc agctgctgct ctgagttatg 3000

ttaaaatccg ctagagcagc ccaaattttt ctcagtttgt atagagttca tcccagcccc 3060

aattttctgg ggctcctcac atagctaccc aaaagagaaa aaaaattaag acaagcctgg 3120

caacacacct ggtgaagagt agtttactag cttttcaaac aagaatgtcc cttttcctaa 3180

gtcactttga ggtgtctcaa tctgatctga gtgagaggcg acaggagtat tttttttttt 3240

ttacagcttt acacacacag atgtgggctt tgatttccaa gtaatataat ggaagagaaa 3300

tctcatactc ccccacagtt tgatgtcatt aatgtgttgg gaaaaaggcc tctgtcccgg 3360

aagagtcatg ggaggtgaaa ggggcacgtt tgaagatgcg agcgctatct tcacatagtt 3420

ctccagttgt atggagcctc ttctgccaag agagggccat gcaattcatc ccagaggaac 3480

ctgaggcctg aaggaggtga gagaagacct ctgtgaggaa agcacacagt caccttctcg 3540

gcaactaagc agtccctgag accatttaac atgcaacccg aaggttatgg tcaatcccaa 3600

aagtcaccac tccattccca actagacatt accaaagtga cctacccaga gattgcttct 3660

catccccagt cccaatgcac atccattccc aagaaatgct ttgtcttcag cctctccagg 3720

caccatctcc cttcctgtgg gagcagagag cttagcctgg agcacctttc cttcaagcca 3780

gcaacacaga gcactaggtt caattccctg aaggtggcca ctttaagaga gaaatctgaa 3840

aaccccattt gctttctttt ctcccatatt ggcatggatt tctgtcttct ctaacacctt 3900

gtgaccttct ctatatcatg ctttaaagtg taataatatg attttttaaa agaaatttat 3960

tacttgttgc aaaggtcttt ttaaaccagt ttagatttca agaaaaaata aatggaaatc 4020

atcgaaaatt catttcacat taatggtcta aaaataaacc aaaggacatt atgtgtgcat 4080

gtgtgtataa gtgcacacag aaatatatat acatatgtag actatataca tgtgtgtata 4140

tatgtgtata tatacataca cttgtataaa tgtatataca catataccta taatgtgtgt 4200

atgtgtattt attgaagaaa cagataccat actcatttct aaaagaatat tcagagaata 4260

tcaagatgat tctggctgaa aaaggccagt ggaaattcag gtgaaaatgt tcatcaattc 4320

ccattgcatc acctctgtaa tttttcagct ctctgtataa acattaaatg tcttatatag 4380

cagcaaaaat ataaaatagt tgtccatatt ttcacaggtg tggtgtaatt tataaaatta 4440

gaaagcaact tatcagctac ttaagagaaa tggcaagttt tgatatgagt atacaatata 4500

taaaaatata tatagtgcta tatatataaa tatttggtct ctatttcatt ttttgcatca 4560

gtattaatac taaaatatgt ctcgctagtg atgtttttat gatatccctg atcctaactg 4620

aagagacagt tatttatagt catttatttt aaaaaatgaa aataagtgaa taataattag 4680

gttaacattg ttgctccctg tgacaaaatt ttataagcaa atttcaaaag acatgttgta 4740

aattaggagg ctcaacaata aaacattatg ctccagaaaa aaaaaaa 4787

<210>  91
<211>  5820
<212>  DNA
<213>  Homo sapiens

<400>  91
agccgctgcg cccgagctgg cctgcgagtt cagggctcct gtcgctctcc aggagcaacc      60

tctactccgg acgcacaggc attccccgcg cccctccagc cctcgccgcc ctcgccaccg     120

ctcccggccg ccgcgctccg gtacacacag gatccctgct gggcaccaac agctccacca     180

tggggctggc ctggggacta ggcgtcctgt tcctgatgca tgtgtgtggc accaaccgca     240

ttccagagtc tggcggagac aacagcgtgt ttgacatctt tgaactcacc ggggccgccc     300

gcaaggggtc tgggcgccga ctggtgaagg gccccgaccc ttccagccca gctttccgca     360

tcgaggatgc caacctgatc ccccctgtgc ctgatgacaa gttccaagac ctggtggatg     420

ctgtgcgggc agaaaagggt ttcctccttc tggcatccct gaggcagatg aagaagaccc     480

ggggcacgct gctggccctg gagcggaaag accactctgg ccaggtcttc agcgtggtgt     540

ccaatggcaa ggcgggcacc ctggacctca gcctgaccgt ccaaggaaag cagcacgtgg     600

tgtctgtgga agaagctctc ctggcaaccg gccagtggaa gagcatcacc ctgtttgtgc     660

aggaagacag ggcccagctg tacatcgact gtgaaaagat ggagaatgct gagttggacg     720

tccccatcca aagcgtcttc accagagacc tggccagcat cgccagactc cgcatcgcaa     780

aggggggcgt caatgacaat ttccaggggg tgctgcagaa tgtgaggttt gtctttggaa     840

ccacaccaga agacatcctc aggaacaaag gctgctccag ctctaccagt gtcctcctca     900

cccttgacaa caacgtggtg aatggttcca gccctgccat ccgcactaac tacattggcc     960

acaagacaaa ggacttgcaa gccatctgcg gcatctcctg tgatgagctg tccagcatgg    1020

tcctggaact caggggcctg cgcaccattg tgaccacgct gcaggacagc atccgcaaag    1080

tgactgaaga gaacaaagag ttggccaatg agctgaggcg gcctccccta tgctatcaca    1140

acggagttca gtacagaaat aacgaggaat ggactgttga tagctgcact gagtgtcact    1200

gtcagaactc agttaccatc tgcaaaaagg tgtcctgccc catcatgccc tgctccaatg    1260

ccacagttcc tgatggagaa tgctgtcctc gctgttggcc cagcgactct gcggacgatg    1320

gctggtctcc atggtccgag tggacctcct gttctacgag ctgtggcaat ggaattcagc    1380

agcgcggccg ctcctgcgat agcctcaaca accgatgtga gggctcctcg gtccagacac    1440

ggacctgcca cattcaggag tgtgacaaga gatttaaaca ggatggtggc tggagccact    1500

ggtccccgtg gtcatcttgt tctgtgacat gtggtgatgg tgtgatcaca aggatccggc    1560

tctgcaactc tcccagcccc cagatgaacg ggaaaccctg tgaaggcgaa gcgcgggaga    1620

ccaaagcctg caagaaagac gcctgcccca tcaatggagg ctggggtcct tggtcaccat    1680

gggacatctg ttctgtcacc tgtggaggag gggtacagaa acgtagtcgt ctctgcaaca 1740

accccacacc ccagtttgga ggcaaggact gcgttggtga tgtaacagaa aaccagatct 1800

gcaacaagca ggactgtcca attgatggat gcctgtccaa tccctgcttt gccggcgtga 1860

agtgtactag ctaccctgat ggcagctgga aatgtggtgc ttgtccccct ggttacagtg 1920

gaaatggcat ccagtgcaca gatgttgatg agtgcaaaga agtgcctgat gcctgcttca 1980

accacaatgg agagcaccgg tgtgagaaca cggaccccgg ctacaactgc ctgccctgcc 2040

ccccacgctt caccggctca cagcccttcg gccagggtgt cgaacatgcc acggccaaca 2100

aacaggtgtg caagccccgt aacccctgca cggatgggac ccacgactgc aacaagaacg 2160

ccaagtgcaa ctacctgggc cactatagcg accccatgta ccgctgcgag tgcaagcctg 2220

gctacgctgg caatggcatc atctgcgggg aggacacaga cctggatggc tggcccaatg 2280

agaacctggt gtgcgtggcc aatgcgactt accactgcaa aaaggataat tgccccaacc 2340

ttcccaactc agggcaggaa gactatgaca aggatggaat tggtgatgcc tgtgatgatg 2400

acgatgacaa tgataaaatt ccagatgaca gggacaactg tccattccat tacaacccag 2460

ctcagtatga ctatgacaga gatgatgtgg gagaccgctg tgacaactgt ccctacaacc 2520

acaacccaga tcaggcagac acagacaaca atggggaagg agacgcctgt gctgcagaca 2580

ttgatggaga cggtatcctc aatgaacggg acaactgcca gtacgtctac aatgtggacc 2640

agagagacac tgatatggat ggggttggag atcagtgtga caattgcccc ttggaacaca 2700

atccggatca gctggactct gactcagacc gcattggaga tacctgtgac aacaatcagg 2760

atattgatga agatggccac cagaacaatc tggacaactg tccctatgtg cccaatgcca 2820

accaggctga ccatgacaaa gatggcaagg gagatgcctg tgaccacgat gatgacaacg 2880

atggcattcc tgatgacaag gacaactgca gactcgtgcc caatcccgac cagaaggact 2940

ctgacggcga tggtcgaggt gatgcctgca aagatgattt tgaccatgac agtgtgccag 3000

acatcgatga catctgtcct gagaatgttg acatcagtga gaccgatttc cgccgattcc 3060

agatgattcc tctggacccc aaagggacat cccaaaatga ccctaactgg gttgtacgcc 3120

atcagggtaa agaactcgtc cagactgtca actgtgatcc tggactcgct gtaggttatg 3180

atgagtttaa tgctgtggac ttcagtggca ccttcttcat caacaccgaa agggacgatg 3240

actatgctgg atttgtcttt ggctaccagt ccagcagccg cttttatgtt gtgatgtgga 3300

agcaagtcac ccagtcctac tgggacacca accccacgag ggctcaggga tactcgggcc 3360

tttctgtgaa agttgtaaac tccaccacag ggcctggcga gcacctgcgg aacgccctgt 3420

ggcacacagg aaacacccct ggccaggtgc gcaccctgtg gcatgaccct cgtcacatag 3480

gctggaaaga tttcaccgcc tacagatggc gtctcagcca caggccaaag acgggtttca 3540

ttagagtggt gatgtatgaa gggaagaaaa tcatggctga ctcaggaccc atctatgata 3600

aaacctatgc tggtggtaga ctagggttgt ttgtcttctc tcaagaaatg gtgttcttct 3660

ctgacctgaa atacgaatgt agagatccct aatcatcaaa ttgttgattg aaagactgat 3720

cataaaccaa tgctggtatt gcaccttctg gaactatggg cttgagaaaa cccccaggat 3780

cacttctcct tggcttcctt cttttctgtg cttgcatcag tgtggactcc tagaacgtgc 3840

gacctgcctc aagaaaatgc agttttcaaa aacagactca gcattcagcc tccaatgaat 3900

aagacatctt ccaagcatat aaacaattgc tttggtttcc ttttgaaaaa gcatctactt 3960

gcttcagttg ggaaggtgcc cattccactc tgcctttgtc acagagcagg gtgctattgt 4020

gaggccatct ctgagcagtg gactcaaaag cattttcagg catgtcagag aagggaggac 4080

tcactagaat tagcaaacaa aaccaccctg acatcctcct tcaggaacac ggggagcaga 4140

ggccaaagca ctaaggggag ggcgcatacc cgagacgatt gtatgaagaa aatatggagg 4200

aactgttaca tgttcggtac taagtcattt tcaggggatt gaaagactat tgctggattt 4260

catgatgctg actggcgtta gctgattaac ccatgtaaat aggcacttaa atagaagcag 4320

gaaagggaga caaagactgg cttctggact tcctccctga tccccaccct tactcatcac 4380

ctgcagtggc cagaattagg gaatcagaat caaaccagtg taaggcagtg ctggctgcca 4440

ttgcctggtc acattgaaat tggtggcttc attctagatg tagcttgtgc agatgtagca 4500

ggaaaatagg aaaacctacc atctcagtga gcaccagctg cctcccaaag gaggggcagc 4560

cgtgcttata tttttatggt tacaatggca caaaattatt atcaacctaa ctaaaacatt 4620

ccttttctct tttttcctga attatcatgg agttttctaa ttctctcttt tggaatgtag 4680

atttttttta aatgctttac gatgtaaaat atttattttt tacttattct ggaagatctg 4740

gctgaaggat tattcatgga acaggaagaa gcgtaaagac tatccatgtc atctttgttg 4800

agagtcttcg tgactgtaag attgtaaata cagattattt attaactctg ttctgcctgg 4860

aaatttaggc ttcatacgga aagtgtttga gagcaagtag ttgacattta tcagcaaatc 4920

tcttgcaaga acagcacaag gaaaatcagt ctaataagct gctctgcccc ttgtgctcag 4980

agtggatgtt atgggattct ttttttctct gttttatctt ttcaagtgga attagttggt 5040

tatccatttg caaatgtttt aaattgcaaa gaaagccatg aggtcttcaa tactgtttta 5100

ccccatccct tgtgcatatt tccagggaga aggaaagcat atacactttt ttctttcatt 5160

tttccaaaag agaaaaaaat gacaaaaggt gaaacttaca tacaaatatt acctcatttg 5220

ttgtgtgact gagtaaagaa tttttggatc aagcggaaag agtttaagtg tctaacaaac 5280

ttaaagctac tgtagtacct aaaaagtcag tgttgtacat agcataaaaa ctctgcagag 5340

aagtattccc aataaggaaa tagcattgaa atgttaaata caatttctga aagttatgtt 5400

ttttttctat catctggtat accattgctt tatttttata aattattttc tcattgccat 5460

tggaatagat atctcagatt gtgtagatat gctatttaaa taatttatca ggaaatactg 5520

cctgtagagt tagtatttct atttttatat aatgtttgca cactgaattg aagaattgtt 5580

ggttttttct tttttttgtt ttgttttttt tttttttttt ttttgctttt gacctcccat 5640

ttttactatt tgccaatacc tttttctagg aatgtgcttt tttttgtaca catttttatc 5700

cattttacat tctaaagcag tgtaagttgt atattactgt ttcttatgta caaggaacaa 5760

caataaatca tatggaaatt tatatttata aaaaaaaaaa aaaaaaaaaa aaaaaaaaaa 5820

<210> 92
<211> 1648
<212> DNA
<213> Homo sapiens

<400> 92
cagagaaggc ttaggctccc gagtcaacag ggcattcacc gcctggggcg cctgagtcat 60

caggacactg ccaggagaca cagaacccta gatgccctgc agaatccttc ctgttacggt 120

ccccctccct gaaacatcct tcattgcaat atttccagga aaggaagggg gctggctcgg 180

aggaagagag gtggggaggt gatcagggtt cacagaggag ggaactgaat gacatcccag 240

gattacataa actgtcagag gcagccgaag agttcacaag tgtgaagcct ggaagccggc 300

gggtgccgct gtgtaggaaa gaagctaaag cacttccaga gcctgtccgg agctcagagg 360

ttcggaagac ttatcgacca tggagcgcgc gtcctgcttg ttgctgctgc tgctgccgct 420

ggtgcacgtc tctgcgacca cgccagaacc ttgtgagctg gacgatgaag atttccgctg 480

cgtctgcaac ttctccgaac ctcagcccga ctggtccgaa gccttccagt gtgtgtctgc 540

agtagaggtg gagatccatg ccggcggtct caacctagag ccgtttctaa agcgcgtcga 600

tgcggacgcc gacccgcggc agtatgctga cacggtcaag gctctccgcg tgcggcggct 660

cacagtggga gccgcacagg ttcctgctca gctactggta ggcgccctgc gtgtgctagc 720

gtactcccgc ctcaaggaac tgacgctcga ggacctaaag ataaccggca ccatgcctcc 780

gctgcctctg gaagccacag gacttgcact ttccagcttg cgcctacgca acgtgtcgtg 840

ggcgacaggg cgttcttggc tcgccgagct gcagcagtgg ctcaagccag gcctcaaggt 900

actgagcatt gcccaagcac actcgcctgc cttttcctgc gaacaggttc gcgccttccc 960

ggcccttacc agcctagacc tgtctgacaa tcctggactg ggcgaacgcg gactgatggc 1020

ggctctctgt ccccacaagt tcccggccat ccagaatcta gcgctgcgca acacaggaat 1080

ggagacgccc acaggcgtgt gcgccgcact ggcggcggca ggtgtgcagc cccacagcct 1140

agacctcagc cacaactcgc tgcgcgccac cgtaaaccct agcgctccga gatgcatgtg 1200

gtccagcgcc ctgaactccc tcaatctgtc gttcgctggg ctggaacagg tgcctaaagg 1260

actgccagcc aagctcagag tgctcgatct cagctgcaac agactgaaca gggcgccgca 1320

gcctgacgag ctgcccgagg tggataacct gacactggac gggaatccct tcctggtccc 1380

tggaactgcc ctcccccacg agggctcaat gaactccggc gtggtcccag cctgtgcacg 1440

ttcgaccctg tcggtggggg tgtcgggaac cctggtgctg ctccaagggg cccggggctt 1500

tgcctaagat ccaagacaga ataatgaatg gactcaaact gccttggctt caggggagtc 1560

ccgtcaggac gttgaggact tttcgaccaa ttcaaccctt tgccccacct ttattaaaat 1620

cttaaacaac gggtcaaaaa aaaaaaaa 1648

<210> 93
<211> 1616
<212> DNA
<213> Homo sapiens

<400> 93
   gccaaggctg gggcagggga gtcagcagag gcctcgctcg ggcgcccagt ggtcctgccg 60
   cctggtctca cctcgctatg gttcgtctgc ctctgcagtg cgtcctctgg ggctgcttgc 120
   tgaccgctgt ccatccagaa ccacccactg catgcagaga aaaacagtac ctaataaaca 180
   gtcagtgctg ttctttgtgc cagccaggac agaaactggt gagtgactgc acagagttca 240
   ctgaaacgga atgccttcct tgcggtgaaa gcgaattcct agacacctgg aacagagaga 300
   cacactgcca ccagcacaaa tactgcgacc ccaacctagg gcttcgggtc cagcagaagg 360
   gcacctcaga aacagacacc atctgcacct gtgaagaagg ctggcactgt acgagtgagg 420
   cctgtgagag ctgtgtcctg caccgctcat gctcgcccgg ctttggggtc aagcagattg 480
   ctacaggggt ttctgatacc atctgcgagc cctgcccagt cggcttcttc tccaatgtgt       540
   catctgcttt cgaaaaatgt cacccttgga caagctgtga gaccaaagac ctggttgtgc       600
   aacaggcagg cacaaacaag actgatgttg tctgtggtcc ccaggatcgg ctgagagccc 660
   tggtggtgat ccccatcatc ttcgggatcc tgtttgccat cctcttggtg ctggtcttta 720
   tcaaaaaggt ggccaagaag ccaaccaata aggcccccca ccccaagcag gaaccccagg 780
   agatcaattt tcccgacgat cttcctggct ccaacactgc tgctccagtg caggagactt 840
   tacatggatg ccaaccggtc acccaggagg atggcaaaga gagtcgcatc tcagtgcagg 900
   agagacagtg aggctgcacc cacccaggag tgtggccacg tgggcaaaca ggcagttggc 960
   cagagagcct ggtgctgctg ctgctgtggc gtgagggtga ggggctggca ctgactgggc 1020
   atagctcccc gcttctgcct gcacccctgc agtttgagac aggagacctg gcactggatg 1080
   cagaaacagt tcaccttgaa gaacctctca cttcaccctg gagcccatcc agtctcccaa 1140
   cttgtattaa agacagaggc agaagtttgg tggtggtggt gttggggtat ggtttagtaa 1200
   tatccaccag accttccgat ccagcagttt ggtgcccaga gaggcatcat ggtggcttcc 1260
   ctgcgcccag gaagccatat acacagatgc ccattgcagc attgtttgtg atagtgaaca 1320
   actggaagct gcttaactgt ccatcagcag gagactggct aaataaaatt agaatatatt 1380
   tatacaacag aatctcaaaa acactgttga gtaaggaaaa aaaggcatgc tgctgaatga 1440
   tgggtatgga actttttaaa aaagtacatg cttttatgta tgtatattgc ctatggatat 1500
   atgtataaat acaatatgca tcatatattg atataacaag ggttctggaa gggtacacag 1560
   aaaacccaca gctcgaagag tggtgacgtc tggggtgggg aagaagggtc tggggg 1616

<210> 94
<211> 2757
<212> DNA
<213> Homo sapiens

<400> 94
gacaagtact gagtgaactc aaaccctctg taaagtaaca gaagttagaa ggggaaatgt 60

cgcctctctg aagattaccc aaagaaaaag tgatttgtca ttgctttata gactgtaaga 120

agagaacatc tcagaagtgg agtcttaccc tgaaatcaaa ggatttaaag aaaaagtgga 180

atttttcttc agcaagctgt gaaactaaat ccacaacctt tggagaccca ggaacaccct 240

ccaatctctg tgtgttttgt aaacatcact ggagggtctt ctacgtgagc aattggattg 300

tcatcagccc tgcctgtttt gcacctggga agtgccctgg tcttacttgg gtccaaattg 360

ttggctttca cttttgaccc taagcatctg aagccatggg ccacacacgg aggcagggaa 420

catcaccatc caagtgtcca tacctcaatt tctttcagct cttggtgctg gctggtcttt 480

ctcacttctg ttcaggtgtt atccacgtga ccaaggaagt gaaagaagtg gcaacgctgt 540

cctgtggtca caatgtttct gttgaagagc tggcacaaac tcgcatctac tggcaaaagg 600

agaagaaaat ggtgctgact atgatgtctg gggacatgaa tatatggccc gagtacaaga 660

accggaccat ctttgatatc actaataacc tctccattgt gatcctggct ctgcgcccat 720

ctgacgaggg cacatacgag tgtgttgttc tgaagtatga aaaagacgct ttcaagcggg 780

aacacctggc tgaagtgacg ttatcagtca aagctgactt ccctacacct agtatatctg 840

actttgaaat tccaacttct aatattagaa ggataatttg ctcaacctct ggaggttttc 900

cagagcctca cctctcctgg ttggaaaatg gagaagaatt aaatgccatc aacacaacag 960

tttcccaaga tcctgaaact gagctctatg ctgttagcag caaactggat ttcaatatga 1020

caaccaacca cagcttcatg tgtctcatca agtatggaca tttaagagtg aatcagacct 1080

tcaactggaa tacaaccaag caagagcatt ttcctgataa cctgctccca tcctgggcca 1140

ttaccttaat ctcagtaaat ggaatttttg tgatatgctg cctgacctac tgctttgccc 1200

caagatgcag agagagaagg aggaatgaga gattgagaag ggaaagtgta cgccctgtat 1260

aacagtgtcc gcagaagcaa ggggctgaaa agatctgaag gtcccacctc catttgcaat 1320

tgacctcttc tgggaacttc ctcagatgga caagattacc ccaccttgcc ctttacgtat 1380

ctgctcttag gtgcttcttc acttcagttg ctttgcagga agtgtctaga ggaatatggt 1440

gggcacagaa gtagctctgg tgaccttgat caaggtgttt tgaaatgcag aattcttgag 1500

ttctggaagg gactttagag aataccagtg ttattaatga caaaggcact gaggcccagg 1560

gaggtgaccc gaattataaa ggccagcgcc agaacccaga tttcctaact ctggtgctct 1620

ttccctttat cagtttgact gtggcctgtt aactggtata tacatatata tgtcaggcaa 1680

agtgctgctg gaagtagaat ttgtccaata acaggtcaac ttcagagact atctgatttc 1740

ctaatgtcag agtagaagat tttatgctgc tgtttacaaa agcccaatgt aatgcatagg 1800

aagtatggca tgaacatctt taggagacta atggaaatat tattggtgtt tacccagtat 1860

tccatttttt tcattgtgtt ctctattgct gctctctcac tcccccatga ggtacagcag 1920

aaaggagaac tatccaaaac taatttcctc tgacatgtaa gacgaatgat ttaggtacgt 1980

caaagcagta gtcaaggagg aaagggatag tccaaagact taactggttc atattggact 2040

gataatctct ttaaatggct ttatgctagt ttgacctcat ttgtaaaata tttatgagaa 2100

agttctcatt taaaatgaga tcgttgttta cagtgtatgt actaagcagt aagctatctt 2160

caaatgtcta aggtagtaac tttccatagg gcctccttag atccctaaga tggctttttc 2220

tccttggtat ttctgggtct ttctgacatc agcagagaac tggaaagaca tagccaactg 2280

ctgttcatgt tactcatgac tcctttctct aaaactgcct tccacaattc actagaccag 2340

aagtggacgc aacttaagct gggataatca cattatcatc tgaaaatctg gagttgaaca 2400

gcaaaagaag acaacatttc tcaaatgcac atctcatggc agctaagcca catggctggg 2460

atttaaagcc tttagagcca gcccatggct ttagctacct cactatgctg cttcacaaac 2520

cttgctcctg tgtaaaacta tattctcagt gtagggcaga gaggtctaac accaacataa 2580

ggtactagca gtgtttcccg tattgacagg aatacttaac tcaataattc ttttcttttc 2640

catttagtaa cagttgtgat gactatgttt ctattctaag taattcctgt attctacagc 2700

agatactttg tcagcaatac taagggaaga aacaaagttg aaccgtttct ttaataa 2757

<210> 95
<211> 2207
<212> DNA
<213> Homo sapiens

<400> 95
cacttcctcc ccagacaggg gtagtgcgag gccgggcaca gccttcctgt gtggttttac 60

cgcccagaga gcgtcatgga cctggggaaa ccaatgaaaa gcgtgctggt ggtggctctc 120

cttgtcattt tccaggtatg cctgtgtcaa gatgaggtca cggacgatta catcggagac 180

aacaccacag tggactacac tttgttcgag tctttgtgct ccaagaagga cgtgcggaac 240

tttaaagcct ggttcctccc tatcatgtac tccatcattt gtttcgtggg cctactgggc 300

aatgggctgg tcgtgttgac ctatatctat ttcaagaggc tcaagaccat gaccgatacc 360

tacctgctca acctggcggt ggcagacatc ctcttcctcc tgacccttcc cttctgggcc 420

tacagcgcgg ccaagtcctg ggtcttcggt gtccactttt gcaagctcat ctttgccatc 480

tacaagatga gcttcttcag tggcatgctc ctacttcttt gcatcagcat tgaccgctac 540

gtggccatcg tccaggctgt ctcagctcac cgccaccgtg cccgcgtcct tctcatcagc 600

aagctgtcct gtgtgggcat ctggatacta gccacagtgc tctccatccc agagctcctg 660

tacagtgacc tccagaggag cagcagtgag caagcgatgc gatgctctct catcacagag 720

catgtggagg cctttatcac catccaggtg gcccagatgg tgatcggctt tctggtcccc 780

ctgctggcca tgagcttctg ttaccttgtc atcatccgca ccctgctcca ggcacgcaac 840

tttgagcgca acaaggccat caaggtgatc atcgctgtgg tcgtggtctt catagtcttc 900

cagctgccct acaatggggt ggtcctggcc cagacggtgg ccaacttcaa catcaccagt 960

agcacctgtg agctcagtaa gcaactcaac atcgcctacg acgtcaccta cagcctggcc 1020

tgcgtccgct gctgcgtcaa ccctttcttg tacgccttca tcggcgtcaa gttccgcaac 1080

gatctcttca agctcttcaa ggacctgggc tgcctcagcc aggagcagct ccggcagtgg 1140

tcttcctgtc ggcacatccg gcgctcctcc atgagtgtgg aggccgagac caccaccacc 1200

ttctccccat aggcgactct tctgcctgga ctagagggac ctctcccagg gtccctgggg 1260

tggggatagg gagcagatgc aatgactcag gacatccccc cgccaaaagc tgctcaggga 1320

aaagcagctc tcccctcaga gtgcaagccc ctgctccaga agatagcttc accccaatcc 1380

cagctacctc aaccaatgcc aaaaaaagac agggctgata agctaacacc agacagacaa 1440

cactgggaaa cagaggctat tgtcccctaa accaaaaact gaaagtgaaa gtccagaaac 1500

tgttcccacc tgctggagtg aaggggccaa ggagggtgag tgcaaggggc gtgggagtgg 1560

cctgaagagt cctctgaatg aaccttctgg cctcccacag actcaaatgc tcagaccagc 1620

tcttccgaaa accaggcctt atctccaaga ccagagatag tggggagact tcttggcttg 1680

gtgaggaaaa gcggacatca gctggtcaaa caaactctct gaacccctcc ctccatcgtt 1740

ttcttcactg tcctccaagc cagcgggaat ggcagctgcc acgccgccct aaaagcacac 1800

tcatcccctc acttgccgcg tcgccctccc aggctctcaa caggggagag tgtggtgttt 1860

cctgcaggcc aggccagctg cctccgcgtg atcaaagcca cactctgggc tccagagtgg 1920

ggatgacatg cactcagctc ttggctccac tgggatggga ggagaggaca agggaaatgt 1980

caggggcggg gagggtgaca gtggccgccc aaggcccacg agcttgttct ttgttctttg 2040

tcacagggac tgaaaacctc tcctcatgtt ctgctttcga ttcgttaaga gagcaacatt 2100

ttacccacac acagataaag ttttcccttg aggaaacaac agctttaaaa gaaaaagaaa 2160

aaaaaagtct ttggtaaatg gcaaaaaaaa aaaaaaaaaa aaaaaaa 2207

<210>  96
<211>  2307
<212>  DNA
<213>  Homo sapiens

<400>  96
gattatcaca gattctggag aagagtgagg acttgggttc accacctaca gcctggctcc      60

cgcgacgccg gaggtgaagg tggcttgctc cgaagatgtg gacttgccct gcaccgcccc     120

ctgggatccg caggttccct acacggtctc ctgggtcaag ttattggagg gtggtgaaga     180

gaggatggag acaccccagg aagaccacct caggggacag cactatcatc agaaggggca     240

aaatggttct ttcgacgccc ccaatgaaag gccctattcc ctgaagatcc gaaacactac     300

cagctgcaac tcggggacat acaggtgcac tctgcaggac ccggatgggc agagaaacct     360

aagtggcaag gtgatcttga gagtgacagg atgccctgca cagcgtaaag aagagacttt     420

taagaaatac agagcggaga ttgtcctgct gctggctctg gttattttct acttaacact     480

catcattttc acttgtaagt ttgcacggct acagagtatc ttcccagatt tttctaaagc     540

tggcatggaa cgagcttttc tcccagttac ctccccaaat aagcatttag ggctagtgac     600

tcctcacaag acagaactgg tatgagcagg atttctgcag gttcttcttc ctgaagctga     660

ggctcagggg tgtgcctgtc tgttacactg gaggagagaa gaatgagcct acgctgaaga     720

tggcatcctg tgaagtcctt cacctcactg aaaacatctg gaaggggatc ccaccccatt     780

ttctgtgggc aggcctcgaa aaccatcaca tgaccacata gcatgaggcc actgctgctt     840

ctccatggcc accttttcag cgatgtatgc agctatctgg tcaacctcct ggacattttt     900

tcagtcatat aaaagctatg gtgagatgca gctggaaaag ggtcttggga aatatgaatg     960

cccccagctg gcccgtgaca gactcctgag gacagctgtc ctcttctgca tcttggggac    1020

atctctttga attttctgtg ttttgctgta ccagcccaga tgttttacgt ctgggagaaa    1080

ttgacagatc aagctgtgag acagtgggaa atatttagca aataatttcc tggtgtgaag    1140

gtcctgctat tactaaggag taatctgtgt acaaagaaat aacaagtcga tgaactattc    1200

cccagcaggg tcttttcatc tgggaaagac atccataaag aagcaataaa gaagagtgcc    1260

acatttattt ttatatctat atgtacttgt caaagaaggt ttgtgttttt ctgcttttga    1320

aatctgtatc tgtagtgaga tagcattgtg aactgacagg cagcctggac atagagaggg    1380

agaagaagtc agagagggtg acaagataga gagctattta atggccggct ggaaatgctg    1440

ggctgacggt gcagtctggg tgctcgccca cttgtcccac tatctgggtg catgatcttg    1500

agcaagttcc ttctggtgtc tgctttctcc attgtaaacc acaaggctgt tgcatgggct    1560

aatgaagatc atatacgtga aaattatttg aaaacatata aagcactata cagattcgaa    1620

actccattga gtcattatcc ttgctatgat gatggtgttt tggggatgag agggtgctat    1680

ccatttctca tgttttccat tgtttgaaac aaagaaggtt accaagaagc ctttcctgta 1740

gccttctgta ggaattcttt tggggaagtg aggaagccag gtccacggtc tgttcttgaa 1800

gcagtagcct aacacactcc aagatatgga cacacgggag ccgctggcag aagggacttc 1860

acgaagtgtt gcatggatgt tttagccatt gttggctttc ccttatcaaa cttgggccct 1920

tcccttcttg gtttccaaag gcattttatt gcttgagtta tatgttcact gtccccctaa 1980

tattagggag taaaacggat accaagttga tttagtgttt ttacctctgt cttggctttc 2040

atgttattaa acgtatgcat gtgaagaaag ggtgtttttc tgttttatat tcaactcata 2100

agactttggg ataggaaaaa tgagtaatgg ttactaggct taatacctgg gtgattacat 2160

aatctgtaca atgaaccccc atgatgtaag tttacctatg taacaaacct gcacttatac 2220

ccatgaactt aaaatgaaag ttaaaaataa aaaacatata caaataaaaa aatcccgact 2280

ttgggatgag tgctaggatg ttgtaaa 2307

<210> 97
<211> 2348
<212> DNA
<213> Homo sapiens

<400> 97
agatgtgagt cctcaatgag ctataaccac agccataaat atctctcaaa gatgaggaac 60

attctcatga tgttgacact gcaatttttt gacaatttcc caacactctt aagaaacatt 120

ccccaatctc acacgaaaag tgggggtttt aattttcttg ttcaacttct aaagagaaat 180

tggagaagat aaaactggac actggggaga ccacaacttc atgctgcgtg ggatctccca 240

gctacctgca gtggccacca tgtcttgggt cctgctgcct gtactttggc tcattgttca 300

aactcaagca atagccataa agcaaacacc tgaattaacg ctccatgaaa tagtttgtcc 360

taaaaaactt cacattttac acaaaagaga gatcaagaac aaccagacag aaaagcatgg 420

caaagaggaa aggtatgaac ctgaagttca atatcagatg atcttaaatg gagaagaaat 480

cattctctcc ctacaaaaaa ccaagcacct cctggggcca gactacactg aaacattgta 540

ctcacccaga ggagaggaaa ttaccacgaa acctgagaac atggaacact gttactataa 600

aggaaacatc ctaaatgaaa agaattctgt tgccagcatc agtacttgtg acgggttgag 660

aggatacttc acacatcatc accaaagata ccagataaaa cctctgaaaa gcacagacga 720

gaaagaacat gccgtcttta catctaacca ggaggaacaa gacccagcta accacacatg 780

tggtgtgaag agcactgacg ggaaacaagg cccaattcga atctctagat cactcaaaag 840

cccagagaaa gaagactttc ttcgggcaca gaaatacatt gatctctatt tggtgctgga 900

taatgccttt tataagaact ataatgagaa tctaactctg ataagaagct ttgtgtttga 960

tgtgatgaac ctactcaatg tgatatataa caccatagat gttcaagtgg ccttggtagg 1020

tatggaaatc tggtctgatg gggataagat aaaggtggtg cccagcgcaa gcaccacgtt 1080

tgacaacttc ctgagatggc acagttctaa cctggggaaa aagatccacg accatgctca 1140

gcttctcagc gggattagct tcaacaatcg acgtgtggga ctggcagctt caaattcctt 1200

gtgttcccca tcttcggttg ctgttattga ggctaaaaaa aagaataatg tggctcttgt 1260

aggagtgatg tcacatgagc tgggccatgt ccttggtatg cctgatgttc cattcaacac 1320

caagtgtccc tctggcagtt gtgtgatgaa tcagtatctg agttcaaaat tcccaaagga 1380

tttcagtaca tcttgccgtg cacattttga aagatacctt ttatctcaga aaccaaagtg 1440

cctgctgcaa gcacctattc ctacaaatat aatgacaaca ccagtgtgtg ggaaccacct 1500

tctagaagtg ggagaagact gtgattgtgg ctctcctaag gagtgtacca atctctgctg 1560

tgaagcccta acgtgtaaac tgaagcctgg aactgattgc ggaggagatg ctccaaacca 1620

taccacagag tgaatccaaa agtctgcttc actgagatgc taccttgcca ggacaagaac 1680

caagaactct aactgtccca ggaatcttgt gaattttcac ccataatggt ctttcacttg 1740

tcattctact ttctatattg ttatcagtcc aggaaacagg taaacagatg taattagaga 1800

cattggctct ttgtttaggc ctaatctttc tttttacttt tttttttctt ttttcttttt 1860

ttttaaagat catgaatttg tgacttagtt ctgccctttg gagaacaaaa gaaagcagtc 1920

ttccatcaaa tcaccttaaa atgcacggct aaactattca gagttaacac tccagaattg 1980

ttaaattaca agtactatgc tttaatgctt ctttcatctt actagtatgg cctataaaaa 2040

aaataatacc acttgatggg tgaaggcttt ggcaatagaa agaagaatag aattcaggtt 2100

ttatgttatt cctctgtgtt cacttcgcct tgctcttgaa agtgcagtat ttttctacat 2160

catgtcaaga atgattcaat gtaaatattt ttcattttat catgtatatc ctatacacac 2220

atctccttca tcatcatata tgaagtttat tttgagaagt ctacattgct tacattttaa 2280

ttgagccagc aaagaaggct taatgattta ttgaaccata atgtcaataa aaacacaact 2340

tttgaggc 2348

<210> 98
<211> 2517
<212> DNA
<213> Homo sapiens

<400> 98
aaaacactca ttttgtttta tagcatgaca ggctgtctga ttccatcttt ataaccaaag 60

ccaattaaga tcttaaaacc aaacatataa cttcatcttt ttacaagtac ttagagcctg 120

agttgctcca caggaatcca ggaactgggc acaggaaaag gatctaagct ggtggtgtgg 180

gaagatggaa accaacttct ccattcctct gaatgaaact gaggaggtgc tccctgagcc 240

tgctggccac accgttctgt ggatcttctc attgctagtc cacggagtca cctttgtctt 300

cggggtcctg ggcaatgggc ttgtgatctg ggtggctgga ttccggatga cacgcacagt 360

caacaccatc tgttacctga acctggccct agctgacttc tctttcagtg ccatcctacc 420

attccgaatg gtctcagtcg ccatgagaga aaaatggcct tttggctcat tcctatgtaa 480

gttagttcat gttatgatag acatcaacct gtttgtcagt gtctacctga tcaccatcat 540

tgctctggac cgctgtattt gtgtcctgca tccagcctgg gcccagaacc atcgcaccat 600

gagtctggcc aagagggtga tgacgggact ctggattttc accatagtcc ttaccttacc 660

aaatttcatc ttctggacta caataagtac tacgaatggg gacacatact gtattttcaa 720

ctttgcattc tggggtgaca ctgctgtaga gaggttgaac gtgttcatta ccatggccaa 780

ggtctttctg atcctccact tcattattgg cttcagcgtg cctatgtcca tcatcacagt 840

ctgctatggg atcatcgctg ccaaaattca cagaaaccac atgattaaat ccagccgtcc 900

cttacgtgtc ttcgctgctg tggtggcttc tttcttcatc tgttggttcc cttatgaact 960

aattggcatt ctaatggcag tctggctcaa agagatgttg ttaaatggca aatacaaaat 1020

cattcttgtc ctgattaacc caacaagctc cttggccttt tttaacagct gcctcaaccc 1080

aattctctac gtctttatgg gtcgtaactt ccaagaaaga ctgattcgct ctttgcccac 1140

tagtttggag agggccctga ctgaggtccc tgactcagcc cagaccagca acacagacac 1200

cacttctgct tcacctcctg aggagacgga gttacaagca atgtgaggtc ggggatattt 1260

ttgggctctg tctctttcta ccctgcgtta agcggaaaaa aaaaattctg acagtgtttt 1320

tcttcctctt tcataccacc accaccacaa tcatcaacat aaaggaagtc tgtaccaaat 1380

ctgtaggggg tttttcccac aaccaagcaa tagacaccag ctgggtgtcc tacaattaaa 1440

ttccaacact atctacctgg agctactgtc agatcccaca ggtttaaggg ctcattcccc 1500

aagtctgctc ctccagttga gacacaagtc acaaatccag gcttctgaga cttcggacca 1560

accagcttca atcagggttc ccactacccc ctctttgggg gtagagtggc tcatggaact 1620

cagagaaaca tttatttcgg cttgctggtt tattataaaa gcaaggttta ttataaaaga 1680

tactacaaag gatacagatg aagaggcaca tagggcaagg tacggggttc cacgccctcc 1740

ctgagtgcat caccctctgg gaacctccgt gtgttcacgt ctcatgaagc tctccaaatc 1800

cagtcctctt gggtttttat ggaagcttca tgatgtcagc attctttcct ccagtgtata 1860

ggatgggatc ctctctgggg agggtcttaa gacccacaat tagaaaggca agggaagatt 1920

agagtcctgc tttggggtag atgaaaggaa aggagagaga ttctgtttcc tgaggcttaa 1980

tacacccaac attataacaa aggactgtag caagggctat gggagttctg aagcagaaac 2040

catgggctaa aaccaacata catcttaata ccagataccc taatcccagt cctaacttca 2100

tttaaccttg gtcacattga gtcattccag gatgagtggc tcaagtattt cctcagggaa 2160

aatacttctg tgccccctga tttgagggta agaagtagat aatgaggcca ctgtgggtgt 2220

tattttttca tgtctggacc tcagcctata tcctgagact aagtggaagt gggaaaagag 2280

tacaagagaa gagacaaagt ggggatattt gtaaggctta gatgagatag tgttttttta 2340

gaaaaaaact ttatcttacc attaagtaaa atgtttgcca taggctttct ggggctttct 2400

ctttttaaag tcagactgtt gaaggtttct tctattctta tttgttaaga gttttctttt 2460

attgtttaaa tcatgaatga atgttgaatt ttattaaatg cagtttctgt aaatatt 2517

<210> 99
<211> 2790
<212> DNA
<213> Homo sapiens

<400> 99
ccctttctgt atttgagttc taccgtcagt cctggcatta tttctctctc tacaaggagc 60

cttaggaggt acggggagct cgcaaatact ccttttggtt tattcttacc accttgcttc 120

tgtgttcctt gggaatgctg ctgtgcttat gcatctggtc tctttttgga gctacagtgg 180

acaggcattt gtgacagcac tatgggactg agtaacattc tctttgtgat ggccttcctg 240

ctctctggtg ctgctcctct gaagattcaa gcttatttca atgagactgc agacctgcca 300

tgccaatttg caaactctca aaaccaaagc ctgagtgagc tagtagtatt ttggcaggac 360

caggaaaact tggttctgaa tgaggtatac ttaggcaaag agaaatttga cagtgttcat 420

tccaagtata tgggccgcac aagttttgat tcggacagtt ggaccctgag acttcacaat 480

cttcagatca aggacaaggg cttgtatcaa tgtatcatcc atcacaaaaa gcccacagga 540

atgattcgca tccaccagat gaattctgaa ctgtcagtgc ttgctaactt cagtcaacct 600

gaaatagtac caatttctaa tataacagaa aatgtgtaca taaatttgac ctgctcatct 660

atacacggtt acccagaacc taagaagatg agtgttttgc taagaaccaa gaattcaact 720

atcgagtatg atggtattat gcagaaatct caagataatg tcacagaact gtacgacgtt 780

tccatcagct tgtctgtttc attccctgat gttacgagca atatgaccat cttctgtatt 840

ctggaaactg acaagacgcg gcttttatct tcacctttct ctatagagct tgaggaccct 900

cagcctcccc cagaccacat tccttggatt acagctgtac ttccaacagt tattatatgt 960

gtgatggttt tctgtctaat tctatggaaa tggaagaaga agaagcggcc tcgcaactct 1020

tataaatgtg gaaccaacac aatggagagg gaagagagtg aacagaccaa gaaaagagaa 1080

aaaatccata tacctgaaag atctgatgaa gcccagcgtg tttttaaaag ttcgaagaca 1140

tcttcatgcg acaaaagtga tacatgtttt taattaaaga gtaaagccca tacaagtatt 1200

cattttttct accctttcct ttgtaagttc ctgggcaacc tttttgattt cttccagaag 1260

gcaaaaagac attaccatga gtaataaggg ggctccagga ctccctctaa gtggaatagc 1320

ctccctgtaa ctccagctct gctccgtatg ccaagaggag actttaattc tcttactgct 1380

tcttttcact tcagagcaca cttatgggcc aagcccagct taatggctca tgacctggaa 1440

ataaaattta ggaccaatac ctcctccaga tcagattctt ctcttaattt catagattgt 1500

gttttttttt taaatagacc tctcaatttc tggaaaactg ccttttatct gcccagaatt 1560

ctaagctggt gccccactga attttgtgta cctgtgacta aacaactacc tcctcagtct 1620

gggtgggact tatgtattta tgaccttata gtgttaatat cttgaaacat agagatctat 1680

gtactgtaat agtgtgatta ctatgctcta gagaaaagtc tacccctgct aaggagttct 1740

catccctctg tcagggtcag taaggaaaac ggtggcctag ggtacaggca acaatgagca 1800

gaccaaccta aatttgggga aattaggaga ggcagagata gaacctggag ccacttctat 1860

ctgggctgtt gctaatattg aggaggcttg ccccacccaa caagccatag tggagagaac 1920

tgaataaaca ggaaaatgcc agagcttgtg aaccctgttt ctcttgaaga actgactagt 1980

gagatggcct ggggaagctg tgaaagaacc aaaagagatc acaatactca aaagagagag 2040

agagagaaaa aagagagatc ttgatccaca gaaatacatg aaatgtctgg tctgtccacc 2100

ccatcaacaa gtcttgaaac aagcaacaga tggatagtct gtccaaatgg acataagaca 2160

gacagcagtt tccctggtgg tcagggaggg gttttggtga tacccaagtt attgggatgt 2220

catcttcctg gaagcagagc tggggaggga gagccatcac cttgataatg ggatgaatgg 2280

aaggaggctt aggactttcc actcctggct gagagaggaa gagctgcaac ggaattagga 2340

agaccaagac acagatcacc cggggcttac ttagcctaca gatgtcctac gggaacgtgg 2400

gctggcccag catagggcta gcaaatttga gttggatgat tgtttttgct caaggcaacc 2460

agaggaaact tgcatacaga gacagatata ctgggagaaa tgactttgaa aacctggctc 2520

taaggtggga tcactaaggg atggggcagt ctctgcccaa acataaagag aactctgggg 2580

agcctgagcc acaaaaatgt tcctttattt tatgtaaacc ctcaagggtt atagactgcc 2640

atgctagaca agcttgtcca tgtaatattc ccatgttttt accctgcccc tgccttgatt 2700

agactcctag cacctggcta gtttctaaca tgttttgtgc agcacagttt ttaataaatg 2760

cttgttacat tcatttaaaa aaaaaaaaaa 2790

<210> 100
<211> 2312
<212> DNA
<213> Homo sapiens

<400> 100
ctccacaggt ccgccccaat ccccgctcac acttgggaaa cttgggactg cgctggggcc 60

gcgtgtggca cctcaggggg gcggcccccg gcctcaagag gagggggagg agaaggagga 120

agaggaggaa gtgagcccga aggatccgct cggagctgtt tgtccagctg tttctattcg 180

cacccggagc agtacagcca gaagggggcc gagccgaagg tggctggctt taggcgctaa 240

tttccaactc ttttcctcac agcttgtctt ttccaggcac cctggagtcc cctcaggcca 300

gctcggtggg cgcgcacctg ccagccgccc ctgacctcgc aggccaggcg acctccgagc 360

ctgagaagat ggcccagtcc aagctcgatt gccgctcacc tgtcggcctc gactgctgca 420

actgctgcct ggacctggcc catcggagtg ggctccagcg aggcagcagc ggggagaaca 480

acaacccggg cagccctaca gtgagcaact ttcggcagct gcaggaaaag ctggtctttg 540

agaacctcaa taccgacaag ctcaacagca taatgcggca ggattcgcta gagccggtgc 600

tgcgggaccc ctgctacctg atcaacgagg gcatctgcaa ccgcaacatc gaccagacca 660

tgctctccat cctgctcttc ttccacagtg cctccggagc cagcgtggtg gccatagaca 720

acaagatcga acaggccatg gatctggtga agaatcatct gatgtatgct gtgagagagg 780

aggtggagat cctgaaggag cagatccgag agctggtgga gaagaactcc cagctagagc 840

gtgagaacac cctgttgaag accctggcaa gcccagagca gctggagaag ttccagtcct 900

gtctgagccc tgaagagcca gctcccgaat ccccacaagt gcccgaggcc cctggtggtt 960

ctgcggtgta agtggctctg tcctcagggt gggcagagcc actaaacttg ttttacctag 1020

ttctttccag tttgtttttg gctccccaag catcatctca cgaggagaac tttacaccta 1080

gcacagctgg tgccaagaga tgtcctaagg acatggccac ctgggtccac tccagcgaca 1140

gacccctgac aagagcaggt ctctggaggc tgagttgcat ggggcctagt aacaccaagc 1200

cagtgagcct ctaatgctac tgcgccctgg gggctcccag ggcctgggca acttagctgc 1260

aactggcaaa ggagaagggt agtttgaggt gtgacaccag tttgctccag aaagtttaag 1320

gggtctgttt ctcatctcca tggacatctt caacagcttc acctgacaac gactgttcct 1380

atgaagaagc cacttgtgtt ttaagcagag gcaacctctc tcttctcctc tgtttcgtga 1440

aggcagggga cacagatggg agagattgag ccaagtcagc cttctgttgg ttaatatggt 1500

ataatgcatg gctttgtgca cagcccagtg tgggattaca gctttgggat gaccgcttac 1560

aaagttctgt ttggttagta ttggcatagt ttttctatat agccataaat gcgtatatat 1620

acccataggg ctagatctgt atcttagtgt agcgatgtat acatatacac atccacctac 1680

atgttgaagg gcctaaccag ccttgggagt attgactggt cccttacctc ttatggctaa 1740

gtctttgact gtgttcattt accaagttga cccagtttgt cttttaggtt aagtaagact 1800

cgagagtaaa ggcaaggagg ggggccagcc tctgaatgcg gccacggatg ccttgctgct 1860

gcaacccttt ccccagctgt ccactgaaac gtgaagtcct gttttgaatg ccaaacccac 1920

cattcactgg tgctgactac atagaatggg gttgagagaa gatcagtttg ggcttcacag 1980

tgtcatttga aaacgttttt tgttttgttt tgtaattatt gtggaaaact ttcaagtgaa 2040

cagaaggatg gtgtcctact gtggatgagg gatgaacaag gggatggctt tgatccaatg 2100

gagcctggga ggtgtgccca gaaagcttgt ctgtagcggg ttttgtgaga gtgaacactt 2160

tccacttttt gacaccttat cctgatgtat ggttccagga tttggatttt gattttccaa 2220

atgtagcttg aaatttcaat aaactttgct ctgtttttct aaaaataaaa aaaaaaaaaa 2280

aaaaaaaaaa aaaaaaaaaa aaaaaaaaaa aa 2312

<210> 101
<211> 1944
<212> DNA
<213> Homo sapiens

<400> 101
aatttctcac tgcccctgtg ataaactgtg gtcactggct gtggcagcaa ctattataag 60

atgctctgaa aactcttcag acactgaggg gcaccagagg agcagactac aagaatggca 120

cacgctatgg aaaactcctg gacaatcagt aaagagtacc atattgatga agaagtgggc 180

tttgctctgc caaatccaca ggaaaatcta cctgattttt ataatgactg gatgttcatt 240

gctaaacatc tgcctgatct catagagtct ggccagcttc gagaaagagt tgagaagtta 300

aacatgctca gcattgatca tctcacagac cacaagtcac agcgccttgc acgtctagtt 360

ctgggatgca tcaccatggc atatgtgtgg ggcaaaggtc atggagatgt ccgtaaggtc 420

ttgccaagaa atattgctgt tccttactgc caactctcca agaaactgga actgcctcct 480

attttggttt atgcagactg tgtcttggca aactggaaga aaaaggatcc taataagccc 540

ctgacttatg agaacatgga cgttttgttc tcatttcgtg atggagactg cagtaaagga 600

ttcttcctgg tctctctatt ggtggaaata gcagctgctt ctgcaatcaa agtaattcct 660

actgtattca aggcaatgca aatgcaagaa cgggacactt tgctaaaggc gctgttggaa 720

atagcttctt gcttggagaa agcccttcaa gtgtttcacc aaatccacga tcatgtgaac 780

ccaaaagcat ttttcagtgt tcttcgcata tatttgtctg gctggaaagg caacccccag 840

ctatcagacg gtctggtgta tgaagggttc tgggaagacc caaaggagtt tgcagggggc 900

agtgcaggcc aaagcagcgt ctttcagtgc tttgacgtcc tgctgggcat ccagcagact 960

gctggtggag gacatgctgc tcagttcctc caggacatga gaagatatat gccaccagct 1020

cacaggaact tcctgtgctc attagagtca aatccctcag tccgtgagtt tgtcctttca 1080

aaaggtgatg ctggcctgcg ggaagcttat gacgcctgtg tgaaagctct ggtctccctg 1140

aggagctacc atctgcaaat cgtgactaag tacatcctga ttcctgcaag ccagcagcca 1200

aaggagaata agacctctga agacccttca aaactggaag ccaaaggaac tggaggcact 1260

gatttaatga atttcctgaa gactgtaaga agtacaactg agaaatccct tttgaaggaa 1320

ggttaatgta acccaacaag agcacatttt atcatagcag agacatctgt atgcattcct 1380

gtcattaccc attgtaacag agccacaaac taatactatg caatgtttta ccaataatgc 1440

aatacaaaag acctcaaaat acctgtgcat ttcttgtagg aaaacaacaa aaggtaatta 1500

tgtgtaatta tactagaagt tttgtaatct gtatcttatc attggaataa aatgacattc 1560

aataaataaa aatgcataag atatattctg tcggctgggc gcggtggctc acgcctgtaa 1620

tcccagcact ttgggaggcc gaggcgggcg gatcacaagg tcaggagatc gagaccatct 1680

tggctaacac ggtgaaaccc cgtctctact aaaaatacaa aaaattagcc gggcgcggtg 1740

gcgggcacct gtagtcccag ctactcggga ggctgaggca ggagaatggc gtgaacctgg 1800

gaggcggagc ttgcagtgag ccaagattgt gccactgcaa tccggcctgg gctaaagagc 1860

gggactccgt ctcaaaaaaa aaaaaaaaaa gatatattct gtcataataa ataaaaatgc 1920

ataagatata aaaaaaaaaa aaaa 1944

<210> 102
<211> 5266
<212> DNA
<213> Homo sapiens

<400> 102
gcttaaaaat ttctgtgtct tacacagaag atagaaaaaa tagagtgtct ccaattggat 60

ggatttttta aaaaatttgg ttattgtaat ggatttattt tttcttagag ctgagctgat 120

tgtactttgg ccaactaatg ggttaatact gtcaagggaa attagccctg actaaacatt 180

gccgctggct catgaatgca ctaggcttgg ggcagtataa aaactcagag aaatcagtgt 240

gtaggagaca cagaaatcag tgtcactcag tgacagaagc aacaataatt gtgaaaaata 300

cttcagcagt tatggactca tctgtcattc aaaggaaaaa agtagctgtc attggtggtg 360

gcttggttgg ctcattacaa gcatgctttc ttgcaaagag gaatttccag attgatgtat 420

atgaagctag ggaagatact cgagtggcta ccttcacacg tggaagaagc attaacttag 480

ccctttctca tagaggacga caagccttga aagctgttgg cctggaagat cagattgtat 540

cccaaggtat tcccatgaga gcaagaatga tccactctct ttcaggaaaa aagtctgcaa 600

ttccctatgg gacaaagtct cagtatattc tttctgtaag cagagaaaat ctaaacaagg 660

atctattgac tgctgctgag aaatacccca atgtgaaaat gcactttaac cacaggctgt 720

tgaaatgtaa tccagaggaa ggaatgatca cagtgcttgg atctgacaaa gttcccaaag 780

atgtcacttg tgacctcatt gtaggatgtg atggagccta ttcaactgtc agatctcacc 840

tgatgaagaa acctcgcttt gattacagtc agcagtacat tcctcatggg tacatggagt 900

tgactattcc acctaagaac ggagattatg ccatggaacc taattatctg catatttggc 960

ctagaaatac ctttatgatg attgcacttc ctaacatgaa caaatcattc acatgtactt 1020

tgttcatgcc ctttgaagag tttgaaaaac ttctaaccag taatgatgtg gtagatttct 1080

tccagaaata ctttccggat gccatccctc taattggaga gaaactccta gtgcaagatt 1140

tcttcctgtt gcctgcccag cccatgatat ctgtaaagtg ctcttcattt cactttaaat 1200

ctcactgtgt actgctggga gatgcagctc atgctatagt gccgtttttt gggcaaggaa 1260

tgaatgcggg ctttgaagac tgcttggtat ttgatgagtt aatggataaa ttcagtaacg 1320

accttagttt gtgtcttcct gtgttctcaa gattgagaat cccagatgat cacgcgattt 1380

cagacctatc catgtacaat tacatagaga tgcgagcaca tgtcaactca agctggttca 1440

tttttcagaa gaacatggag agatttcttc atgcgattat gccatcgacc tttatccctc 1500

tctatacaat ggtcactttt tccagaataa gataccatga ggctgtgcag cgttggcatt 1560

ggcaaaaaaa ggtgataaac aaaggactct tttcttgggg atcactgata gccatcagca 1620

gtacctacct acttatacac tacatgtcac cacgatcttt cctccgcttg agaagaccat 1680

ggaactggat agctcacttc cggaatacaa catgtttccc cgcaaaggcc gtggactccc 1740

tagaacaaat ttccaatctc attagcaggt gatagaaagg ttttgtggta gcaaatgcat 1800

gatttctctg tgaccaaaat taagcatgaa aaaaatgttt ccattgccat atttgattca 1860

ctagtggaag atagtgttct gcttataatt aaactgaatg tagagtatct ctgtatgtta 1920

attgcaatta ctggttgggg ggtgcatttt aaaagatgaa acatgcagct tccctacatt 1980

acacacactc aggttgagtc attctaacta taaaagtgca atgactaaga tccttcactt 2040

ctctgaaagt aaggccctag atgcctcagg gaagacagta atcatgcctt ttctttaaaa 2100

gacacaatag gactcgcaac agcattgact caacacctag gactaaaaat cacaacttaa 2160

ctagcatgtt aactgcactt ttcattacgt gaatggaact tacctaacca cagggctcag 2220

acttactaga taaaaccaga aatggaaata aggaattcag gggagttcca gagacttaca 2280

aaatgaactc attttatttt cccaccttca aatataagta ttatcatcta tctgtttatc 2340

gtctatctat ctatcatcta tctatctatc tatcatctat ctatctatct atctatctat 2400

ctatctatct atctatctct atttatttat gtatttagag atcaggtctc actctgttga 2460

ccaggctgga gtgcagtggt gagatctggg ttcactgcaa cctctgcctc ctgggctcaa 2520

gcaatcctcc cacttcagcc tcccaaatag ctggggctac catggtattt ttcagtagag 2580

accgggtctt gccatgctgc ccaggccagt ctcaaactcc tggcctcatg tgatctgccc 2640

acctcagcct cccaaagtac agggattaga gttgtgagcc accgctgcca gcccagagtt 2700

accctctaaa gataagaaaa aggctattaa tatcatacta agtgaaggac aggaaagggt 2760

tttattcata aattaaatgt ctacatgtgc cagaatggaa aggaaacaag gggagacaac 2820

ttttatagaa atacaaagcc attactttat tcaatttcag accctcagaa gcaatttact 2880

aatttattct tcgactacat actgcagcag aaccagcaat acacttgatt tttaaaagca 2940

catttagtga aatgttttct ttggttcatc cttctttaac aggctgctga gtcactcaga 3000

aatccttcaa acatgattaa ttatgaagat gaaacactag agtcatataa gaaataaaaa 3060

ttgggcaata aaataaaatg attcagtgtt tcttttctat attgtcaatg aaaaccttga 3120

gttctaataa tccatgttca gtttgtaggg aaagaaaaaa taatttttcc ttctacccac 3180

tttaggttcc ttggctgggg cccctataac aaaagacaga ttgacaagag aaaaacaaac 3240

ataaatttat tagcgggtat atgtaatata tatgtgggaa atacagggga atgagcaaat 3300

ctcaaagagc tggcgtctta gaactccctg gcttatatag catcgacaaa gaacagtaaa 3360

tttttagaga aacaacaaaa caaagaaaaa gagctttgag tctgtagggg cagcaatttg 3420

ggggaagcaa atatatggga gtttgccttg tagattcctc tggtgctggt ctccaggctg 3480

acaaggattc aaagttgtct ctgaaactcc tctttgtcat actgcacata taaaacgtct 3540

tttgtttcca acaagaggat tttctttttc attctagaat tatctccttg ataacttgat 3600

cagatatagg acatgacact gaatagagtc caacagtaca aaaaaaattc agtatgttct 3660

agctacttca cacatgtgta cgcgacagtt atttttacag taaggtattt tcgagaaaaa 3720

tgcattacgt gttttggaaa atagagtaat ttaaaaaata tatttgaaat gaaaatctcc 3780

aacacattag aagatgatga tgttagatgc ccatcgtgtg ccacaagtgg ttttttcatt 3840

atgtaaagca cccgttgaat taaaagaatt tgtttttgtt caacctcttc ctgaggccca 3900

agagcatatg ggcaattcgg atttcctgct ggaccacaag gttctgttga tattacatag 3960

aacgggtatt ccagacactt cttatgatga aagtccaaaa gtggcatcca atttaaggcc 4020

ccatctttcg ttgccattct tcattcctac aaaggacgaa cttggattac atcaactttg 4080

gacccattgg ttttgtcgct gtcgtcaact gacagtgatt catcactggt gatgataaaa 4140

atgatggaag aagagttgaa agtcactttt ttctttggcc tgtccccatc tttctgtgac 4200

atcacaatgg gtctgatctg catttcactt ccagctgctg gtaggtcttt agcaggcctc 4260

tggcacctca gcagtcggag gcacagaagc tgcaaaaggg atcttcgaaa ctgggcagag 4320

aaaaaataaa gtggaatatt aagtaaaagt tgggcactaa tctggattaa cattcgagga 4380

aatcagttga gctgaattta agttgttttt tgtttgttag caggtgtgga tgtggggtta 4440

tgtggtcatg ctcagatcta cctaaatcac cccagagctt tatgtctttt attcattcta 4500

attcttatta accggaatat gtaggaccat ttcaatacct tgtaatcctc caagcttcaa 4560

tctgcacaca ctttctatga gggcaggtac aactattaag agattttgaa cattaagtta 4620

gtccacaaat attcagtggg catctactag gtgacagcca ctgtgctata attagagact 4680

ttttactata agcatcaaaa acagataagg ctcttcctgg cagagtttac agcctggtgt 4740

acttgctaat gtctctttaa ttaggtgaag aatttttttt ttctatcgaa attactaatc 4800

agttggggaa aaaaatacta tagcagacag cactaatgtc atcaacaaac attgttcttc 4860

tccgtgtcct gggtacaaca tcgaataata tttcttggcc tcctttccgc ttctcctctc 4920

tgctgttcct ctctacaaga acctgggagg ccaacgccta aagatcataa tatcacacaa 4980

tggaaggaac ctagattcct aaatgactgc ataggacaga tcccatctcc tccacccaat 5040

acattattag actgaactgt gacctgaaat gagcaataaa ctctgtatta attcactgaa 5100

atgttggggt tgcttgttat agtagtcggt ccatcatgac cagtaaaaca taaatcaaaa 5160

gttaatgtaa ttgttatccc attatttaga gcgaaataaa tgttgaatat atggactttc 5220

tcagattagg aaataccaat taaaaatata ataaatagct acattg 5266

<210> 103
<211> 2217
<212> DNA
<213> Homo sapiens

<400> 103
ccccgccgcc gccgcccttc gcgccctggg ccatctccct cccacctccc tccgcggagc 60

agccagacag cgagggcccc ggccgggggc agggggacgg ccccgtccgg ggcacccccc 120

cggctctgag ccgcccgcgg ggccggcctc ggcccggagc ggaggaagga gtcgccgagg 180

agcagcctga ggccccagag tctgagacga gccgccgccg cccccgccac tgcggggagg 240

agggggagga ggagcgggag gagggacgag ctggtcggga gaagaggaaa aaaacttttg 300

agacttttcc gttgccgctg ggagccggag gcgcggggac ctcttggcgc gacgctgccc 360

cgcgaggagg caggacttgg ggaccccaga ccgcctccct ttgccgccgg ggacgcttgc 420

tccctccctg ccccctacac ggcgtccctc aggcgccccc attccggacc agccctcggg 480

agtcgccgac ccggcctccc gcaaagactt ttccccagac ctcgggcgca ccccctgcac 540

gccgccttca tccccggcct gtctcctgag cccccgcgca tcctagaccc tttctcctcc 600

aggagacgga tctctctccg acctgccaca gatcccctat tcaagaccac ccaccttctg 660

gtaccagatc gcgcccatct aggttatttc cgtgggatac tgagacaccc ccggtccaag 720

cctcccctcc accactgcgc ccttctccct gaggacctca gctttccctc gaggccctcc 780

taccttttgc cgggagaccc ccagcccctg caggggcggg gcctccccac cacaccagcc 840

ctgttcgcgc tctcggcagt gccggggggc gccgcctccc ccatgccgcc ctccgggctg 900

cggctgctgc cgctgctgct accgctgctg tggctactgg tgctgacgcc tggccggccg 960

gccgcgggac tatccacctg caagactatc gacatggagc tggtgaagcg gaagcgcatc 1020

gaggccatcc gcggccagat cctgtccaag ctgcggctcg ccagcccccc gagccagggg 1080

gaggtgccgc ccggcccgct gcccgaggcc gtgctcgccc tgtacaacag cacccgcgac 1140

cgggtggccg gggagagtgc agaaccggag cccgagcctg aggccgacta ctacgccaag 1200

gaggtcaccc gcgtgctaat ggtggaaacc cacaacgaaa tctatgacaa gttcaagcag 1260

agtacacaca gcatatatat gttcttcaac acatcagagc tccgagaagc ggtacctgaa 1320

cccgtgttgc tctcccgggc agagctgcgt ctgctgaggc tcaagttaaa agtggagcag 1380

cacgtggagc tgtaccagaa atacagcaac aattcctggc gatacctcag caaccggctg 1440

ctggcaccca gcgactcgcc agagtggtta tcttttgatg tcaccggagt tgtgcggcag 1500

tggttgagcc gtggagggga aattgagggc tttcgcctta gcgcccactg ctcctgtgac 1560

agcagggata acacactgca agtggacatc aacgggttca ctaccggccg ccgaggtgac 1620

ctggccacca ttcatggcat gaaccggcct ttcctgcttc tcatggccac cccgctggag 1680

agggcccagc atctgcaaag ctcccggcac cgccgagccc tggacaccaa ctattgcttc 1740

agctccacgg agaagaactg ctgcgtgcgg cagctgtaca ttgacttccg caaggacctc 1800

ggctggaagt ggatccacga gcccaagggc taccatgcca acttctgcct cgggccctgc 1860

ccctacattt ggagcctgga cacgcagtac agcaaggtcc tggccctgta caaccagcat 1920

aacccgggcg cctcggcggc gccgtgctgc gtgccgcagg cgctggagcc gctgcccatc 1980

gtgtactacg tgggccgcaa gcccaaggtg gagcagctgt ccaacatgat cgtgcgctcc 2040

tgcaagtgca gctgaggtcc cgccccgccc cgccccgccc cggcaggccc ggccccaccc 2100

cgccccgccc ccgctgcctt gcccatgggg gctgtattta aggacacccg tgccccaagc 2160

ccacctgggg ccccattaaa gatggagaga ggactgcgga aaaaaaaaaa aaaaaaa 2217

<210> 104
<211> 1629
<212> DNA
<213> Homo sapiens

<400> 104
acacatcagg ggcttgctct tgcaaaacca aaccacaaga cagacttgca aaagaaggca 60

tgcacagctc agcactgctc tgttgcctgg tcctcctgac tggggtgagg gccagcccag 120

gccagggcac ccagtctgag aacagctgca cccacttccc aggcaacctg cctaacatgc 180

ttcgagatct ccgagatgcc ttcagcagag tgaagacttt ctttcaaatg aaggatcagc 240

tggacaactt gttgttaaag gagtccttgc tggaggactt taagggttac ctgggttgcc 300

aagccttgtc tgagatgatc cagttttacc tggaggaggt gatgccccaa gctgagaacc 360

aagacccaga catcaaggcg catgtgaact ccctggggga gaacctgaag accctcaggc 420

tgaggctacg gcgctgtcat cgatttcttc cctgtgaaaa caagagcaag gccgtggagc 480

aggtgaagaa tgcctttaat aagctccaag agaaaggcat ctacaaagcc atgagtgagt 540

ttgacatctt catcaactac atagaagcct acatgacaat gaagatacga aactgagaca 600

tcagggtggc gactctatag actctaggac ataaattaga ggtctccaaa atcggatctg 660

gggctctggg atagctgacc cagccccttg agaaacctta ttgtacctct cttatagaat 720

atttattacc tctgatacct caacccccat ttctatttat ttactgagct tctctgtgaa 780

cgatttagaa agaagcccaa tattataatt tttttcaata tttattattt tcacctgttt 840

ttaagctgtt tccatagggt gacacactat ggtatttgag tgttttaaga taaattataa 900

gttacataag ggaggaaaaa aaatgttctt tggggagcca acagaagctt ccattccaag 960

cctgaccacg ctttctagct gttgagctgt tttccctgac ctccctctaa tttatcttgt 1020

ctctgggctt ggggcttcct aactgctaca aatactctta ggaagagaaa ccagggagcc 1080

cctttgatga ttaattcacc ttccagtgtc tcggagggat tcccctaacc tcattcccca 1140

accacttcat tcttgaaagc tgtggccagc ttgttattta taacaaccta aatttggttc 1200

taggccgggc gcggtggctc acgcctgtaa tcccagcact ttgggaggct gaggcgggtg 1260

gatcacttga ggtcaggagt tcctaaccag cctggtcaac atggtgaaac cccgtctcta 1320

ctaaaaatac aaaaattagc cgggcatggt ggcgcgcacc tgtaatccca gctacttggg 1380

aggctgaggc aagagaattg cttgaaccca ggagatggaa gttgcagtga gctgatatca 1440

tgcccctgta ctccagcctg ggtgacagag caagactctg tctcaaaaaa taaaaataaa 1500

aataaatttg gttctaatag aactcagttt taactagaat ttattcaatt cctctgggaa 1560

tgttacattg tttgtctgtc ttcatagcag attttaattt tgaataaata aatgtatctt 1620

attcacatc 1629

<210> 105
<211> 12
<212> PRT
<213> Artificial Sequence

<220>
<223> peptide

<400> 105

His His Leu Gly Gly Ala Lys Gln Ala Gly Asp Val
1               5                   10